# EUROPEAN PATENT SPECIFICATION

(11) **EP 2 021 324 B1**
(45) Date of publication and mention of the grant of the patent: **14.12.2011**
(21) Application number: 07729447.8
(22) Date of filing: 23.05.2007
(51) Int. Cl.: C07D 209/20, C07D 239/34, C07D 239/52, C07D 239/60, C07D 261/14, C07D 401/12, C07D 401/14, C07D 403/12, C07D 405/04, C07D 405/06, C07D 413/12, C07D 417/04, A61K 31/40, A61K 31/41, A61K 31/435

(54) **NITRATED HETEROCYCLIC COMPOUNDS AS ENDOTHELIN RECEPTOR ANTAGONIST**
NITRIERTE HETEROCYCLISCHE VERBINDUNGEN ALS ENDOTHELINREZEPTORANTAGONISTEN
COMPOSÉS HÉTÉROCYCLIQUES NITRÉS EN TANT QU'ANTAGONISTES DE RÉCEPTEUR DE L'ENDOTHÉLINE

(30) Priority: 29.05.2006 EP 06114617
(43) Date of publication of application: 11.02.2009
(73) Proprietor: Nicox S.A., 06560 Sophia Antipolis - Valbonne (FR)
(72) Inventor: ALMIRANTE, Nicoletta, 20155 Milano (IT); BIONDI, Stefano, 20016 Pero (Milano) (IT); ONGINI, Ennio, 20090 Segrate (IT)
(74) Representative: Barchielli, Giovanna
(86) International application number: PCT/EP2007/055012
(87) International publication number: WO 2007/137980

(56) References cited:
- WO-A-00/42035
- WO-A-97/04774
- WO-A-99/11629
- NEIDHART W ET AL: "Discovery of Ro 48-5695: a potent mixed endothelin receptor antagonist optimized from Bosentan" BIOORGANIC & MEDICINAL CHEMISTRY LETTERS, OXFORD, GB, vol. 7, no. 17, 9 September 1997 (1997-09-09), pages 2223-2228, XP004136417 ISSN: 0960-894X

## Description

The present invention relates to Endothelin receptor antagonist derivatives. More particularly, the present invention relates to Endothelin receptor antagonist nitroderivatives, pharmaceutical compositions containing them and their use for the treatment of endothelial-related disorders, renal, pulmonary, cardiac and vascular diseases, and inflammatory processes.

Endothelins are a family of closely related 21-amino acid peptides (ET-1, ET-2, and ET-3) with ET-1 being one of the most potent vasoconstrictors identified to date. These peptides cause numerous biological effects in addition to vasoconstriction. The endothelins have been implicated in a variety of disease states including hypertension, congestive heart failure, renal failure, pulmonary hypertension, and metastatic prostate cancer. The endothelins exert their physiological activities via two specific G-protein coupled receptors termed ET_{A} and ET_{B}. The ET_{A} receptor is selective for ET-1 and is expressed predominately in vascular smooth muscle cells where it mediates vasoconstrictive and proliferative responses. The ET_{B} receptor is nonselective and binds all three ET isopeptides with equal affinity. The ET_{B} receptors are mostly found on endothelial cells and mediate ET-1-induced vasodilation possibly through the release of nitric oxide. A small population of ET_{B} receptors is also found on some smooth muscle cells where their activation leads to vasoconstriction (J. Med. Chem. 2000, 43, 3111).

With the Endothelin receptor antagonist derivatives a class of compounds is intended, comprising as main components Ambrisentan, Atrasentan, Avosentan, Bosentan, Clazosentan, Darusentan, Tezosentan, Sitaxsentan etc.

US 6,635,273 discloses methods for treating vascular diseases characterized by nitric oxide insufficiency by administering to a patient a therapeutically effective amount of at least one antioxidant, or a pharmaceutically acceptable salt thereof, and at least one of isosorbide dinitrate and isosorbide mononitrate, and, optionally, at least one nitrosated angiotensin-converting enzyme inhibitor, nitrosated beta-adrenergic blocker, nitrosated calcium channel blocker, nitrosated endothelin antagonist, nitrosated angiotensin II receptor antagonist, nitrosated renin inhibitor, and/or at least one compound used to treat cardiovascular diseases.

WO 2005/023182 describes novel nitrosated and/or nitrosylated cardiovascular compounds or pharmaceutically acceptable salts thereof, and novel compositions comprising at least one nitrosated and/or nitrosylated cardiovascular compound, and, optionally, at least one nitric oxide donor and/or at least one therapeutic agent. The nitrosated and/or nitrosylated cardiovascular compounds are selected from: aldosterone antagonists, angiotensin II antagonists, calcium channel blockers, nitrosated and/or nitrosylated endothelin antagonists, hydralazine compounds, neutral endopeptidase inhibitors and renin inhibitors.

CA 2391818 discloses methods for treating physiological conditions in which NO production is at least partially inhibited, such as erectile dysfunction, by administering to a patient an effective amount of endothelin antagonists.

Neidhart W et Al., Bioorganic & Medicinal Chemistry Letters Vol. 7, No. 17, pp. 2223-2228 describes the identification of Ro 48-5695 as potent orally active endothelin receptor antagonist.

WO 00/42035 discloses new inhibitors of endothelin receptors and their use for the treatment of disorders which are associated with abnormal vascular tone and endothelial dysfunction.

It was now object of the present invention to provide new derivatives of Endothelin receptor antagonists having an improved pharmacological activity compared with their parent compounds.

In particular, it has been recognized that the Endothelin receptor antagonist nitroderivatives of the present invention can be employed for treating or preventing congestive heart failure, coronary diseases, left ventricular dysfunction and hypertrophy, cardiac fibrosis, myocardial ischemia, stroke, subarachnoid hemorrhage, cerebrovascular vasospasm, coronary vasospasm, atherosclerosis, restenosis post angioplasty, renal ischemia, renal failure, renal and pulmonary fibrosis, glomerulonephritis, renal colic, ocular hypertension, glaucoma, systemic hypertension, pulmonary arterial hypertension (PAH), diabetic complications such as nephropathy, vasculopathy and neuropathy, peripheral vascular diseases, liver fibrosis, portal hypertension, metabolic syndromes, erectile dysfunction, complications after vascular or cardiac surgery, complications of treatment with immunosuppressive agents after organ transplantation, hyperaldosteronism, lung fibrosis, scleroderma, sickle cell disease, benign prostatic hyperplasia, cancer, anxiety, cognitive disorders.

Object of the present invention are, therefore, Endothelin receptor antagonist nitroderivatives of general formula (I) and pharmaceutically acceptable salts or stereoisomers thereof: 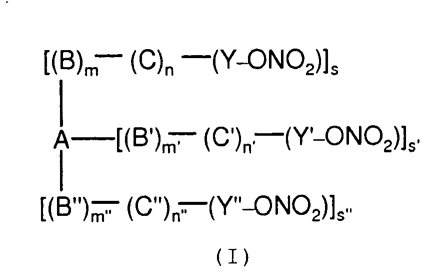 wherein:
m, m' and m" are equal to 0 or 1;
n, n' and n" are equal to 0 or 1;
s, s' and s" are equal to 0 or 1;
A is selected from the group consisting of: 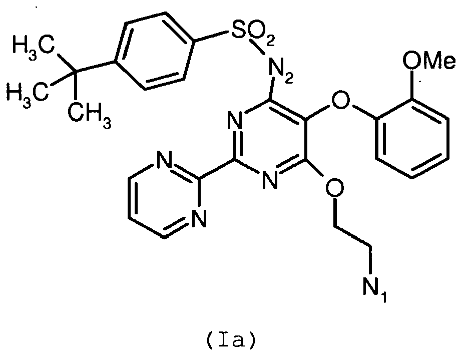 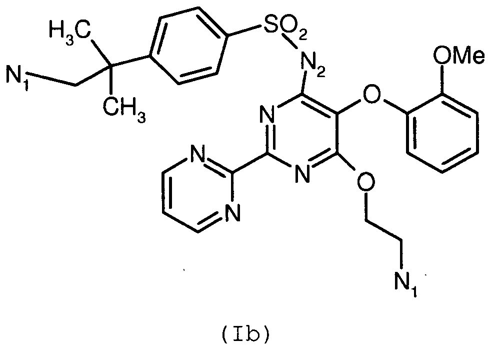 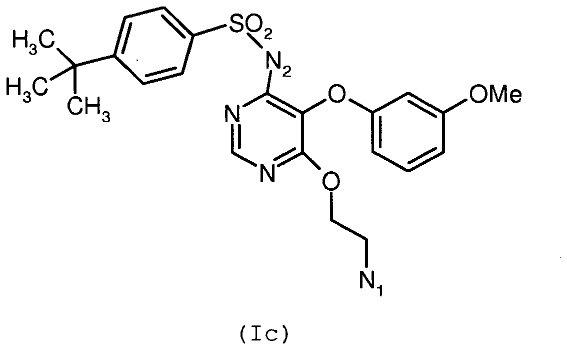 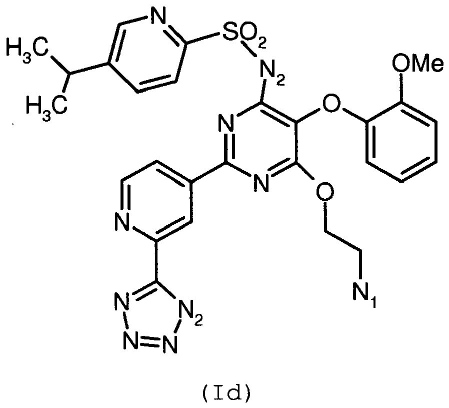 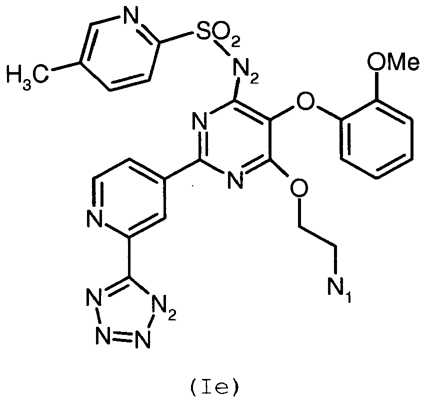 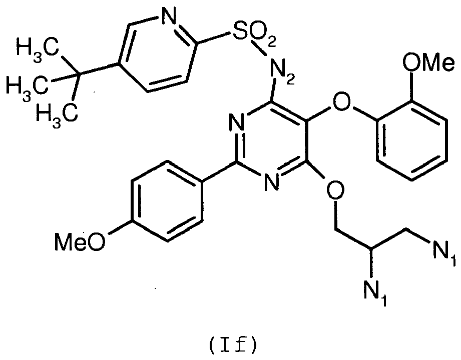 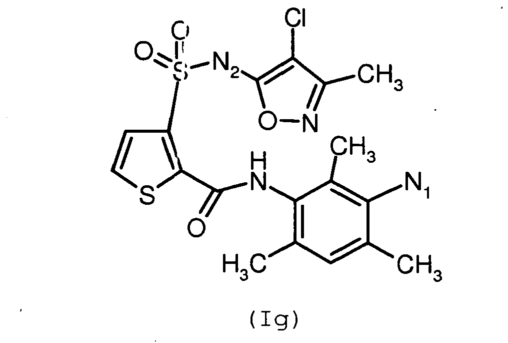 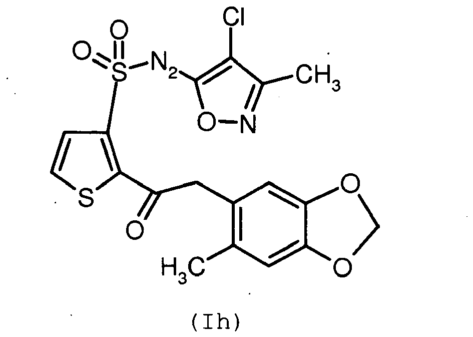 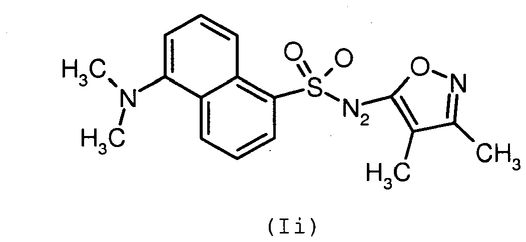 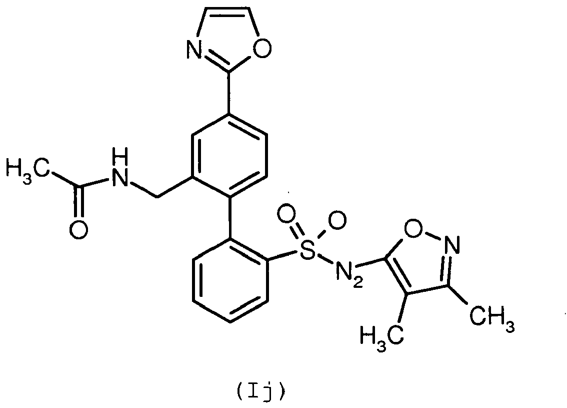 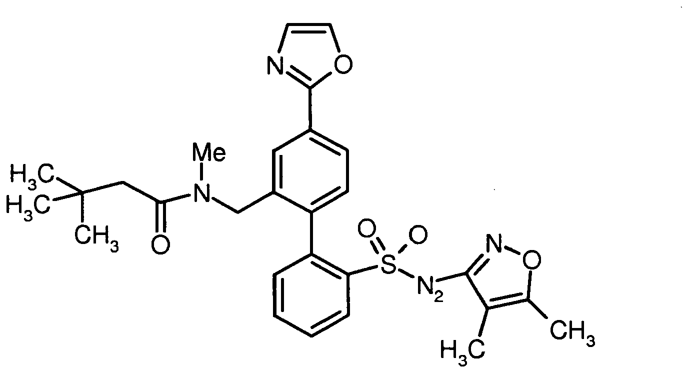 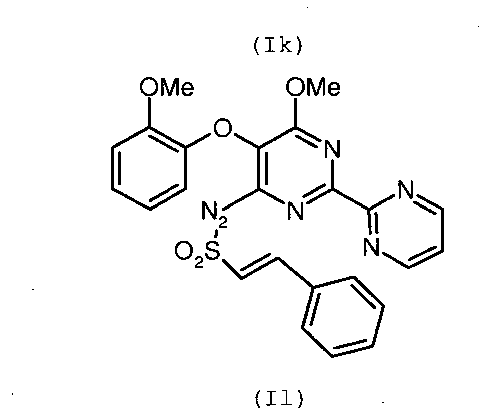 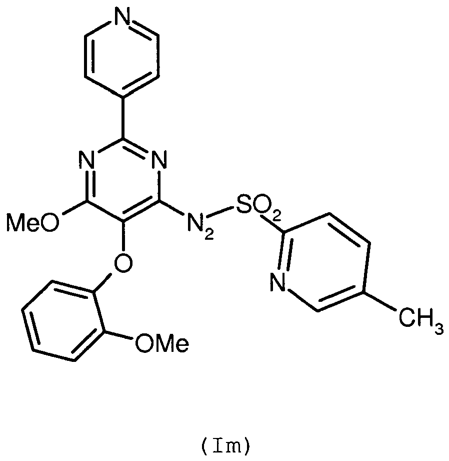 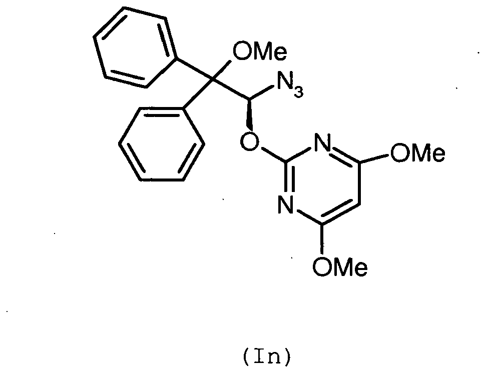 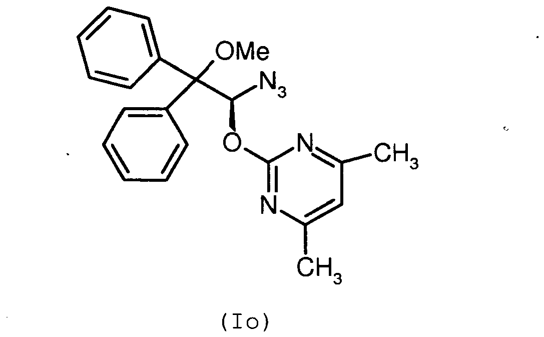 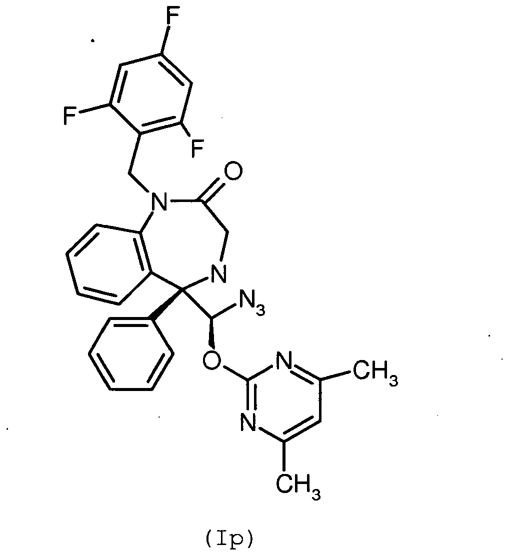 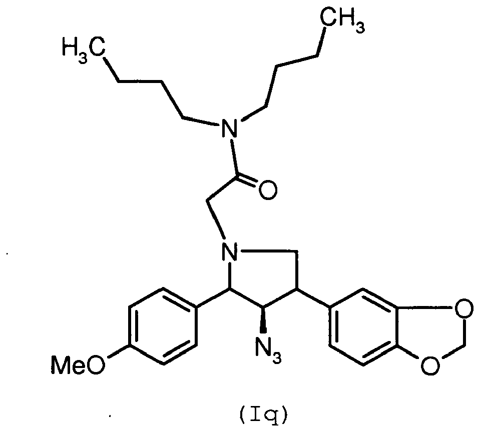 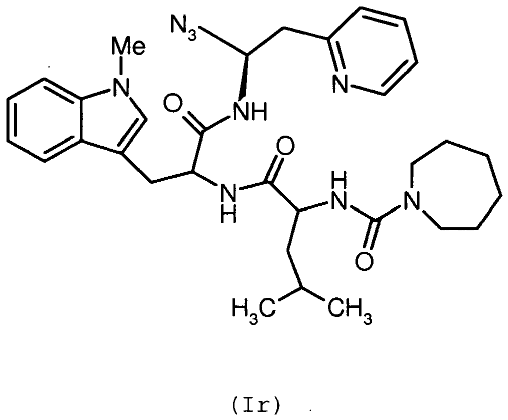 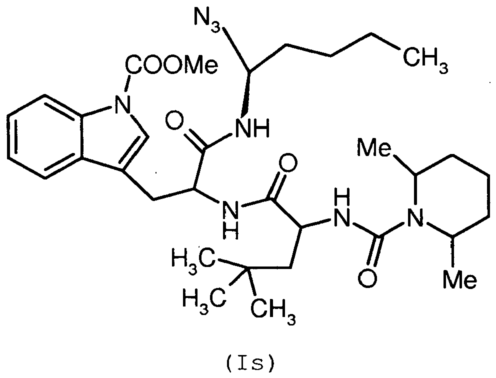 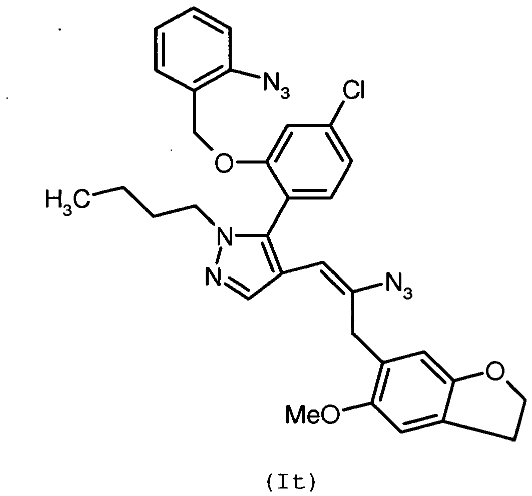 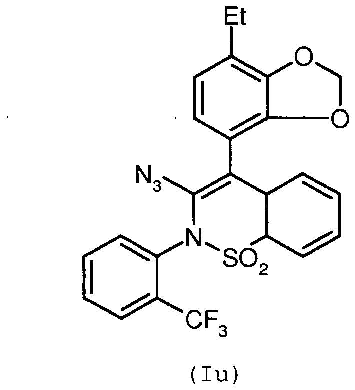 wherein:
   N₁ is -O-, -OH;
   N₂ is -N-, -NH-;
   N₃ is -C(O)O-, -C (O) NH-;
In general formula (I):
B, B' and B" are -CO-, -C(O)O-;
C, C' and C' ' are: 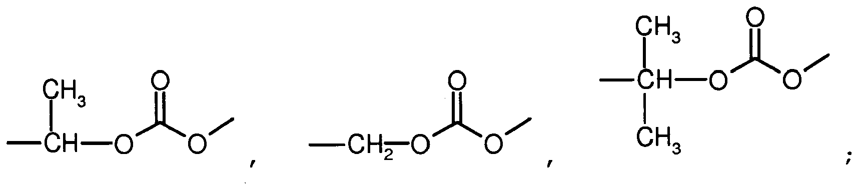 with the proviso that:
   1) when A is selected from the group consisting of: (Ia), (Ib), (Ic), (Id), (Ie), (If), (Ig), (Ih), (Ii), (Ij), (Ik), (Il) and (Im), at least one of m, m', m" , n, n' or n" is 1;
   2) when A is selected from the group consisting of: (In), (Io), (Ip), (Iq), (Ir), (Is) and (Iu), then s', s" and m are 0; while n is 0 or 1;
   3) when A is (It), then m, m' and s" are 0; while n and n' are 0 or 1;
   4) at least one of N₁ or N₂ is a group -O- or -N- able to bind at least one of the groups -[(B)ₘ-(C)ₙ-(Y-ONO₂)], - [(B')_{m'}-(C')_{n'}-(Y'-ONO₂)] or - [(B")_{m"}-(C")ₙ"-(Y"-ONO₂)].
Y, Y' and Y" are a bivalent radical having the following meaning:
   a)
      - straight or branched C₁-C₂₀ alkylene, preferably having from 1 to 10 carbon atoms, being optionally substituted with one or more of the substituents selected from the group consisting of: halogen atoms, hydroxy, -ONO₂ or Tₐ, wherein Tₐ is
      - OC(O)(C₁-C₁₀alkyl)-ONO₂ or -O(C₁-C₁₀alkyl)-ONO₂;
      - cycloalkylene with 5 to 7 carbon atoms into cycloalkylene ring, the ring being optionally substituted with side chains T, wherein T is straight or branched alkyl with from 1 to 10 carbon atoms, preferably CH₃;
   b) 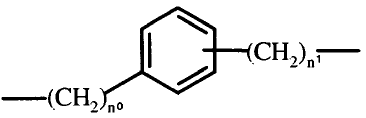
   c) 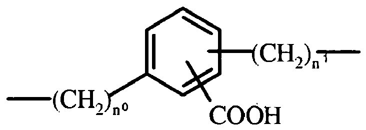 wherein n⁰ is an integer from 0 to 20, and n¹ is an integer from 1 to 20;
   d) 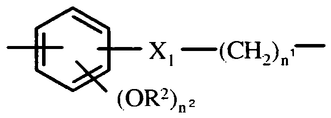 wherein:
      n¹ is as defined above and n² is an integer from 0 to 2;
      X₁ = -OCO- or -COO- and R² is H or CH₃;
   e) 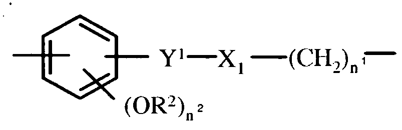 wherein:
      n¹, n²,R² and X₁ are as defined above;
      Y¹ is -CH₂-CH₂- or -CH=CH- (CH₂)ₙ²-;
   f) 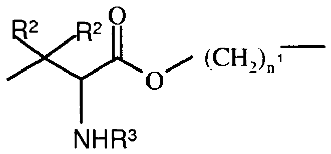 wherein:
      n¹ and R² are as defined above, R³ is H or -COCH₃;
      with the proviso that when Y is selected from the bivalent radicals mentioned under b)-f), the -ONO₂ group is linked to a -CH₂ group;
   g) 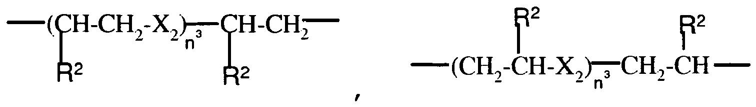 wherein X₂ is -O- or -S-, n³ is an integer from 1 to 6, preferably from 1 to 4, R² is as defined above;
   h) 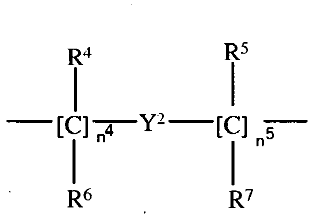 wherein:
      n⁹ is an integer from 0 to 10;
      n⁵ is an integer from 1 to 10;
      R⁴, R⁵, R⁶, R⁷ are the same or different, and are H or
      straight or branched C₁-C₄ alkyl, preferably R⁴, R⁵, R⁶, R⁶ R⁷ are H;
      wherein the -ONO₂ group is linked to 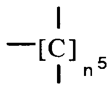 wherein n⁵ is as defined above;
      Y² is an heterocyclic saturated, unsaturated or aromatic 5 or 6 members ring, containing one or more heteroatoms selected from nitrogen, oxygen, sulfur,
      and is selected from the group consisting of:

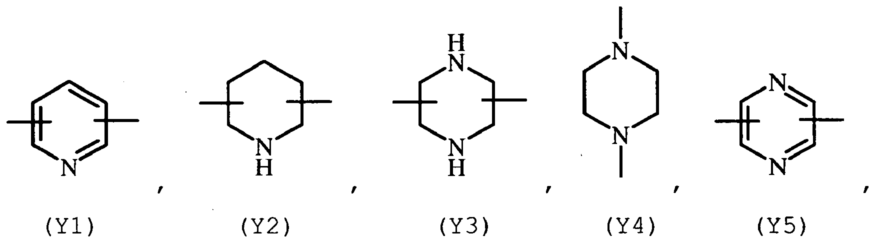

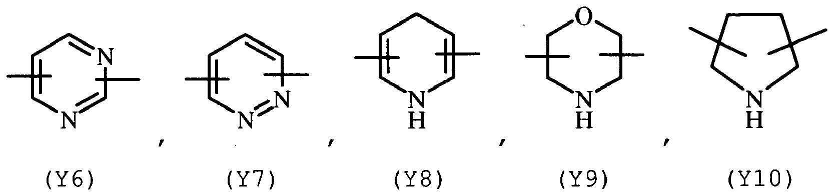

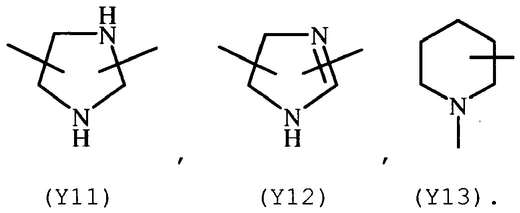

The term "C₁-C₂₀ alkylene" as used herein refers to branched or straight chain C₁-C₂₀ hydrocarbon, preferably having from 1 to 10 carbon atoms such as methylene, ethylene, propylene, isopropylene, n-butylene, pentylene, n-hexylene and the like.

The term "C₁-C₁₀ alkyl" as used herein refers to branched or straight chain alkyl groups comprising one to ten carbon atoms, including methyl, ethyl, n-propyl, isopropyl, n-butyl, isobutyl, t-butyl, pentyl, hexyl, octyl and the like.

The term "cycloalkylene" as used herein refers to ring having from 5 to 7 carbon atoms including, but not limited to, cyclopentylene, cyclohexylene optionally substituted with side chains such as straight or branched (C₁-C₁₀)-alkyl, preferably CH₃.

The term "heterocyclic" as used herein refers to saturated, unsaturated or aromatic 5 or 6 members ring, containing one or more heteroatoms selected from nitrogen, oxygen, sulphur, such as for example pyridine, pyrazine, pyrimidine, pyrrolidine, morpholine, imidazole and the like.

Another aspect of the present invention provides the use of the compounds of formula (I) in combination with at least a compound used to treat cardiovascular disease selected from the group consisting of: aldosterone antagonists, angiotensin II receptor blockers, ACE inhibitors, HMGCoA reductase inhibitors, beta-adrenergic blockers, alpha-adrenergic antagonists, sympatholytics, calcium channel blockers, renin inhibitors, neutral endopeptidase inhibitors, potassium activators, diuretics, vasodilators, antithrombotics such as aspirin. Also is contemplated the combination with nitrosated compounds of the above reported compounds.

Suitable aldosterone antagonists, angiotensin II receptor blockers, ACE inhibitors, HMGCoA reductase inhibitors, beta-adrenergic blockers, alpha-adrenergic antagonists, calcium channel blockers, renin inhibitors, potassium activators, diuretics, vasodilators and antithrombotics are described in the literature such as The Merck Index (13th edition).
Suitable nitrosated compounds are disclosed in WO 98/21193, WO 97/16405, WO 98/09948, WO 2004/105754, WO 2004/106300, WO 2004/110432, WO 2005/011646, WO 2005/053685, WO 2005/054218.

The administration of the compounds above reported can be carried out simultaneously or successively.

The present invention also provides pharmaceutical kits comprising one or more containers filled with one or more of the compounds and/or compositions of the present invention and one or more of the compounds used to treat cardiovascular diseases reported above.

As stated above, the invention includes also the pharmaceutically acceptable salts of the compounds of formula (I) and stereoisomers thereof.

Examples of pharmaceutically acceptable salts are either those with inorganic bases, such as sodium, potassium, calcium and aluminium hydroxides, or with organic bases, such as lysine, arginine, triethylamine, dibenzylamine, piperidine and other acceptable organic amines.

The compounds according to the present invention, when they contain in the molecule one salifiable nitrogen atom, can be transformed into the corresponding salts by reaction in an organic solvent such as acetonitrile, tetrahydrofuran with the corresponding organic or inorganic acids.

Examples of organic acids are: oxalic, tartaric, maleic, succinic, citric acids. Examples of inorganic acids are: nitric, hydrochloric, sulphuric, phosphoric acids. Salts with nitric acid are preferred.

The compounds of the invention which have one or more asymmetric carbon atoms can exist as optically pure enantiomers, pure diastereomers, enantiomers mixtures, diastereomers mixtures, enantiomer racemic mixtures, racemates or racemate mixtures. Within the object of the invention are also all the possible isomers, stereoisomers and their mixtures of the compounds of formula (I).

Preferred compounds are those of formula (I) wherein Y, Y' and Y" have the following meaning:
a)
   - straight or branched C₁-C₁₀ alkylene, being optionally substituted with one -ONO₂ group;
b) 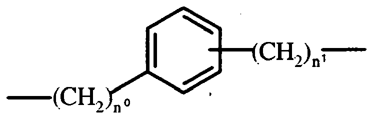 wherein n⁰ is an integer equal to 0 or 1, and n¹ is an integer equal to 1; with the proviso the -ONO₂ group is linked to a -CH₂ group;
g) 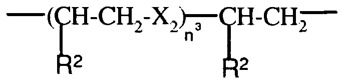 wherein X₂ is -O- or -S-, n³ is an integer equal to 1 and R² is H.

The following are preferred compounds according to the present invention:

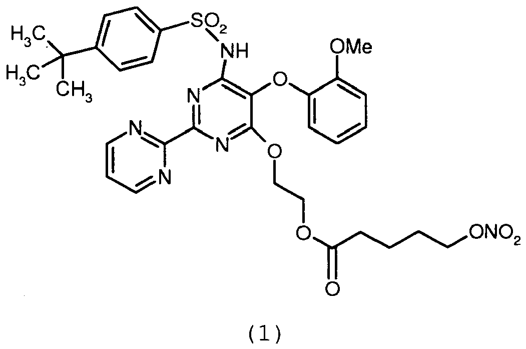

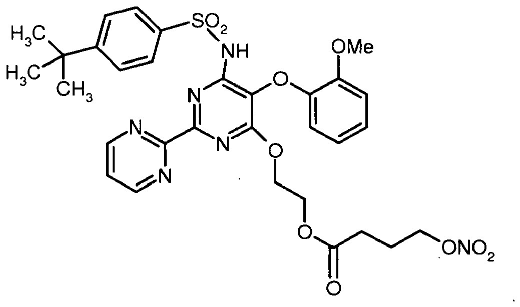

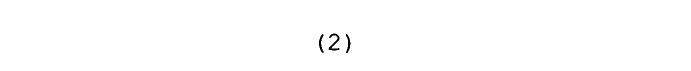

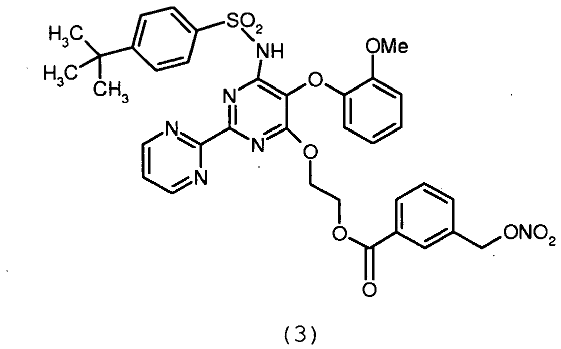

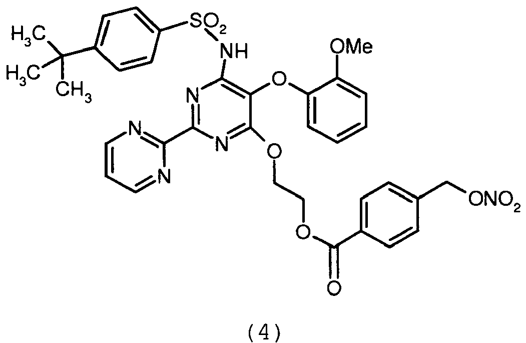

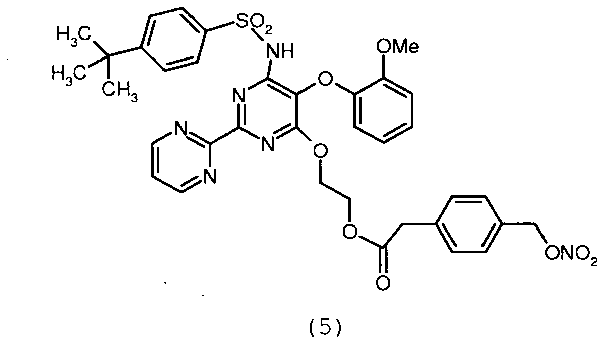

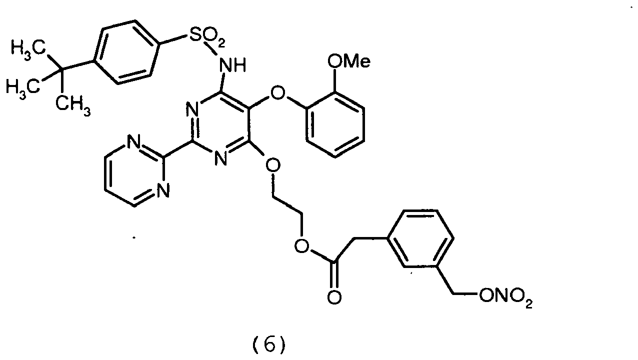

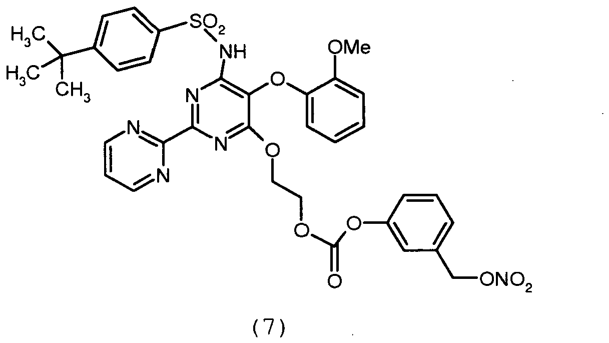

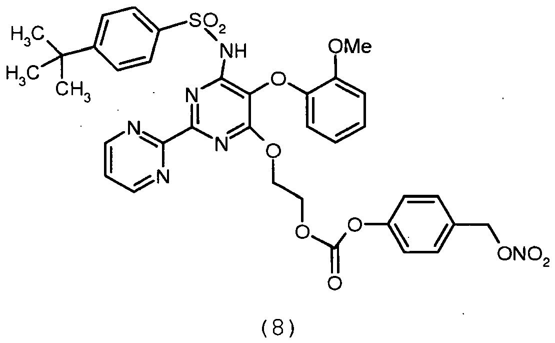

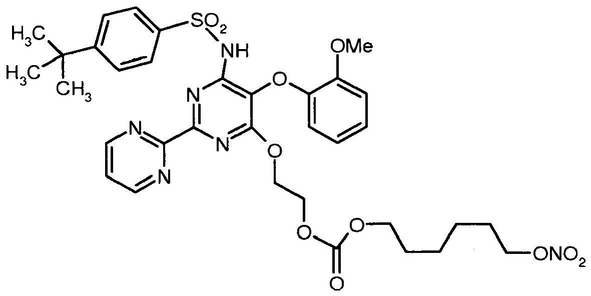

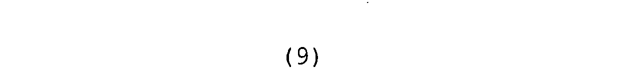

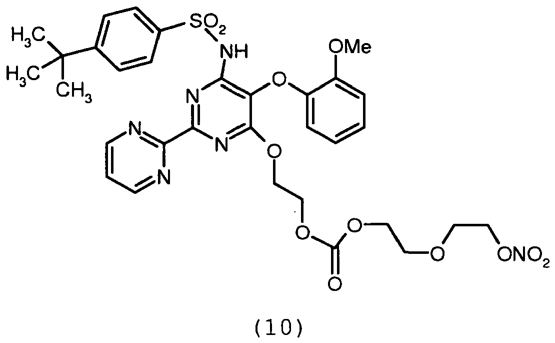

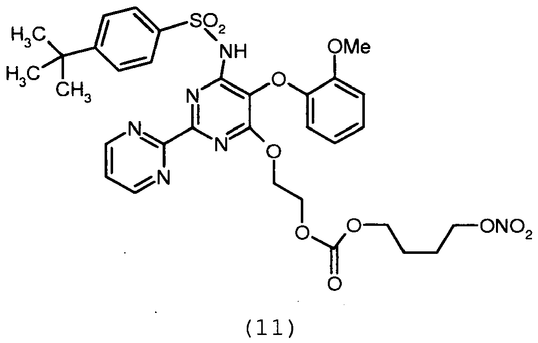

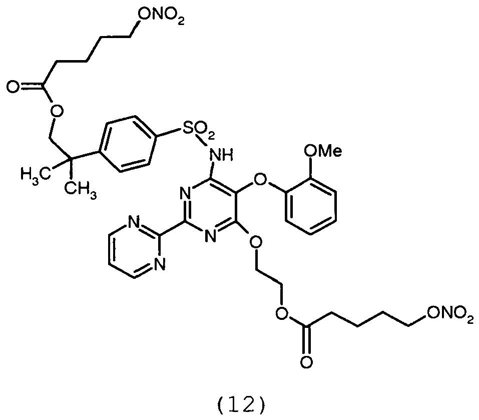

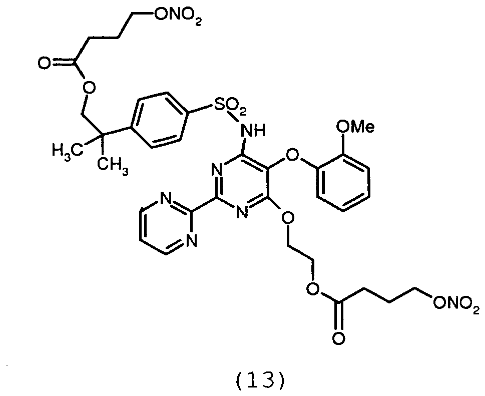

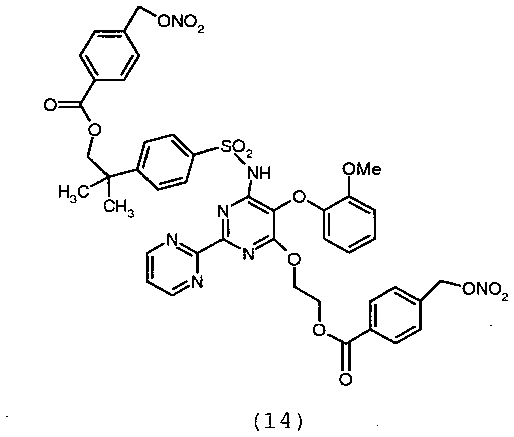

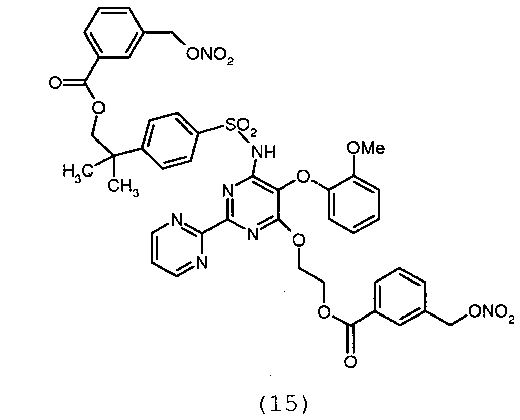

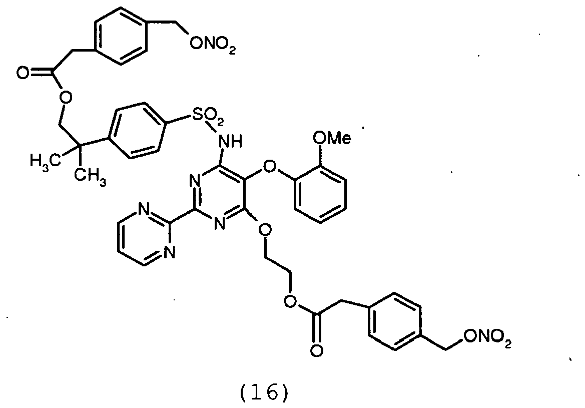

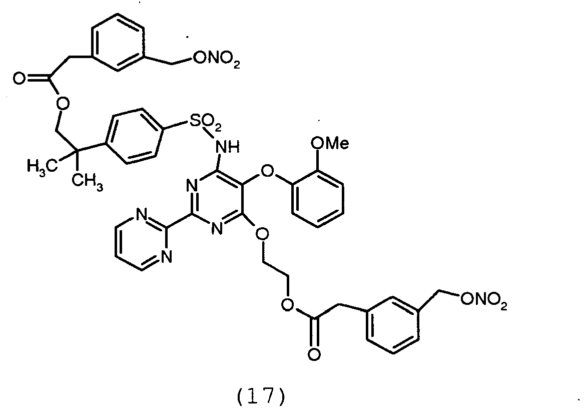

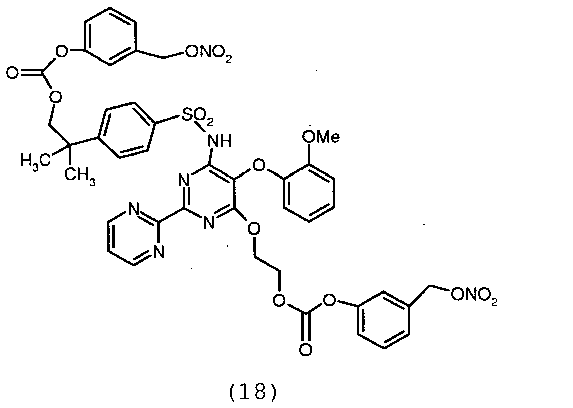

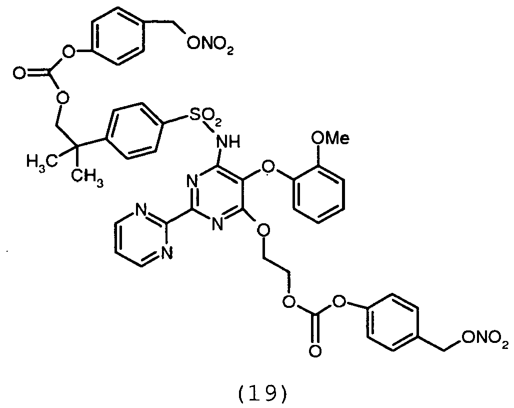

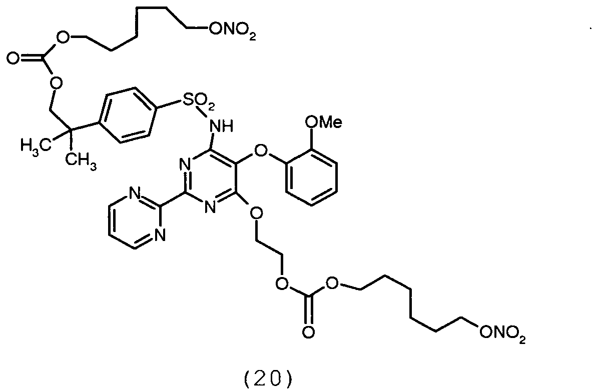

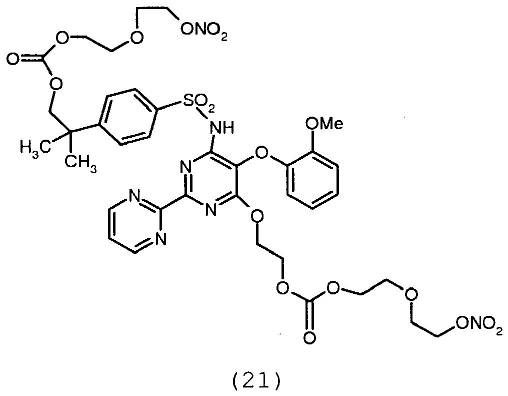

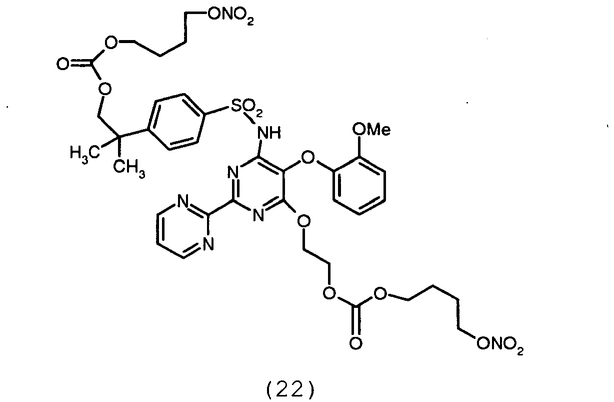

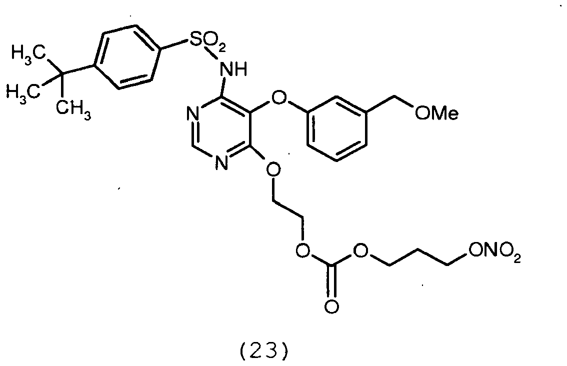

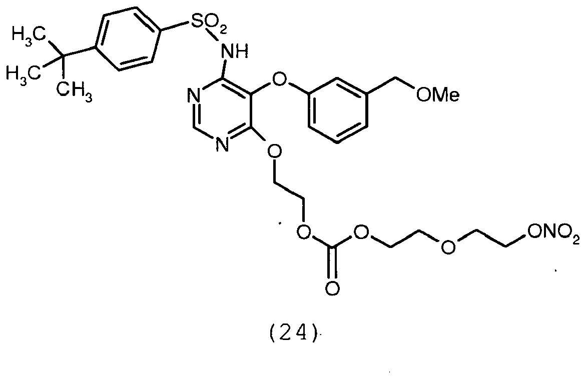

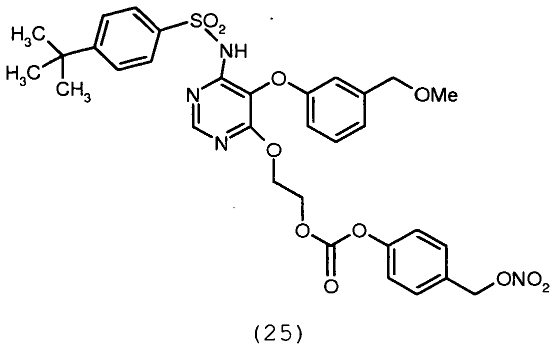

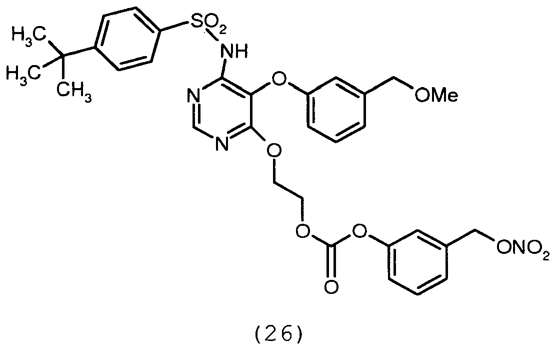

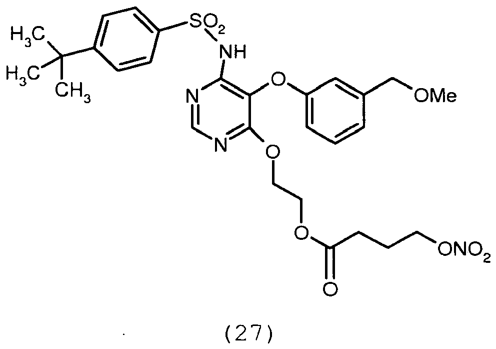

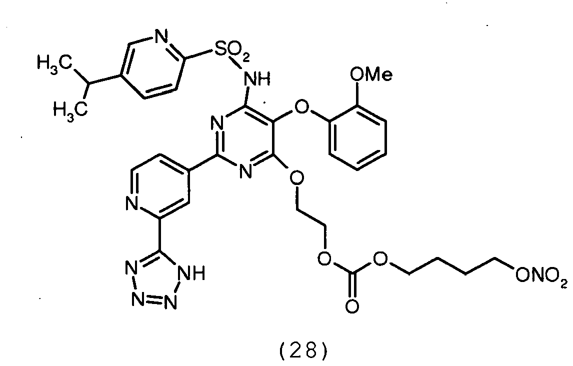

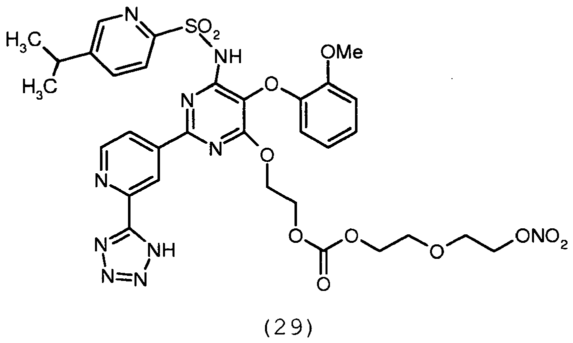

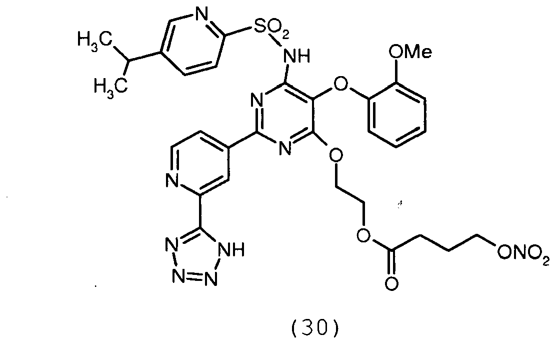

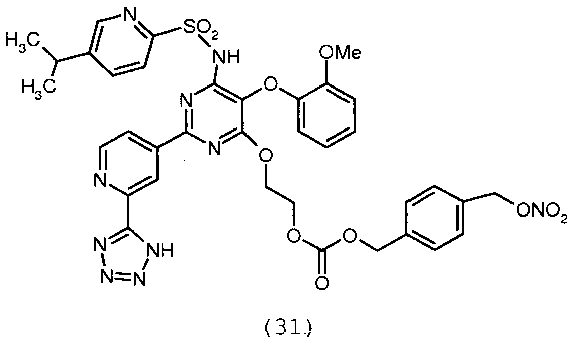

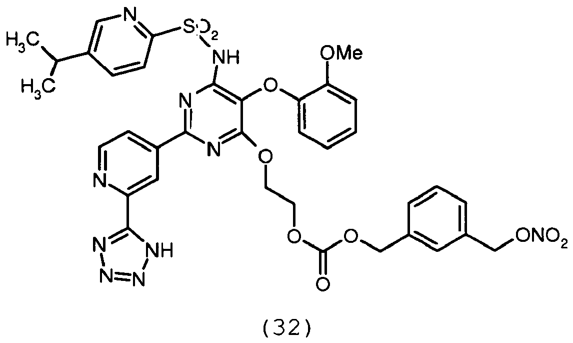

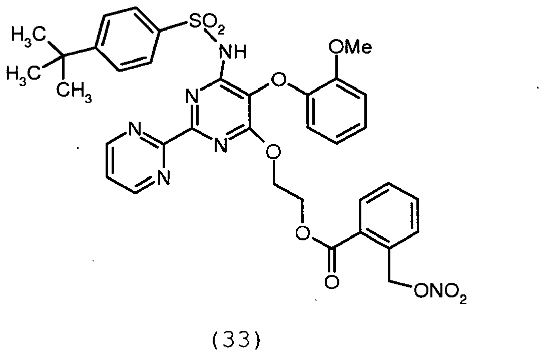

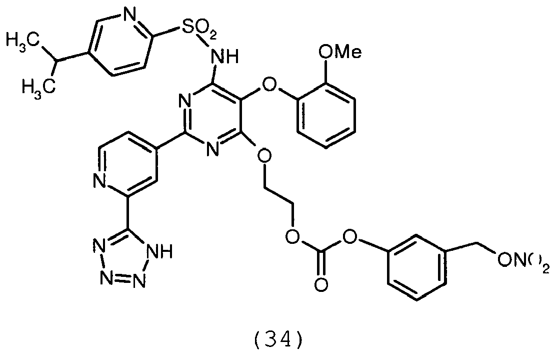

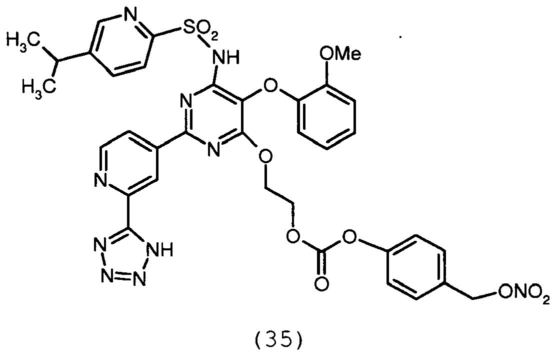

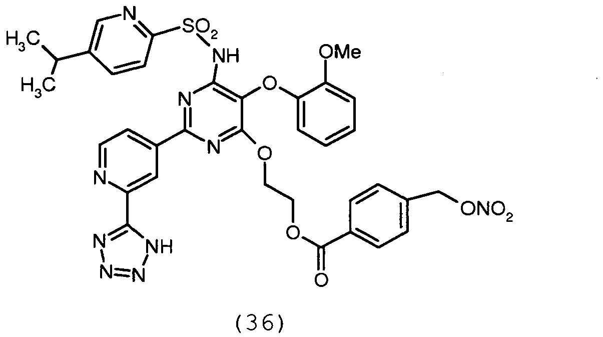

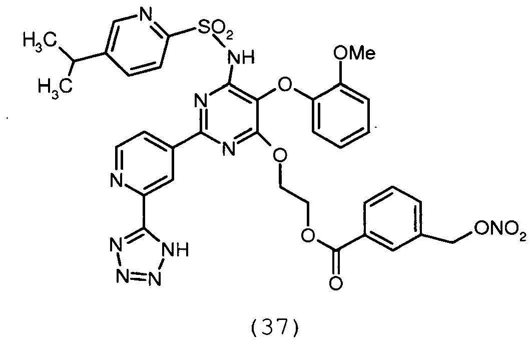

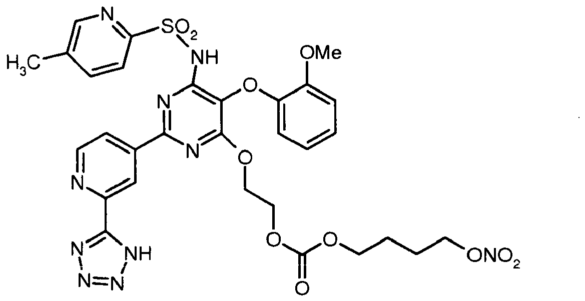

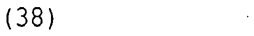

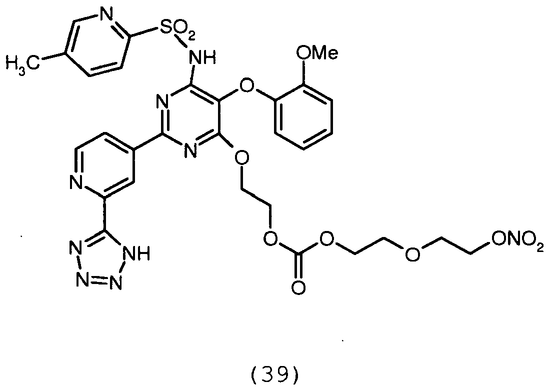

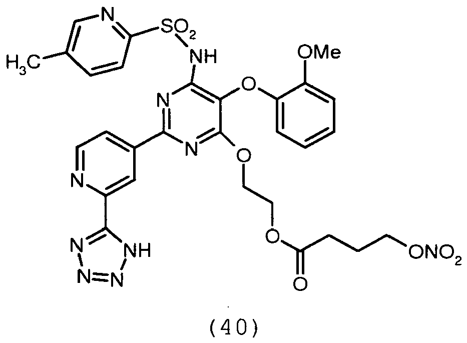

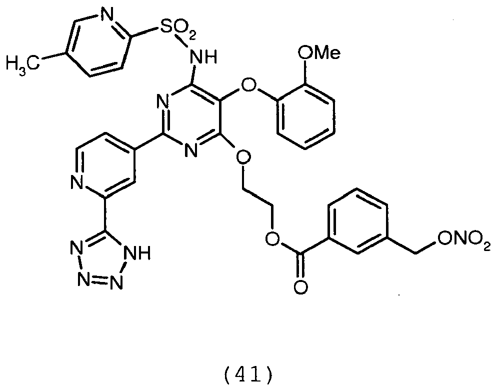

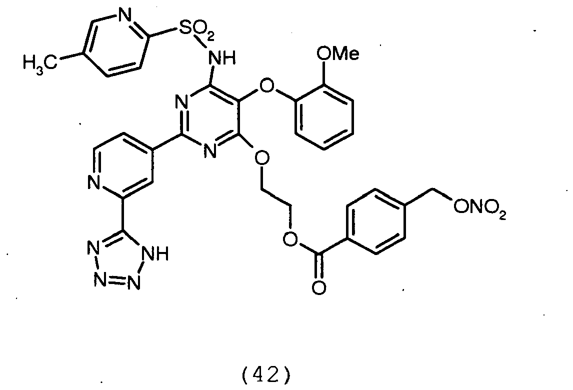

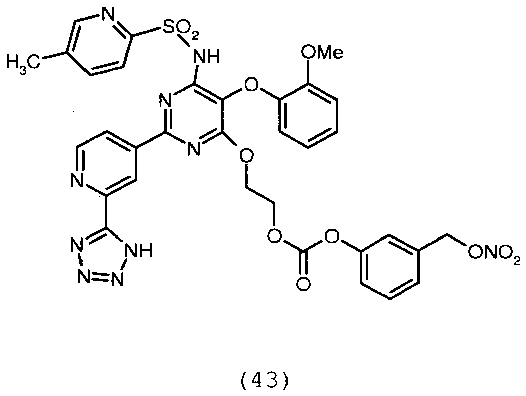

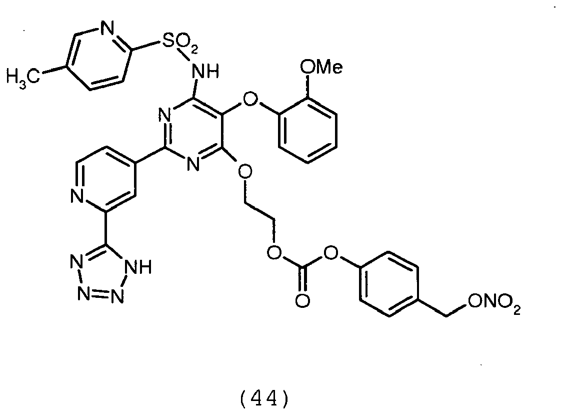

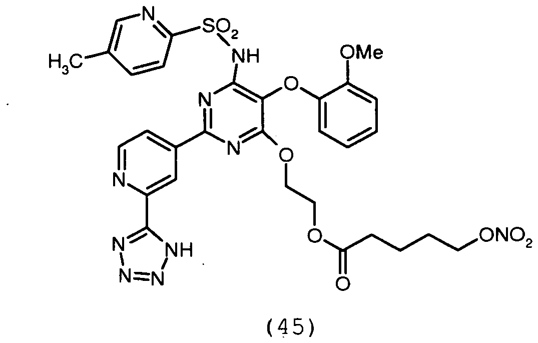

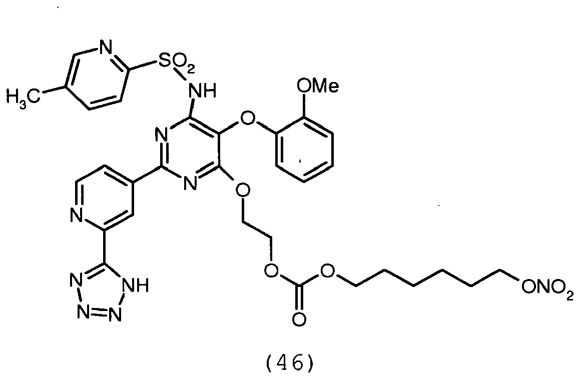

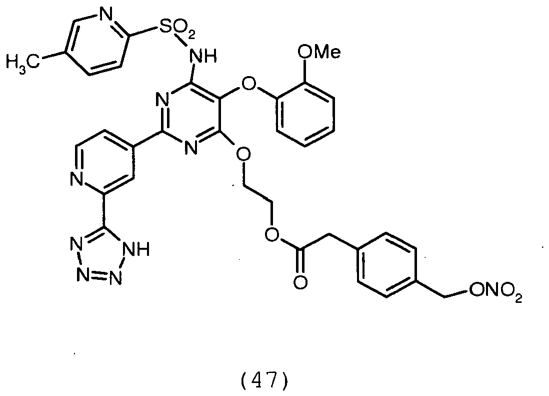

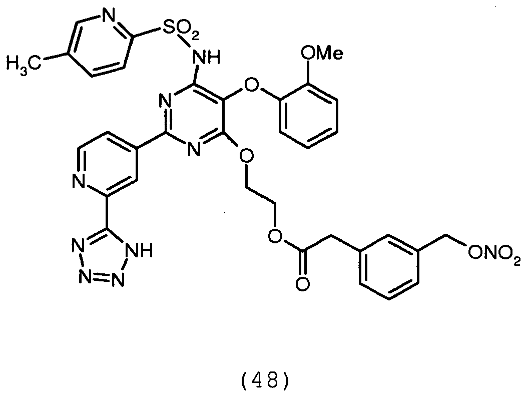

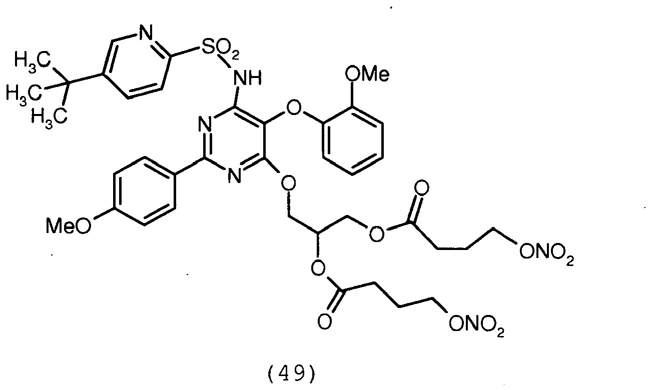

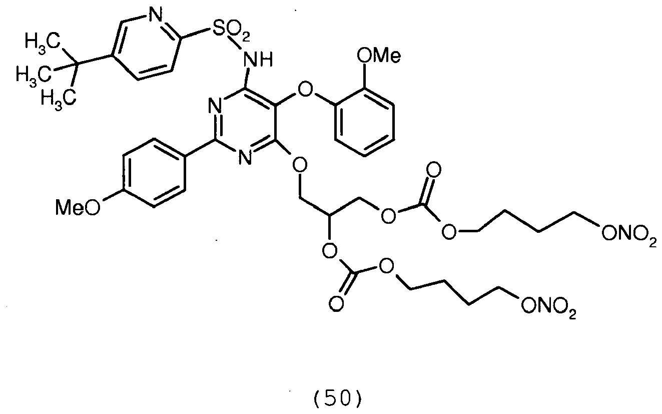

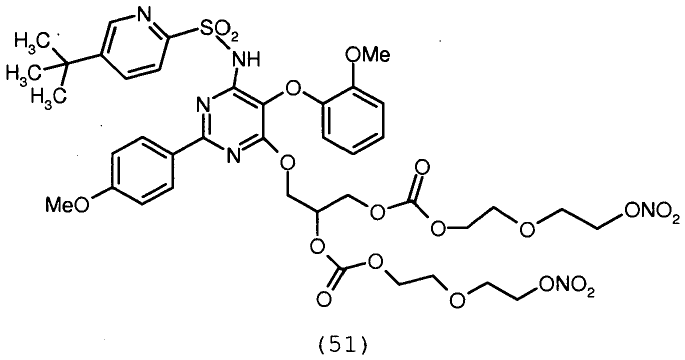

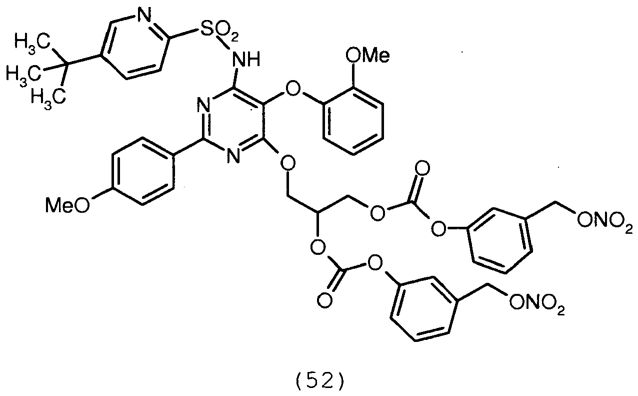

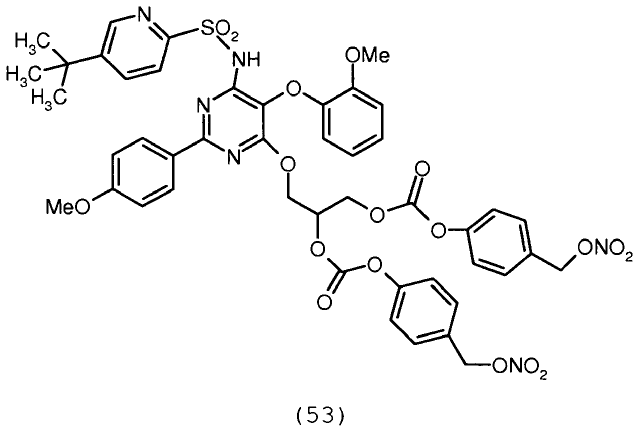

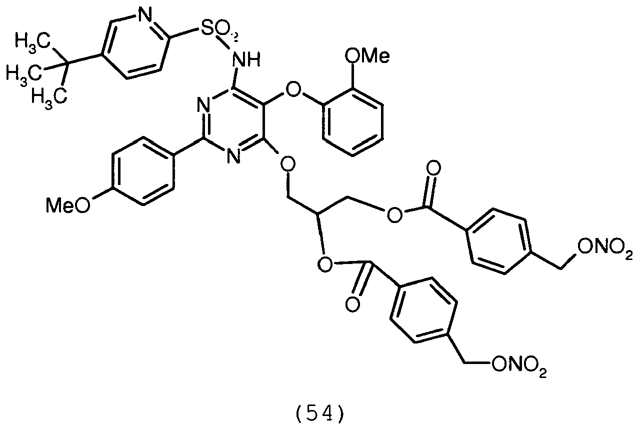

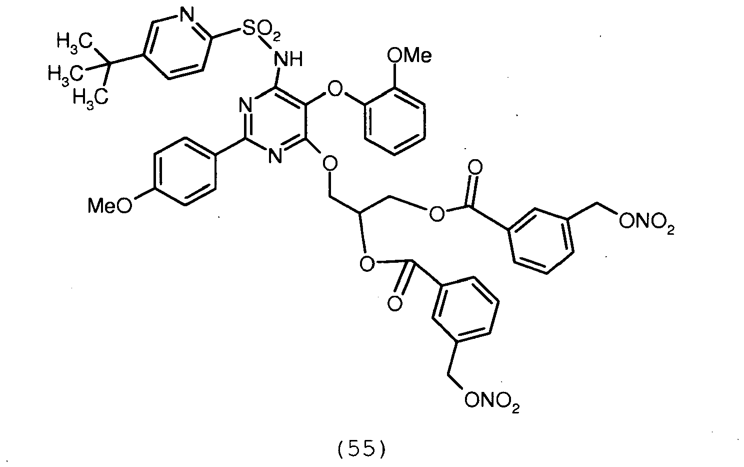

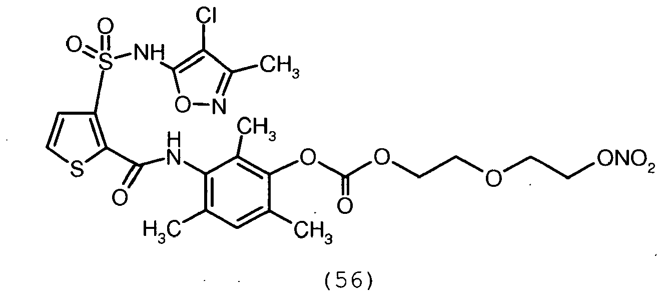

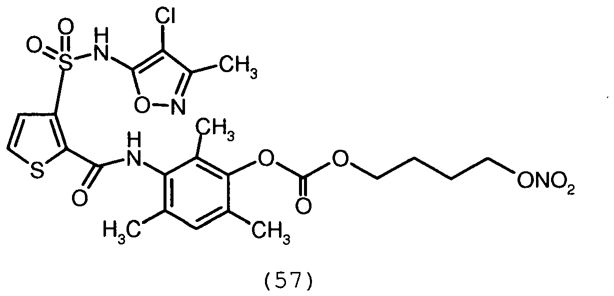

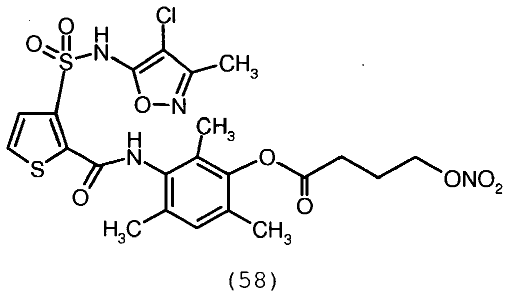

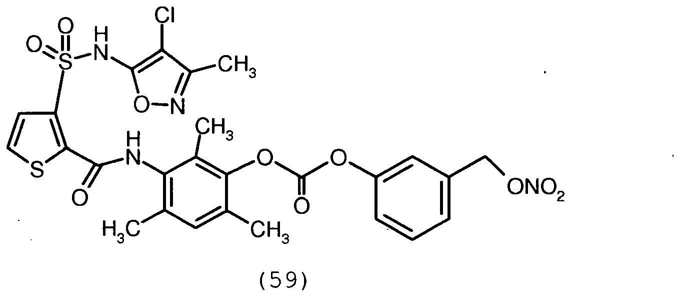

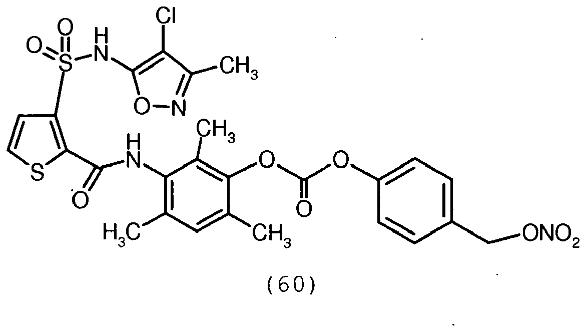

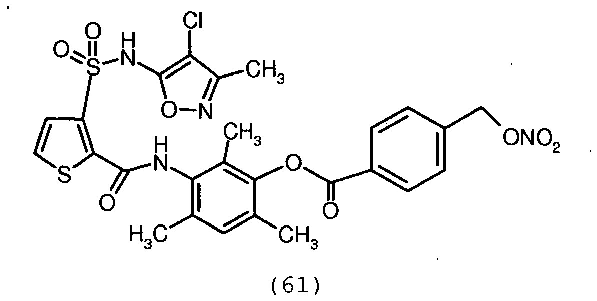

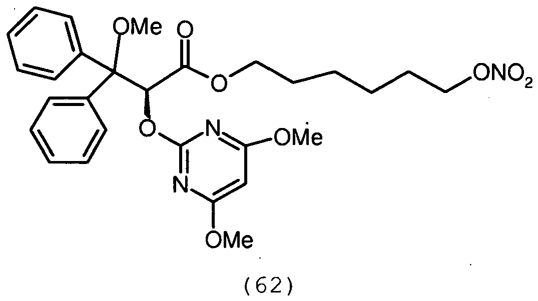

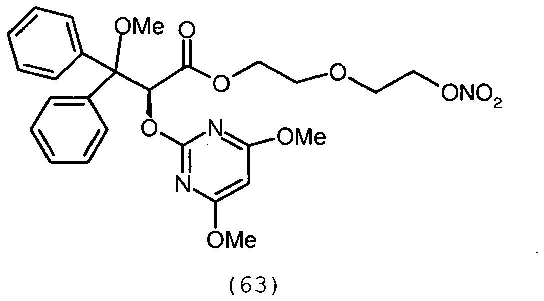

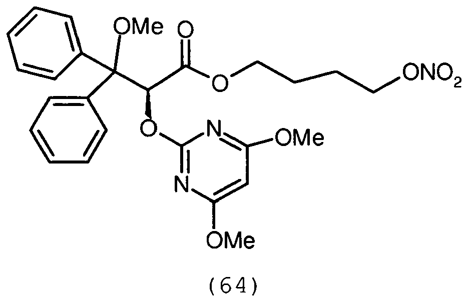

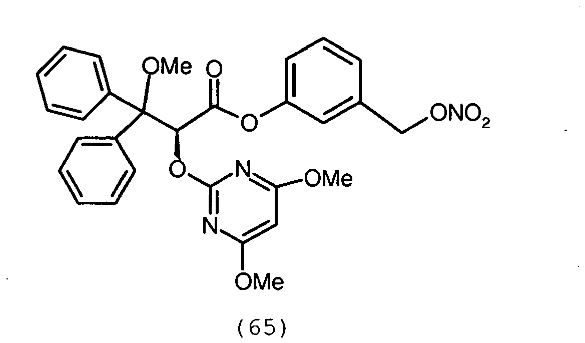

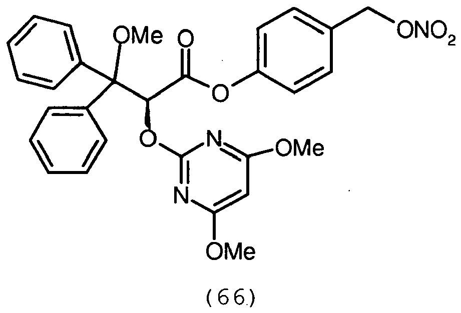

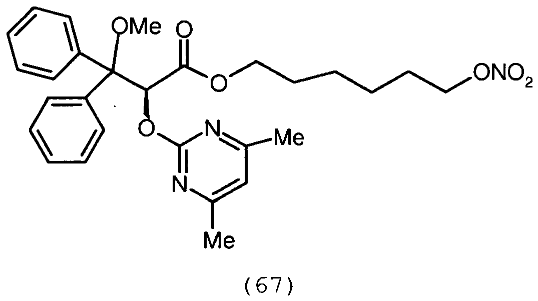

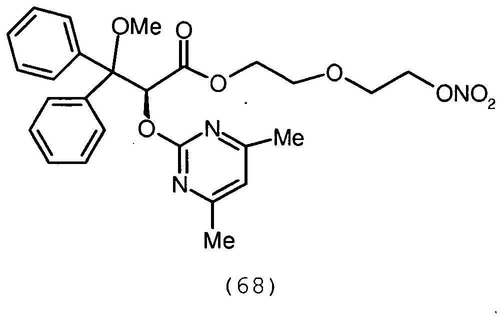

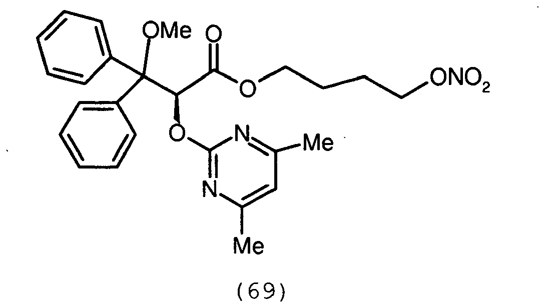

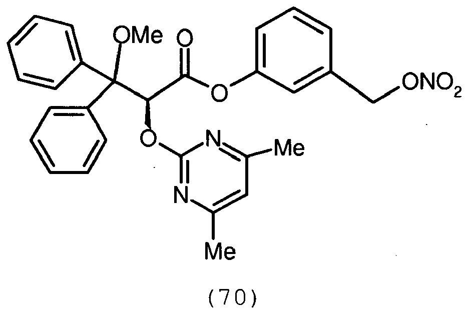

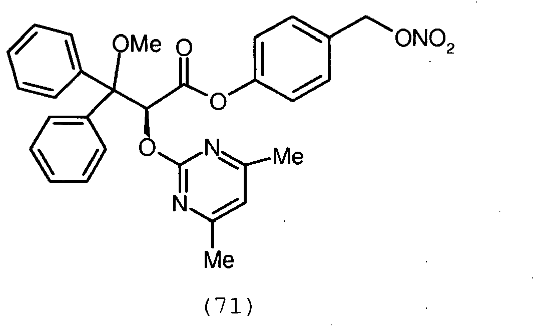

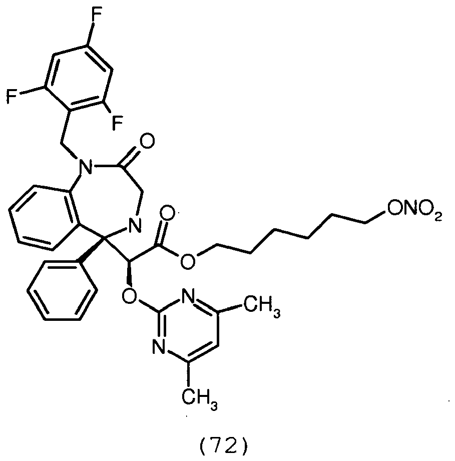

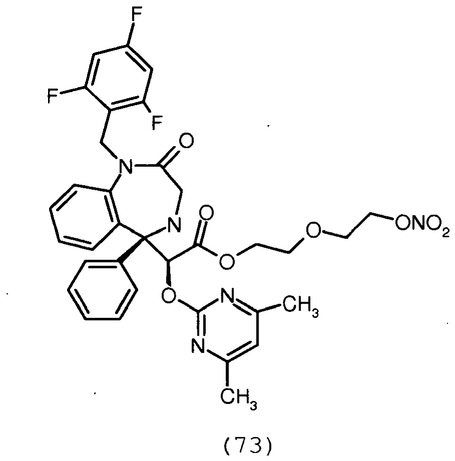

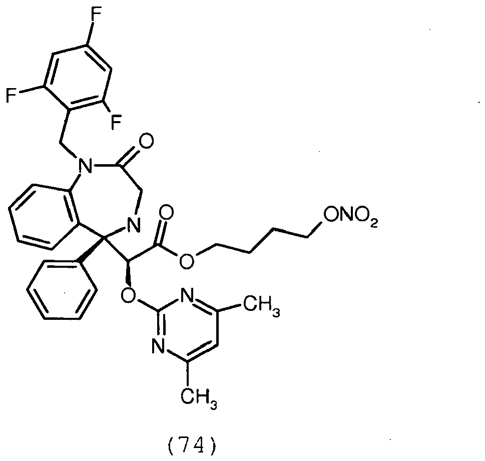

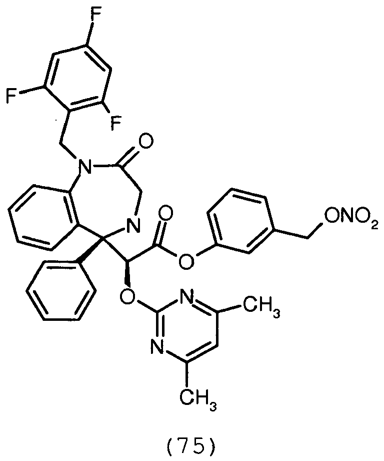

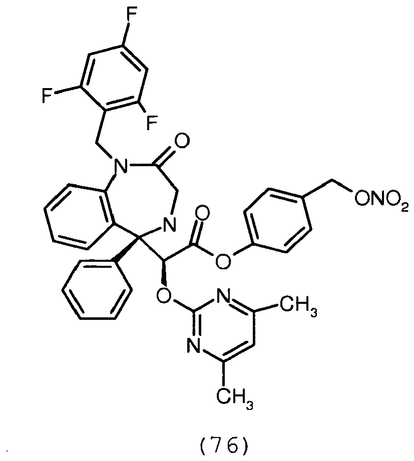

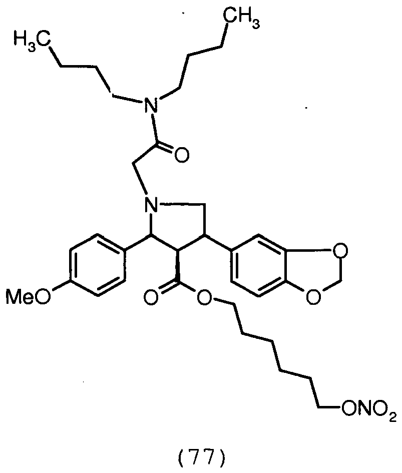

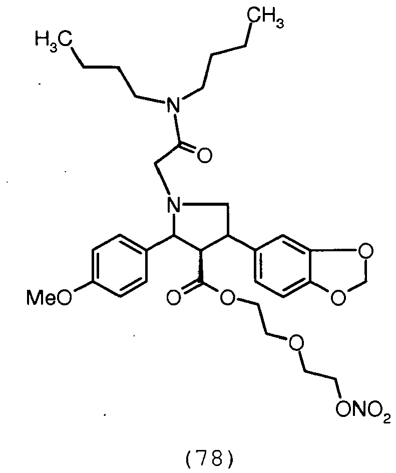

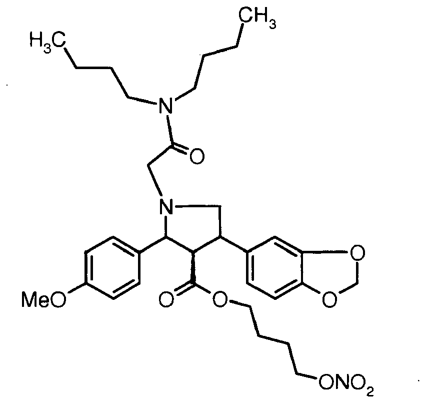

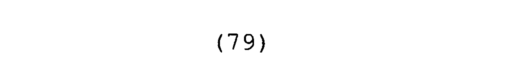

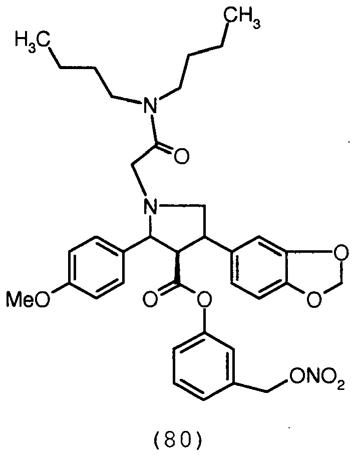

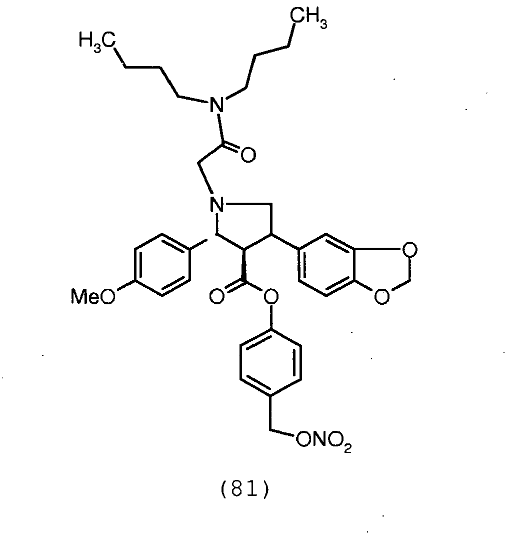

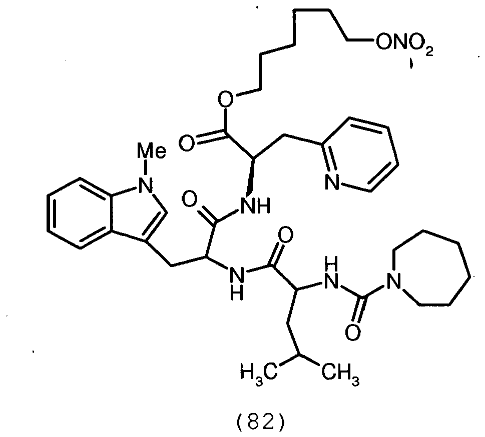

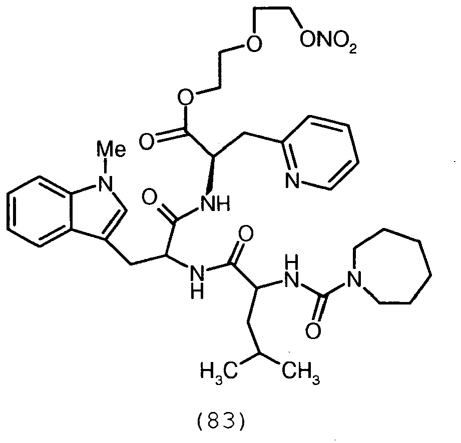

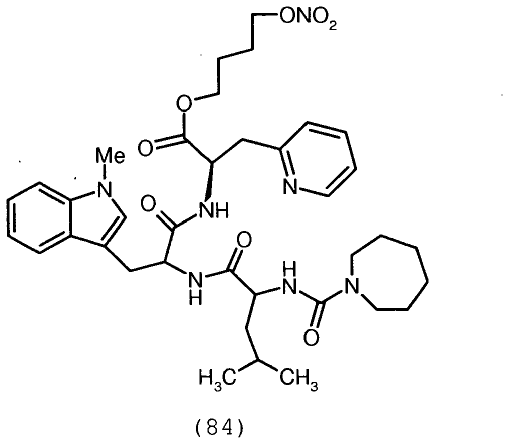

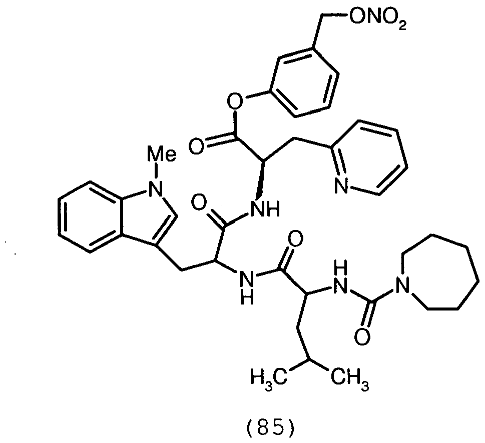

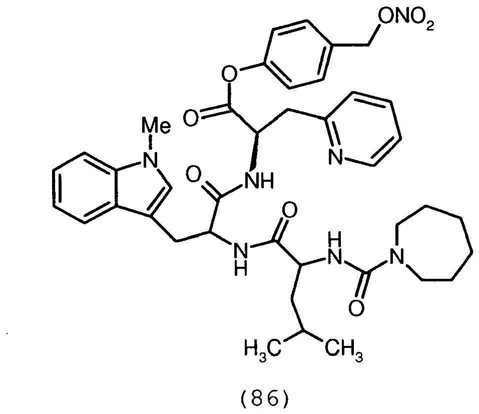

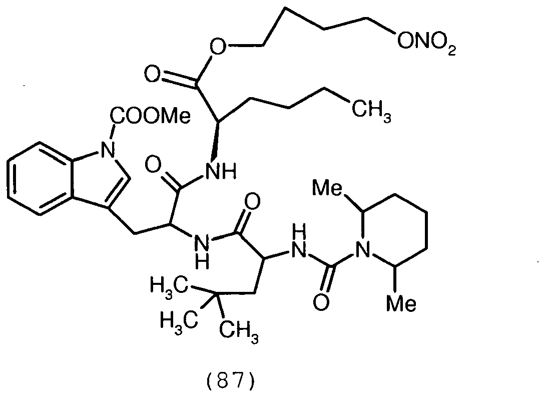

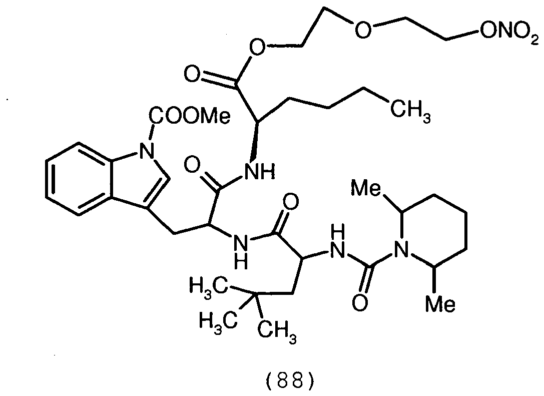

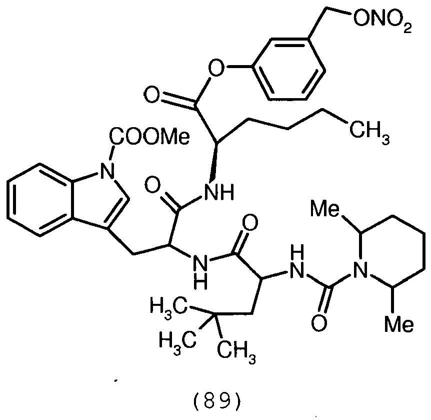

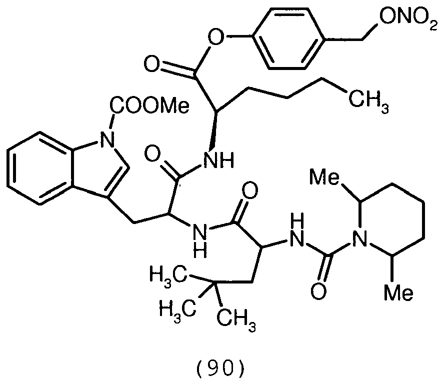

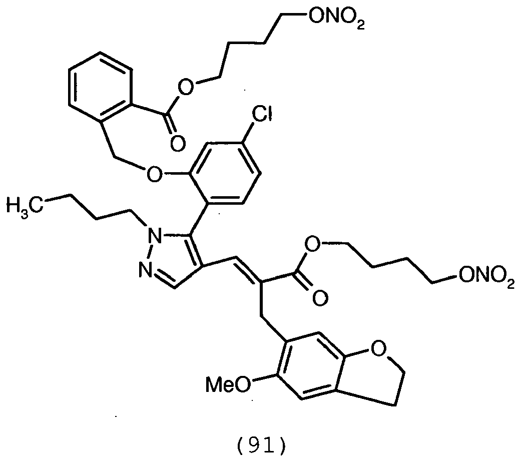

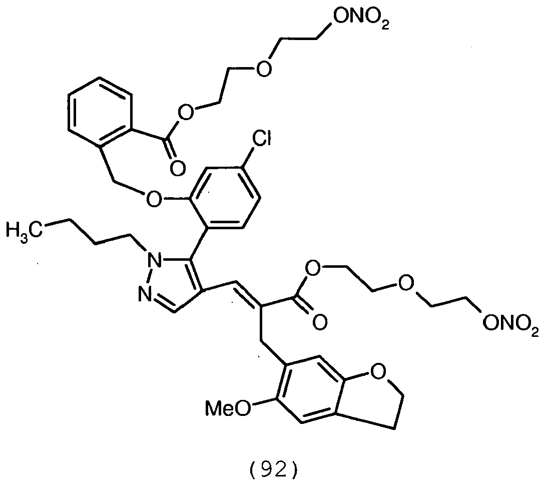

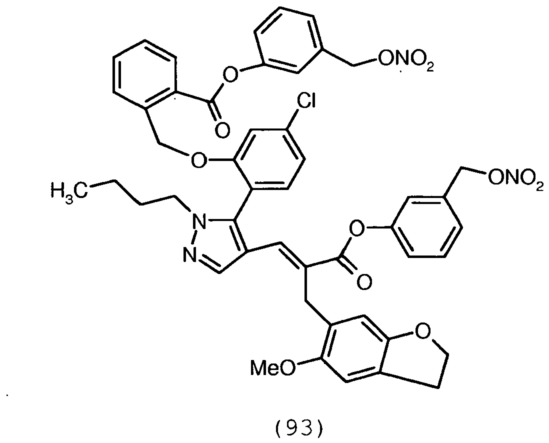

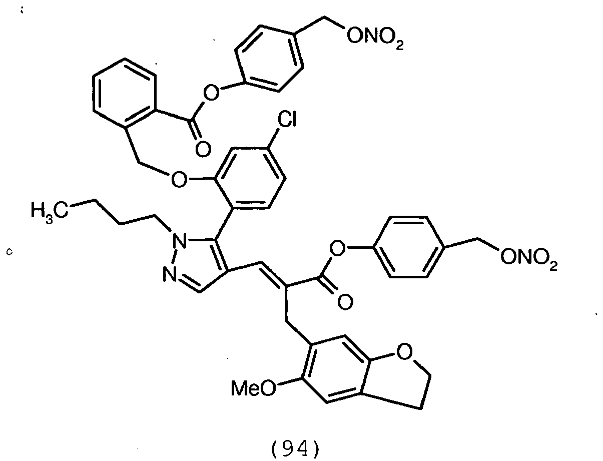

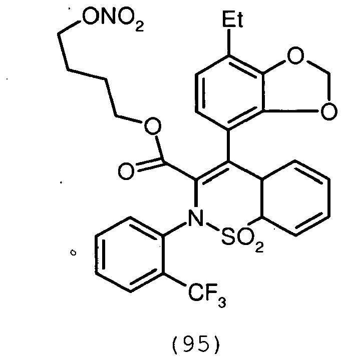

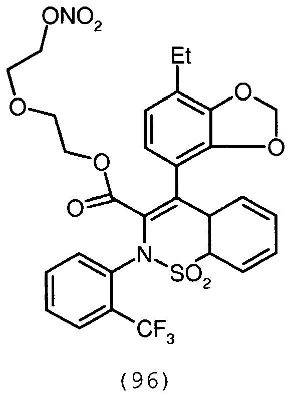

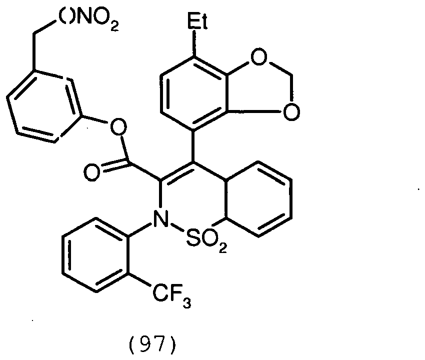

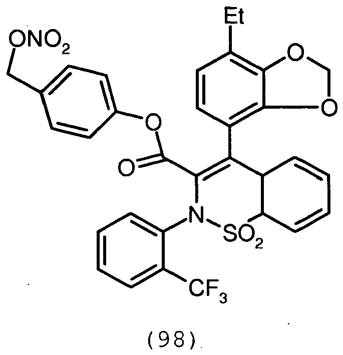

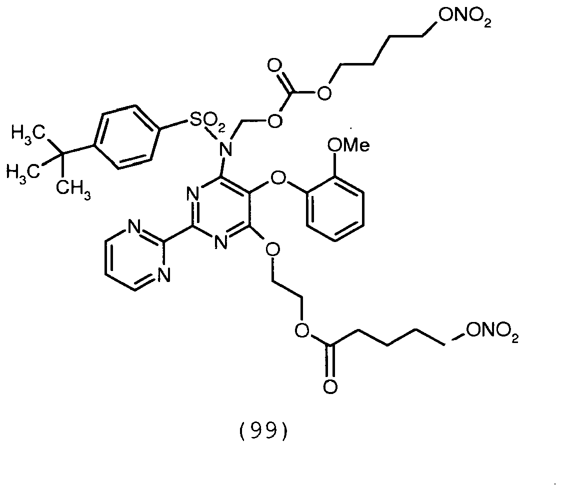

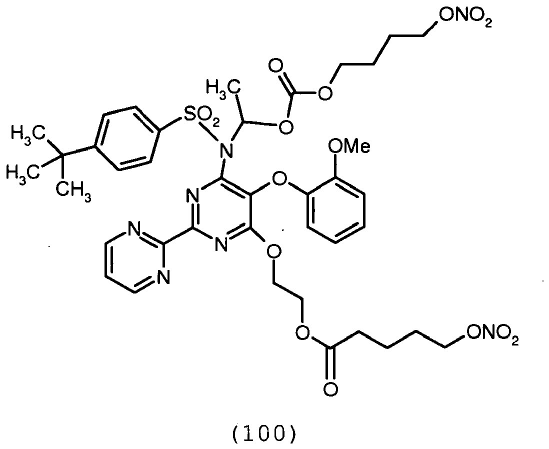

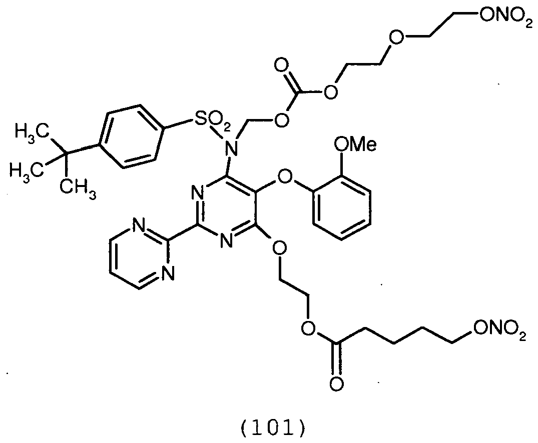

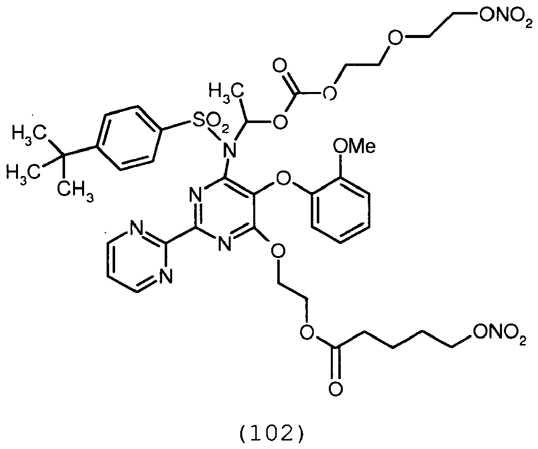

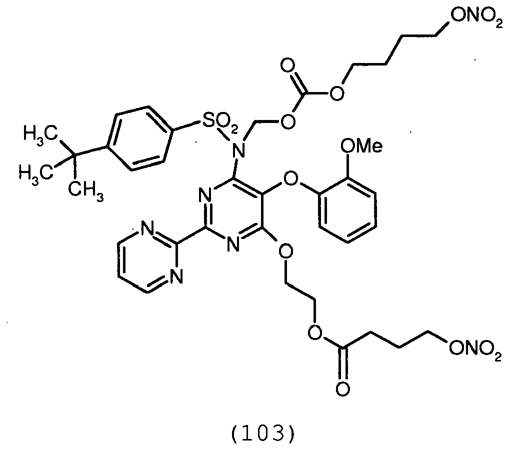

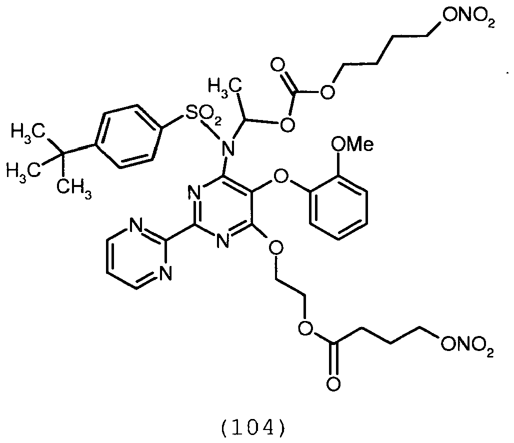

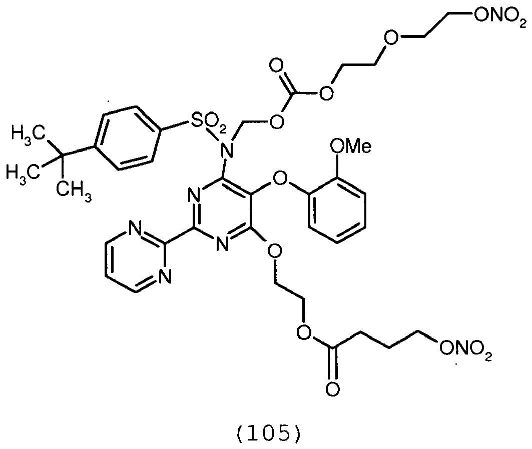

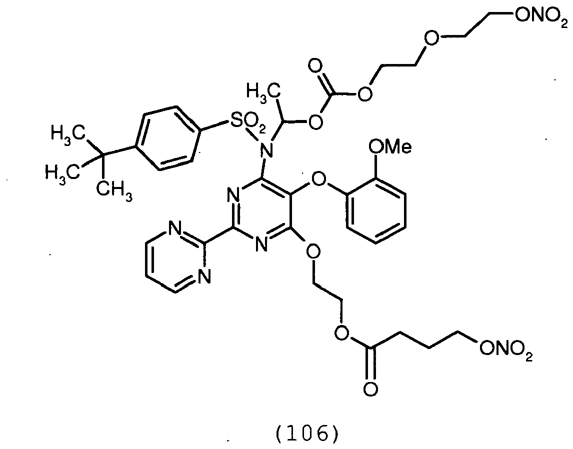

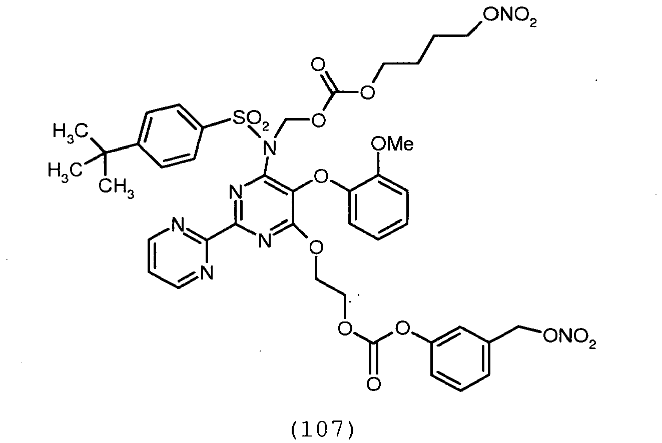

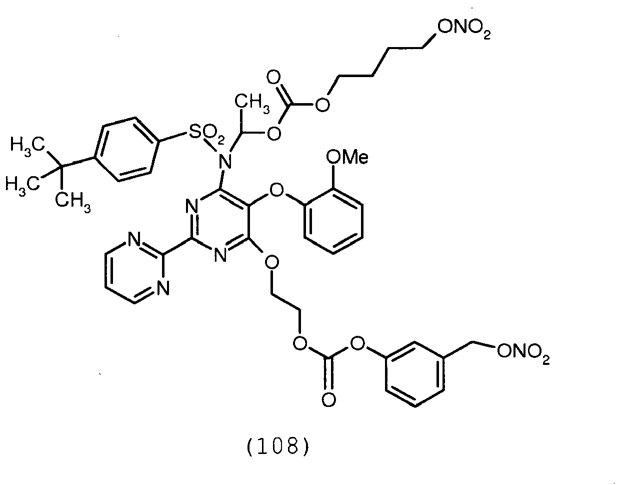

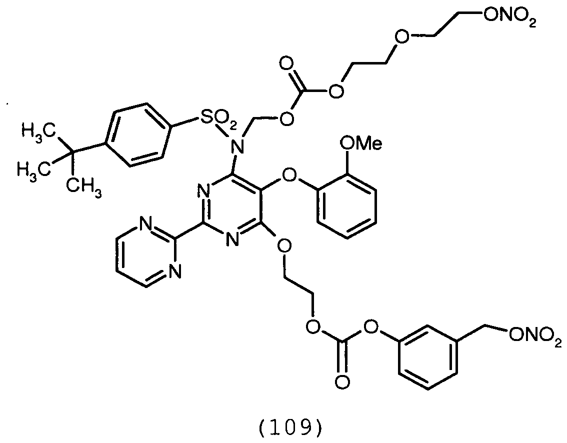

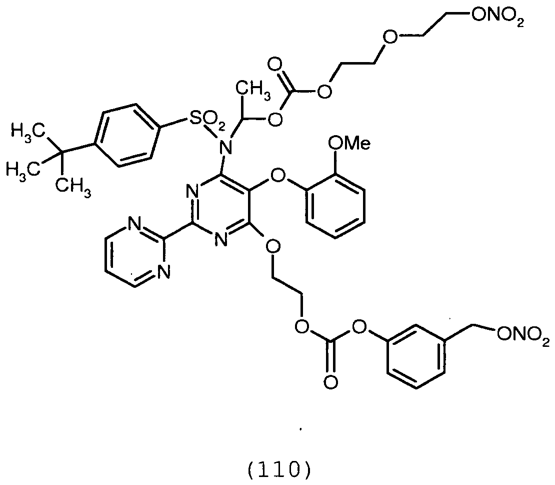

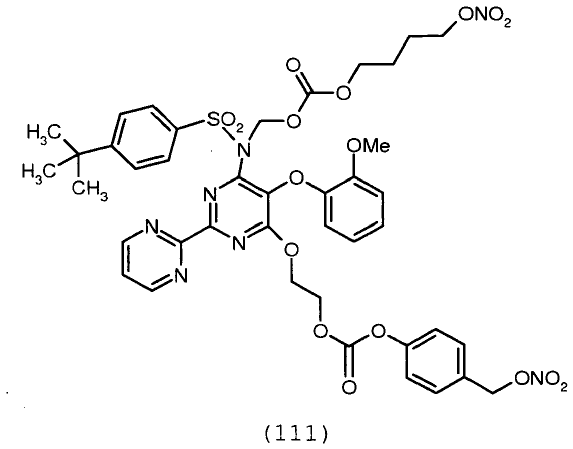

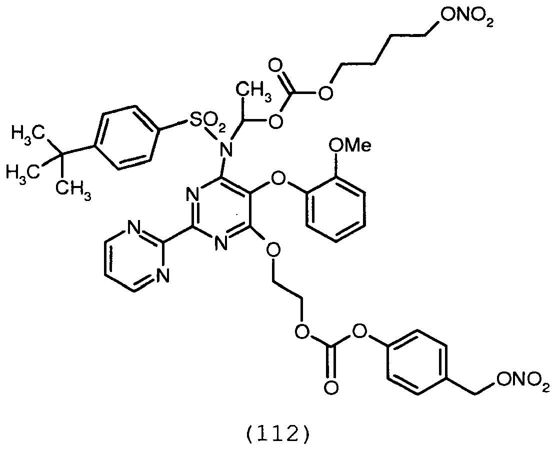

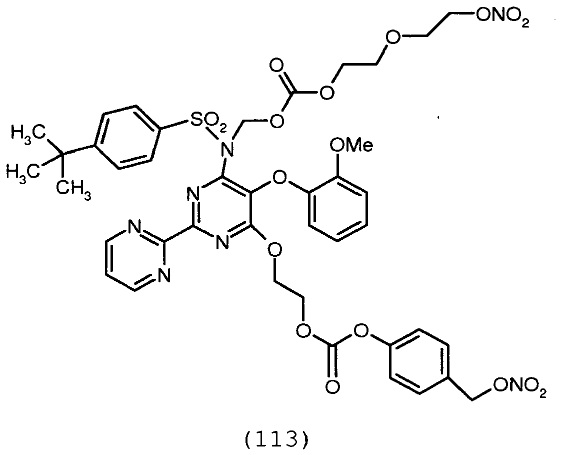

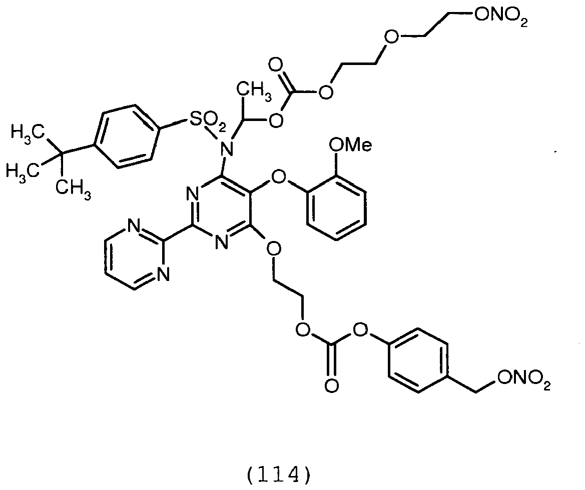

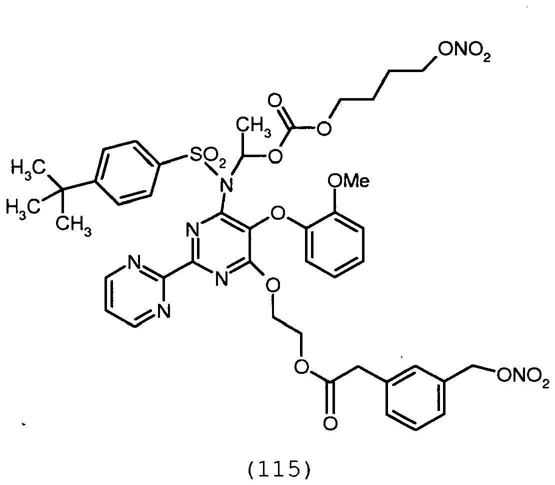

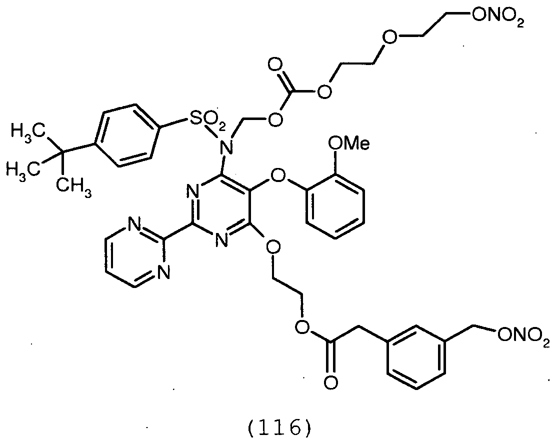

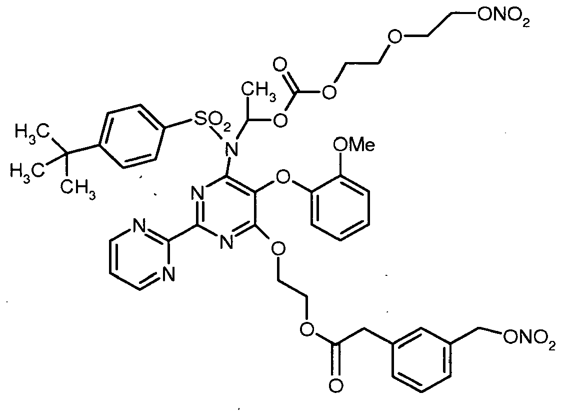

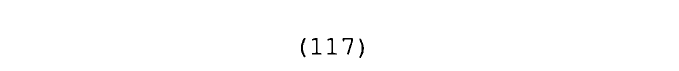

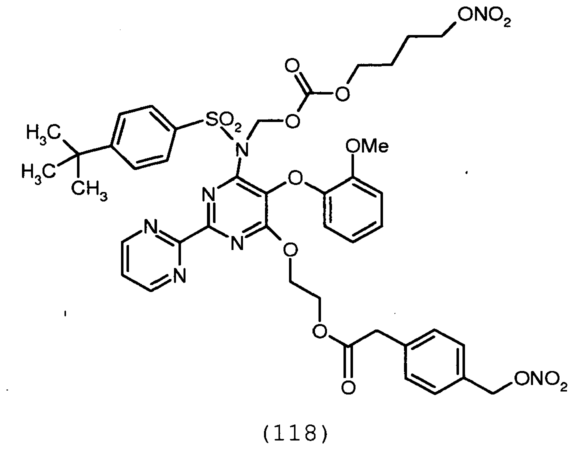

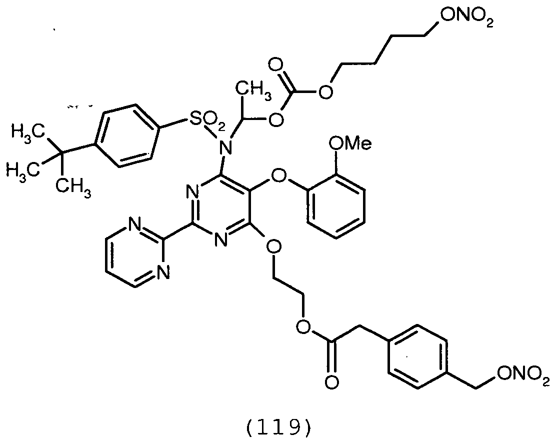

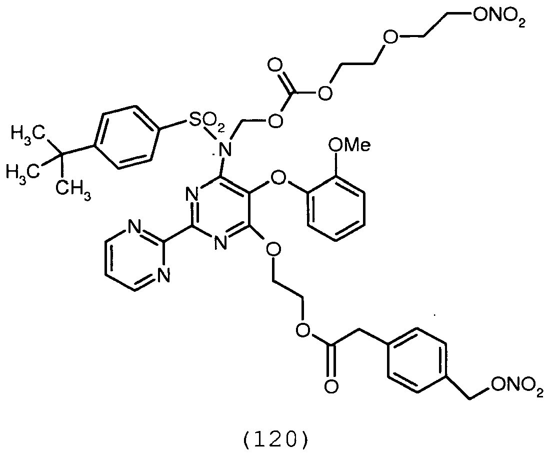

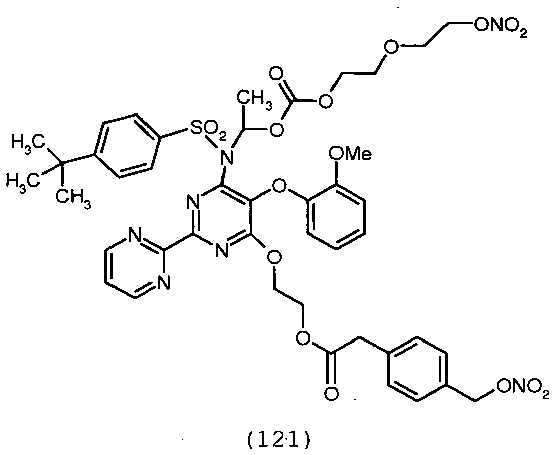

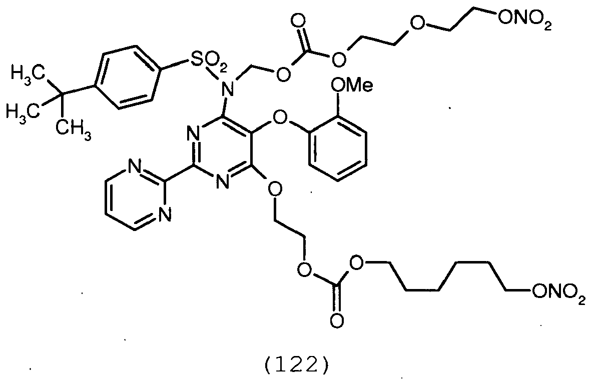

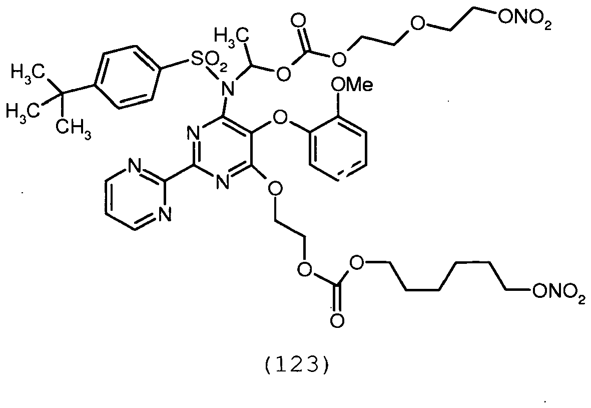

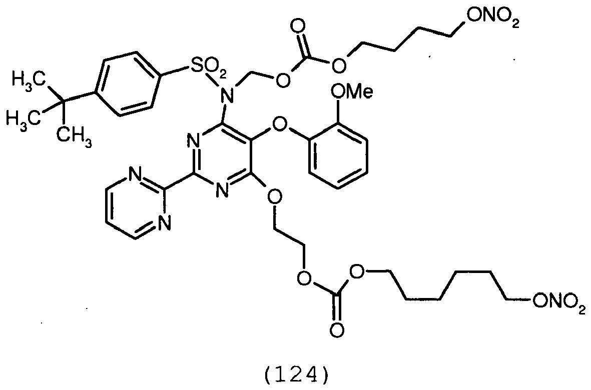

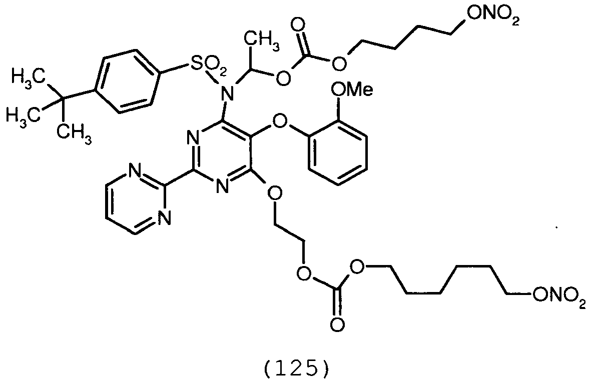

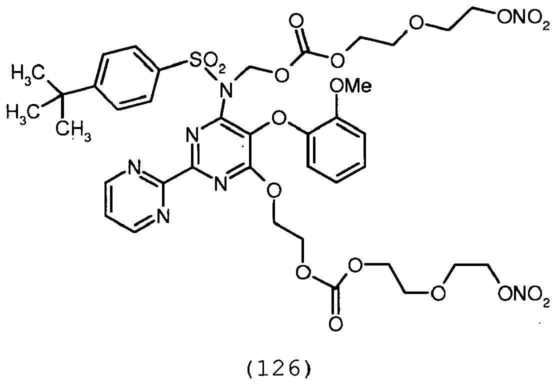

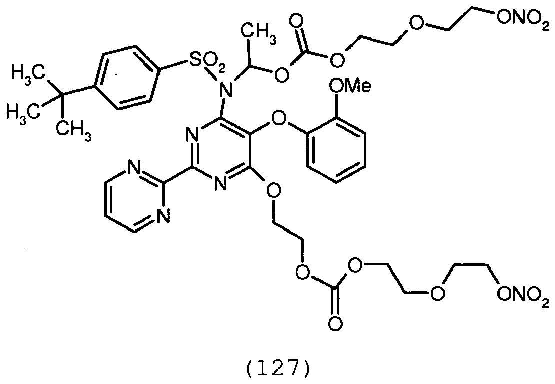

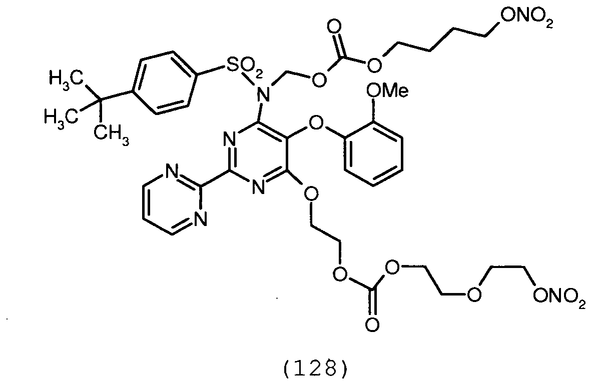

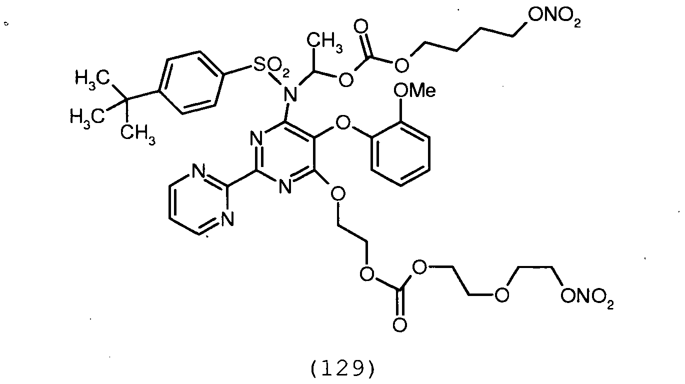

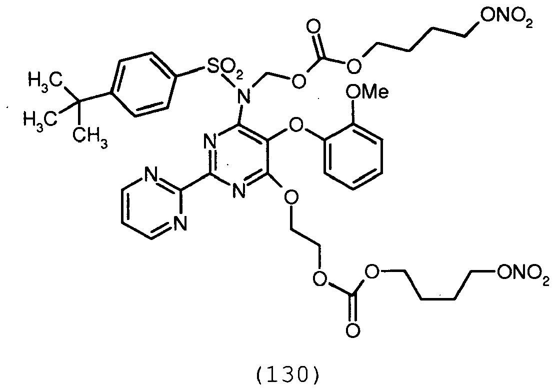

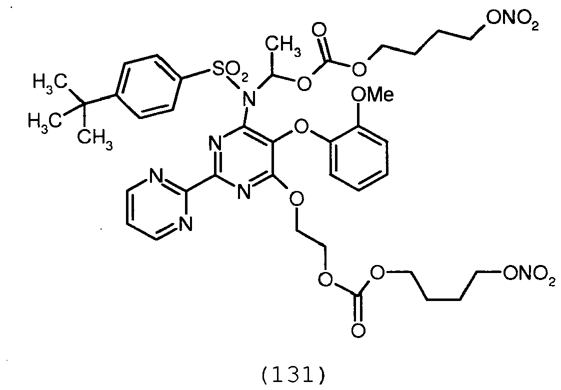

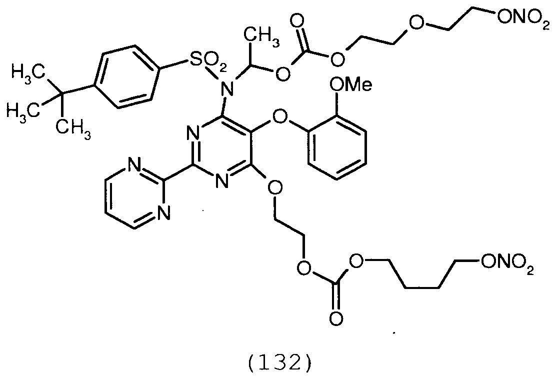

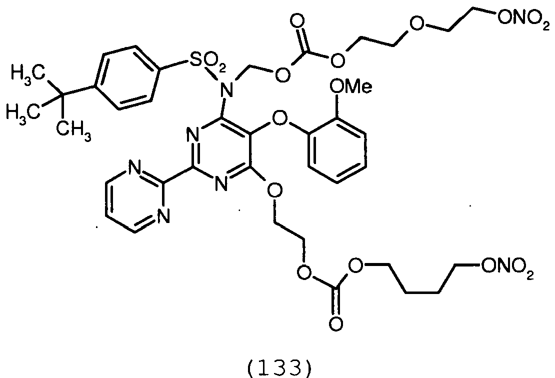

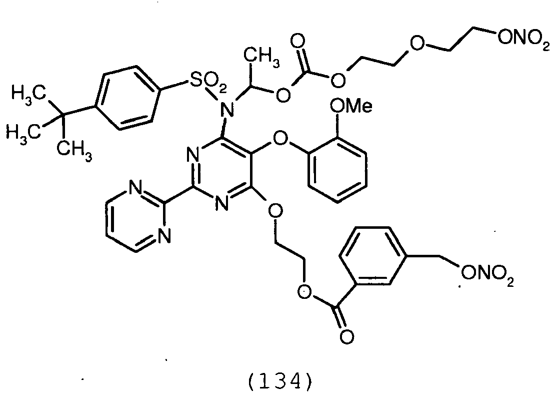

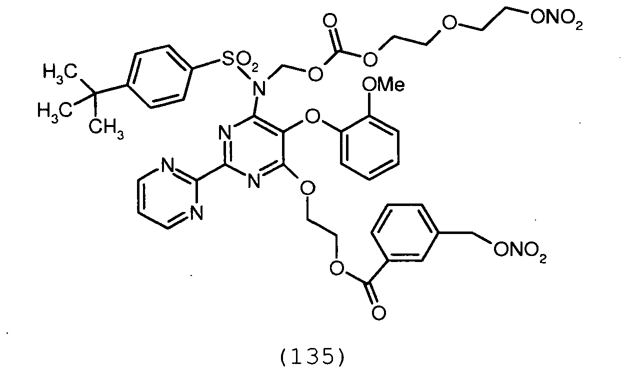

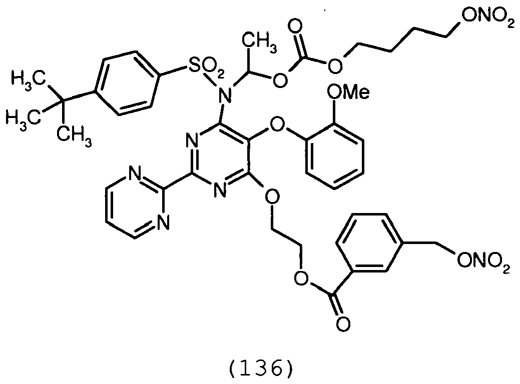

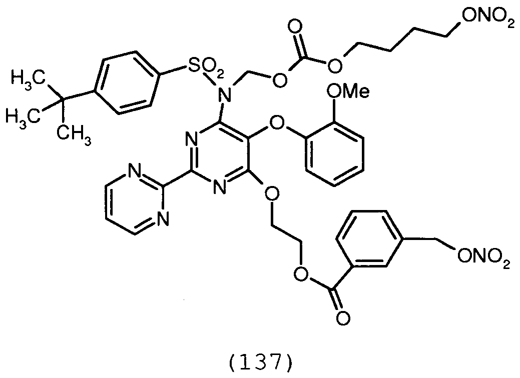

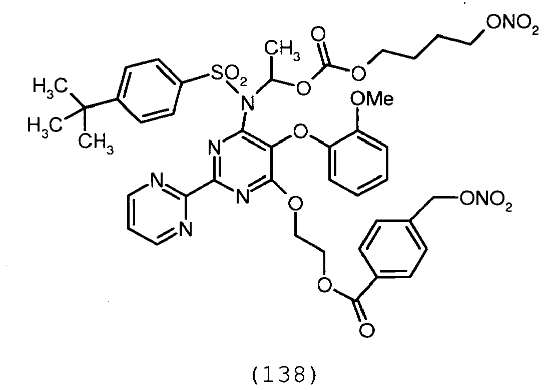

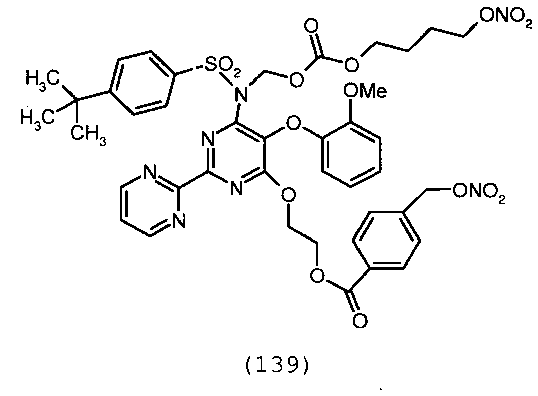

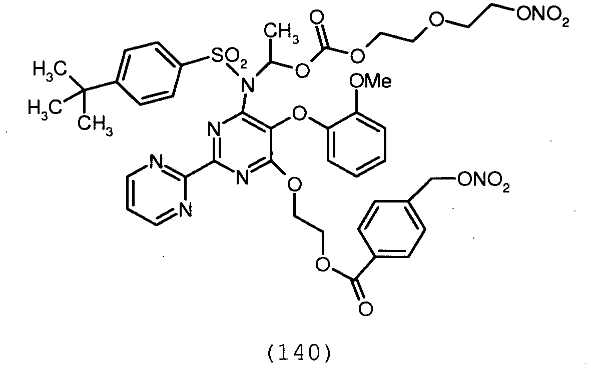

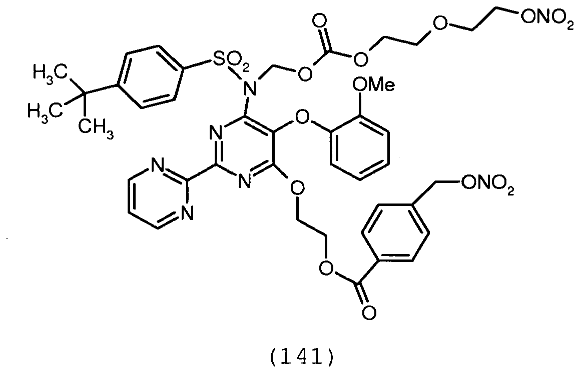

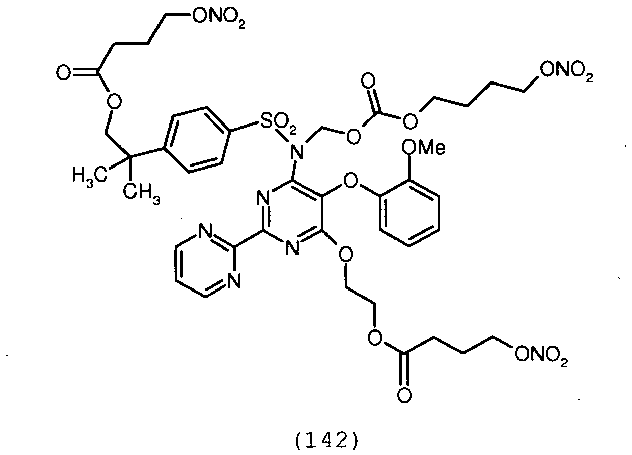

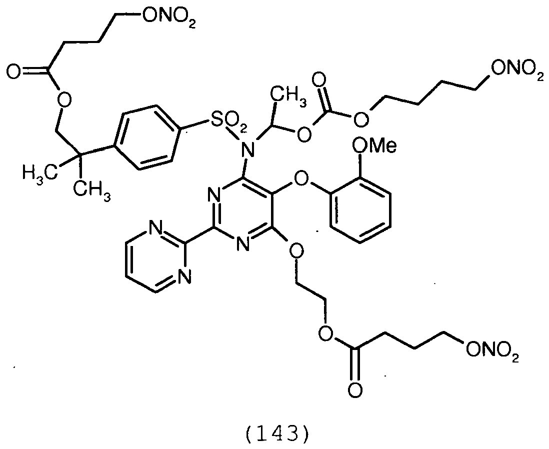

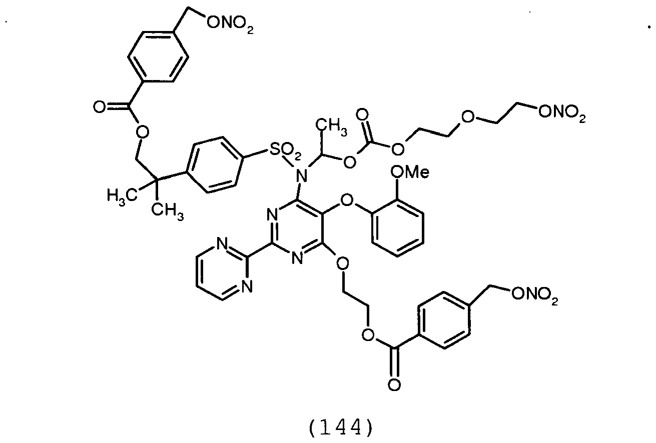

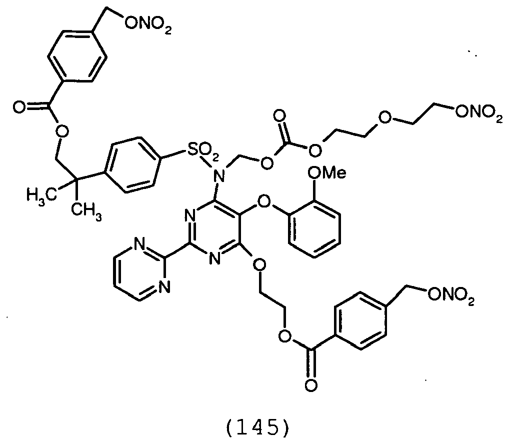

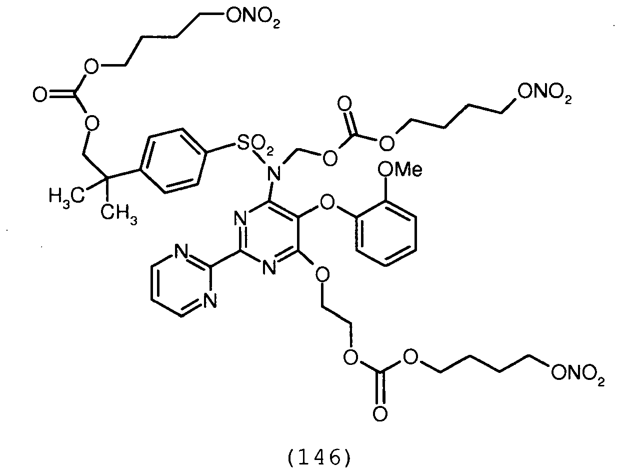

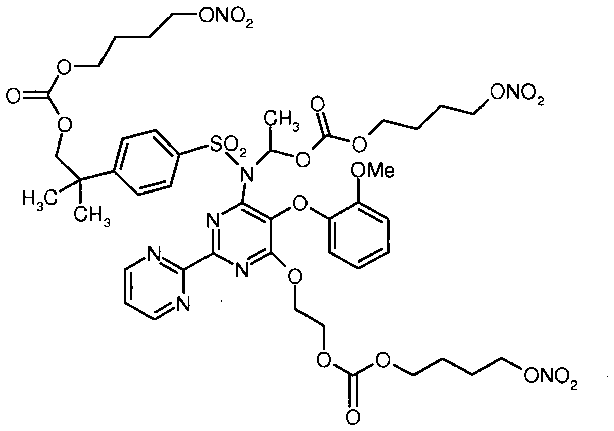

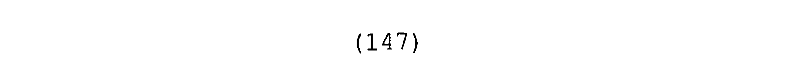

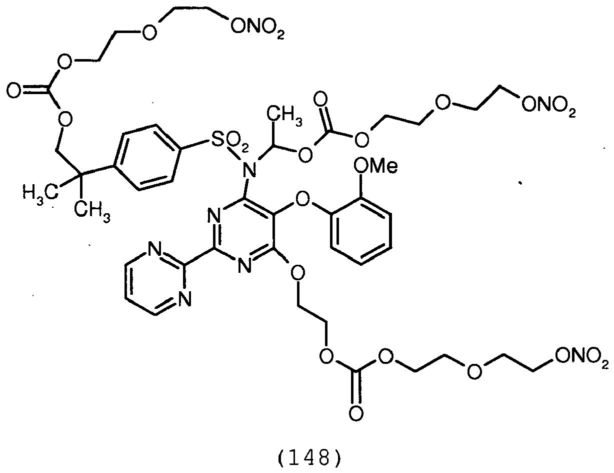

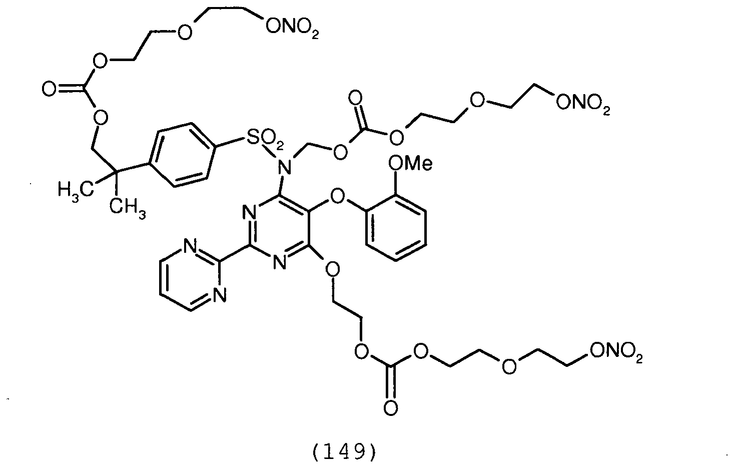

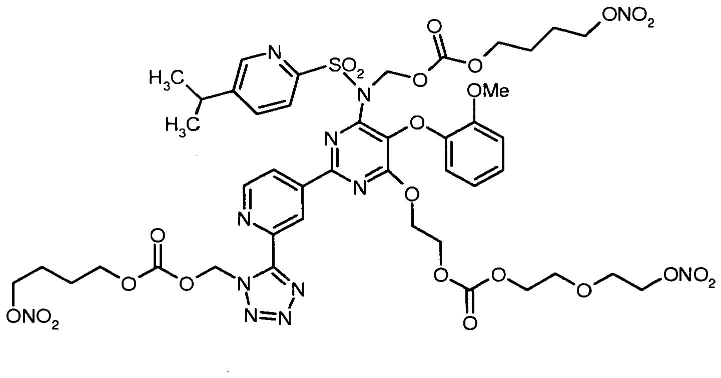

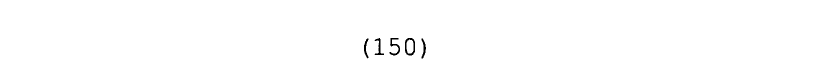

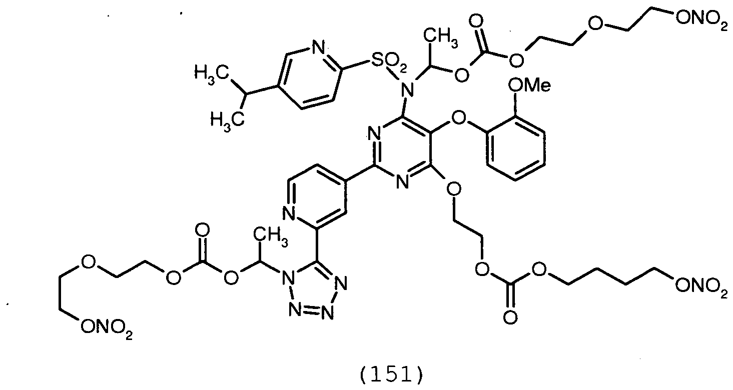

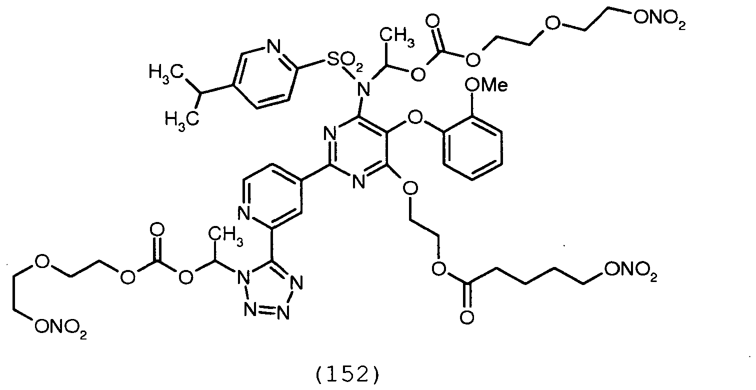

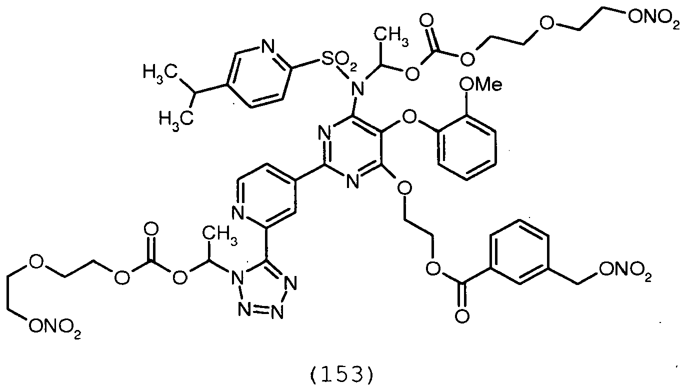

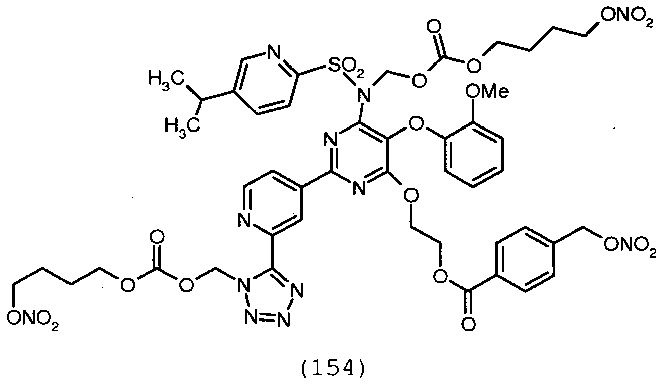

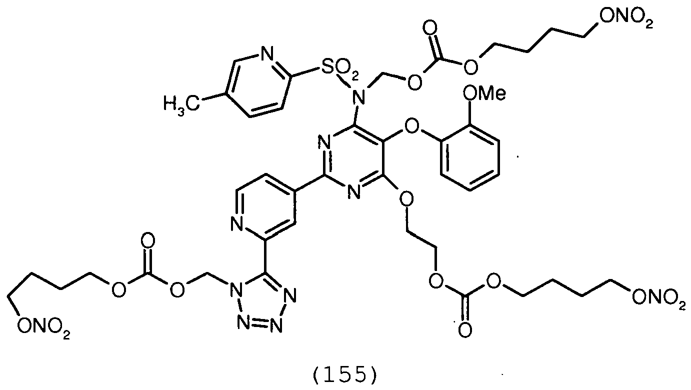

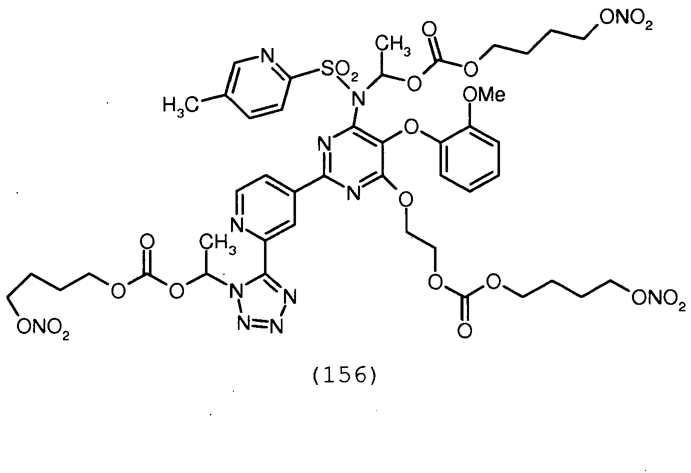

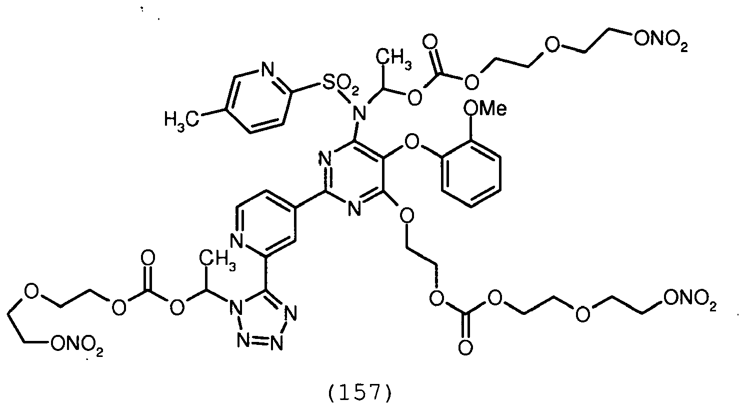

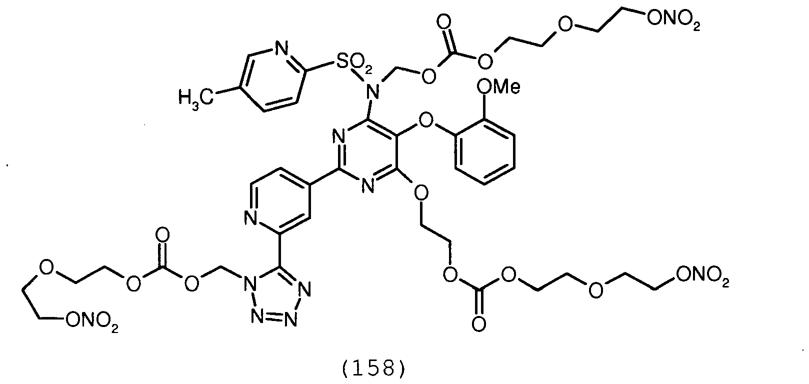

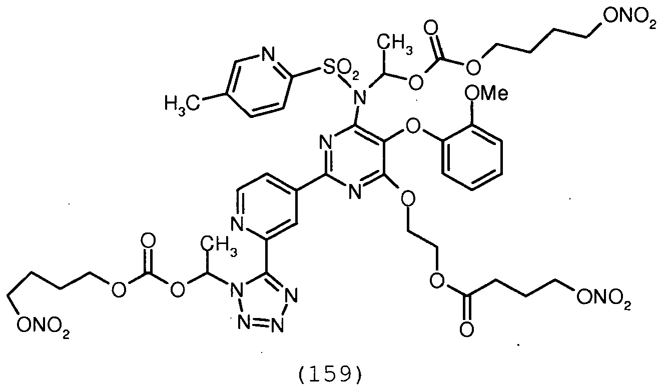

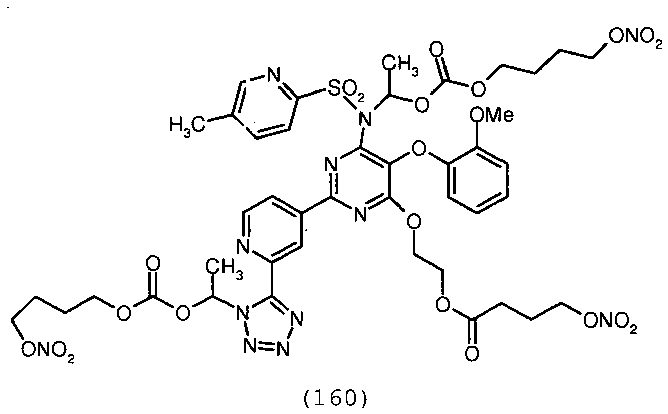

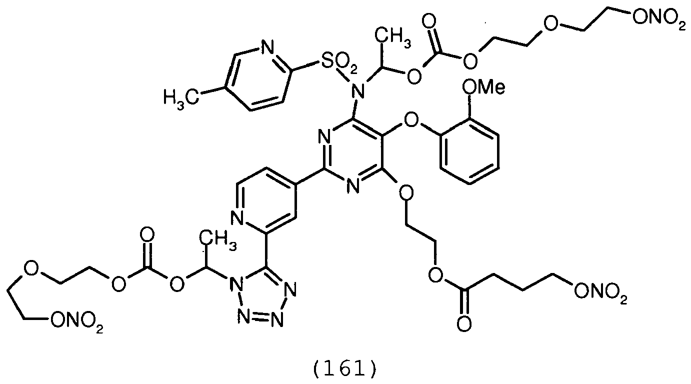

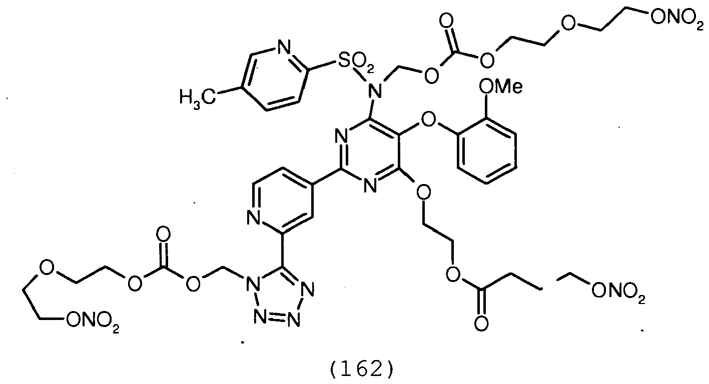

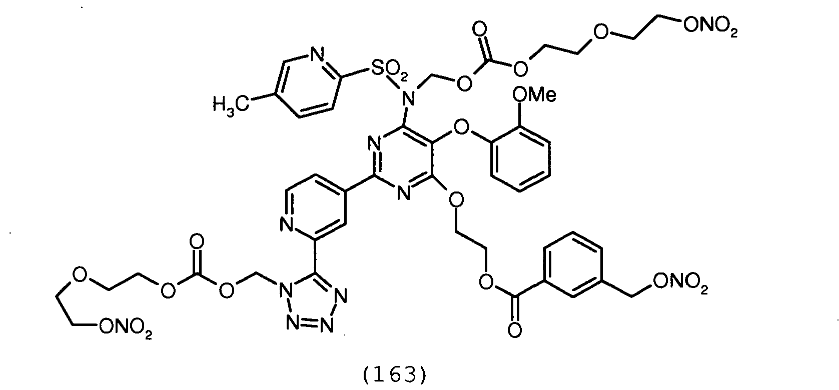

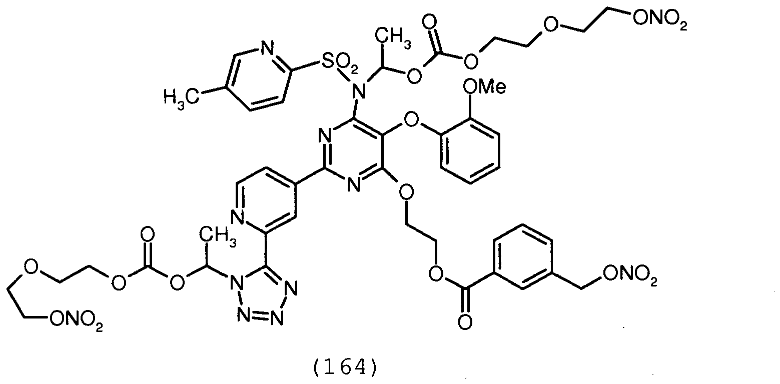

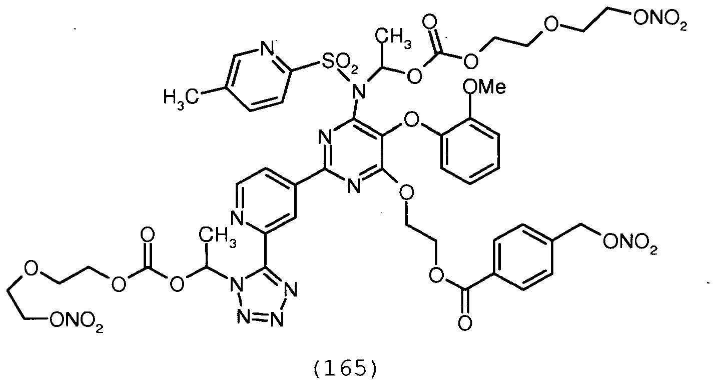

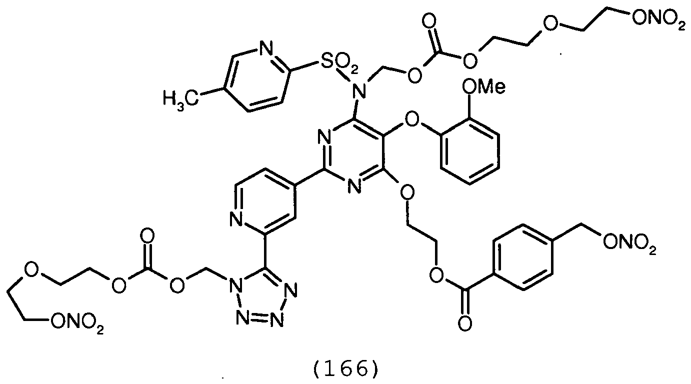

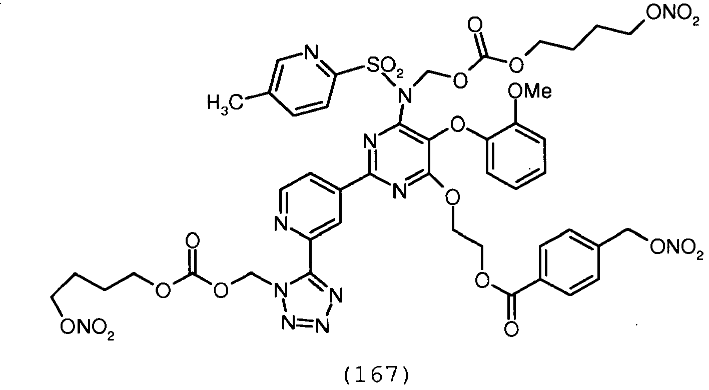

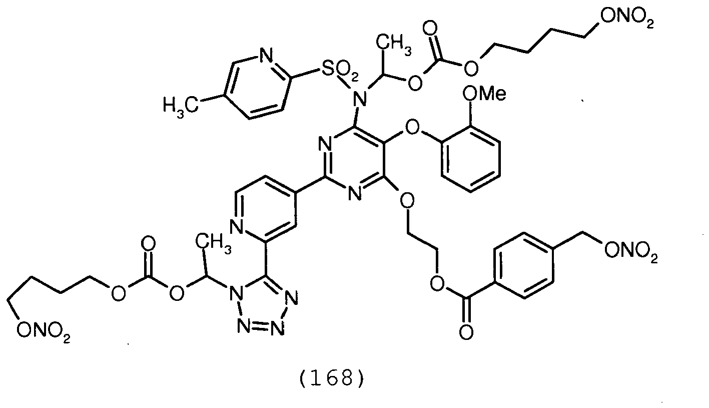

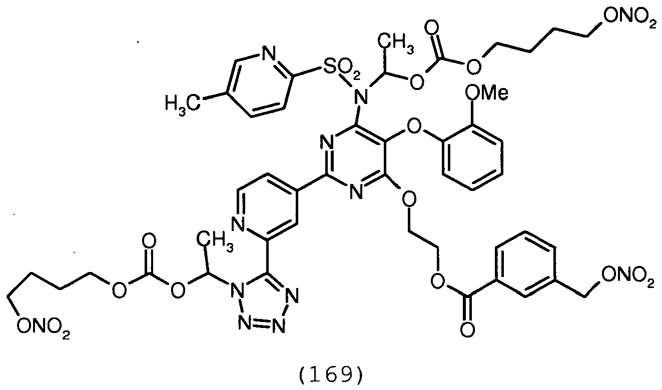

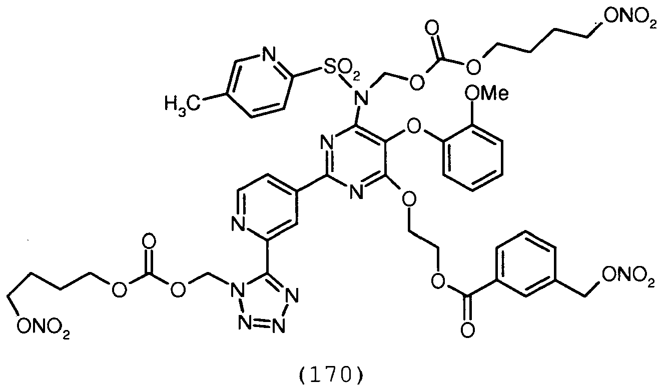

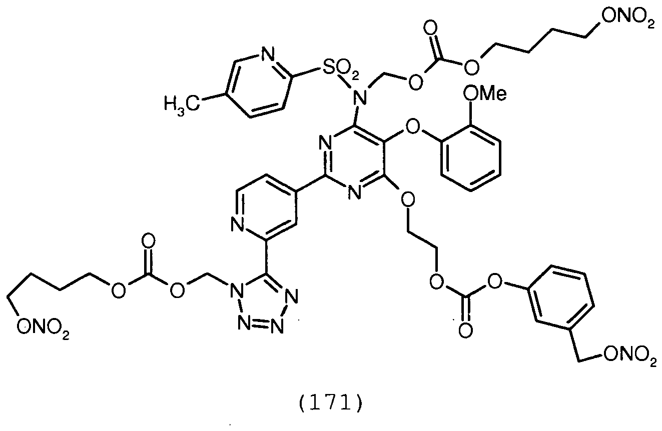

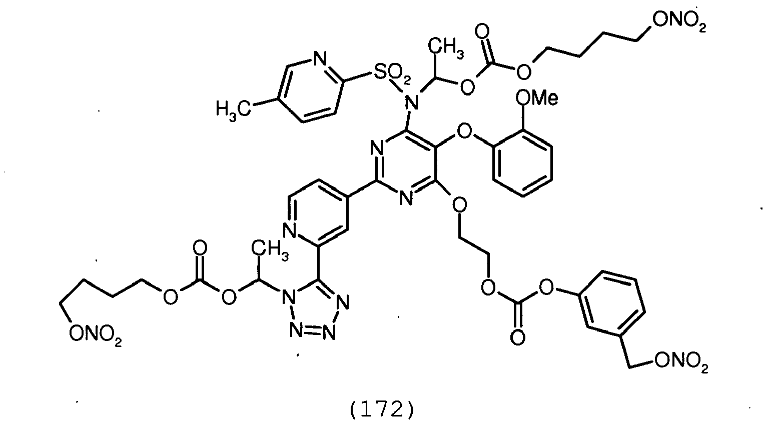

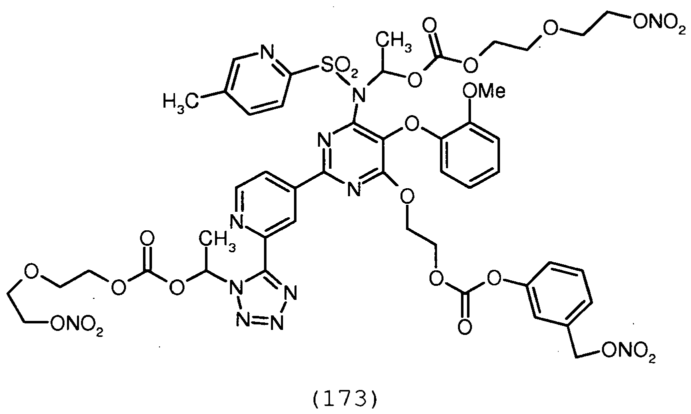

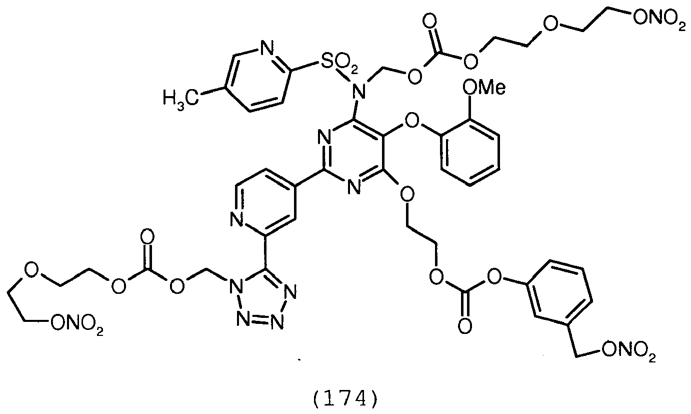

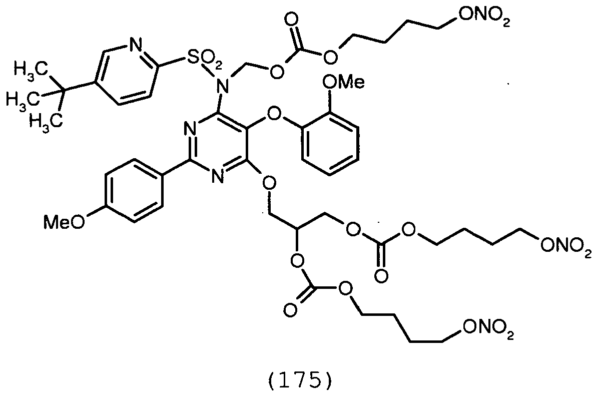

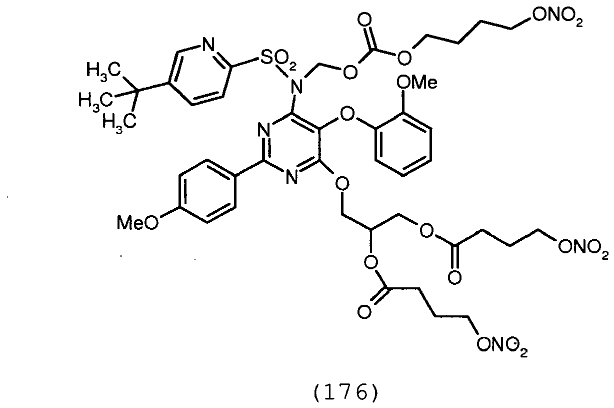

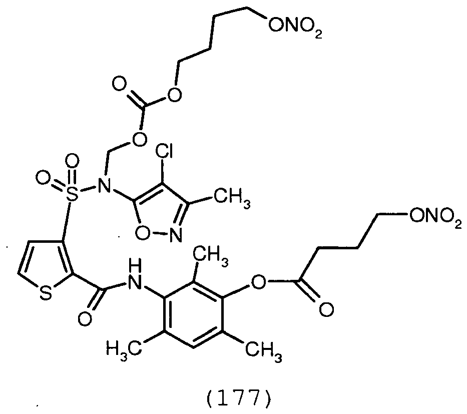

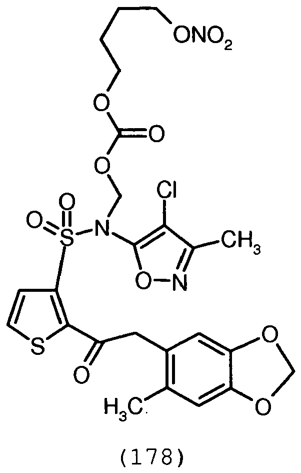

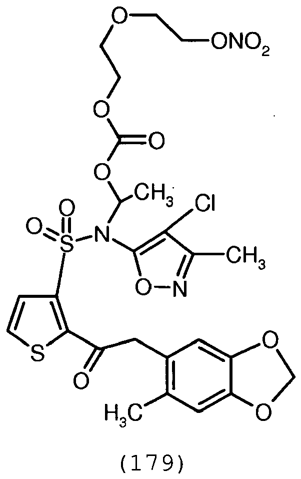

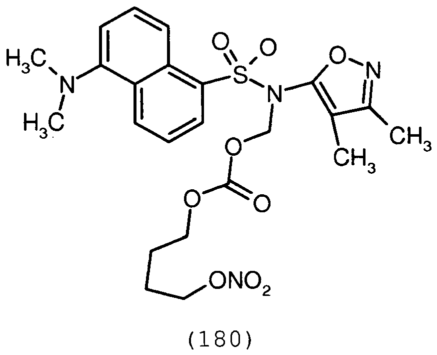

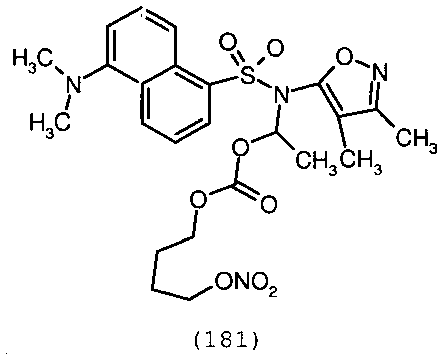

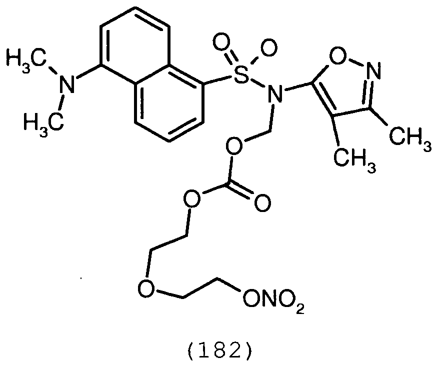

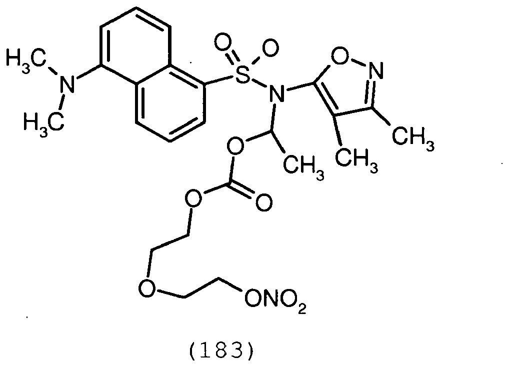

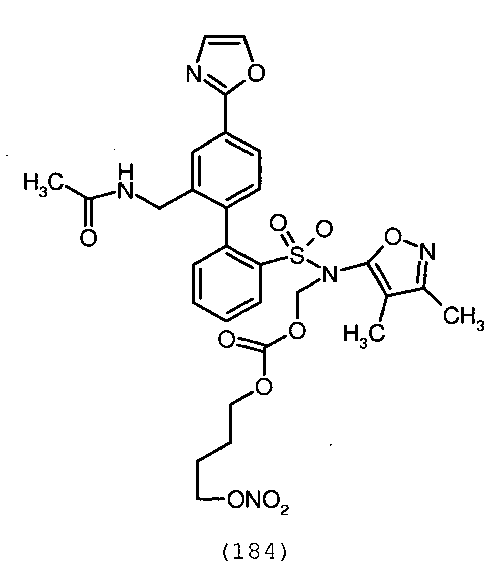

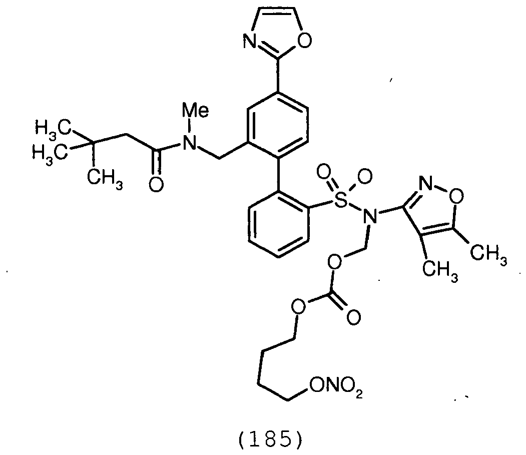

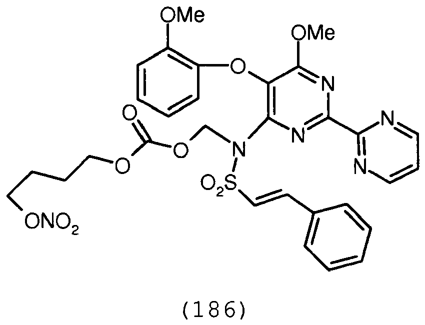

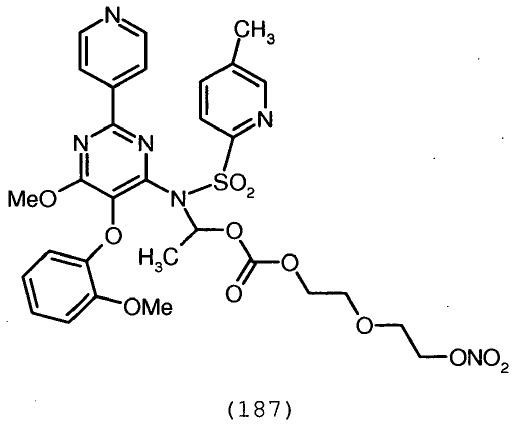

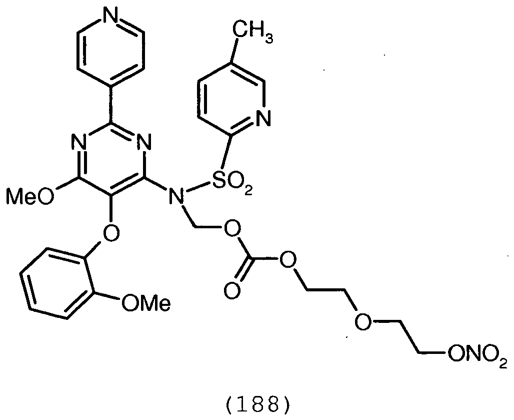

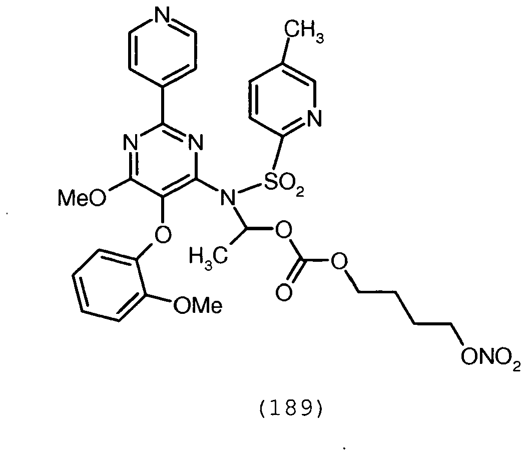

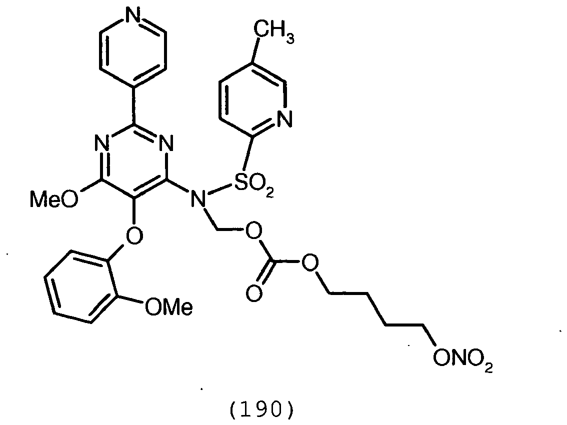

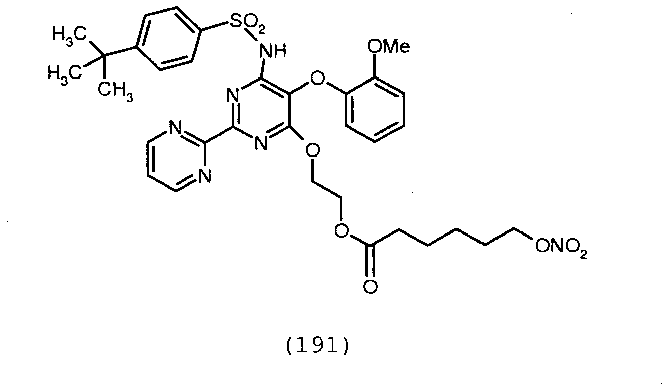

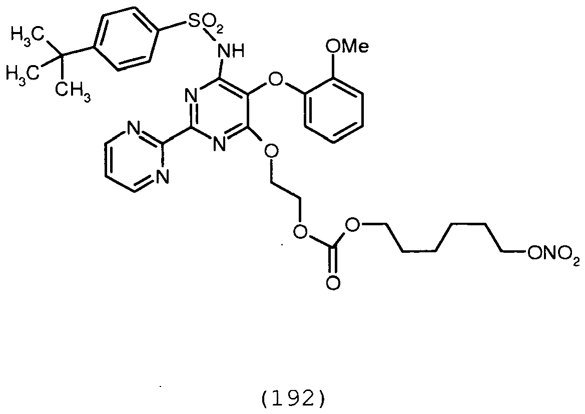

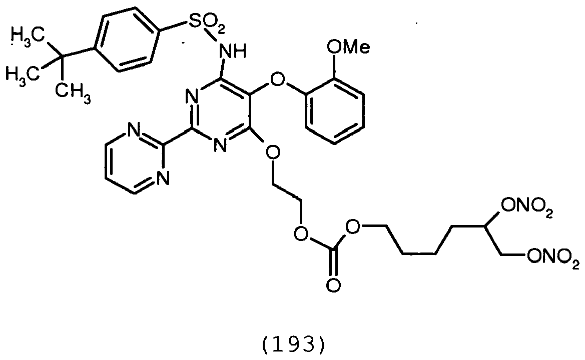

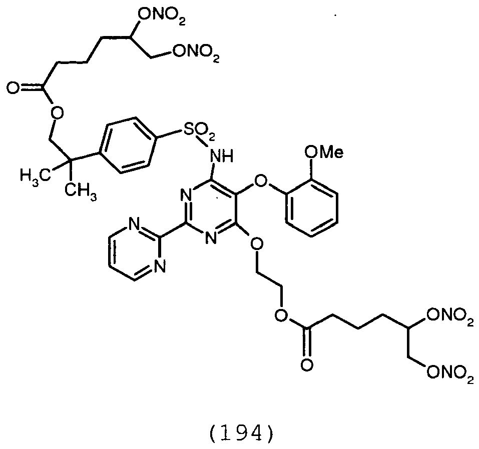

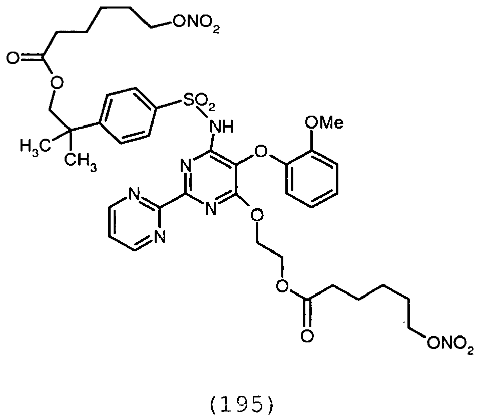

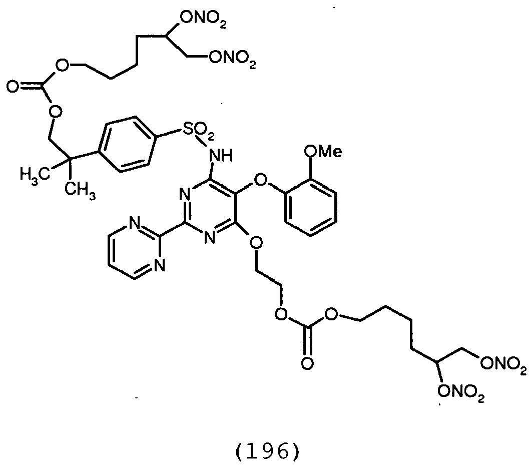

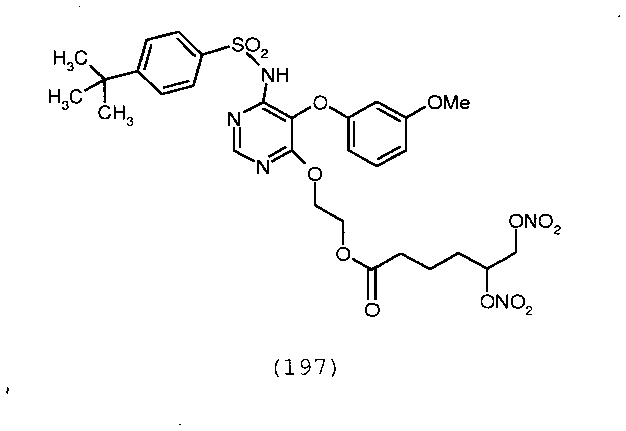

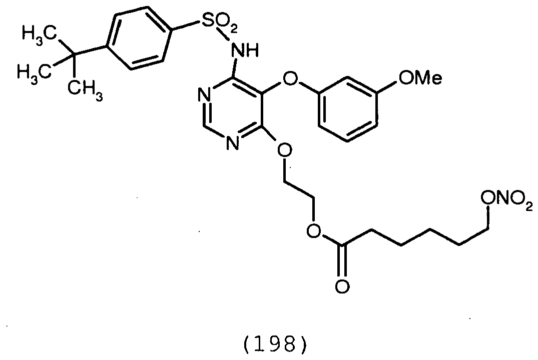

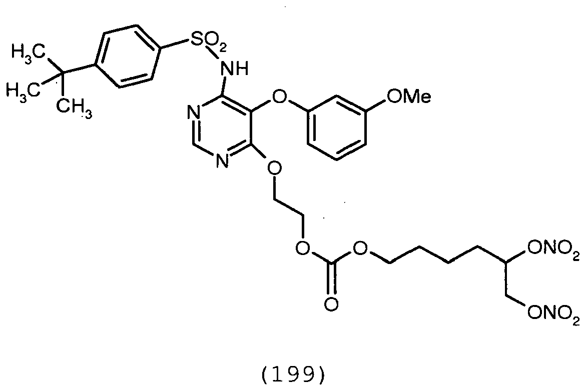

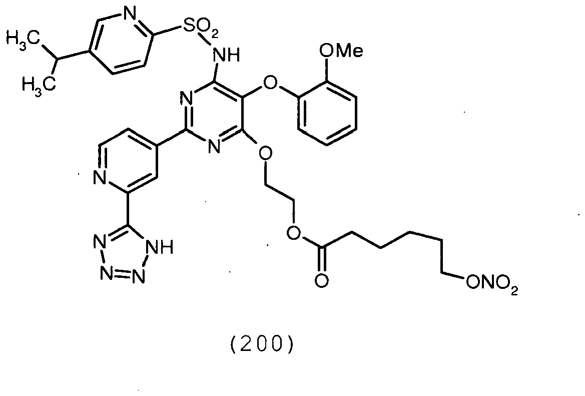

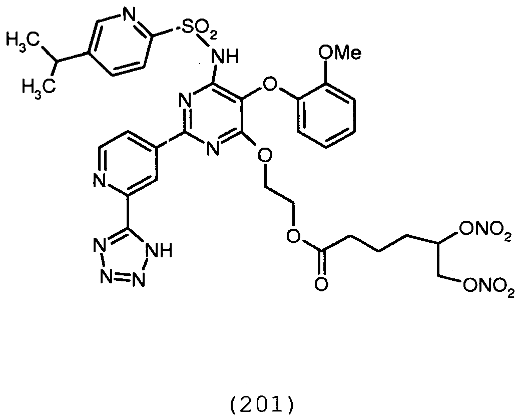

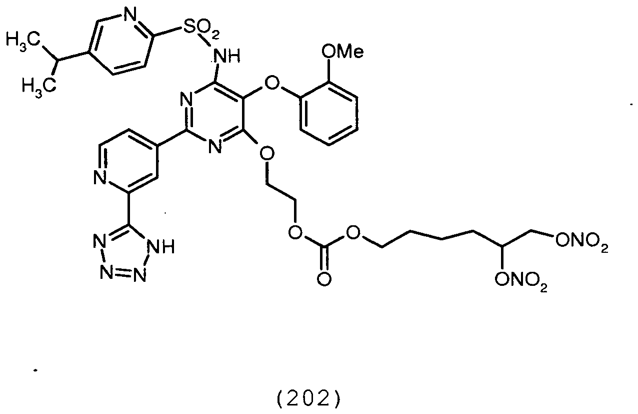

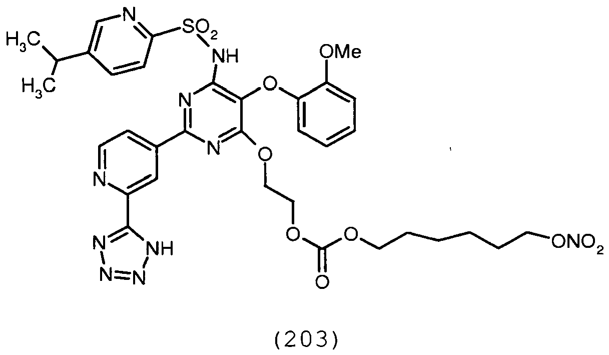

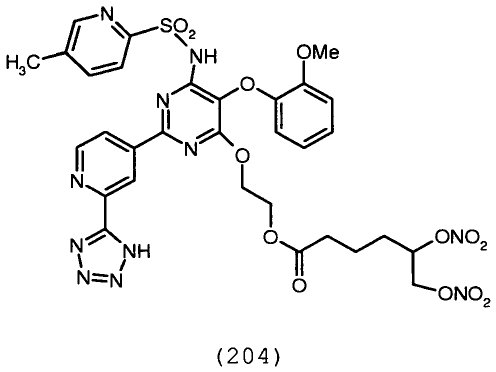

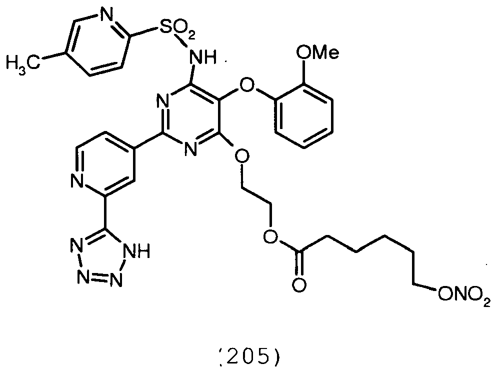

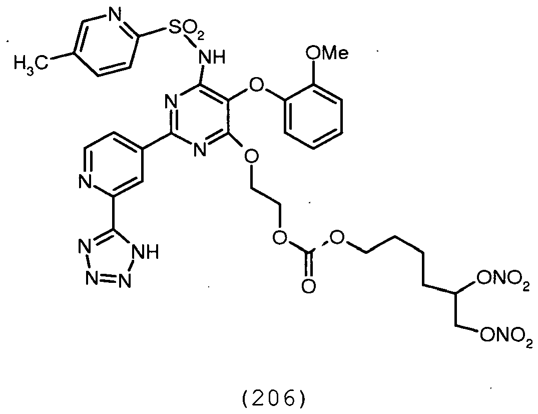

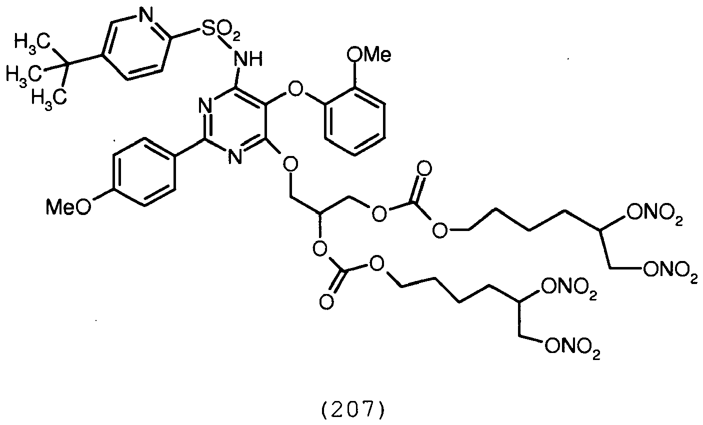

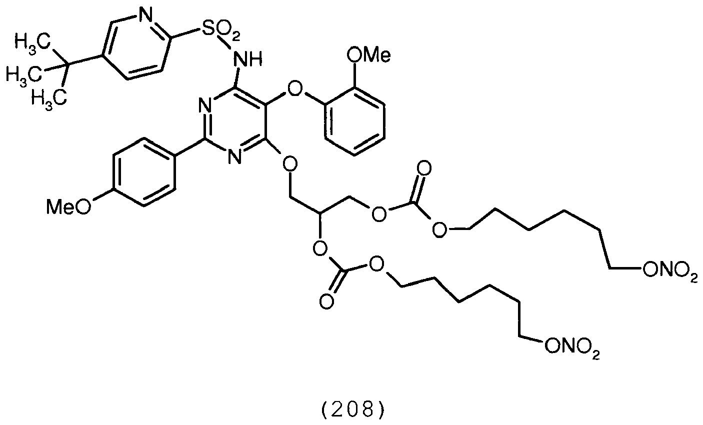

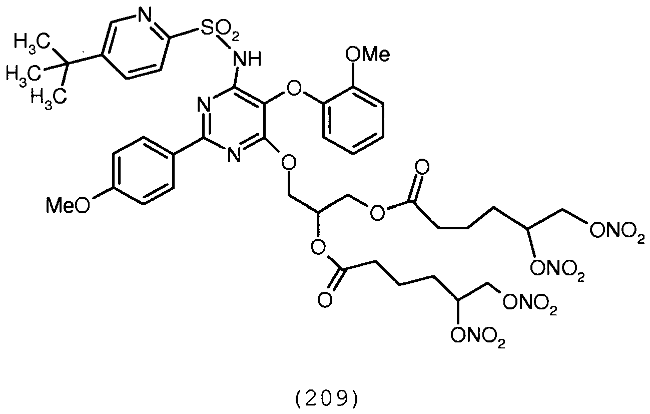

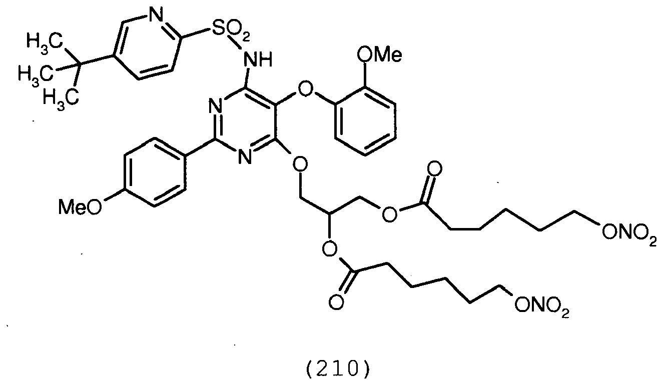

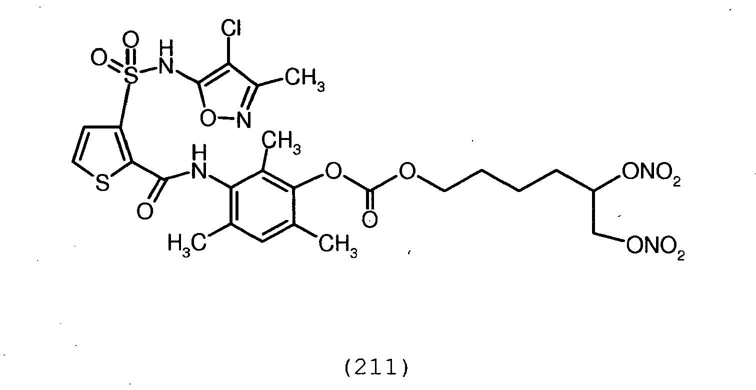

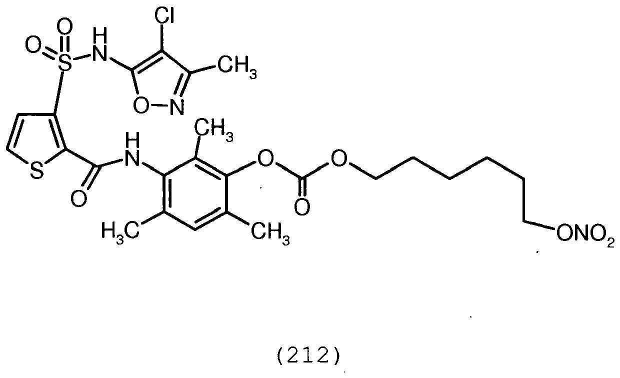

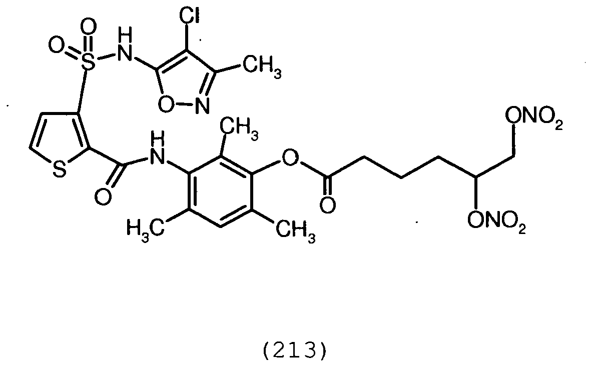

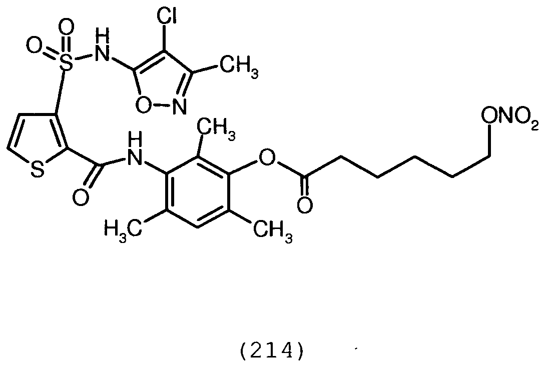

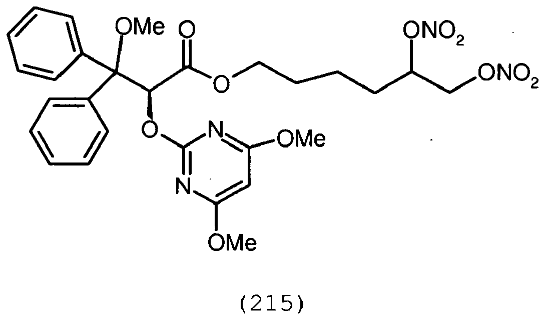

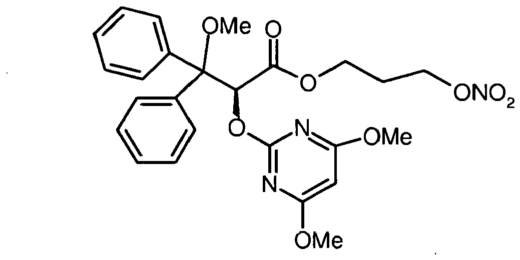

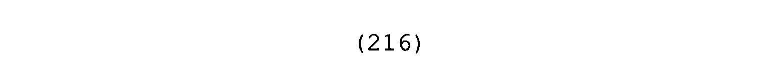

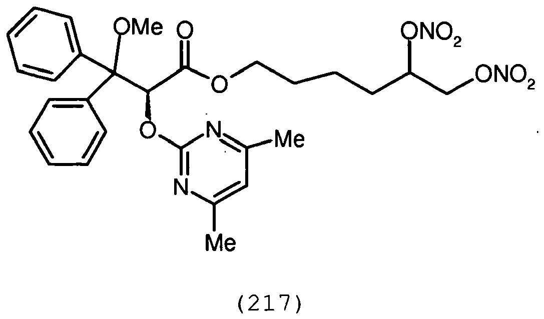

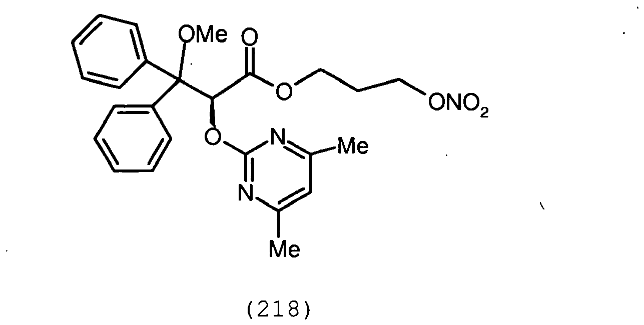

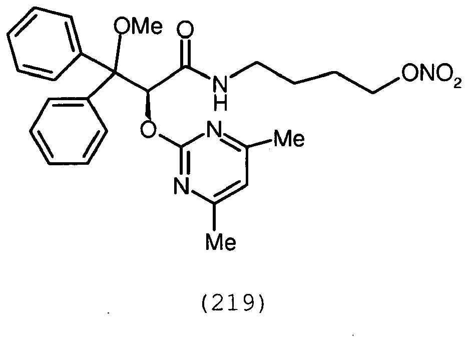

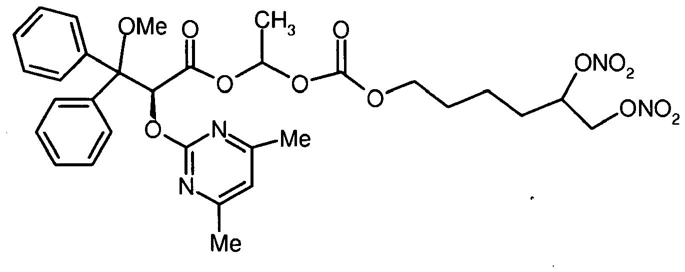

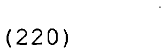

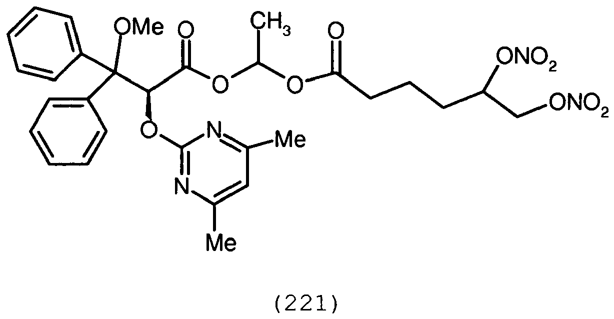

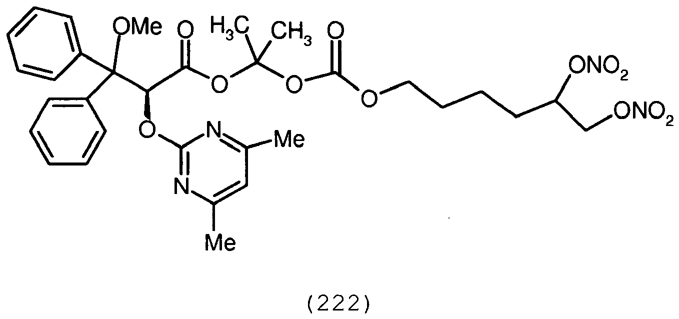

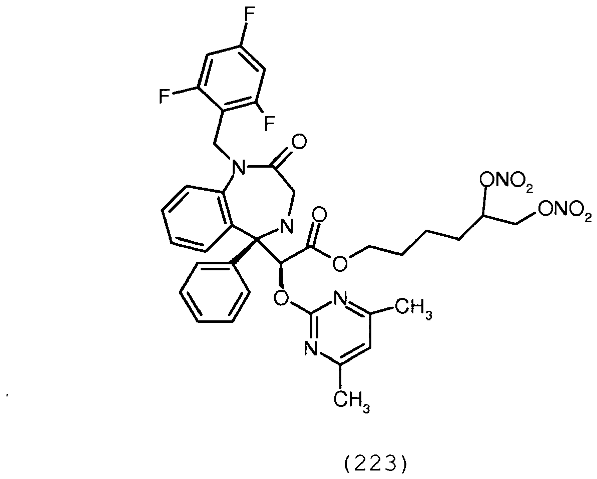

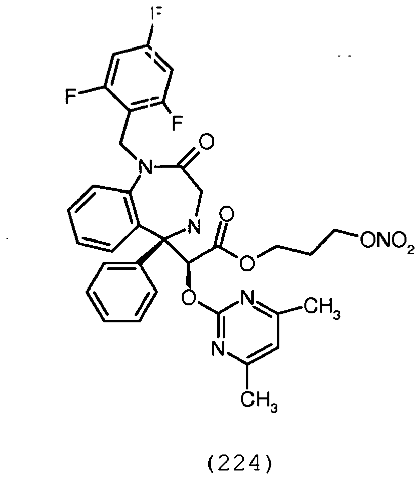

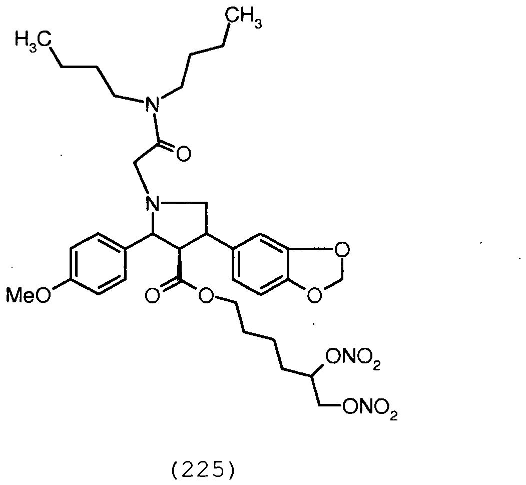

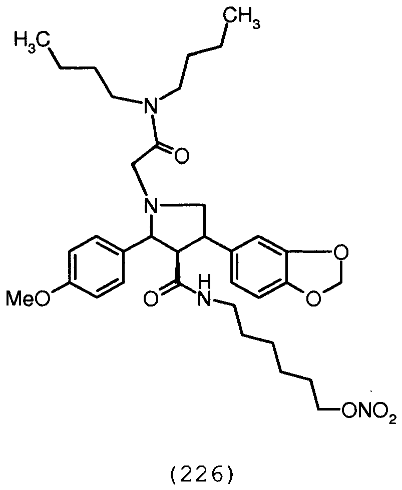

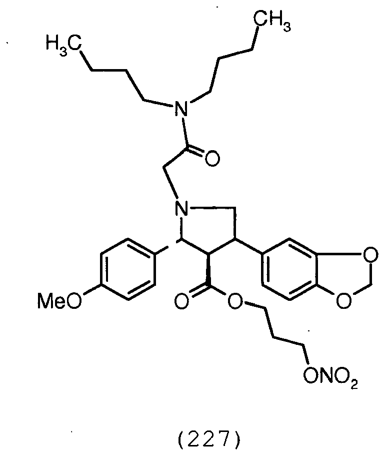

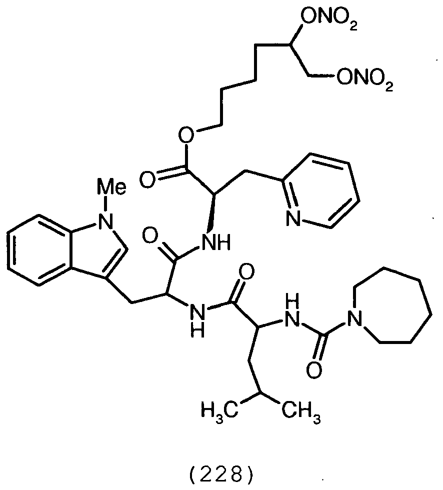

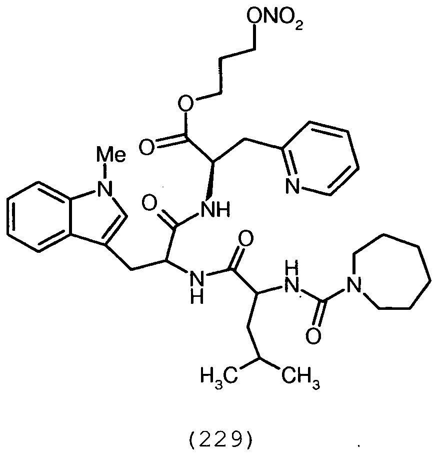

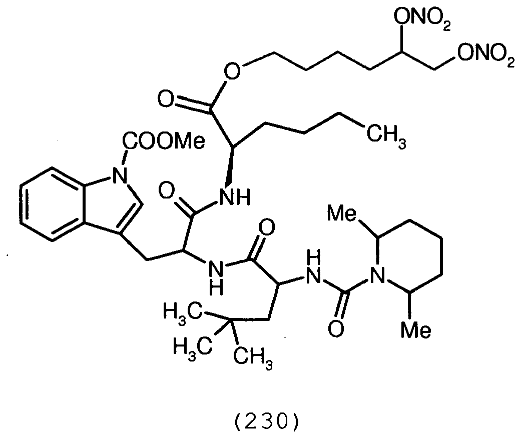

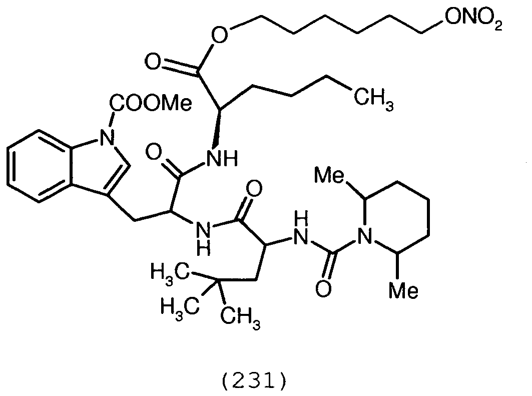

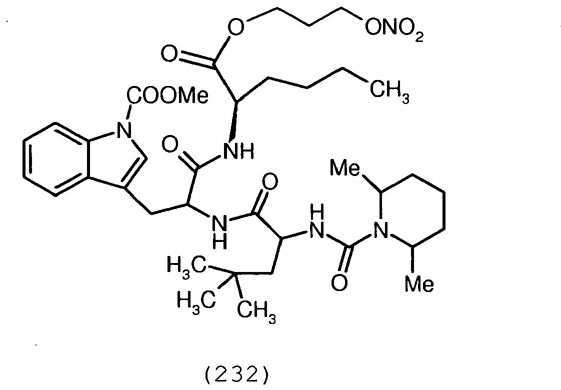

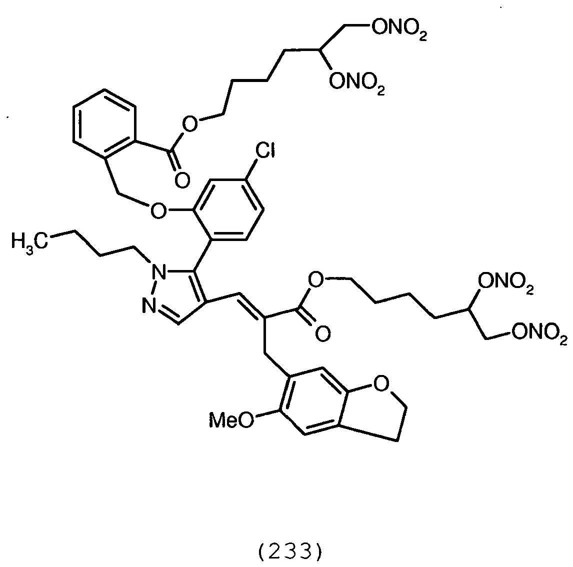

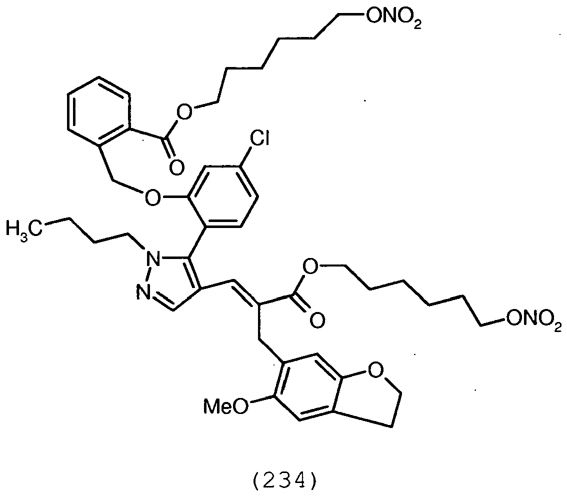

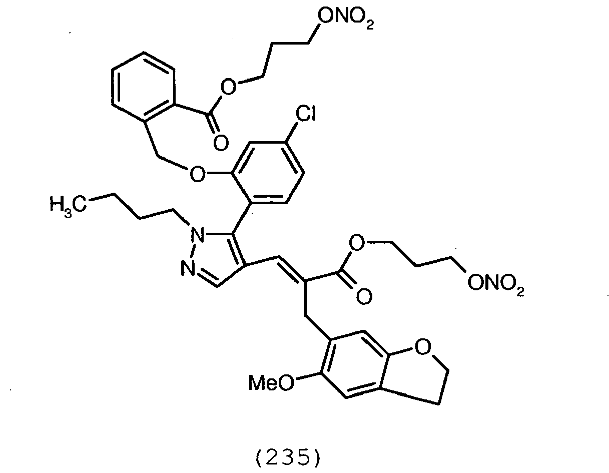

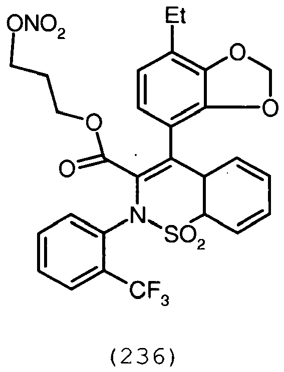

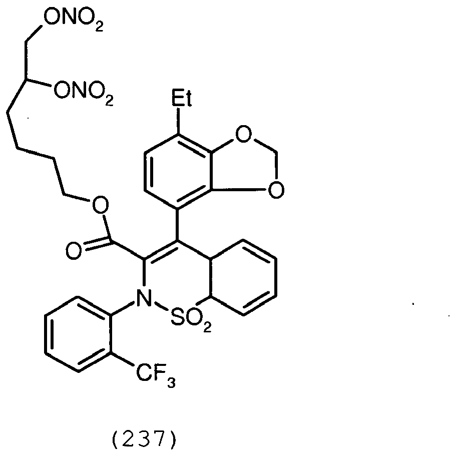

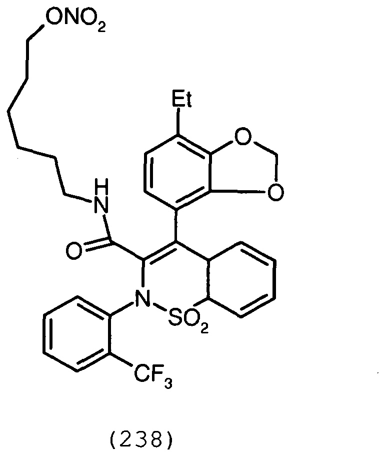

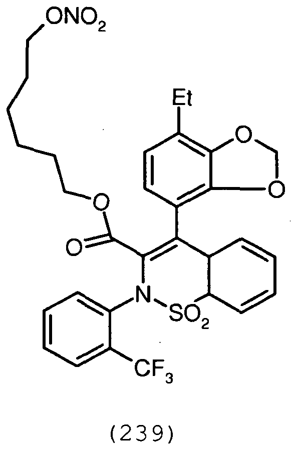

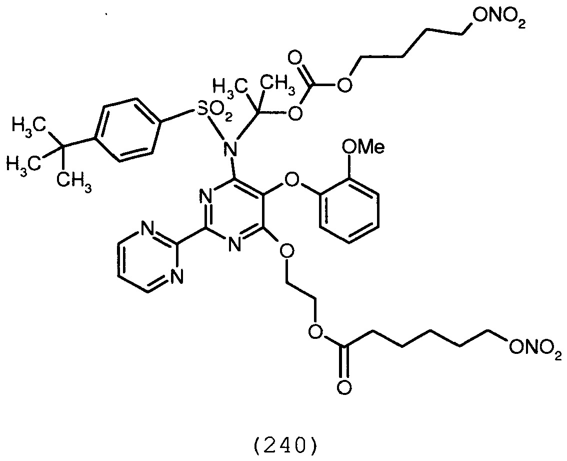

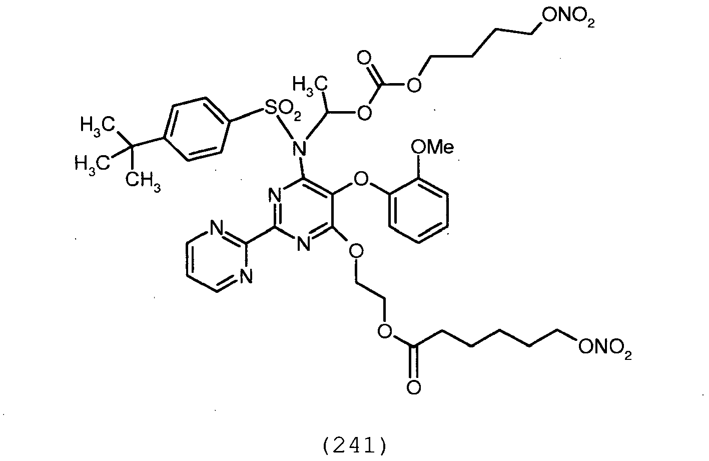

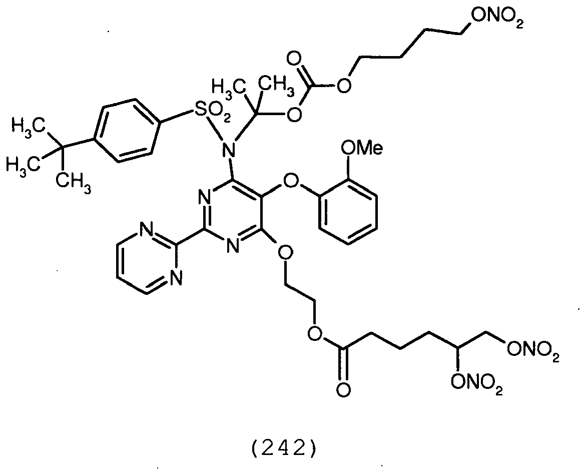

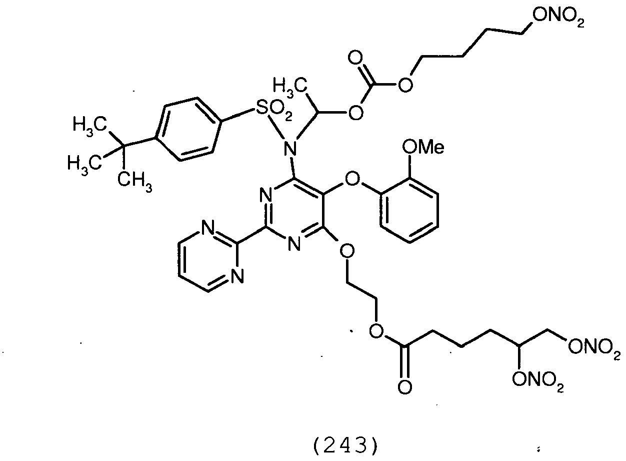

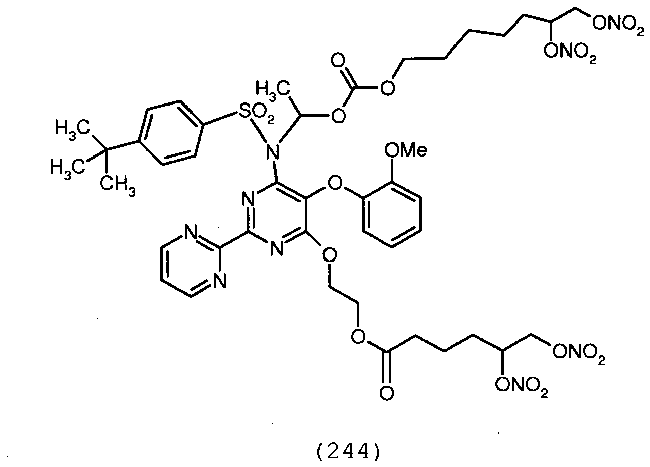

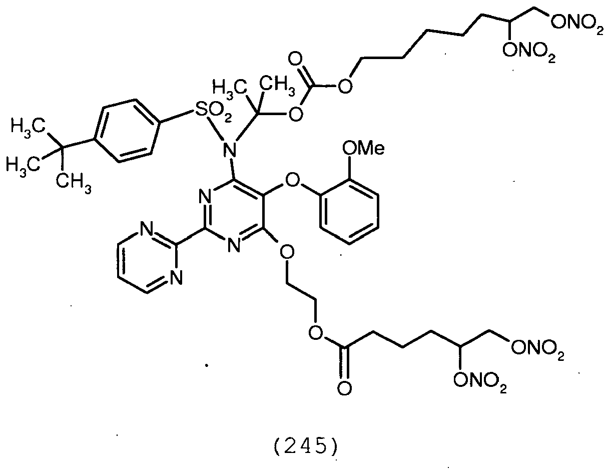

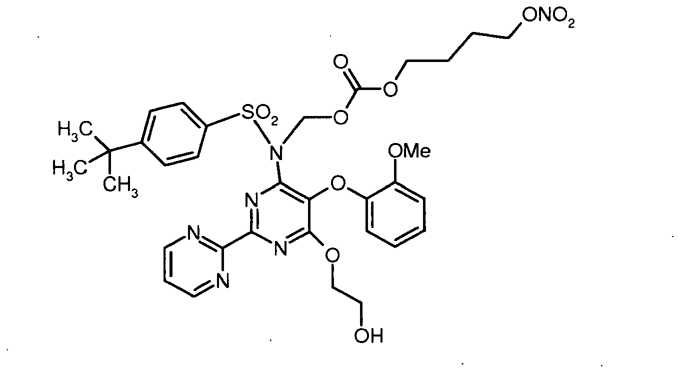

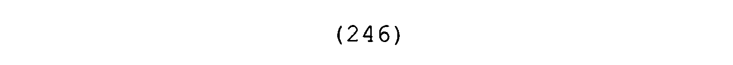

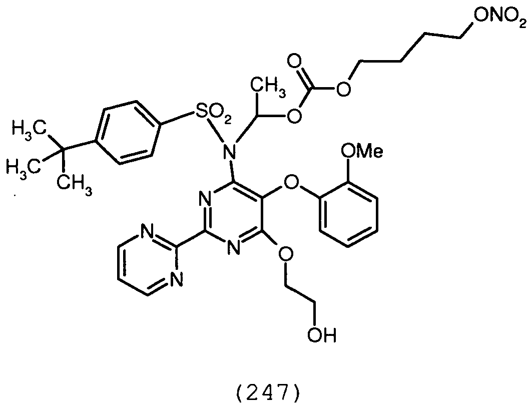

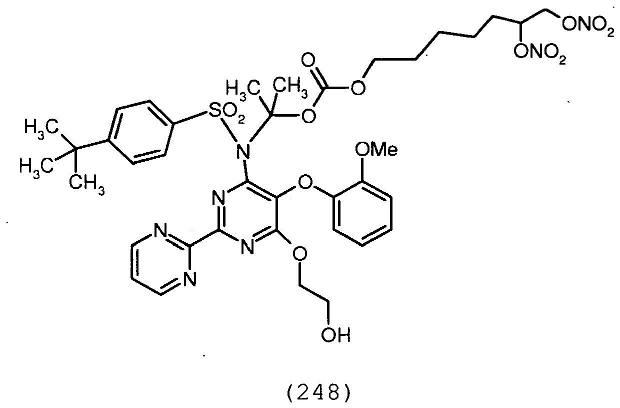

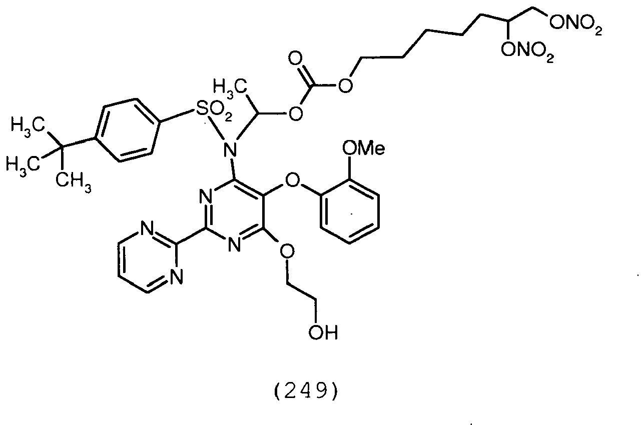

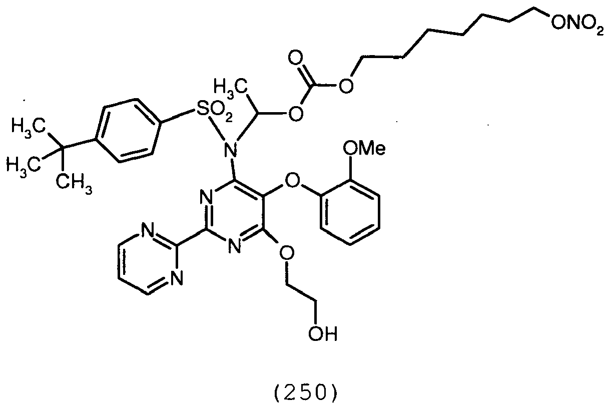

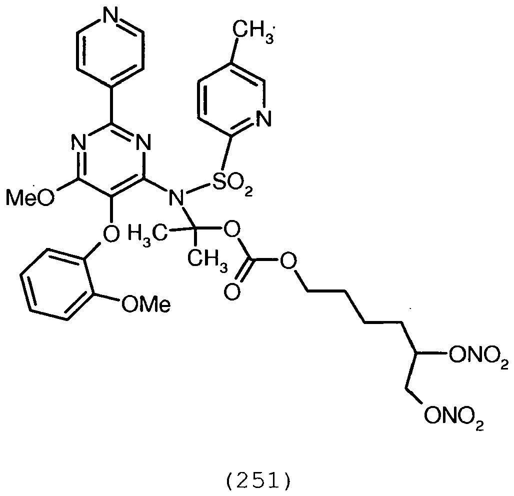

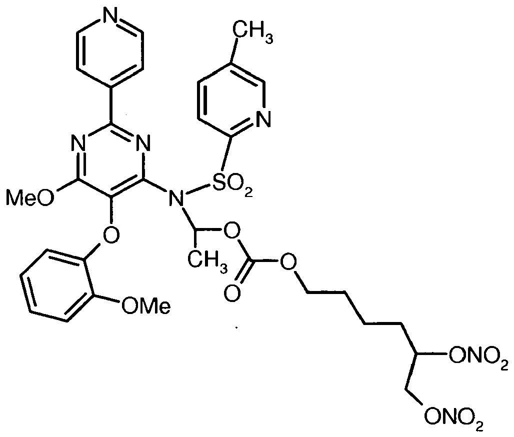

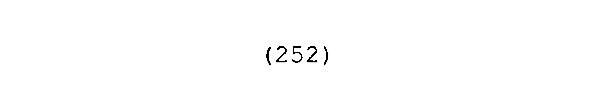

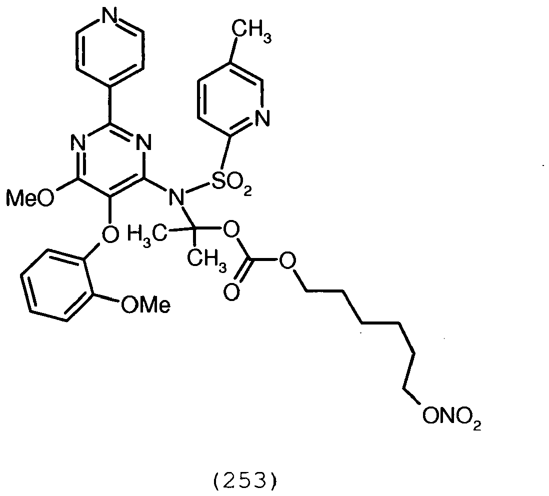

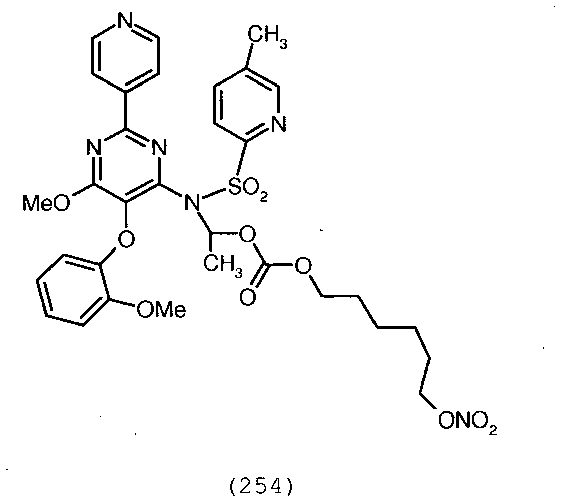

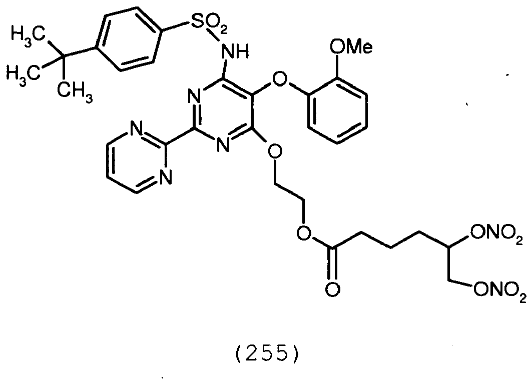

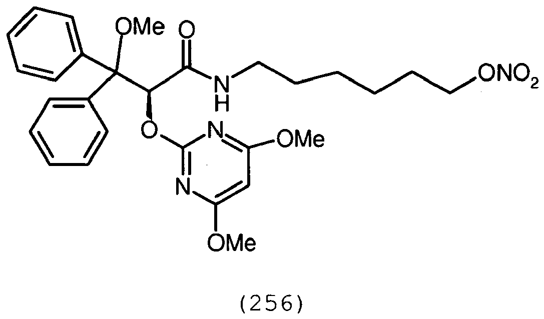

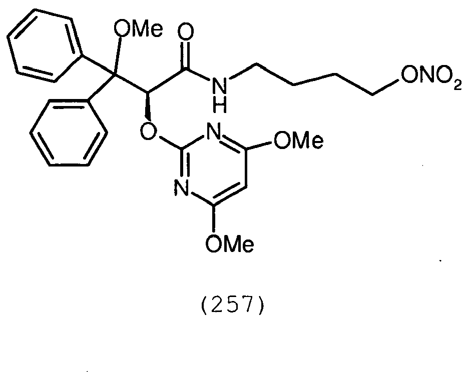

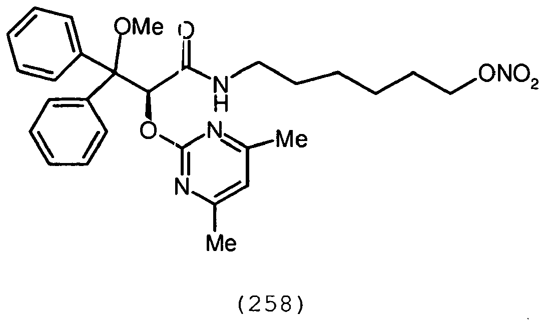

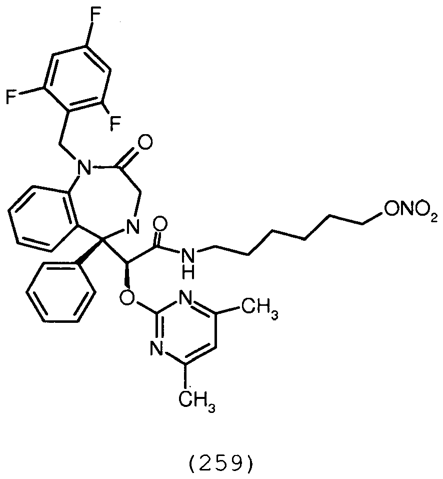

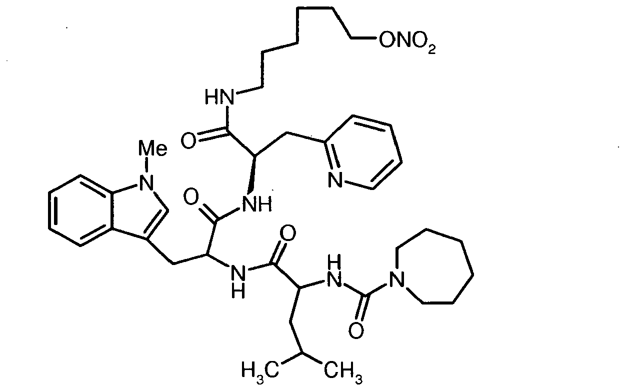

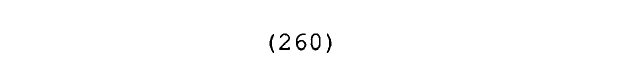

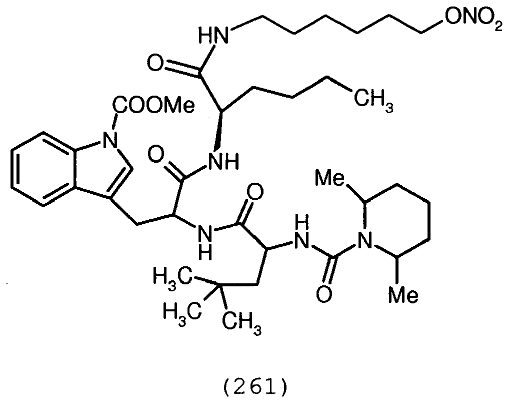

As mentioned above, object of the present invention are also pharmaceutical compositions containing at least a compound of the present invention of formula (I) together with non toxic adiuvants and/or carriers usually employed in the pharmaceutical field.

The daily dose of active ingredient that should be administered can be a single dose or it can be an effective amount divided into several smaller doses that are to be administered throughout the day. Usually, total daily dose may be in amounts preferably from 50 to 500 mg. The dosage regimen and administration frequency for treating the mentioned diseases with the compound of the invention and/or with the pharmaceutical compositions of the present invention will be selected in accordance with a variety of factors, including for example age, body weight, sex and medical condition of the patient as well as severity of the disease, route of administration, pharmacological considerations and eventual concomitant therapy with other drugs. In some instances, dosage levels below or above the aforesaid range and/or more frequent may be adequate, and this logically will be within the judgment of the physician and will depend on the disease state.

The compounds of the invention may be administered orally, parenterally, rectally or topically, by inhalation or aerosol, in formulations eventually containing conventional non-toxic pharmaceutically acceptable carriers, adjuvants and vehicles as desired. Topical administration may also involve the use of transdermal administration such as transdermal patches or iontophoresis devices. The term "parenteral" as used herein, includes subcutaneous injections, intravenous, intramuscular, intrasternal injection or infusion techniques.

Injectable preparations, for example sterile injectable aqueous or oleaginous suspensions may be formulated according to known art using suitable dispersing or wetting agents and suspending agents. The sterile injectable preparation may also be a sterile injectable solution or suspension in a non-toxic parenterally acceptable diluent or solvent. Among the acceptable vehicles and solvents are water, Ringer's solution and isotonic sodium chloride. In addition, sterile, fixed oils are conventionally employed as a solvent or suspending medium. For this purpose any bland fixed oil may be employed including synthetic mono or diglycerides, in addition fatty acids such as oleic acid find use in the preparation of injectables.

Suppositories for rectal administration of the drug can be prepared by mixing the active ingredient with a suitable non-irritating excipient, such as cocoa butter and polyethylene glycols.

Solid dosage forms for oral administration may include capsules, tablets, pills, powders, granules and gels. In such solid dosage forms, the active compound may be admixed with at least one inert diluent such as sucrose, lactose or starch. Such dosage forms may also comprise, as in normal practice, additional substances other than inert diluents, e.g. lubricating agents such as magnesium stearate. In the case of capsules, tablets and pills, the dosage forms may also comprise buffering agents. Tablets and pills can additionally be prepared with enteric coatings.

Liquid dosage forms for oral administration may include pharmaceutically acceptable emulsions, solutions, suspensions, syrups and elixirs containing inert diluents commonly used in the art, such as water. Such compositions may also comprise adjuvants, such as wetting agents, emulsifying and suspending agents, and sweetening, flavouring and the like.
The compounds of the present invention can be synthesized as follows.

### Synthesis procedure

**1.** The compounds of general formula (I) or pharmaceutically acceptable salts thereof: 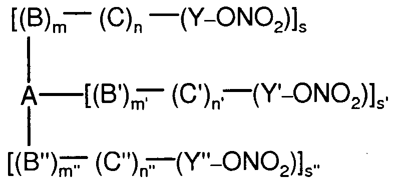 wherein:
   s is equal to 1;
   s' is equal to 0 or 1;
   s" is equal to 0;
   m and m' are equal to 1;
   n and n' are equal to 0;
   B and B' have the same meaning and they are -CO-;
   Y and Y' have the same meaning and they are as above defined;
   A is selected from (Ia) - (Ig) wherein N₁ is -O- and N₂ is - NH-;
   can be obtained by a process comprising:
      **1a.** reacting a compound of formula 1A with a compound of formula (IIa) : 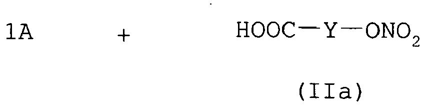 using a ratio 1A/(IIa) 1:1 or 1:2 if more than one N₁ is present; 1A is equal to A with A selected from (Ia)-(Ig) wherein N₁ is -OH and N₂ is -NH-; in presence of a condensing agent like dicyclohexylcarbodiimide (DCC) or N,N'-carbonyldiimidazol (CDI) or other known condensing reagents such as HATU in solvent such as DMF, THF, chloroform at a temperature in the range from -5°C to 50°C in the presence or not of a base as for example DMAP;
         The nitric acid ester compounds of formula (IIa) can be obtained from the corresponding alcohols of formula HOOC-Y-OH (IIb), that are commercially available, by reaction with nitric acid and acetic anhydride in a temperature range from -50°C to 0°C or reacting the corresponding halogen derivatives of formula HOOC-Y-Hal (IIc) wherein Hal is an halogen atom preferably C1, Br, I, that are commercially available, with AgNO₃ as described in WO 2006/008196. Compound 1A of formula (Ia) wherein N₁ is -OH and N₂ is - NH- is a known compound named Bosentan and can be prepared as described in US 5292740.
         Compound 1A of formula (Ib) wherein N₁ is -OH and N₂ is - NH- is a known compound named Ro 48-5033 and can be prepared by radical benzylic oxidation of bosentan with methods known in the literature.
         Compound 1A of formula (Ic) wherein N₁ is -OH and N₂ is - NH- is a known compound named Ro 46-2005 and can be prepared as described in EP 633259.
         Compound 1A of formula (Id) wherein N₁ is -OH and N₂ is - NH- is a known compound named Tezosentan and can be prepared as described in WO 96/19459. Compound 1A of formula (Ie) wherein N₁ is -OH and N₂ is - NH- is a known compound named Clazosentan and can be prepared as described in WO 96/19459.
         Compound 1A of formula (If) wherein N₁ is -OH and N₂ is - NH- is a known compound named Ro 46-8443 and can be prepared as described in EP 633259.
         Compound 1A of formula (Ig) wherein N₁ is -OH and N₂ is - NH- is a known compound named TBC 2576 and can be prepared as described in J. Med. Chem. 1999, 42, 4485-4499.
      **1b.** Reacting a compound of formula 1A as above defined in **1a.** with a compound of formula (IId) 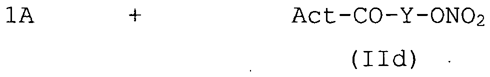 wherein Y is as above defined; Act is an Halogen atom or a carboxylic acid activating group used in peptide chemistry as: 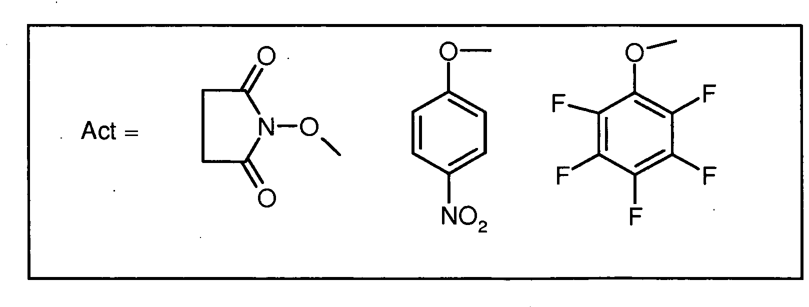 The reaction is generally carried out in presence of a inorganic or organic base in an aprotic polar/non-polar solvent such as DMF, THF or CH₂Cl₂ at temperatures range between 0°-65°C or in a double phase system H₂O/Et₂O at temperatures range between 20°-40°C; or in the presence of DMAP and a Lewis acid such as Sc(OTf)₃ or Bi(OTf)₃ in solvents such as DMF, CH₂Cl₂ using a ratio 1A/(IId) 1:1 or 1:2 if more than one N₁ is present.
         The compounds of formula (IId) can be obtained as described in WO 2006/008196.
      **1c.** Reacting a compound of formula (IIIa) 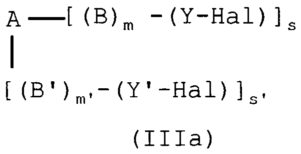 wherein Hal is an halogen atom, A, B, B', Y, Y', m , m', s and s' are as above defined in **1.,** with AgNO₃ as above described. Compounds (IIIa) can be obtained by reacting compound 1A with compounds (IIc) with a condensing reagent such as DCC or CDI as above described using a ratio 1A/(IIc) 1:1 or 1:2 if more than one N₁ is present.
      **1d.** Reacting a compound of formula (IVa): 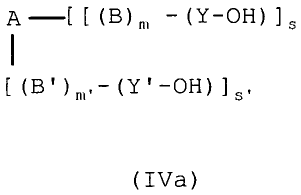 wherein A, B, B', Y, Y', m , m', s and s' are as above defined in **1.,** with triflic anhydride/ tetraalkylammonium nitrate salt in an aprotic polar/non-polar solvent such as DMF, THF or CH₂Cl₂ at temperatures range between -60° to 65°C. Compounds (IVa) can be obtained by reacting compound 1A with compounds (IIb) with a condensing reagent as above described using a ratio 1A/(IIb) 1:1 or 1:2 if more than one N₁ is present.
**2.** The compounds of general formula (I) or pharmaceutically acceptable salts thereof: 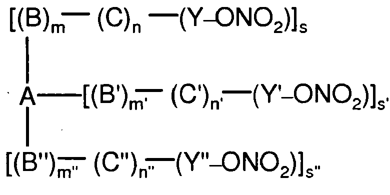 wherein:
   s is equal to 1;
   s' is equal to 0 or 1;
   s" is equal to 0;
   m and m' are equal to 1;
   n and n' are equal to 0;
   B and B' have the same meaning and they are -C(O)O-;
   Y and Y' have the same meaning and they are as above defined;
   A is selected from (Ia)-(Ig) wherein N₁ is -O- and N₂ is - NH-;
   can be obtained by a process comprising:
      **2a.** reacting a compound of formula 1A with a compound of formula (Va): 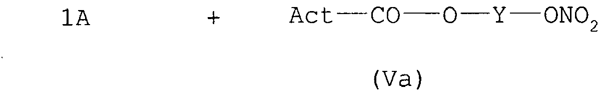 wherein 1A, Act, Y are as above described. The reaction is generally carried out in presence of a inorganic or organic base in an aprotic polar/non-polar solvent such as DMF, THF or CH₂Cl₂ at temperatures range between 0°-65°C or in a double phase system H₂O/Et₂O at temperatures range between 20°-40°C; or in the presence of DMAP and a Lewis acid such as Sc(OTf)₃ or Bi(OTf)₃ in solvents such as DMF, CH₂Cl₂, using a ratio 1A/(Va) 1:1 or 1:2 if more than one N₁ is present.
         The synthesis of compounds (Va) has been described in WO 2006/008196.
      **2b.** Reacting a compound of formula (IIIb) 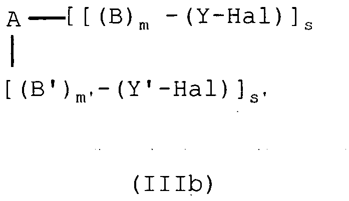 wherein Hal is an halogen atom, A, B, B', Y, Y', m , m', s and s' are as above defined in **2.,** with AgNO₃ as above described.
         The compounds of formula (IIIb) can be obtained by reacting compound 1A with compounds Act-CO-O-Y-Hal (VIa) wherein Act, Y and Hal are as above defined, using a ratio 1A/(VIa) 1:1 or 1:2 if more than one N₁ is present. The reaction is generally carried out in presence of an inorganic or organic base in an aprotic polar/non-polar solvent such as DMF, THF or CH₂Cl₂ at temperatures range between 0°-65°C as above described.
         Compound (VIa) are commercially available or can be synthesized as described in WO 2006/008196.
**3.** The compounds of general formula (I) or pharmaceutically acceptable salts thereof: 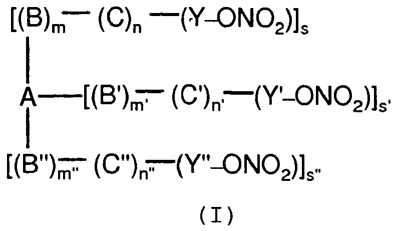 wherein:
   s is equal to 1;
   s' is equal to 0 or 1;
   s" is equal to 0;
   m and m' are equal to 0;
   n and n' are equal to 1;
   Y and Y' have the same meaning and they are as above defined;
   C and C' are equal to: 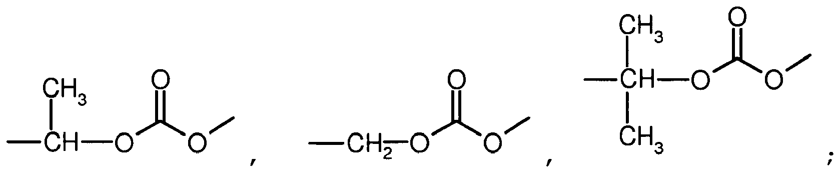
   A is selected from (Ia) - (Ig) or (Ih) - (Im), can be obtained by a process comprising:
      **3a.** W₁ is -CH₂-, -CH(CH₃) - or -C(CH₃)₂-.
         reacting a compound of formula 1A or 2A, wherein 1A is as above described and 2A is equal to A with A selected from (Ih)-(Im) wherein N₂ is -NH-, with compounds of formula (VIIa)

         Hal-W₁-OC(O)O-Y-ONO₂ (VIIa)

         wherein Y is as above described, Hal is an halogen atom and W₁ is -CH₂-, -CH(CH₃)- or -C(CH₃)₂-, using a ratio 1A/(VIIa) or 2A/(VIIa) 1:1 or 1:2 if more than one N₂ is present; in the presence of an organic or inorganic base such as TEA, K₂CO₃ or Ag₂O or HgO, in an aprotic polar/non-polar solvent such as DMF, AcOH, THF or CH₂Cl₂ at temperatures range between 0° to 65°C or in a double phase system H₂O/Et₂O at temperatures range between 20° to 40°C.
         The compounds of formula (VIIa) are obtained:
         **a) W₁ is equal to -CH₂- or -CH(CH₃);**
            by reacting the commercially available haloalkylhalocarbonate of formula (VIIb)

            Hal-W₁-OC(O)Hal (VIIb)

            wherein Hal is as above defined and W₁ is as above defined in a) with a compound of formula (VIIc)

            HO-Y-ONO₂ (VIIc)

            wherein Y is as above defined, in the presence of a inorganic or organic base in an aprotic polar or in an aprotic non-polar solvent such as DMF, THF or CH₂Cl₂ at temperatures range between 0° to 65°C.
         **b) W₁ is equal to -C(CH3)₂-;**
            by first reacting the commercially available compound of formula (VIIba)

            CH₂=C(CH₃)OCOCl (VIIba)

            wherein W₁ is as above defined in b) with compound (VIIc) using the same conditions described in a), to give compound (VIIbc)

            CH₂=C(CH₃)OCO-O-Y-ONO₂ (VIIbc)

            From compound (VIIbc) the final compound (VIIa) wherein Hal is Cl and W₁ is -C(CH₃)2- is obtained by bubbling gaseuos HCl in a solution of (VIIbc) in CH₂Cl₂.
         The compounds of formula (VIIc) are obtained by reacting compounds of formula HO-Y-Hal (VIId) wherein Y and Hal are as above defined, with AgNO₃ in a suitable organic solvent such as acetonitrile or tetrahydrofuran (THF) under nitrogen in the dark at temperatures range between 20°-80°C; alternatively the reaction with AgNO₃ can be performed under microwave irradiation in solvents such acetonitrile or THF at temperatures in the range between about 100-180°C for time range about 1-60 min.
         The compounds of formula (VIId) are commercially available or can be obtained by method well known in the literature. Alternatively compounds (VII_{A}) wherein W₁ is -CH₂- or - CH(CH₃)- can be prepared reacting compound (VIIf):

         Hal-W₁-OC(O)O-Y-OH (VIIf)

         with tetraalkylammonium nitrate and triflic anhydride as previously described. Compounds (VIIf) are prepared from compounds Hal-W₁-OC(O)Hal (VIIb) wherein W₁ is -CH₂- or - CH(CH3₎- by reacting with compounds HO-Y-OH (VIIe) in the presence of a inorganic or organic base in an aprotic polar or in an aprotic non-polar solvent such as DMF, THF or CH₂Cl₂ at temperatures range between 0° to 65°C.
         Compound 2A of formula (Ih) wherein N₂ is -NH- is a known compound named Sitaxsentan and can be prepared as described in J. Med. Chem. 1997, 40, 1690.
         Compound 2A of formula (Ii) wherein N₂ is -NH- is a known compound named BMS 193884 and can be prepared as described in EP 702012.
         Compound 2A of formula (Ij) wherein N₂ is -NH- is a known compound and can be prepared as described in J. Med. Chem. 2000, 43, 3111.
         Compound 2A of formula (Ik) wherein N₂ is -NH- is a known compound named Edonentan and can be prepared as described WO 98/33780.
         Compound 2A of formula (Il) wherein N₂ is -NH- is a known compound named Nebentan and can be prepared as described in Bioorg. Med. Chem. 2001, 9, 2955.
         Compound 2A of formula (Im) wherein N₂ is -NH- is a known compound named Avosentan or SPP301 and can be prepared as described WO 00/52007.
   **3a'. W₁ is equal to -C(CH₃)₂-**
      Reacting a compound of formula (VIIbd) with compound (VIIc) 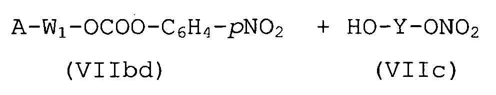 Wherein Y and W₁ are as above defined, A is selected from (Ia)-(Ig) or (Ih)-(Im), in the presence of equimolar amount of DMAP or DMAP and a catalytic amount of Lewis acid such as Sc(OTf)₃ or Bi(OTf)₃ in solvents such as DMF, CH₂Cl₂.
      Compound (VIIbd) are obtained by reacting compounds 1A or 2A already defined with compound (VIIbe)

      Cl-W₁-OCOOC₆H₄-*p*NO₂ (VIIbe)

      In CH₂Cl₂, AcOH or THF with Ag₂O or HgO as bases using a ratio 1A/(VIIbe) or 2A/(VIIbe) 1:1 or 1:2 if more than one N₂ is present. Compound (VIIbe) was prepared as described in Alexander, Jose. (Merck and Co., Inc., USA). PCT Int. Appl. (1996), WO 9618605 A1 19960620 Application: WO 95-US16308 19951208. Priority: US 94-354981 19941213.
   **3b.** When A is selected from (Ih)-(Im) reacting a compound of formula (IVb)

      A-W₁-OC(O)O-Y-OH (IVb)

      wherein W₁ is -CH₂- or -CH(CH₃)- with tetraalkylammonim nitrate as above described.
      Compounds (IVb) can be prepared by reacting 2A, wherein 2A is as above described with compounds of formula Hal-W₁-OC(O)O-Y-OH (VIIf), prepared as above defined, in the presence of a inorganic or organic base in an aprotic polar/non-polar solvent such as DMF, THF or CH₂Cl₂ at temperatures range between 0° to 65°C or in a double phase system H₂O/Et₂O at temperatures range between 20° to 40°C.
**4.** The compound of general formula (I) or pharmaceutically acceptable salts thereof: 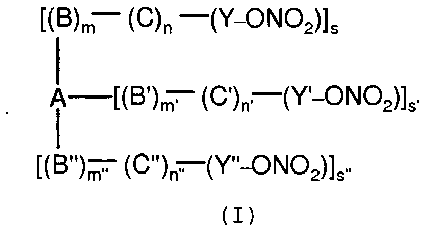 wherein:
   m, m', n and n' are equal to 0;
   s is equal to 1;
   s' is equal to O or 1;
   s" is equal to 0;
   Y and Y' have the same meaning and they are as above defined;
   A is selected from (In)-(Iu) wherein N₃ is -C(O)O- can be obtained by a process comprising:
      **4a.** reacting a compound of formula 3A wherein 3A is equal to A with A selected fron (In)-(Iu) wherein N₃ is equal to -COOH with compound of formula HO-Y-ONO₂ (VIIc), in the presence of a condensing agent like dicyclohexylcarbodiimide (DCC) or N,N'-carbonyldiimidazol (CDI) or other known condensing reagents such as HATU in solvent such as DMF, THF, chloroform at a temperature in the range from -5°C to 50°C in the presence or not of a base as for example DMAP, using a ratio 3A/(VIIc) 1:1 or 1:2 if more than one N₃ is present.
      Compound 3A of formula (In) wherein N₃ is -COOH is a known compound named Darusentan and can be prepared as described in J. Med. Chem. 1999, 42, 3026.
      Compound 3A of formula (Io) wherein N₃ is -COOH is a known compound named Ambrisentan and can be prepared as described in J. Med. Chem. 1996, 39, 2123.
      Compound 3A of formula (Ip) wherein N₃ is -COOH is a known compound and can be prepared as described in J. Med. Chem. 2004, 47, 2776.
      Compound 3A of formula (Iq) wherein N₃ is -COOH is a known compound named Atrasentan and can be prepared as described in J. Med. Chem. 1996, 39, 1039.
      Compound 3A of formula (Ir) wherein N₃ is -COOH is a known compound named FR 139317 and can be prepared as described in EP 457195.
      Compound 3A of formula (Is) wherein N₃ is -COOH is a known compound named BQ 788 and can be prepared as described in European Journal of Medicinal Chemistry 1995, 30 (Suppl., Proceedings of the 13th International Symposium on Medicinal Chemistry, 1994), 371s-83s.
      Compound 3A of formula (It) wherein N₃ are -COOH is a known compound named SB 247083 and can be prepared as described in WO 97/04772.
      Compound 3A of formula (Iu) wherein N₃ is -COOH is a known compound named Fandosentan and can be prepared as described in WO 99/12916.
   **4b.** Reacting a compound of formula (IIIc) 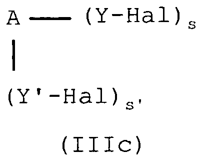
      wherein Y, Y', s, s' and Hal are as above defined;
      A is selected from (In)-(Iu) wherein N₃ is -C(O)O-,
      with AgNO₃ as above described.
      The compounds of formula (IIIc) can be obtained by reacting compound 3A as above defined with compounds HO-Y-Hal (VIId) using conditions previously described in **4a.**
   **4c.** Reacting a compound of formula (IVc) 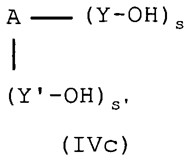
      Y, Y', s and s' are as above defined;
      A is selected from (In)-(Iu) wherein N₃ is -C(O)O- with triflic anhydride and tetraalkylammonium nitrate as above described in **1d.**
      The compounds of formula (IVc) can be obtained by reacting compound 3A as above defined with compounds HO-Y-OH (VIIe) using conditions previously described in **4a.**
**5.** The compound of general formula (I) or pharmaceutically acceptable salts thereof: 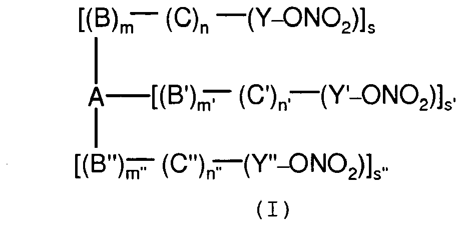
   m and m' are equal to 0;
   n and n' are equal to 1;
   s' is equal to 0 or 1;
   s" is equal to 0;
   Y and Y' have the same meaning and they are as above defined;
   C and C' are equal to: 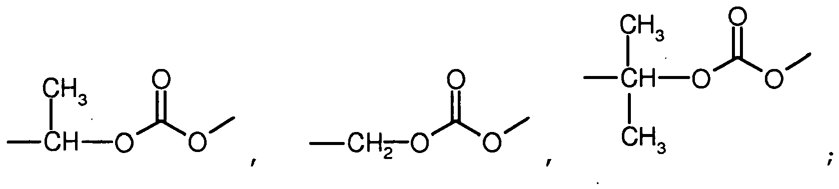
   A is selected from (In)-(Iu) wherein N₃ is -C(O)O- can be obtained by a process comprising:
      **5a.** reacting a compound of formula 3A as above described with compound of formula (VIIa):

         Hal-W₁-OC(O)O-Y-ONO₂ (VIIa)

         as above described in **3a.** using a ratio 3A/(VIIa) 1:1 or 1: 2 if more than one N₃ is present.
      **5a'.** Reacting a compound of formula (VIIbd') with compound (VIIc) as described in **3a'.** 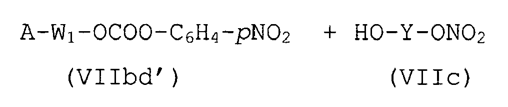 With A selected from (In)-(Iu). Compound (VIIbd') are obtained by reacting compounds 3A already defined with compound (VIIbe)

         3A + Cl-W₁-OCOOC₆H₄-*p*NO₂ (VIIbe)

         using a ratio 3A/(VIIbe) 1:1 or 1: 2 if more than one N₃ is present.
      **5b.** reacting a compound of formula(IVb) 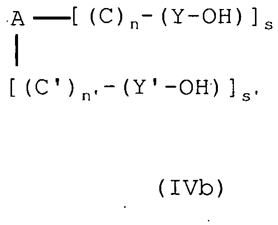 wherein A, C, C' , n, n', Y, Y', s and s' are as defined in **5.,** with tetraalkylammonium nitrate as above described.
         Compounds of formula (IVb) as above defined are obtained reacting compounds 3A with (VIIf) as above described in **3a.**
**6.** The compound of general formula (I) or pharmaceutically acceptable salts thereof: 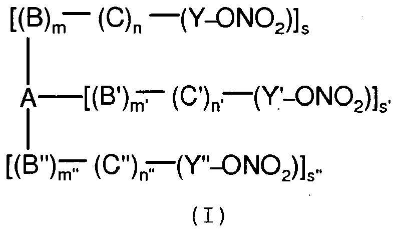
   s and s' are equal to 1;
   s" is equal to 0 or 1;
   m, m', m", n, n' and n" are 0 or 1;
   B, B' and B " have the same meaning and they are -CO-;
   C, C' and C " are equal to: 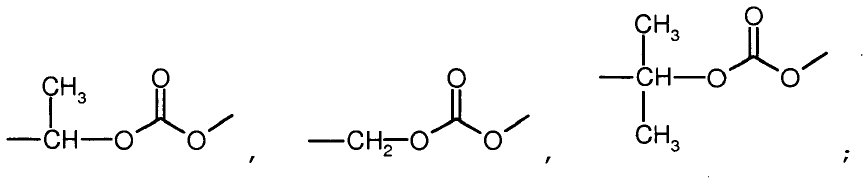 Y, Y' and Y " are as above defined; A is selected from (Ia)-(Ig) wherein N₁ is -O- and N₂ is -N-;
      can be obtained by a process comprising:
      **6a. W₁ is -CH₂-, -CH(CH₃)- or -C(CH₃)2-.**
         When A is selected among (Ia)-(Ic) and (If)-(Ig) reacting a compound of formula (VIIIa) 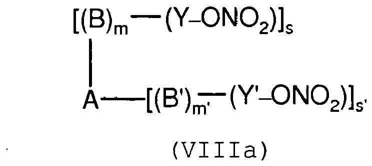
         wherein m and s are equal to 1;
         m' and s' are equal to 0 or 1;
         B and B' are -CO-; Y and Y' are as above defined; A is selected among (Ia)-(Ic) and (If)-(Ig) with N₁ equal to -O- and N₂ equal to -NH- (obtained as described **1a-1d.**) with compounds of formula Hal-W₁-OC(O)O-Y"-ONO₂ (VIIa") as above described in **3a.**
      **6b. W₁ is -CH₂-, -CH (CH₃) - .**
         Reacting a compound of formula (IXa) 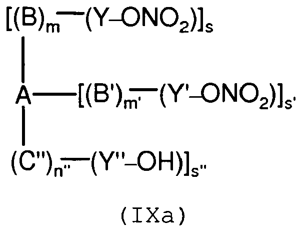 wherein m and s are equal to 1; m' and s' are equal to 0 or 1; n' ' and s" are equal to 1; B and B' are -CO-; C' ' , Y, Y' and Y" are as above defined in **6.;** A is selected among (Ia)-(Ic) and (If)-(Ig) with N₁ equal to -O- and N₂ equal to -N- with tetraalkylammonium nitrate as previously described.
         Compounds (IXa) are obtained by reacting compounds of formula (VIIIa) wherein B, B', m, m', s, s', Y and Y' are as previously described with compounds of formula (VIIf*''*)

         Hal-W₁-OC(O)O-Y*''*-OH (VIIf'')

         Wherein W₁ is equal to -CH₂- or -CH(CH₃)- as described in **3a.**
      **6c.W₁ is -CH₂-, -CH(CH₃)- or -C(CH₃)₂-.**
         When A is selected among (Id)-(Ie) reacting a compound of formula (VIIIb) 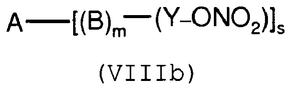
         wherein m and s are equal to 1;
         B is -CO-; Y is as above defined; A is selected among (Id)-(Ie) with N₁ equal to -O- and N₂ equal to -NH- (obtained as described **1a-1d.**) with 2 equivalents of compounds of formula Hal-W₁-OC(O)O-Y'-ONO₂ (VIIa') as above described in **3a.**
      **6d. W₁ is -CH₂-, -CH(CH₃)-.**
         Reacting a compound of formula (IXb) 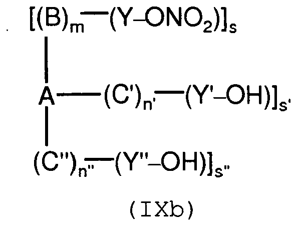 wherein m, n', n", s, s' and s" are equal to 1; B is -CO-; C', C", Y, Y' and Y" are as above defined, A is selected among (Id)-(Ie) with N₁ equal to -O- and N₂ equal to -N- with tetraalkylammonium nitrate as previously described.
         Compounds (IXb) are obtained by reacting compounds of formula (VIIIb) wherein B, m, s, are as previously described with 2 equivalents of compound of formula (VIIf')

         Hal-W₁-OC(O)O-Y'-OH (VIIf')

         Wherein W₁ is equal to -CH₂- or -CH(CH₃)- as described in **3a.**
      **6e. W₁ is -CH₂-, -CH (CH₃)- or -C (CH₃)₂-.**
         Reactinq a compound of formula (Xa) 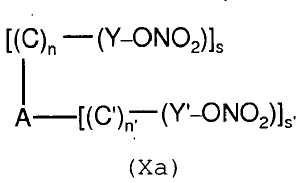 wherein n, n', s and s' are equal to 1; C, C', Y and Y' are as previously described; A is selected among (Id)-(Ie) with N₁ equal to -OH and N₂ equal to -N- (obtained as described in **3a.** or **3a',** with compounds of formula (IIa") or (IId")

         HOOC-Y"-ONO₂ (IIa")

         Act-CO-Y"-ONO₂ (IId")

         wherein Act and Y" are as above described in **1a,b.**
      **6f. W₁ is -CH₂-, -CH(CH₃)- or -C(CH₃)₂-.**
         Reacting a compound of formula (XIa) 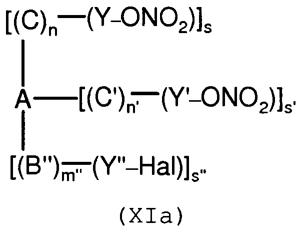 wherein n, n' , m" , s, s' and s" are equal to 1; B" is - CO-; C, C', Y, Y' and Y' ' are as defined in **6.;** A is selected among (Id)-(Ie) with silver nitrate as described in **1c.**
         Compounds (XIa) are obtained reacting compounds (Xa) 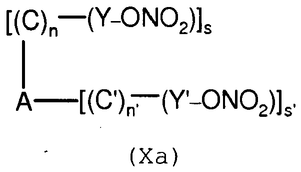 with compounds of formula (IIc")

         HOOC-Y"-Hal (IIc")

         wherein Y" and Hal are as previously described, using procedure described in **1c.**
      **6g. W₁ is -CH₂-, -CH(CH₃)- or -C(CH3)₂-.**
         Reacting a compound of formula (XIIa) 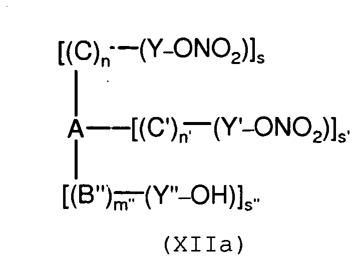 wherein n, n' , m", s, s' and s" are equal to 1; B" is - CO-; C, C', Y, Y' and Y" are as defined in **6.;** A is selected among (Id)-(Ie) with tetraalkylammonium nitrate as described in **1d.**
         Compounds (XIIa) are obtained from compounds (Xa) 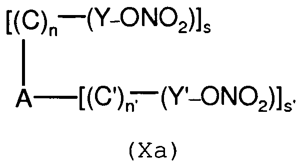 as previously defined with compounds of formula (IIb")

         HOOC-Y''-OH (IIb")

         wherein Y" is as previously described, using procedure described in **1d.**
      6h. **W1 is -CH₂-, -CH(CH₃)- or -C(CH₃)₂-.**
         Reacting a compound of formula (Xb)

         A-[(C)ₙ-(Y-ONO₂)]ₛ (Xb)

         wherein n and s are equal to 1; C and Y are as previously described; A is selected among (Ia)-(Ic) and (If) - (Ig) with N₁ equal to -OH and N₂ equal to -N- (obtained as described in **3a.** or **3a',** with compounds of formula (IIa') or (IId')

         HOOC-Y'-ONO₂ (IIa')

         Act-CO-Y'-ONO₂ (IId')

         wherein Act and Y' are as above described in **1a,b.;** using a ratio (Xb):(IIa') or (Xb):(IId') 1:1 or 1:2 if more than N₁ are present.
      **6i. W₁ is -CH₂-, -CH (CH₃) - or -C (CH₃)₂-.**
         Reacting a compound of formula (XIb) 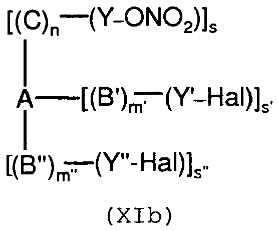 wherein n, m', s and s' are equal to 1; m" and s" can be 0 or 1; B' and B' ' are -CO-; C, Hal, Y, Y' and Y' ' are as defined in **6.;** A is selected among (Ia)-(Ic) and (If)-(Ig) with N₁ equal to -O- and N₂ equal to -N- with silver nitrate as described in **1c.**
         Compounds (XIb) are obtained reacting compounds (Xb)

         A-[(C)ₙ-(Y-ONO₂)]ₛ (Xb)

         with compounds of formula (IIc')

         HOOC-Y'-Hal (IIc')

         wherein Y' and Hal are as previously described, using a ratio (Xb):(IIc') 1:1 1 or 1: 2 if more than N₁ are present, following procedure described in **1c.**
      **6j. W₁ is -CH₂-, -CH(CH₃)- or -C(CH₃)2- .**
         Reacting a compound of formula (XIIb) 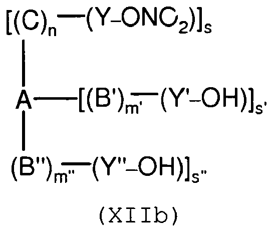 wherein m', n, s and s' are equal to 1; m" and s" are equal to 0 or 1; B' and B" are -CO-; C, Y, Y' and Y" are as defined in **6.;** A is selected among (Ia)-(Ic) and (If)-(Ig) with tetraalkylammonium nitrate as described in **1d.** Compounds (XIIb) are obtained reacting compounds (Xb), as previously defined, with compounds of formula (IIb')

         HOOC-Y'-OH (IIb')

         wherein Y' is as previously described, using procedure described in **1d.**
**7.** The compound of general formula (I) or pharmaceutically acceptable salts thereof: 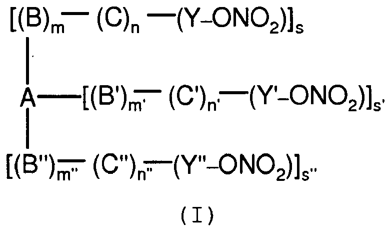
   s and s' are equal to 1;
   s" is equal to 0 or 1;
   m, m', m' ' , n, n', n' ' are 0 or 1;
   B, B' and B" have the same meaning and are -C(O)O-;
   C, C' and C" are equal to: 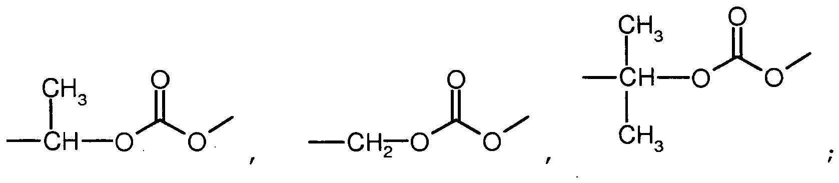
   Y, Y' and Y" are as above defined; A is selected from (Ia)-(Ig) wherein N₁ is -O- and N₂ is -N-;
   can be obtained by a process comprising:
      **7a. W₁ is -CH₂-, -CH(CH₃)- or -C(CH₃)₂-.**
         When A is selected among (Ia)-(Ic) and (If)-(Ig) reacting a compound of formula (VIIIa) 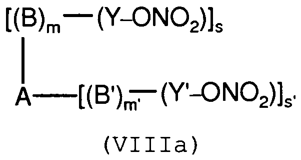
         wherein m and s are equal to 1;
         m' and s' are equal to 0 or 1;
         B and B' are -C(O)O-; Y and Y' are as above defined; A is selected among (Ia)-(Ic) and (If)-(Ig) with N₁ equal to -O- and N₂ equal to -NH-(obtained as described **2a, 2d.**) with compounds of formula Hal-W₁-OC(O)O-Y"-ONO₂ (VIIa") as above described in **3a.**
      **7b. W₁ is -CH₂-, -CH(CH₃)-.**
         Reacting a compound of formula (IXa) 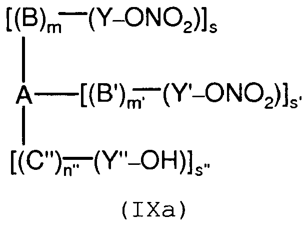 wherein m and s are equal to 1; m' and s' are equal to 0 or 1; n" and s" are equal to 1; B and B' are -C(O)O-; C", Y, Y' and Y" are as above defined, A is selected among (Ia)-(Ic) and (If)-(Ig) with N₁ equal to -O- and N₂ equal to -N- with tetraalkylammonium nitrate as previously described.
         Compounds (IXa) are obtained by reacting compounds of formula (VIIIa) wherein B, B' are -C(O)O-, m, m', s, s' Y and Y' are as previously described with compounds of formula (VIIf")

         Hal-W₁-OC(O)O-Y"-OH (VIIf")

         wherein W₁ is equal to -CH₂- or -CH(CH₃)- as described in **3a.**
      **7c. W₁ is -CH₂-, -CH(CH₃)- or -C(CH₃)₂-.**
         When A is selected among (Id)-(Ie) reacting a compound of formula (VIIIb)

         A-[(B)ₘ-(Y-ONO₂)]ₛ (VIIIb)

         wherein m and s are equal to 1;
         B is -C(O)O-; Y is as above defined; A is selected among (Id)-(Ie) with N₁ equal to -O- and N₂ equal to -NH-(obtained as described **2a, 2b.**) with 2 equivalents of compounds of formula Hal-W₁-OC(O)O-Y'-ONO₂ (VIIa') as above described in **3a.**
      **7d.W₁ is -CH₂-, -CH(CH₃)-.**
         Reacting a compound of formula (IXb) 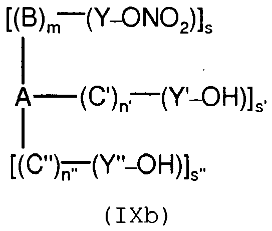 wherein m, n', n" s, s' and s" are equal to 1; B is - C(O)O-; C', C", Y, Y' and Y" are as above defined in **7.,** A is selected among (Id)-(Ie) with N₁ equal to -O- and N₂ equal to -N- with tetraalkylammonium nitrate as previously described.
         Compounds (IXb) are obtained by reacting compounds of formula (VIIIb) wherein B, m, s, are as previously described with 2 equivalents of compound of formula (VIIf')

         Hal-W₁-OC(O)O-Y'-OH (VIIf')

         Wherein W₁ is equal to -CH₂- or -CH(CH₃)- as described in **3a.**
      **7e. W₁ is -CH₂-, -CH (CH₃) - or -C (CH₃)₂-.**
         Reacting a compound of formula (Xa) 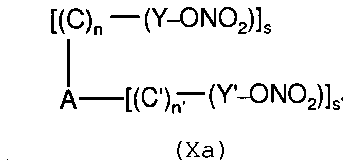 wherein n, n', s and s' are equal to 1; C, C', Y and Y' are as previously described; A is selected among (Id)-(Ie) with N₁ equal to -OH and N₂ equal to -N- (obtained as described in **3a),** with compounds of formula (Va")

         Act-CO-O-Y"-ONO₂ (Va")

         Wherein Act and Y" are as above described using the same procedure described in **2a.**
      **7f. W₁ is -CH₂-, -CH(CH₃) - or -C(CH₃)₂-.**
         Reacting a compound of formula (XIa) 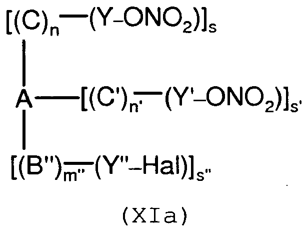 wherein n, n', m", s, s' and s" are equal to 1; B" is - C(O)O-; C, C', Hal, Y, Y' and Y" are as defined in 7.; A is selected among (Id)-(Ie) with silver nitrate as described in **2b.**
         Compounds (XIa) are obtained reacting compounds (Xa) 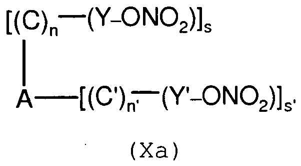 with compounds of formula or (VIa")

         Act-CO-O-Y"-Hal (VIa'')

         wherein Y" and Hal are as previously described, using procedure already described in **2a.**
      **7g. W₁ is -CH₂- , -CH (CH₃)- or -C (CH₃)₂-.**
         Reacting a compound of formula (Xb)

         A-[(C)ₙ-(Y-ONO₂)]ₛ (Xb)

         wherein n and s are equal to 1; C and Y are as previously described; A is selected among (Ia)-(Ic) and (If)-(Ig) with N₁ equal to -OH and N₂ equal to -N- (obtained as described in **3a),** with compounds of formula (Va")

         Act-CO-O-Y" -ONO₂ (Va")

         wherein Act and Y" are as above described in **2a.;** using a ratio (Xb):(Va") 1:1 or 1:2 if more than N₁ are present.
      **7h. W₁ is -CH₂-, -CH(CH₃)- or -C(CH₃)₂-.**
         Reacting a compound of formula (XIb) 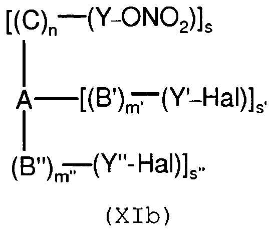 wherein n, m' s and s' are equal to 1; m" and s" can be 0 or 1; B' and B" are -C(O)O-; C, Hal, Y, Y' and Y" are as defined in **7.;** A is selected among (Ia)-(Ic) and (If)-(Ig) with N₁ equal to -O- and N₂ equal to -N- with silver nitrate as described in **2b.**
         Compounds (XIb) are obtained reacting compounds (Xb)

         A-[(C)ₙ-(Y-ONO₂)]ₛ (Xb)

         with compounds of formula or (VIa")

         Act-CO-O-Y"-Hal (VIa")

         wherein Y" and Hal are as previously described, using a ratio (Xb): (VIa") 1:1 or 1:2 if more than N₁ are present, following procedure already described in **2b.**
**8.** The compound of general formula (I) or pharmaceutically acceptable salts thereof: 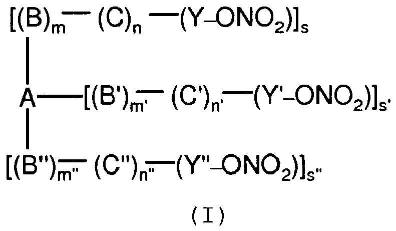 wherein:
   m, m', n and n' are equal to 0;
   s is equal to 1;
   s' is equal to 0 or 1;
   s" is equal to 0;
   Y and Y' have the same meaning and they are as above defined;
   A is selected from (In)-(Iu) wherein N₃ is -C(O)NH- can be obtained by a process comprising:
      **8a.** reacting a compound of formula 3A wherein 3A is equal to A with A selected fron (In)-(Iu) wherein N₃ is equal to -COOH with compound of formula NH₂-Y-ONO₂ (VIIg), in the presence of a condensing agents like dicyclohexylcarbodiimide (DCC) or N,N'-carbonyldiimidazol (CDI) or other known condensing reagents such as HATU in solvent such as DMF, THF, chloroform at a temperature in the range from -5°C to 50°C in the presence or not of a base as for example DMAP, using a ratio 3A/(VIIc) 1:1 or 1:2 if more than one N₃ is present.
         Compound (VIIg) can be prepared by acid hydrolysis of compounds (VIIh) to remove the BOC- protective group as known in the literature.

         (CH₃)₃COCO-NH-Y-ONO₂ (VIIh)

         Compounds (VIIh) are prepared from (VIIi)

         (CH₃)₃COCO-NH-Y-OH (VIIi)

         By reacting with triflic anhydride/tetraalkylammonium nitrates as already described in **1d.** Compounds (VIIi) are commercially available or can be easily prepared from commercially available (VIIk)

         NH₂-Y-OH (VIIk)

         and BOC anhydride by known procedures.
      **8b.** Reacting a compound of formula (IVc) 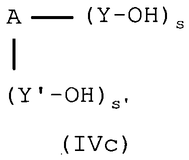
         Y, Y', s and s' are as above defined;
         A is selected from (In)-(Iu) wherein N₃ is -C(O)NH- with triflic anhydride and tetraalkylammonium nitrate as above described in **1d.**
         The compounds of formula (IVc) can be obtained by reacting compound 3A as above defined with compounds NH₂-Y-OH (VIIk) using conditions previously described in **8a.**

The following examples are to further illustrate the invention without limiting it.

### Example 1

### Synthesis of compound (2)

To a solution of Bosentan (0.3 g, 0.54 mmol) DMAP (0.066 g, 0.54 mmol) and TEA (0.1 ml, 0.54 mmol) in CH₂C1₂ (10 ml) cooled to 0°C, a solution of 4-(nitrooxy)butanoic acid pentafluorophenyl ester (0.173 g, 0.54 mmol) in CH₂Cl₂ (2 ml) was added and the mixture was reacted overnight.
Then the mixture was diluted with CH₂Cl₂ (100 ml), washed with water (150 ml) and brine (100 ml). The organic phase was then dried over MgSO₄ and the solvent was removed in vacuum. The residue was purified by flash chromatography (CH₂Cl₂/MeOH 95/5) yielding title compound **(2)** (0.21 g 57%) as an off white solid.
¹H-NMR (400 MHz, DMSO-*d*₆): δ 8.98 (2H, d), 7.85 (2H, d), 7.59 (1H, t), 7.27 (2H, d), 7.01 (1H, dd), 6.88 (1H, td), 6.71 (1H, td), 6.36 (1H, dd), 4.50-4.40 (4H, m), 4.22-4.15 (2H, m), 3.82 (3H,s), 2.22 (2H, t), 1.77 (2H, q), 1.23 (9H, s).

### Example 2

### Synthesis of compound (191)

Following the same procedure described in **Example 1** but using 6-(nitrooxy)hexanoic acid pentafluorophenyl ester (0.185 g, 0.54 mmol), the title compound **(191)** (0.3 g, 78%) was obtained as an off white solid.
¹H-NMR (400 MHz, CDCl₃): δ 8.98 (2H, d), 8.35 (1H, s), 7.52- 7.32 (2H, m), 7.18-7.04 (2H, m), 6.97 (1H, d), 6.82 (1H, t), 4.79-4.65 (2H, m), 4.38 (2H, t), 4.37-4.28 (2H, m), 3.94 (3H,s), 2.24 (2H, t), 1.75-1.50 (7H, m), 1.45-1.32 (2H, m), 1.29 (9H, s).

### Example 3

### Synthesis of compound (4)

To a solution of Bosentan (0.45 g, 0.82 mmol) DMAP (0.45 g, 3.68 mmol) and Sc(OTf)₃ (0.16 g, 0.328 mmol) in CH₂Cl₂ (10 ml) cooled to 0°C, a solution of 4-(nitrooxymethyl)benzoic acid pentafluorophenyl ester (0.3 g, 0.82 mmol) in CH₂Cl₂ (5 ml) was added and the mixture was reacted overnight. Then the mixture was diluted with CH₂Cl₂ (150 ml), washed with water (200 ml) and brine (200 ml). The organic phase was then dried over MgSO₄ and the solvent was removed in vacuum. The residue was purified by flash chromatography (CH₂Cl₂/MeOH 90/10) yielding title compound **(4)** (0.139 g 23%) as an off white solid.
¹H-NMR (400 MHz, CDCl₃): δ 9.00 (2H, d), 8. 80 (1H, s), 8.39 (2H, d) 8.01-7.87 (2H, m), 7.47- 7.39 (5H, m), 7.06 (1H, d), 6.97 (1H, t), 6.93-6.85 (1H, m), 6.60-6.54 (1H, m), 5.47 (2H, s), 4.93-4.81 (2H, m), 4.67-4.50 (2H, m), 3.90 (3H,s), 1.29 (9H, s).

### Example 4

### Synthesis of compound (11)

To a solution of Bosentan (0.67 g, 1.21 mmol) DMAP (0.25 g, 2.12 mmol) and Sc(OTf)₃ (0.104 g, 0.22 mmol) in CH₂Cl₂ (10 ml) cooled to 0°C, a solution of 4-(nitrooxy)butyl p-nitrophenyl carbonate (0.45 g, 1.5 mmol) in CH₂Cl₂ (10 ml) was added and the mixture was reacted overnight.
Then the mixture was diluted with CH₂Cl₂ (50 ml), washed with water (100 ml). The organic phase was then dried over MgSO₄ and the solvent was removed in vacuum. The residue was purified by flash chromatography (CH₂Cl₂/MeOH 9.5/0.5) yielding title compound **(11)** (0.136 g 15%) as an off white solid.
¹H-NMR (400 MHz, DMSO-*d*₆): δ 8.96 (2H, d), 7.93-7.77 (2H, m), 7.66-7.54 (1H, m), 7.27 (2H, d), 7.01 (1H, d), 6.89 (1H, t), 6.70 (1H, t), 6.55-6.48 (1H, m), 6.04 (1H, m), 4.62(4H, m), 4.30-4.20 (2H, m), 3.98 (2H, t), 3.87-3.78 (3H, m), 1.73-1.52 (4H, m), 1.23 (9H, s).

### Example 5

### Synthesis of compound (192)

Following the same procedure described in **Example 4** but using 6-(nitrooxy)hexyl p-nitrophenyl carbonate (0.492 g, 1.5 mmol), the title compound **(192)** (0.133 g, 15%) was obtained as an off white solid.
¹H-NMR (400 MHz, DMSO-*d*₆): δ 8.98 (2H, d), 7.84 (2H, d), 7.60 (1H, t), 7.27 (2H, d), 7.00 (1H, d), 6.89 (1H, t), 6.69 (1H, t), 6.35 (1H, d), 4.55-4.43 (4H, m), 4.23 (2H, m), 4.02-3.90 (2H, m), 3.88-3.79 (2H, m), 3.40-3.25 (6H, m), 1.71-1.57 (2H, m), 1.57-1.44 (2H, m), 1.23 (9H, s).

### Example 6

### Synthesis of compounds (247)

### A) Preparation of 1-chloroethyl 4-(nitrooxy)butyl carbonate

To a solution of 1,4-butanediol (5.0 ml, 56.2 mmol) and pyridine (5.9 ml, 73.1 mmol) in CH₂Cl₂ (60 ml) cooled to 0°C 1-chloethyl chlorocarbonate (6.7 ml, 61.9 mmol) was added and the mixture was reacted for 4 hrs at room temperature. Then the reaction was diluted with a pH 3 solution of citric acid (200 ml) and extracted with CH₂Cl₂ (2 X 150 ml), dried over MgSO₄ and the sovent was evaporated under vacuum. The residue was purified by flash chromatography (Cyclohexane/EtOAc 8/2) yielding **1-chloroethyl 4-hydroxybutyl carbonate** (3.28 g, 30 %) as a thin oil.
To a solution of 1-chloroethyl 4-hydroxybutyl carbonate (3.73 g, 17.0 mmol) tetraethylammonium nitrate (6.4 g, 33.0 mmol) 2,6-di-t-butyl-4-methylpyridine (7.5 g, 36.0 mmol) in CH₂Cl₂ (100 ml) cooled to -70°C, a solution of triflic anhydride (5.6 ml, 33.0 mmol) in CH₂Cl₂ (70 ml) was added and the mixture was stirred at -70°C, for 1 hrs then gradually warmed to room temperature and stirred overnight. Then the reaction was diluted with water (400 ml) and extracted with CH₂Cl₂ (2 X 150 ml). The organic phase was washed with brine (2 X 150 ml), dried over MgSO₄ and the solvent was evaporated under vacuum. The residue was purified by flash chromatography (Cyclohexane 100, then cyclohexane/EtOAc 95/5, then EtOAC 100) yielding 1-**chloroethyl 4-(nitooxy)butyl carbonate** (2.97 g, 74%) as an oil.

### B) Synthesis of (247)

To a solution of Bosentan (0.10 g, 0.18 mmol) and Cs₂CO₃ ( 0.09 mg, 0.27 mmol) in DMF ( 2 ml) a solution of 1-chloroethyl 4-(nitrooxy)butyl carbonate ( 0.11, 0.45mmol) in DMF (2 ml) was added and the mixture was stirred for 60h. The mixture was diluted with NaH₂PO₄ 5% (5 ml) and extracted with AcOEt (3 x 10 ml). The organic layer was washed with water (6 x 20 ml), dried over Na₂SO₄, filtered and evaporated under vacuum. The residue was purified by flash chromatography (CH₂Cl₂/ CH₃OH 95/5) yielding title compound **(247)** (0.04 g, 30%) as a yellow solid.
¹H-NMR (300 MHz, CDCl₃): 5 9.01 (2H, m), 8.04 (2H, d), 7.44 (3H, m), 7.15-6.86 (4H, m), 4.76 (2H, d), 4.58-4.30 (4H, m), 4.30-4.05 (2H, m), 3.98 (3H, s), 3.79-3.58 (1H, m), 1.94-1.59 (7H, m),1.29 (9H, s).

### Example 7

### Synthesis of compounds (246)

### A) Preparation of chloromethyl 4-(nitrooxy)butyl carbonate

To a solution of 1,4-butanediol (4.5 ml, 51.0 mmol) and pyridine (5.4 ml, 66.0 mmol) in CH₂Cl₂ (60 ml) cooled to 0°C chloromethyl chlorocarbonate (5.0 ml, 56.0 mmol) was added and the mixture was reacted for 4 hrs at room temperature. Then the reaction was diluted with a pH 3 solution of citric acid (200 ml) and extracted with CH₂Cl₂ (2 X 150 ml), dried over MgSO₄ and the solvent was evaporated under vacuum. The residue was purified by flash chromatography (Cyclohexane/EtOAc 8/2) yielding **chloromethyl 4-hydroxybutyl carbonate** (2.7 g, 29 %) as a thin oil.
To a solution of chloromethyl 4-hydroxybutyl carbonate (2.0 g, 10.0 mmol) tetraethylammonium nitrate (4.2 g, 20.0 mmol) 2,6-di-t-butyl-4-methylpyridine (4.9 g, 24.0 mmol) in CH₂Cl₂ (100 ml) cooled to -70°C, a solution of triflic anhydride (3.7 ml, 20.0 mmol) in CH₂Cl₂ (15 ml) was added and the mixture was stirred at -70°C, for 1. hrs then gradually warmed to room temperature and stirred overnight. Then the reaction was diluted with water (500 ml) and extracted with CH₂Cl₂ (2 X 150 ml). The organic phase was washed with brine (2 X 150 ml), dried over MgSO₄ and the solvent was evaporated under vacuum. The obtained waxy solid was suspended in cyclohexane/EtOAc 1:1 (200 ml). The precipitate was filtered off and the solution was concentrated at reduced pressure affording **chloromethyl 4-(nitrooxy)butyl carbonate** (2.0 g, 87%) as a yellow oil.

### B) Synthesis of (246)

To a solution of Bosentan (1.0 g, 1.81 mmol) and Cs₂CO₃ ( 1.5 g, 4.5mmol) in DMF ( 20 ml) a solution of chloromethyl 4-(nitrooxy)butyl carbonate (1.03 g, 4.5 mmol) in DMF (5 ml) was added and the mixture was stirred for 48h. The mixture was diluted with Na2HP04 5% (500 ml) and extracted with EtOAc (2 x 200 ml). The organic layer was dried over MgS04 and concentrated under vacuum. The crude material was purified twice over preparative MPLC using two 20 g cartridges and a linear gradient cyclohexane/EtOAc from 1:1 to 0:100 in 10 minutes (flow 15ml/min) affording compound titled **(246)** (0.52 g, 38%) as a yellow solid.
¹H-NMR (400 MHz, DMSO-d⁶) : δ 9.06-9.05 (2H, d), 8.28-8.26 (2H, d), 7.63 (1H, s), 7.53-7.51 (2H, d), 7.05-7.00 (2H, m), 6.78-6.68 (2H, dd), 4.94 (2H, s), 4.49-4.47 (2H, t), 4.40 (2H, bm), 4.06-4.04 (2H, t),3.74 (3H, s), 3.67 (2H,bm), 1.67-1.61 (4H, m), 1.23 (9H, s).

### Studies on vascular tone

The ability of Endothelin receptor antagonist nitroderivatives to induce vasorelaxation in comparison to native Endothelin receptor antagonists, was tested in vitro in isolated rabbit thoracic aorta preparations (Wanstall J.C. et al., Br. J. Pharmacol., 134:463-472, 2001). Male New Zealand rabbits were anaesthetized with thiopental-Na (50 mg/kg, iv), sacrificed by exsanguinations and then the thorax was opened and the aorta dissected. Aortic ring preparations (4 mm in length) were set up in physiological salt solution (PSS) at 37°C in small organ chambers (5 ml). The composition of PSS was (mM): NaCl 130, NaHCO₃ 14.9, KH₂PO₄ 1.2, MgSO₄ 1.2, HEPES 10, CaCl₂, ascorbic acid 170 and glucose 1.1 (95% O₂ /5% CO₂ ; pH 7.4). Each ring was mounted under 2 g passive tension. Isometric tension was recorded with a Grass transducer (Grass FT03) attached to a BIOPAC MP150 System. Preparations were allowed to equilibrate for 1h, and then contracted submaximally with noradrenaline (NA, 1 µM) and, when the contraction was stable, acetylcholine (ACh, 10 µM) was added. A relaxant response to ACh indicated the presence of a functional endothelium. Vessels that were unable to contract NA or showed no relaxation to Ach were discarded. When a stable precontraction was reached, a cumulative concentration-response curve to either of the vasorelaxant agents was obtained in the presence of a functional endothelium. Each arterial ring was exposed to only one combination of inhibitor and vasorelaxant. Moreover, the effect of the soluble guanylyl cyclase inhibitor ODQ (1-H-(1,2,4)-oxadiazol(4,3-a)quinoxalin-1-one) on vasorelaxation elicited by the compounds was examined preincubating the aortic rings with ODQ (10 µM) for 20 min.
Responses to relaxing agents are expressed as a percentage of residual contraction and plotted against concentration of test compound. EC₅₀ values (where EC₅₀ is the concentration producing 50% of the maximum relaxation to the test compound) were interpolated from these plots. During the experimental period, the plateau obtained with NA was stable without significant spontaneous loss of contraction in the aortic rings. Under these experimental conditions, the bosentan did not produce relaxation at any of the concentration tested, the curve being not different from that built up in the presence of vehicle alone.
As shown in Table 1, the compound of the example 1 of the invention were able to induce relaxation in a concentration-dependent manner. Furthermore, in experiments performed in the presence of ODQ (10 µM), the vasorelaxant responses to tested compounds were inhibited.

**Table 1**

| **Compound** | **EC₅₀ (µM) ± sem** |
|---|---|
| Bosentan | no effect |
| Compound of **EX.1** | 33.9 ± 2.5 |

### Study of antihypertensive activity of bosentan nitroderivative in vivo

Ten days prior to the beginning of the experiment, spontaneously hypertensive rats (SHR) are trained daily for the measurement of blood pressure by the tail-cuff method (Whitesall S.E et Al.; Am. J. Physiol. Heart Circ. Physiol 286: H2408-H2415, 2004) using model BP-2000 Blood Pressure Analysis System from U.Basile (Comerio, VA Italy).
Each animal is placed in individuals cages into a warming cupboard (37°C) for 15 minutes. Systolic blood pressure is evaluated with tail-cuff method before (baseline) and after (i.e. 1, 3, 6, 24 hours) treatment by oral administration of bosentan, bosentan nitroderivative or vehicle. Average of blood pressure values from individual rats are evaluated from 3/5 different consecutive measurements. The determination is considered valid only when 3 to 5 readings do not differ by more than 0.00667 mbar (5mm Hg).
The data are processed both as the absolute value or as a delta between the absolute value and its own baseline.
As shown in Table 2, differently from the parent compound bosentan, the nitroderivative (compound of Ex. 1) was able to induce a clear reduction in blood pressure.

| **Δ SBP (Systolic Blood Pressure) (mmHg)** | | | |
|---|---|---|---|
| **Compound** | **1 hr** | **3 hrs** | **6 hrs** |
| Bosentan | 0 | 6 | -1 |
| (100 mpk p.o.) | | | |
| Compound of **EX.1** | -8 | -18 | -6 |
| (equimolar dose p.o.) | | | |

## Claims

1. A compound of general formula (I) or a pharmaceutically acceptable salt or stereoisomer thereof: 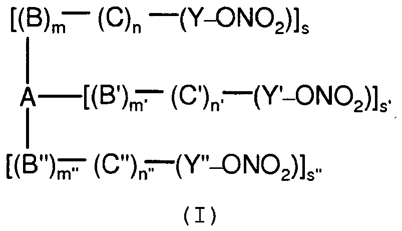 wherein:
m, m' and m" are equal to 0 or 1;
n, n' and n" are equal to 0 or 1;
s, s' and s" are equal to 0 or 1;
A is selected from the group consisting of: 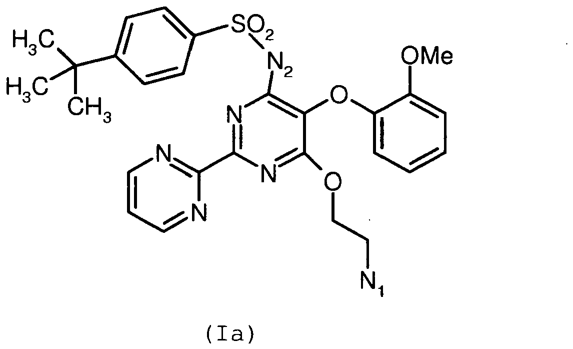 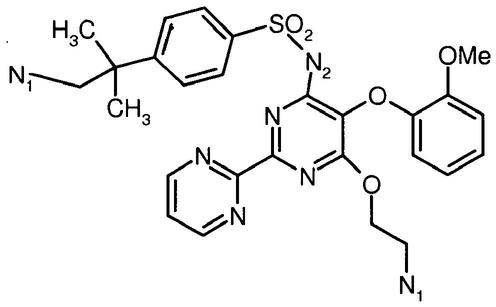 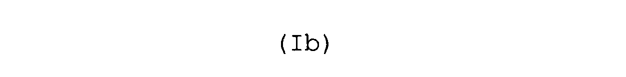 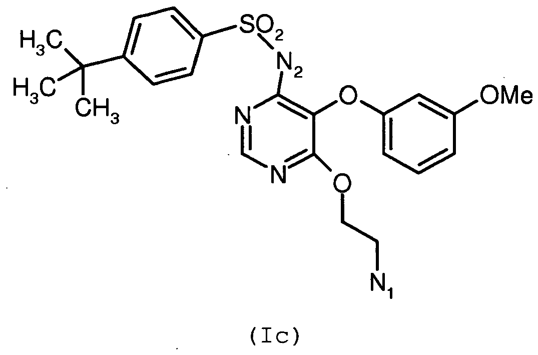 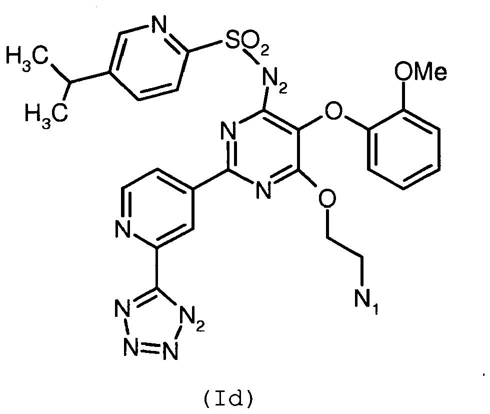 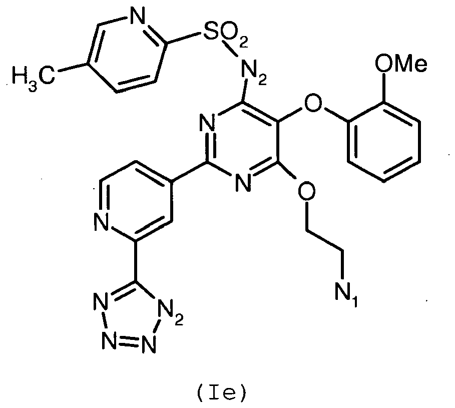 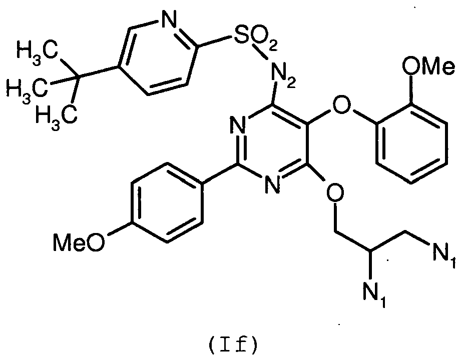 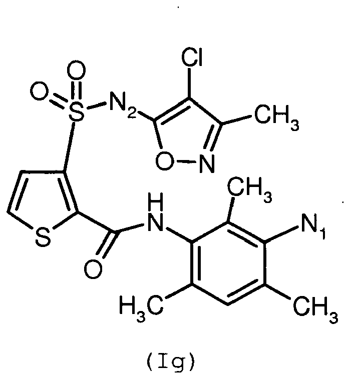 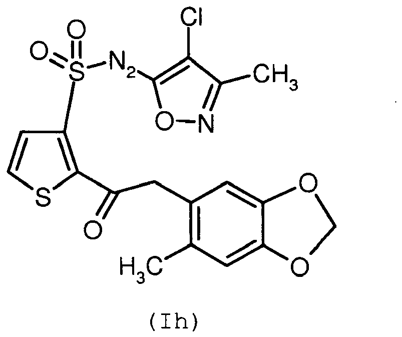 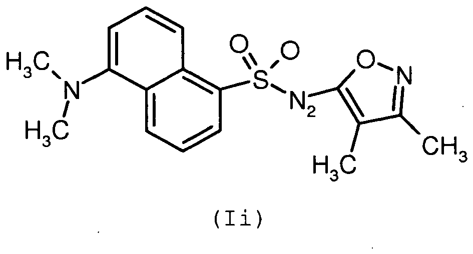 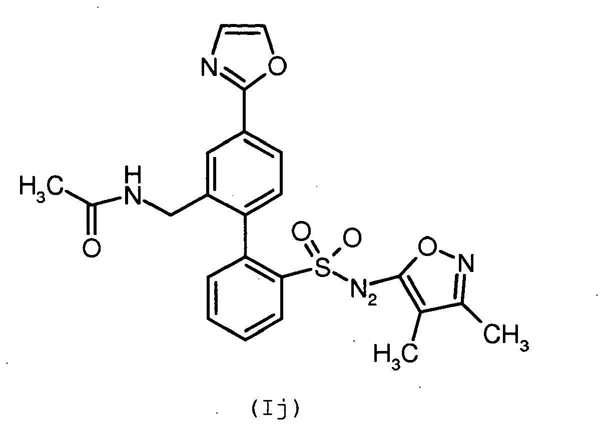 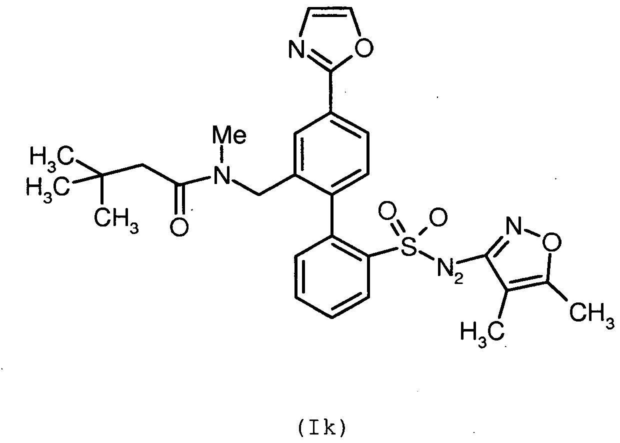 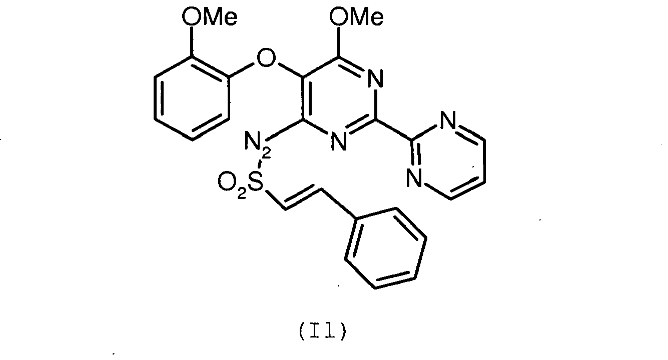 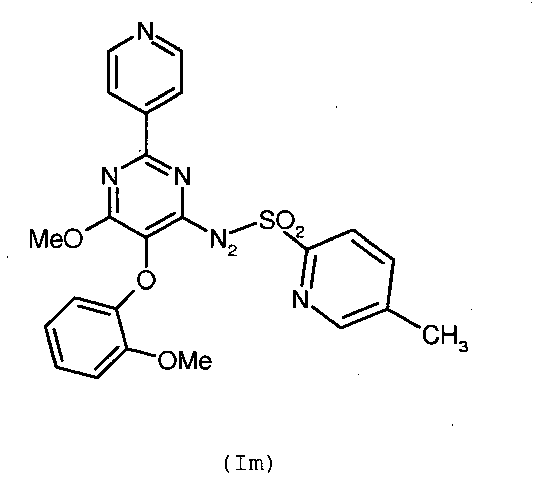 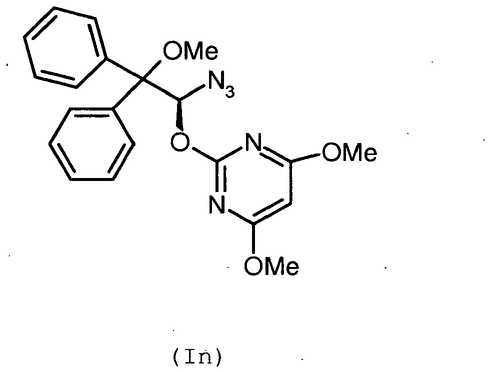 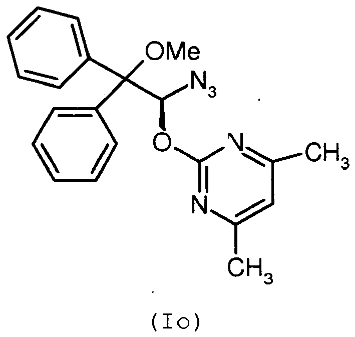 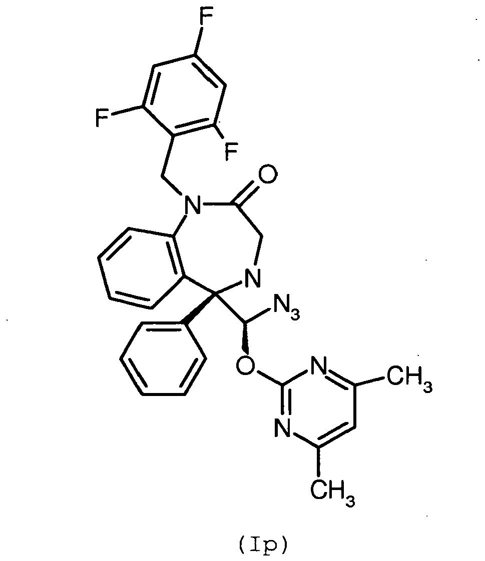 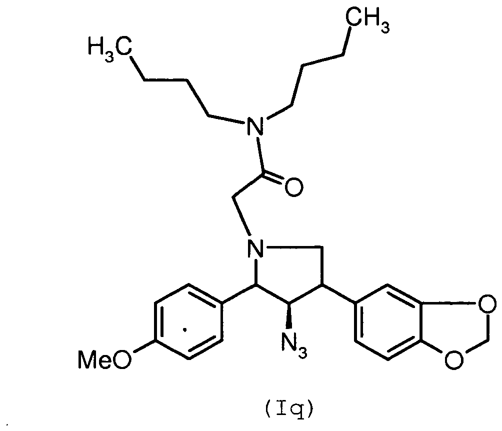 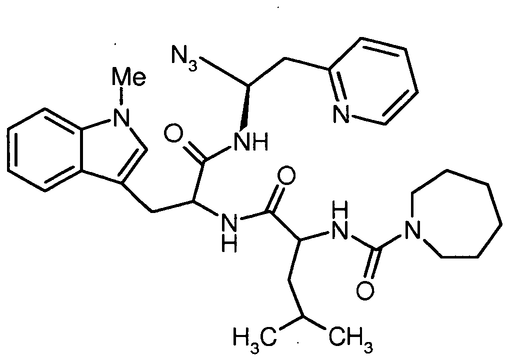 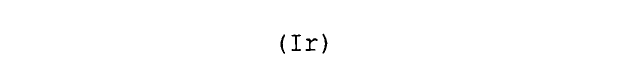 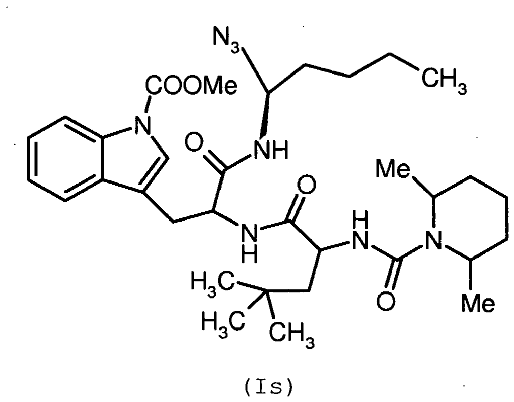 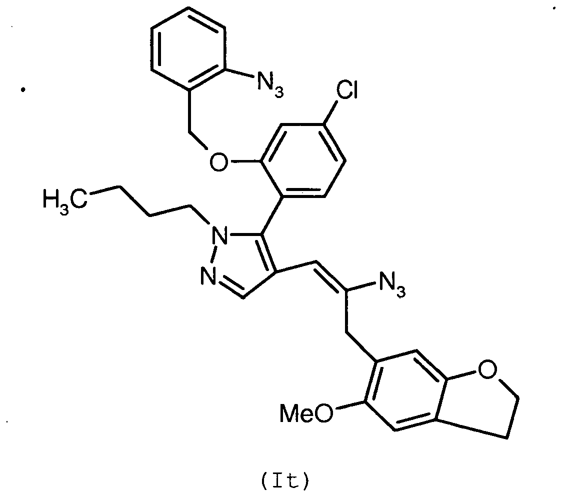 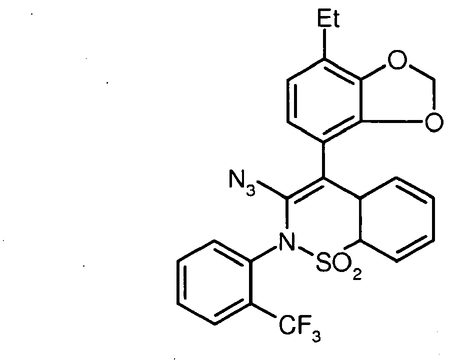 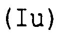 wherein:
N₁ is -O-, -OH;
N₂ is -N-, -NH-;
N₃ is -C(O)O-, -C(O)NH-;
B, B' and B" are -CO-, -C(O)O-, -C(O)NH;
C, C' and C" are: 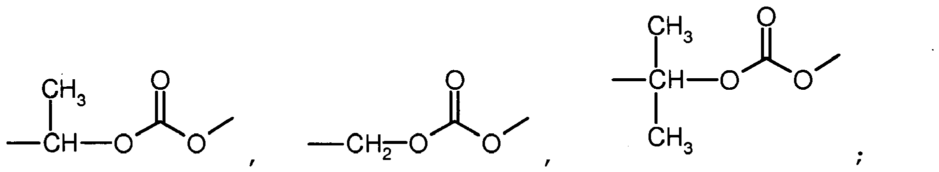 with the proviso that:
1) when A is selected from the group consisting of: (Ia), (Ib), (Ic), (Id), (Ie), (If), (Ig), (Ih), (Ii), (Ij), (Ik), (Il) and (Im), at least one of m, m', m", n, n' or n" is 1;
2) when A is selected from the group consisting of: (In), (Io), (Ip), (Iq), (Ir), (Is) and (Iu), then s', s" and m are 0; while n is 0 or 1;
3) when A is (It), then m, m' and s" are 0; while n and n' are 0 or 1;
4) at least one of N₁ or N₂ is a group -O- or -N- able to bind at least one of the groups -[(B)ₘ-(C)ₙ-(Y-ONO₂)], -[(B')_{m'}-(C')_{n'}-(Y'-ONO₂)] or -[(B")_{m"}-(C")_{n"}-(Y"-ONO₂)];
Y, Y' and Y" are a bivalent radical having the following meaning:
a)
- straight or branched C₁-C₂₀ alkylene, being optionally substituted with one or more of the substituents selected from the group consisting of: halogen atoms, hydroxy, -ONO₂ or Tₐ, wherein Tₐ is
- OC(O) (C₁-C₁₀ alkyl)-ONO₂ or -O(C₁-C₁₀ alkyl)-ONO₂;
- cycloalkylene with 5 to 7 carbon atoms into cycloalkylene ring, the ring being optionally substituted with side chains T, wherein T is straight or branched alkyl with from 1 to 10 carbon atoms;
b) 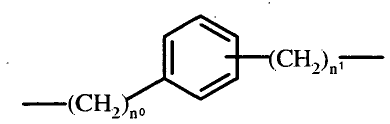
c) 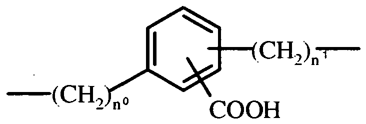 wherein n⁰ is an integer from 0 to 20, and n¹ is an integer from 1 to 20;
d) 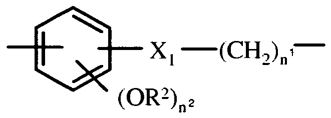 wherein:
n¹ is as defined above and n² is an integer from 0 to 2;
X₁ = -OCO- or -COO- and R² is H or CH₃;
e) 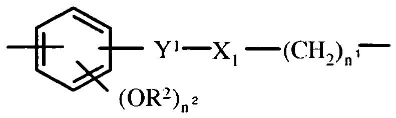 wherein:
n¹, n²,R² and X₁ are as defined above;
Y¹ is -CH₂-CH₂- or -CH=CH- (CH₂)ₙ²-;
f) 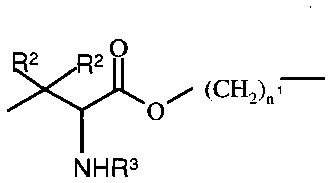 wherein:
n¹and R² are as defined above, R³ is H or -COCH₃;
with the proviso that when Y is selected from the bivalent radicals mentioned under b)-f), the -ONO₂ group is linked to a -CH₂ group;
g) 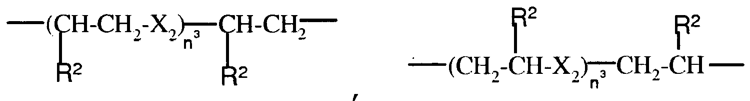 wherein X₂ is -O- or -S-, n³ is an integer from 1 to 6, R² is as defined above;
h) 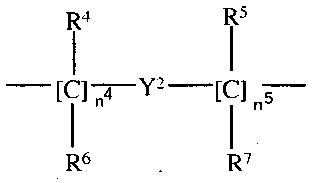 wherein:
n⁴ is an integer from 0 to 10;
n⁵ is an integer from 1 to 10;
R⁴, R⁵, R⁶, R⁷ are the same or different, and are H or straight or branched C₁-C₄ alkyl;
wherein the -ONO₂ group is linked to 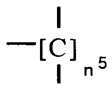
wherein n⁵ is as defined above;
Y² is an heterocyclic saturated, unsaturated or aromatic 5 or 6 members ring, containing one or more heteroatoms selected from nitrogen, oxygen, sulfur,
and is selected from the group consisting of:

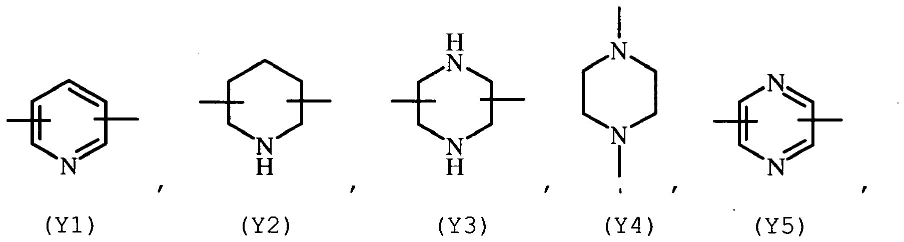

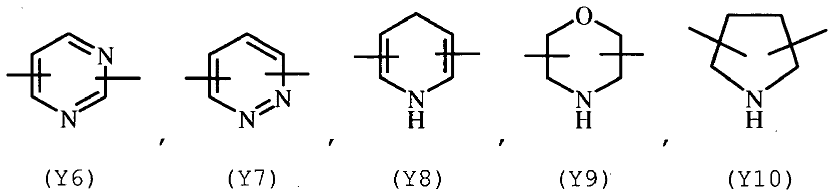

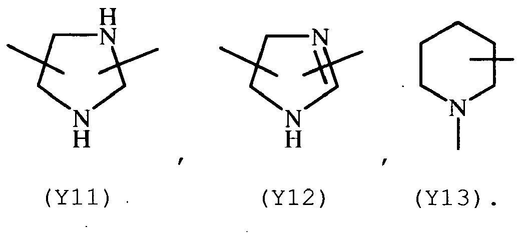

2. A compound of general formula (I) or a pharmaceutically acceptable salt or stereoisomer thereof according to claim 1, wherein Y, Y' and Y" are a bivalent radical having the following meaning:
a)
- straight or branched C₁-C₁₀ alkylene, being optionally substituted with one -ONO₂ group;
b) 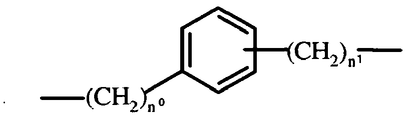 wherein n° is an integer equal to 0 or 1, and n¹ is an integer equal to 1; with the proviso the -ONO₂ group is linked to a -CH₂ group;
g) 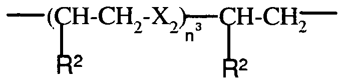 wherein X₂ is -O- or -S-, n³ is an integer equal to 1 and R² is H.

3. A compound according to claims 1-2, selected from the group consisting of:

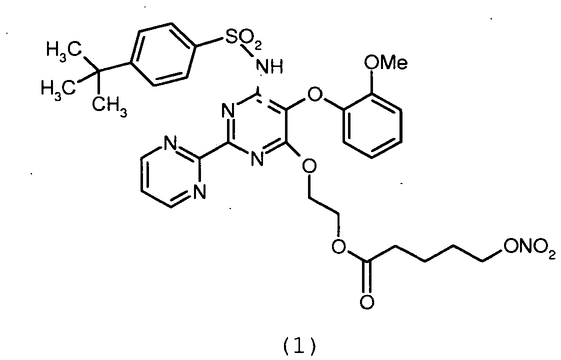

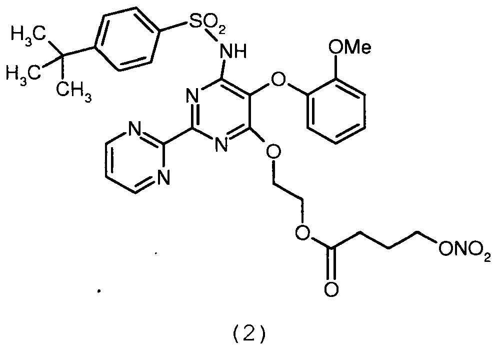

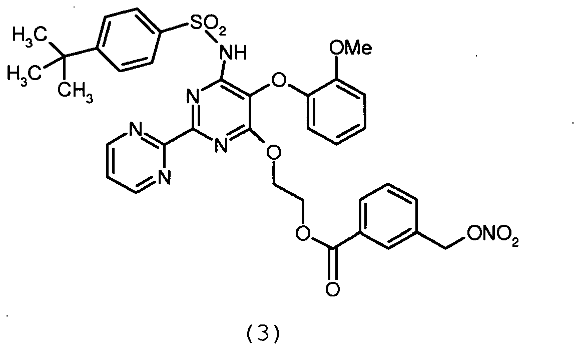

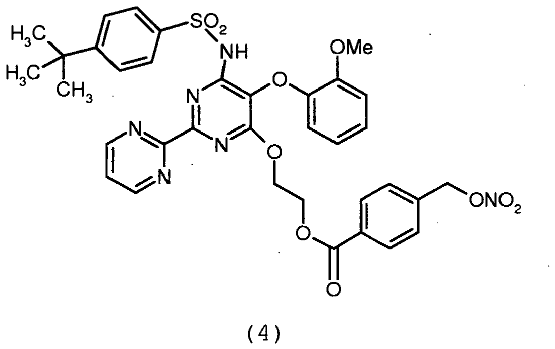

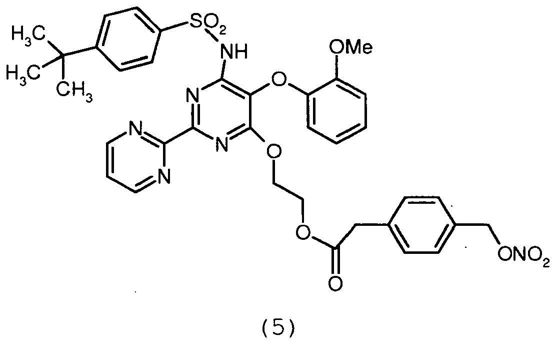

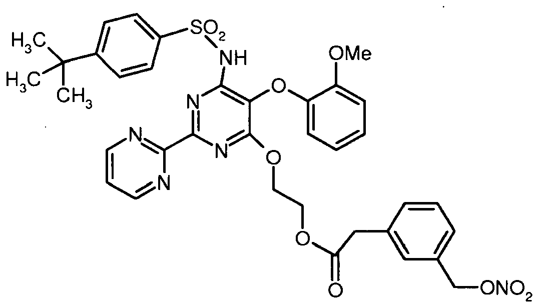

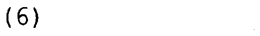

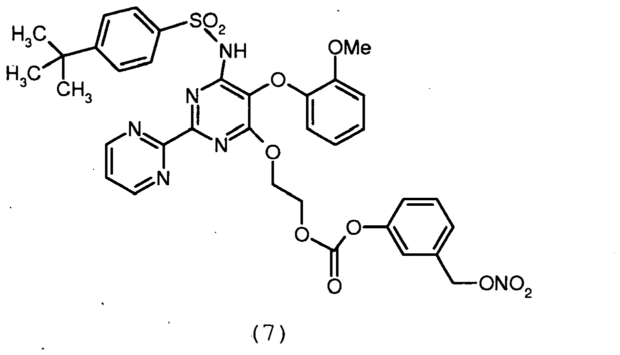

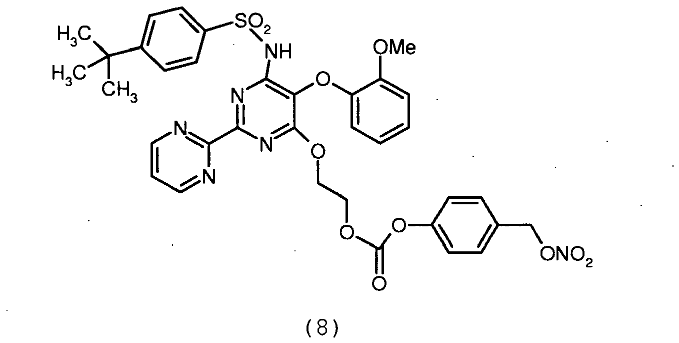

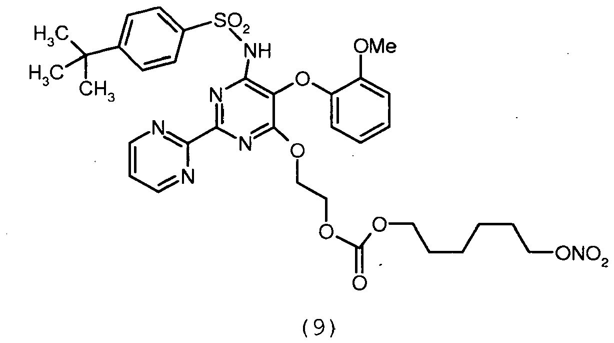

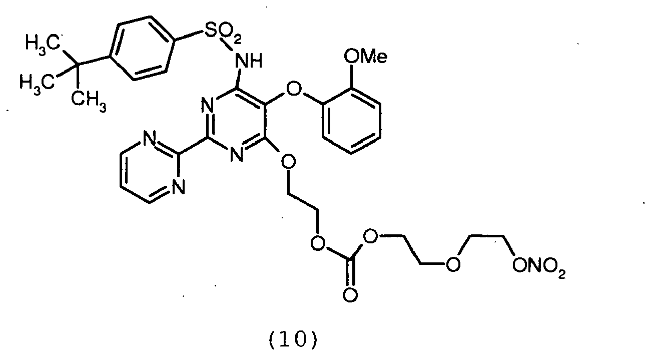

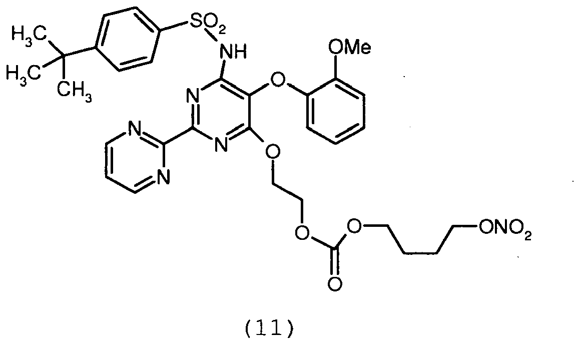

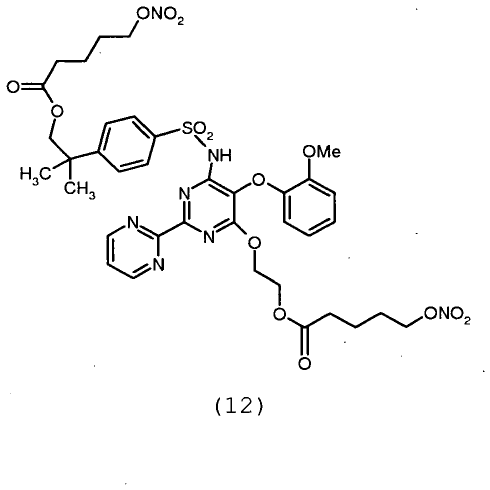

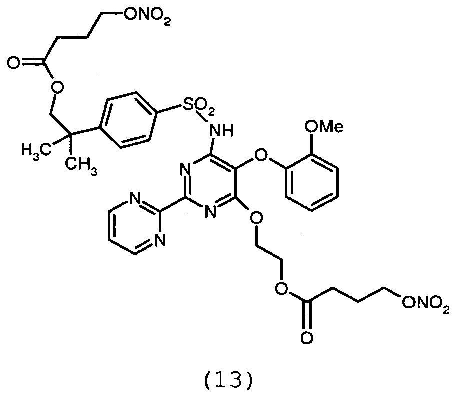

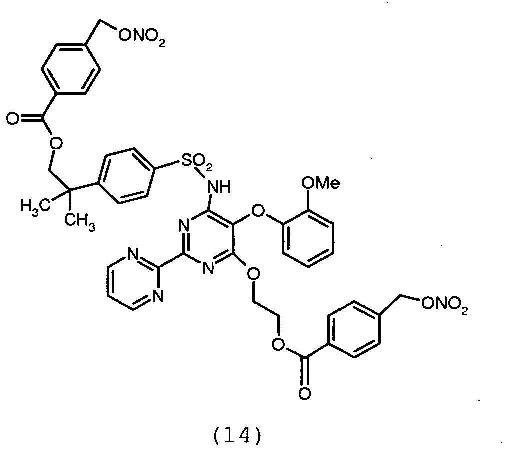

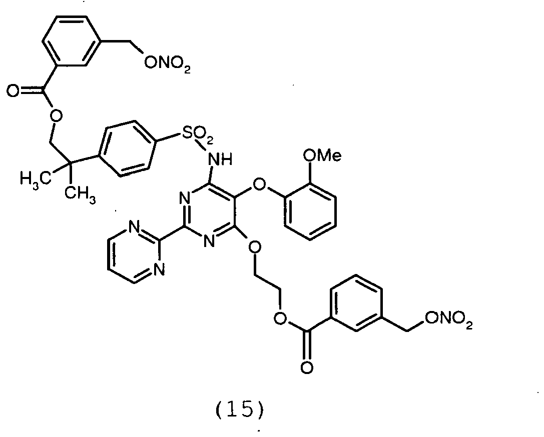

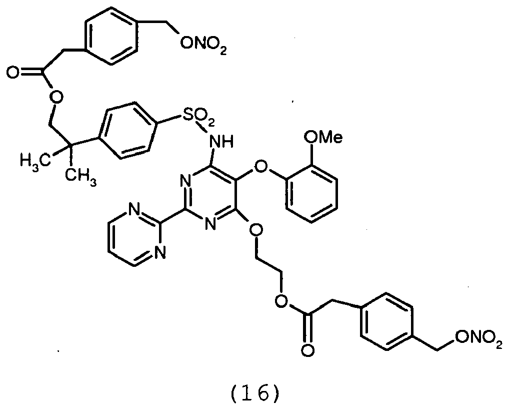

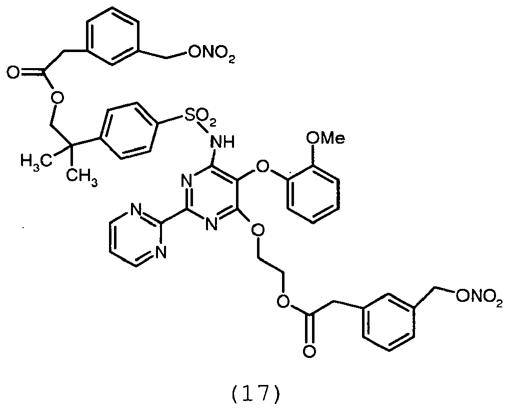

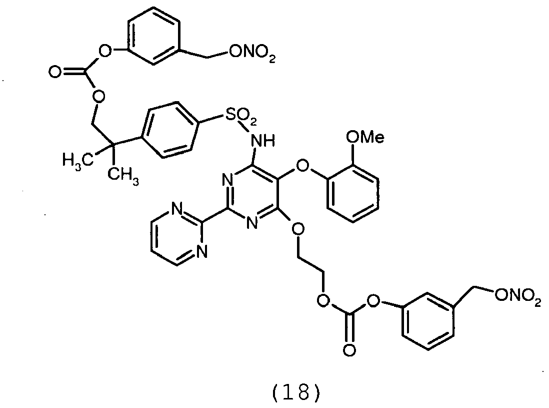

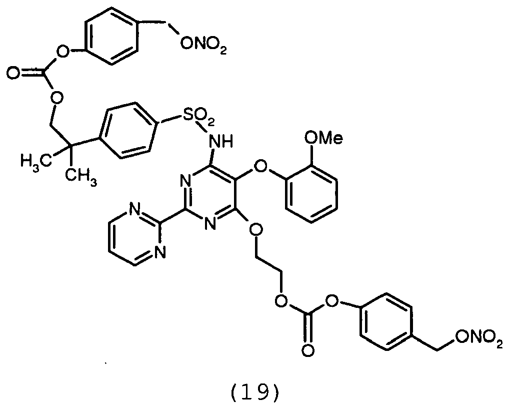

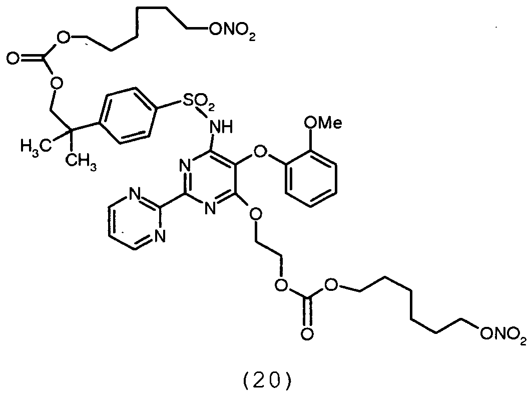

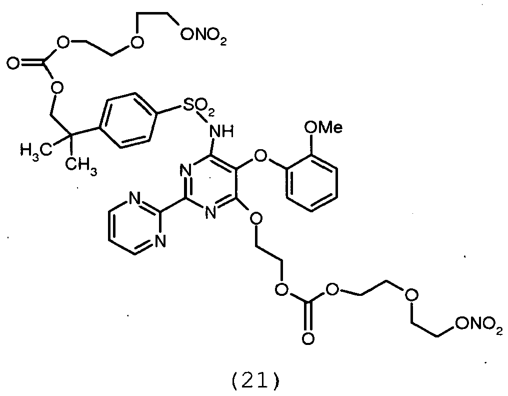

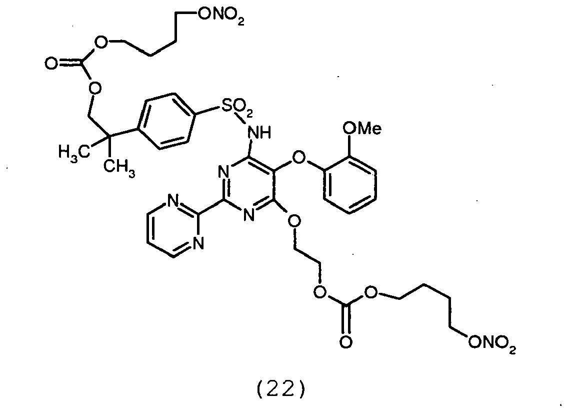

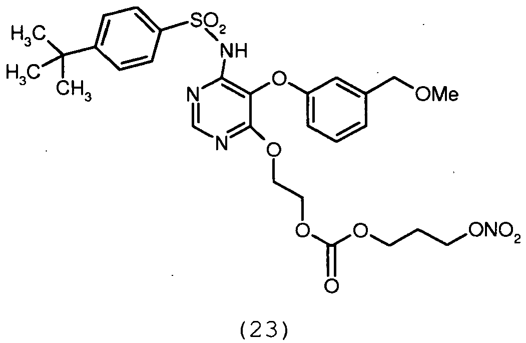

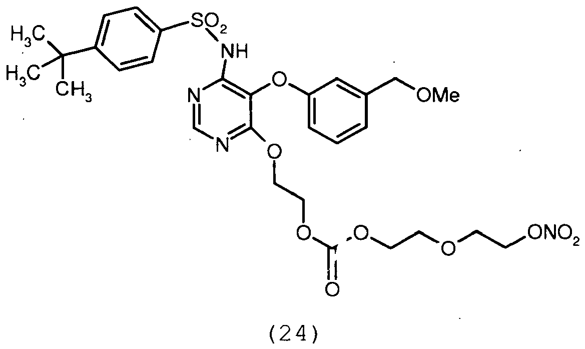

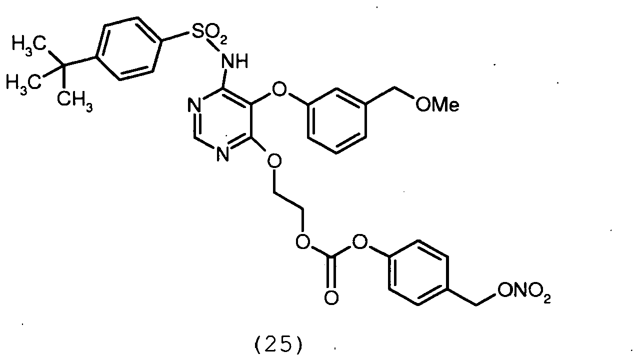

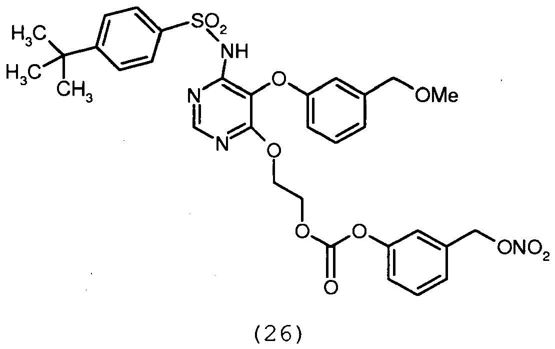

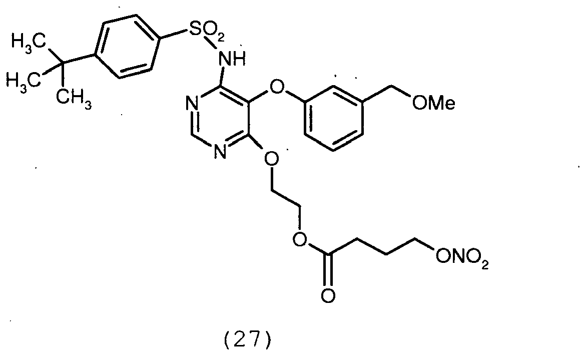

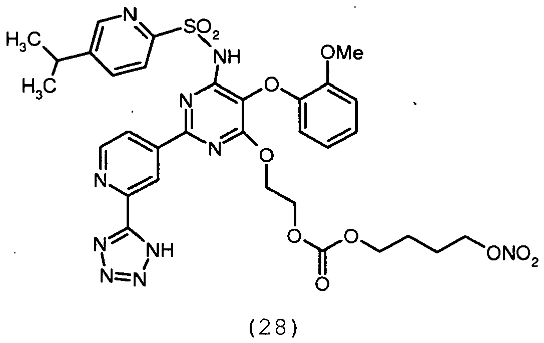

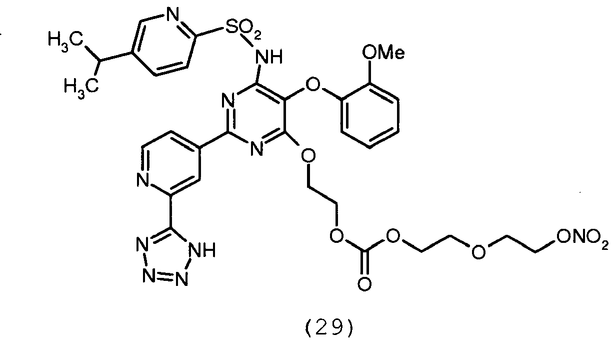

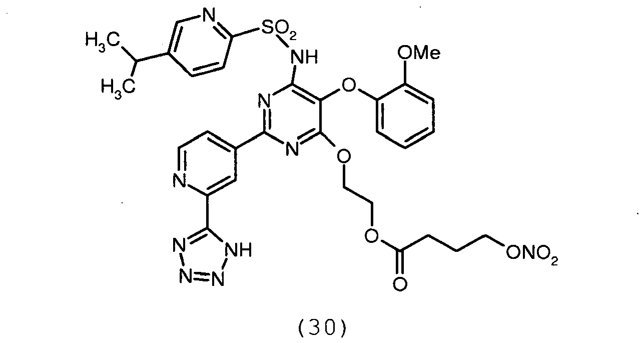

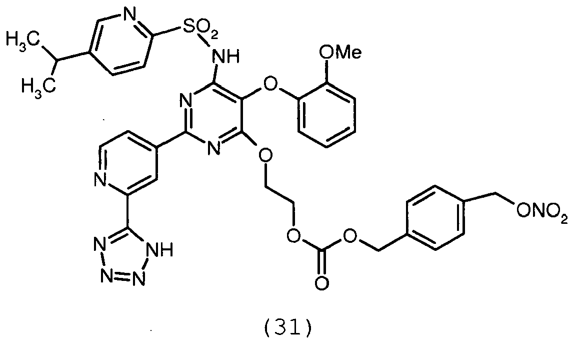

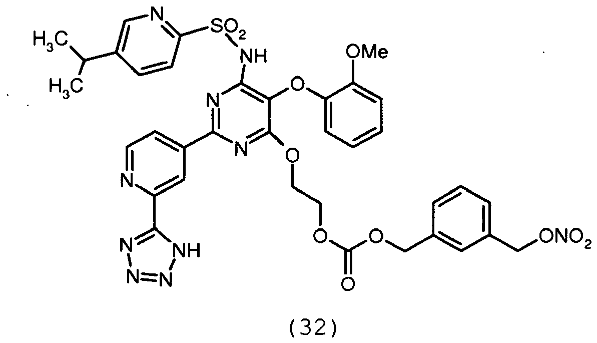

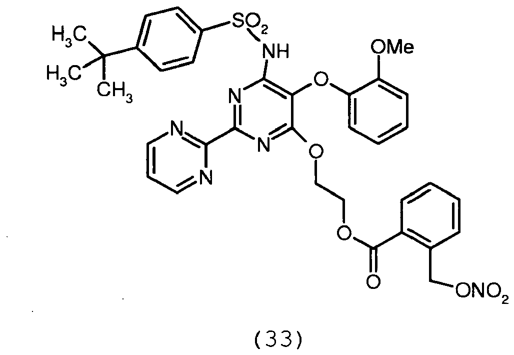

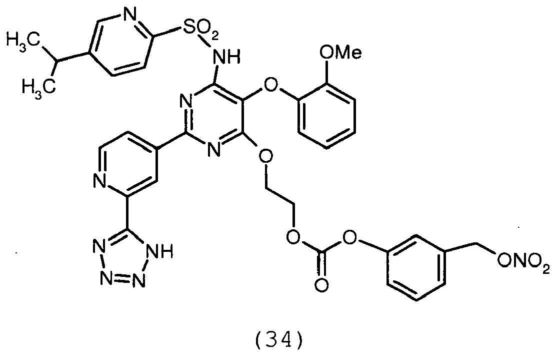

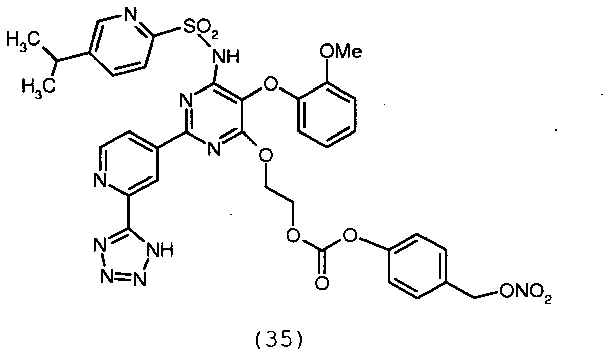

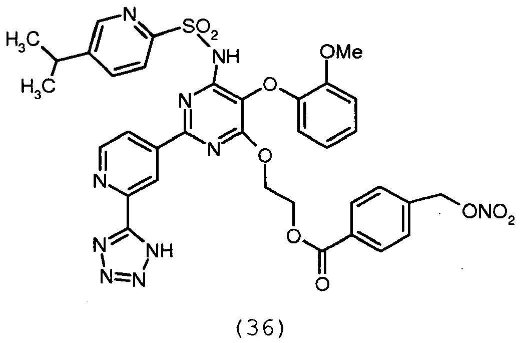

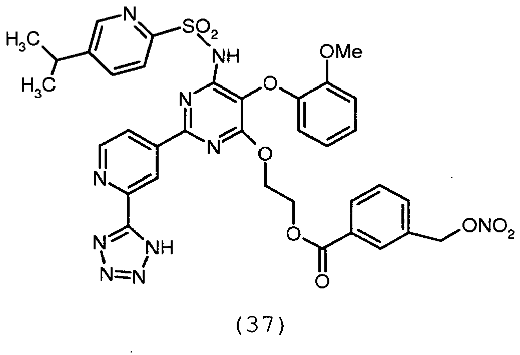

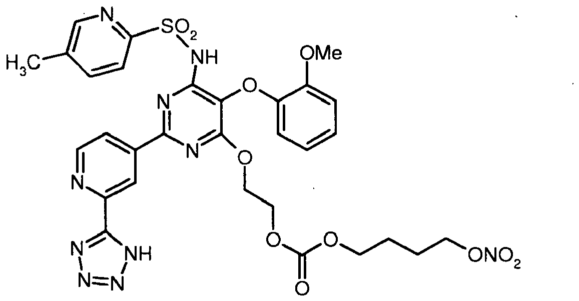

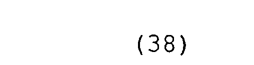

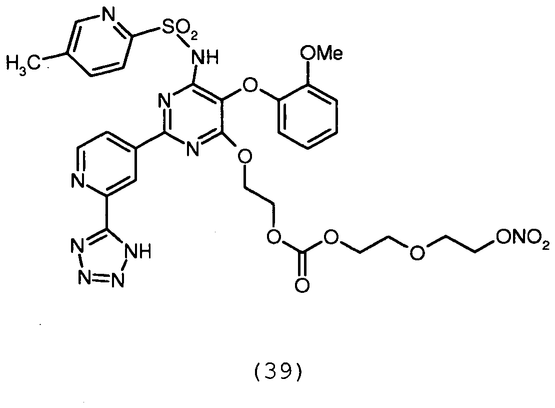

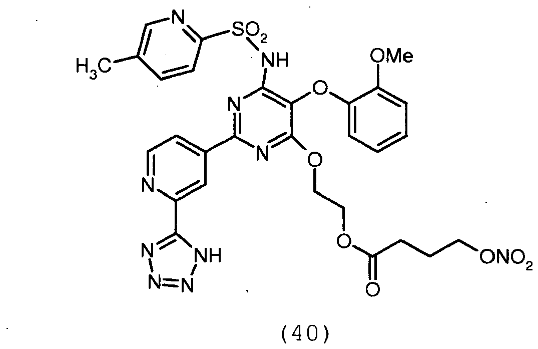

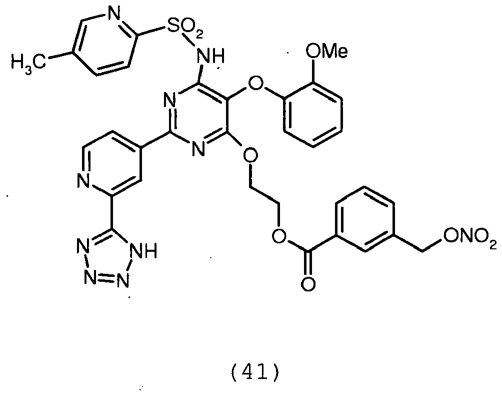

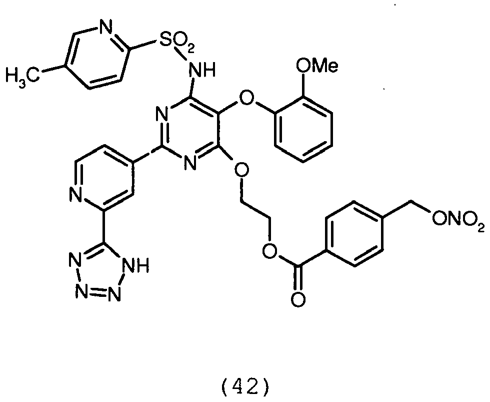

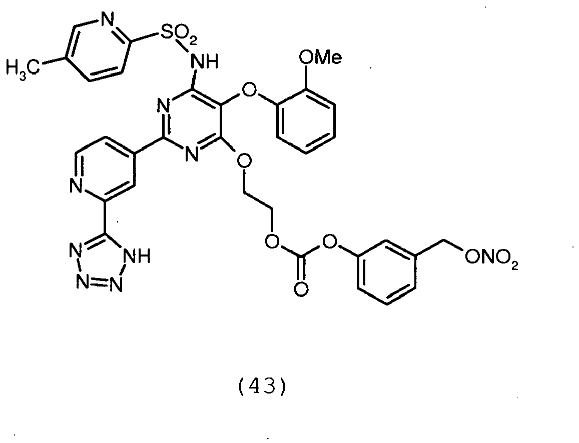

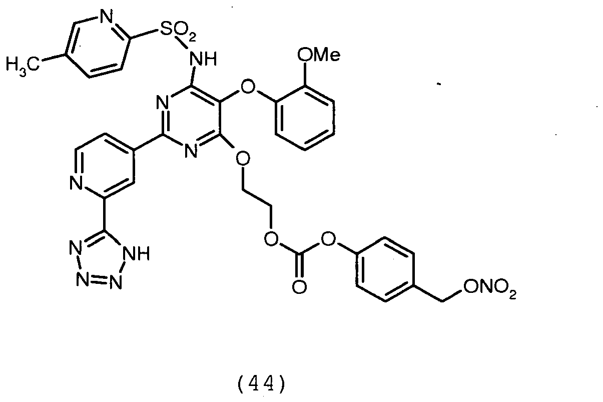

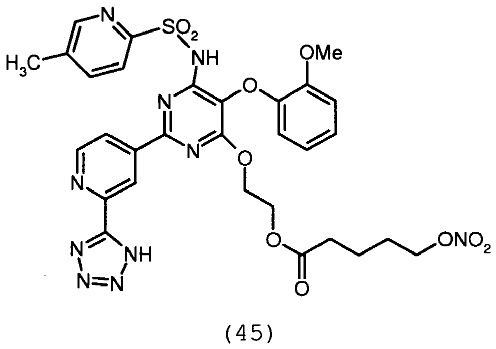

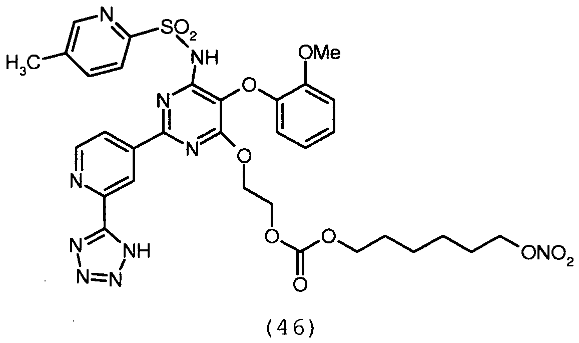

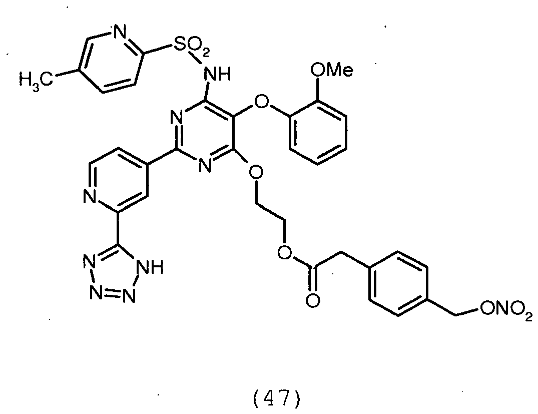

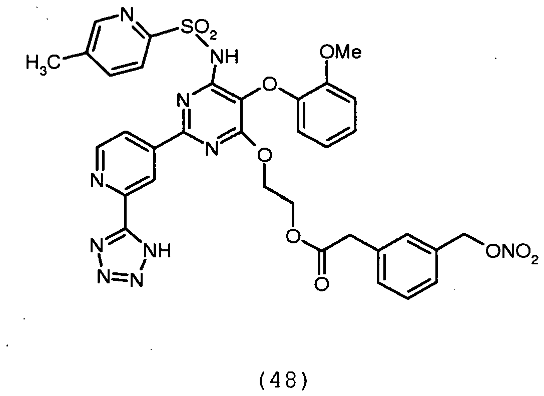

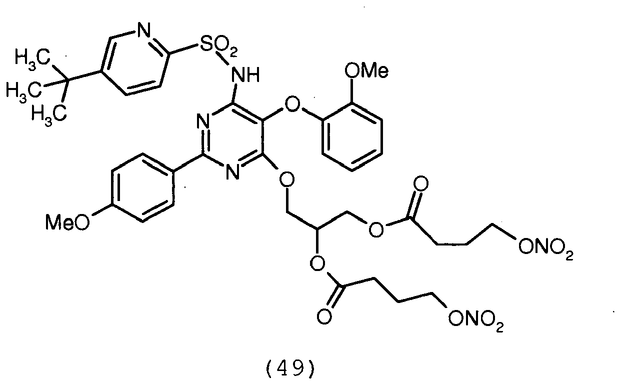

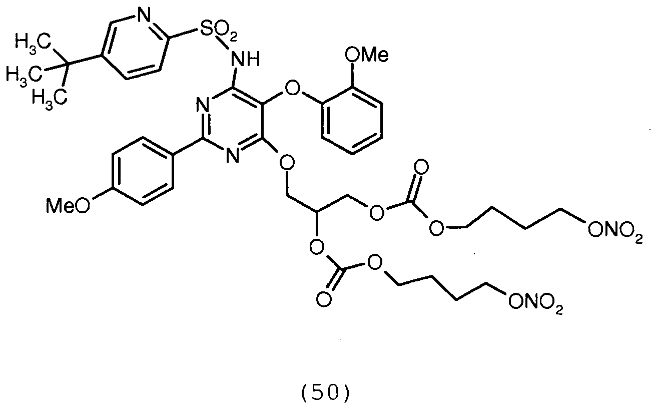

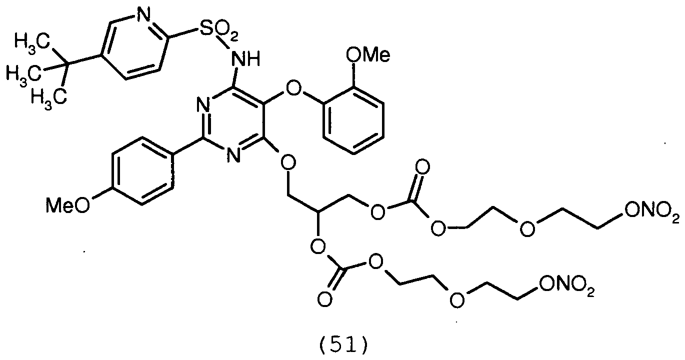

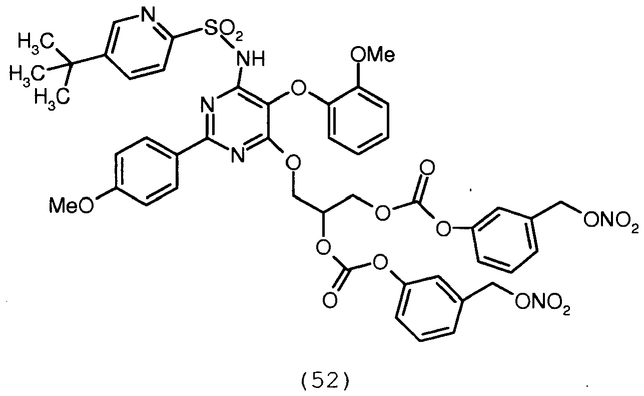

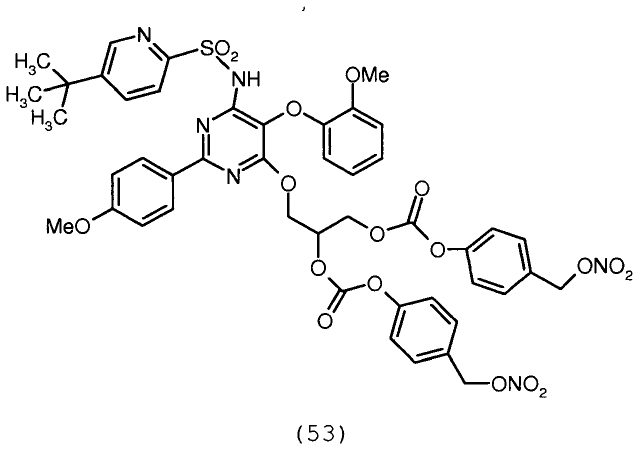

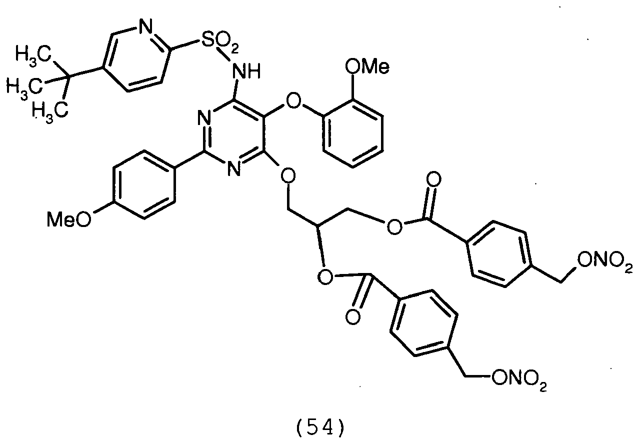

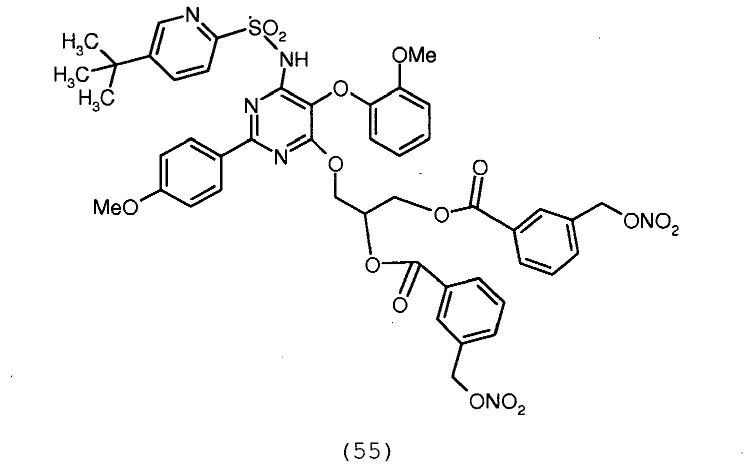

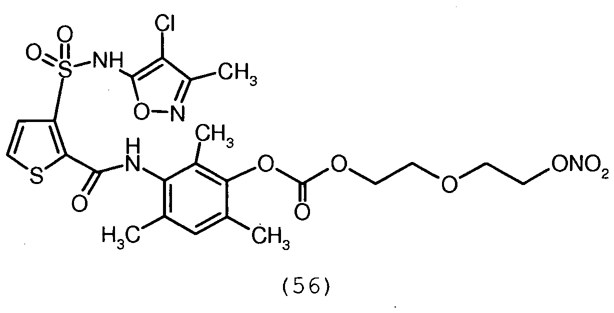

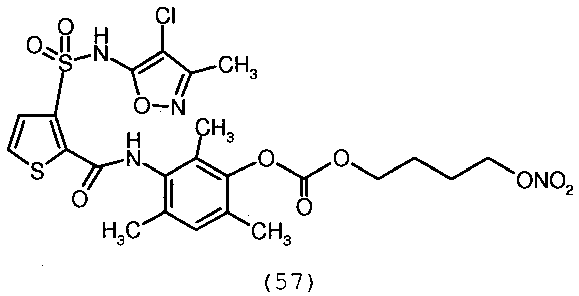

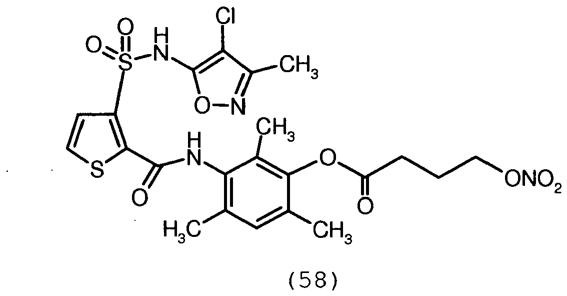

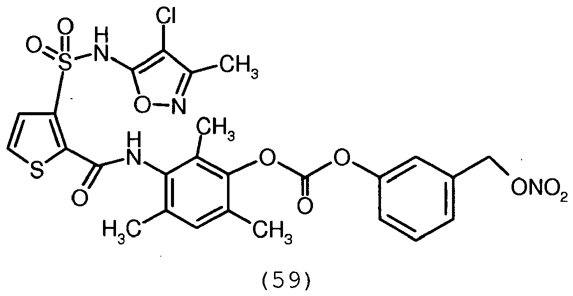

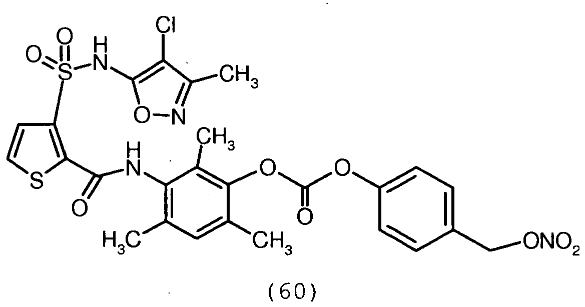

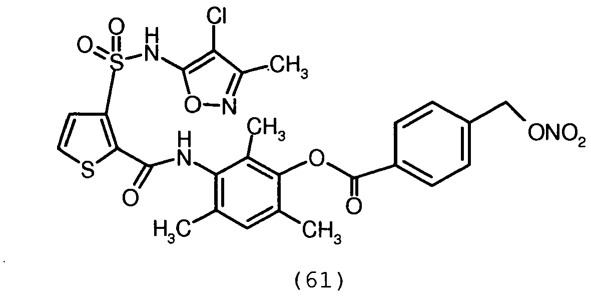

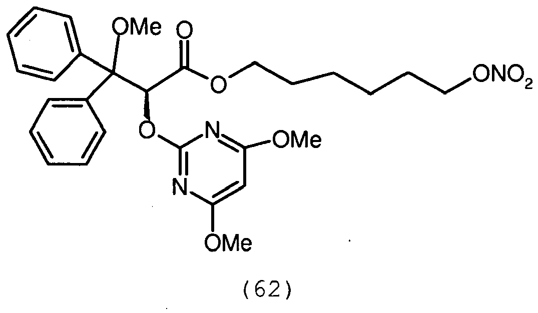

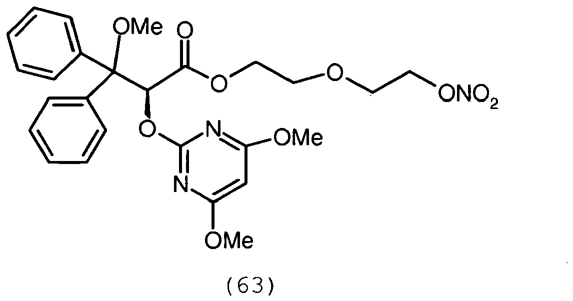

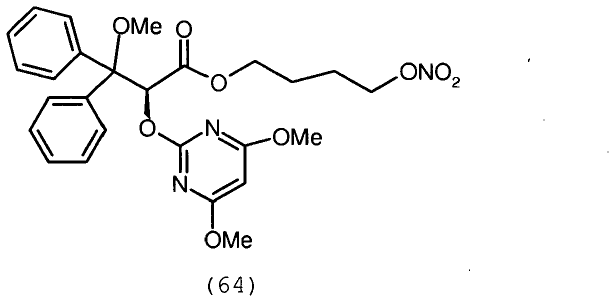

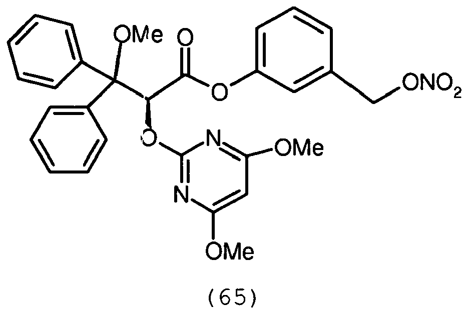

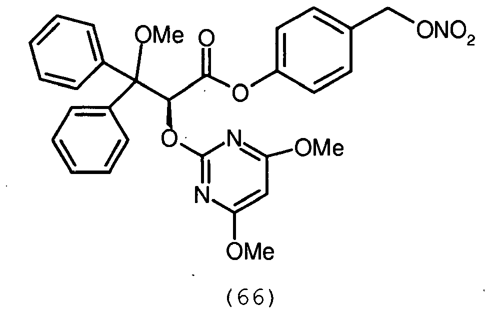

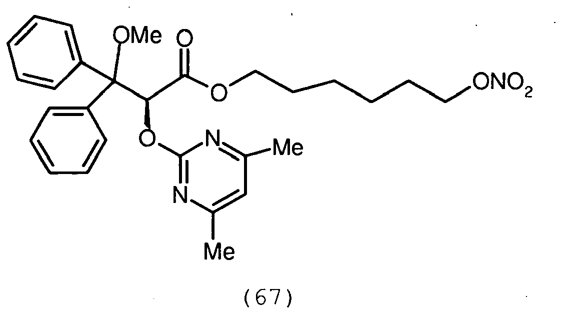

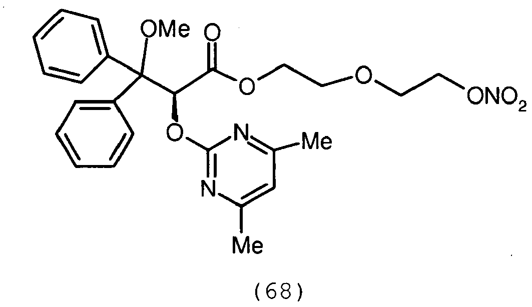

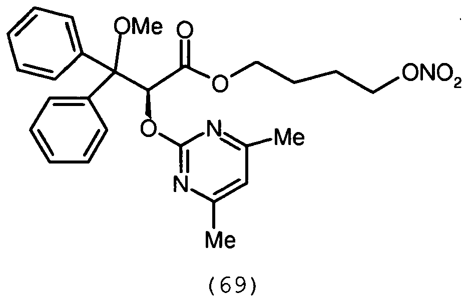

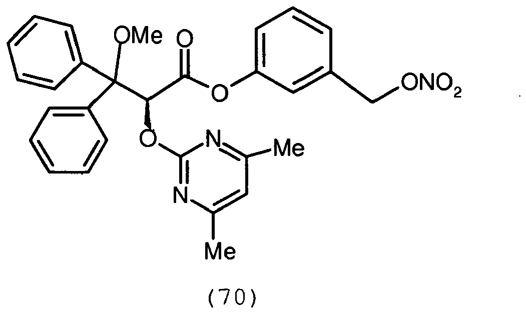

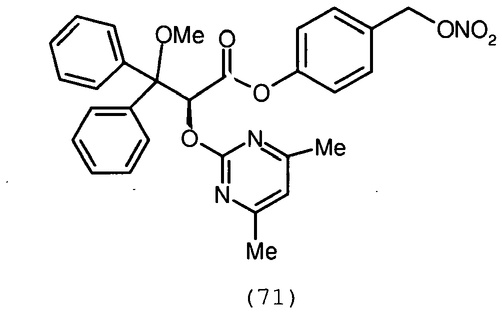

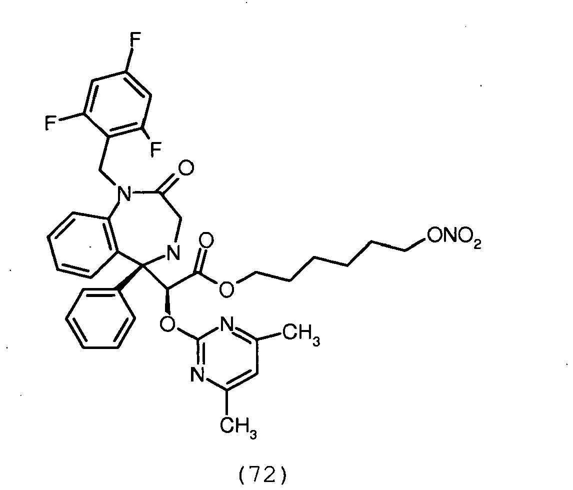

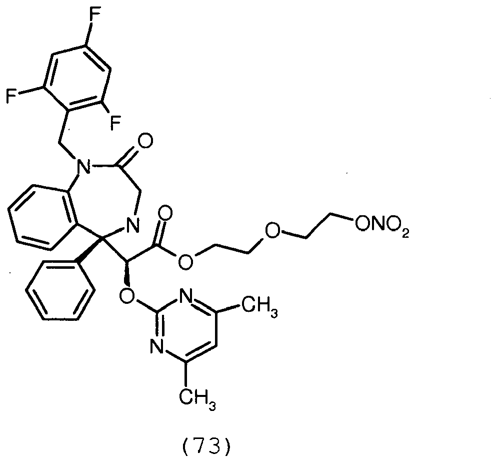

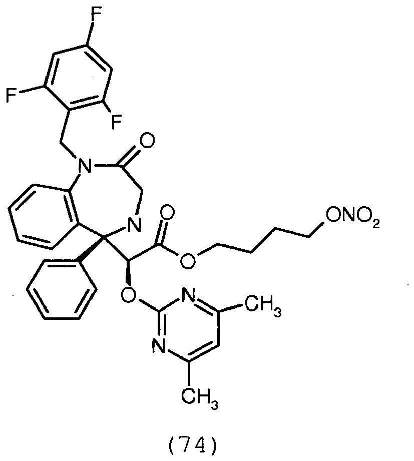

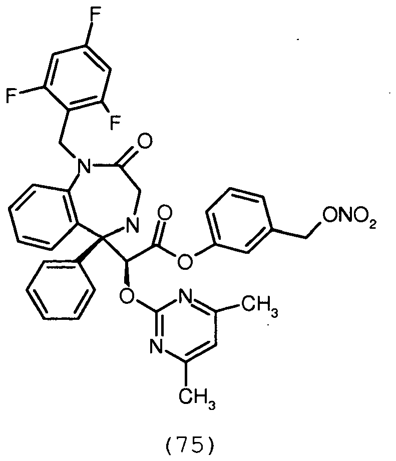

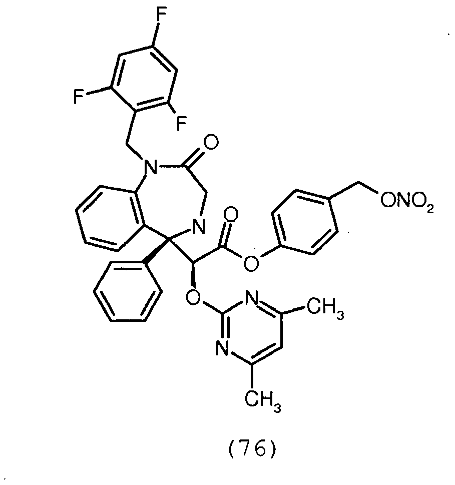

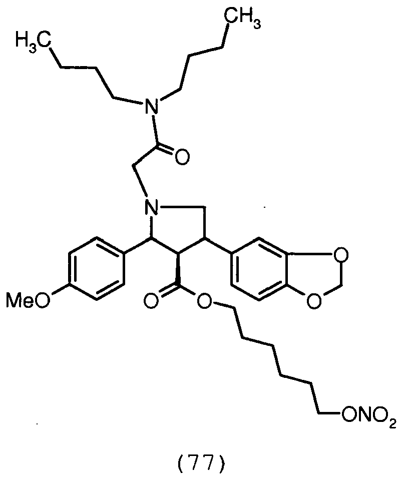

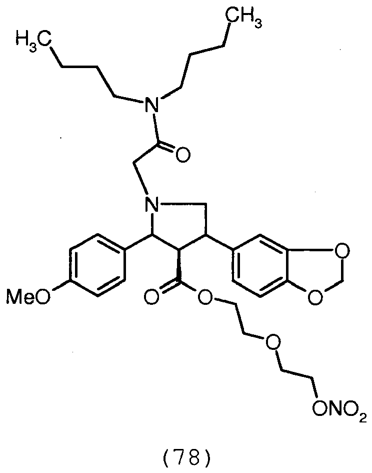

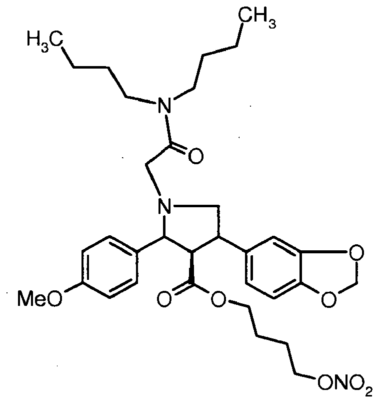

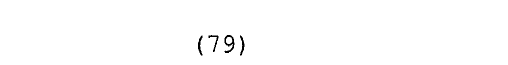

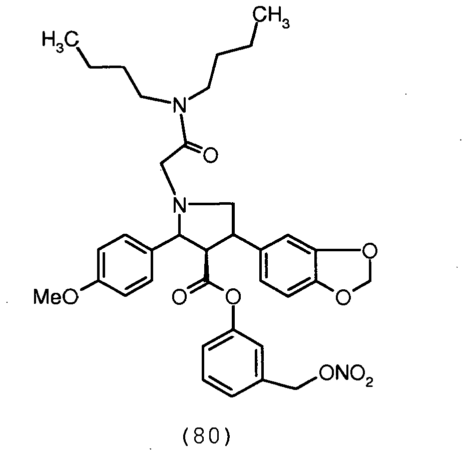

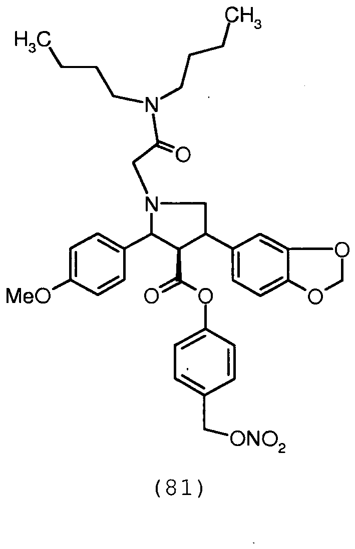

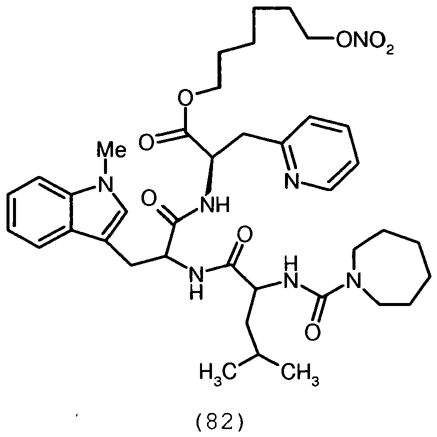

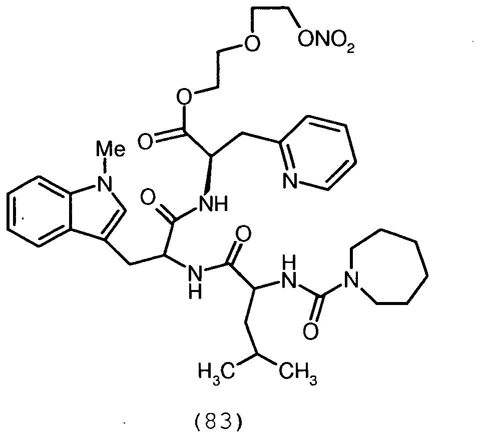

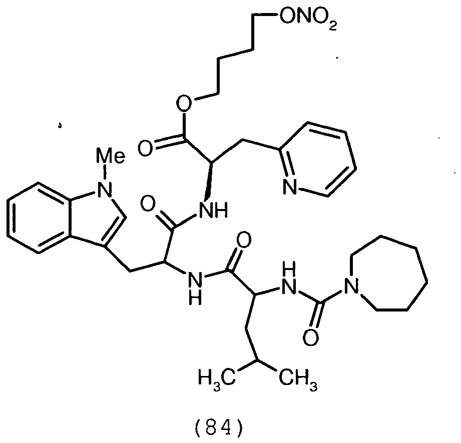

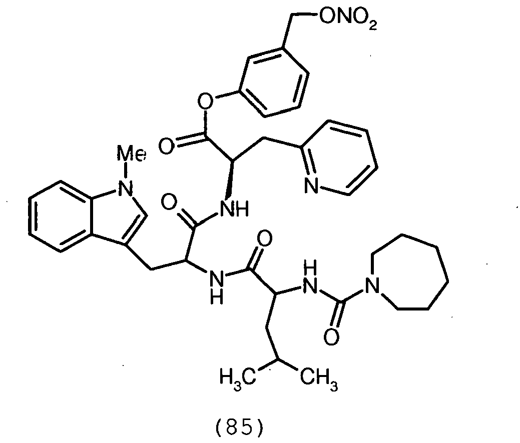

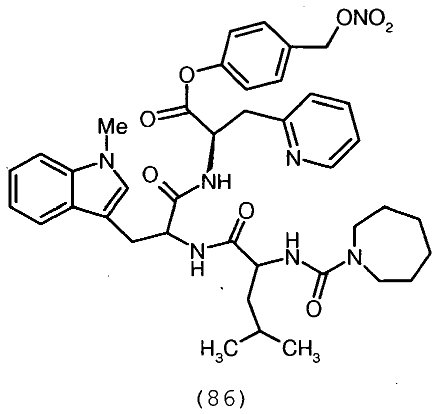

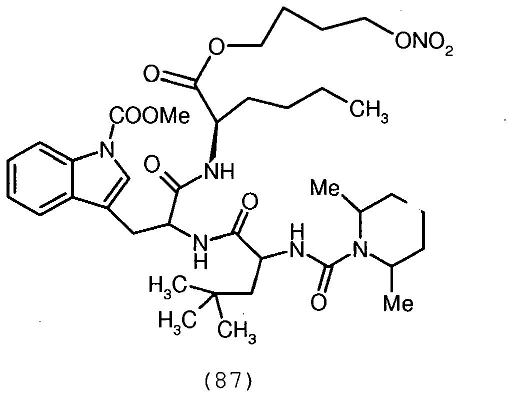

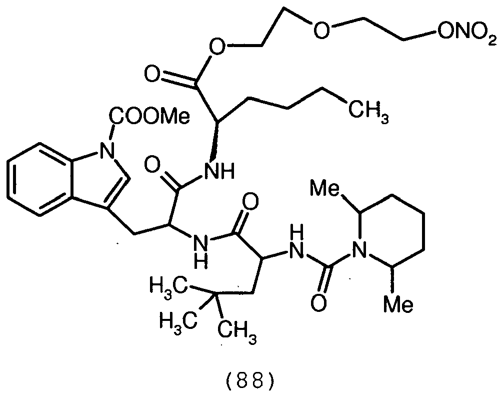

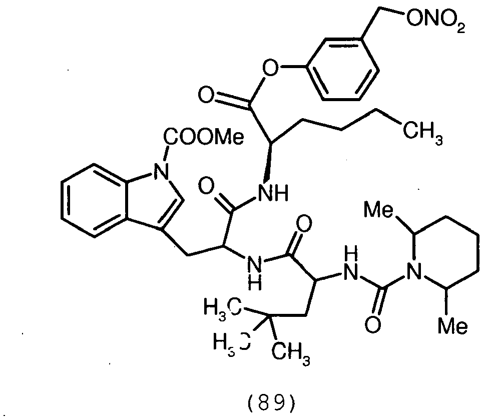

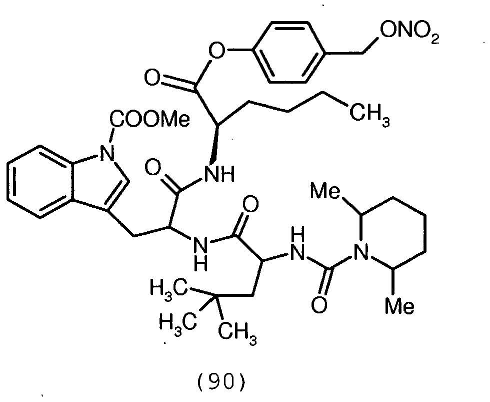

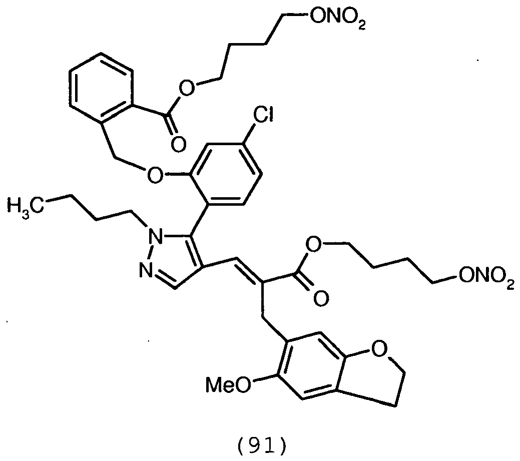

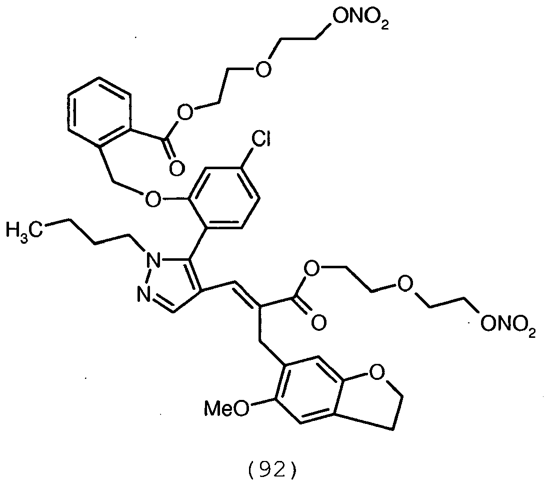

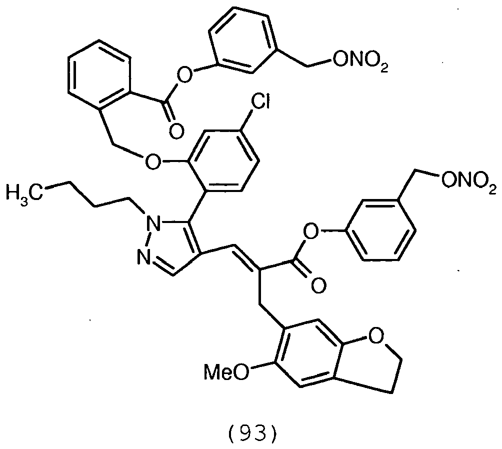

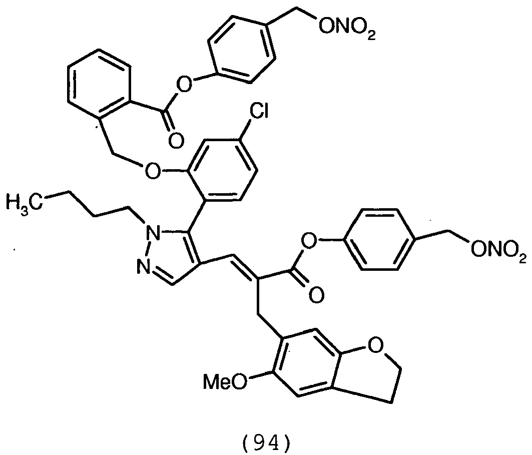

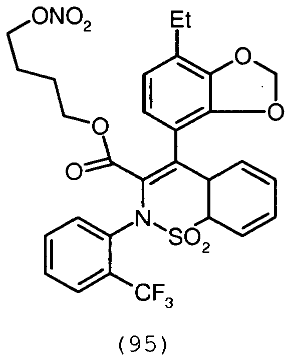

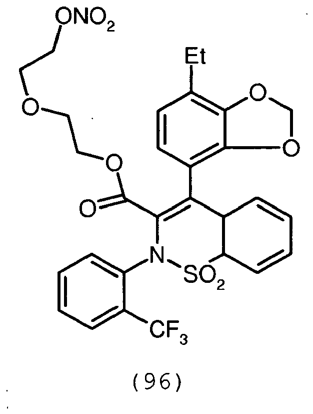

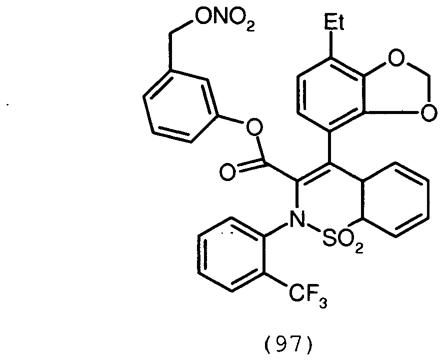

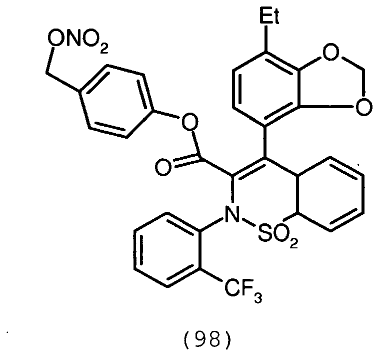

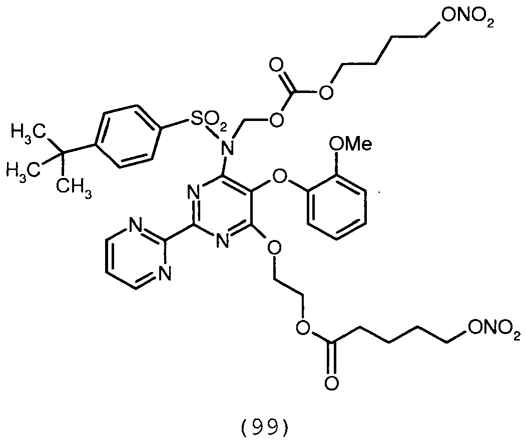

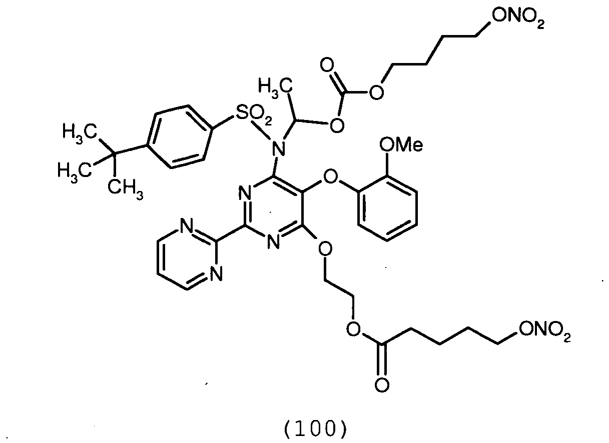

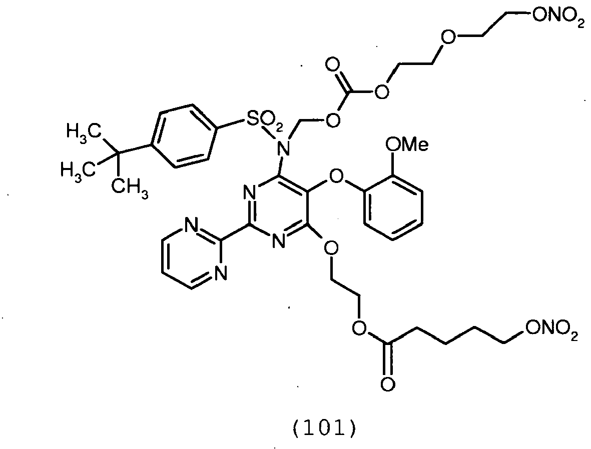

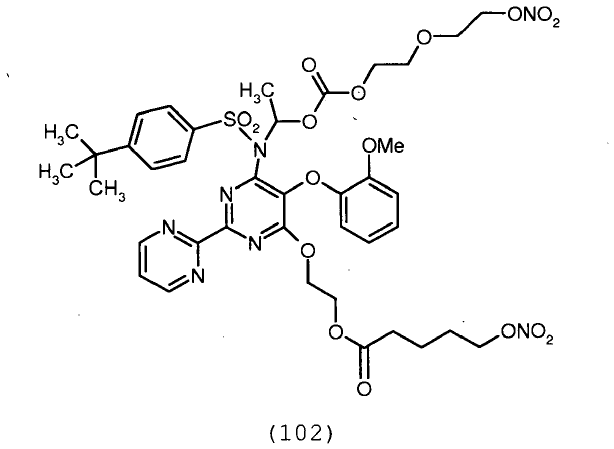

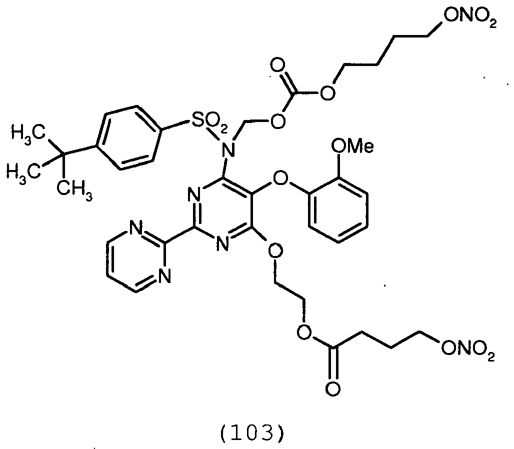

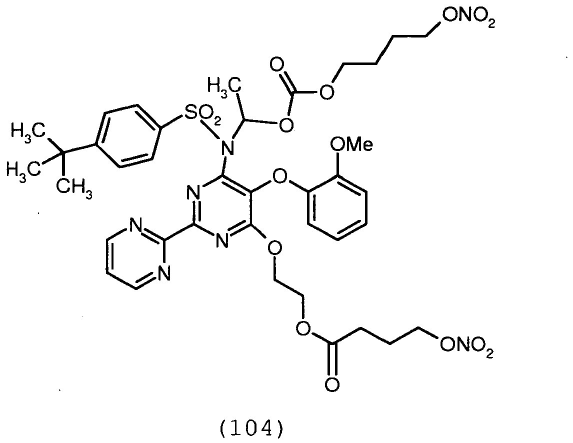

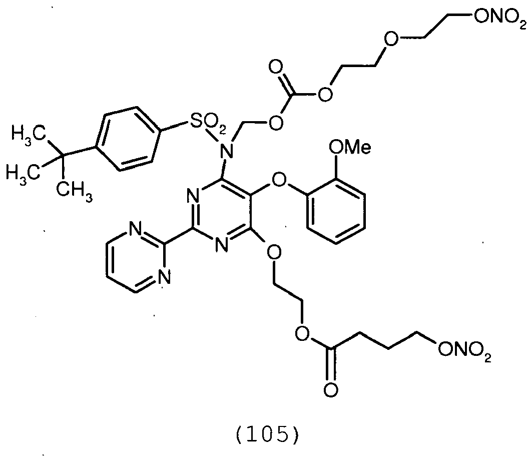

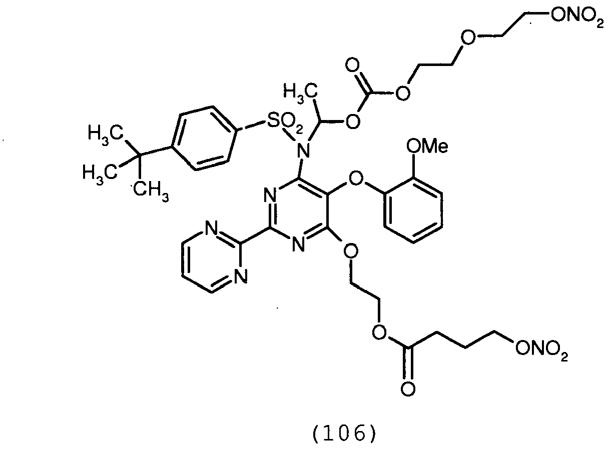

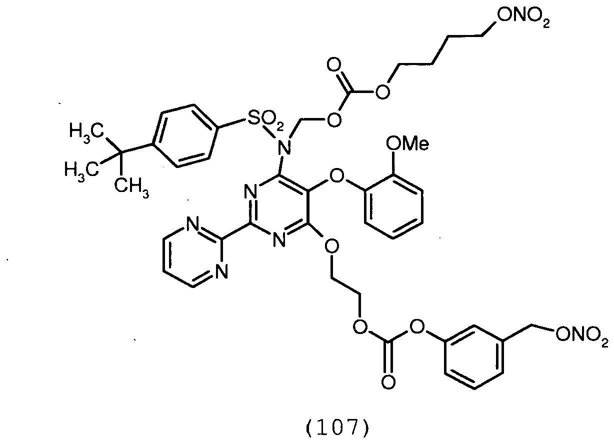

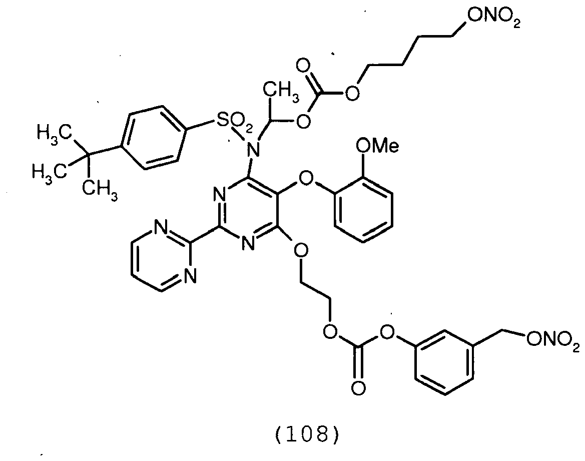

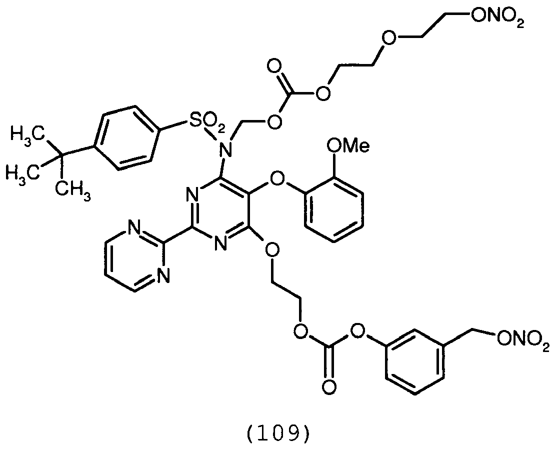

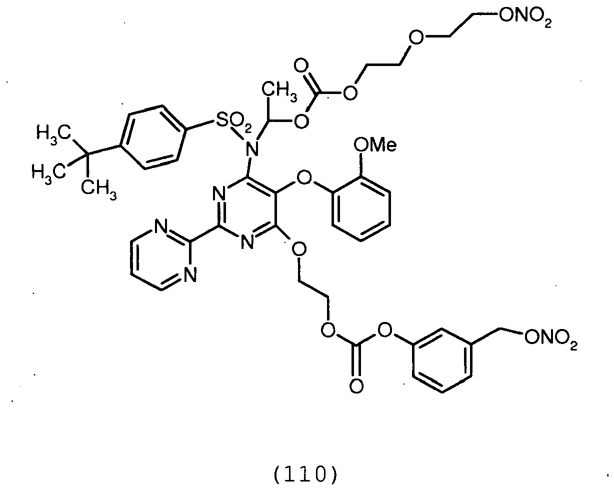

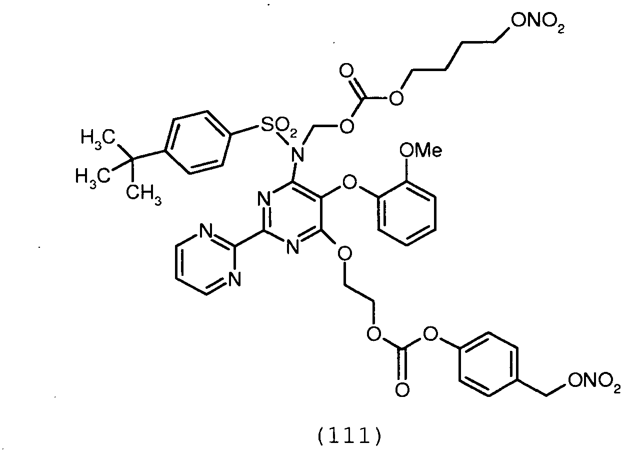

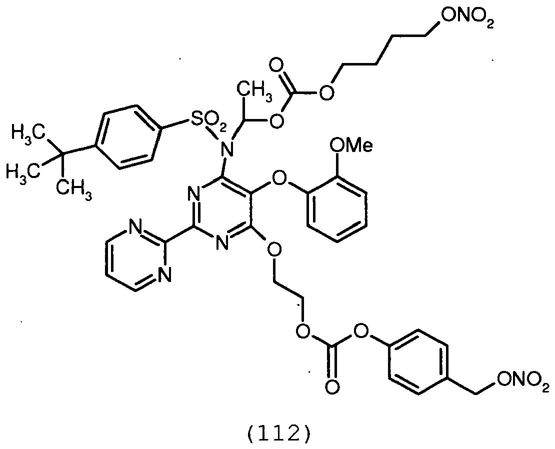

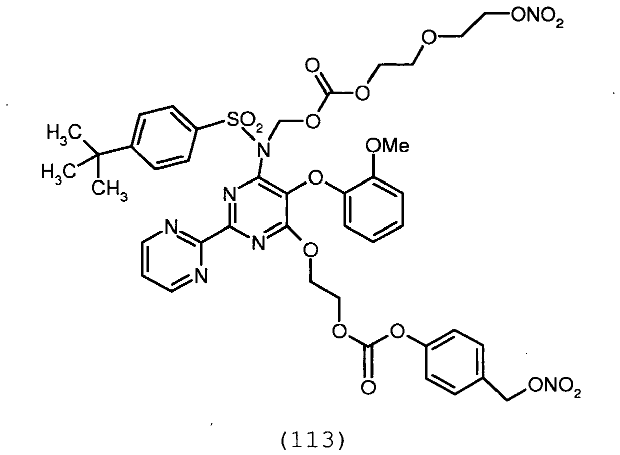

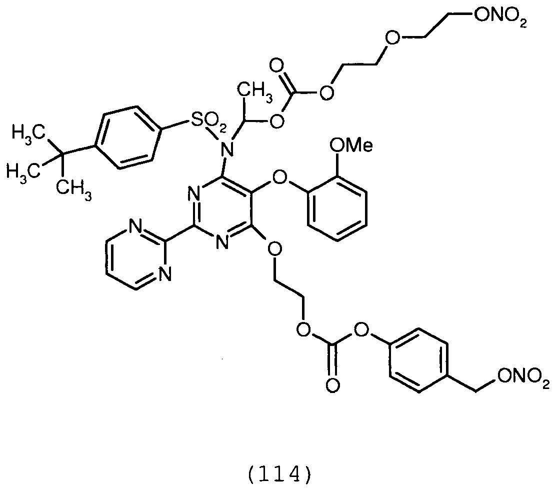

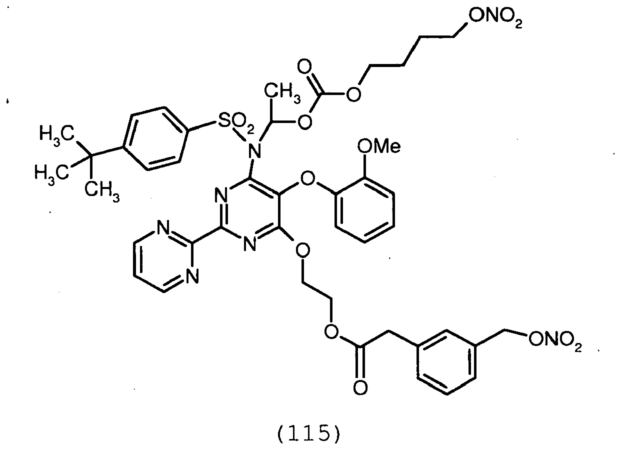

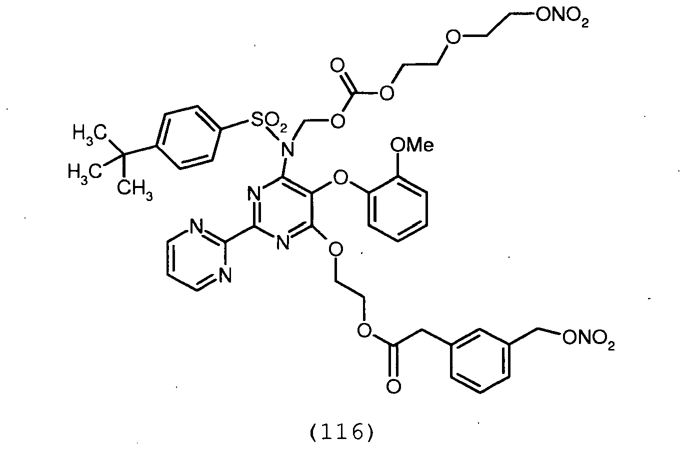

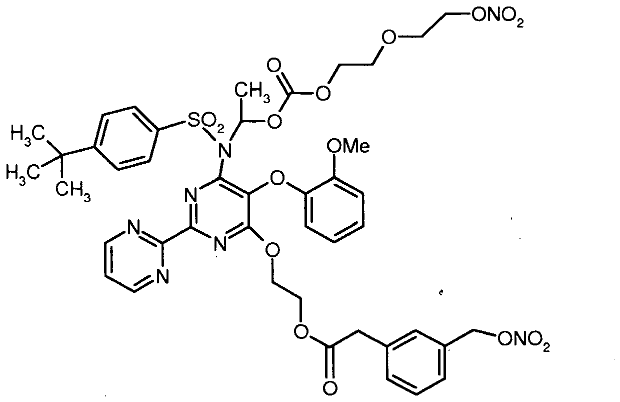

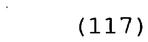

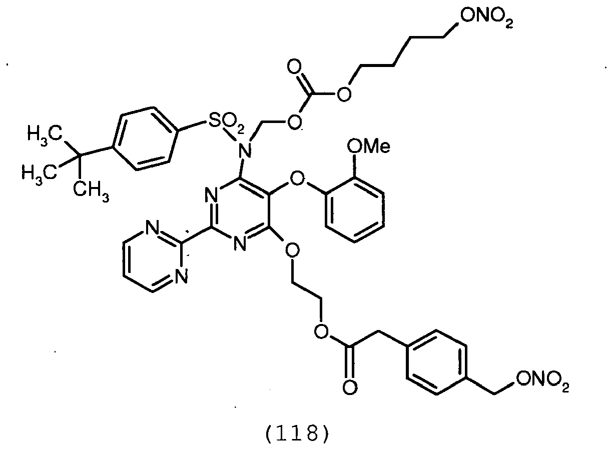

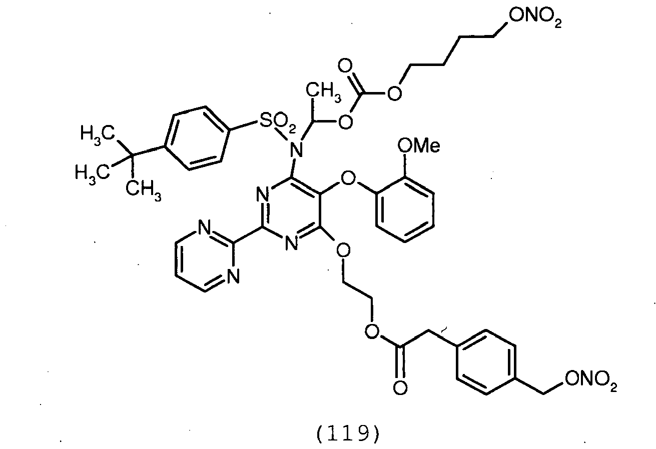

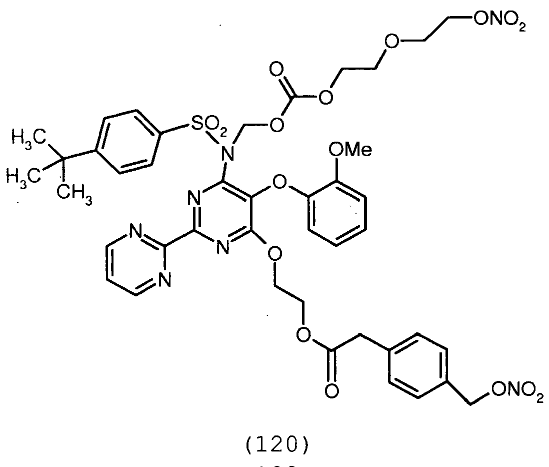

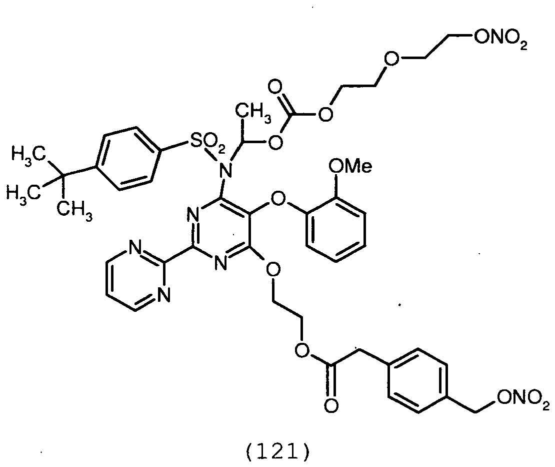

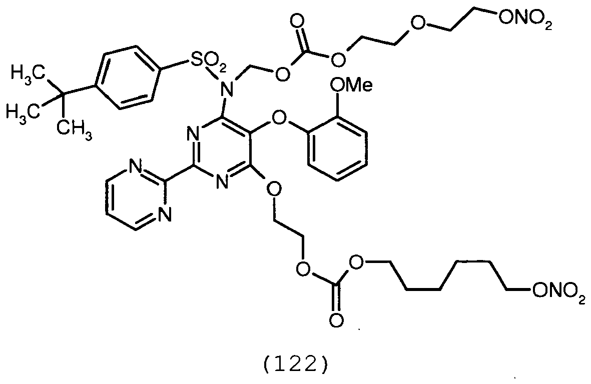

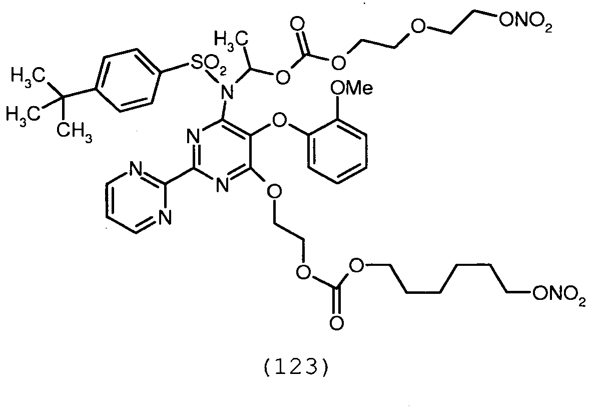

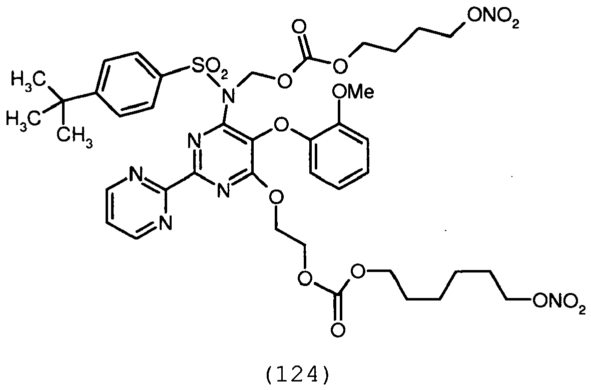

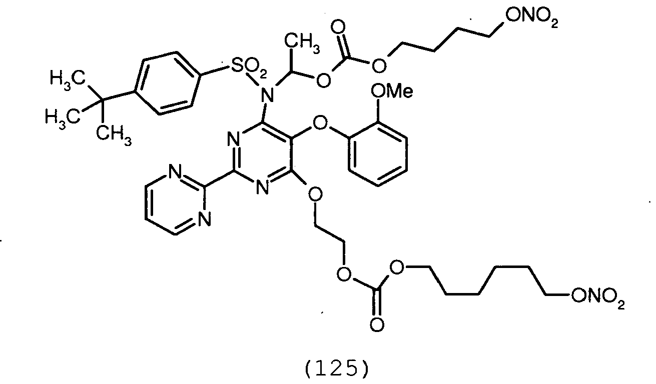

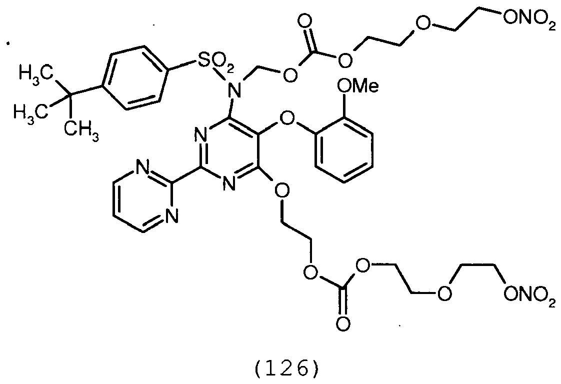

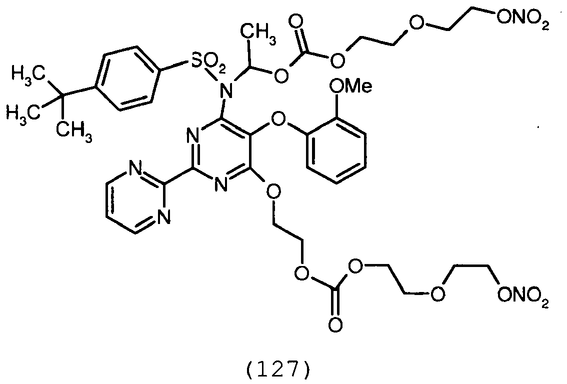

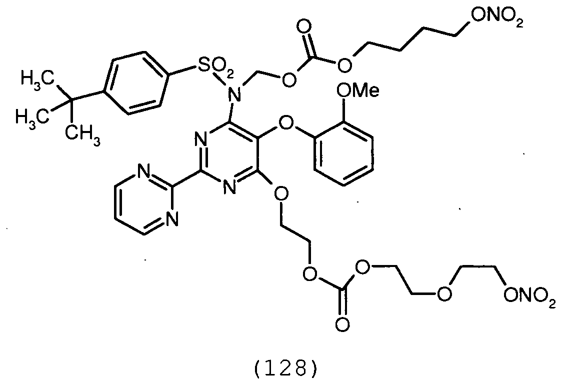

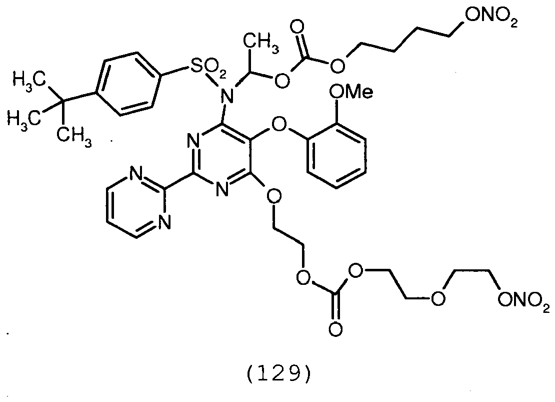

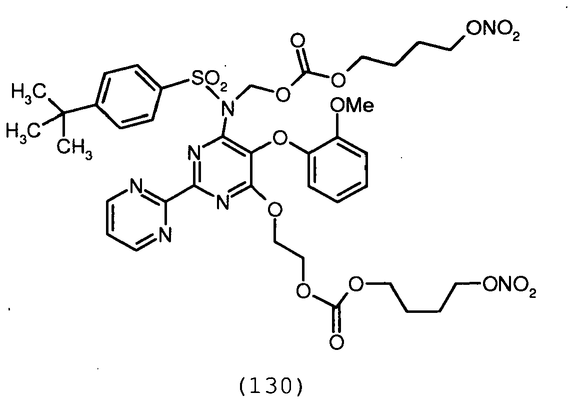

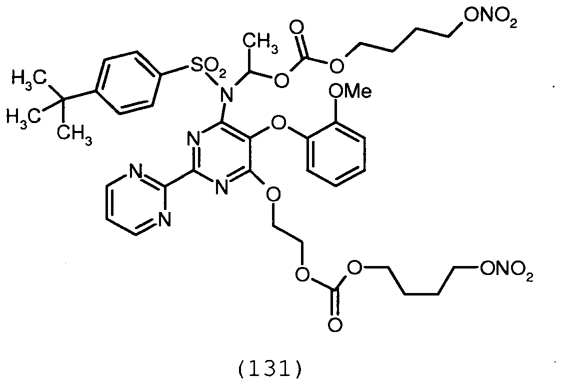

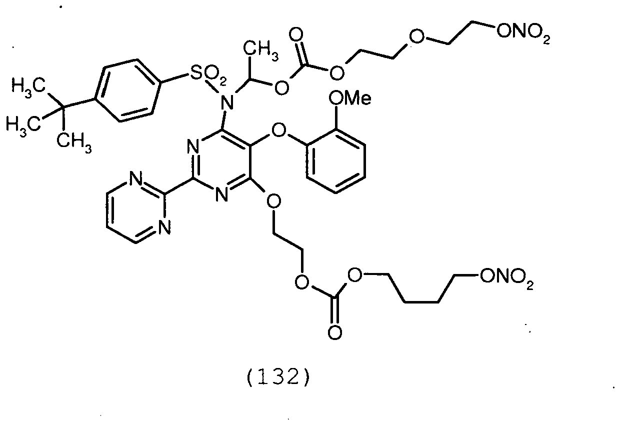

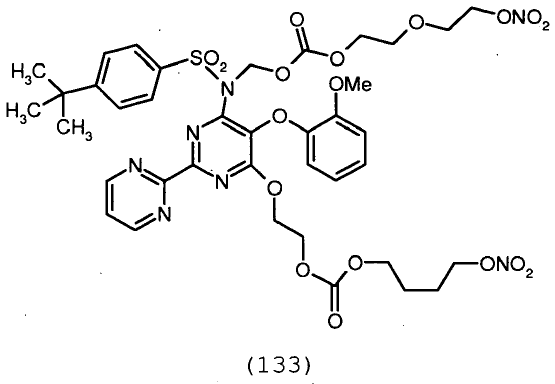

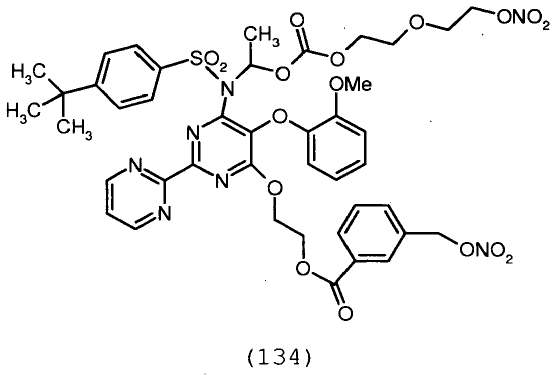

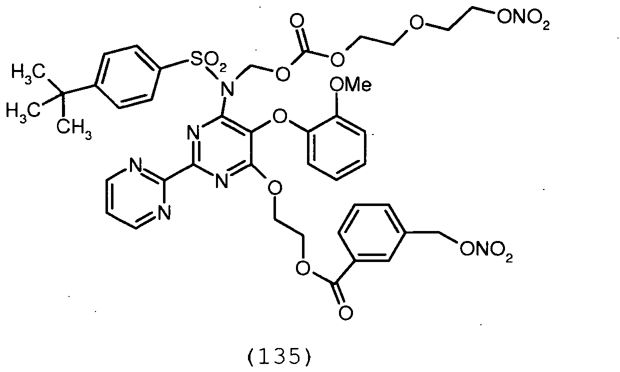

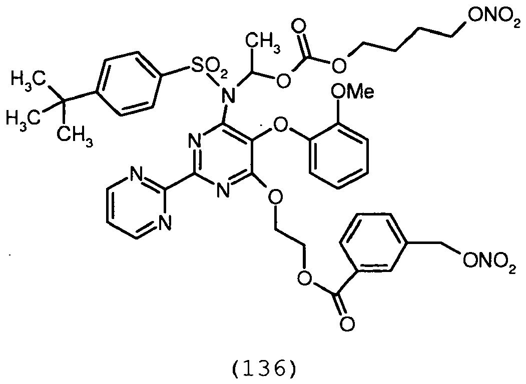

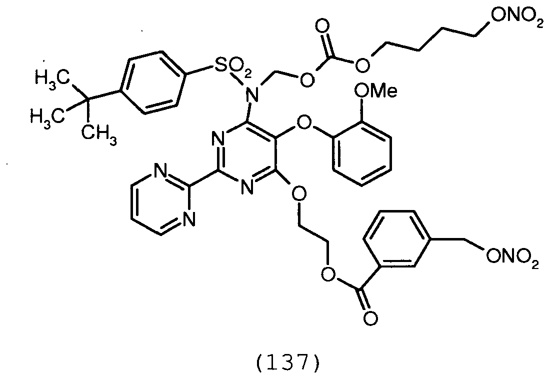

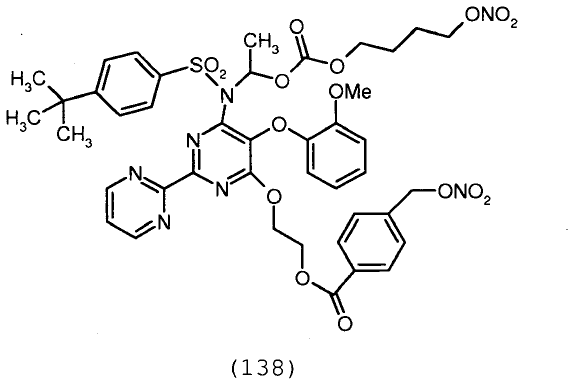

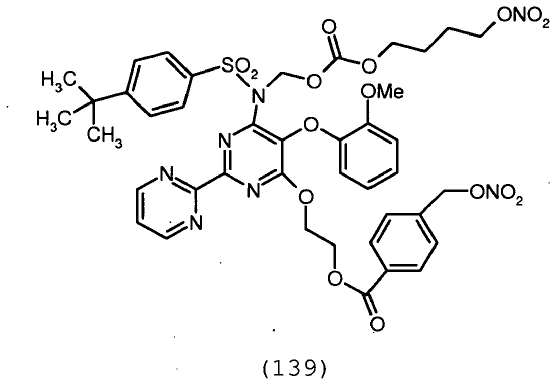

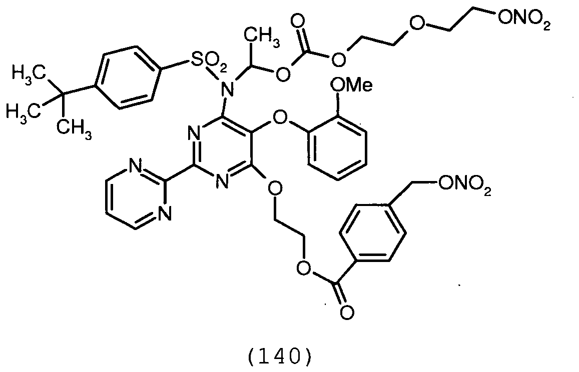

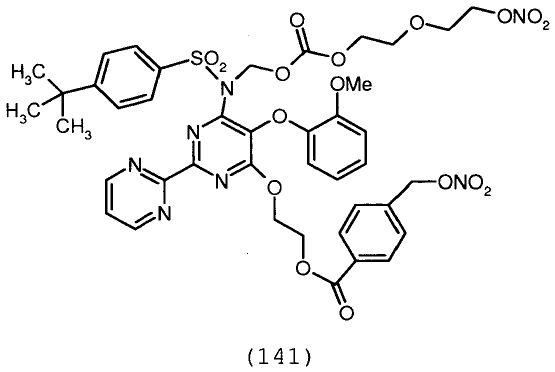

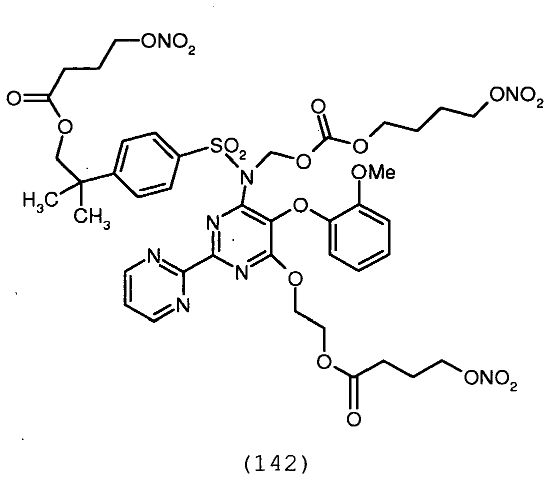

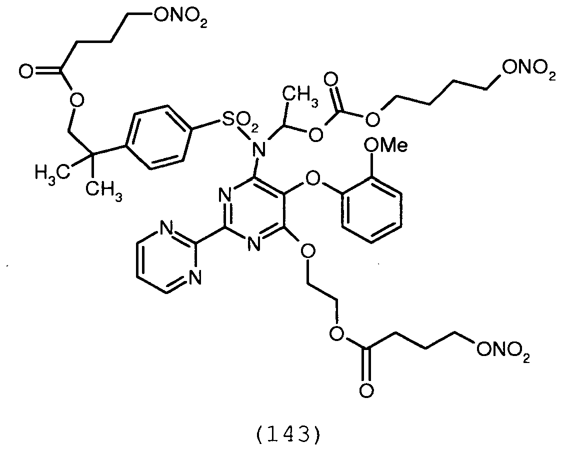

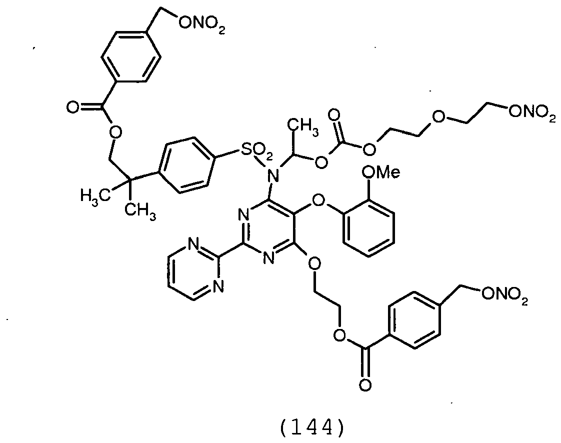

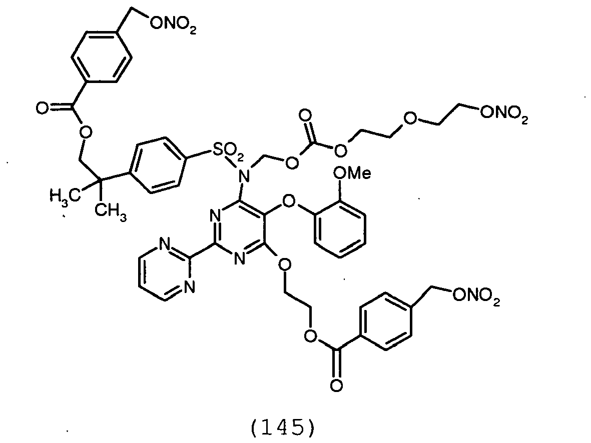

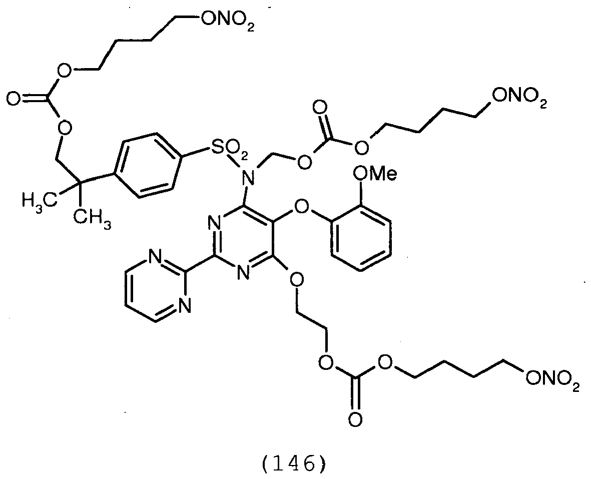

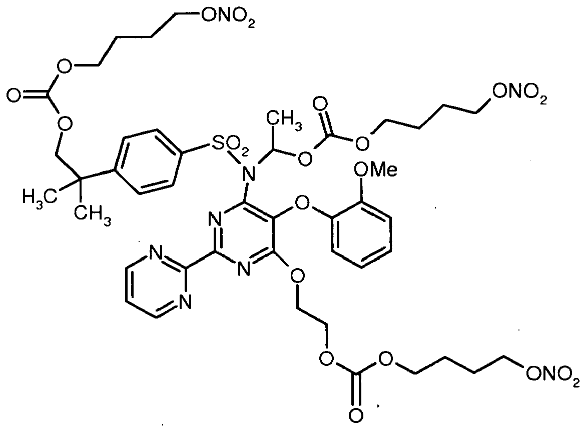

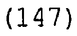

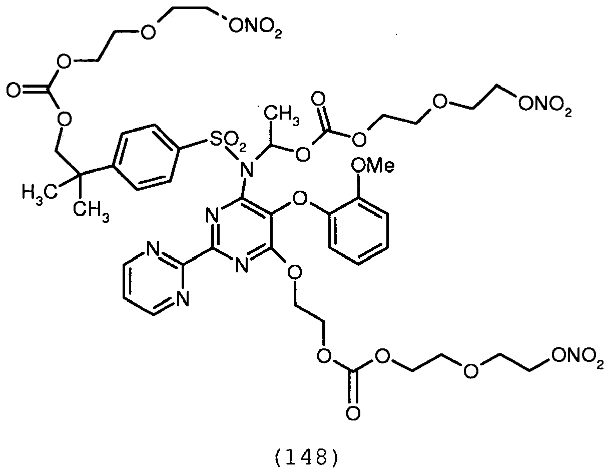

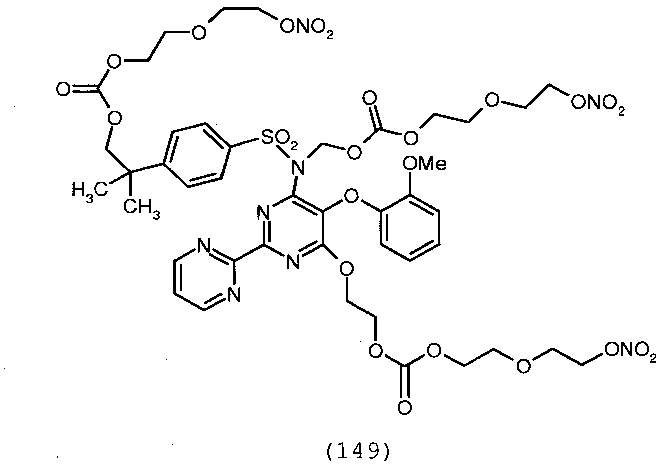

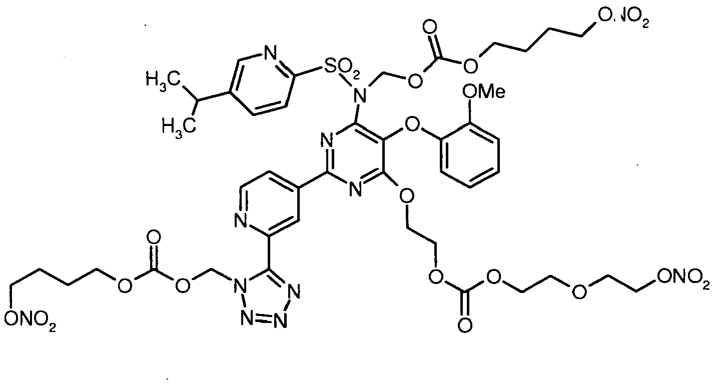

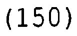

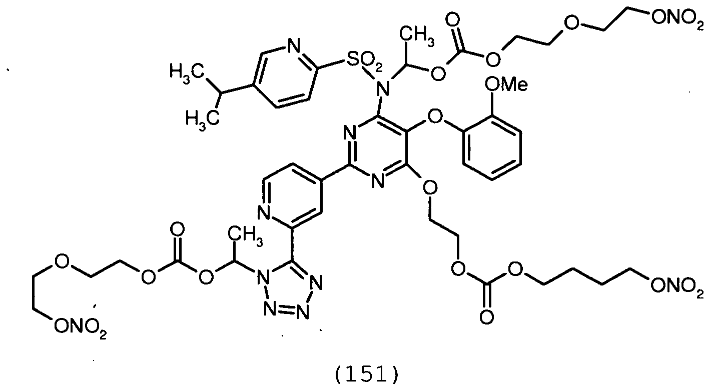

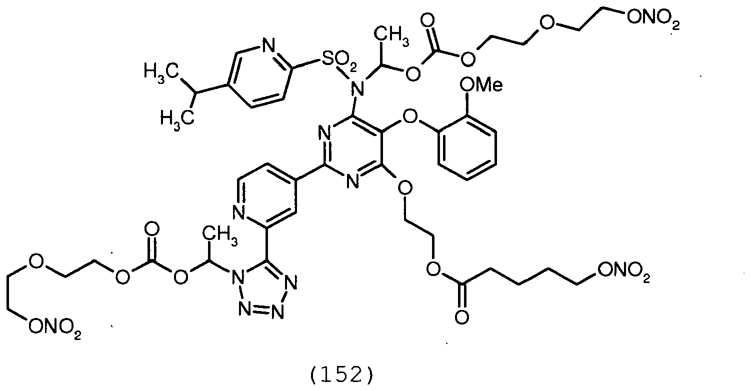

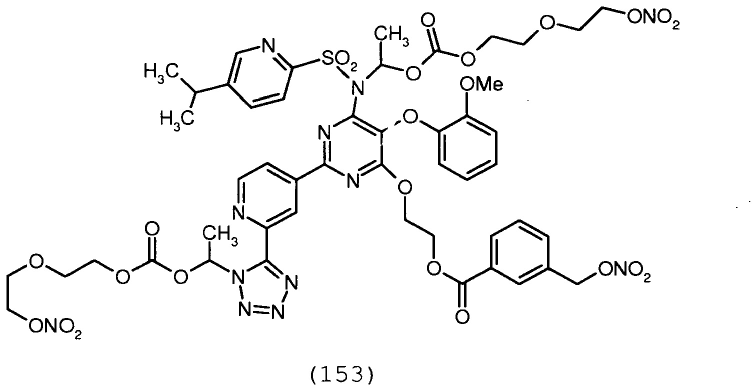

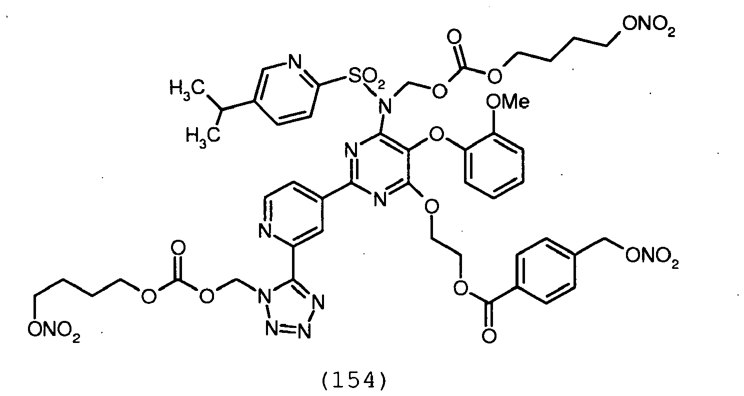

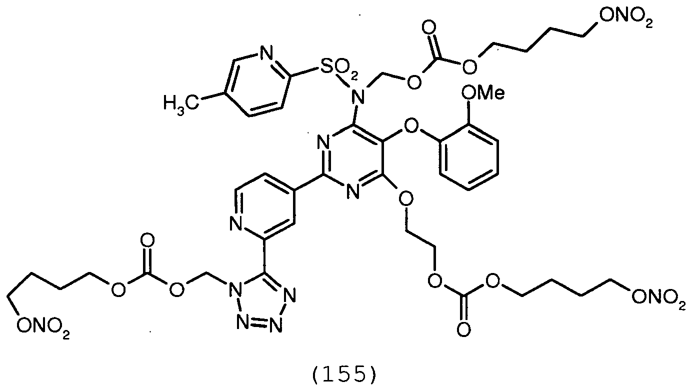

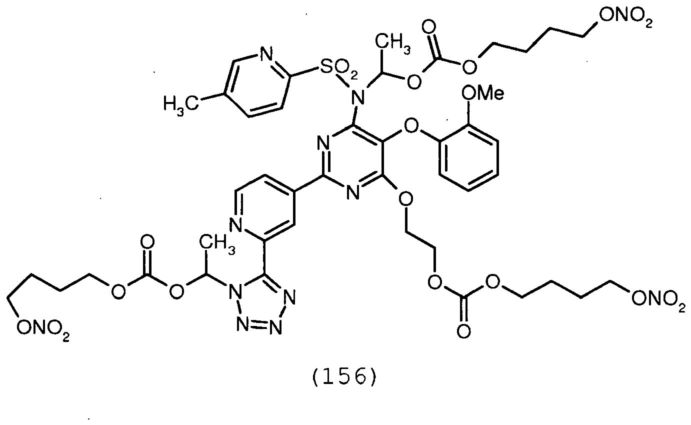

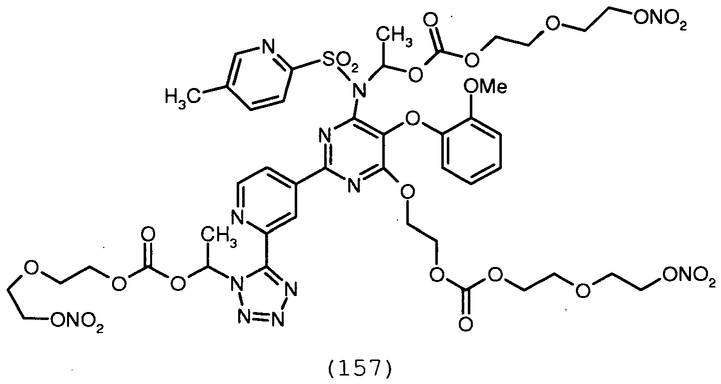

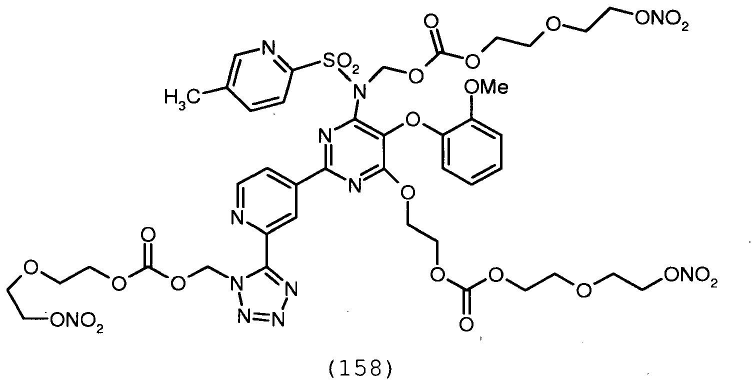

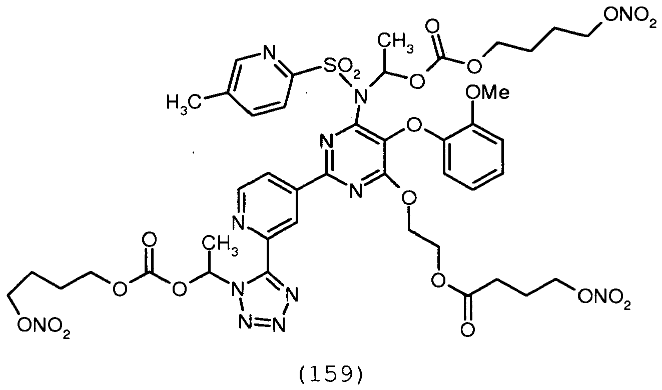

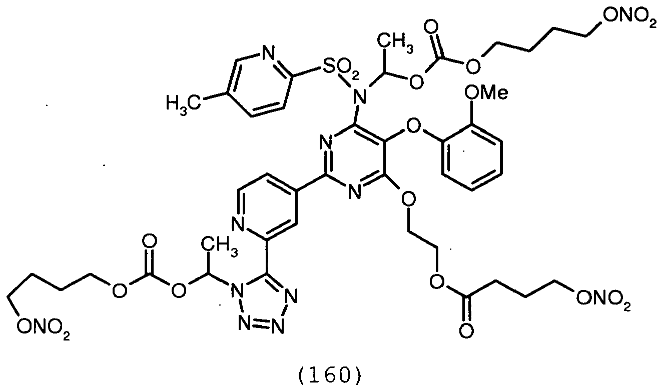

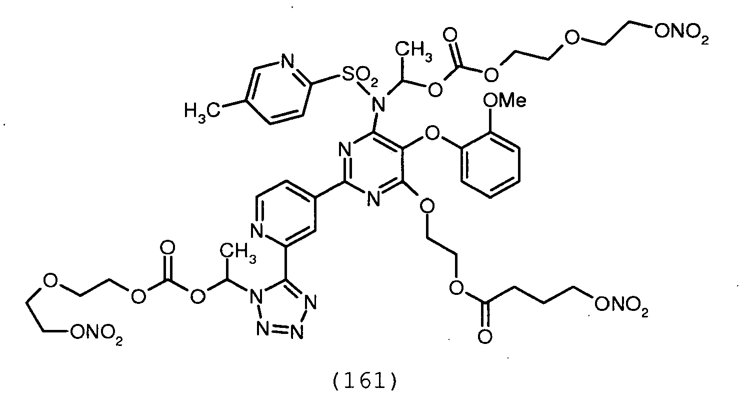

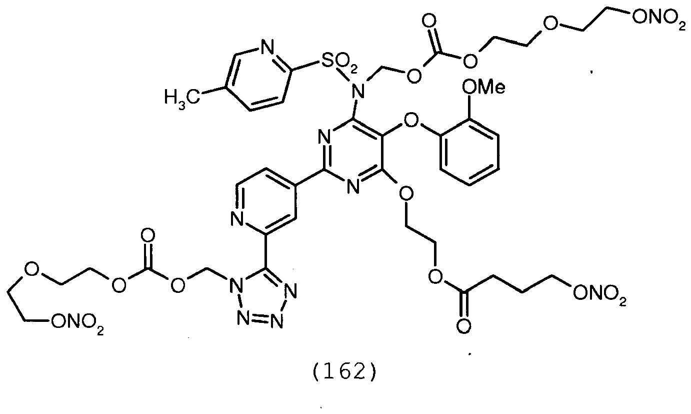

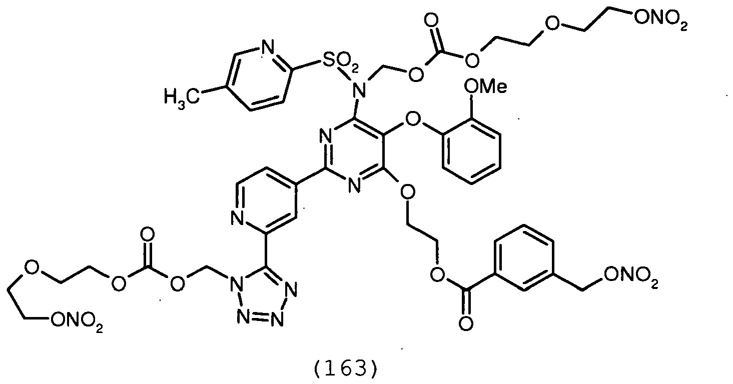

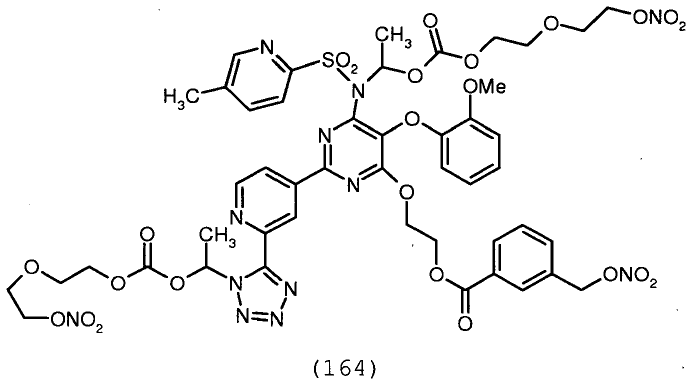

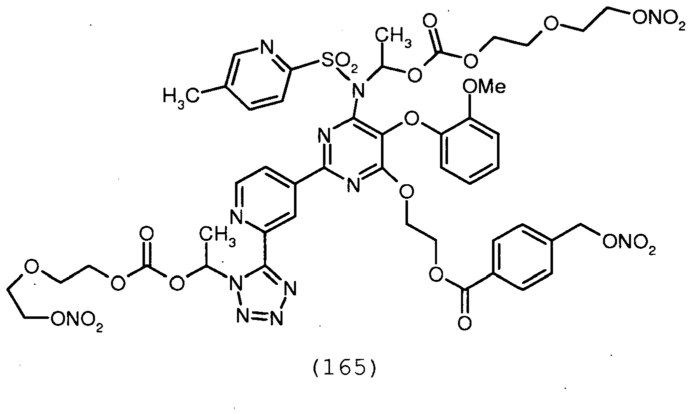

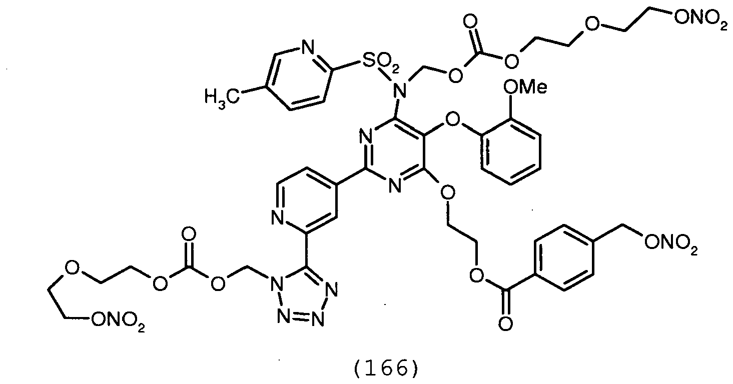

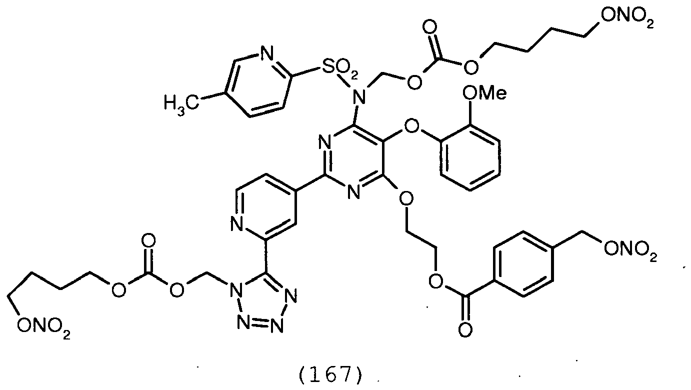

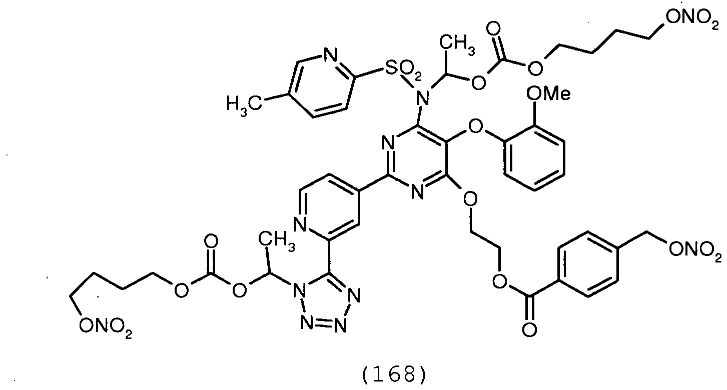

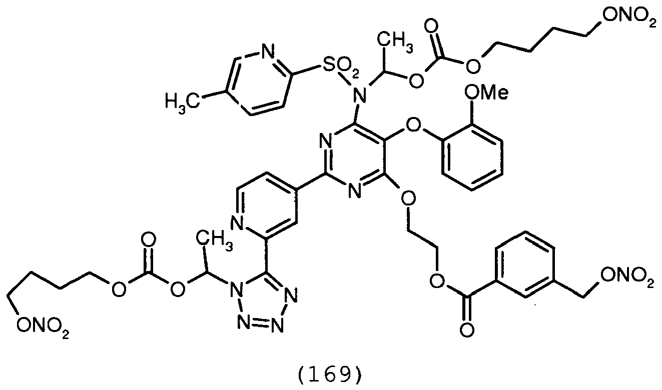

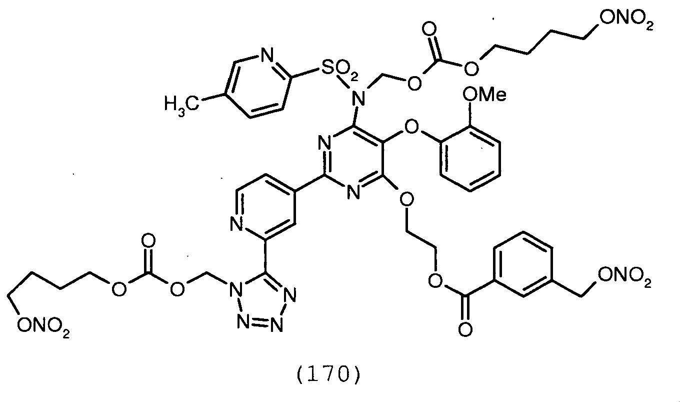

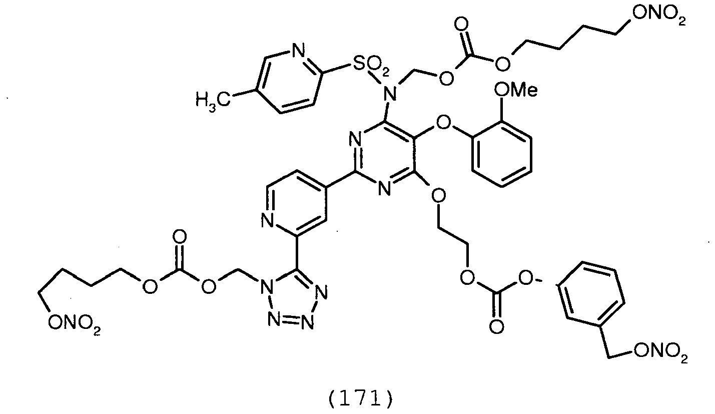

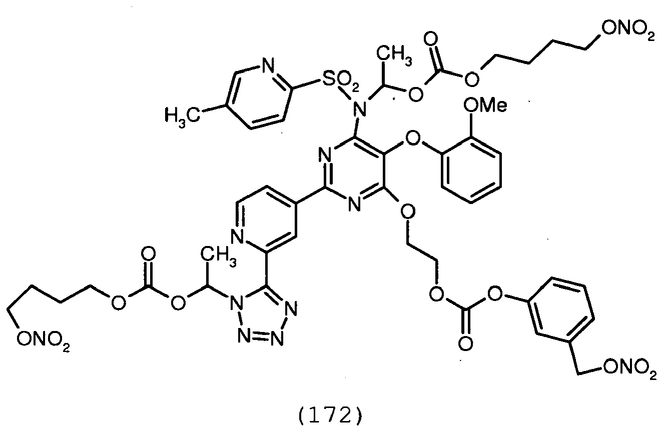

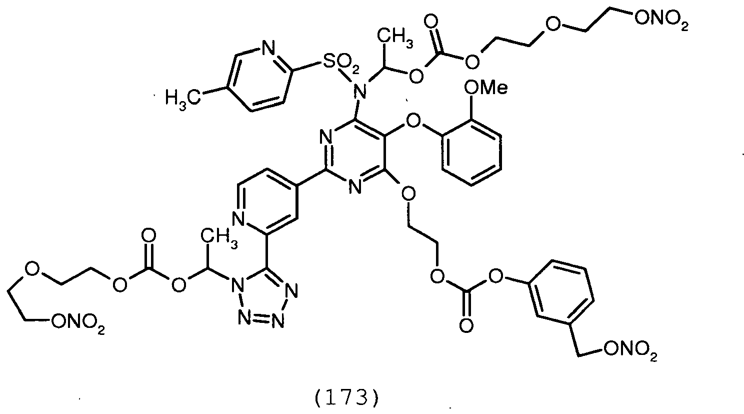

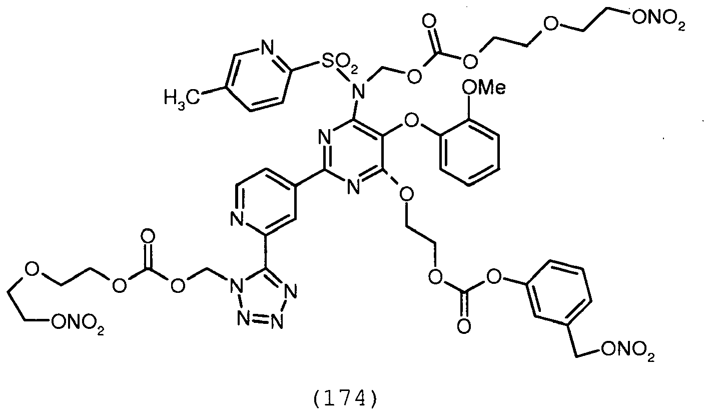

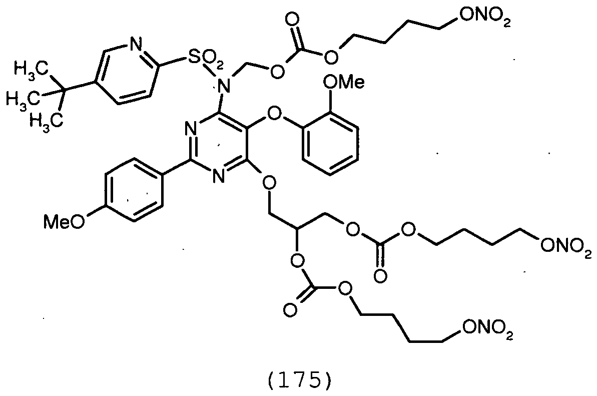

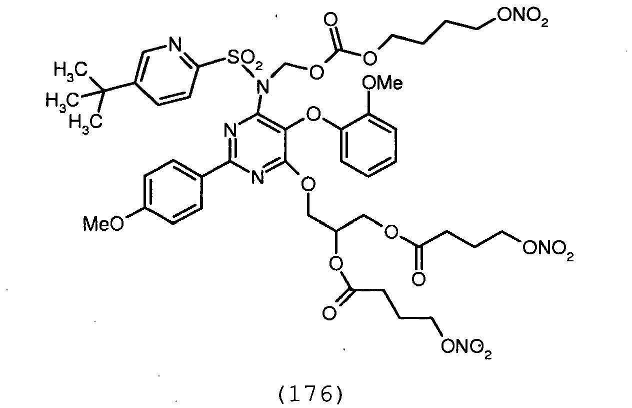

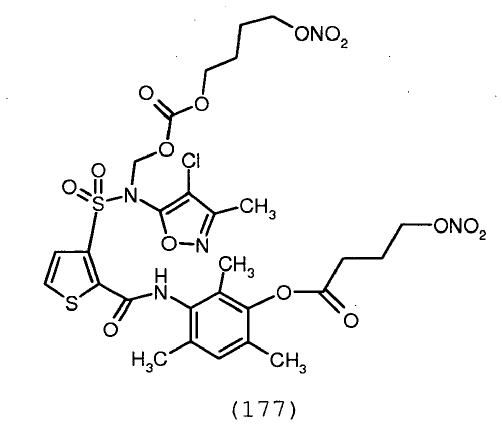

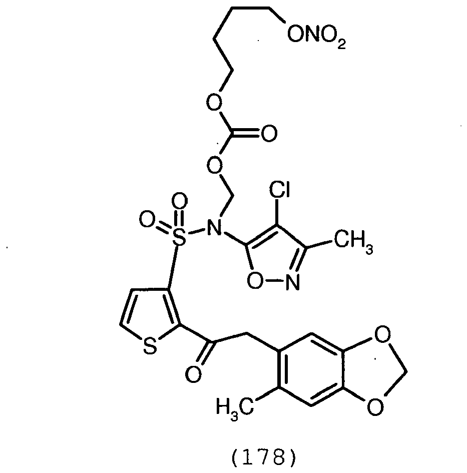

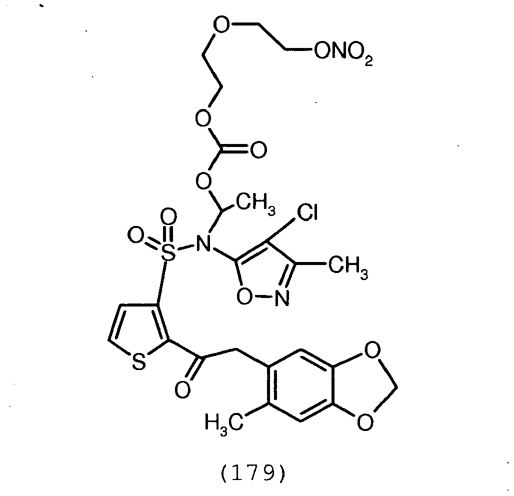

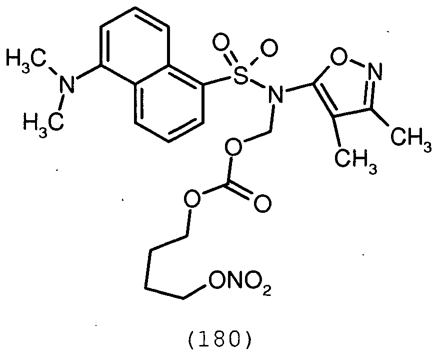

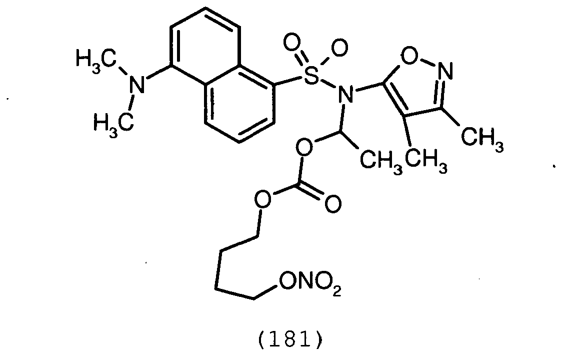

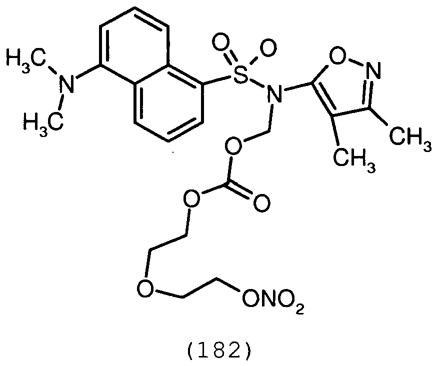

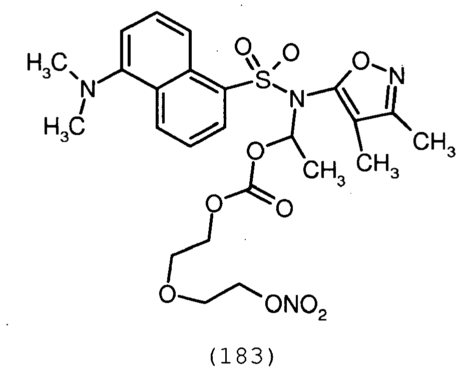

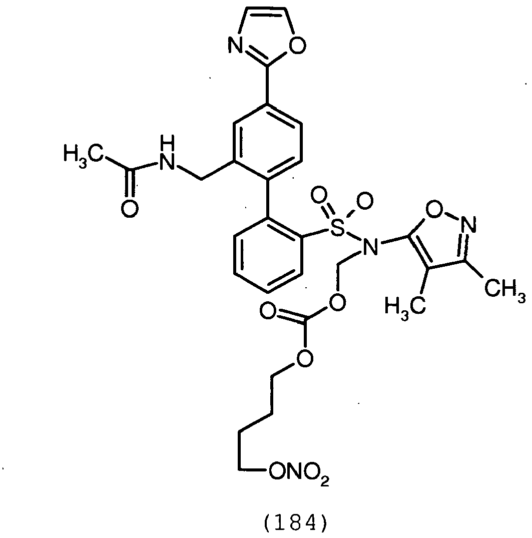

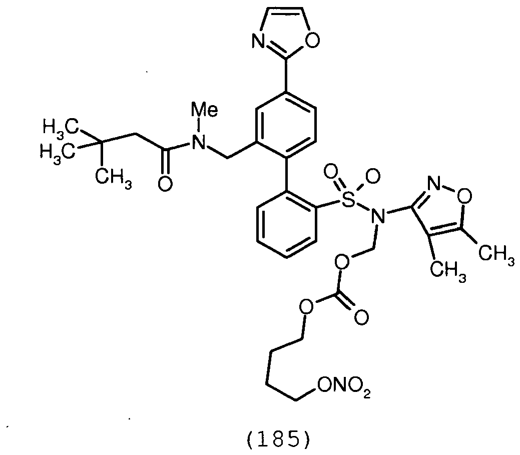

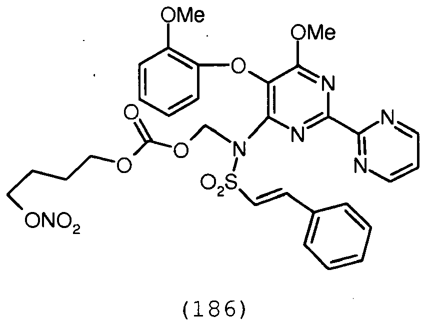

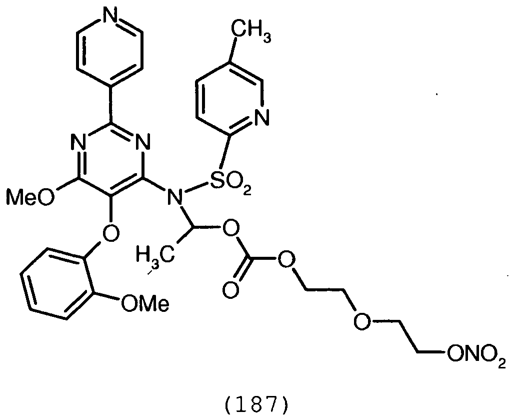

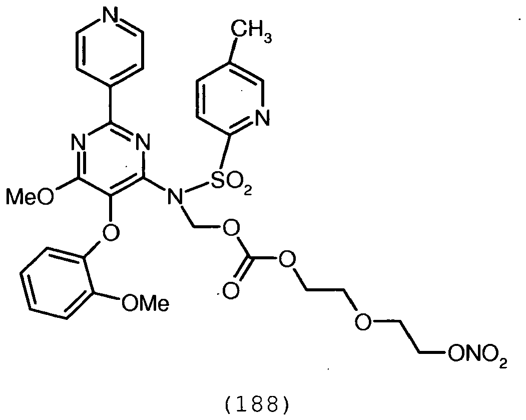

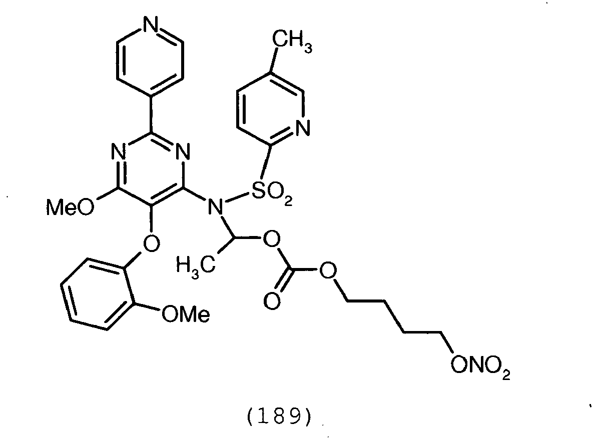

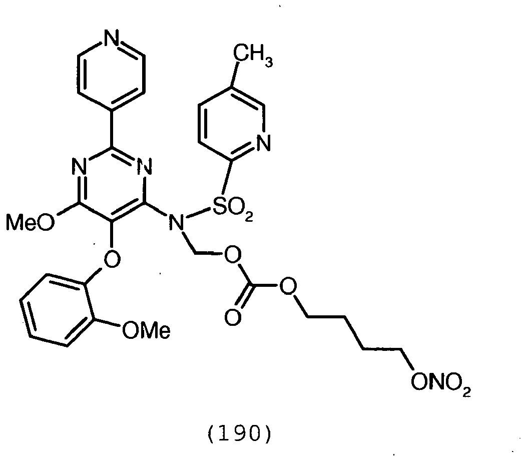

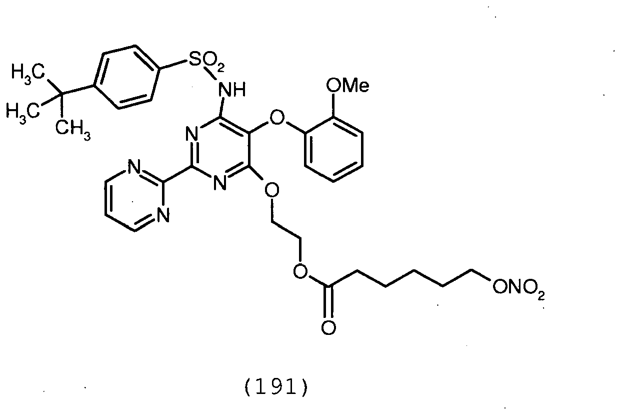

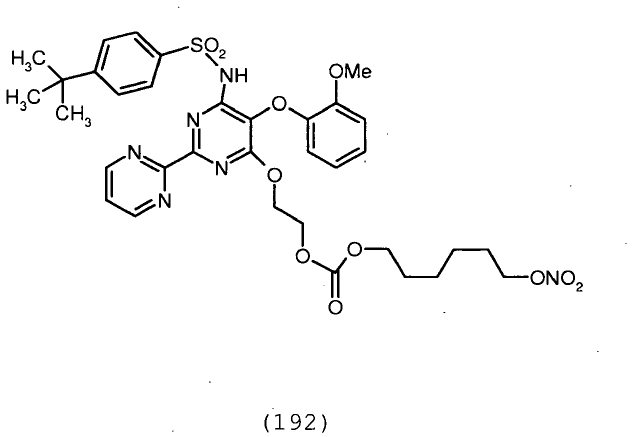

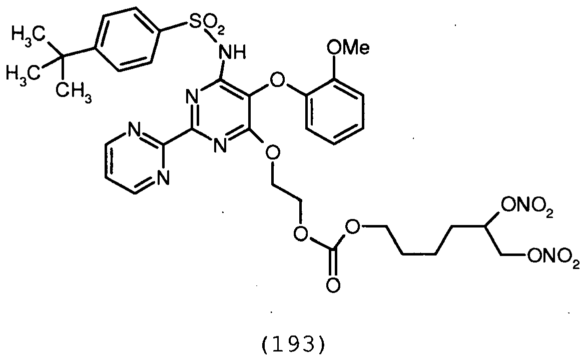

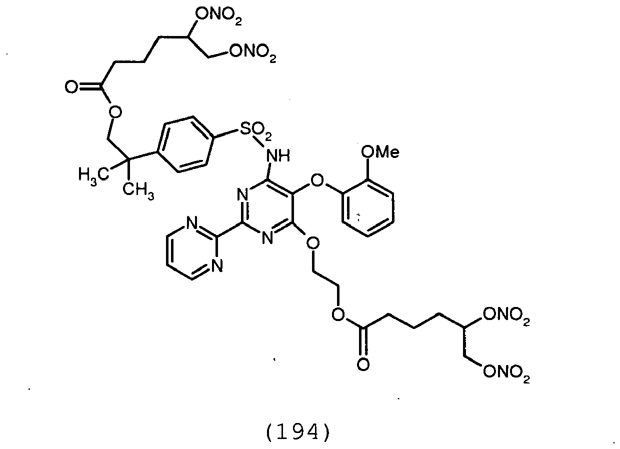

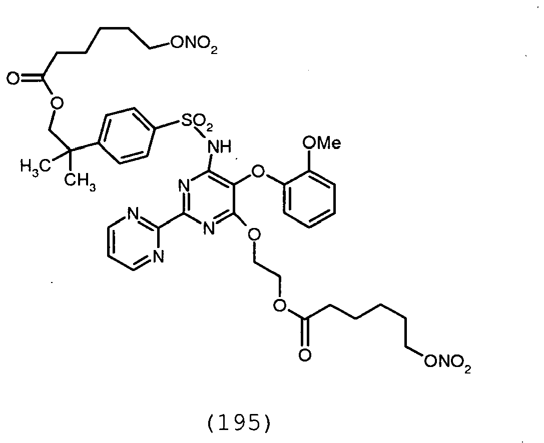

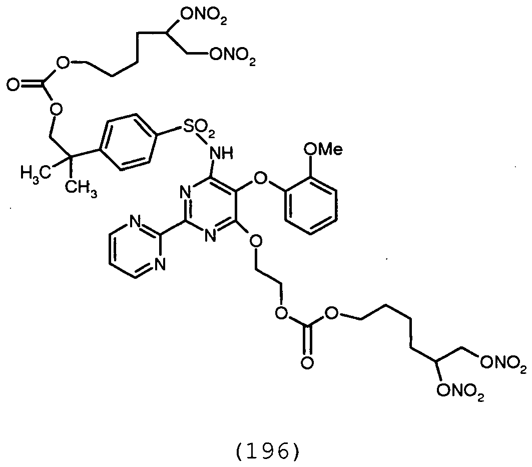

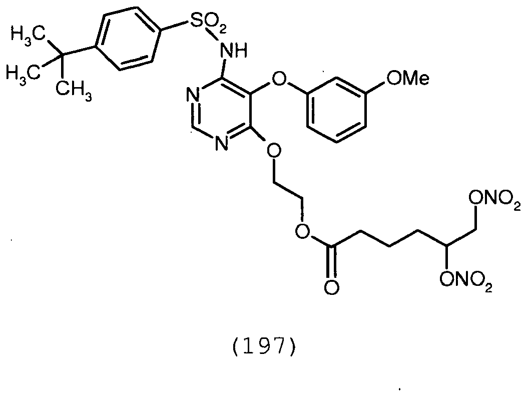

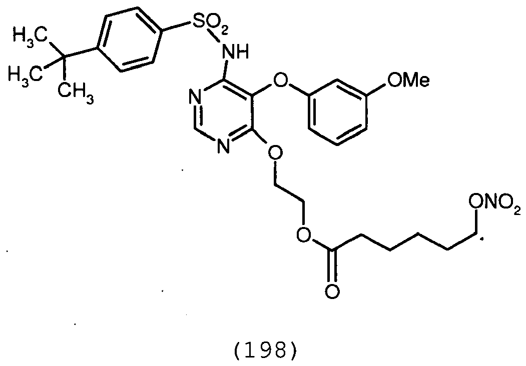

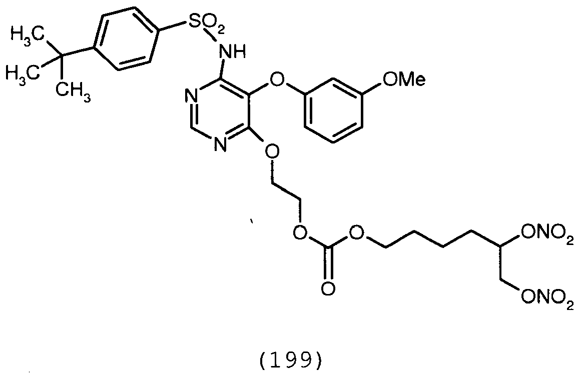

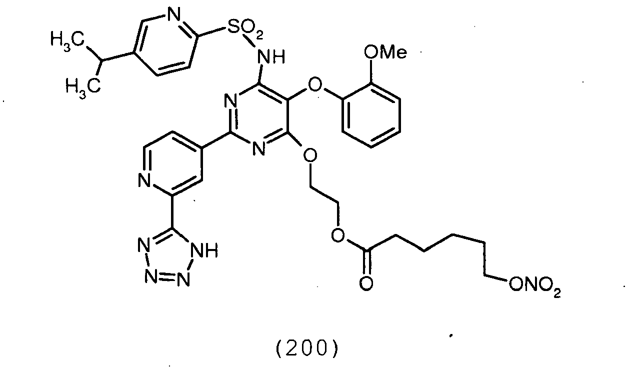

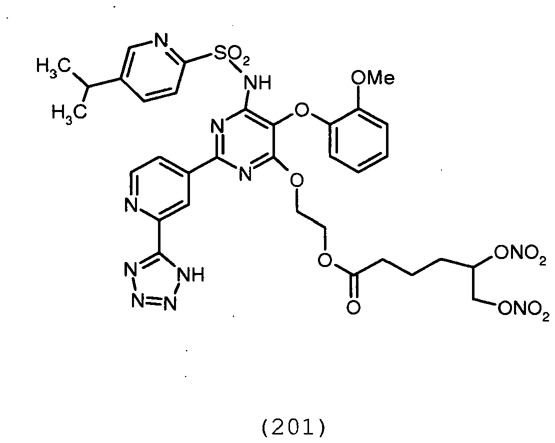

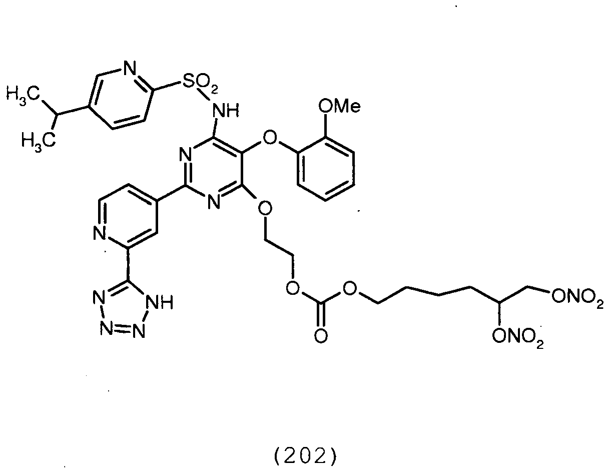

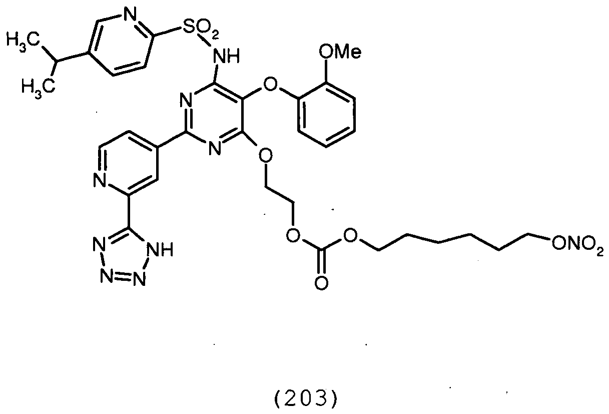

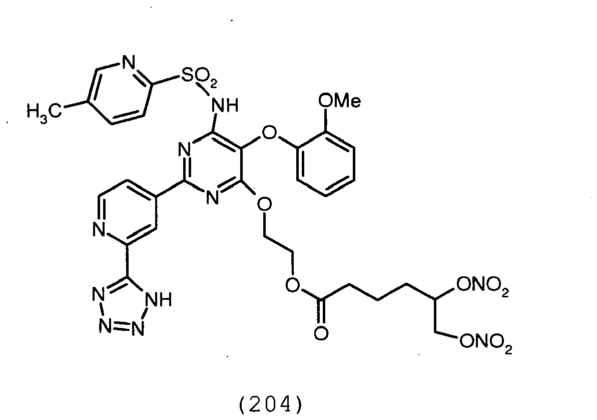

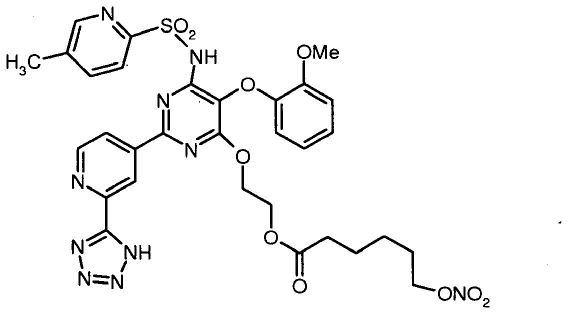

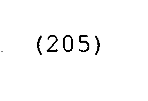

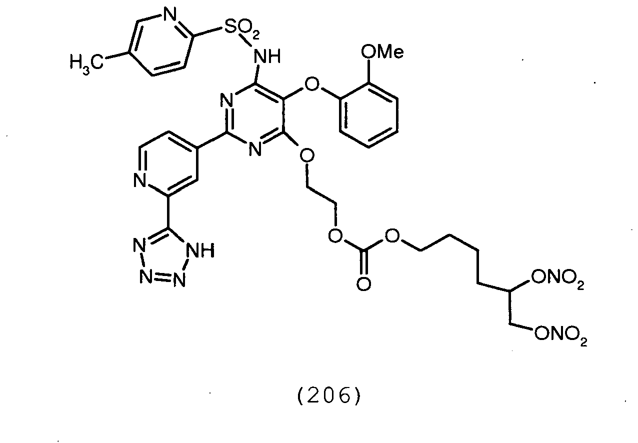

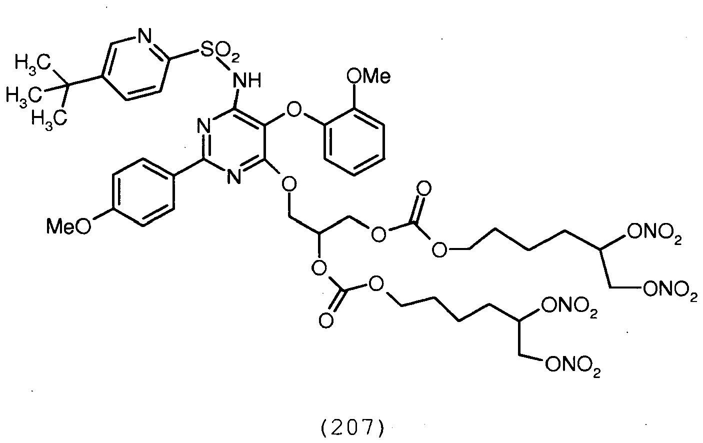

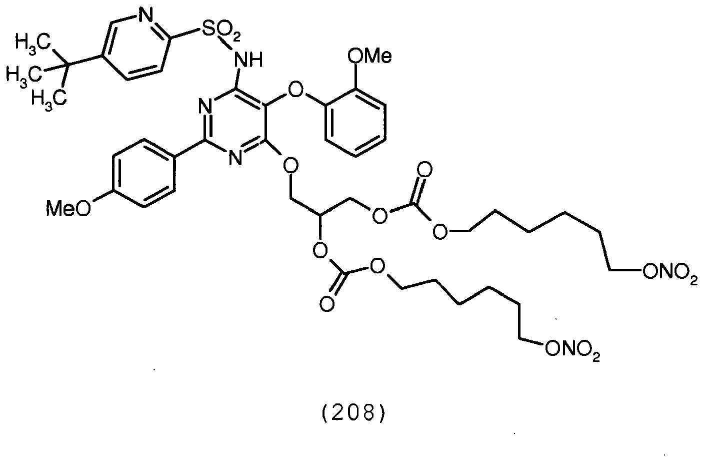

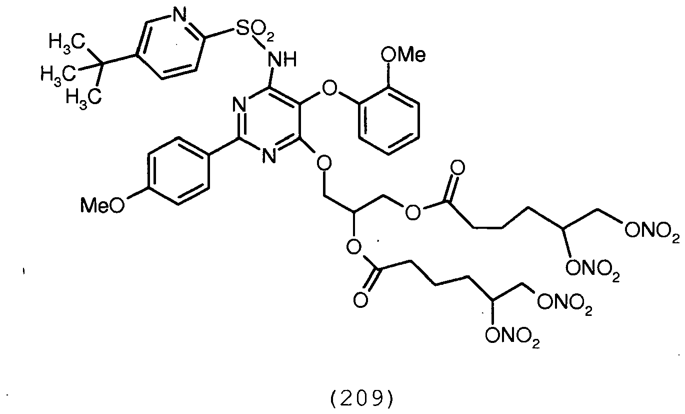

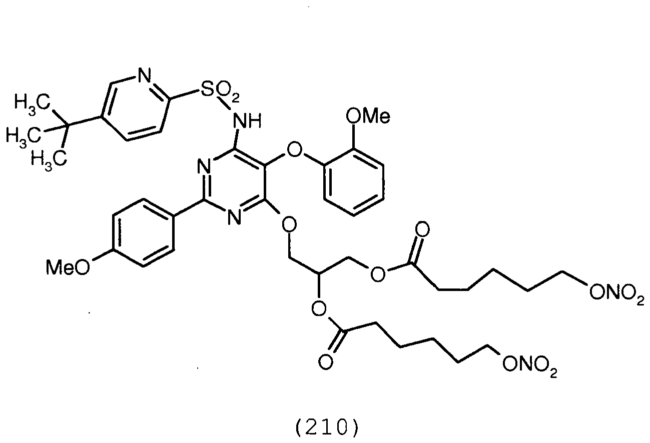

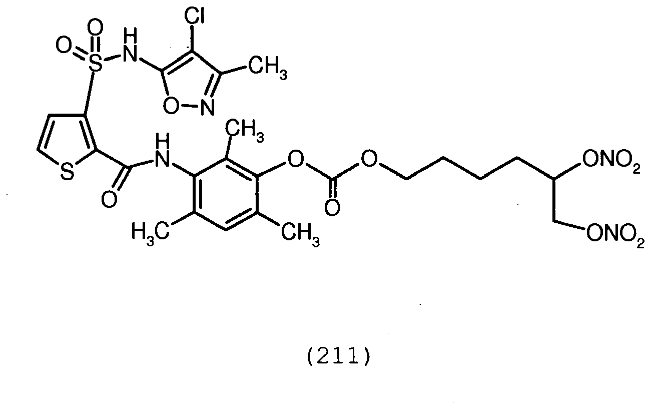

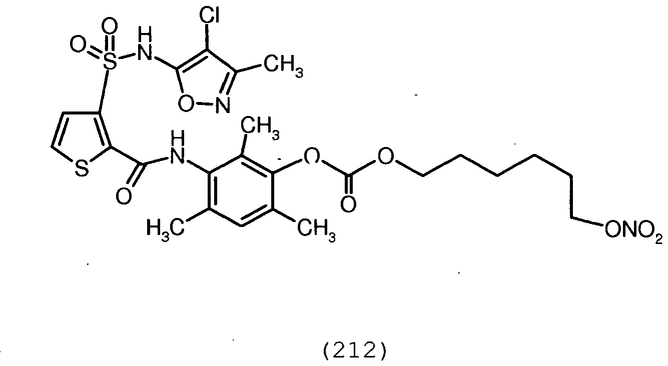

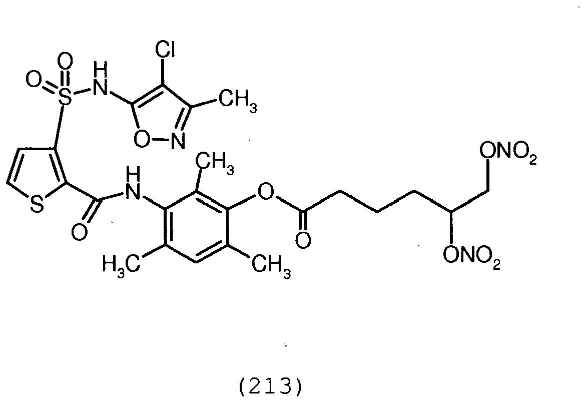

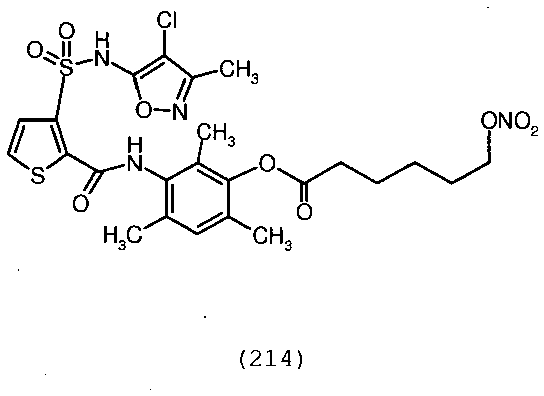

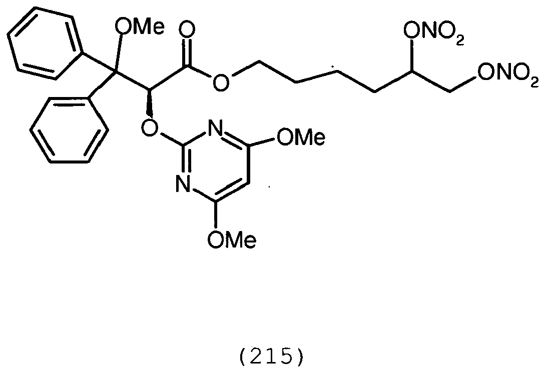

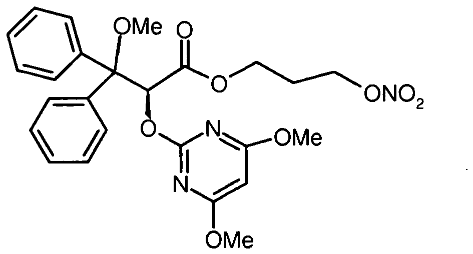

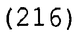

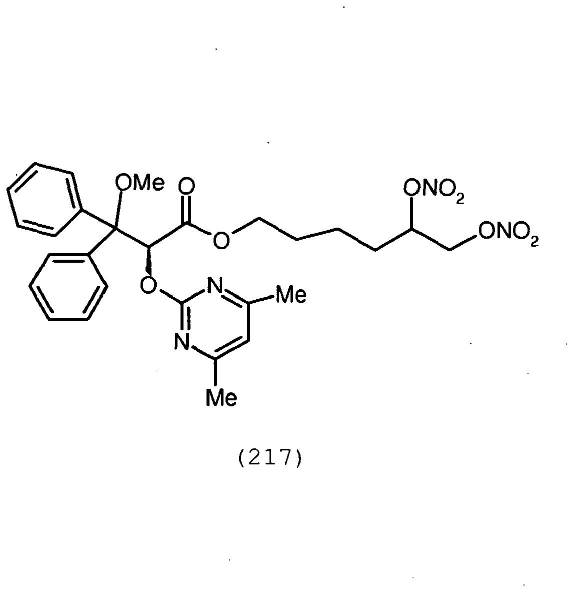

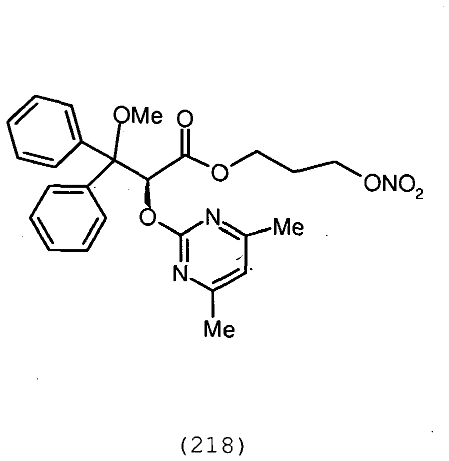

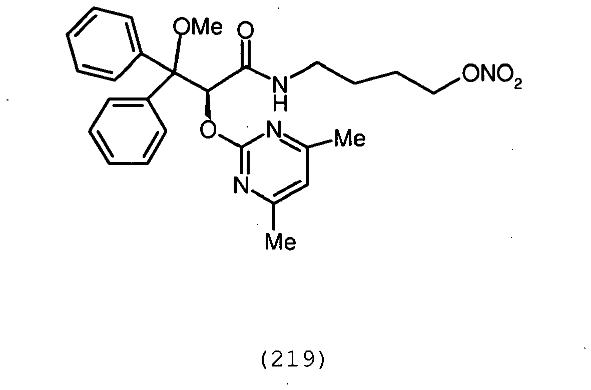

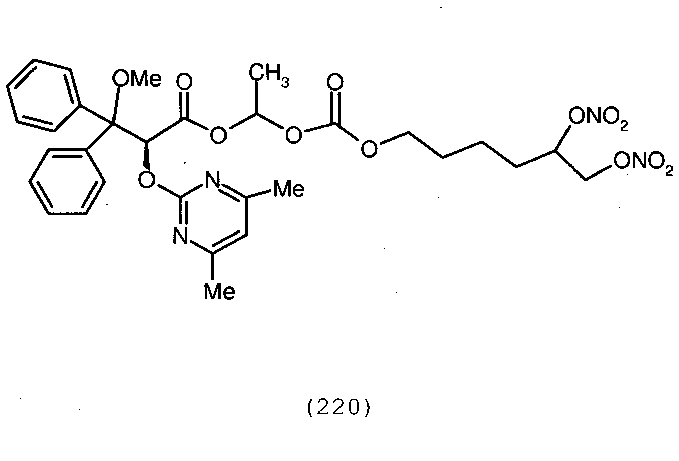

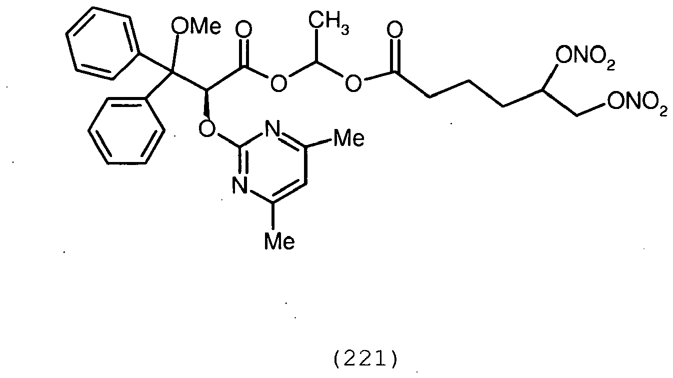

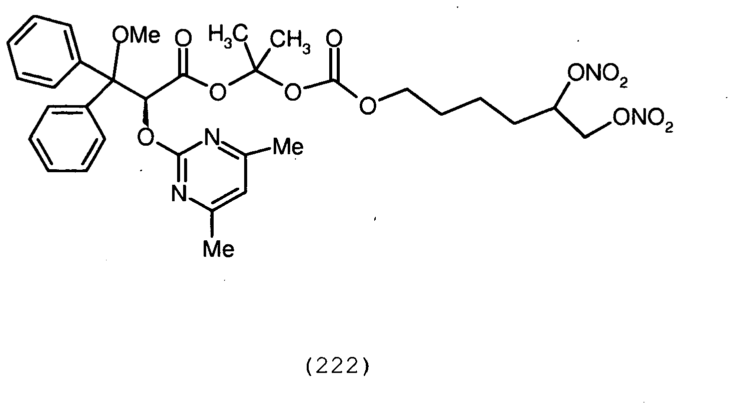

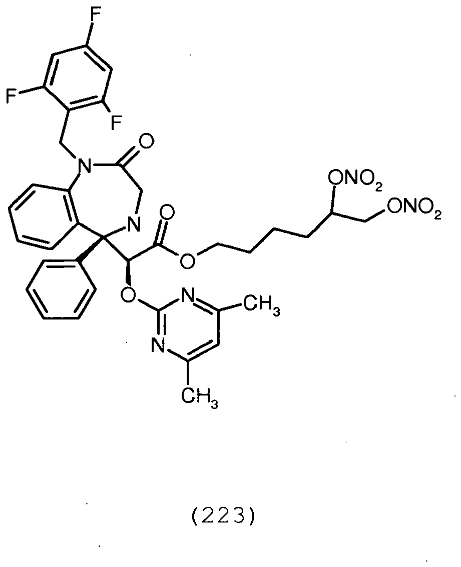

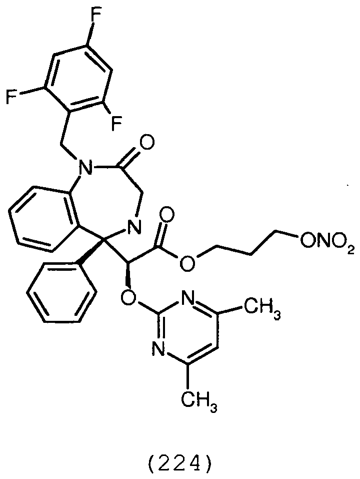

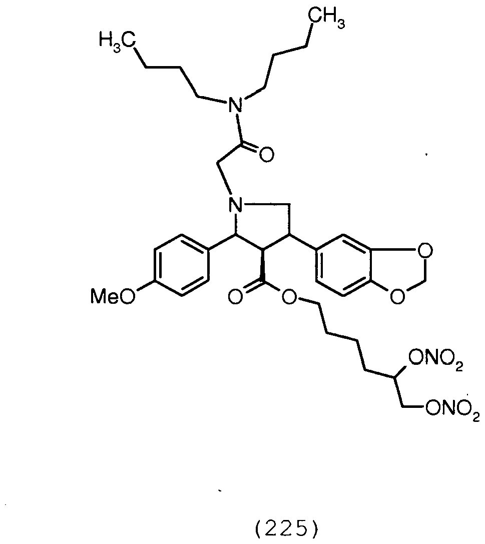

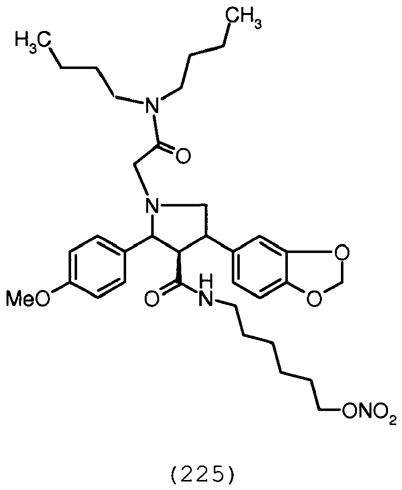

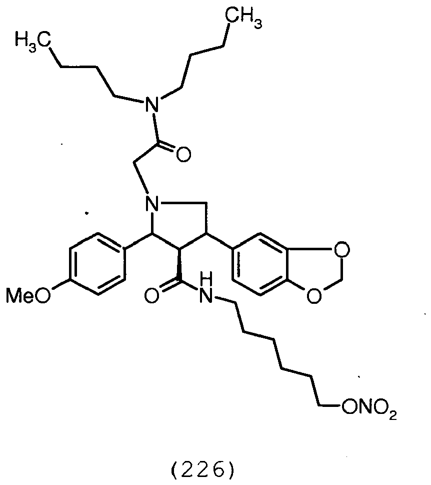

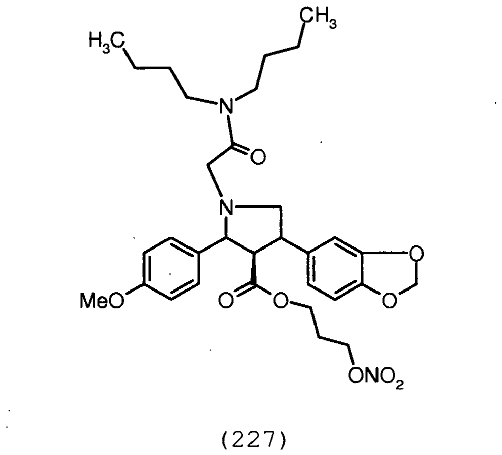

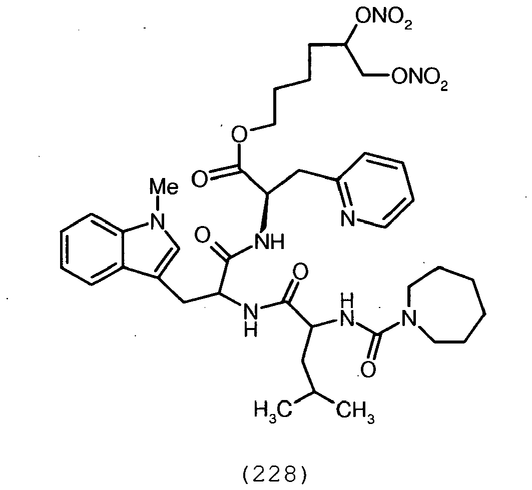

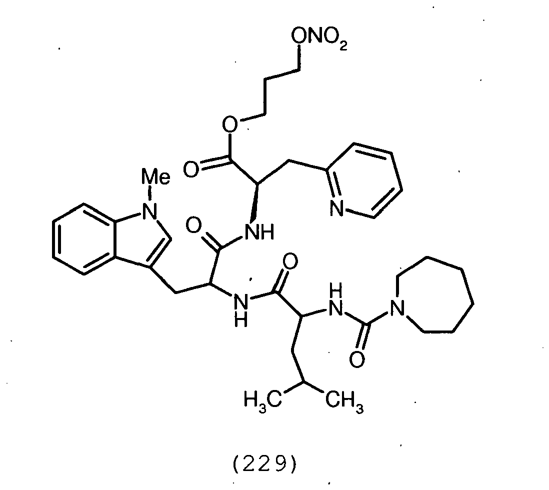

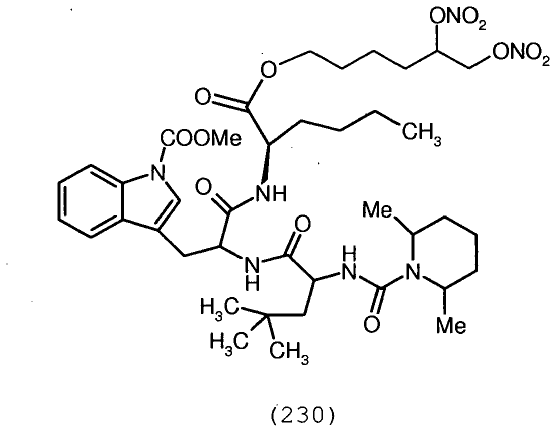

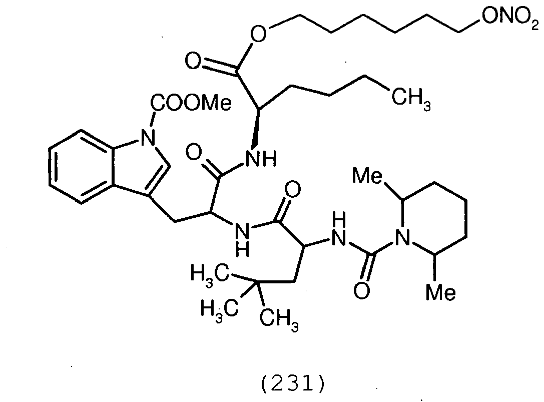

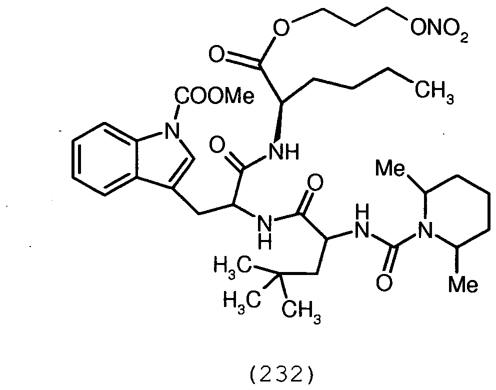

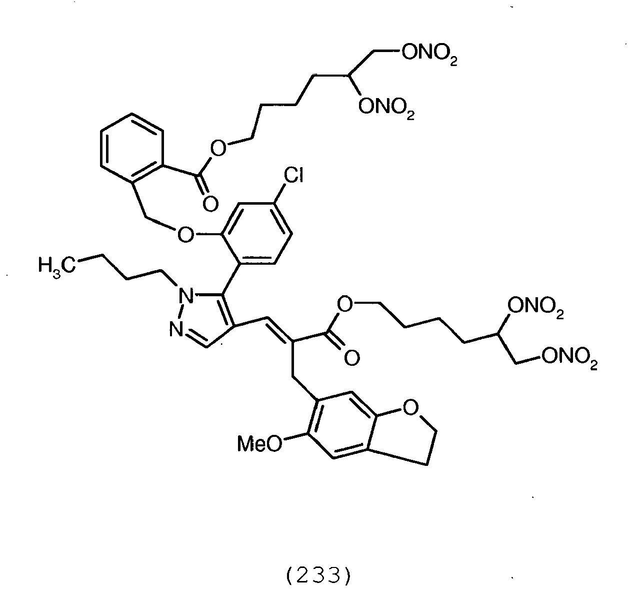

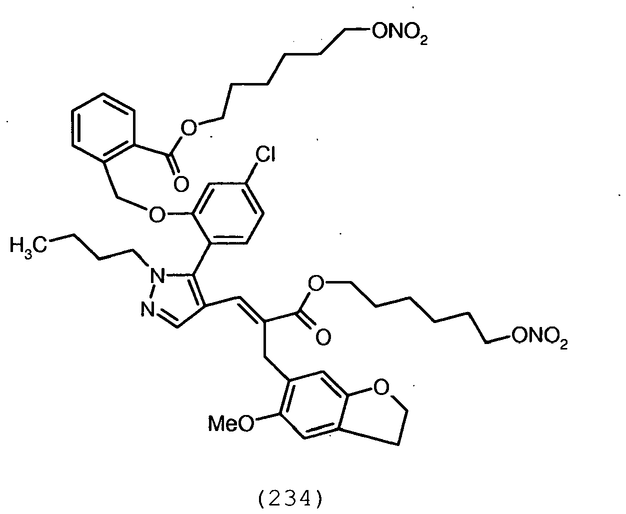

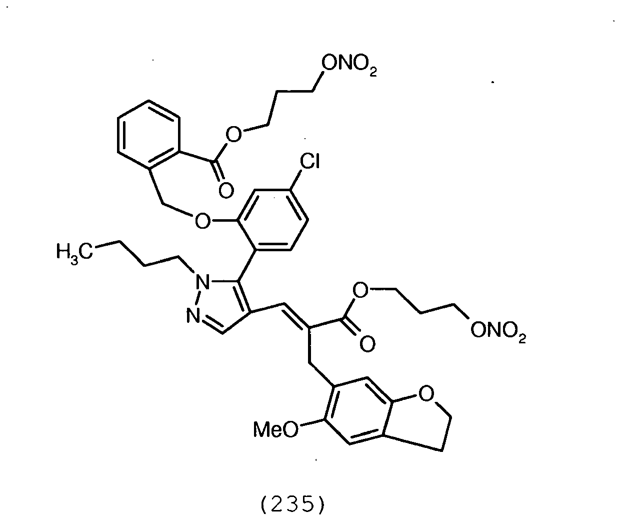

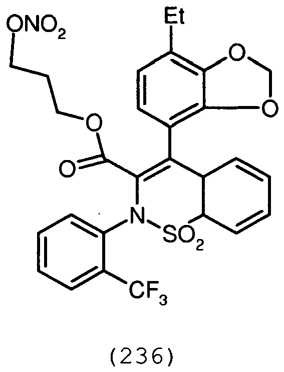

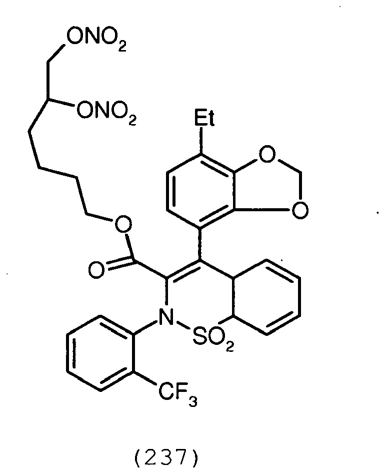

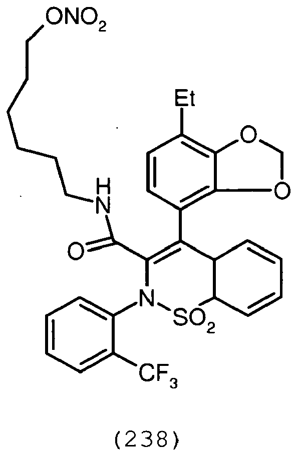

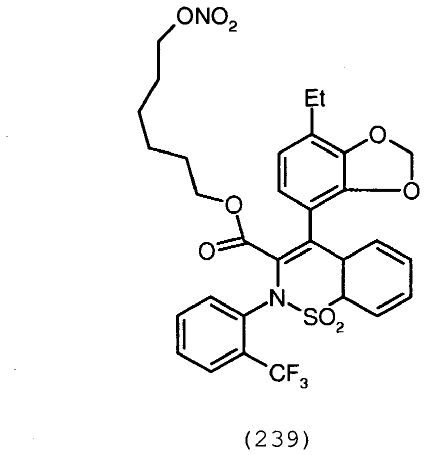

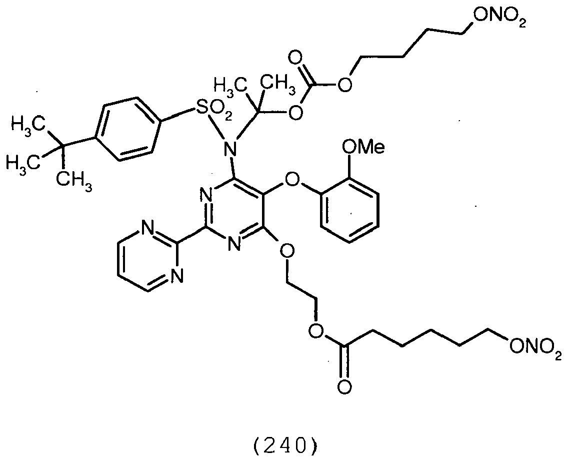

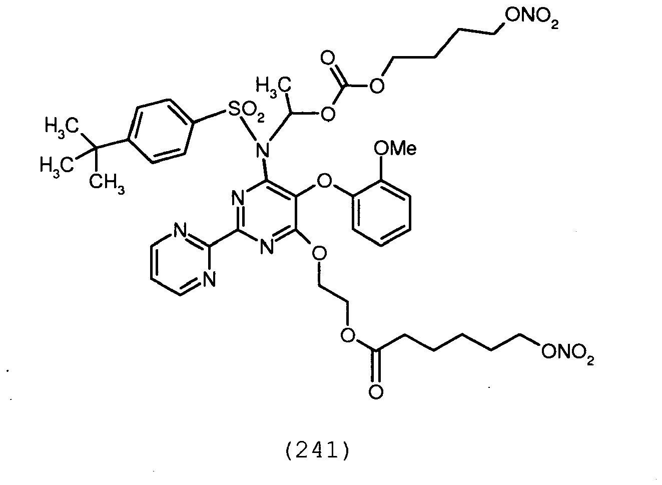

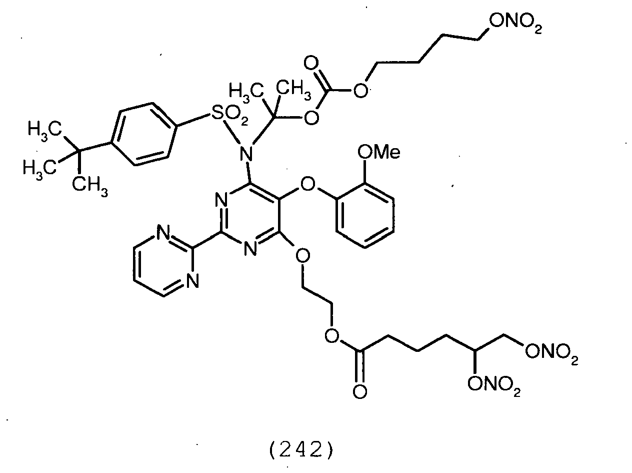

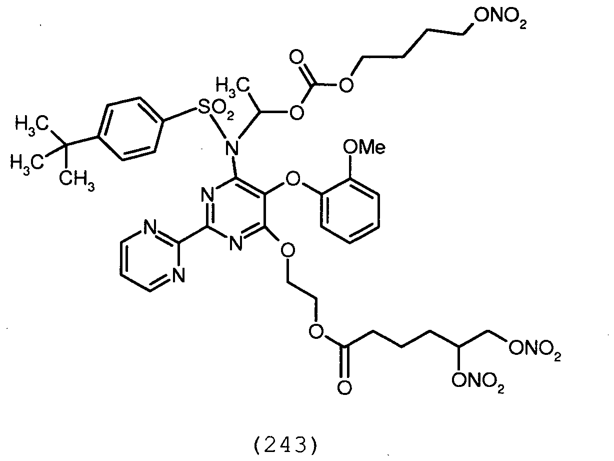

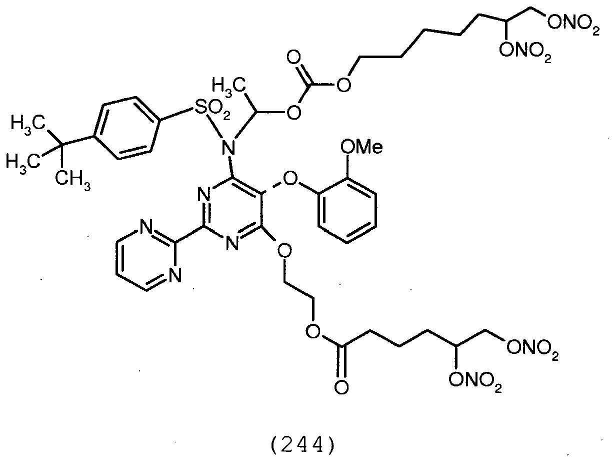

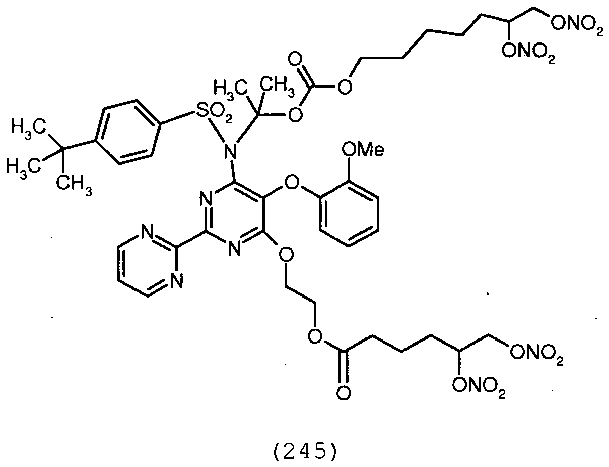

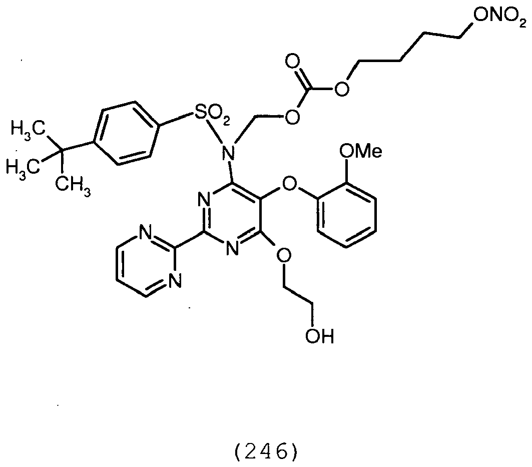

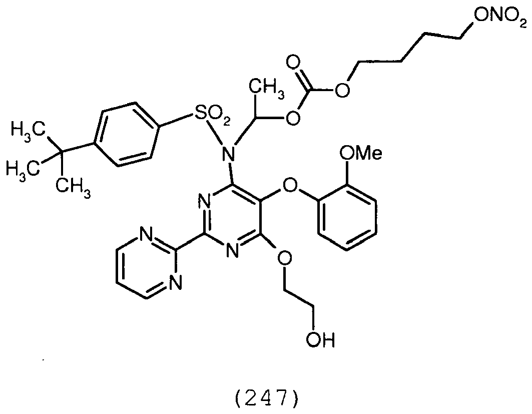

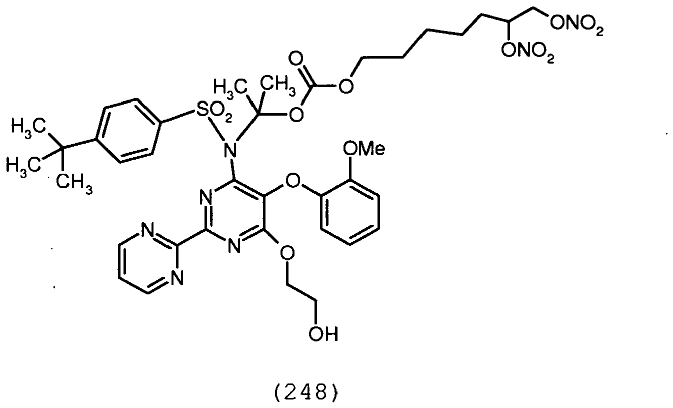

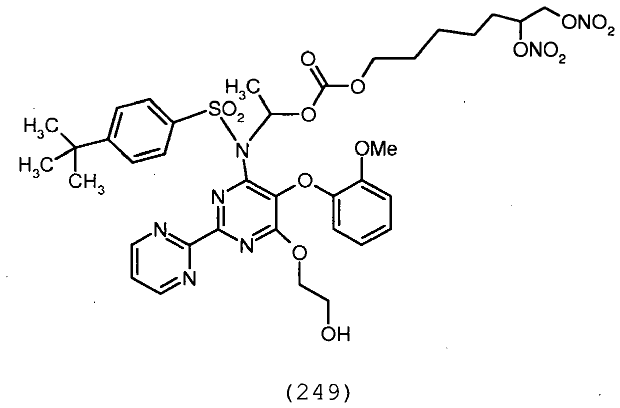

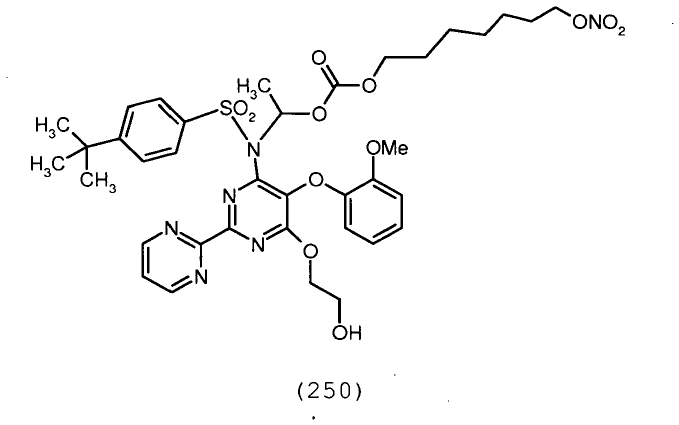

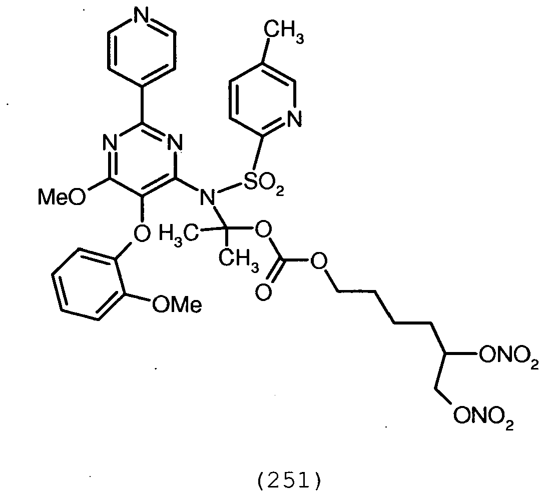

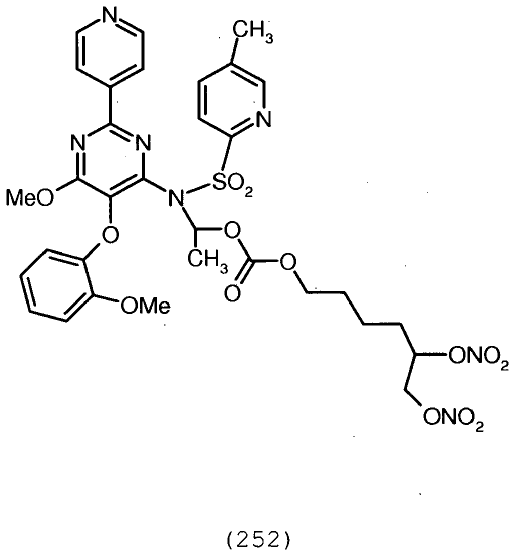

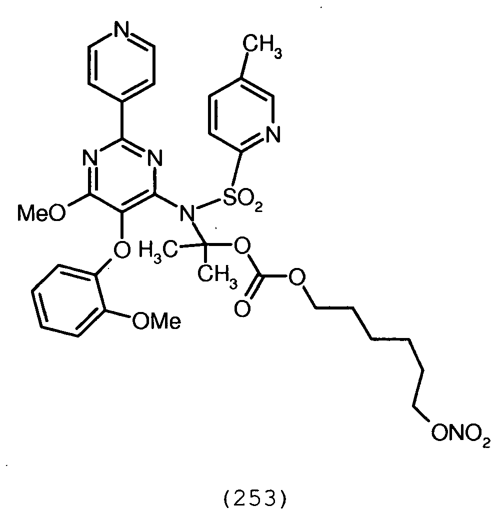

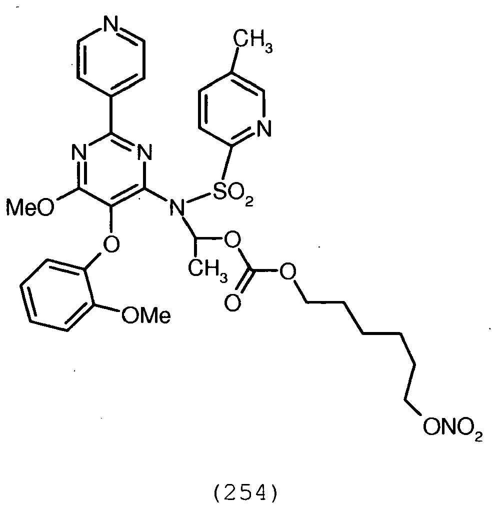

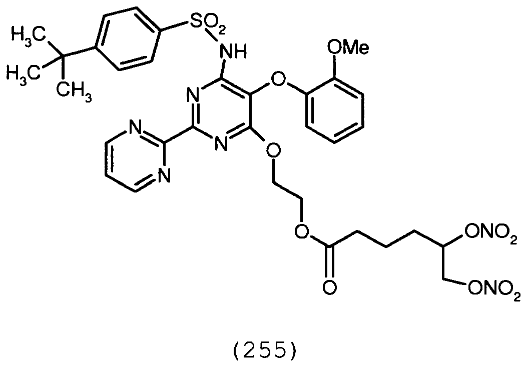

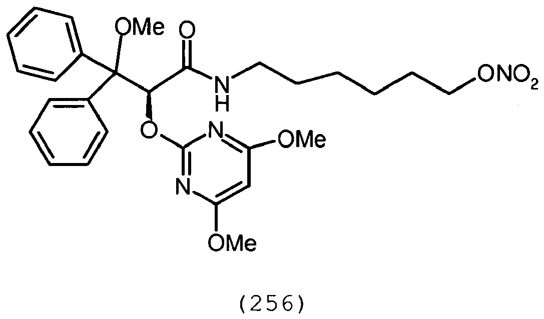

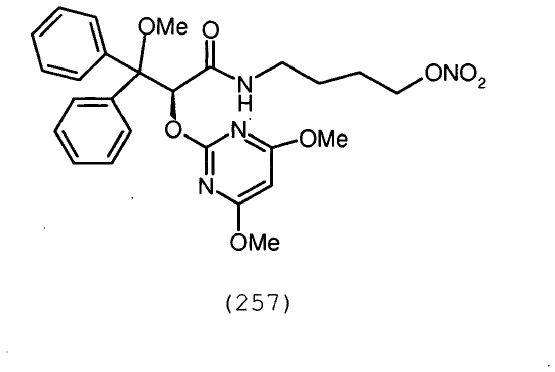

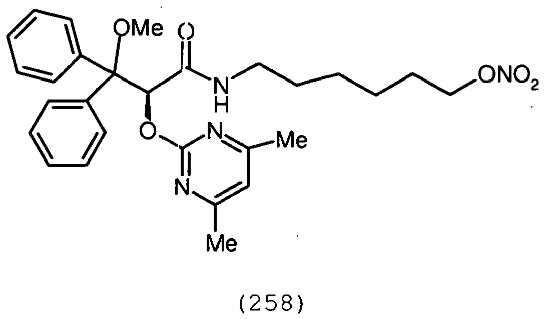

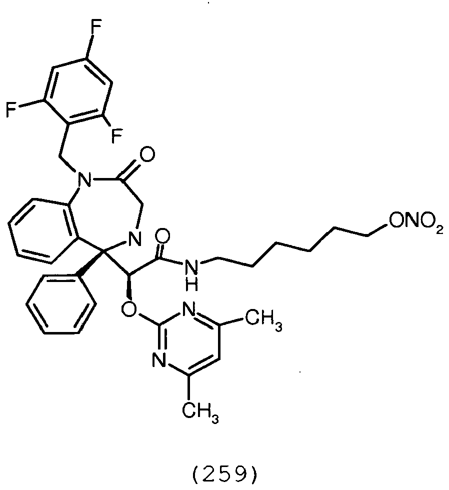

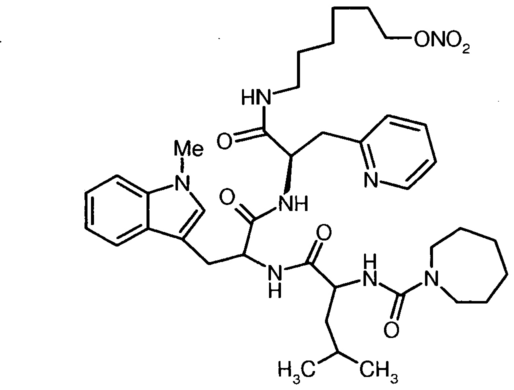

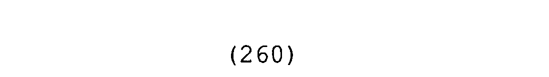

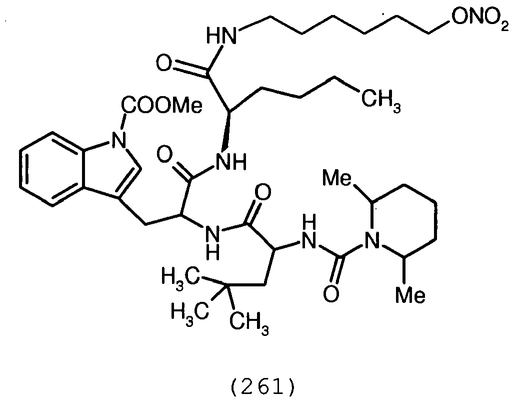

4. A compound of general formula (I) according to claims 1-3 for use as a medicament.

5. Use of a compound according to claims 1-3, for preparing a drug that can be employed in the treatment or prophylaxis of endothelial-related disorders, renal, pulmonary, cardiac and vascular diseases, and inflammatory processes.

6. Use of a compound according to claim 5, for preparing a drug that can be employed in the treatment or prophylaxis of congestive heart failure, coronary diseases, left ventricular dysfunction and hypertrophy, cardiac fibrosis, myocardial ischemia, stroke, subarachnoid hemorrhage, cerebrovascular vasospasm, coronary vasospasm, atherosclerosis, restenosis post angioplasty, renal ischemia, renal failure, renal and pulmonary fibrosis, glomerulonephritis, renal colic, ocular hypertension, glaucoma, systemic hypertension, pulmonary arterial hypertension (PAH), diabetic complications such as nephropathy, vasculopathy and neuropathy, peripheral vascular diseases, liver fibrosis, portal hypertension, metabolic syndromes, erectile dysfunction; complications after vascular or cardiac surgery, complications of treatment with immunosuppressive agents after organ transplantation, hyperaldosteronism, lung fibrosis, scleroderma, sickle cell disease, benign prostatic hyperplasia, cancer, anxiety, cognitive disorders.

7. A pharmaceutical composition comprising a pharmaceutically acceptable carrier and a pharmaceutically effective amount of a compound of general formula (I) or a salt or stereoisomer thereof according to claims 1-3.

8. A pharmaceutical composition according to claim 7 in a suitable form for the oral, parenteral, rectal, topic and transdermic administration, by inhalation spray or aerosol or iontophoresis devices.

9. Liquid or solid pharmaceutical composition for oral, parenteral, rectal, topic and transdermic administration or inhalation in the form of tablets, capsules and pills eventually with enteric coating, powders, granules, gels, emulsions, solutions, suspensions, syrups, elixir, injectable forms, suppositories, in transdermal patches or liposomes, containing a compound of formula (I) or a salt or stereoisomer thereof according to claims 1-3 and a pharmaceutically acceptable carrier.

10. A pharmaceutical composition comprising a compound of general formula (I) according to claims 1-3, at least a compound used to treat cardiovascular disease and a pharmaceutically acceptable carrier.

11. Pharmaceutical composition according to claim 10 wherein the compound used to treat cardiovascular disease is selected from the group consisting of: aldosterone antagonists, angiotensin II receptor blockers, ACE inhibitors, HMGCoA reductase inhibitors, beta-adrenergic blockers, alpha-adrenergic antagonists, sympatholytics, calcium channel blockers, renin inhibitors, neutral endopeptidase inhibitors, potassium activators, diuretics, vasodilators, antithrombotics such as aspirin or nitrosated compounds thereof.

12. A pharmaceutical kit comprising a compound of general formula (I) as defined in claim 1, a compound used to treat cardiovascular disease as combined preparation for simultaneous, separated or sequential use for the treatment of cardiovascular disease.

13. A pharmaceutical kit according to claim 12 wherein the compound used to treat cardiovascular disease is selected from the group consisting of: aldosterone antagonists, angiotensin II receptor blockers, ACE inhibitors, HMGCoA reductase inhibitors, beta-adrenergic blockers, alpha-adrenergic antagonists, sympatholytics, calcium channel blockers, renin inhibitors, neutral endopeptidase inhibitors, potassium activators, diuretics, vasodilators, antithrombotics such as aspirin or nitrosated compounds thereof.

## Patentansprüche

1. Verbindung mit der allgemeinen Formel (I) oder ein pharmazeutisch verträgliches Salz oder Stereoisomer davon: 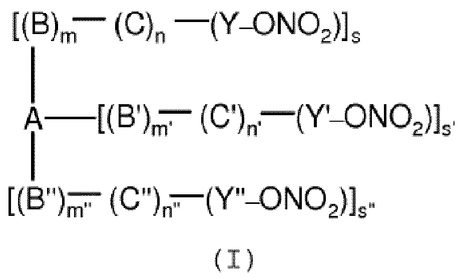 (I) wobei:
m, m'und m" gleich 0 oder 1 sind;
n, n' und n" gleich 0 oder 1 sind;
s, s' und s" gleich 0 oder 1 sind;
A aus der Gruppe ausgewählt ist, bestehend aus: 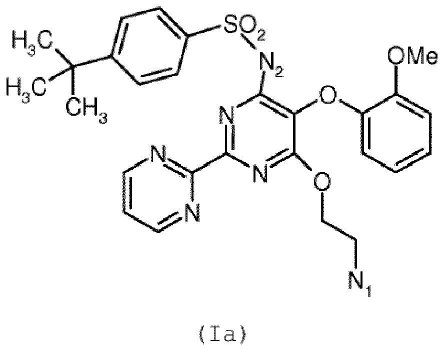 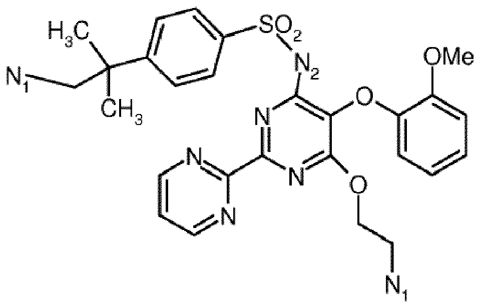 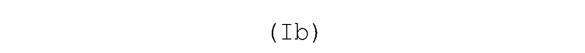 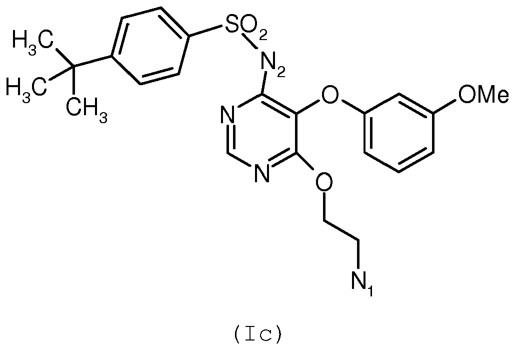 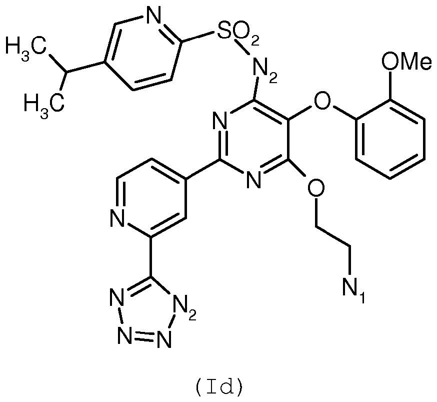 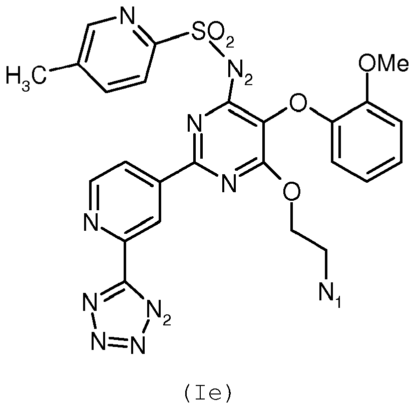 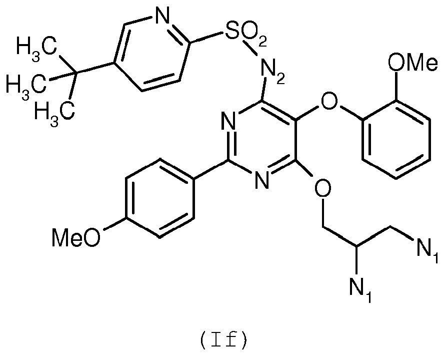 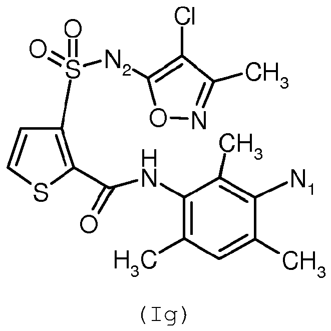 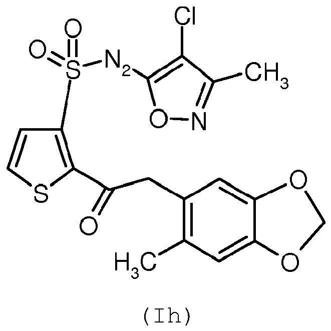 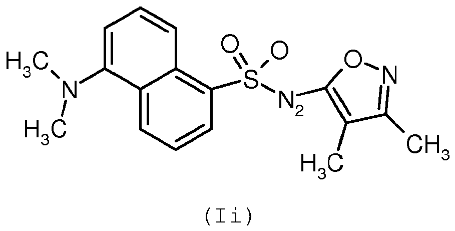 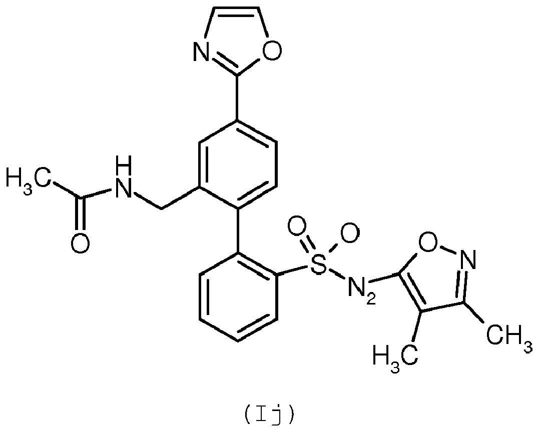 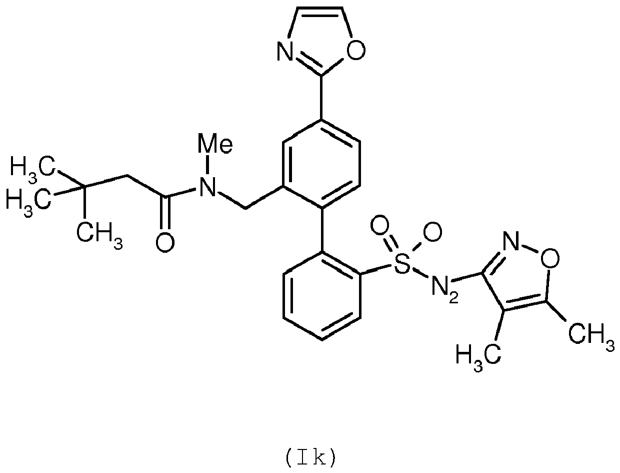 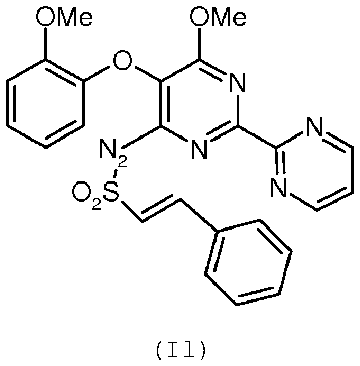 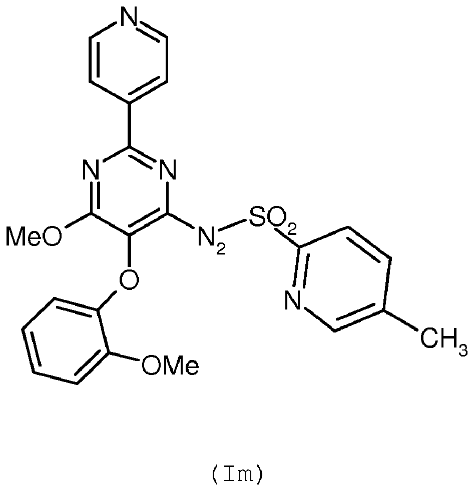 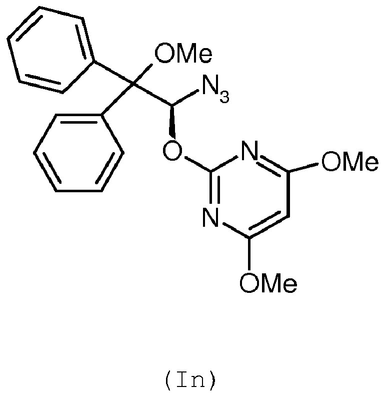 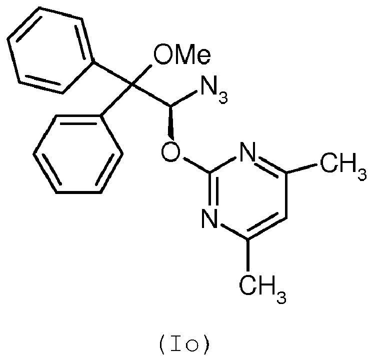 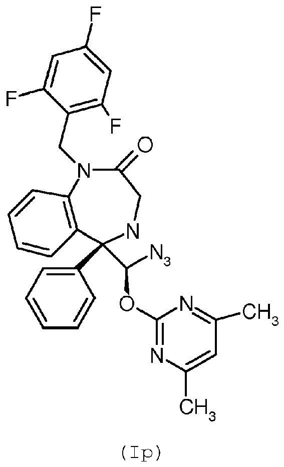 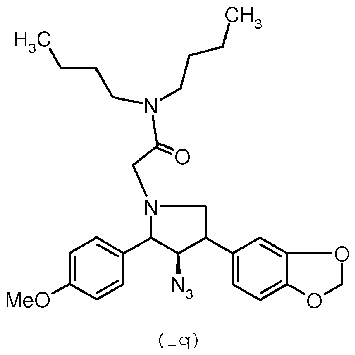 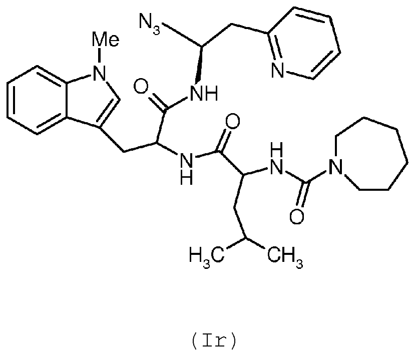 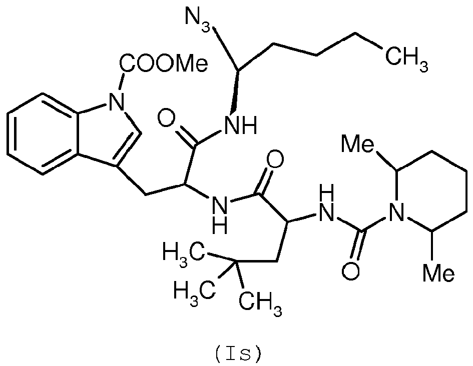 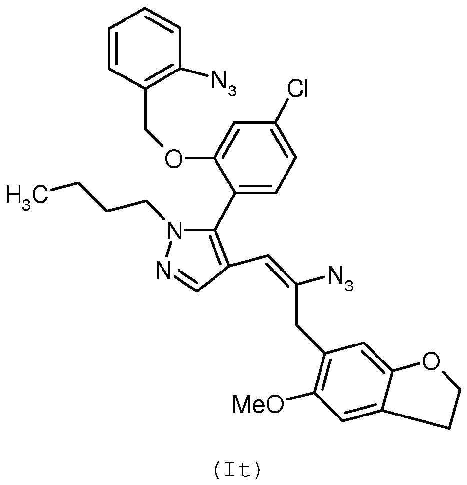 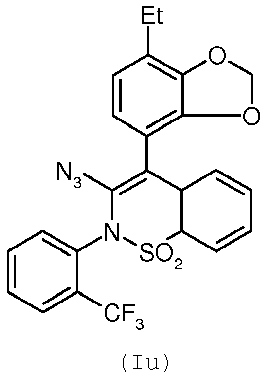 wobei.
N₁-O-,-OH ist;
N₂ -N-, -NH- ist;
N₃-C(O)O-, -C(O)NH- ist;
B, B' und B" -CO-, -C(O)O-, -C(O)NH sind;
C, C' und C" sind: 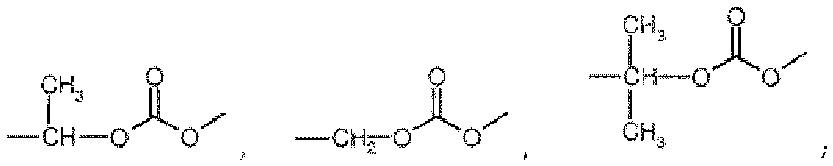 unter der Bedingung, dass:
1.) wenn A aus der Gruppe ausgewählt ist, bestehend aus:
(la), (Ib), (Ic), (Id), (Ie), (If), (Ig), (1h), (li), (Ij), (Ik), (II) und (Im), mindestens eine Zahl unter m, m', m", n, n' oder n" 1 ist;
2.) wenn A aus der Gruppe ausgewählt ist, bestehend aus:
(In), (lo), (1p), (Iq), (Ir), (Is) und (Iu) s', s" und m 0 sind, während n 0 oder 1 ist;
3.) wenn A (It) ist, m, m' und s" 0 sind, während n und n' 0 oder 1 sind;
4.) mindestens eine der Gruppen N₁ oder N₂ -O- oder -N- ist und zu einer Bindung mit mindestens einer der Gruppen -[(B)ₘ-(C)ₙ-(Y-ONO₂)], -[(B')_{m'}-(C')_{n'}-(Y'-ONO₂)] oder -[(B")_{n"}-(C")_{n"}-(Y"-ONO₂)] in der Lage ist;
Y, Y'und Y" ein bivalenter Rest mit der folgender Bedeutung sind:
a)
- geradkettiges oder verzweigtes C₁-C₂₀ Alkylen, optional substituiert durch einen oder mehrere der Substituent(en), ausgewählt aus der Gruppe bestehend aus: Halogenatomen, Hydroxy, -ONO₂ oder Tₐ, wobei Tₐ
- OC(O) C₁-C₁₀ Alkyl)-ONO₂ oder -O(C₁-C₁₀) Alkyl)-ONO₂ ist;
- Cycloalkylen mit 5 bis 7 Kohlenstoffatomen in einem Cycloalkylenring ist, wobei der Ring optional durch Seitenketten T substituiert ist, wobei T ein geradkettiges oder verzweigtes Alkyl mit 1 bis 10 Kohlenstoffatomen ist.
b) 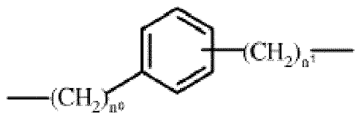
c) 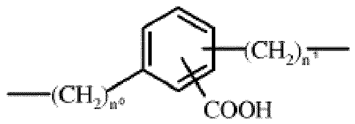 wobei n° eine ganze Zahl von 0 bis 20 ist, und n¹ eine ganze Zahl von 1 bis 20 ist;
d) 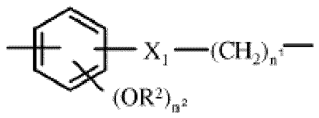 wobei:
n¹ wie oben definiert ist und n² eine ganze Zahl von 0 bis 2 ist;
X₁ = -OCO- oder -COO- und R₂ H oder CH₃ ist:
e) 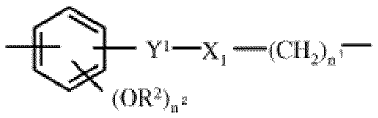 wobei:
n¹ n² , R² und X₁ wie oben definiert sind:
Y¹ -CH₂-CH₂- oder -CH=CH-(CH2)n²- ist;
f) 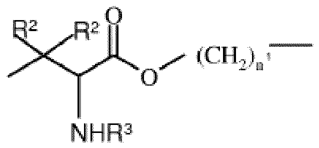 wobei:
n¹ und R² wie oben definiert sind, R³ H oder -COCH₃ ist,
unter der Bedingung, dass wenn Y unter den in b)-f) erwähnten bivalenten Resten
ausgewählt ist, die -ONO₂-Gruppe mit einer -CH₂-Gruppe verknüpft ist;
g) 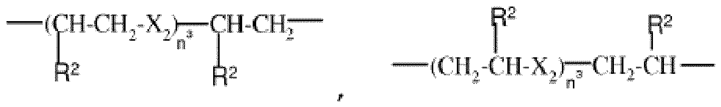 wobei X₂ -O- oder -S- ist, n³ eine ganze Zahl von 1 bis 6 ist, R², wie oben definiert ist;
h) 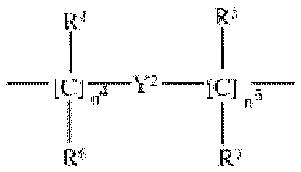 wobei:
n⁴ eine ganze Zahl von 0 bis 10 ist;
n⁵ eine ganze Zahl von 1 bis 10 ist;
R⁴, R⁵, R⁶, R⁷ gleich oder unterschiedlich sind und H oder geradkettiges oder verzweigtes C₁-C₄ Alkyl sind,
wobei die -ONCO₂-Gruppe verknüpft ist mit 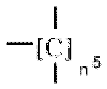 wobei n⁵ wie oben definiert ist;
Y² ein heterozyklischer, gesättigter, ungesättigter oder aromatischer 5- oder 6-gliedriger Ring ist, enthaltend ein oder mehrere Heteroatom(e), ausgewählt unter Stickstoff, Sauerstoff, Schwefel,
und aus der Gruppe ausgewählt ist, bestehend aus:

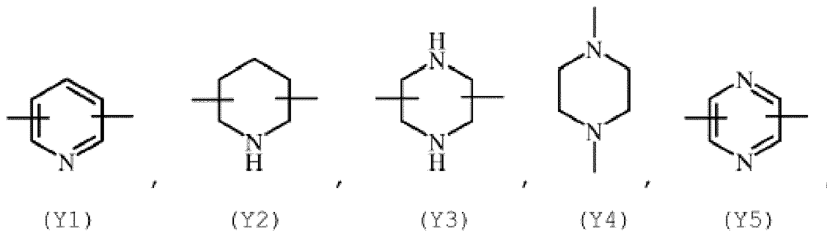

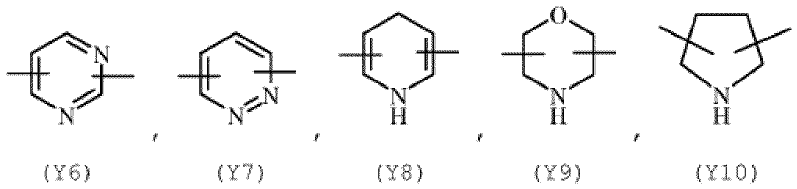

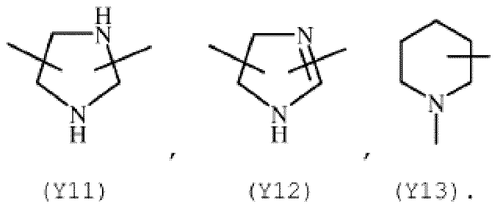

2. Verbindung mit der allgemeinen Formel (I) oder ein pharmazeutisch verträgliches Salz oder Stereoisomer davon nach Anspruch 1, wobei Y, Y' und Y" ein bivalenter Rest sind mit der folgenden Bedeutung:
a)
- geradkettiges oder verzweigtes C₁-C₁₀ Alkylen, optional substituiert durch eine -ONC₂-Gruppe;
b) 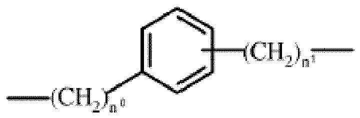 wobei n° eine ganze Zahl gleich 0 oder 1 ist und n¹ eine ganze Zahl gleich 1 ist, unter der Bedingung, dass die -ONO₂-Gruppe mit einer -CH₂-Gruppe verknüpft ist;
g) 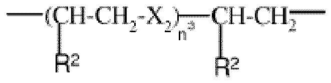 wobei X₂ -O- oder -S- ist, n³ eine ganze Zahl gleich 1 ist und R² H ist.

3. Verbindung nach Ansprüchen 1- 2, ausgewählt aus der Gruppe, bestehend aus

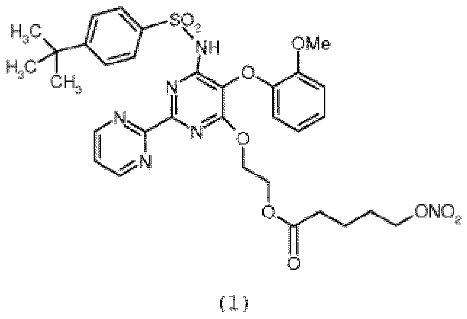

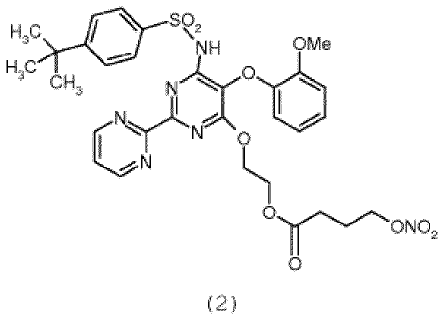

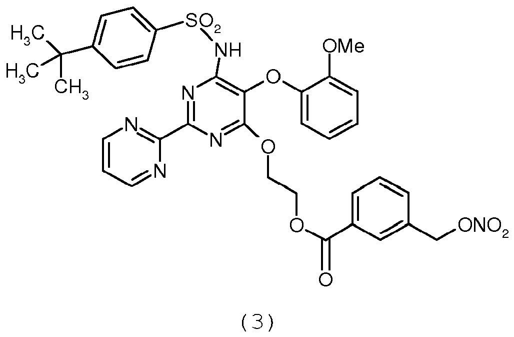

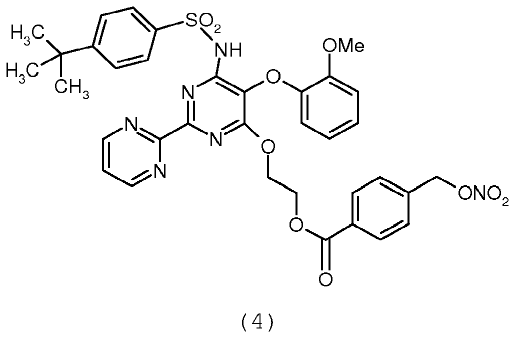

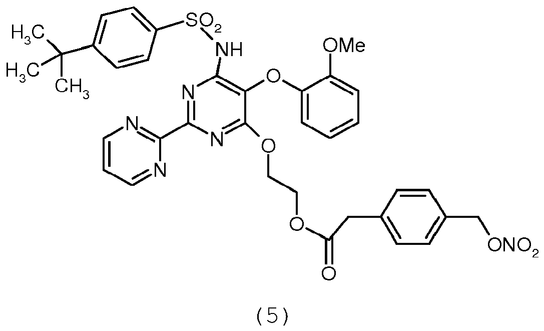

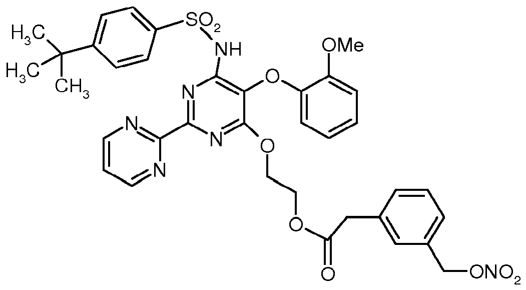

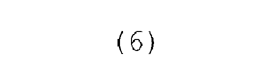

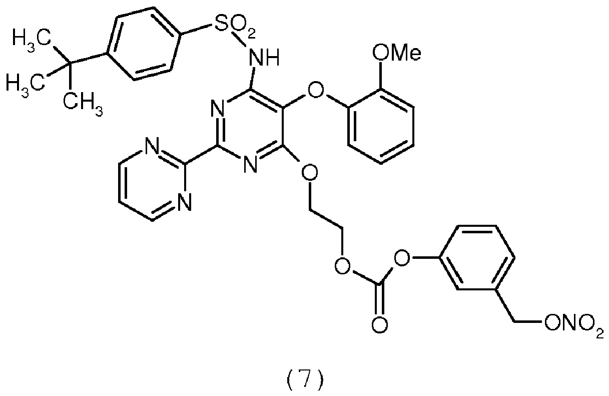

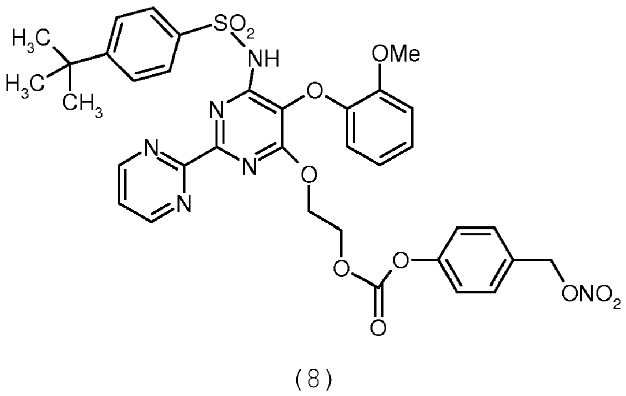

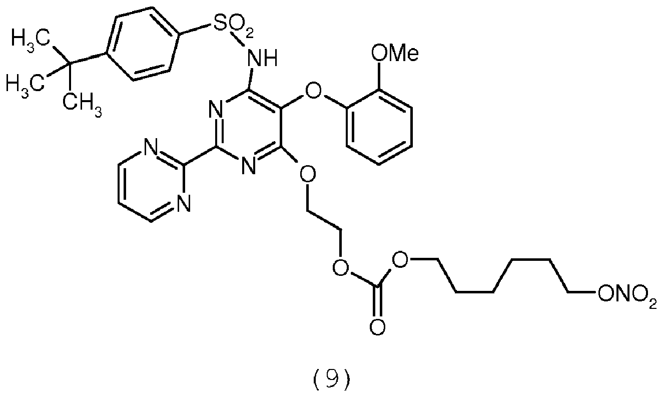

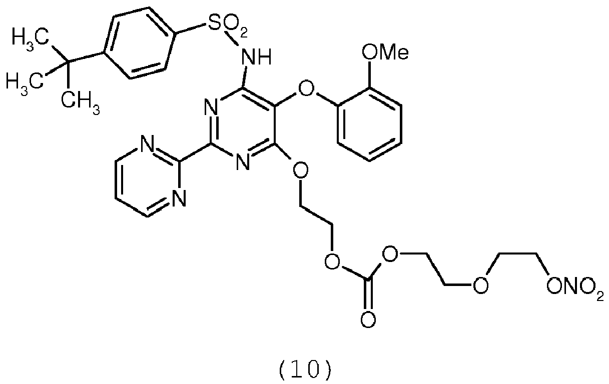

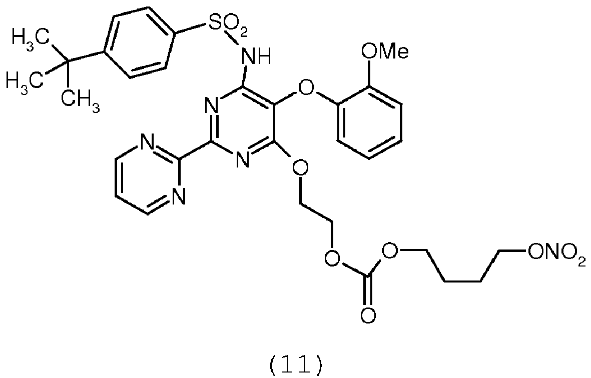

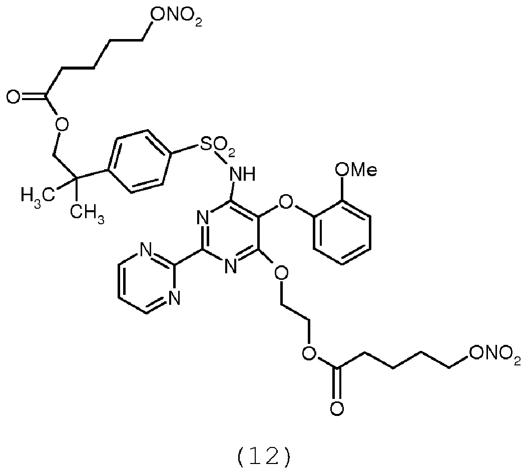

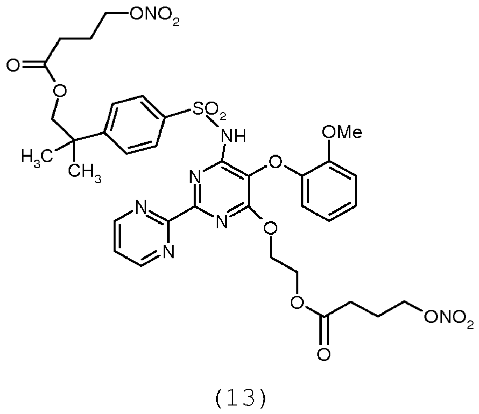

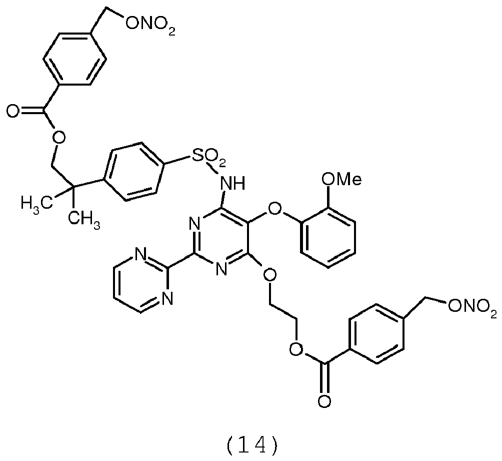

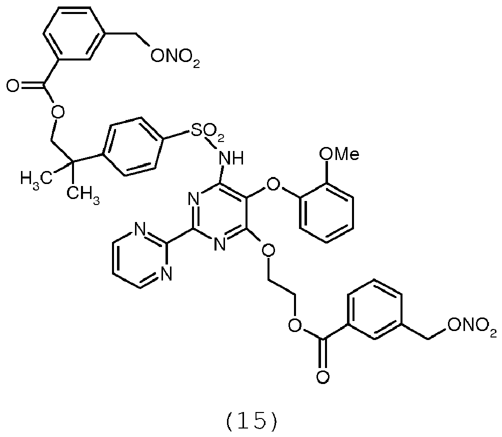

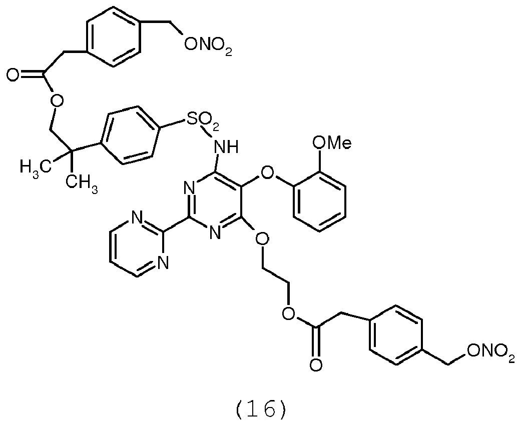

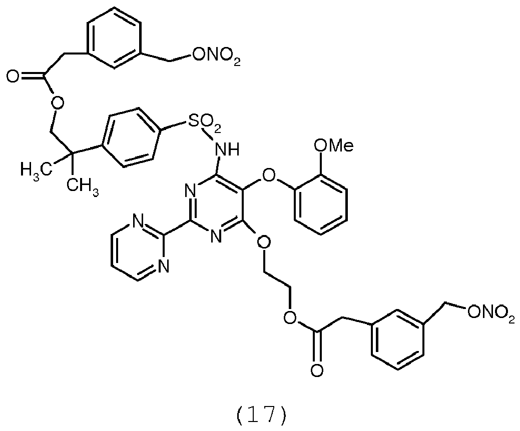

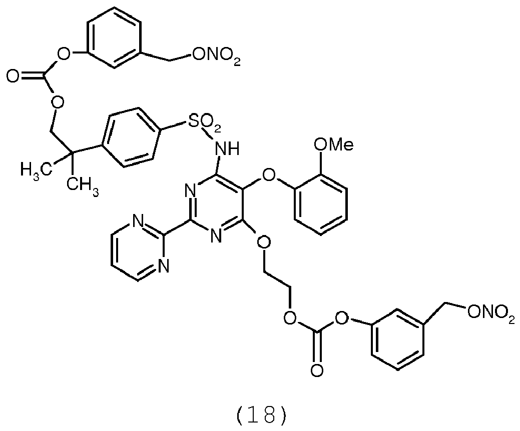

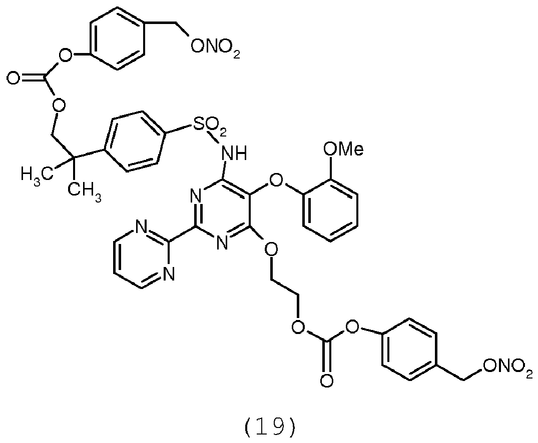

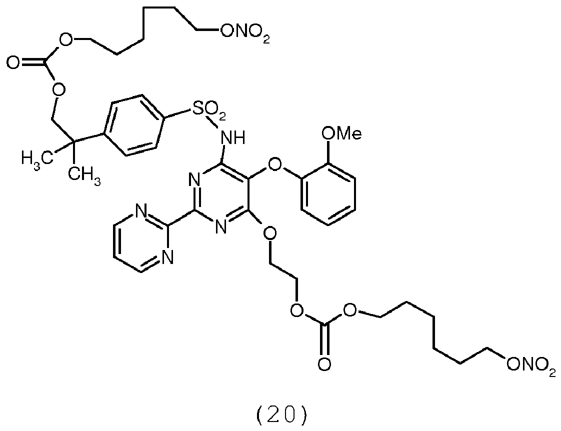

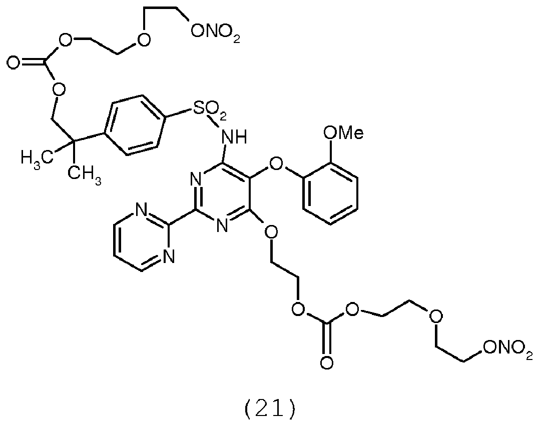

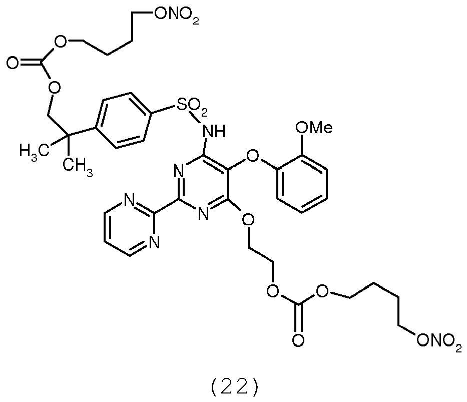

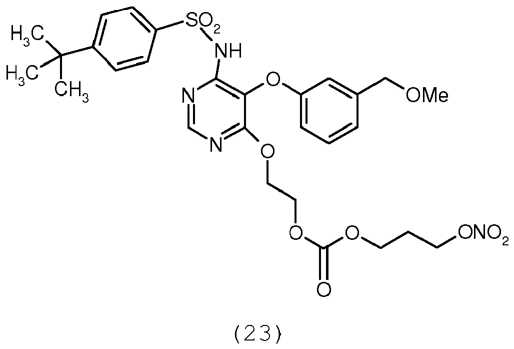

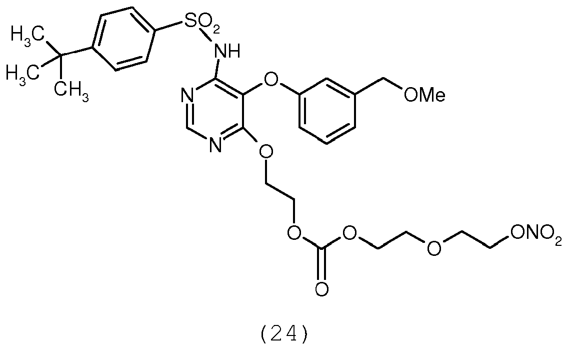

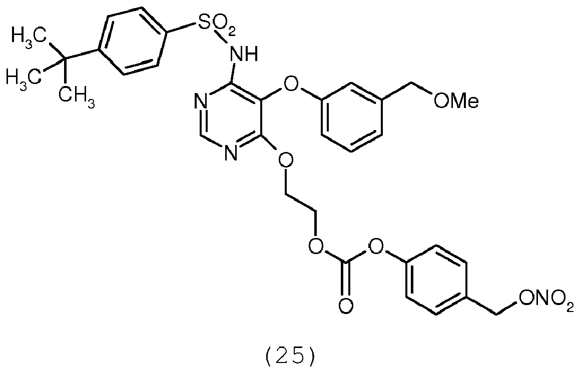

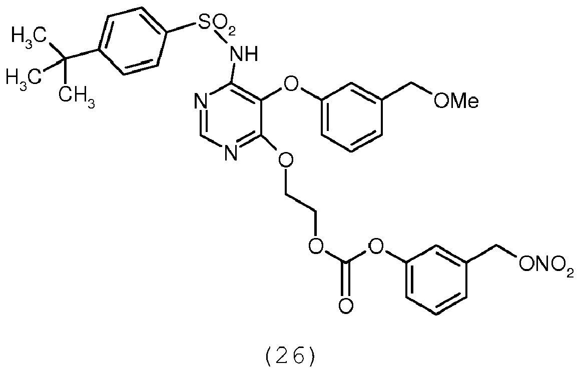

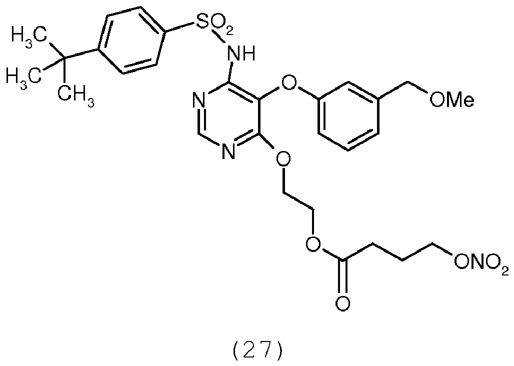

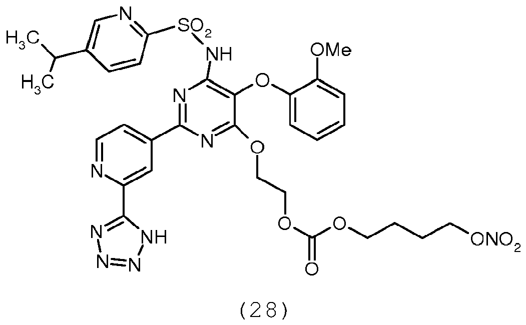

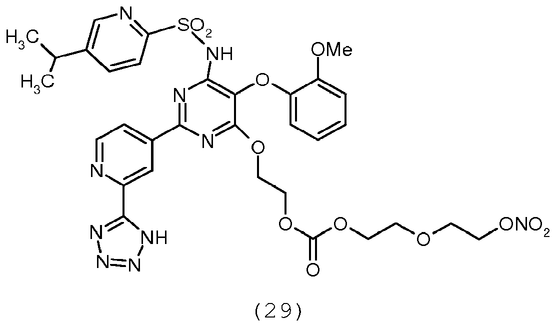

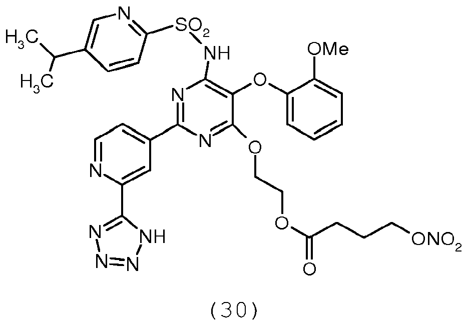

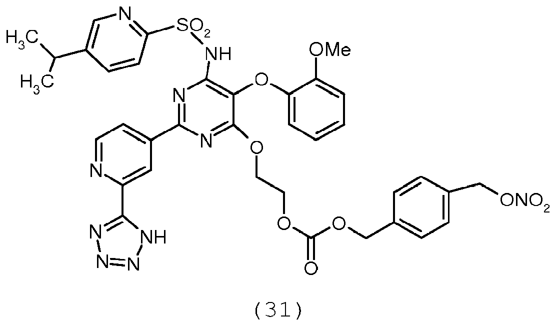

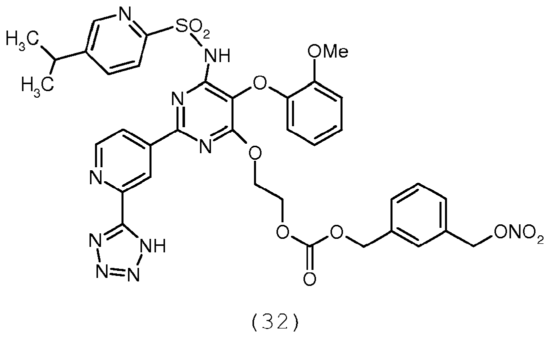

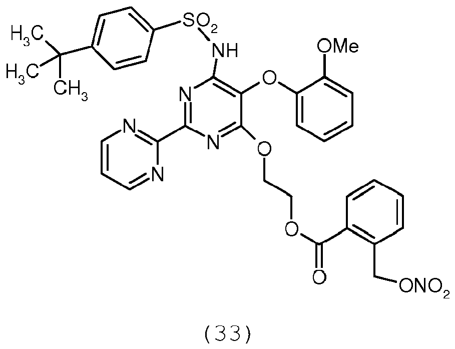

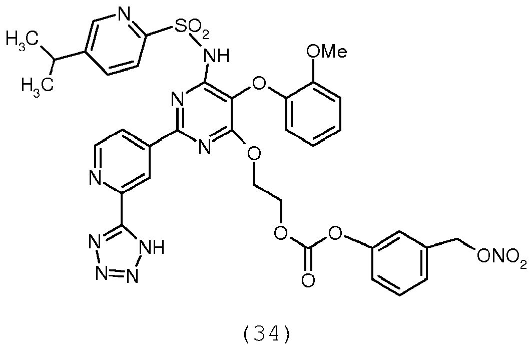

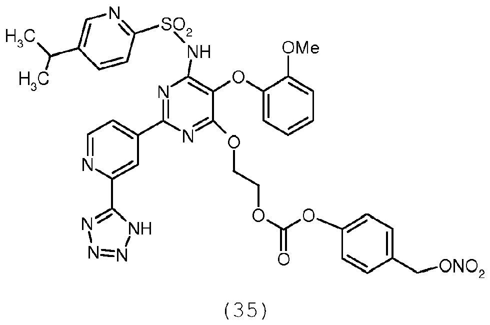

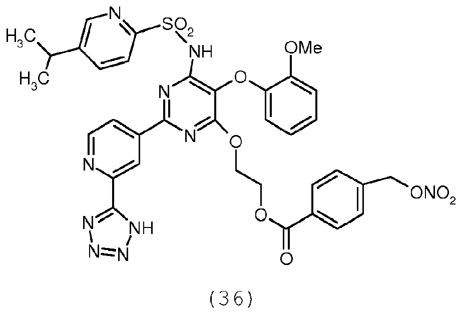

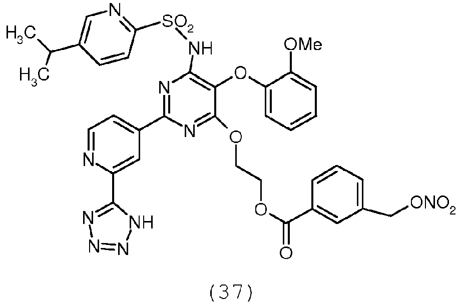

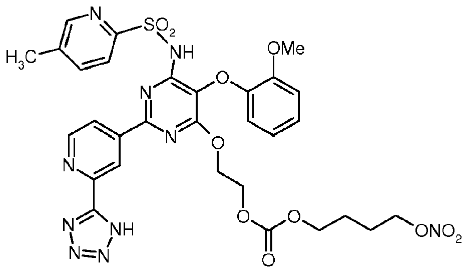

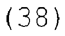

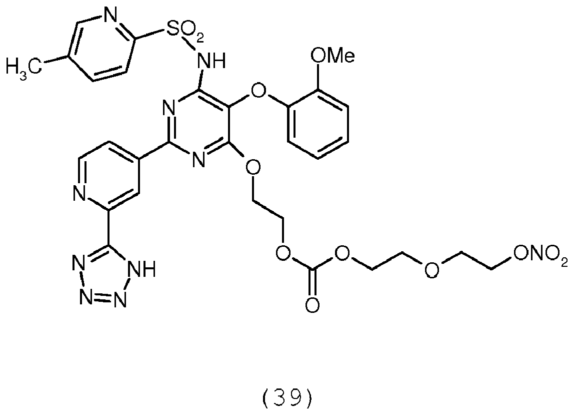

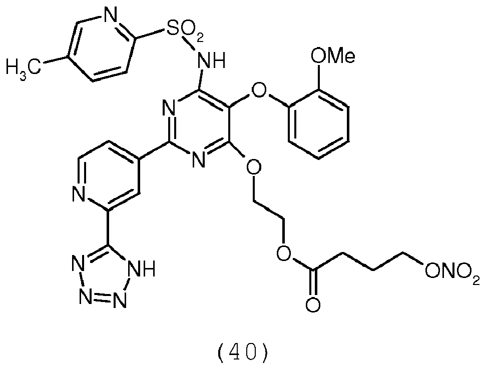

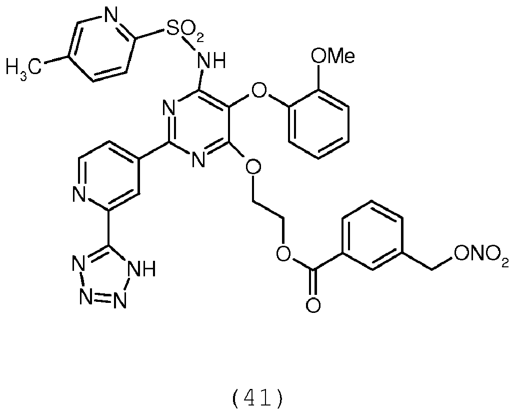

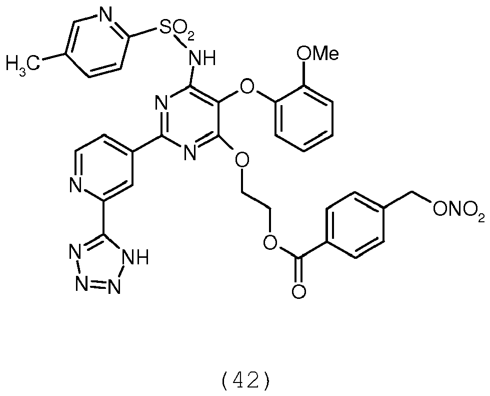

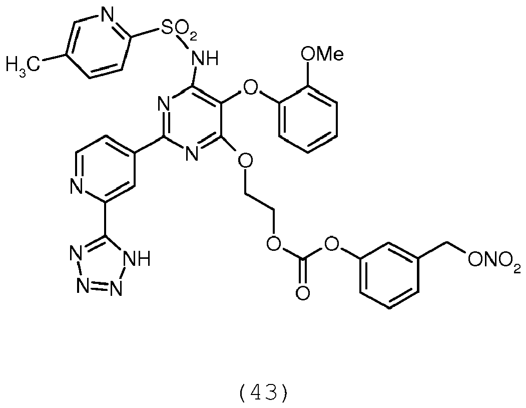

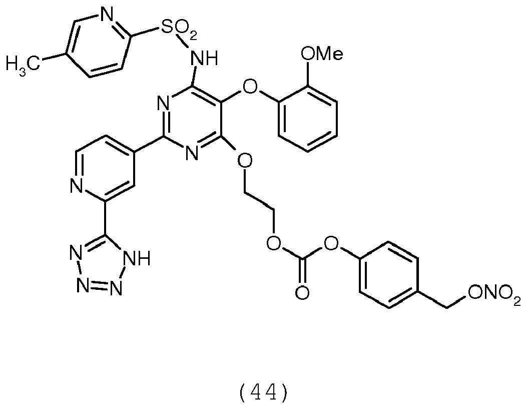

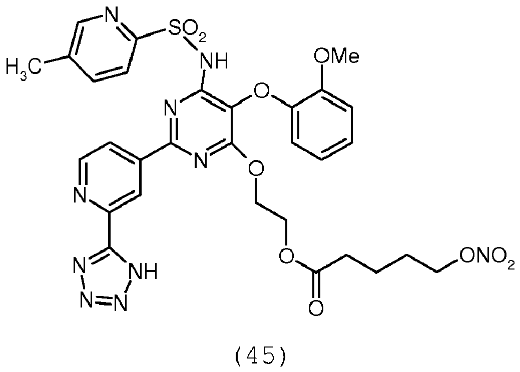

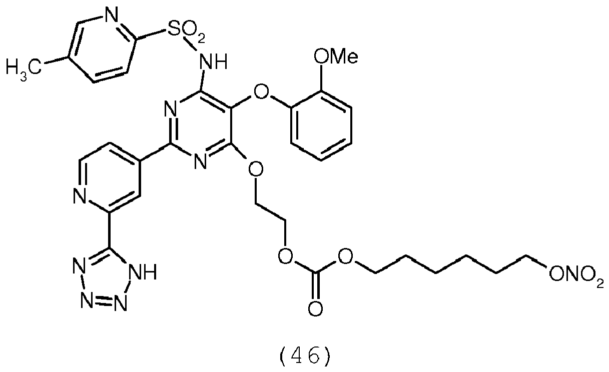

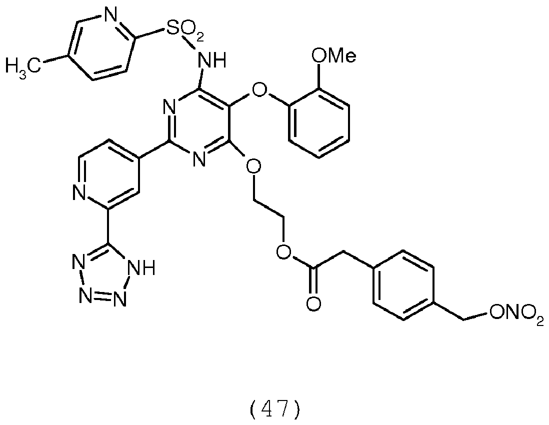

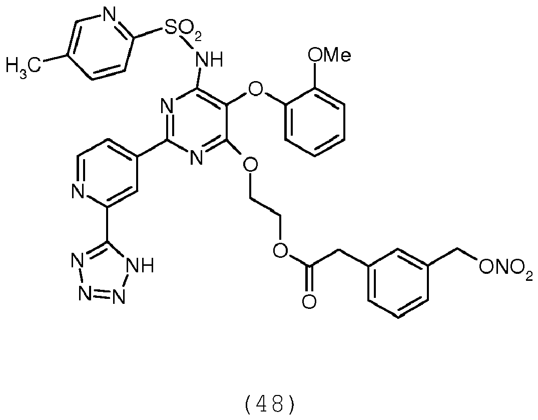

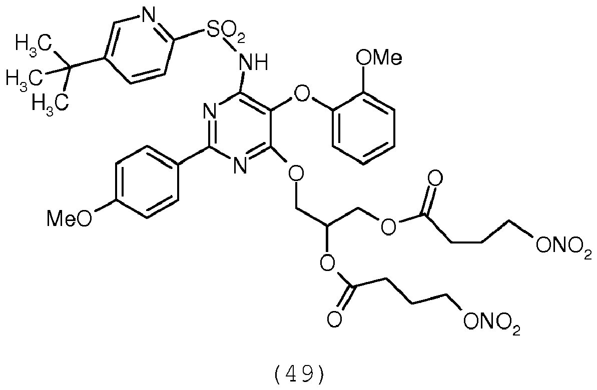

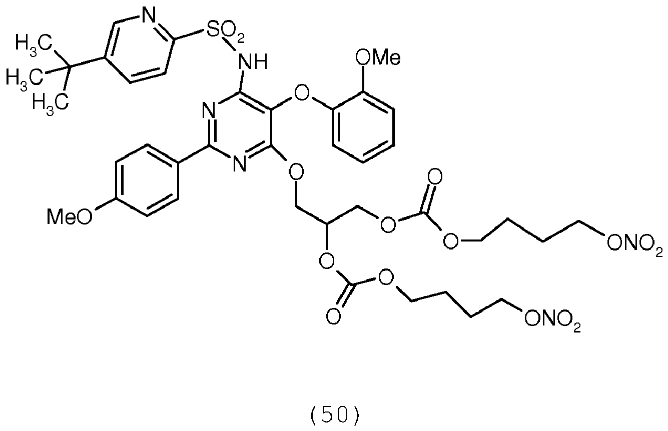

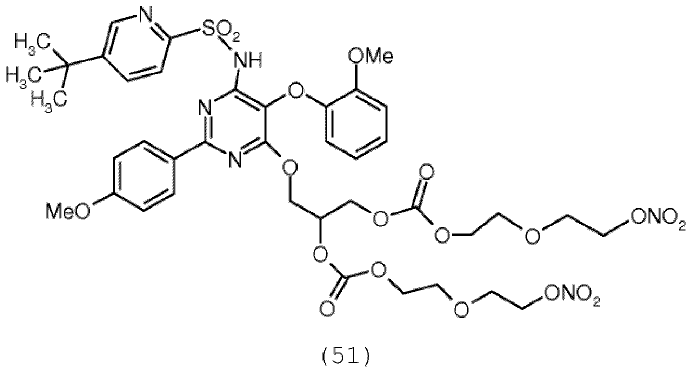

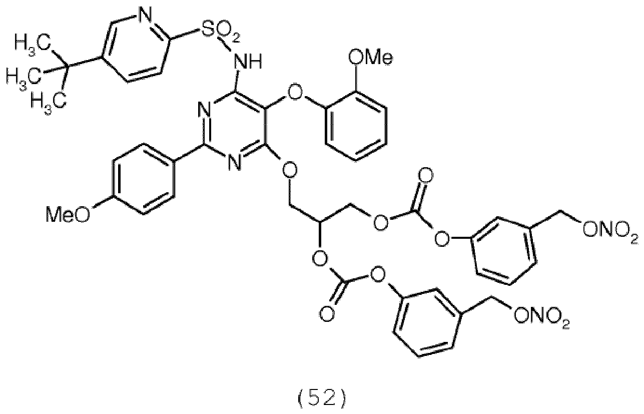

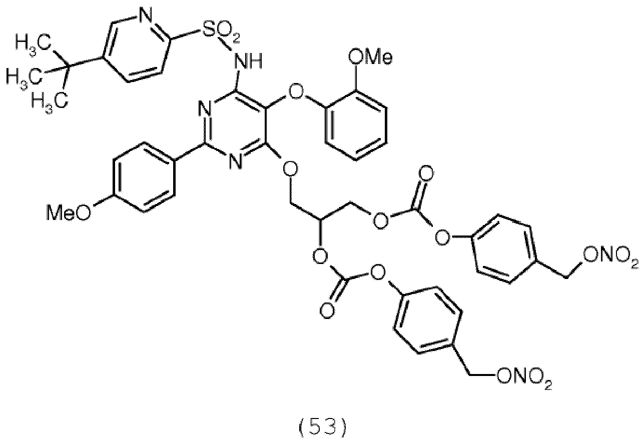

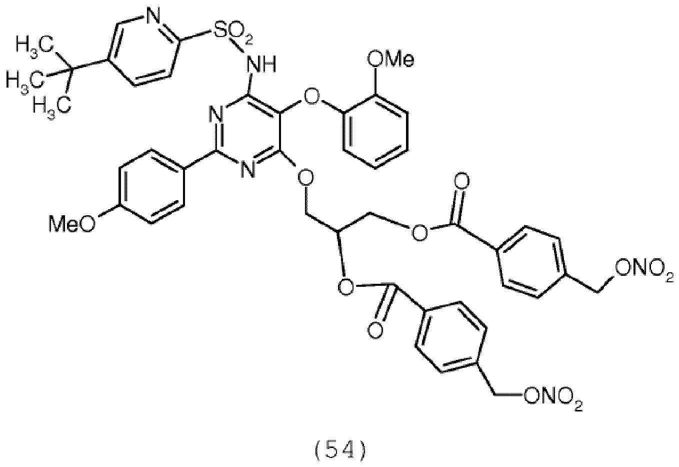

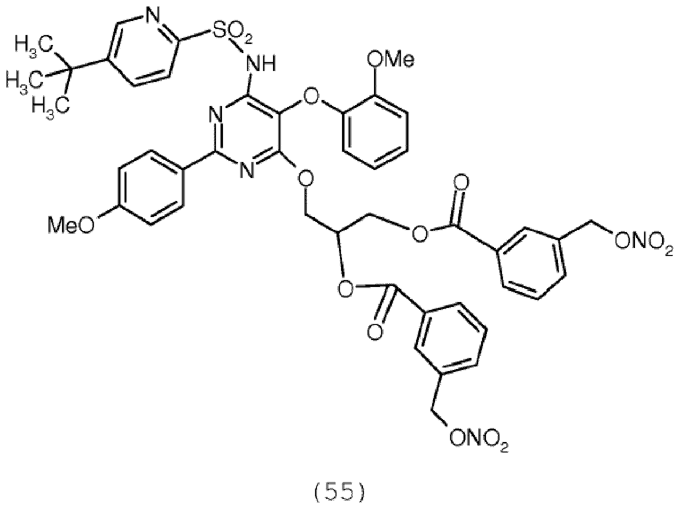

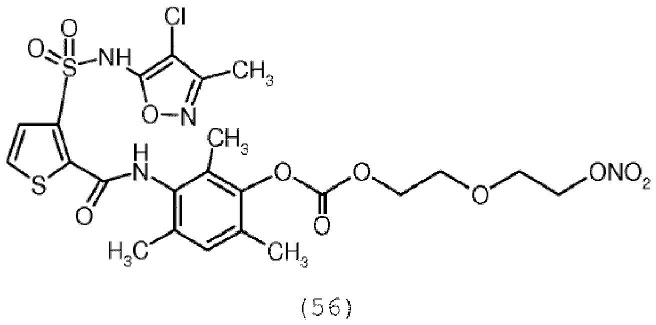

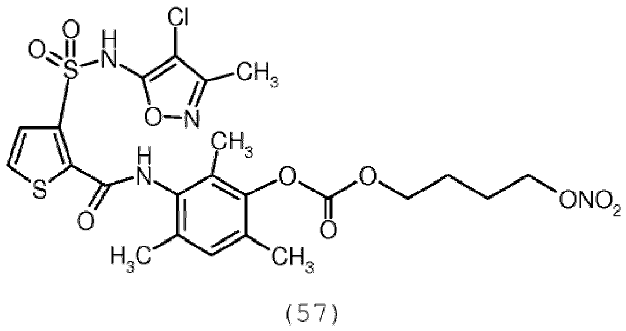

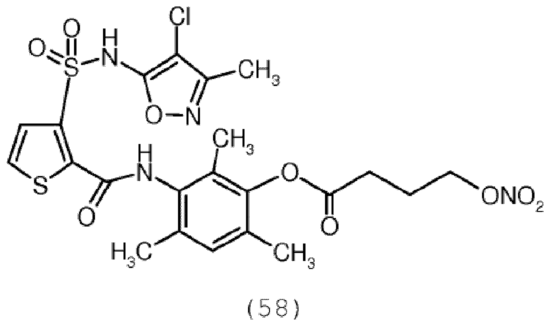

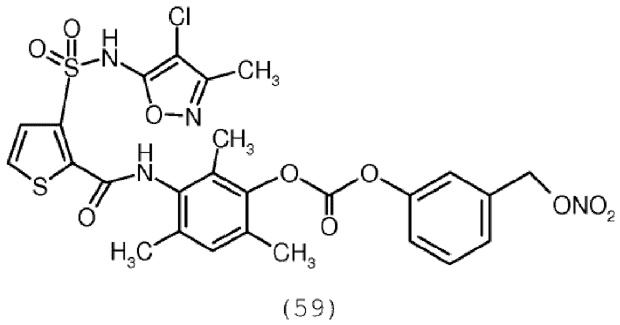

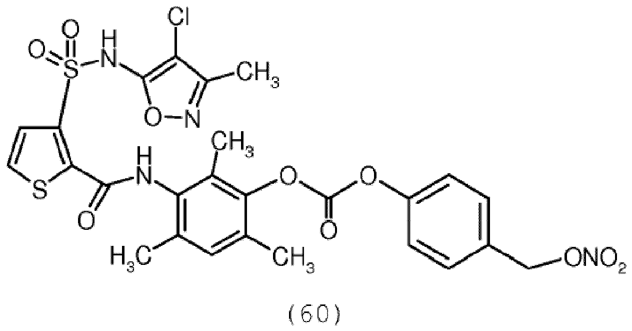

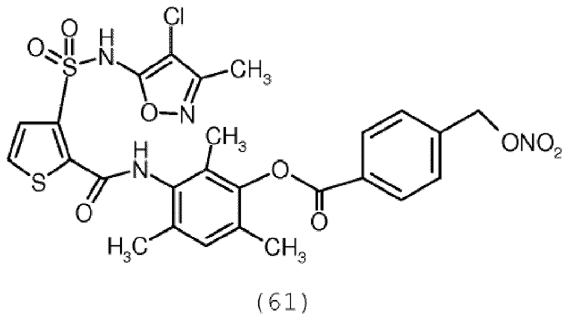

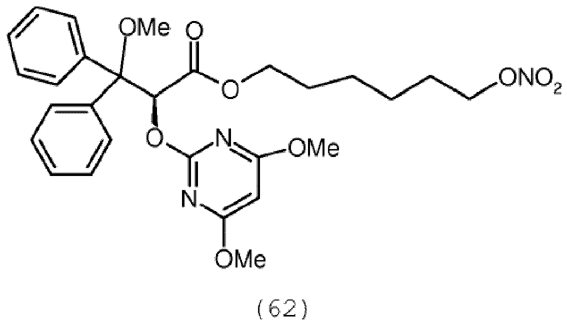

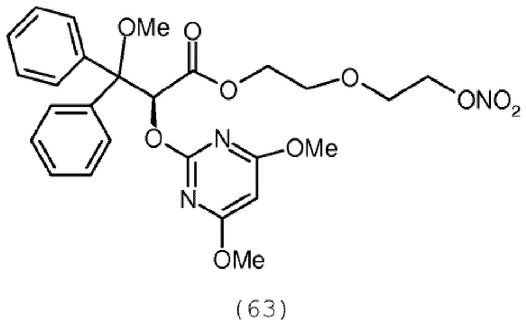

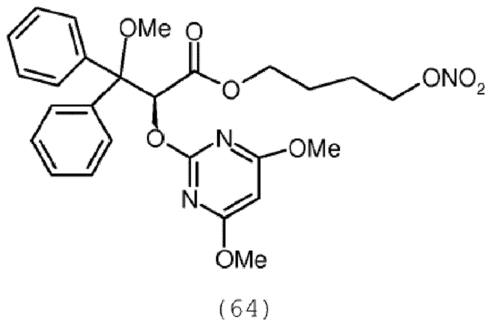

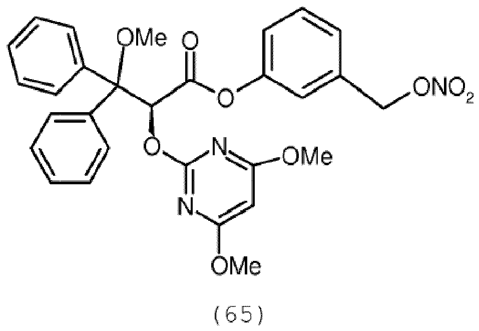

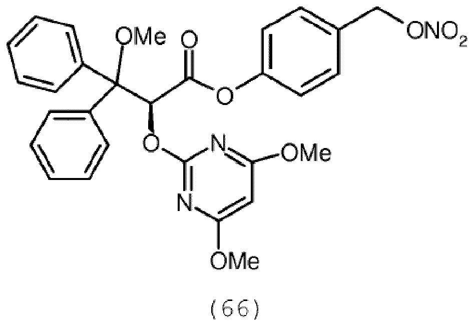

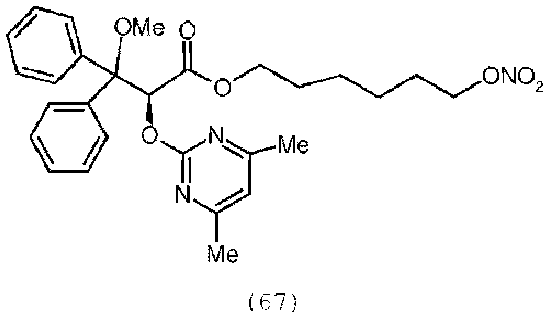

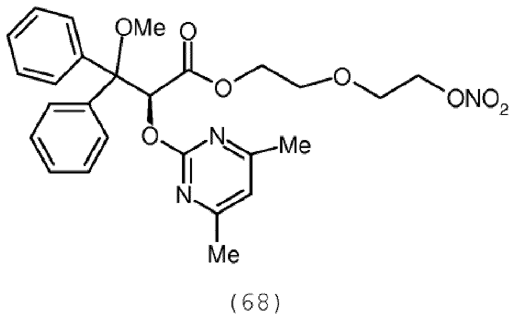

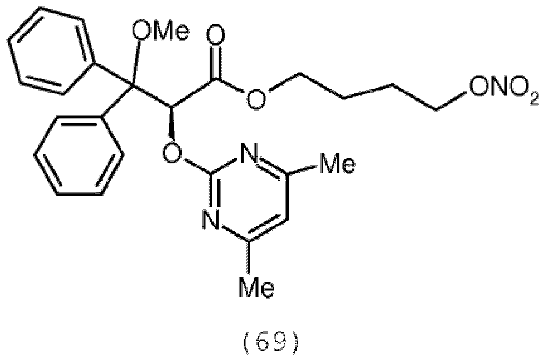

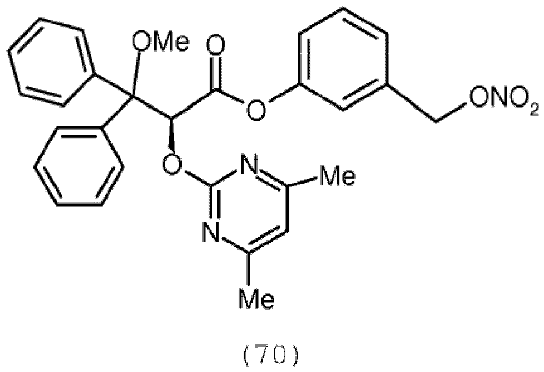

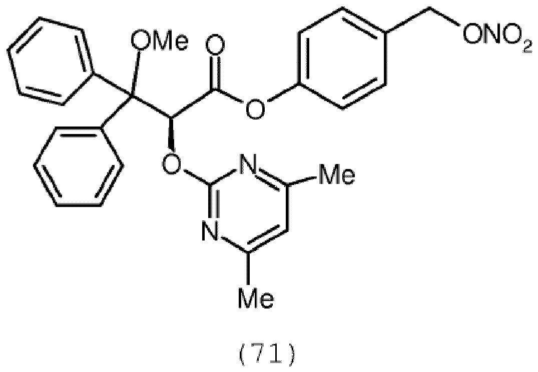

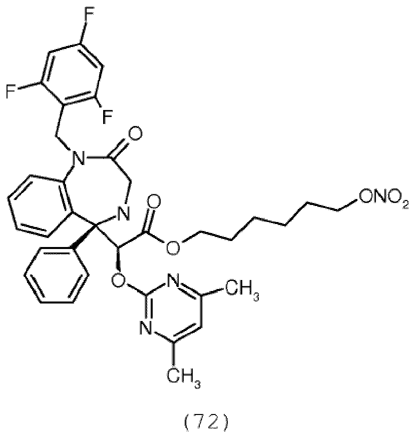

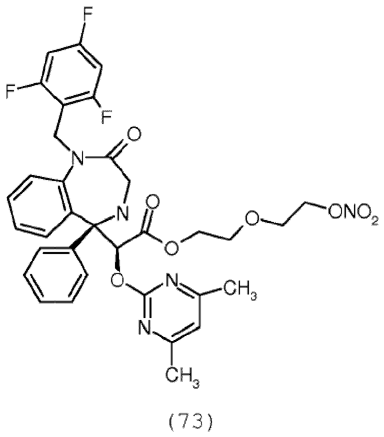

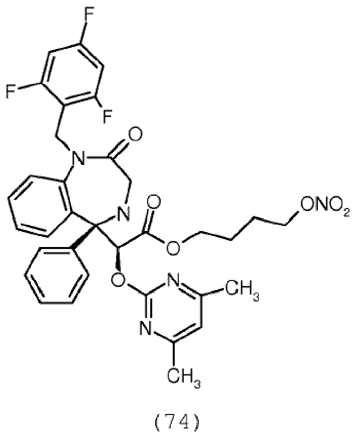

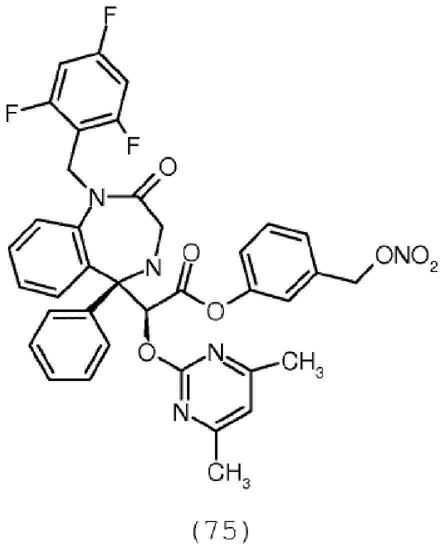

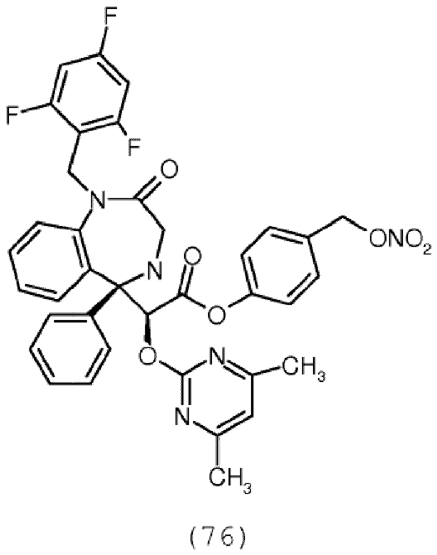

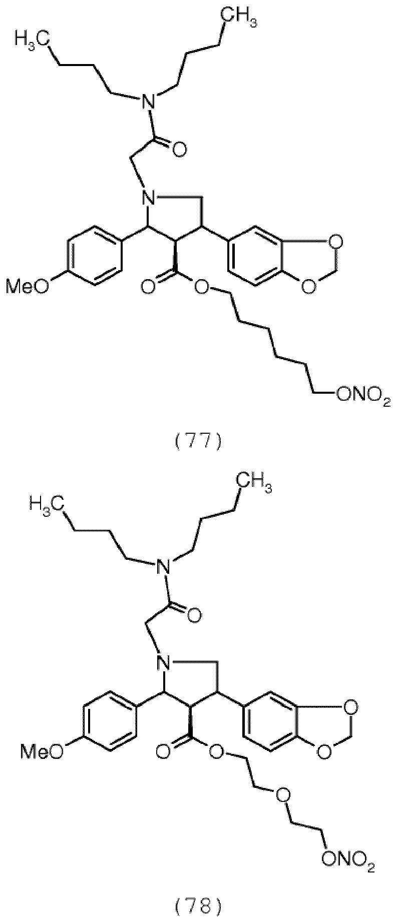

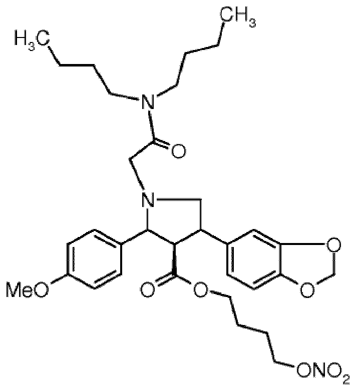

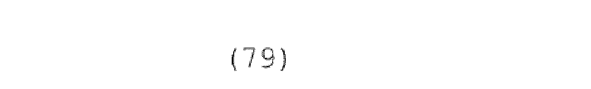

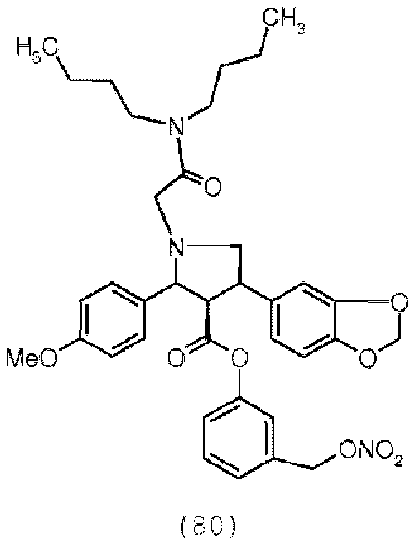

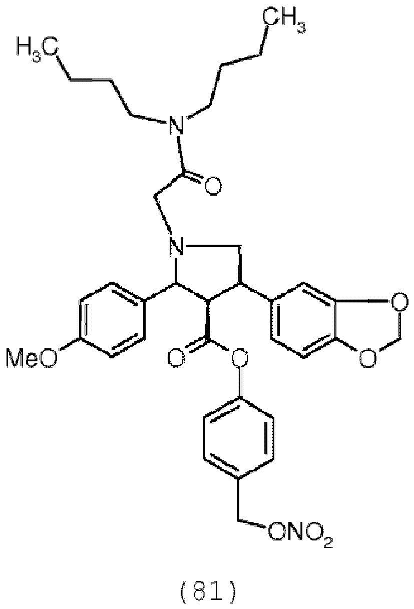

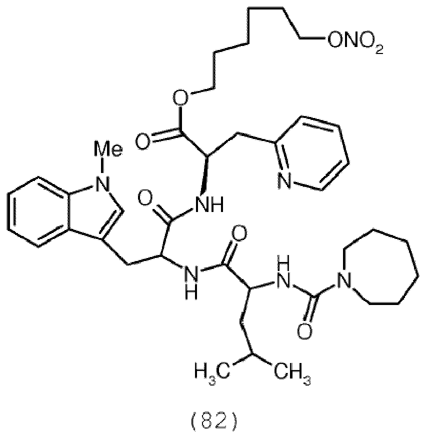

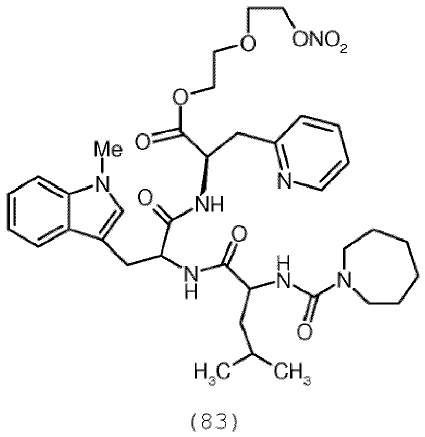

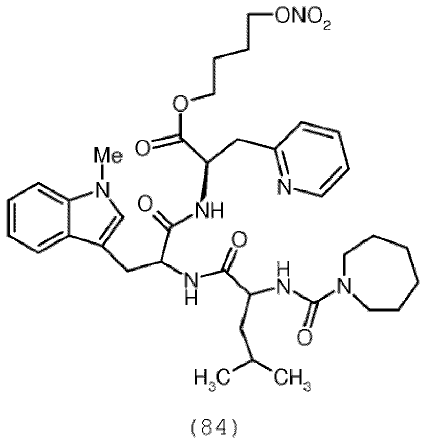

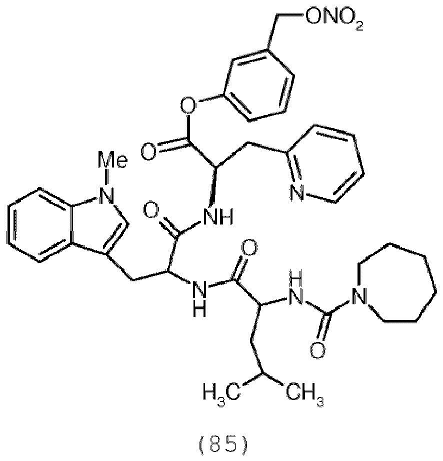

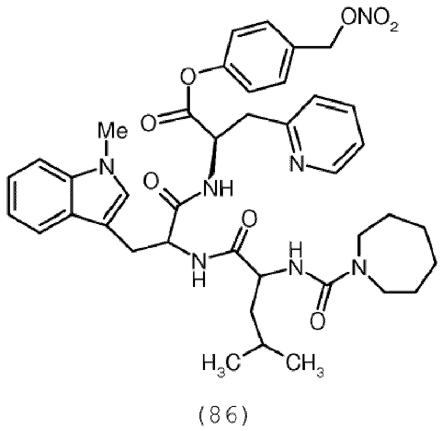

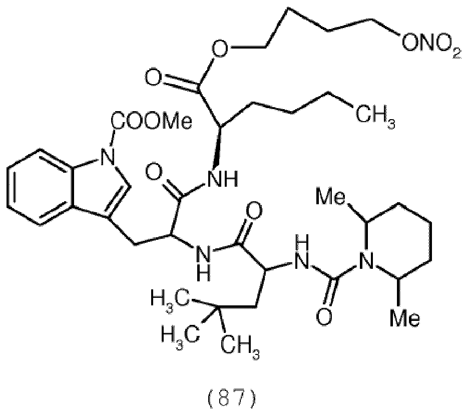

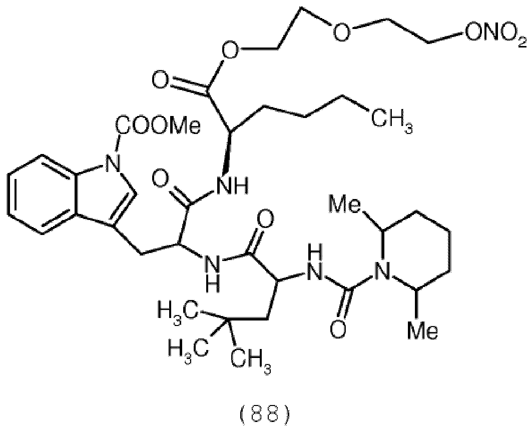

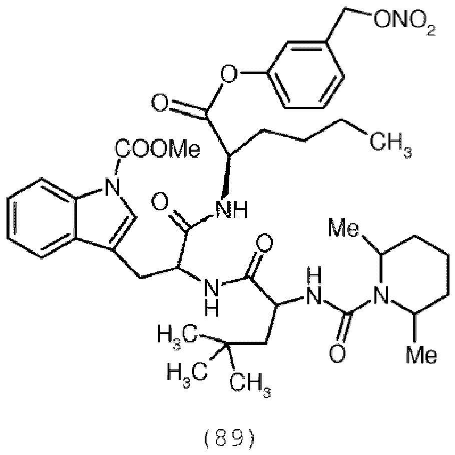

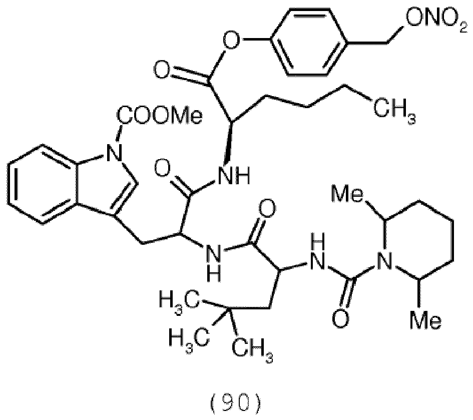

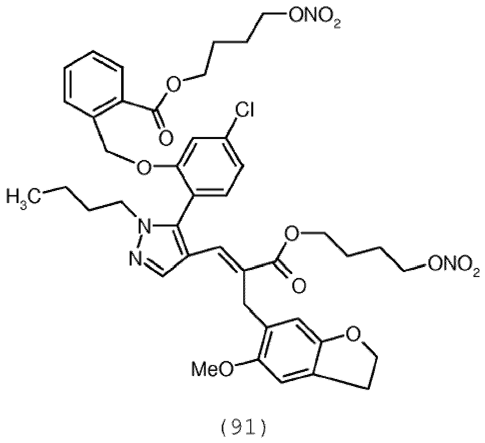

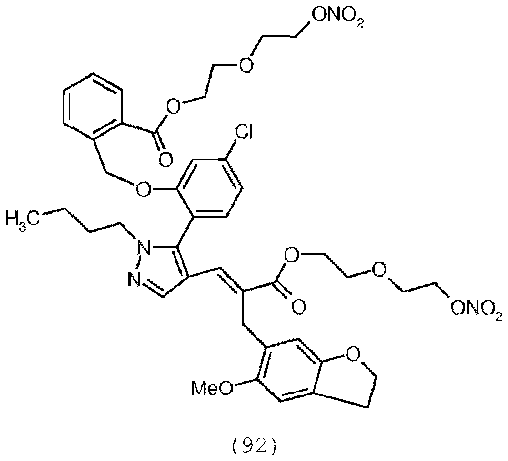

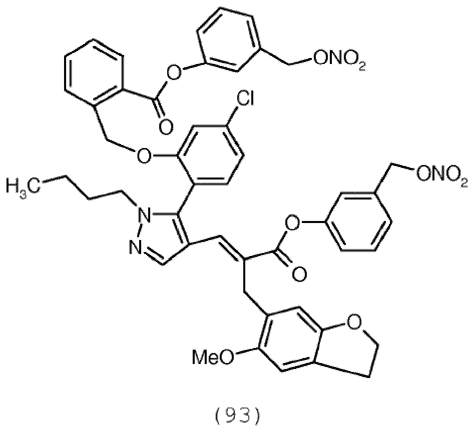

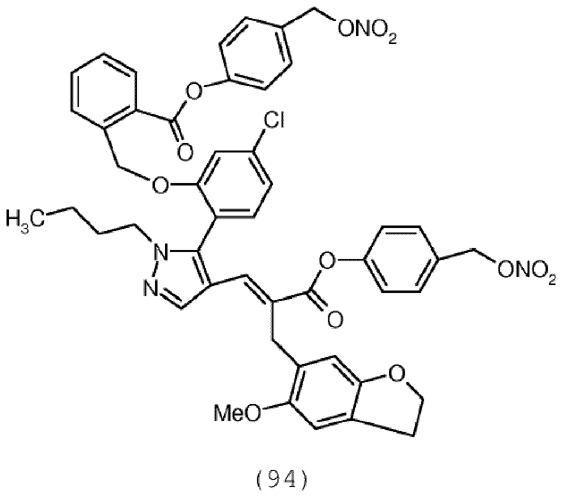

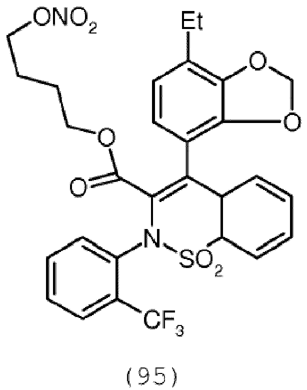

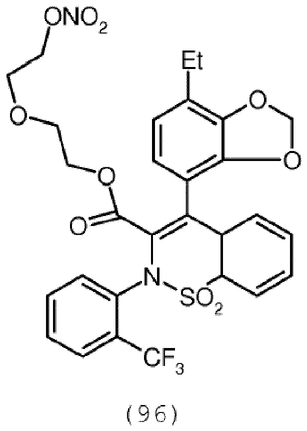

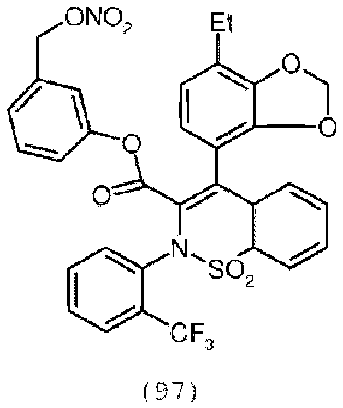

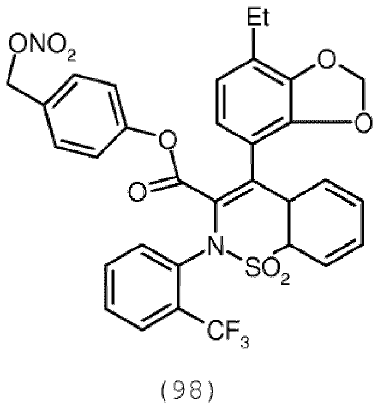

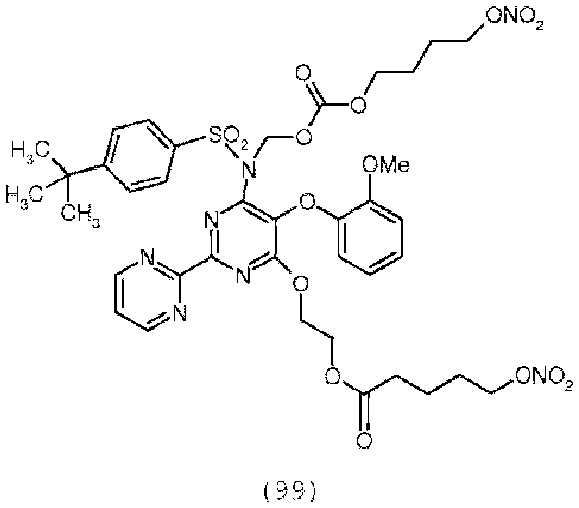

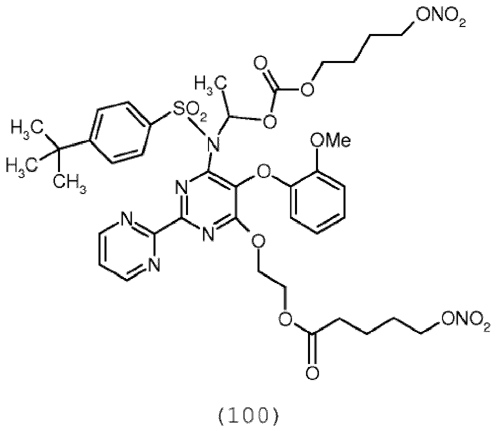

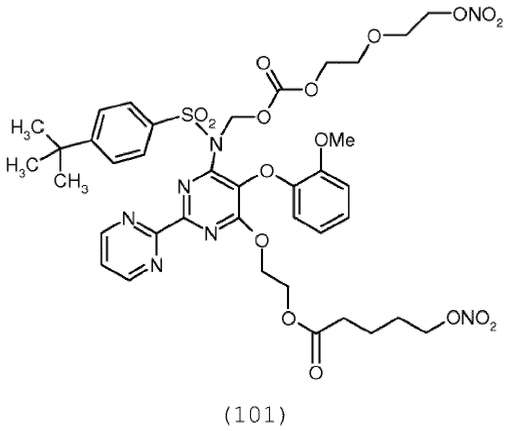

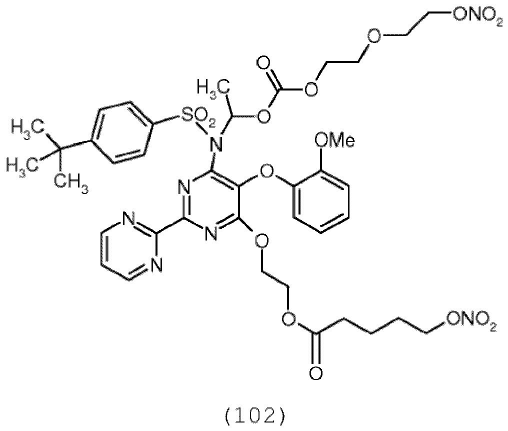

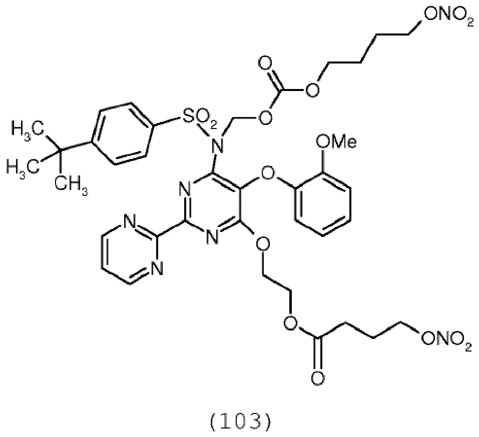

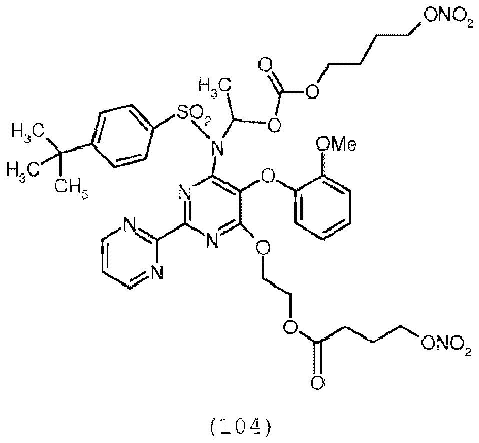

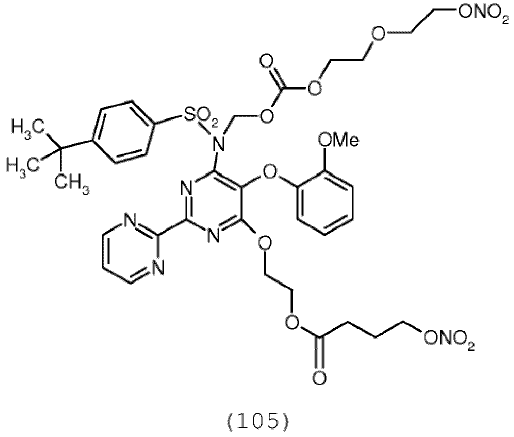

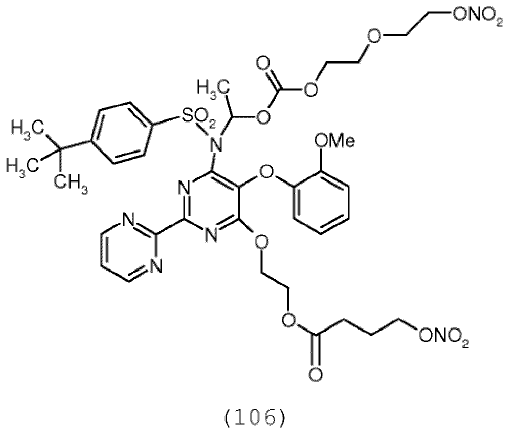

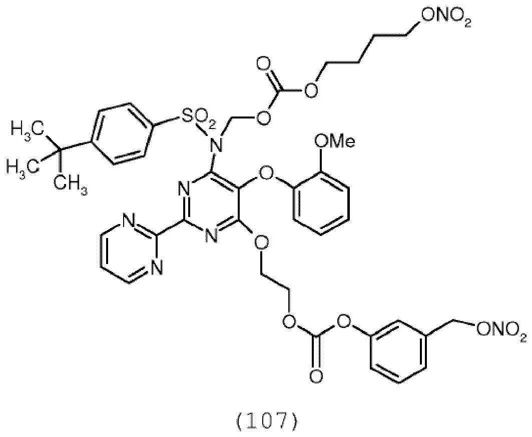

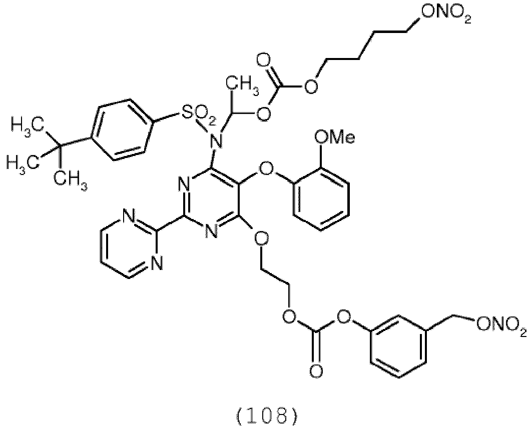

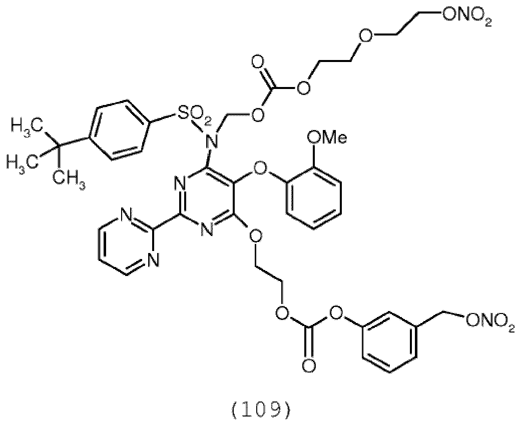

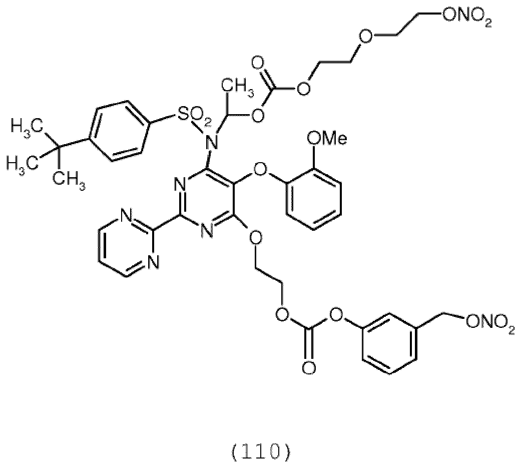

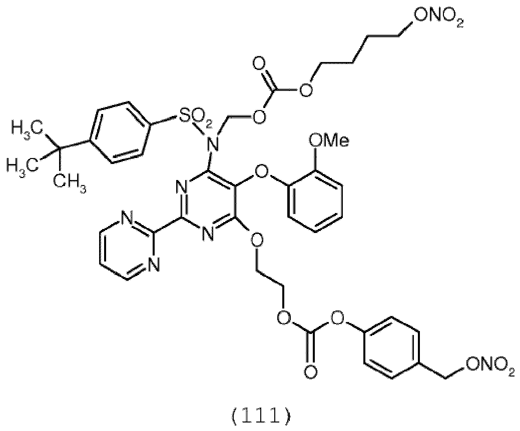

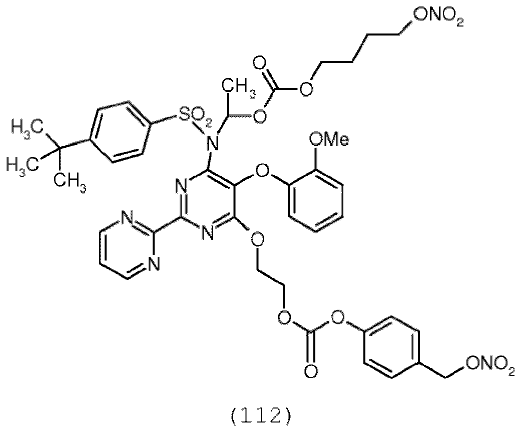

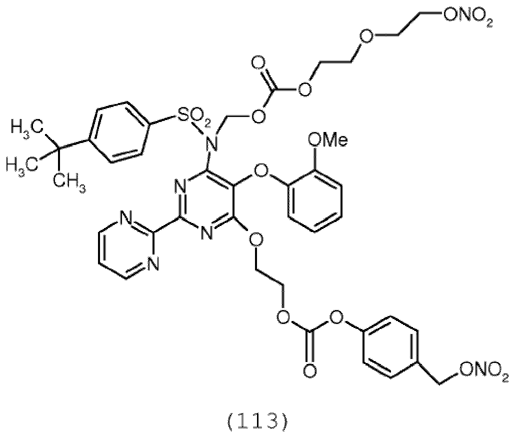

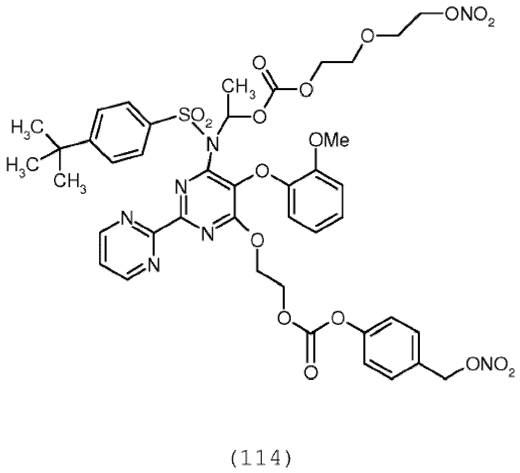

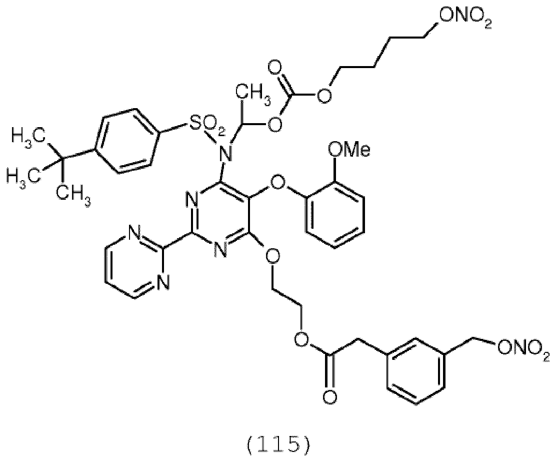

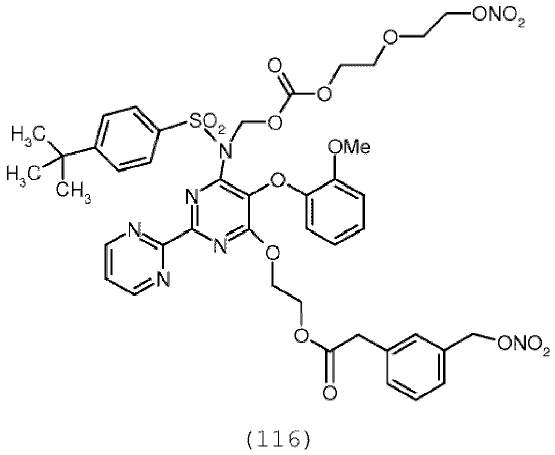

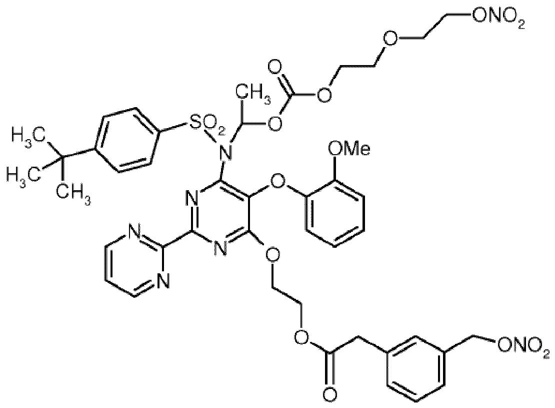

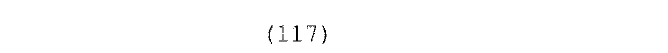

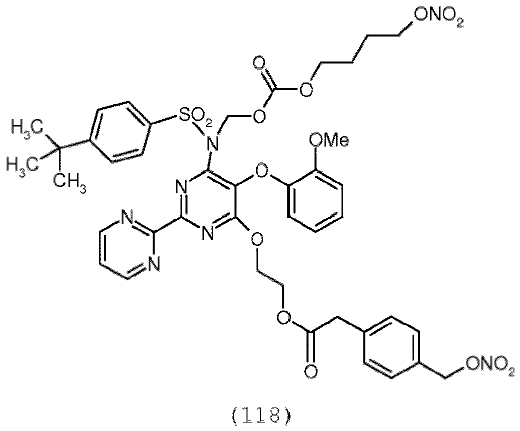

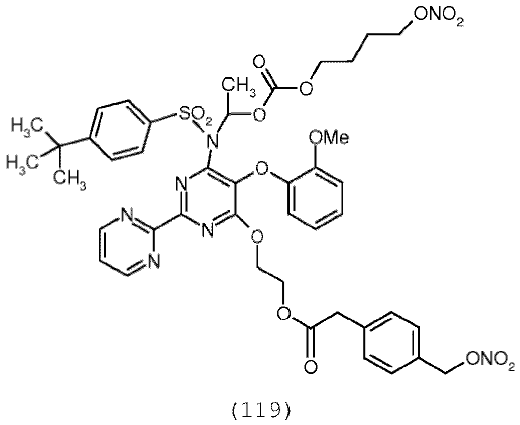

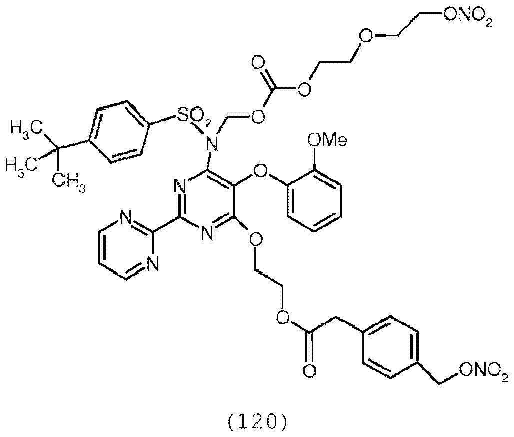

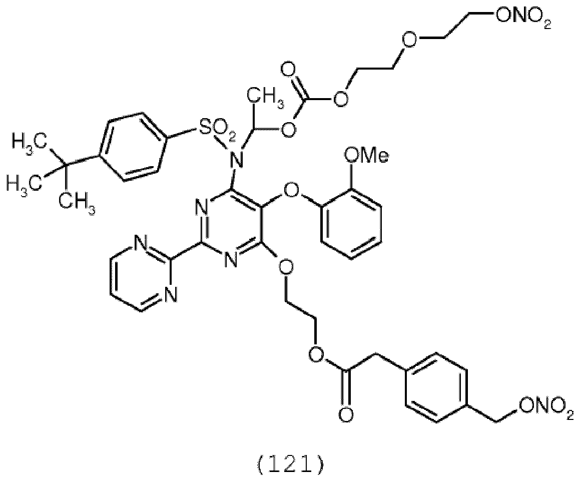

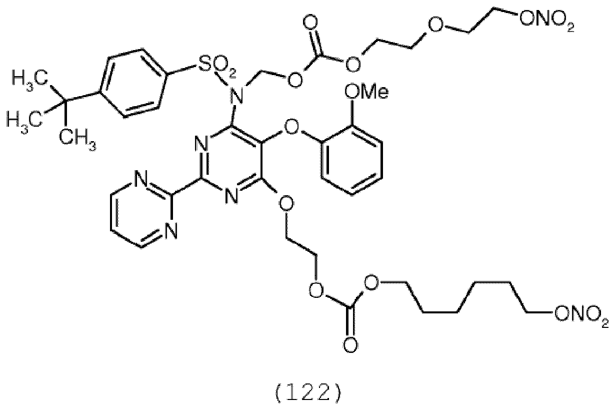

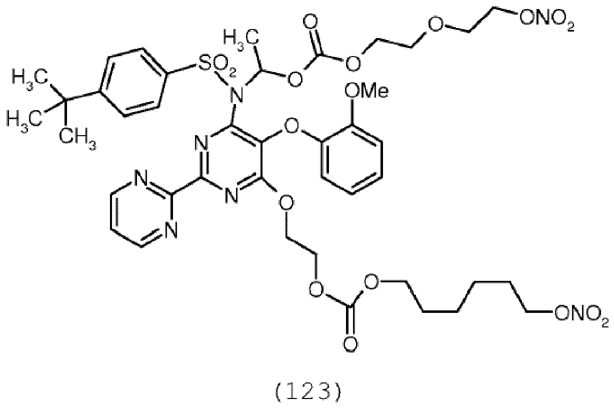

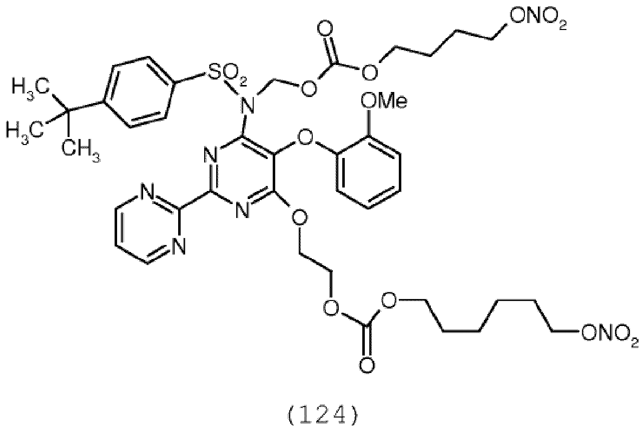

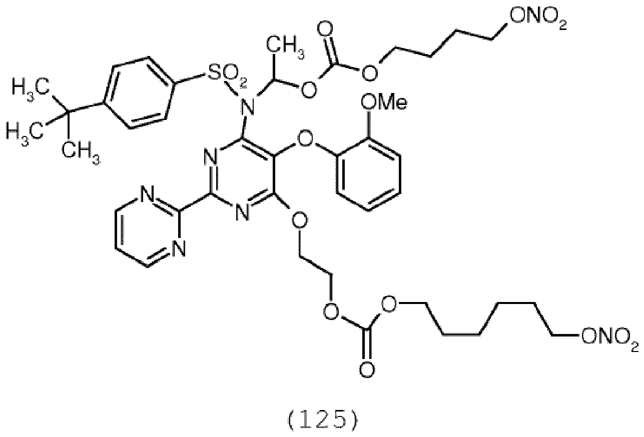

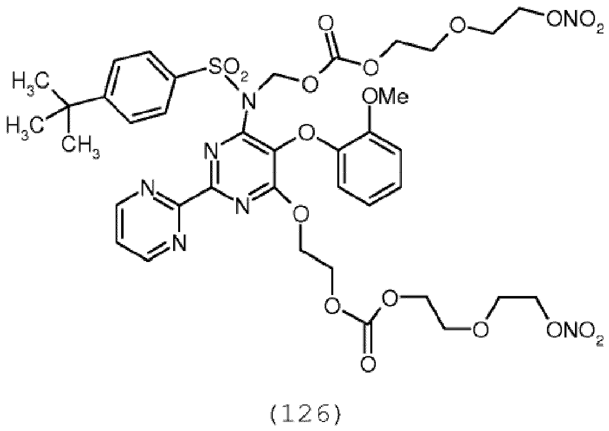

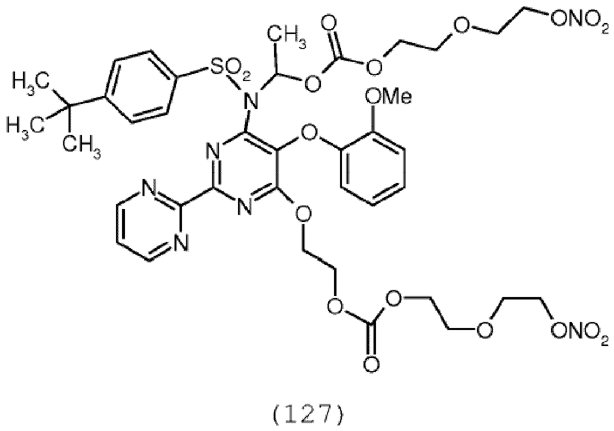

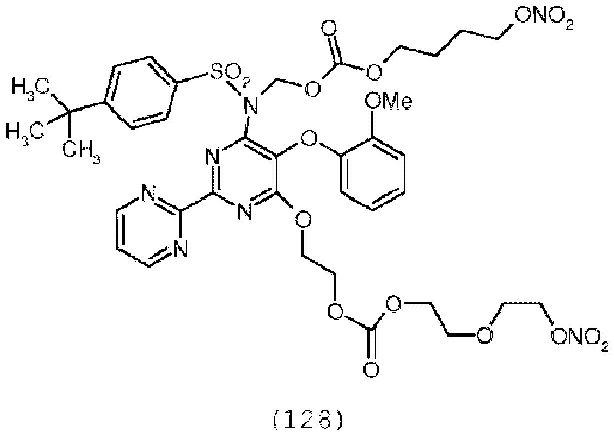

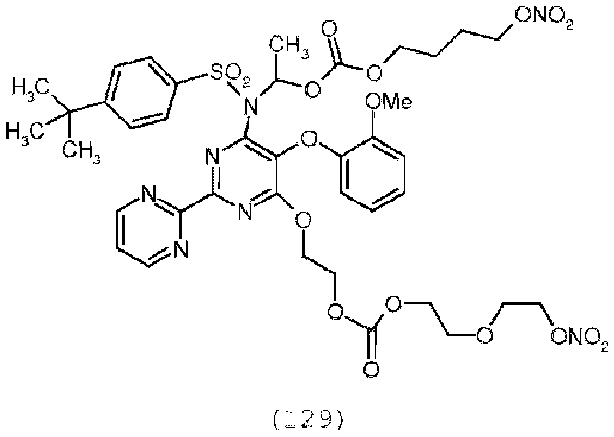

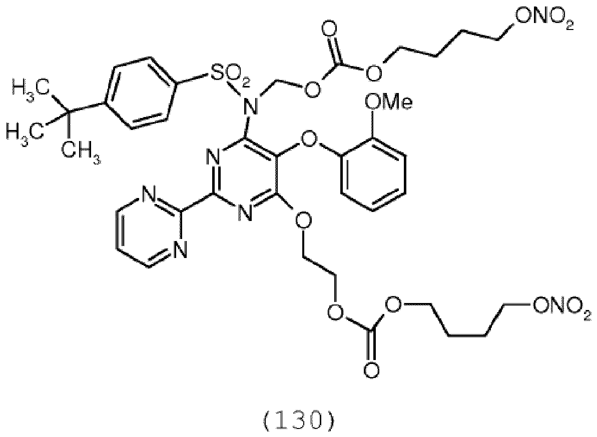

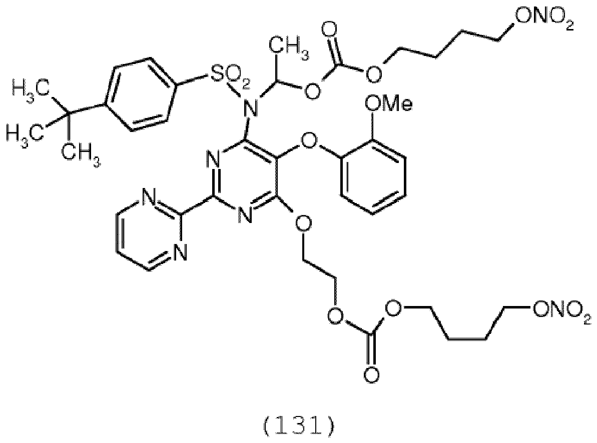

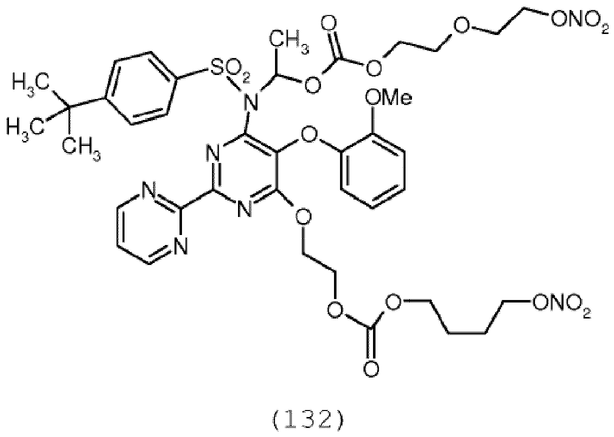

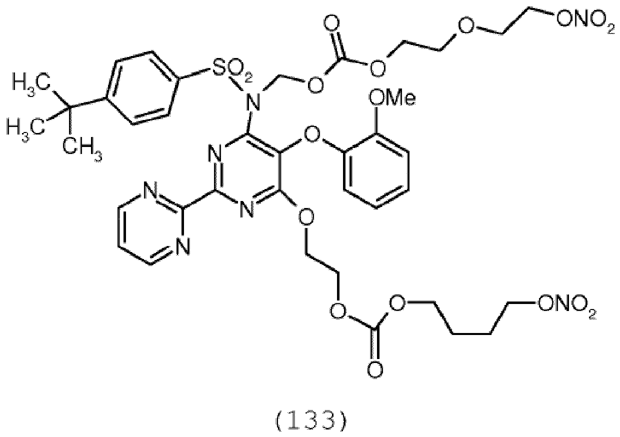

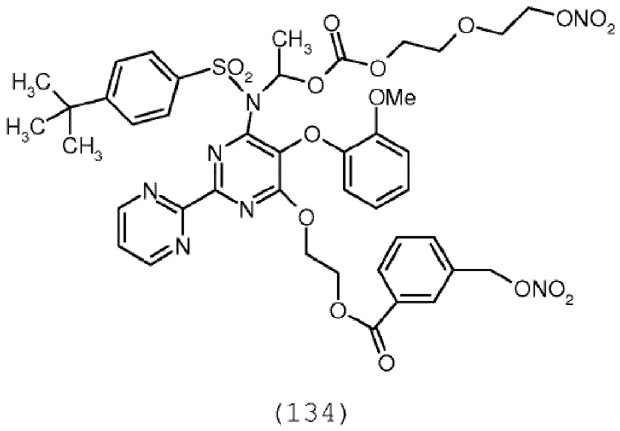

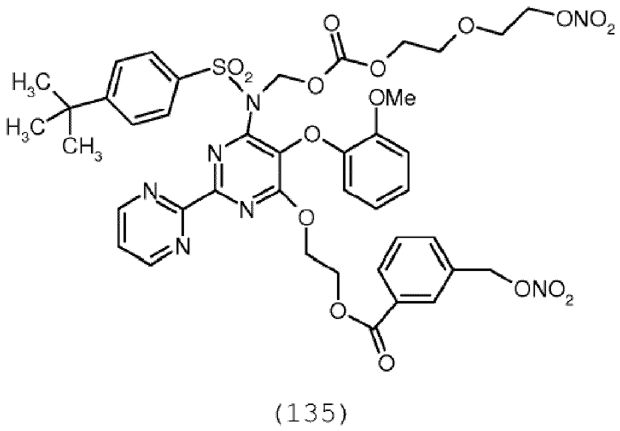

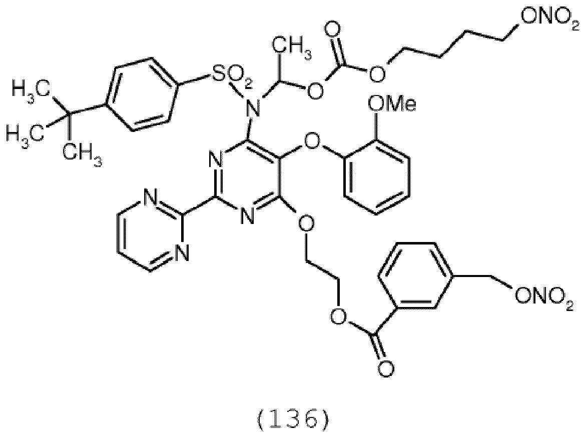

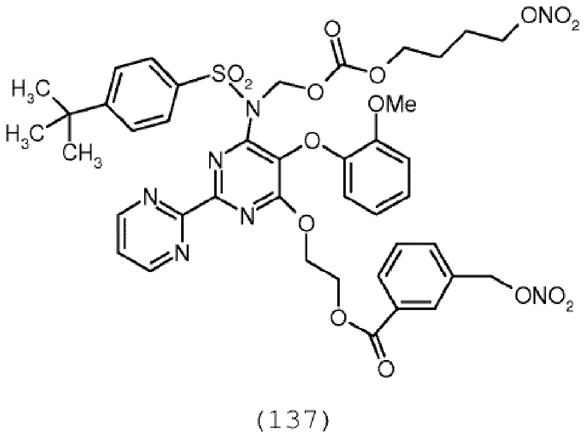

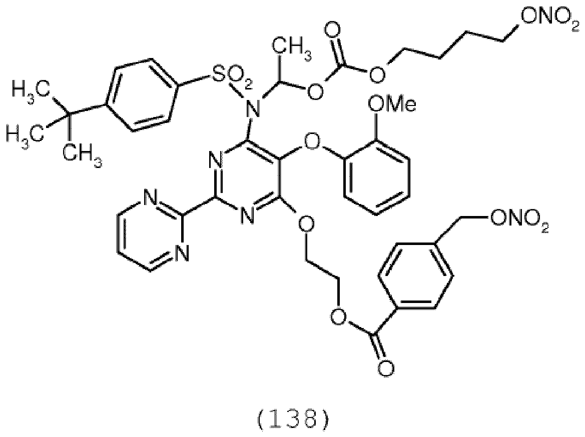

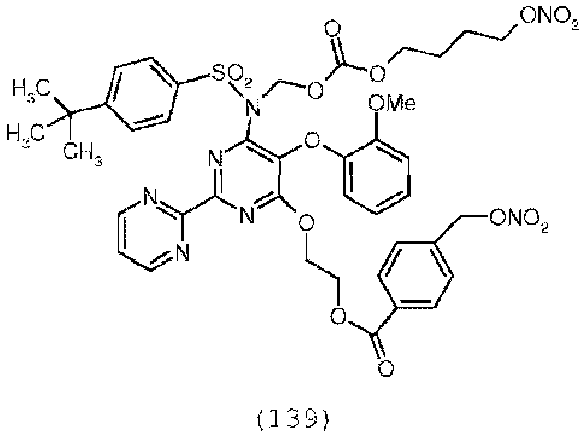

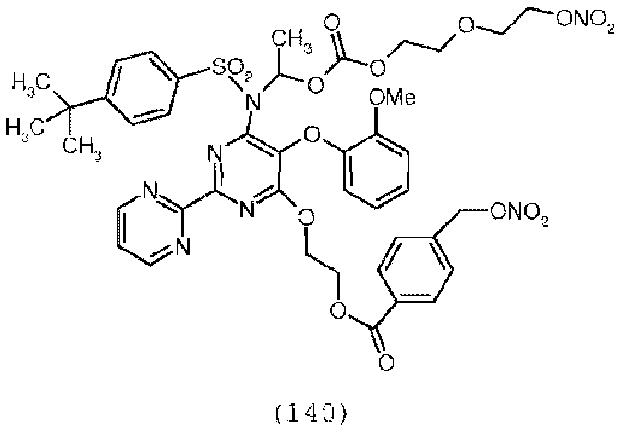

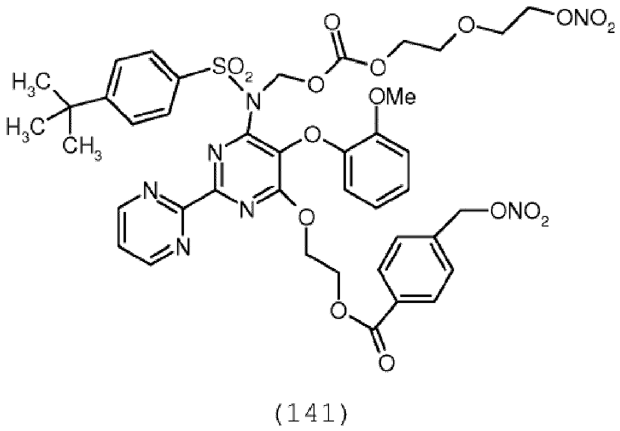

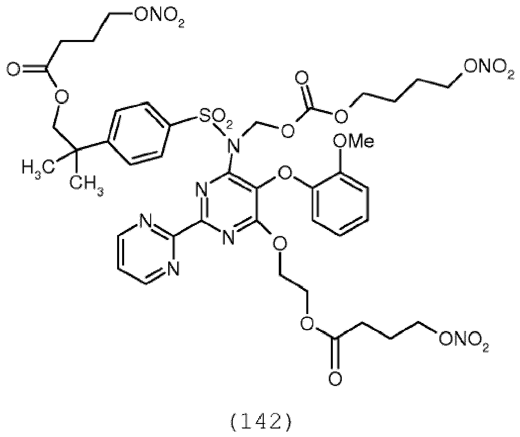

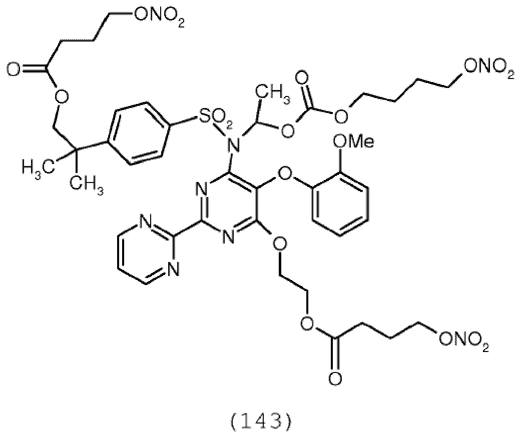

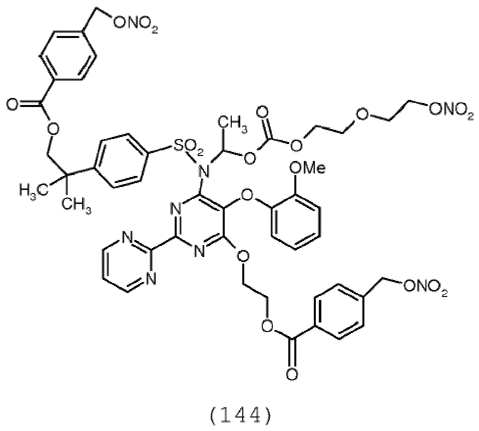

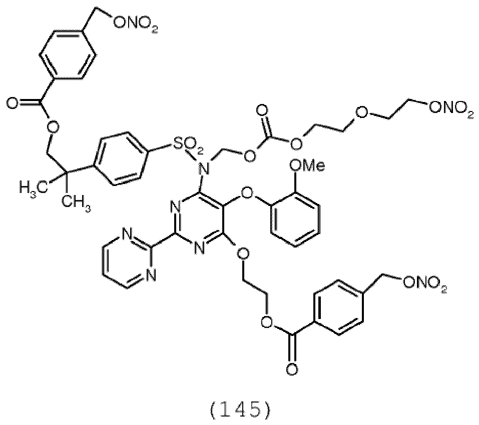

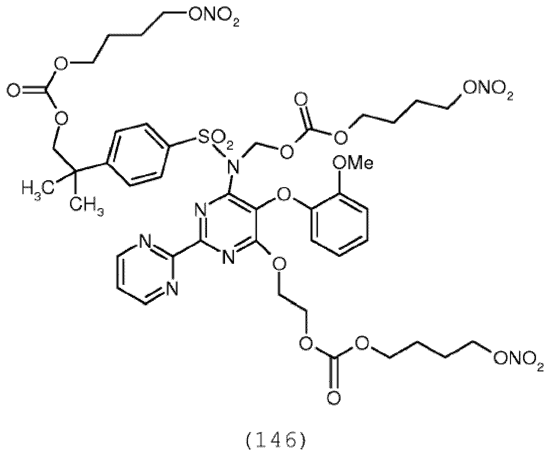

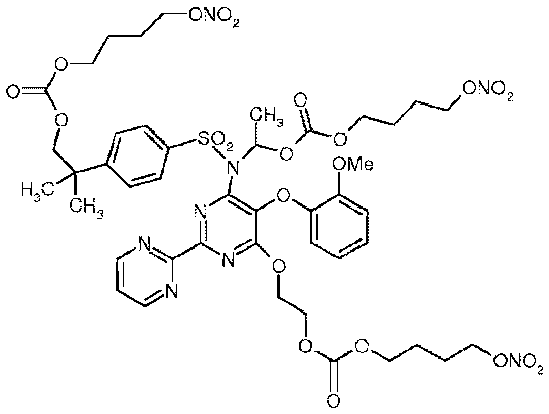

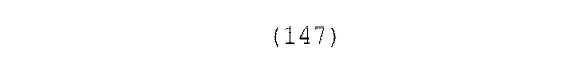

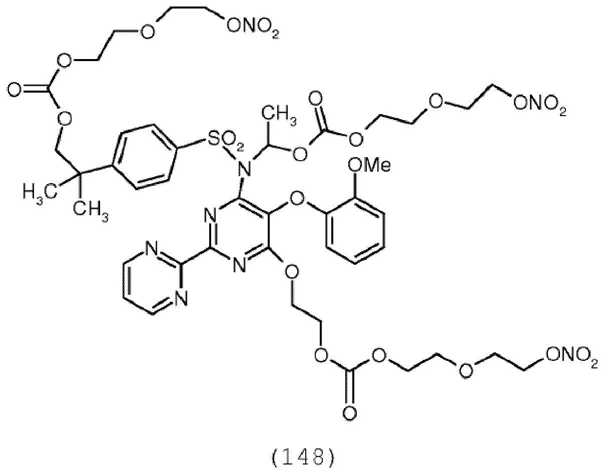

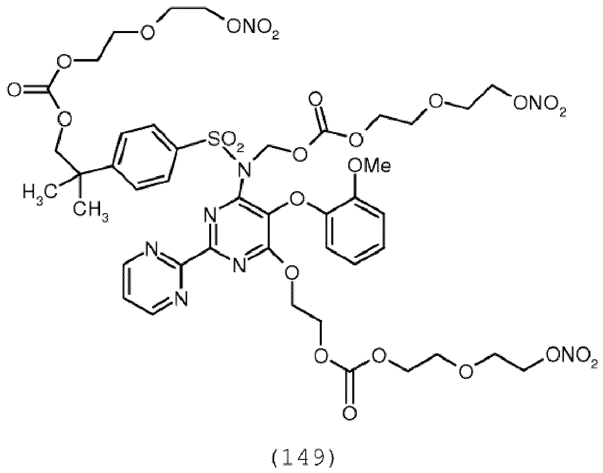

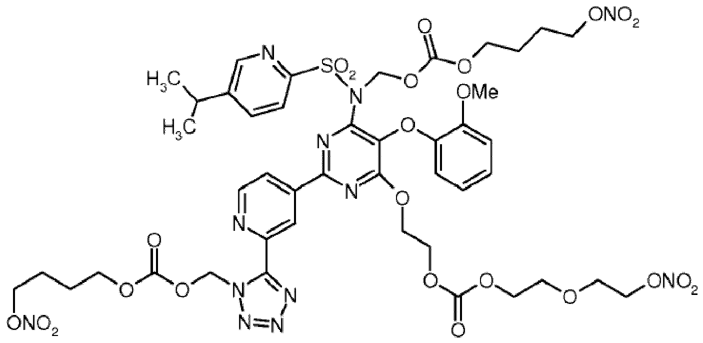

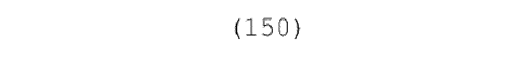

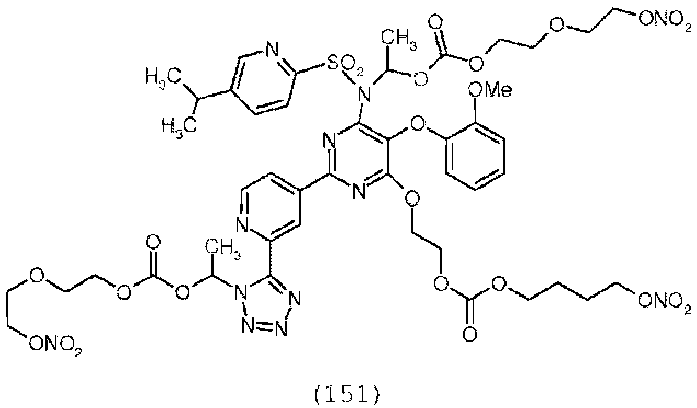

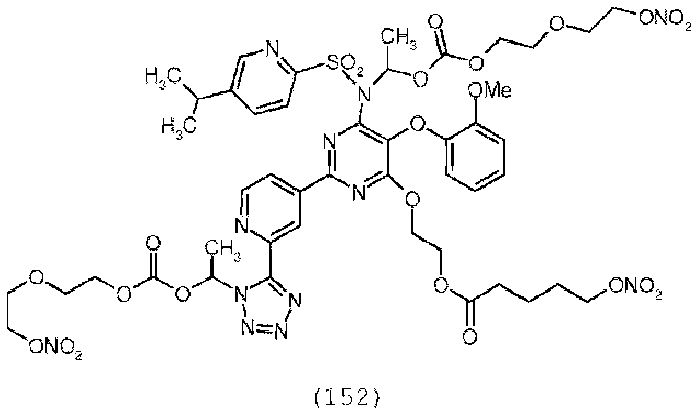

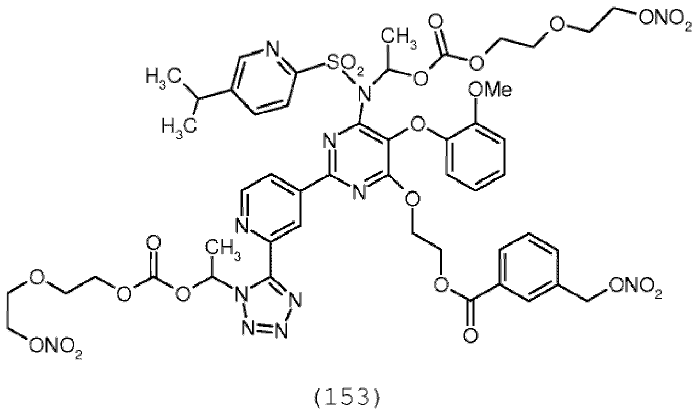

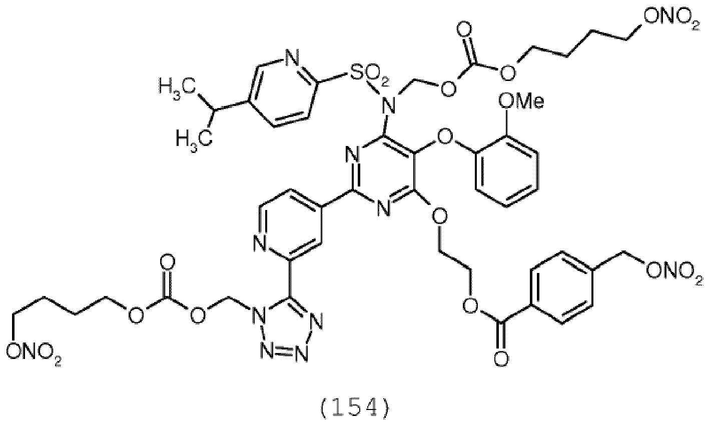

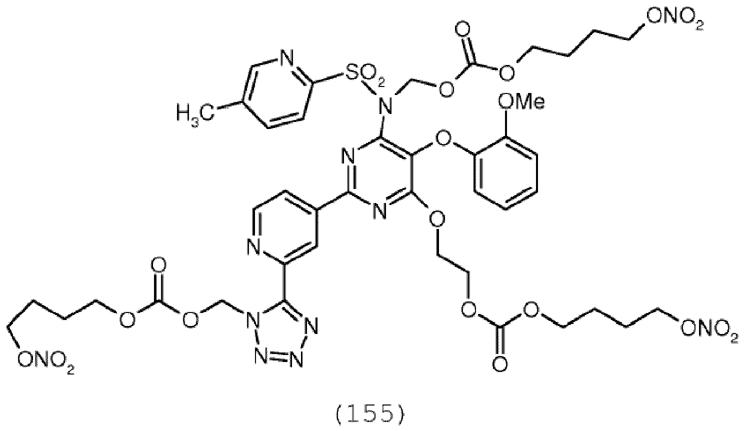

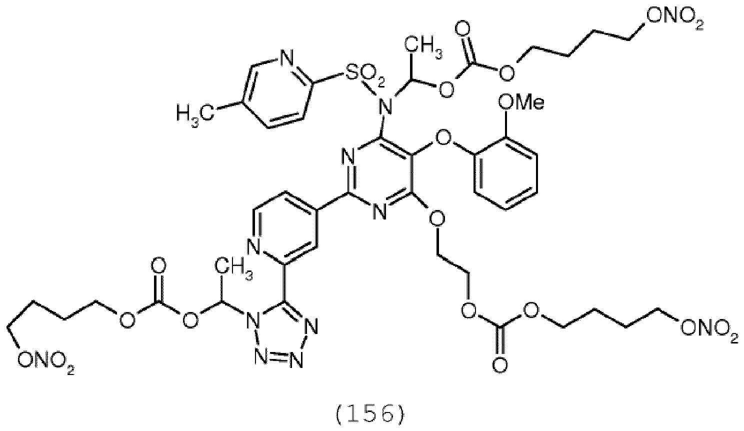

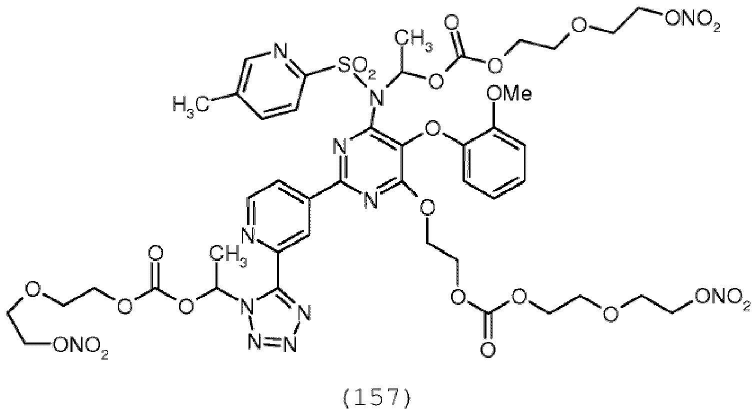

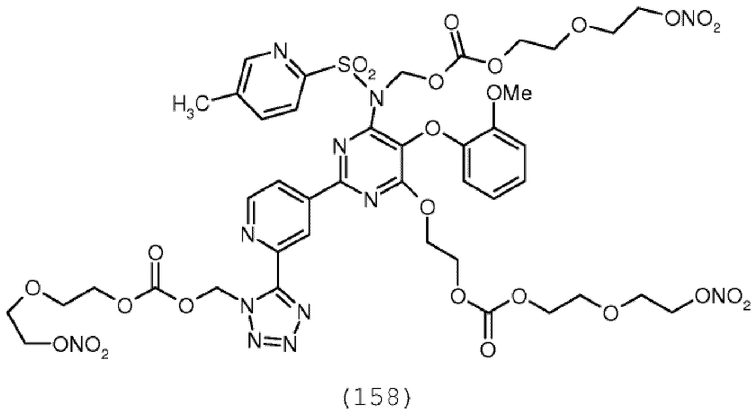

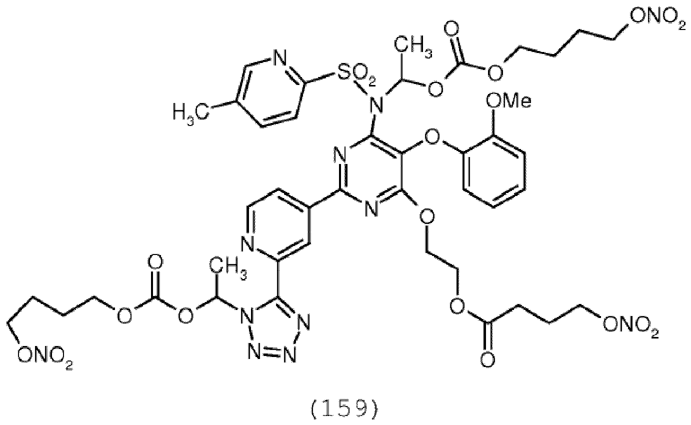

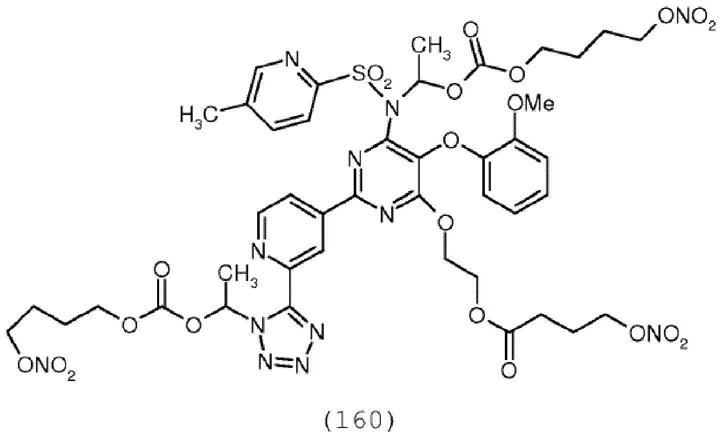

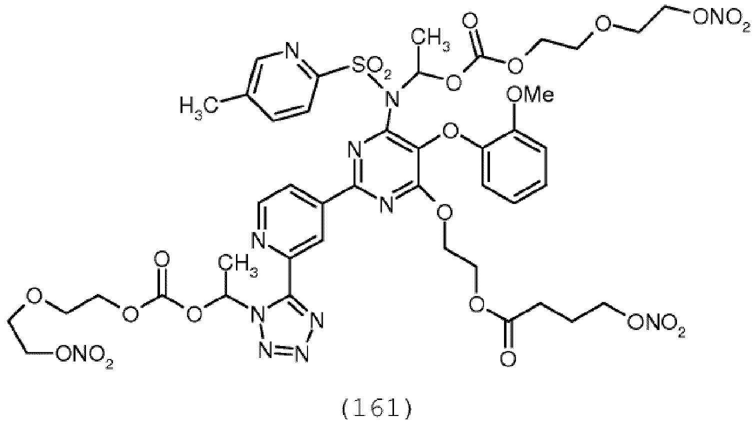

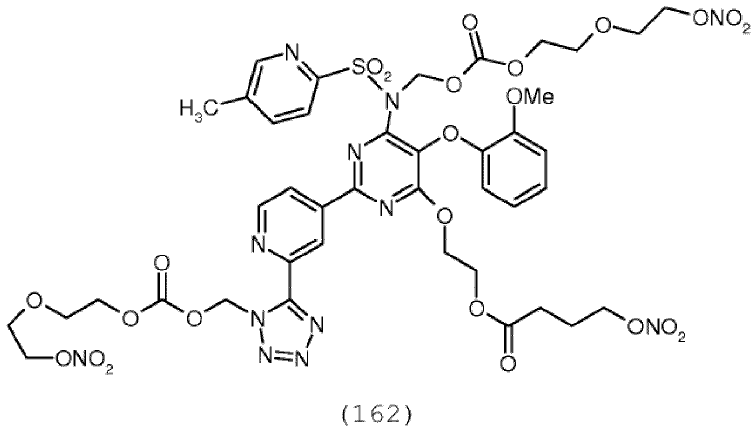

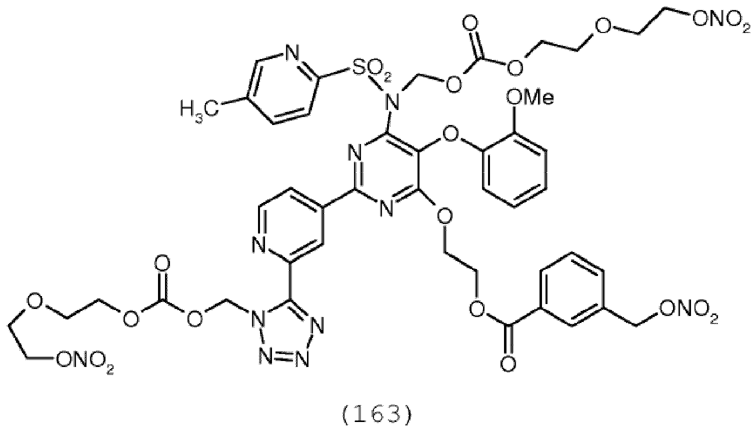

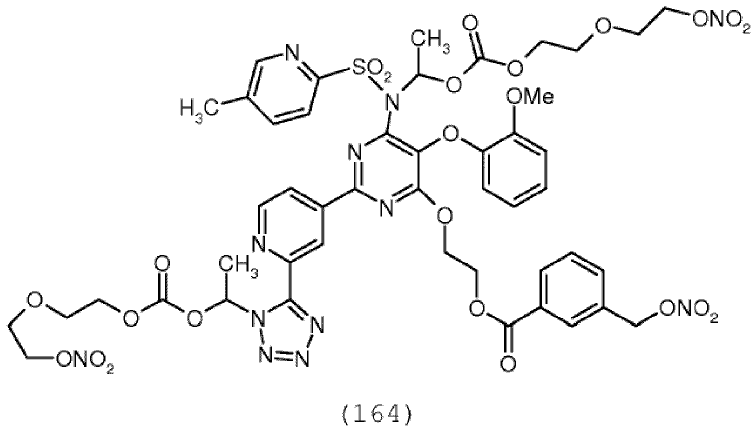

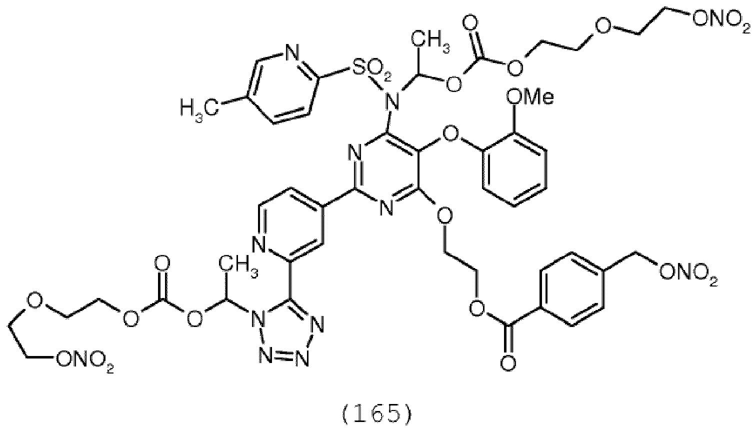

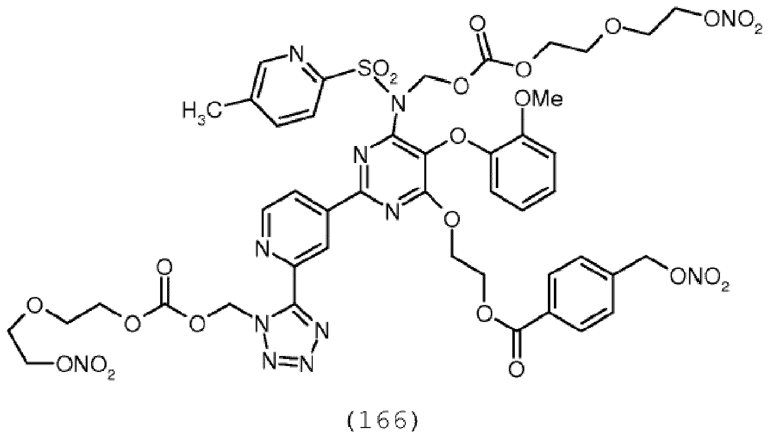

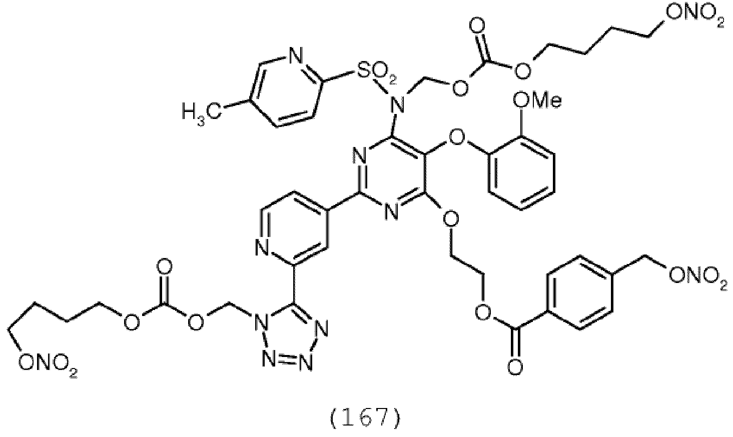

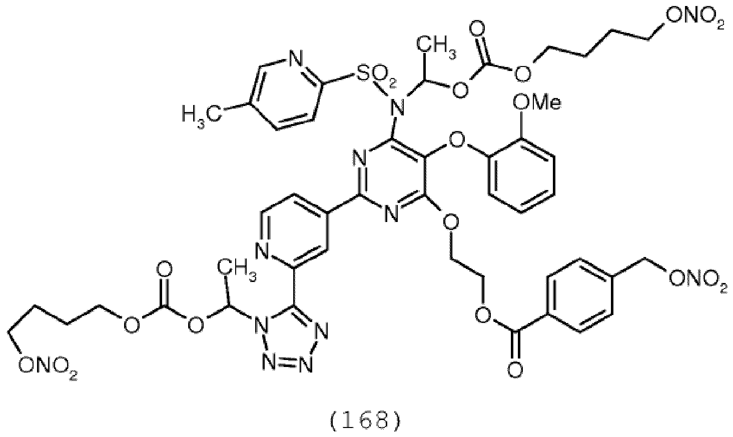

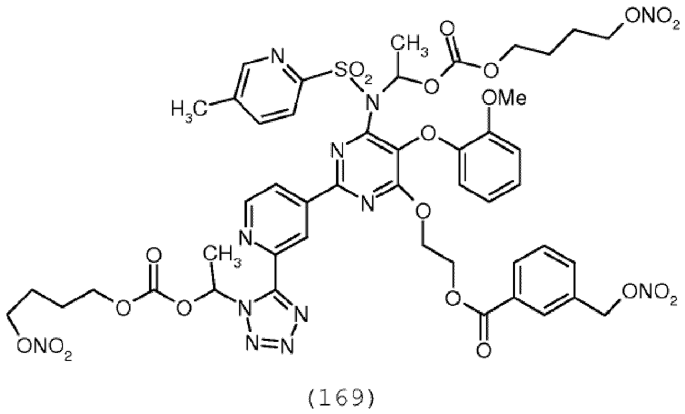

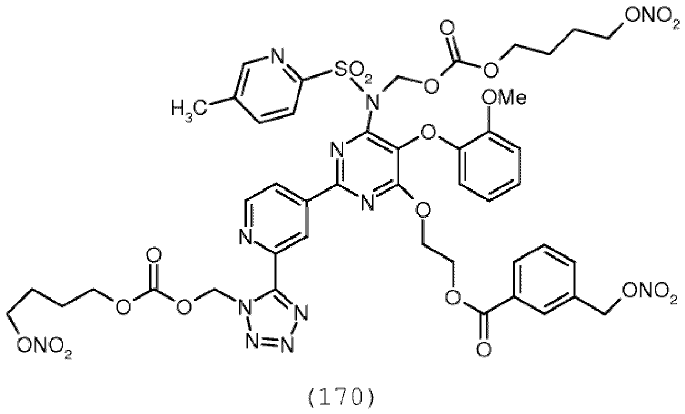

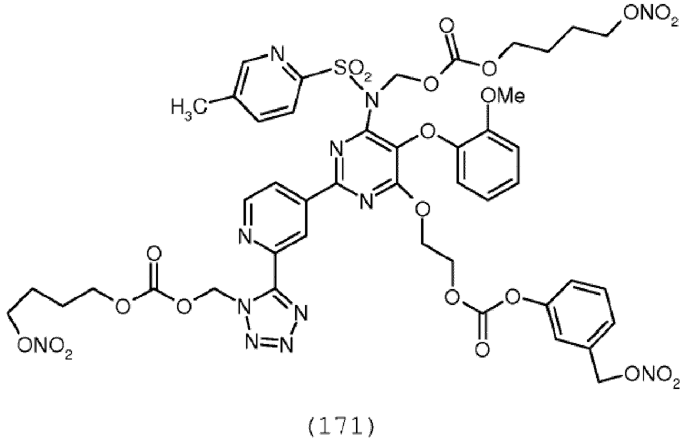

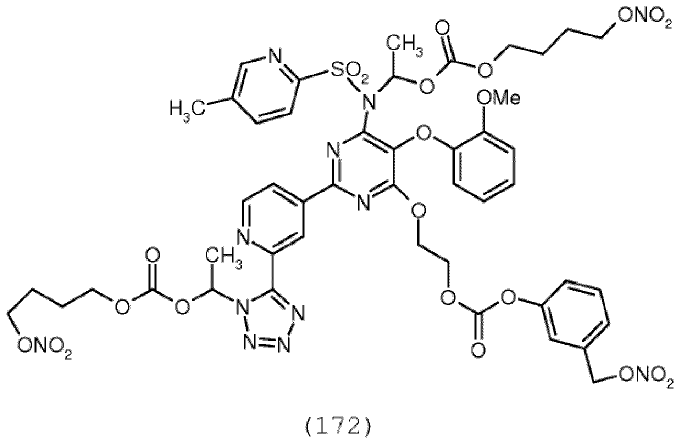

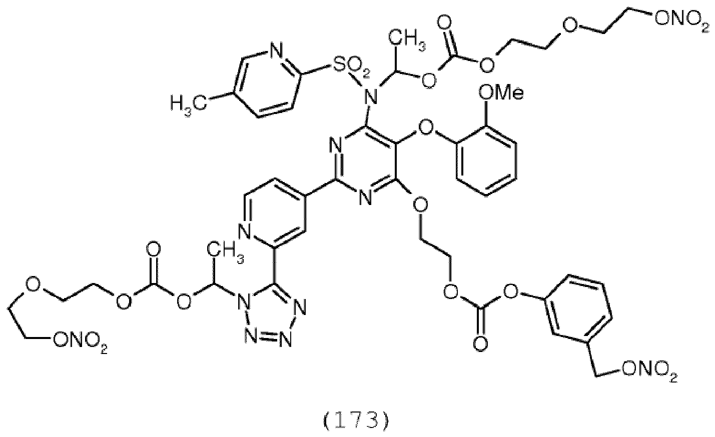

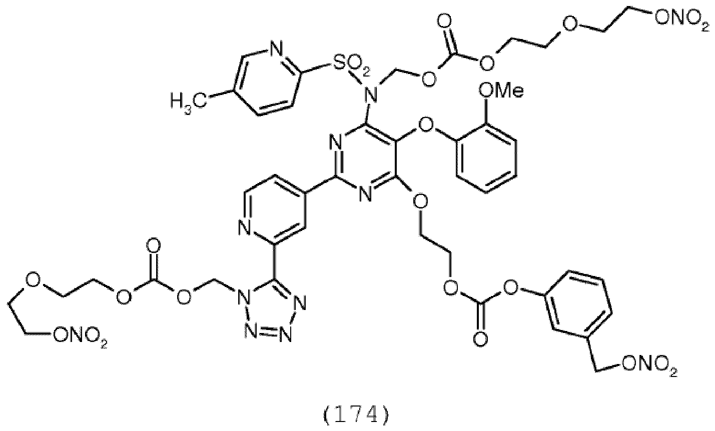

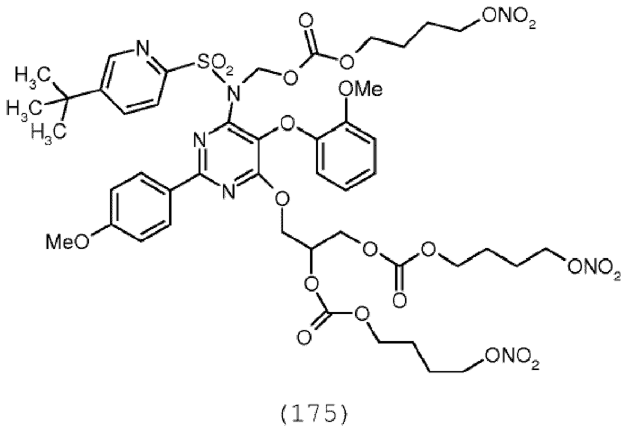

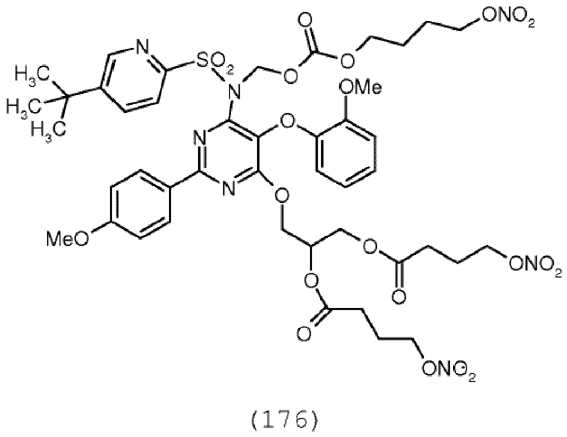

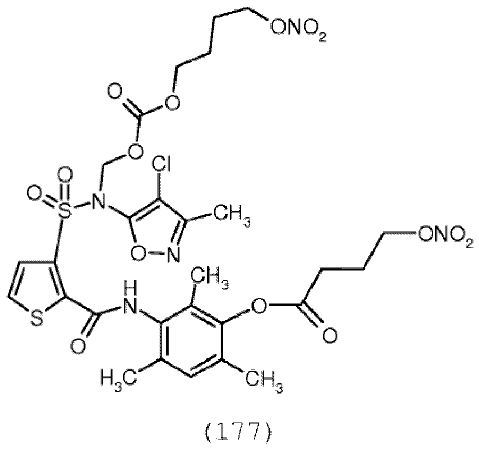

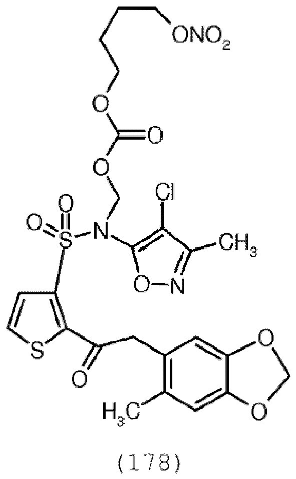

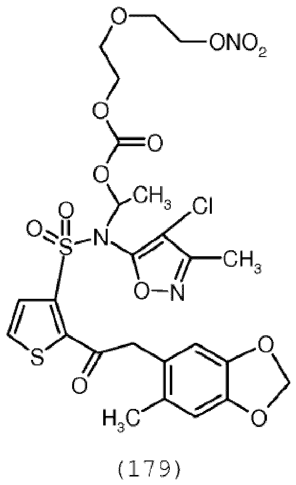

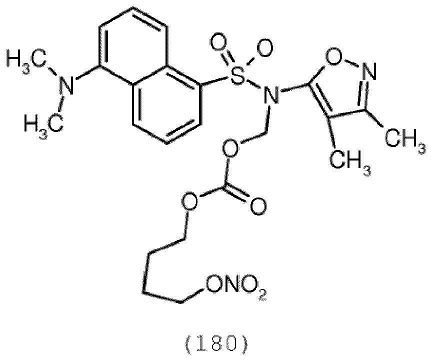

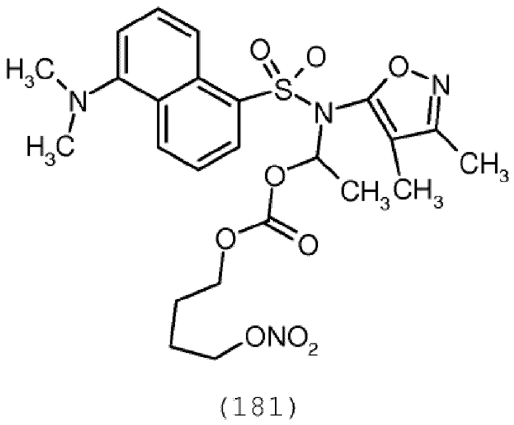

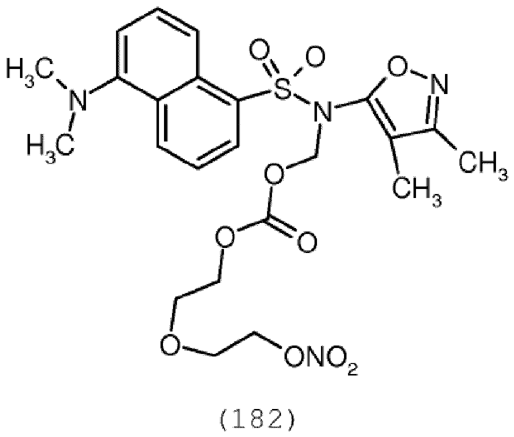

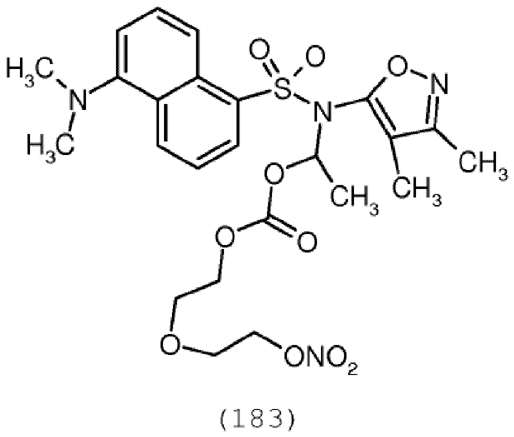

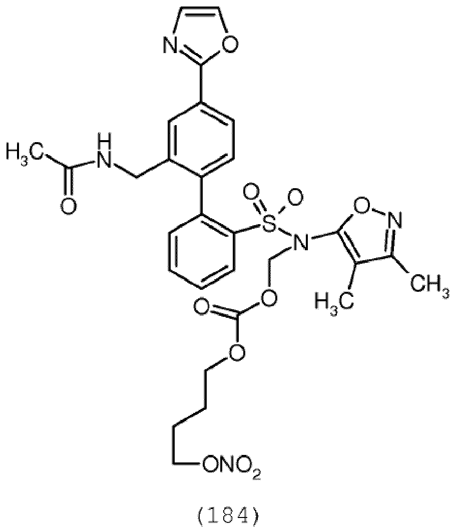

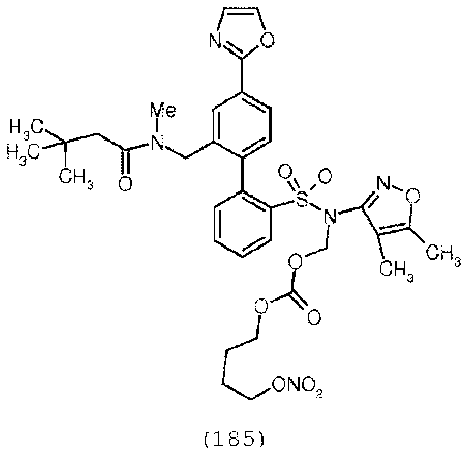

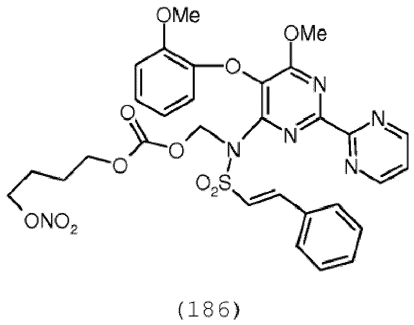

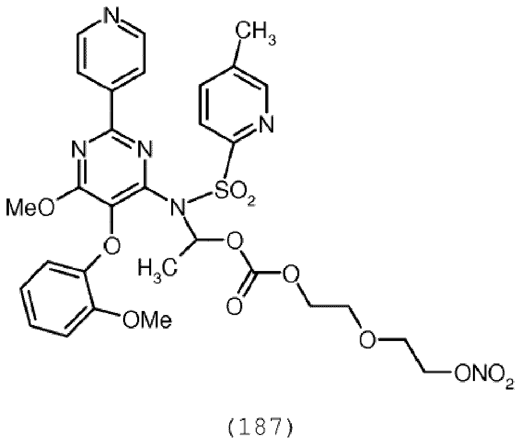

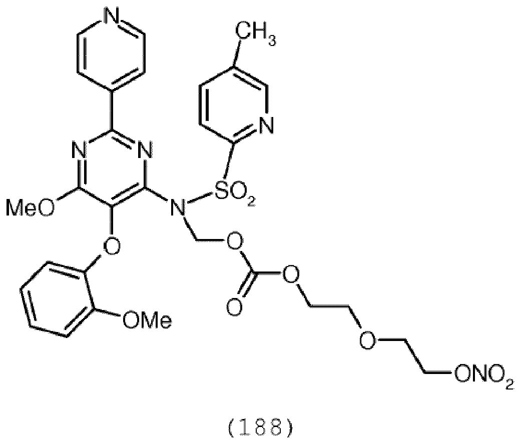

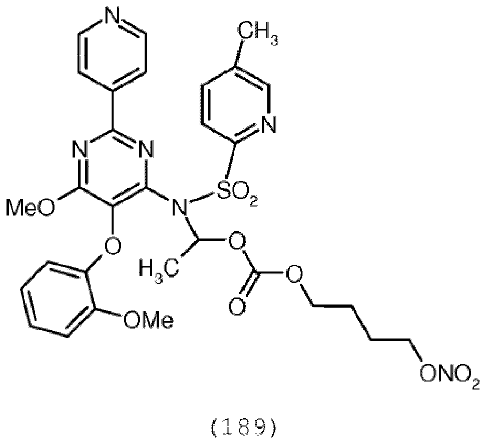

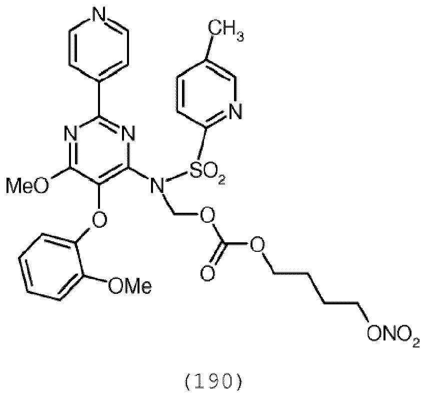

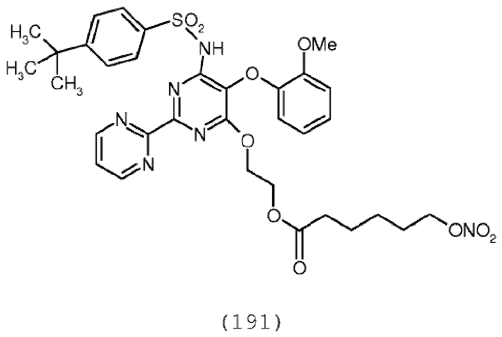

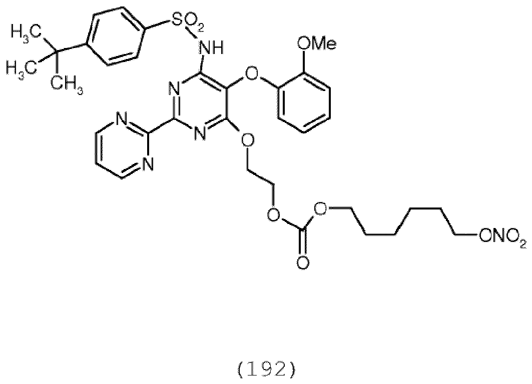

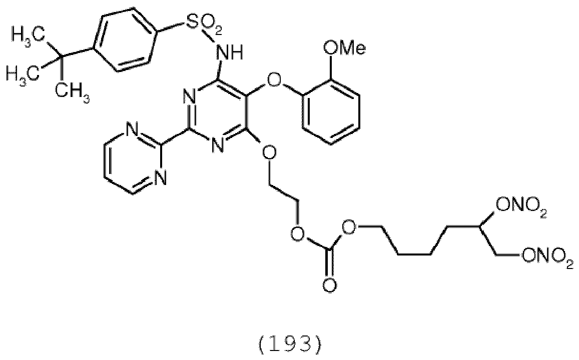

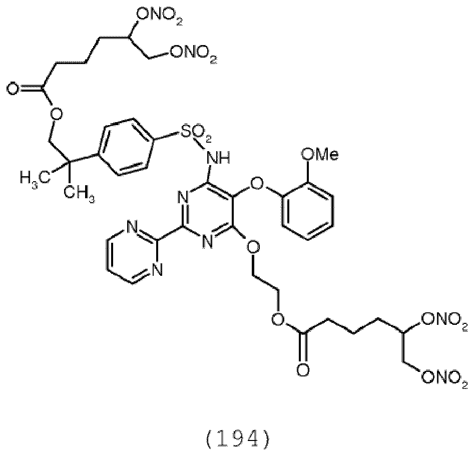

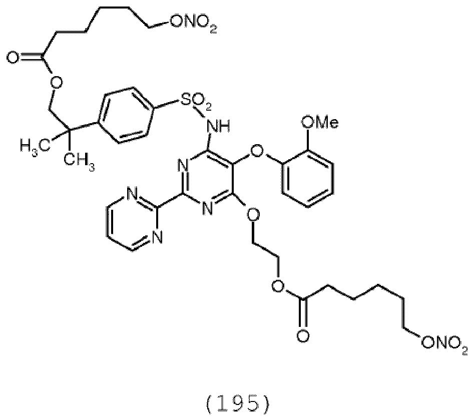

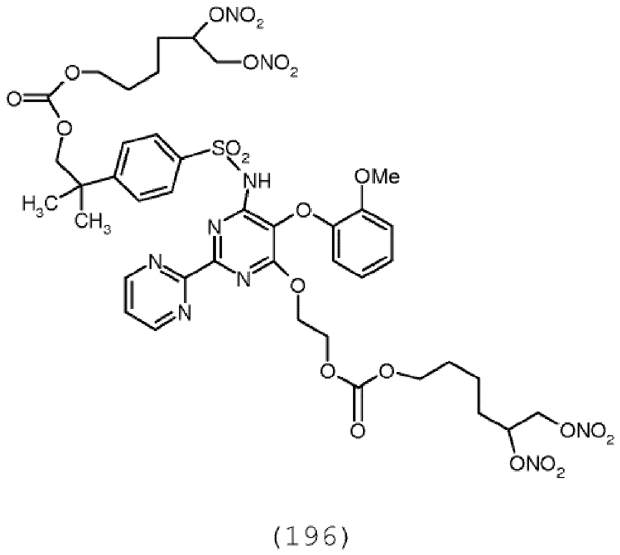

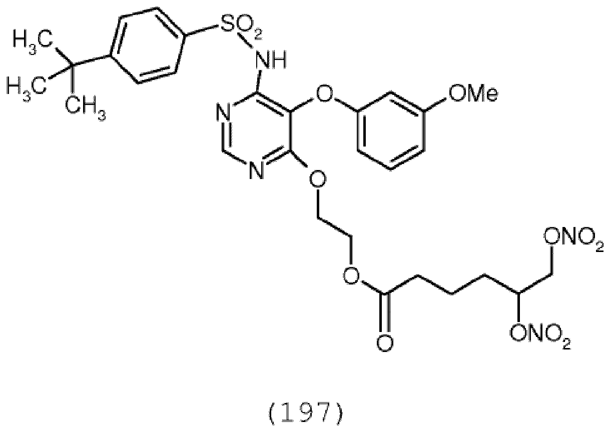

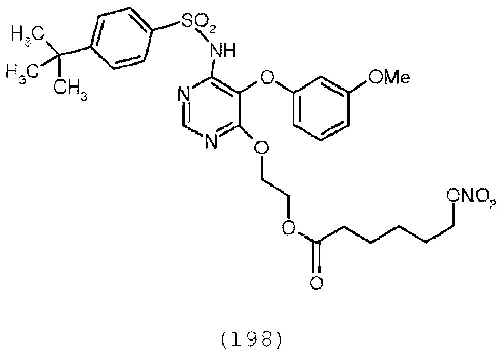

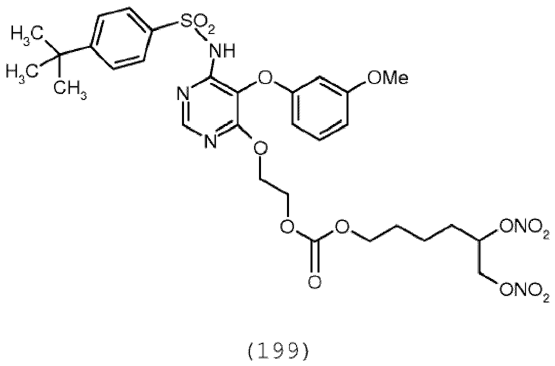

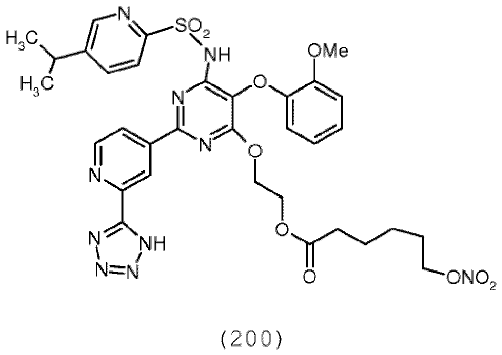

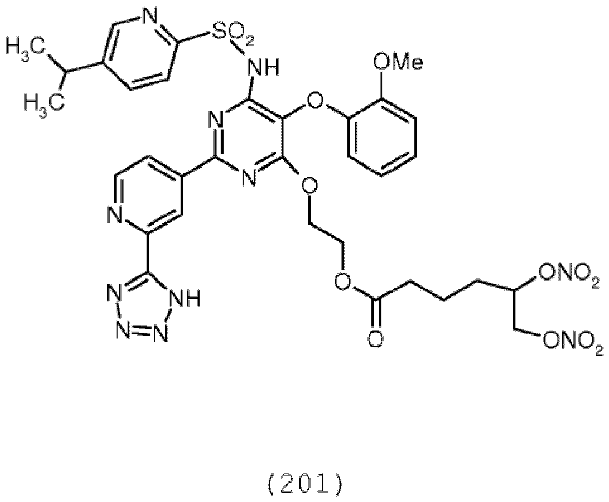

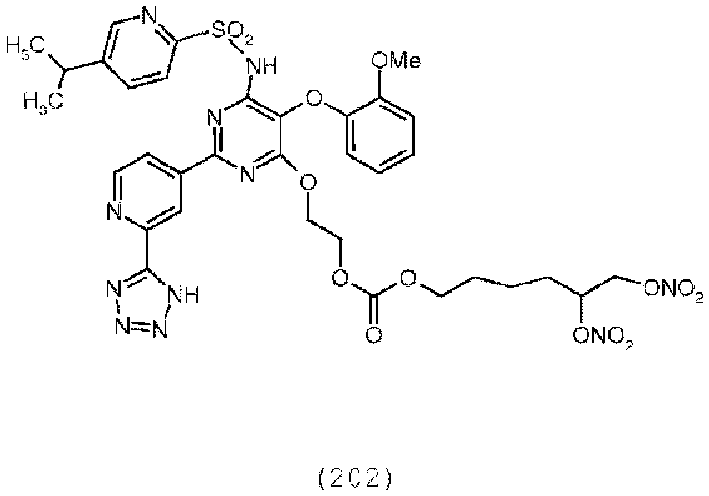

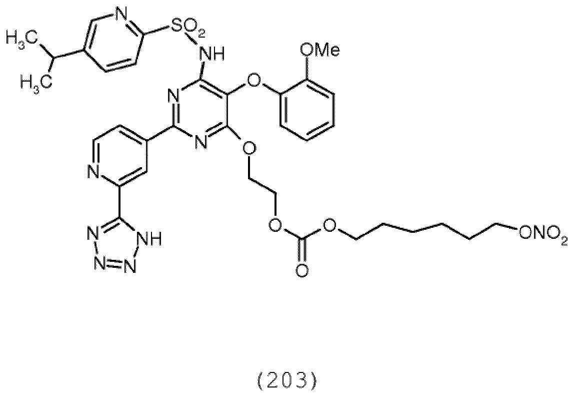

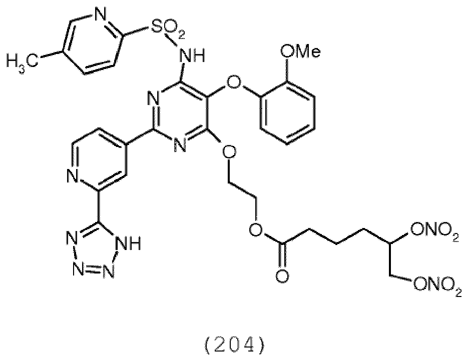

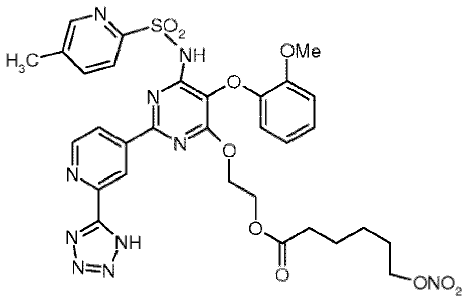

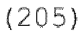

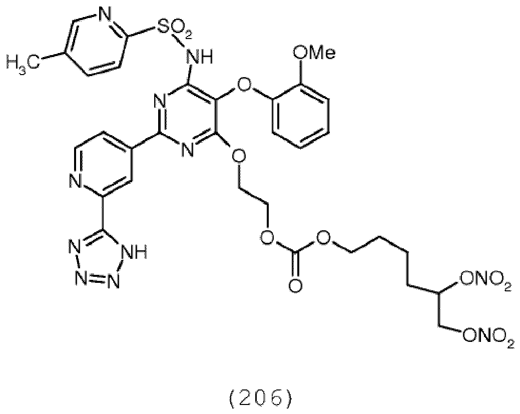

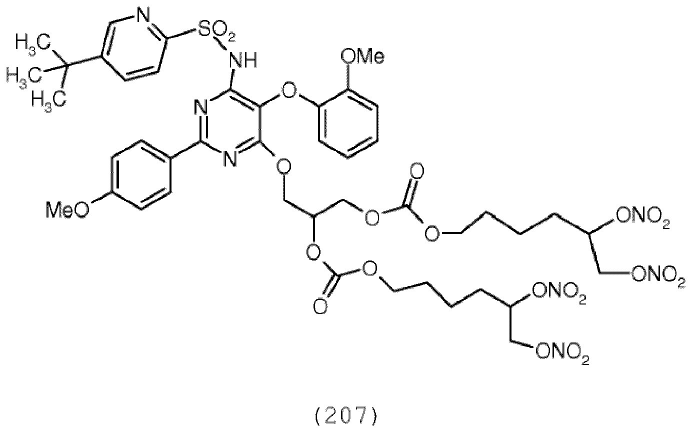

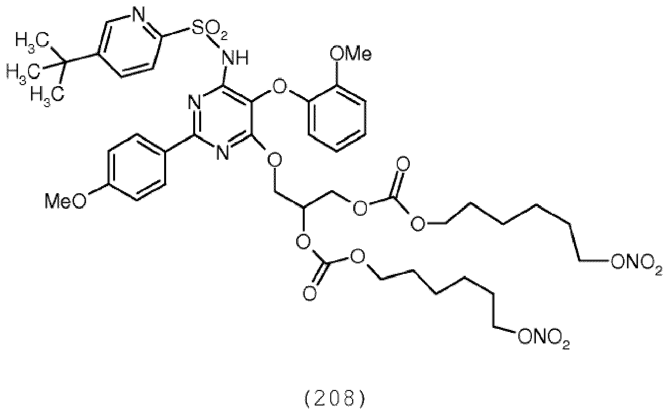

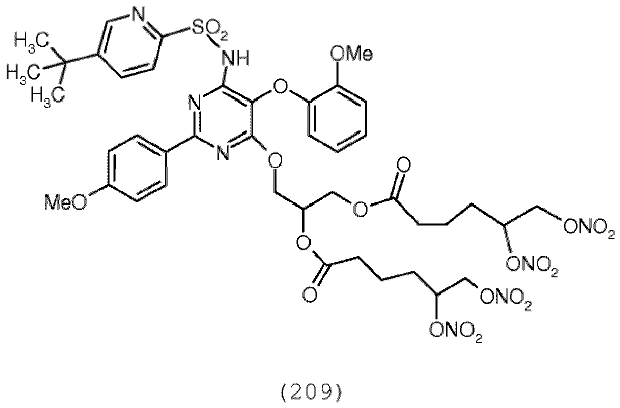

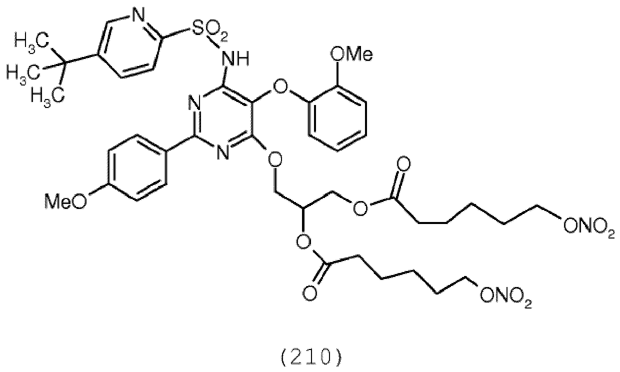

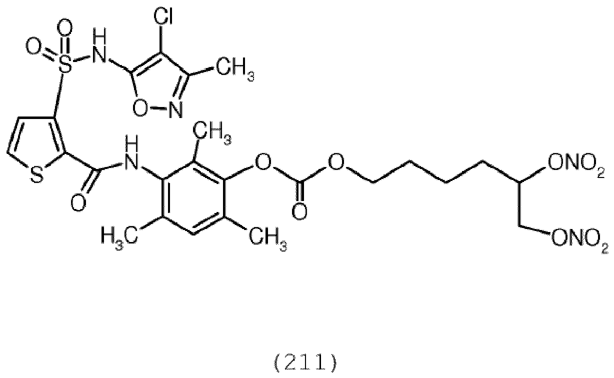

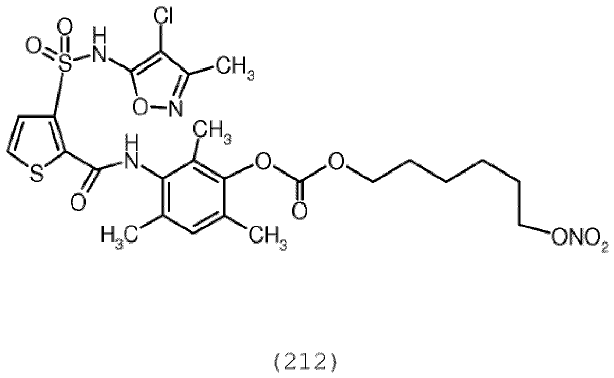

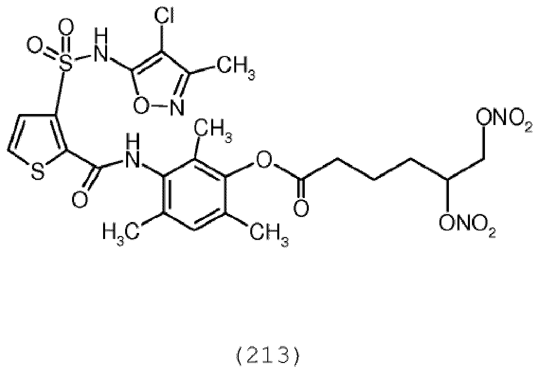

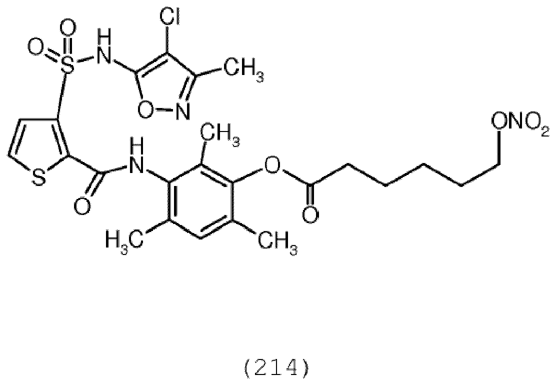

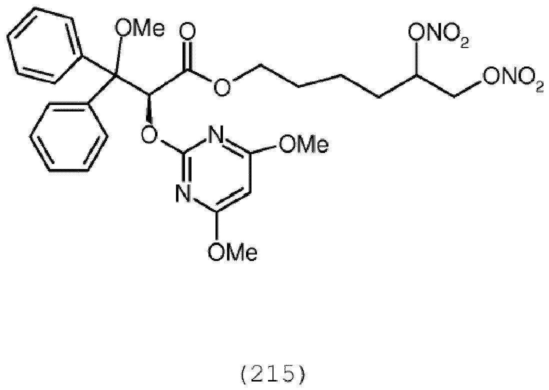

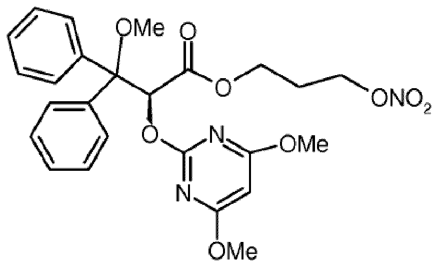

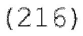

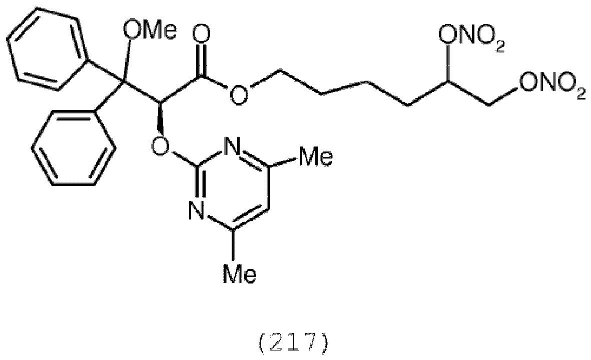

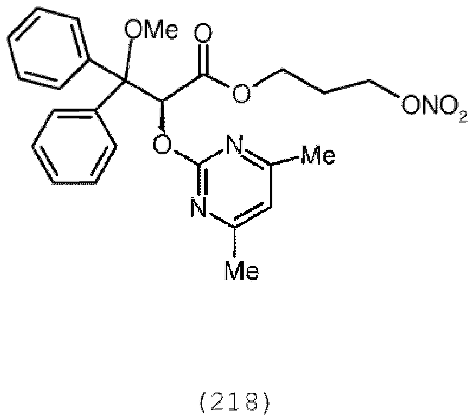

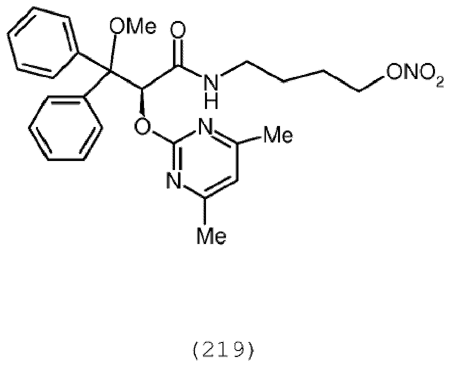

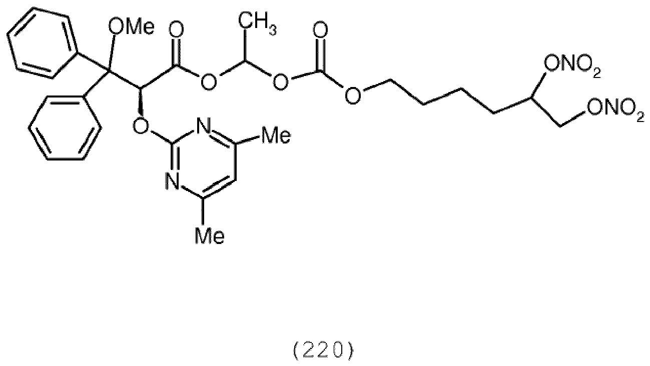

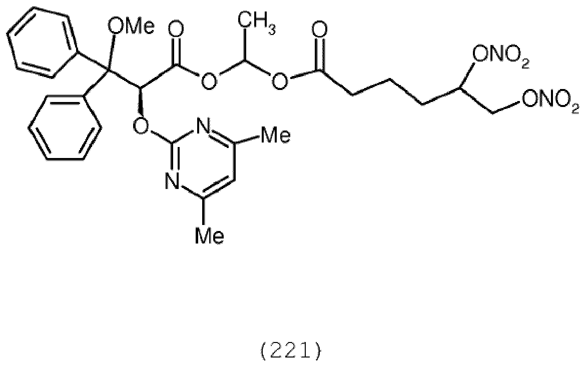

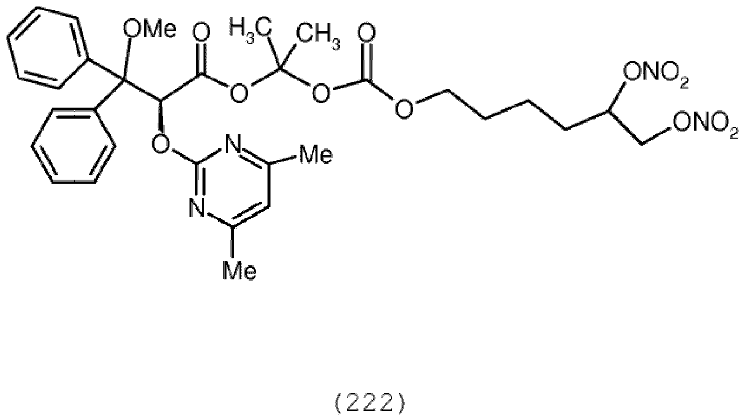

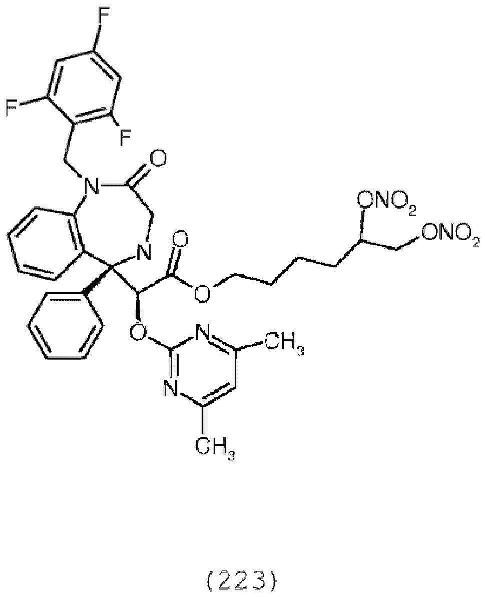

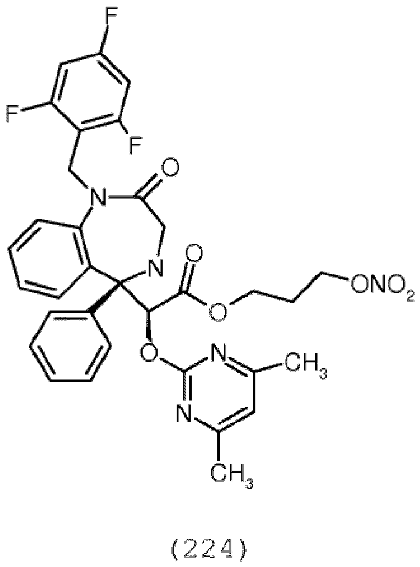

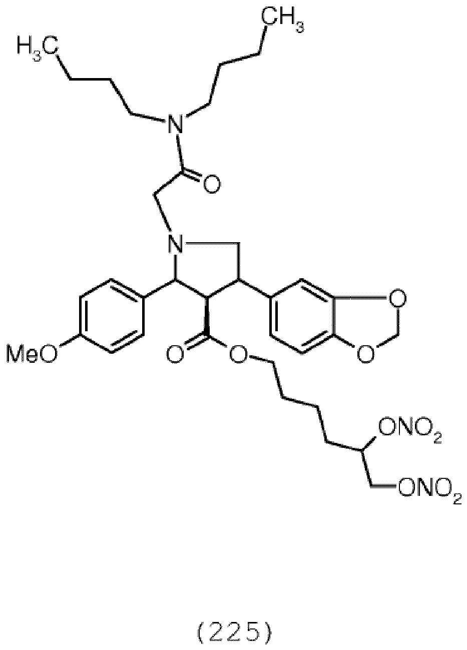

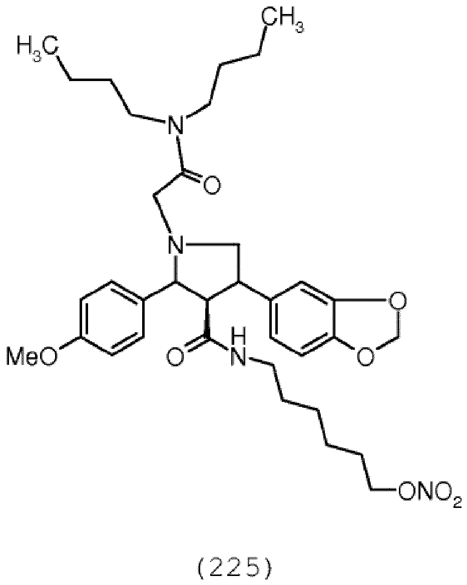

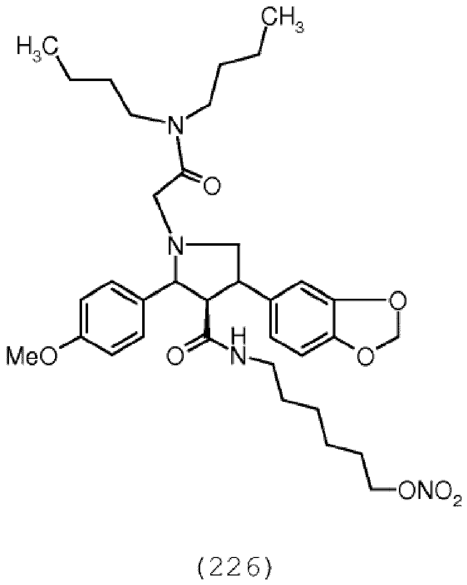

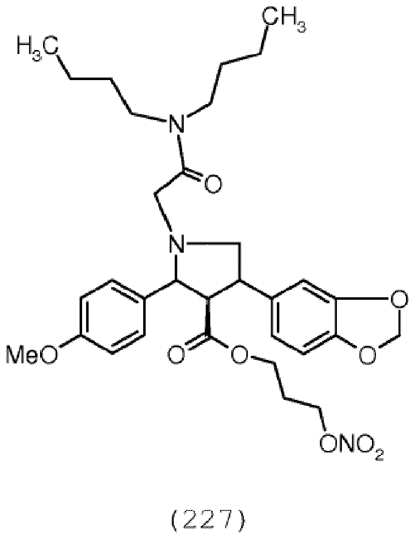

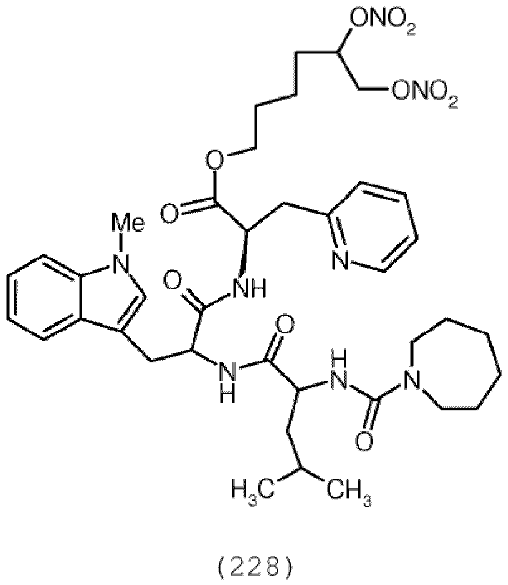

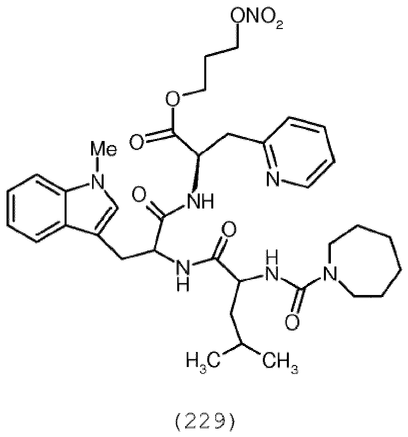

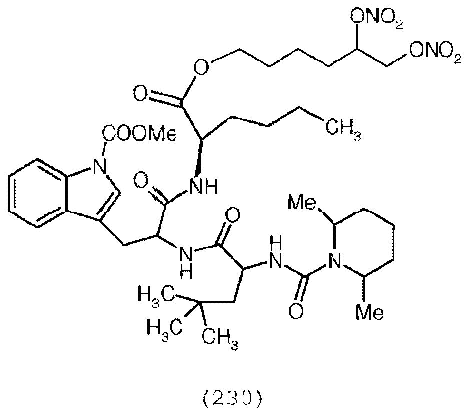

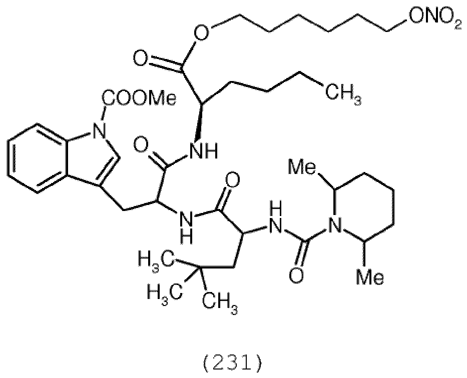

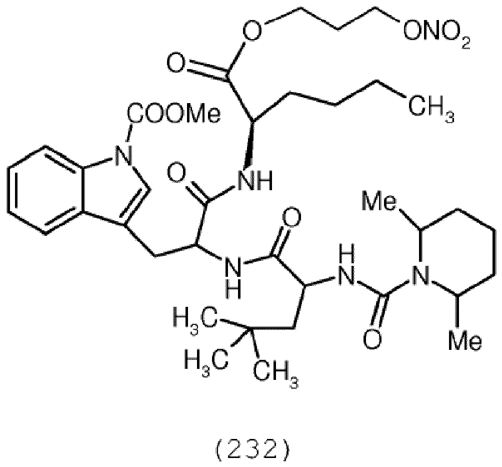

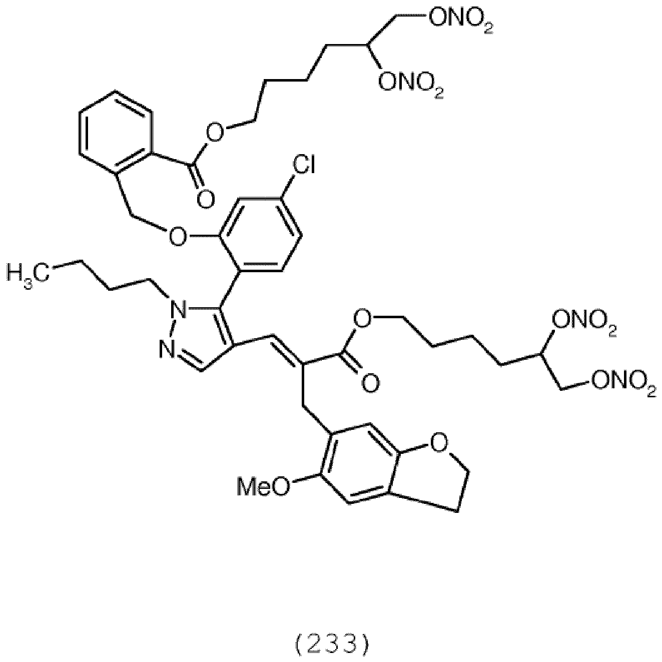

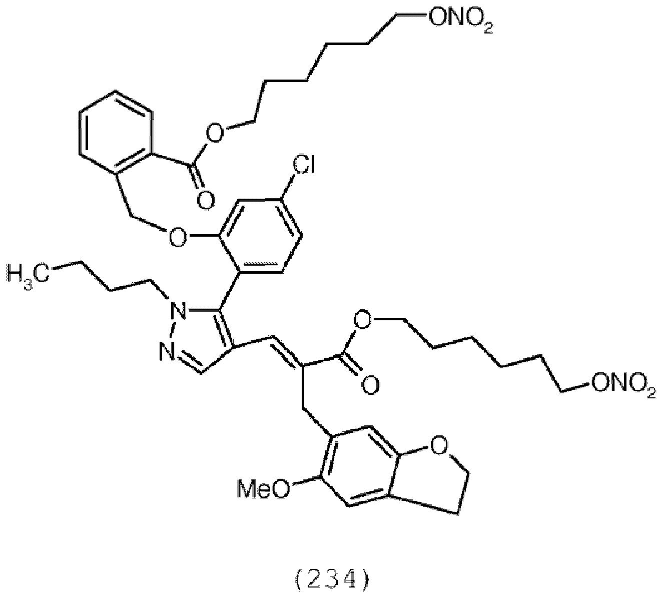

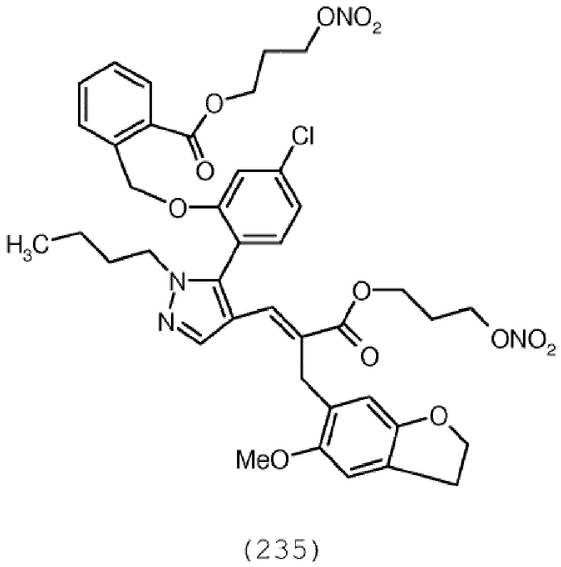

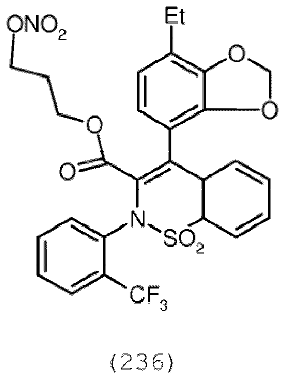

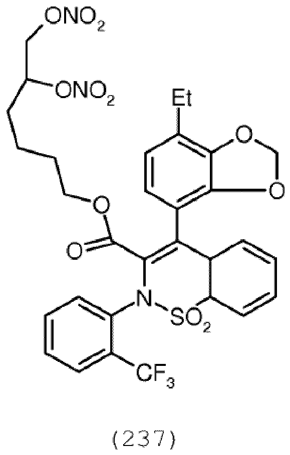

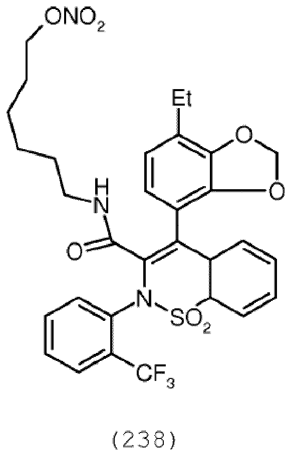

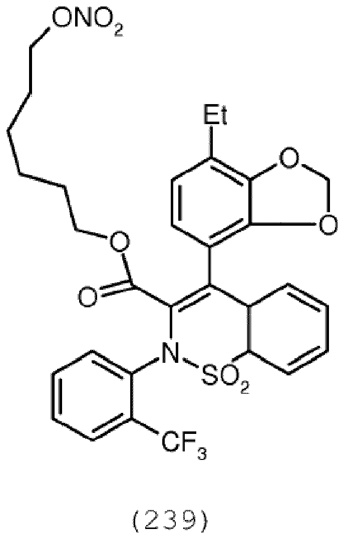

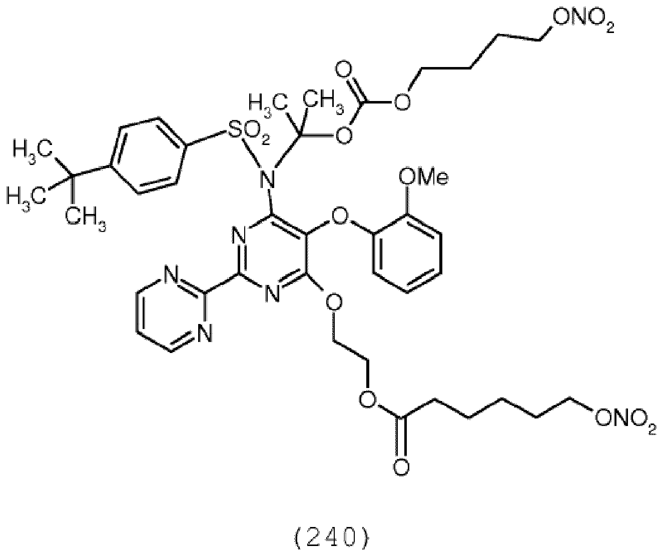

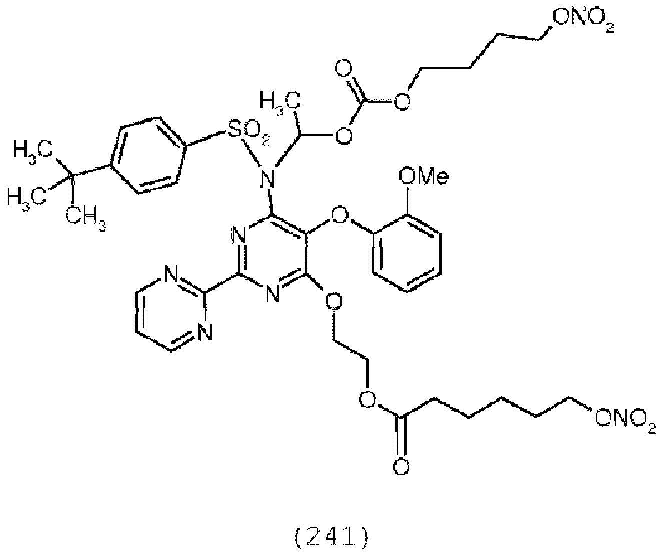

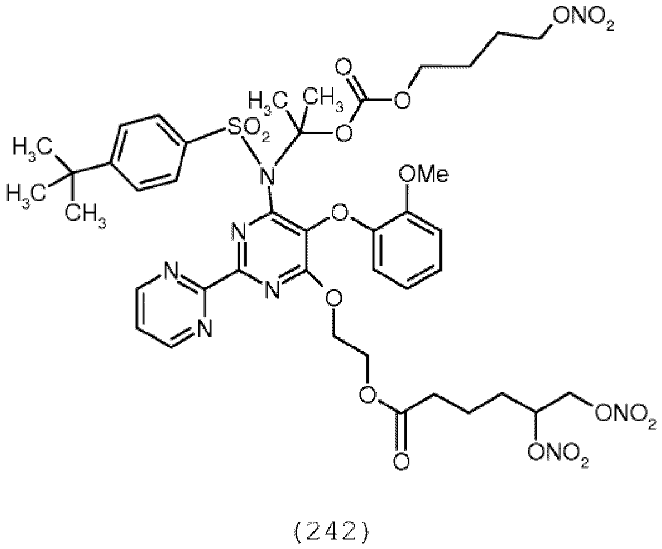

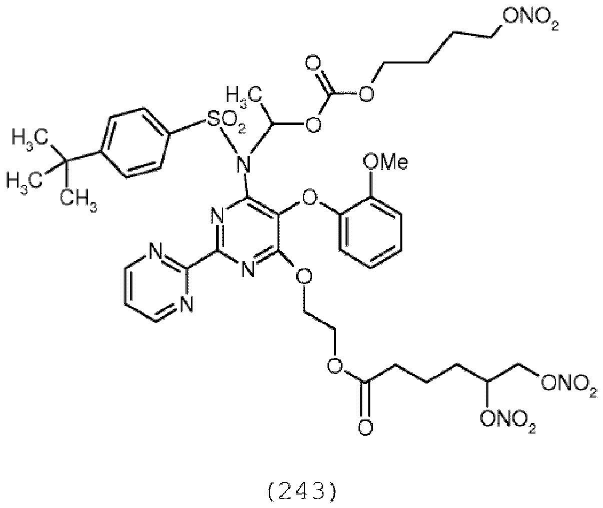

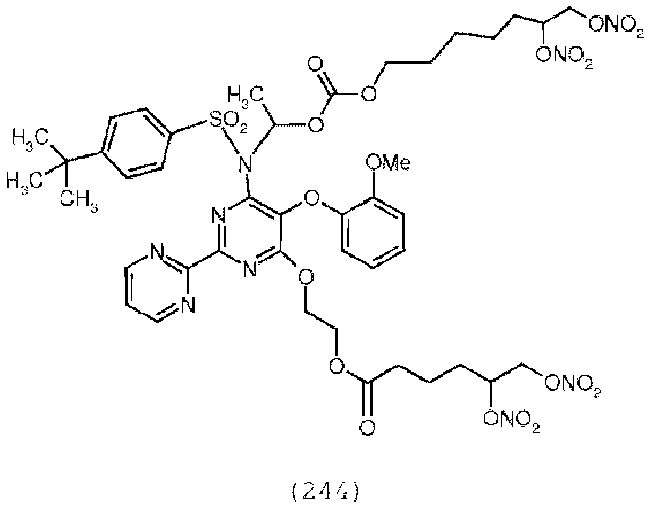

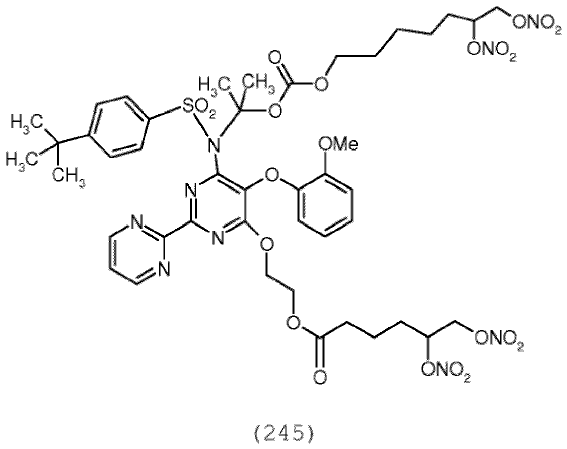

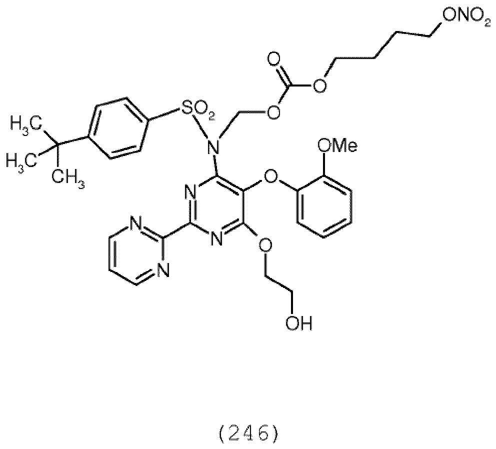

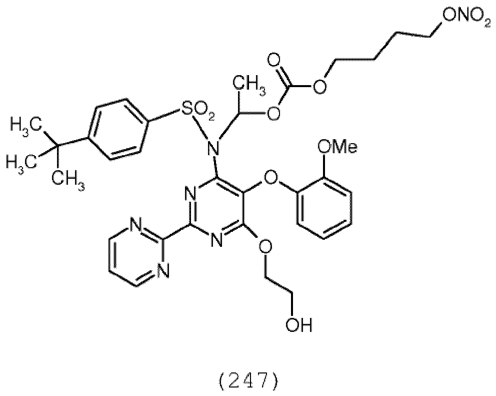

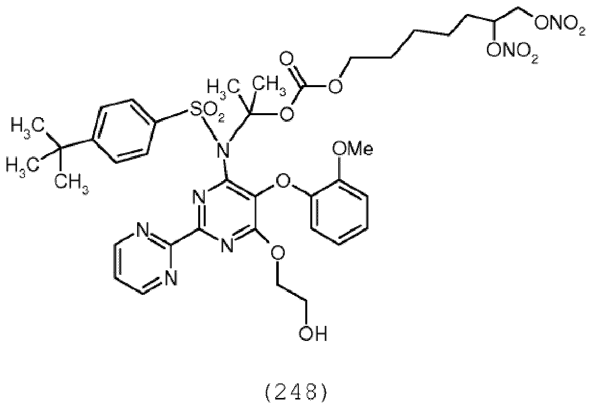

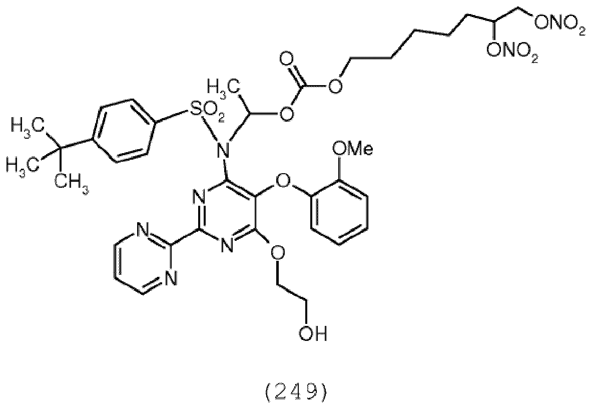

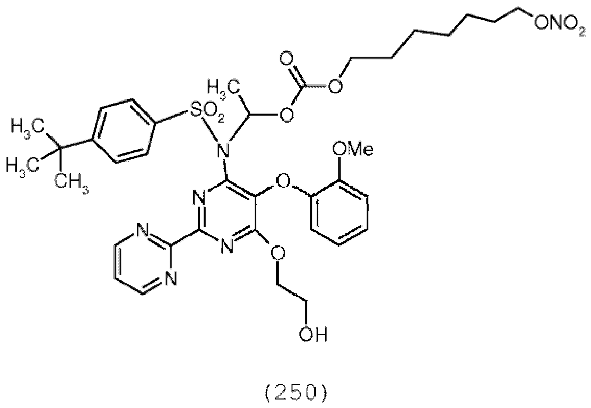

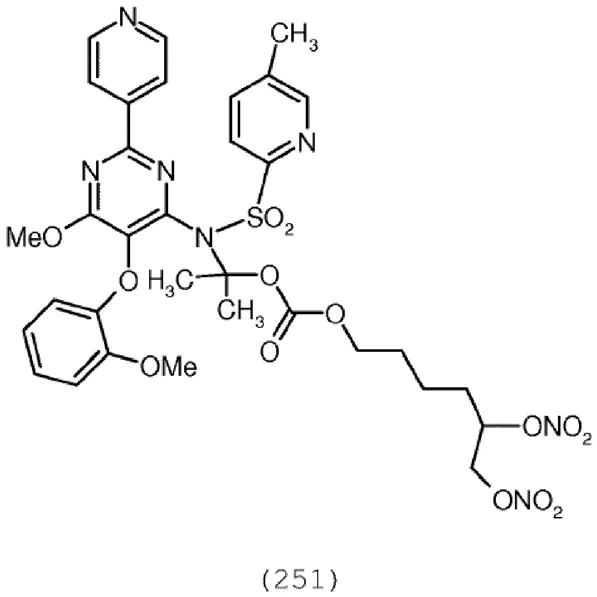

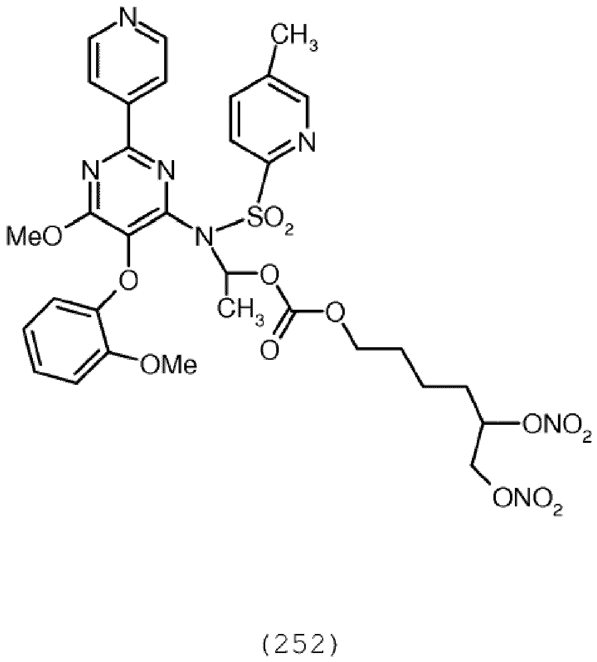

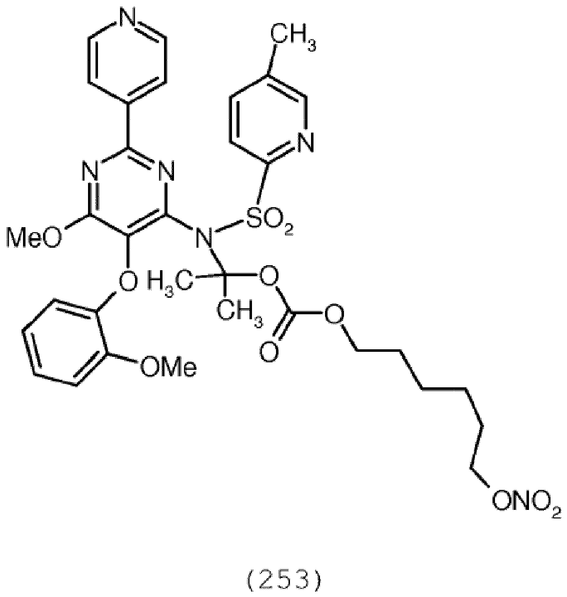

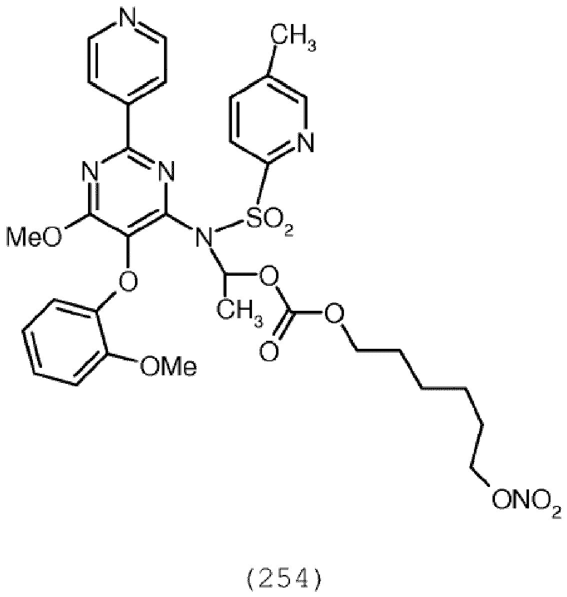

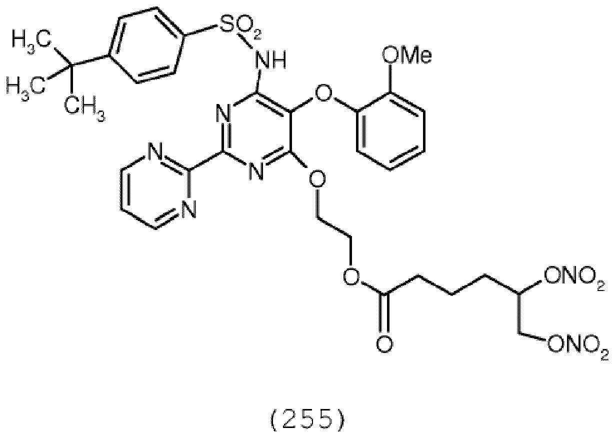

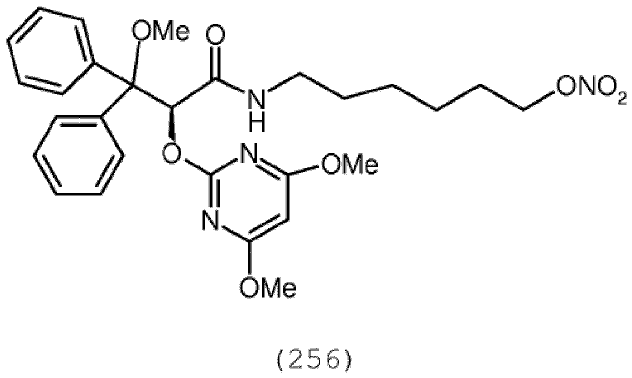

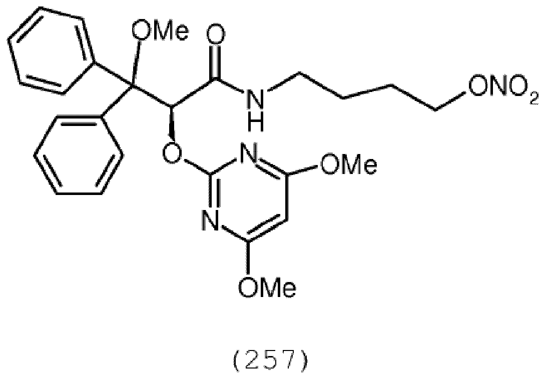

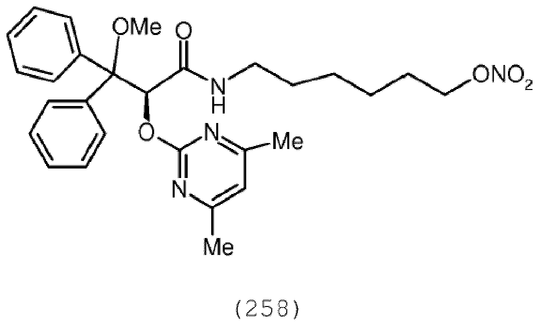

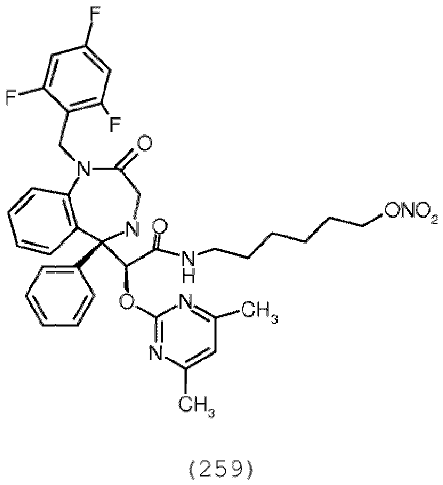

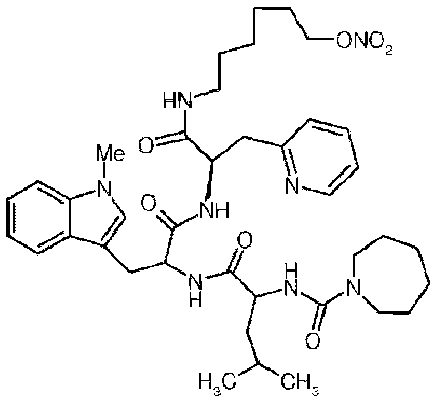

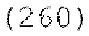

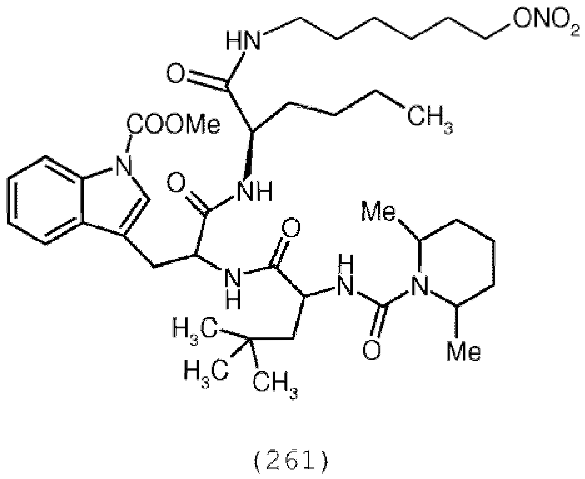

4. Verbindung mit der allgemeinen Formel (I) nach Ansprüchen 1-3 zur Verwendung als Medikament.

5. Verwendung einer Verbindung nach Ansprüchen 1-3 zur Herstellung eines Arzneimittels, das für die Behandlung oder Prophylaxe von durch Störungen des Endothelials bedingten Erkrankungen, Nieren-, Lungen-, Herz- und Gefäßkrankheiten und Entzündungsvorgängen verwendet werden kann.

6. Verwendung einer Verbindung nach Anspruch 5 zur Herstellung eines Arzneimittels, das zur Behandlung oder Prophylaxe von Herzinsuffizienz, Herzkrankheiten, linksventrikulärer Dysfunktion und Hypertrophie, kardialer Fibrose, Myokardischämie, Schlaganfall, Subarachnoidalblutung, zerebrovaskulärem Gefäßspasmus, koronarem Gefäßspasmus, Atheriosklerose, Restenose nach Angioplastie, Nierenischämie, Niereninsuffizienz, Nieren- und Lungenfibrose, Glomerulonephritis, Nierenkolik, okulärer Hypertonie, Glaukom, systemischer Hypertonie, pulmonal-arterieller Hypertonie (PAH), diabetischen Komplikationen wie beispielsweise Nephropathie, Vaskulopathie und Neuropathie, peripheren vaskulären Erkrankungen, Leberfibrose, portaler Hypertonie, metabolischem Syndrom, erektiler Dysfunktion, Komplikationen nach Gefäß- oder Herzchirurgie, Komplikationen der Behandlung mit Immunsuppressiva nach Organtransplantation, Hyperaldosteronismus, Lungenfibrose, Skleroderma, Sichelzellanämie, gutartiger Prostatahyperplasie, Krebs, Angstzustände, kognitiven Störungen.

7. Pharmazeutische Zusammensetzung, umfassend einen pharmazeutisch verträglichen Trägerstoff und eine pharmazeutisch wirksame Menge einer Verbindung mit der allgemeinen Formel (I) oder ein Salz oder ein Stereoisomer davon nach Ansprüchen 1- 3.

8. Pharmazeutische Zusammensetzung nach Anspruch 7 in einer geeigneten Form für die orale, parenterale, rektale, topische und transdermale Verabreichung, Verabreichung in Form eines Inhalationssprays oder Aerosols oder mittels lontophorese-Vorrichtungen.

9. Flüssige oder feste pharmazeutische Zusammensetzung für die orale, parenterale, rektale, topische und transdermale Verabreichung oder Inhalation in Form von Tabletten, Kapseln und Pillen, die möglicherweise magensaftresistent überzogen sind, Pulvern, Granulaten, Gelen, Emulsionen, Lösungen, Suspensionen, Sirups, Elixir, injizierbaren Applikationsformen, Suppositorien, in transdermalen Patches oder Liposomen, enthaltend eine Verbindung der Formel (I) oder ein Salz oder Stereoisomer davon nach Ansprüchen 1-3 und einen pharmazeutisch verträglichen Trägerstoff.

10. Pharmazeutische Zusammensetzung, umfassend eine Verbindung mit der allgemeinen Formel (I) nach Ansprüchen 1-3, mindestens eine Verbindung zur Verwendung für die Behandlung kardiovaskulärer Erkrankungen und einen pharmazeutisch verträglichen Trägerstoff.

11. Pharmazeutische Zusammensetzung nach Anspruch 10, wobei die Verbindung zur Verwendung für die Behandlung von kardiovaskulären Erkrankungen aus der Gruppe ausgewählt ist, bestehend aus: Aldosteronantagonisten, Angiotensin-II-Rezeptorblockern, ACE-Hemmern, HMGCoA-Reduktasehemmern, beta-adrenergen Blockern, alpha-adrenergen Antagonisten, Symphatolytika, Kalziumkanalblockern, Reninhemmern, neutralen Endopeptidasehemmern, Kaliumaktivatoren, Diuretika, Vasodilatatoren, Antithrombotika, wie beispielsweise Aspirin oder nitrosierte Verbindungen davon.

12. Pharmazeutisches Kit, umfassend eine Verbindung mit der allgemeinen Formel (I), wie in Anspruch 1 definiert, eine Verbindung zur Verwendung für die Behandlung kardiovaskulärer Erkrankungen in Form eines kombinierten Präparates für gleichzeitige, gesonderte oder nachfolgende Verwendung zur Behandlung einer kardioavskulären Erkrankung.

13. Pharmazeutisches Kit nach Anspruch 12, wobei die Verbindung für die Behandlung kardiovaskulärer Erkrankungen aus der Gruppe ausgewählt ist, bestehend aus: Aldosteronantagonisten, Angiotensin-II-Rezeptorblockern, ACE-Hemmern, HMGCoA-Reduktasehemmern, beta-adrenergen Blockern, alpha-adrenergen Antagonisten, Sympatholytika, Kalziumkanalblockern, Reninhemmern, neutralen Endopeptidasehemmern, Kaliumaktivatoren, Diuretika, Vasodilatatoren, Antithrombotika, wie beispielweise Aspirin oder nitrosierte Verbindungen davon.

## Revendications

1. Composé de formule générale (I) ou un sel ou un stéréoisomère de celui-ci acceptable au plan pharmaceutique de formule : 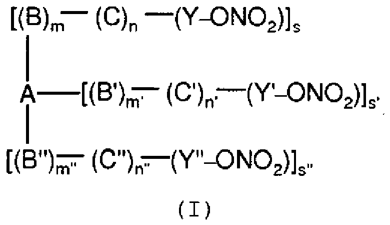 dans laquelle :
m, m' et m" sont égaux à 0 ou 1 ;
n, n' et n" sont égaux à 0 ou 1 ;
s, s' et s" sont égaux à 0 ou 1 ;
A est sélectionné dans le groupe constitué des groupements suivantes : 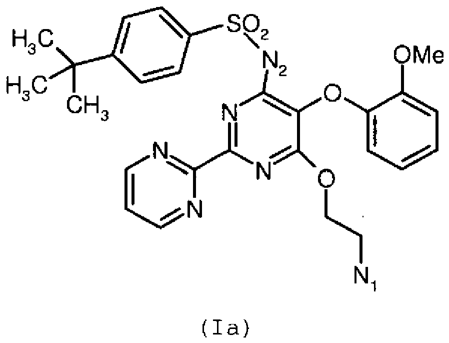 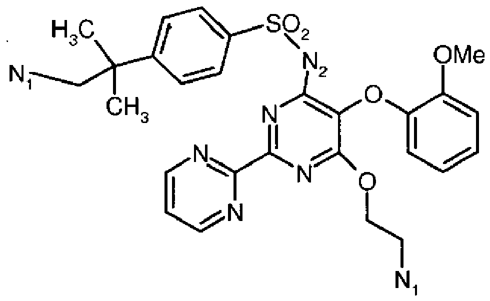 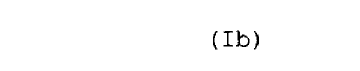 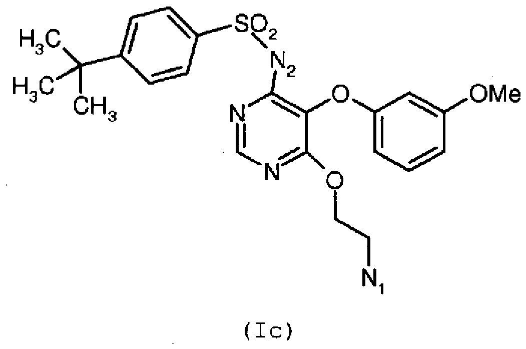 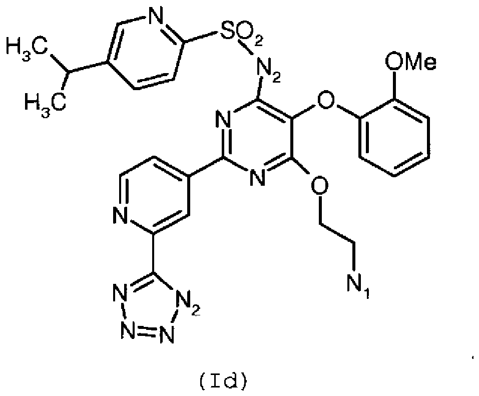 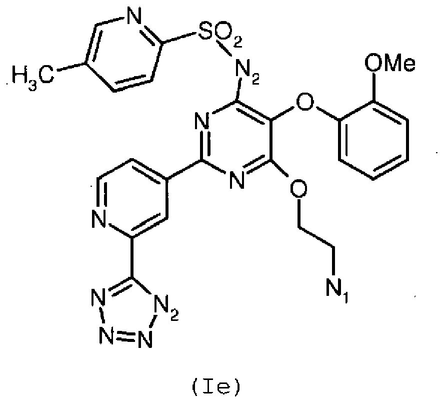 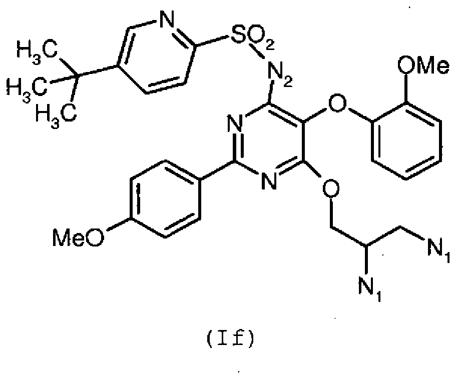 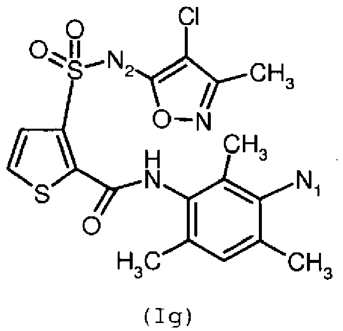 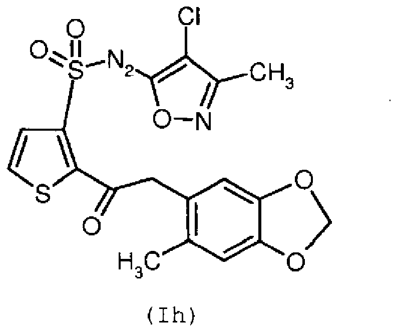 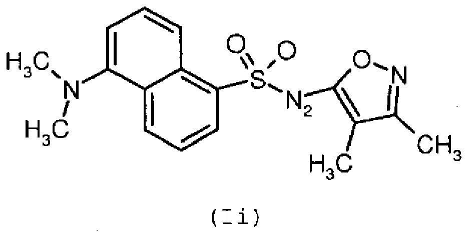 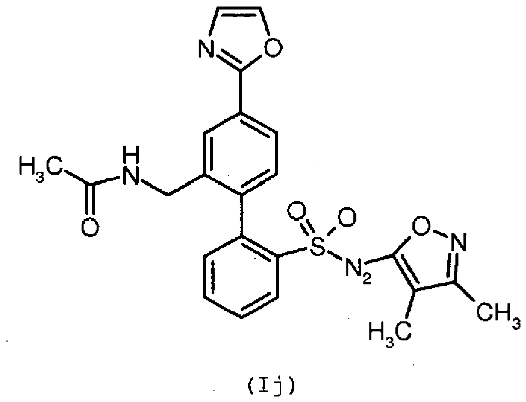 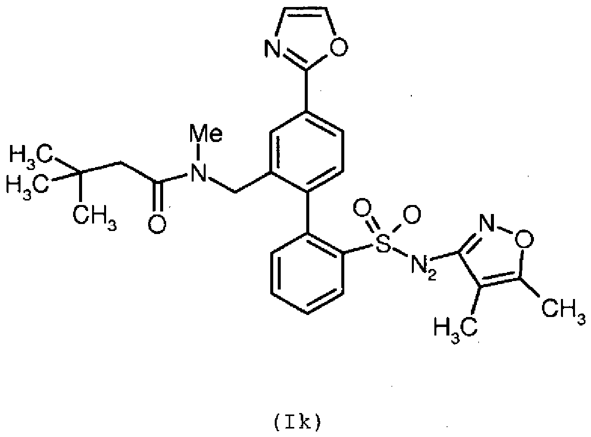 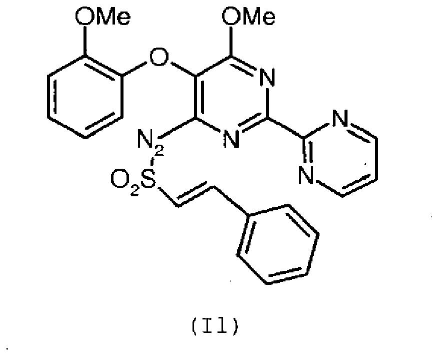 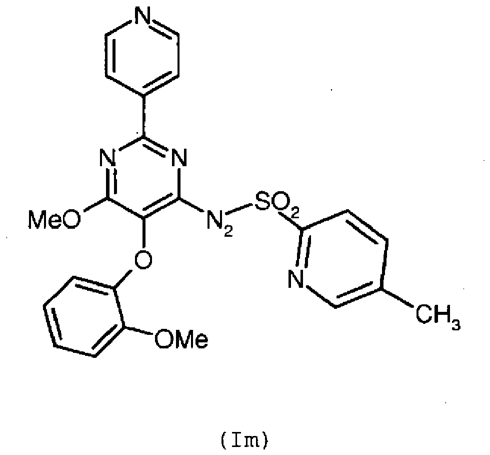 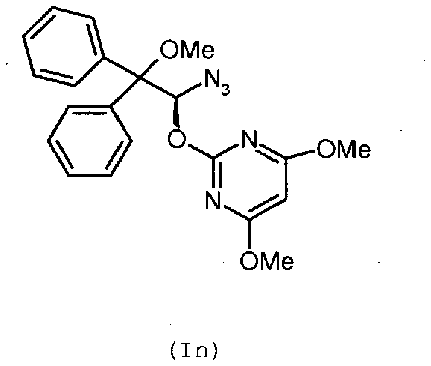 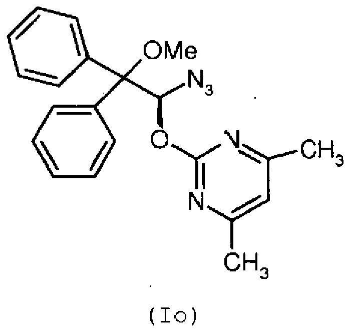 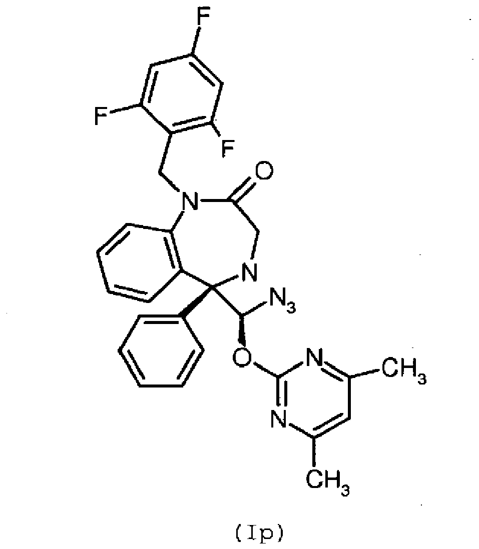 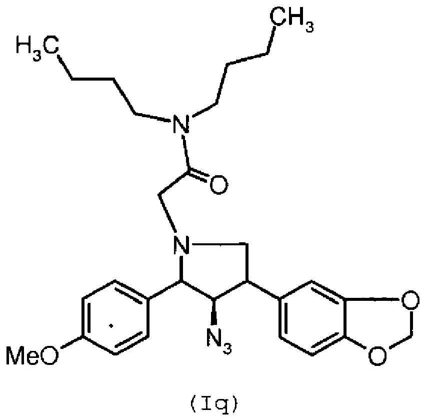 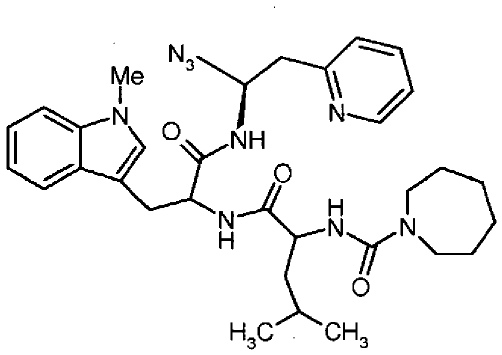 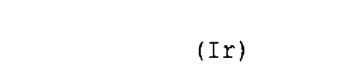 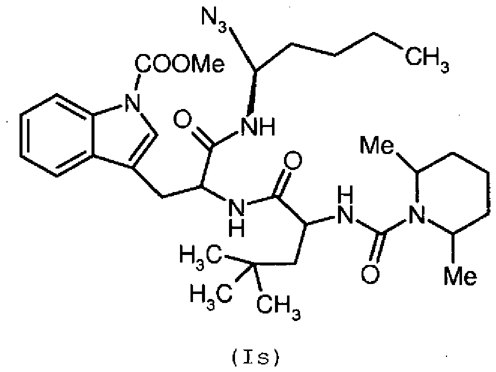 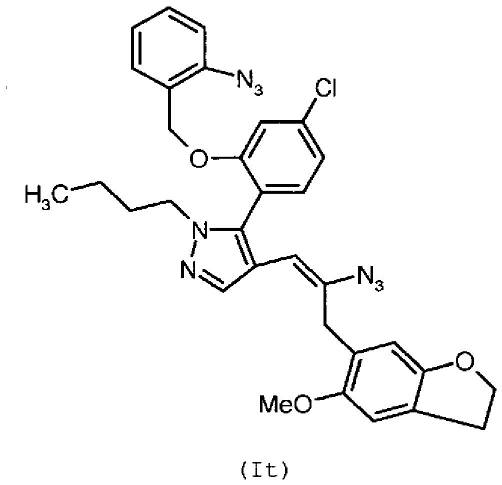 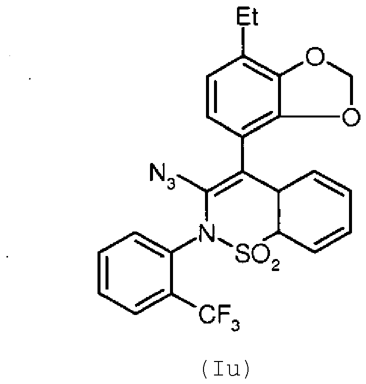 où :
N₁ représente -O-, -OH ;
N₂ représente -N-, -NH- ;
N₃ représente -C(O)O-, -C(O)NH- ;
B, B' et B" représentent -CO-, -C(O)O-, -C(O)NH ;
C, C et C' sont : 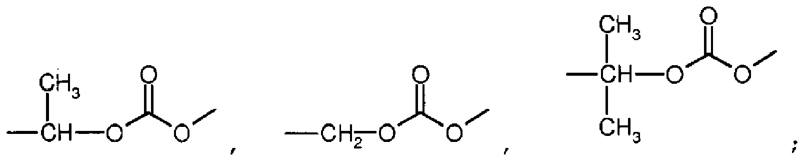 à condition que :
1) lorsque A est sélectionné dans le groupe constitué de (Ia), (Ib), (Ic), (Id), (Ie), (If), (Ig), (Ih), (Ii), (Ij), (Ik), (Il) et (Im), au moins l'un de m, m', m", n, n' ou n" vaut 1 ;
2) lorsque A est sélectionné dans le groupe constitué de (In), (Io), (Ip), (Iq), (Ir), (Is) et (Iu), s', s" et m valent 0 ; tandis que n vaut 0 ou 1 ;
3) lorsque A représente (It), m, m' et s" valent 0 ; tandis que n et n' valent 0 ou 1 ;
4) au moins l'un de N₁ ou N₂ représente un groupement -0- ou -N- capable de se lier à au moins l'un des groupements -[(B)ₘ-(C)ₙ-(Y-ONO₂)], -[(B')_{m'}-(C')_{n'}-(Y'-ONO₂)] ou -[(B")_{m"}-(C")_{n"}-(Y"-ONO₂)];
Y, Y' et Y" représentent un radical bivalent ayant la signification suivante :
a)
- un groupement alkylène en C₁-C₂₀ linéaire ou ramifié, éventuellement substitué par un ou plusieurs des substituants sélectionnés dans le groupe constitué d'atomes d'halogène, d'un hydroxy, de -ONO₂ ou de Tₐ, où Tₐ représente -OC(O)(alkyle en C₁-C₁₀)-ONO₂ ou -O(alkyle en C₁-C₁₀)-ONO₂ ;
- un groupement cycloalkylène ayant 5 à 7 atomes de carbone dans un cycle cycloalkylène, le cycle étant éventuellement substitué par des chaînes latérales T, où T représente un groupement alkyle linéaire ou ramifié de 1 à 10 atomes de carbone ;
b) 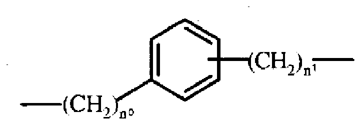
c) 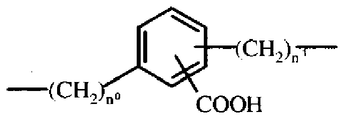 où n° représente un entier de 0 à 20 et n¹ représente un entier de 1 à 20 ;
d) 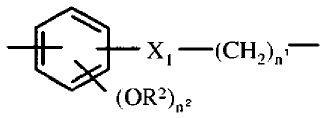 où :
n¹ est tel que défini ci-dessus et n² représente un entier de 0 à 2 ;
X₁ = -OCO- ou -COO- et R² représente H ou CH₃;
e) 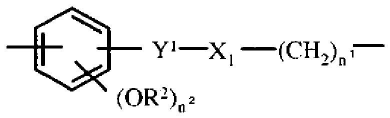 où :
n¹, n², R² et X₁ sont tels que définis ci-dessus ;
Y¹ est -CH₂-CH₂- ou -CH=CH-(CH₂)ₙ²- ;
f) 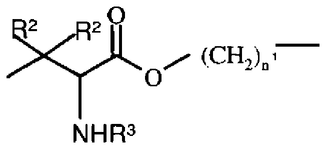 où :
n¹ et R² sont tels que définis ci-dessus, R³ représente H ou -COCH₃ ;
à condition que, lorsque Y est sélectionné parmi les radicaux bivalents mentionnés sous b) à f), le groupement -ONO₂ est lié à un groupement -CH₂ ;
g) 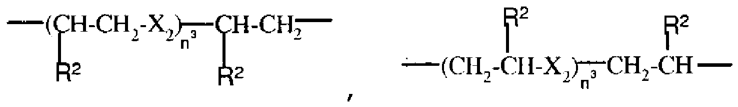 où X₂ représente -0- ou -S-, n³ représente un entier de 1 à 6, R² est tel que défini ci-dessus ;
h) 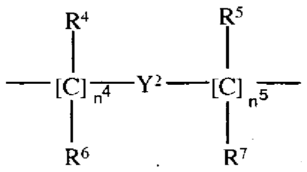 où :
n⁴ représente un entier de 0 à 10 ;
n⁵ représente un entier de 1 à 10 ;
R⁴, R⁵, R⁶, R⁷ sont identiques ou différents et représentent H ou un groupement alkyle en C₁-C₄ linéaire ou ramifié ;
où le groupement -ONO₂ est lié à 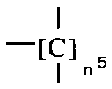
où n⁵ est tel que défini ci-dessus ;
Y² représente un cycle hétérocyclique saturé, insaturé ou aromatique à 5 ou 6 éléments, contenant un ou plusieurs hétéroatomes sélectionnés parmi l'azote, l'oxygène, le soufre,
et est sélectionné dans le groupe constitué des groupements suivantes :

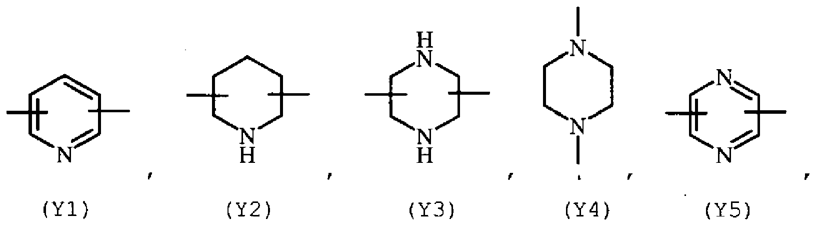

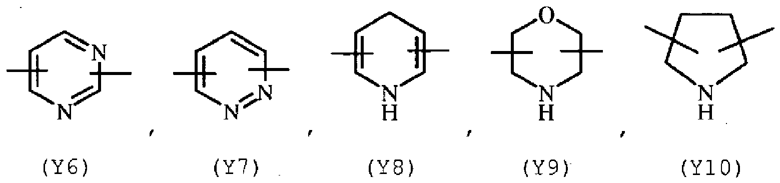

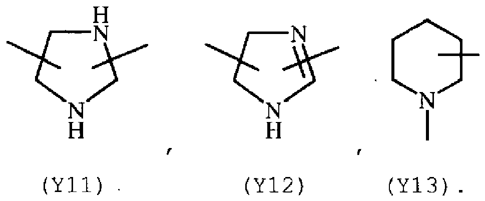

2. Composé de formule générale (I) ou un sel ou un stéréoisomère de celui-ci acceptable au plan pharmaceutique selon la revendication 1, dans lequel Y, Y' et Y" représentent un radical bivalent ayant la signification suivante :
a)
- un groupement alkylène en C₁-C₁₀ linéaire ou ramifié, éventuellement substitué par un groupement -ONO₂ ;
b) 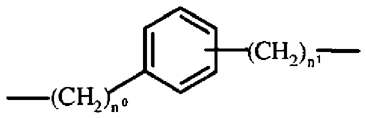 où n° représente un entier égal à 0 ou 1, et n¹ représente un entier égal à 1 ; à condition que le groupement -ONO₂ soit lié à un groupement -CH₂ ;
g) 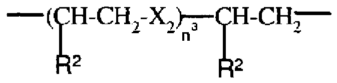 où X₂ représente -0- ou -S-, n³ représente un entier égal à 1 et R² représente H.

3. Composé selon les revendications 1 à 2, sélectionné dans le groupe constitué des composés suivantes :

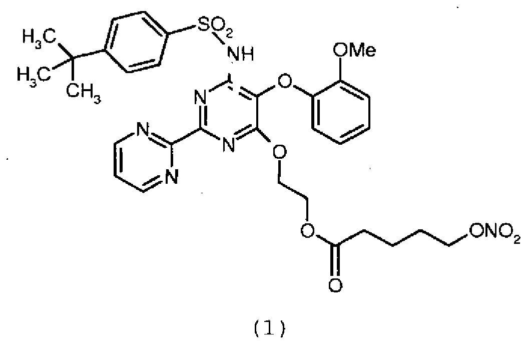

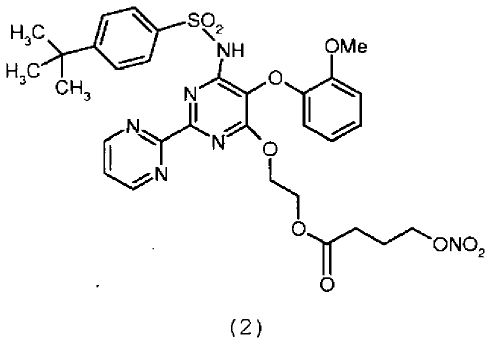

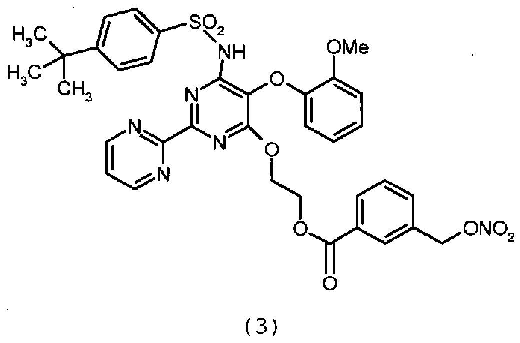

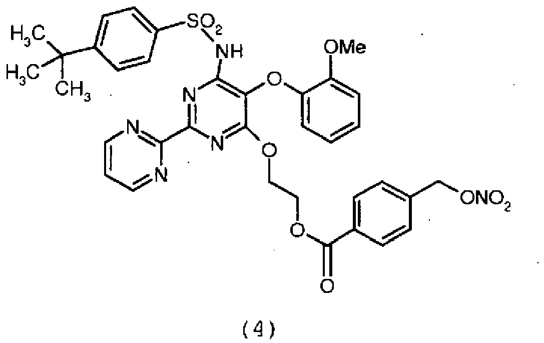

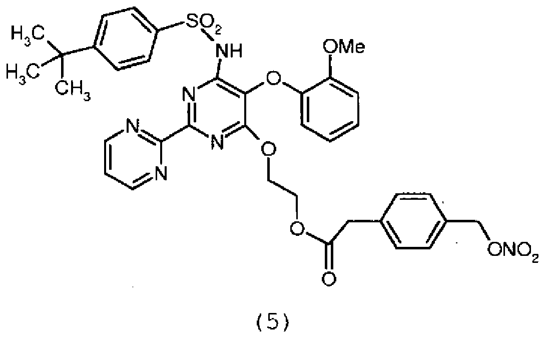

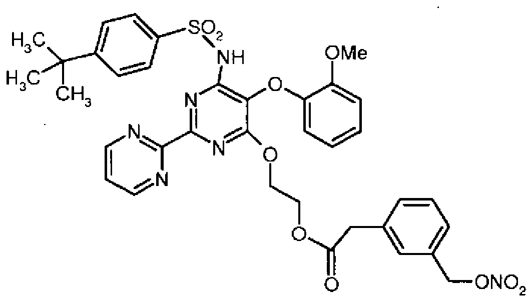

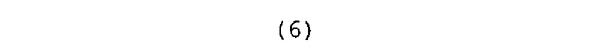

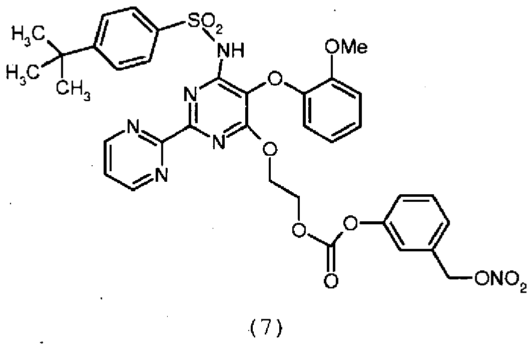

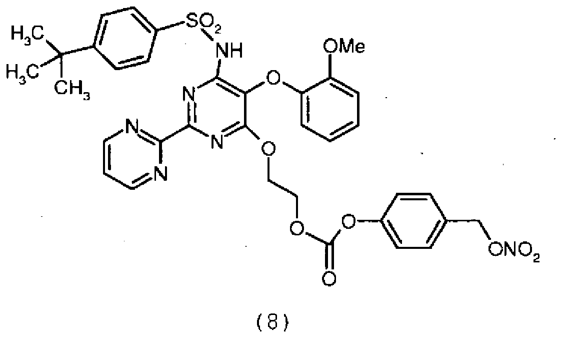

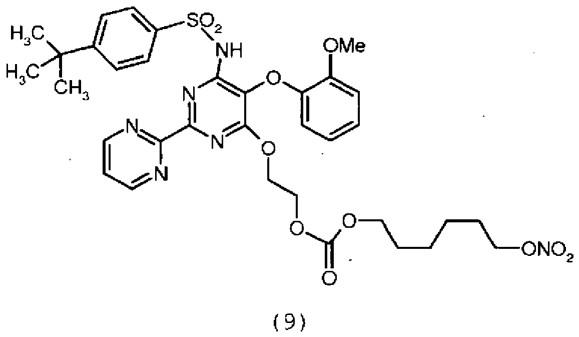

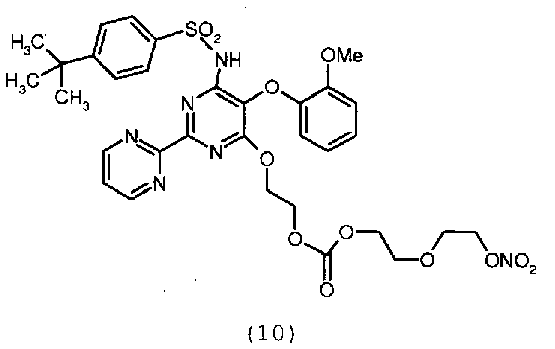

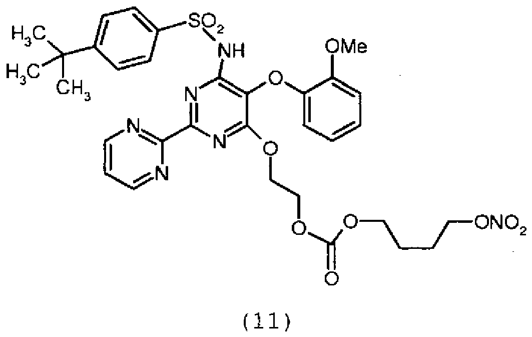

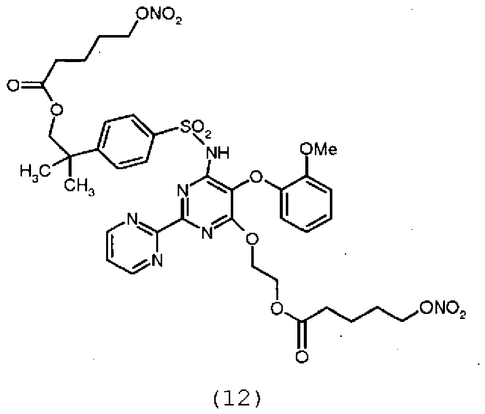

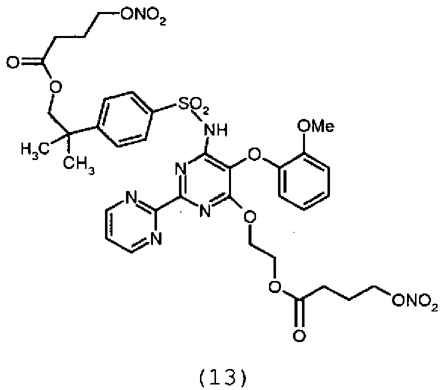

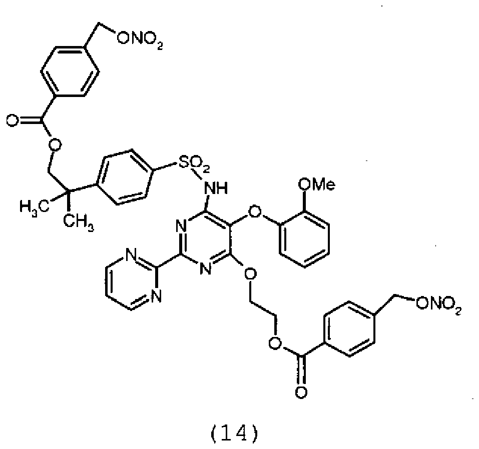

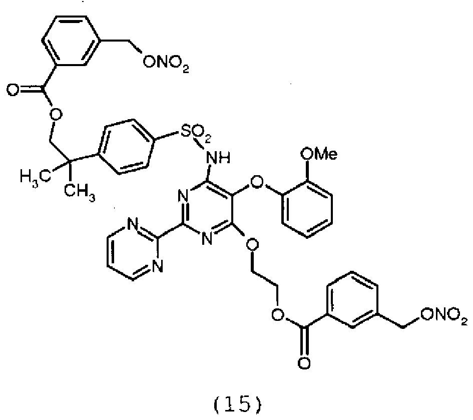

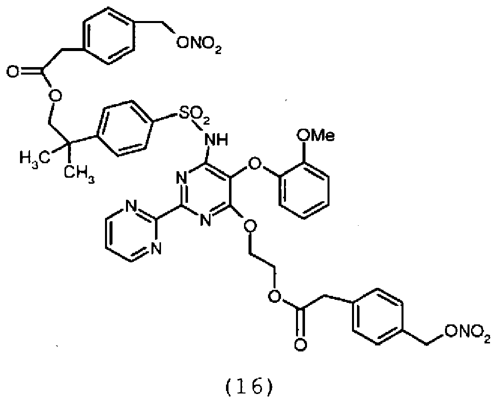

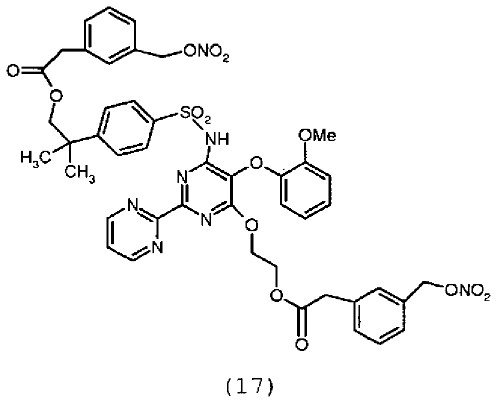

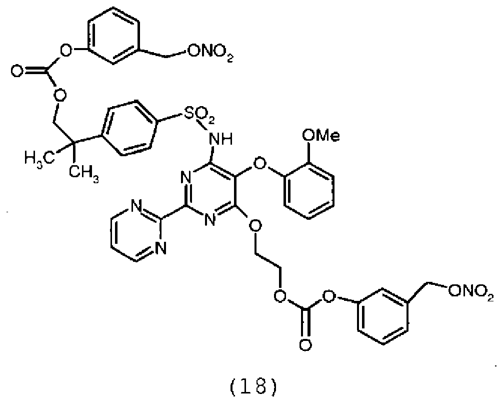

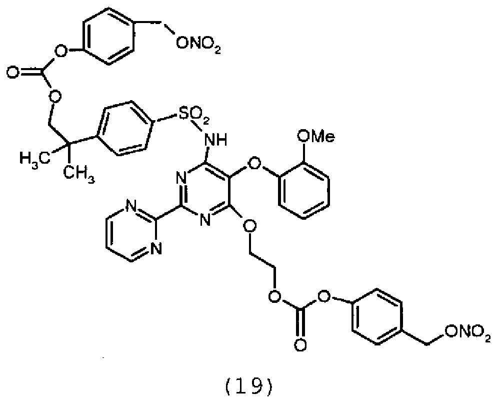

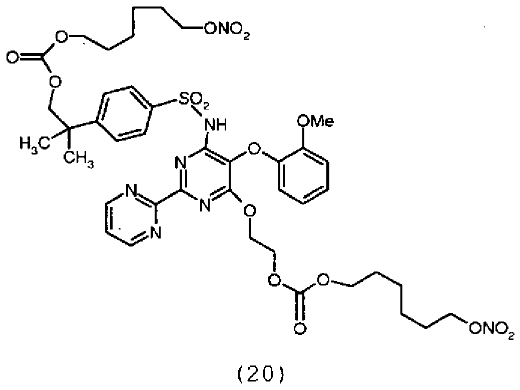

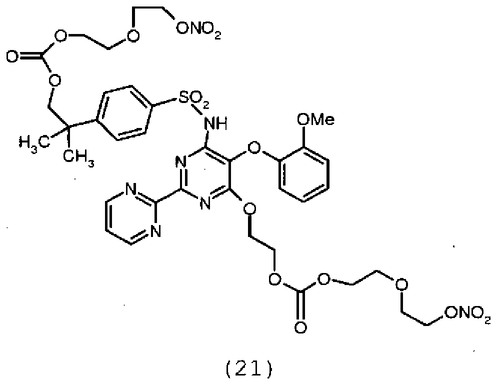

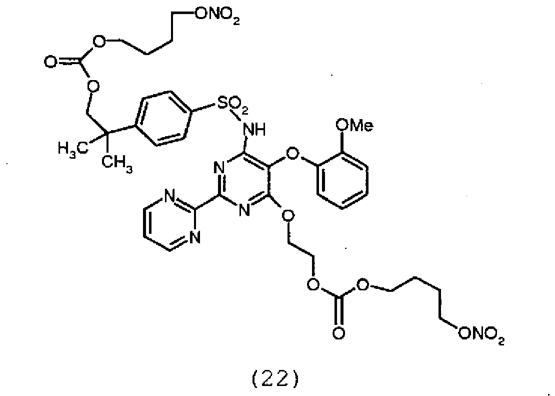

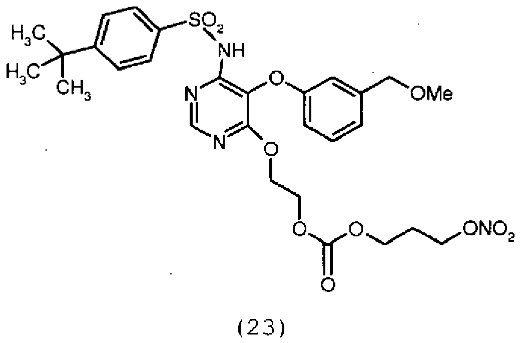

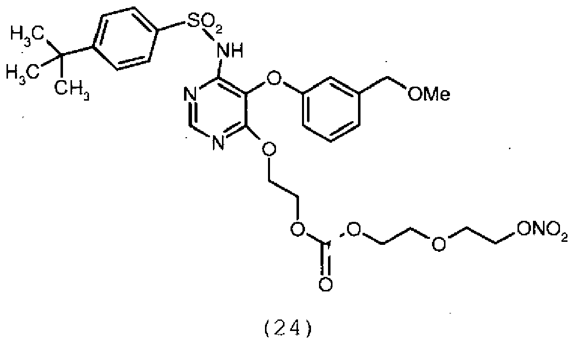

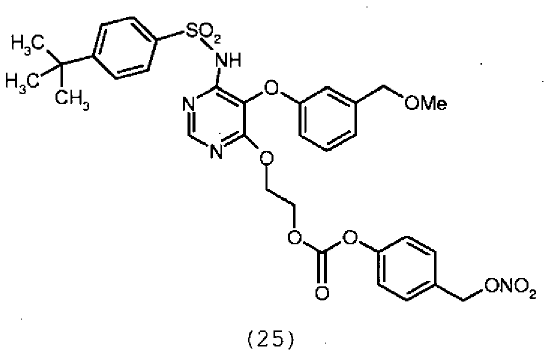

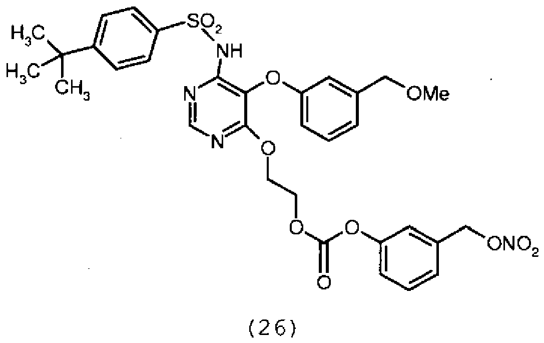

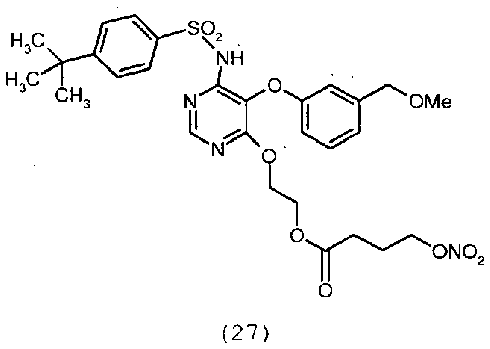

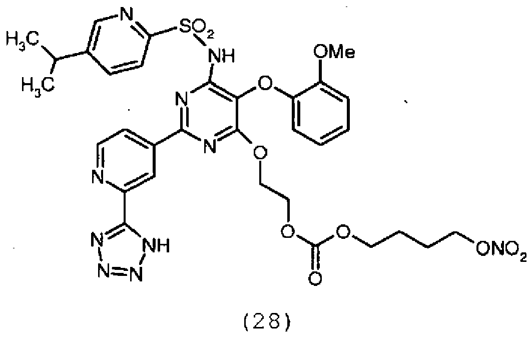

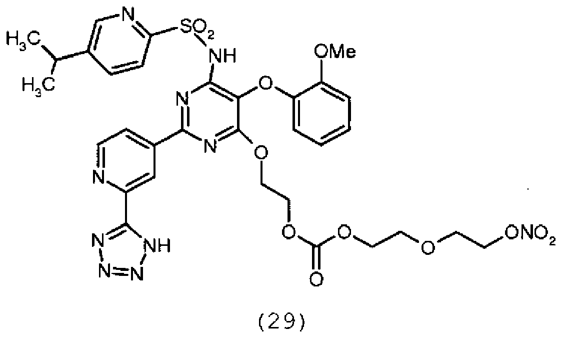

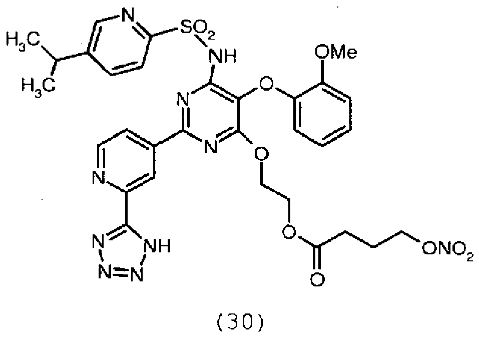

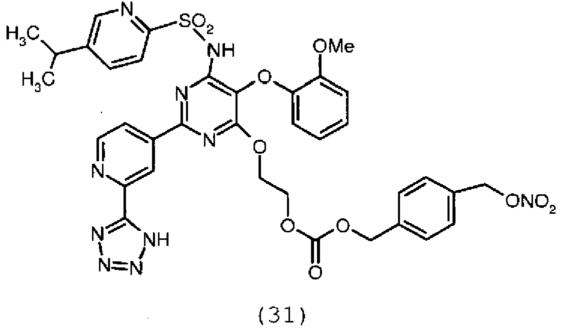

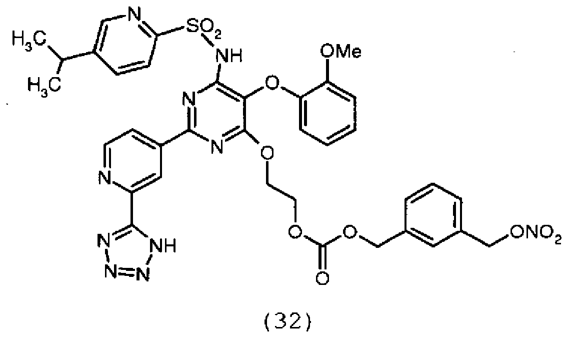

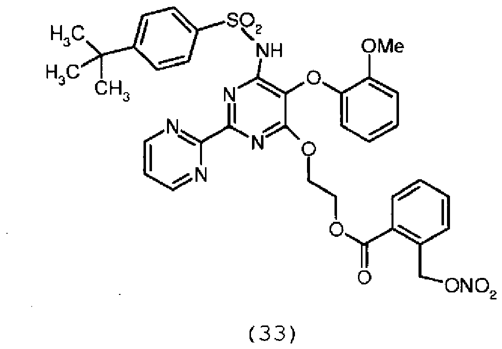

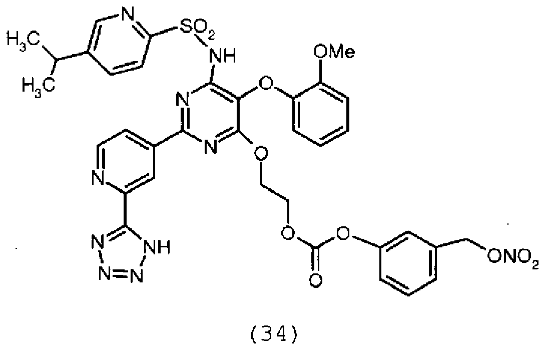

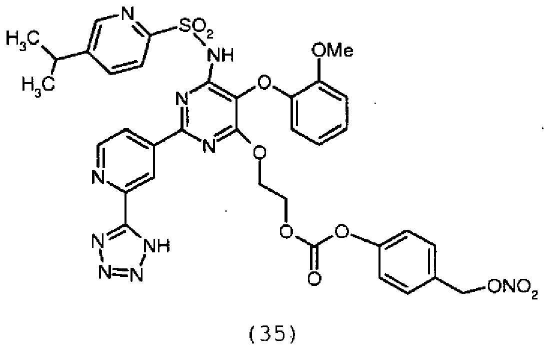

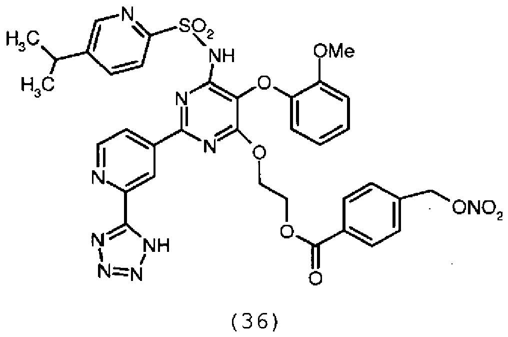

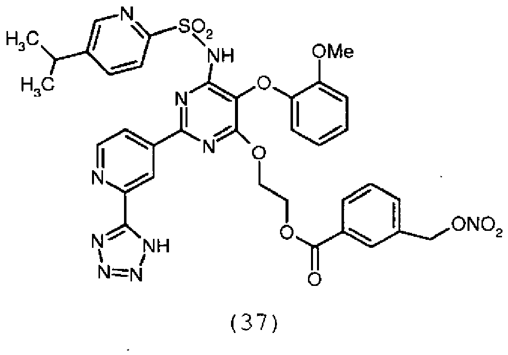

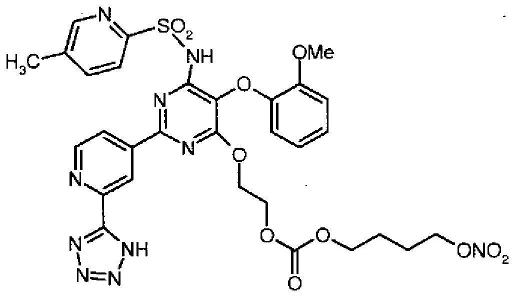

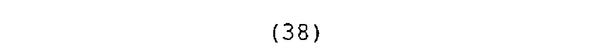

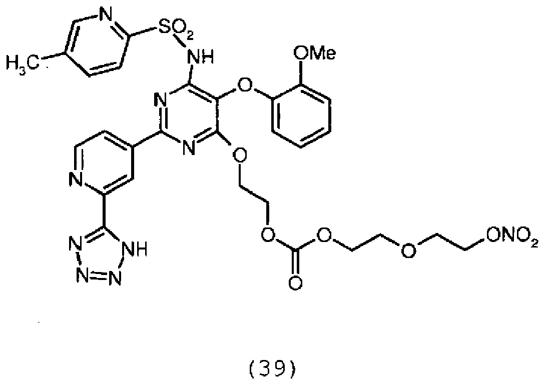

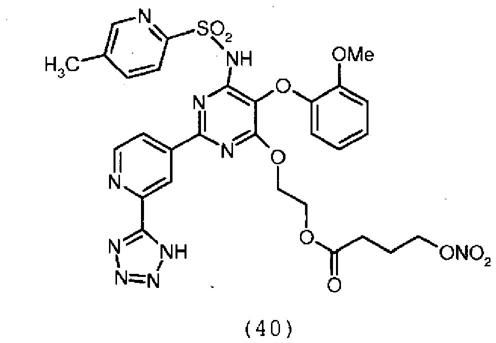

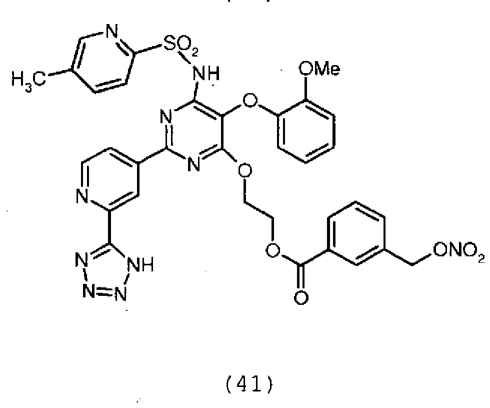

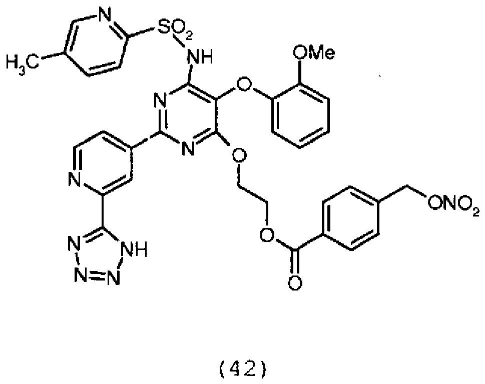

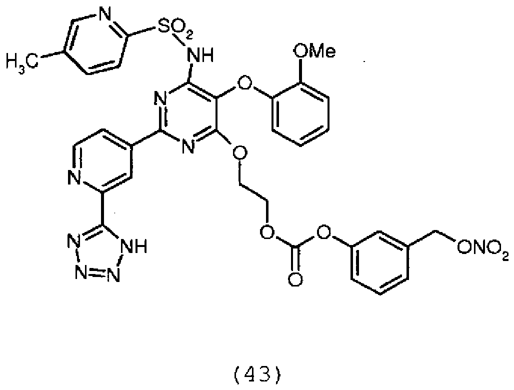

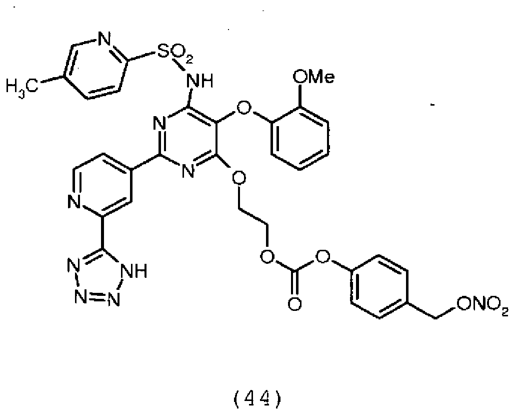

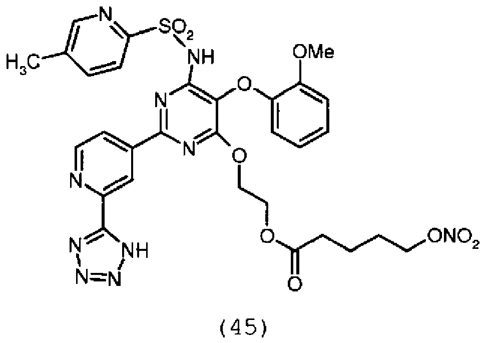

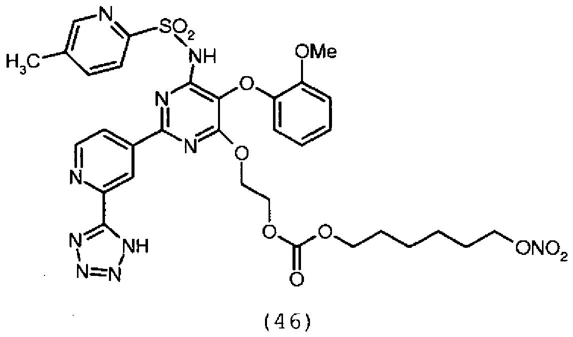

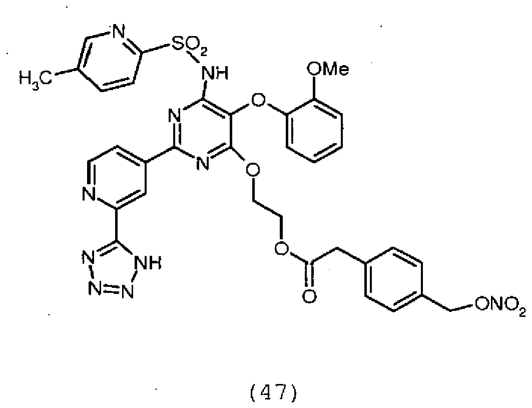

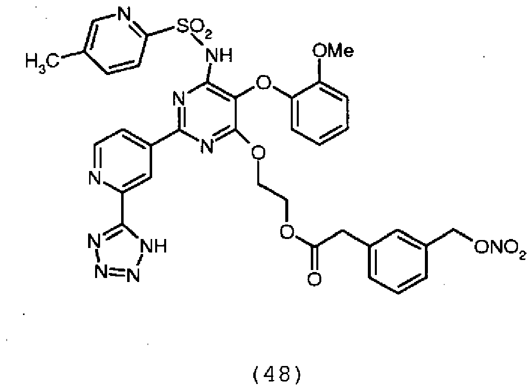

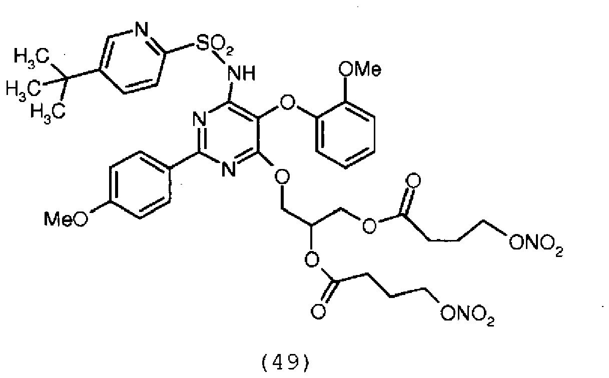

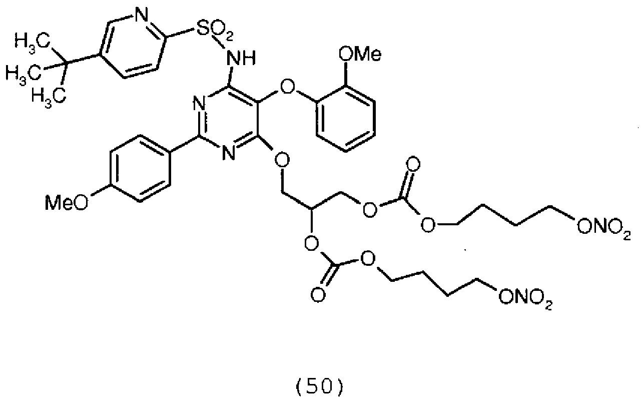

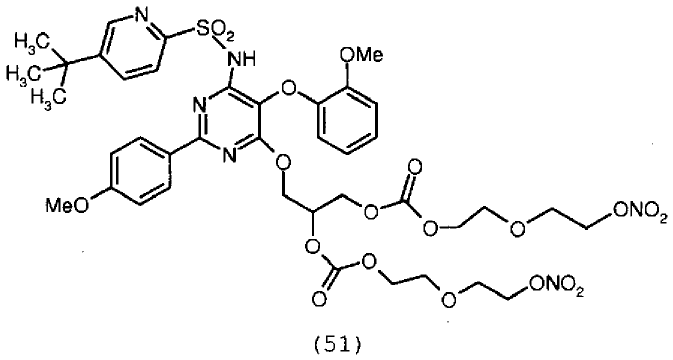

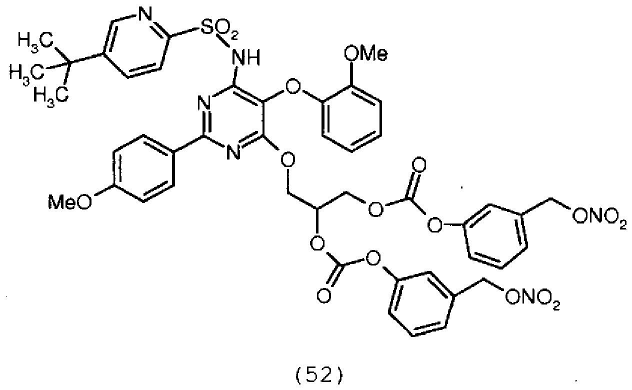

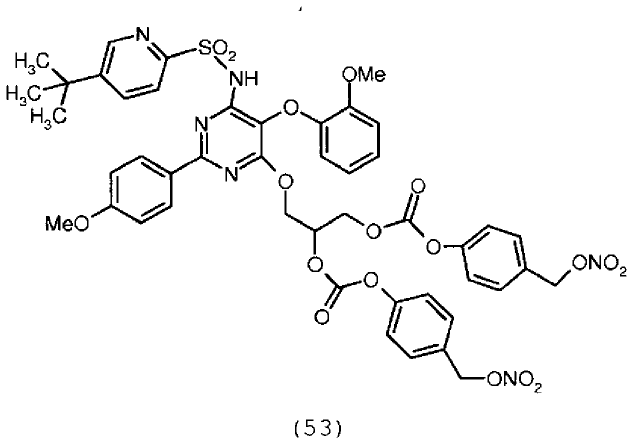

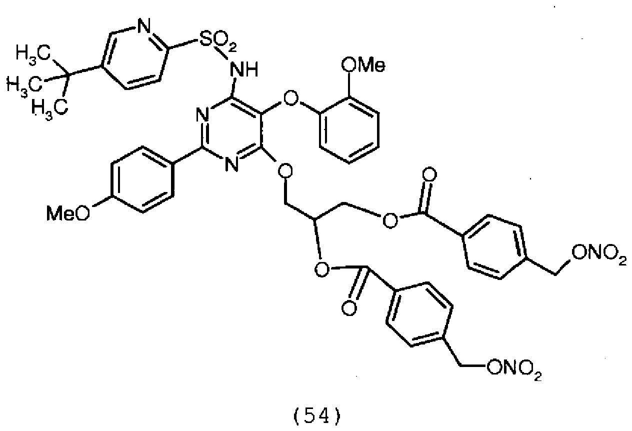

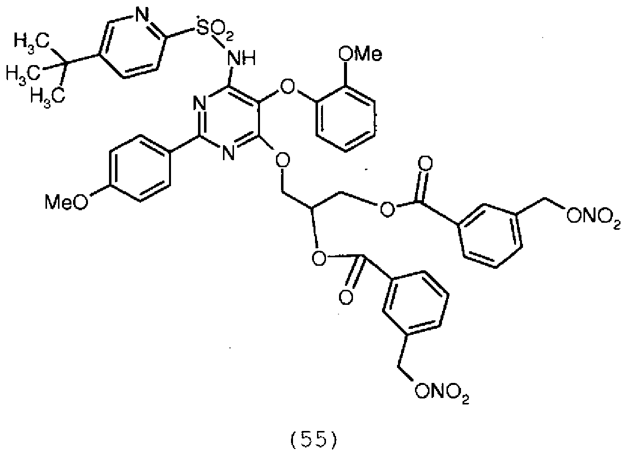

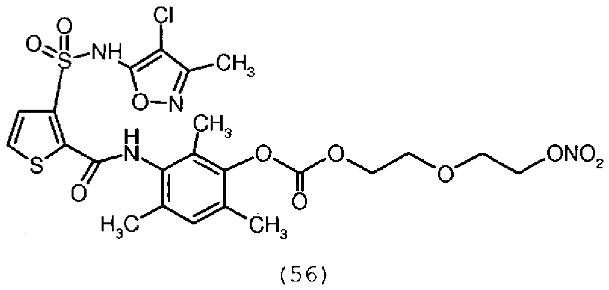

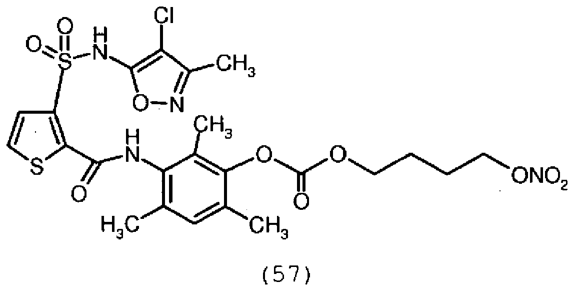

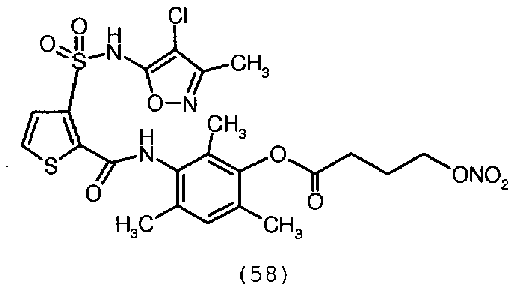

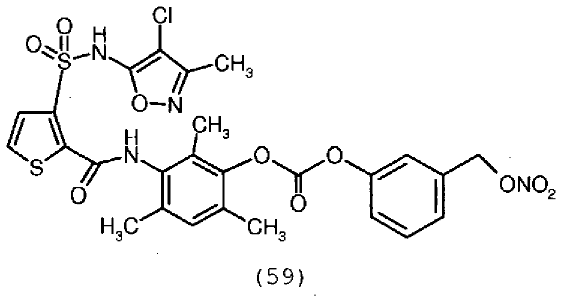

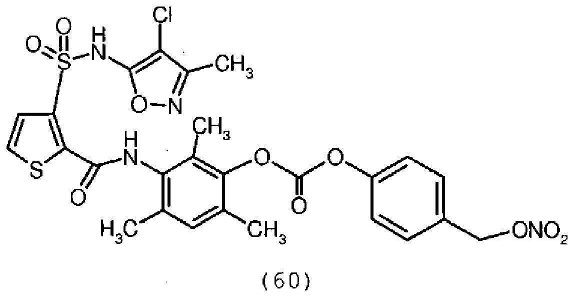

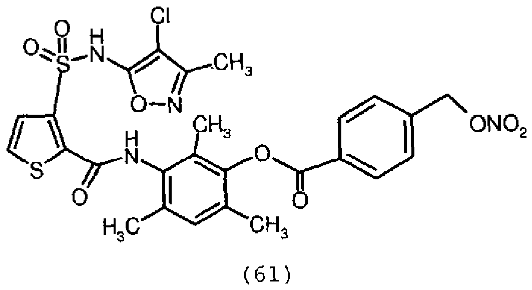

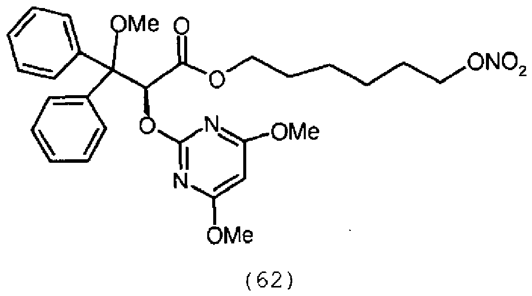

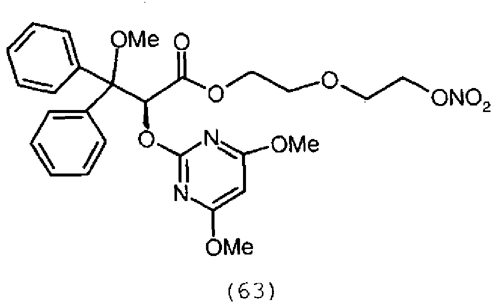

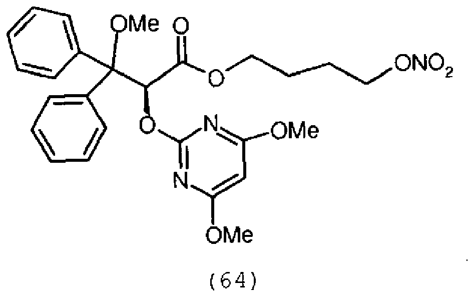

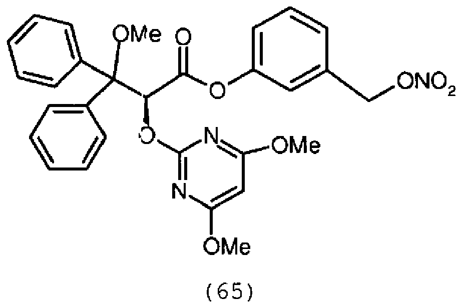

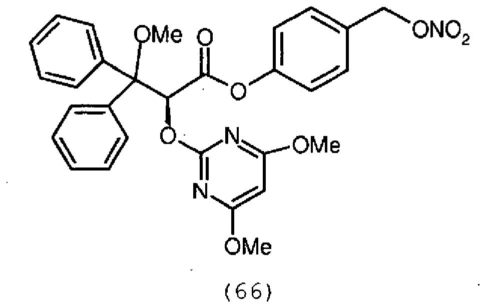

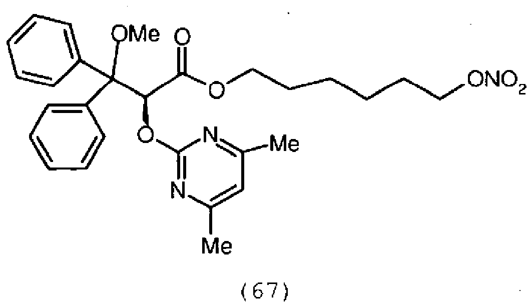

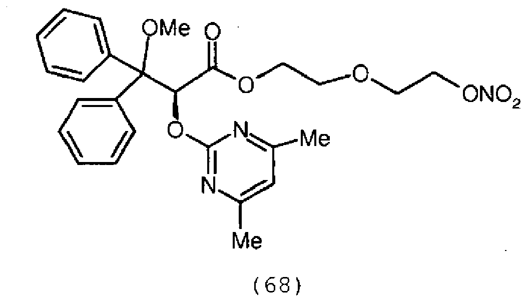

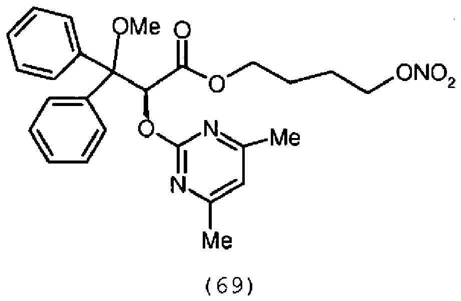

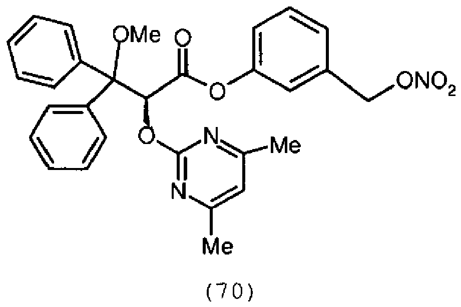

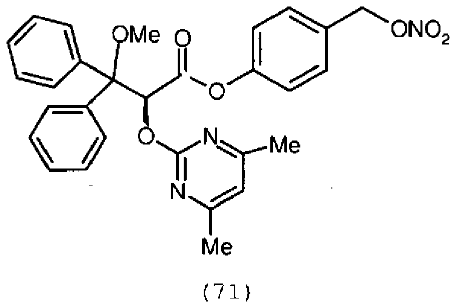

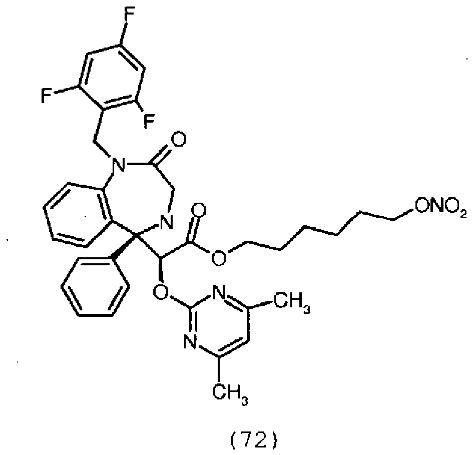

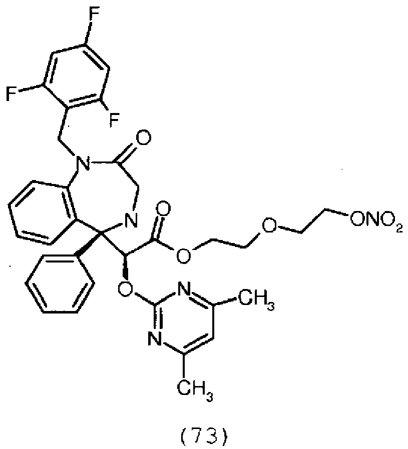

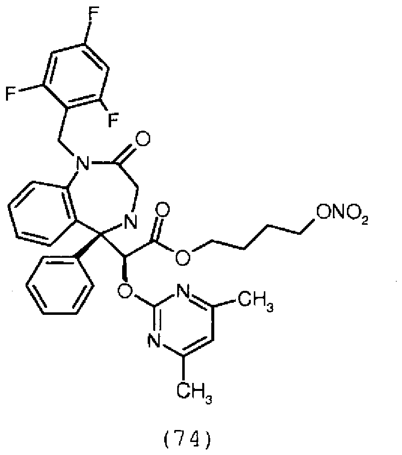

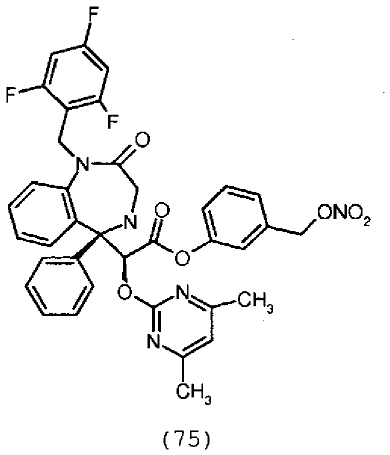

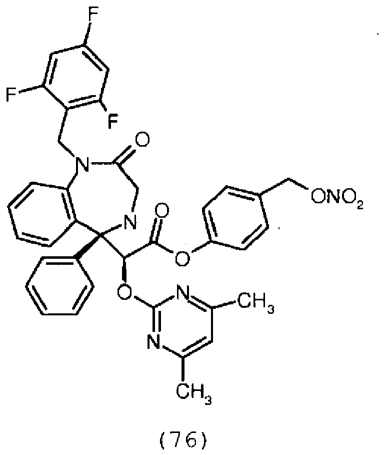

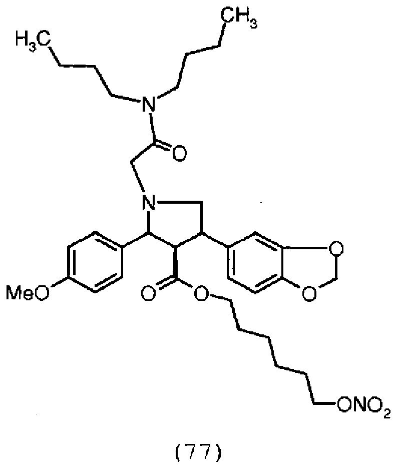

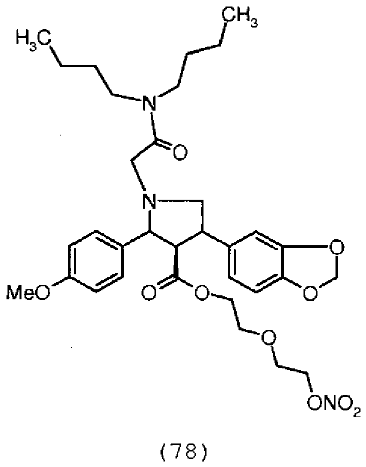

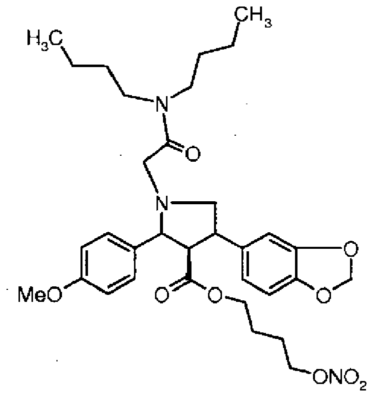

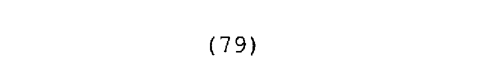

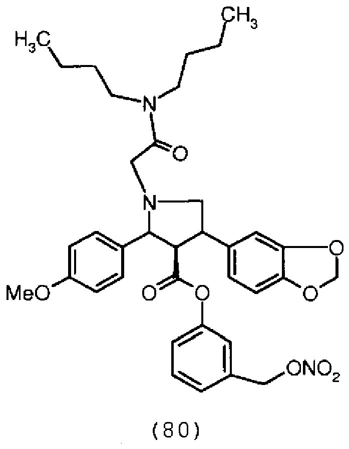

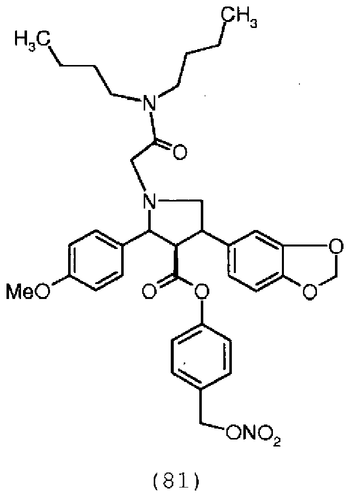

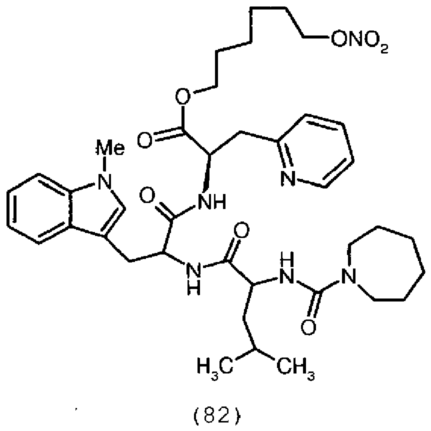

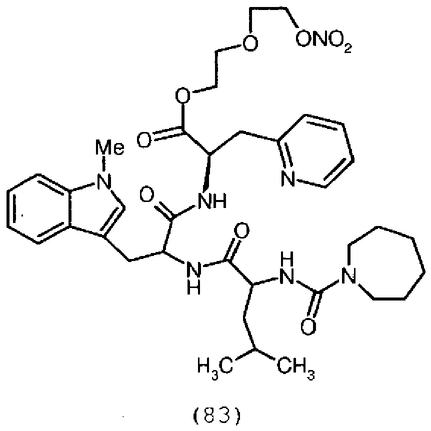

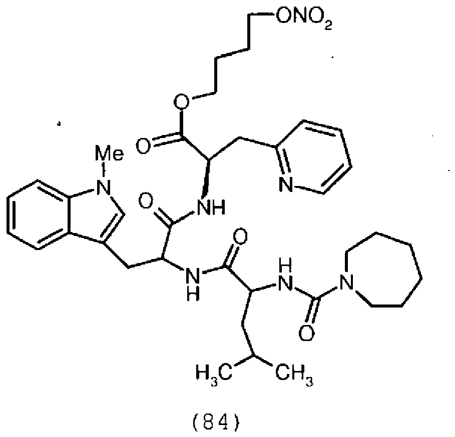

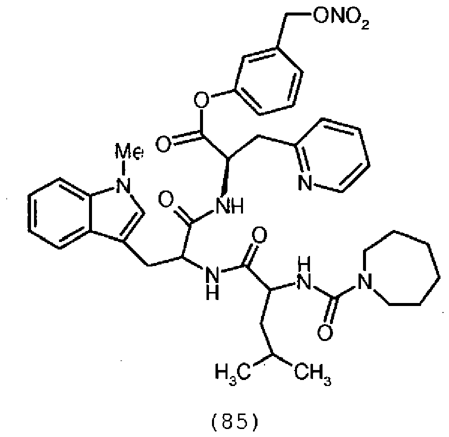

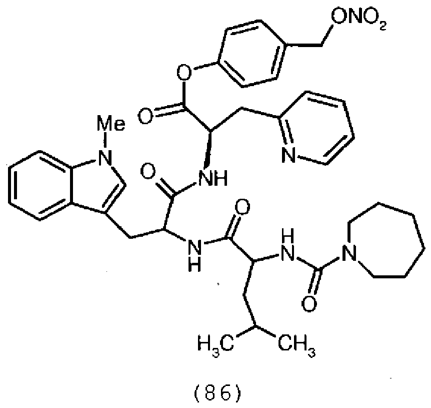

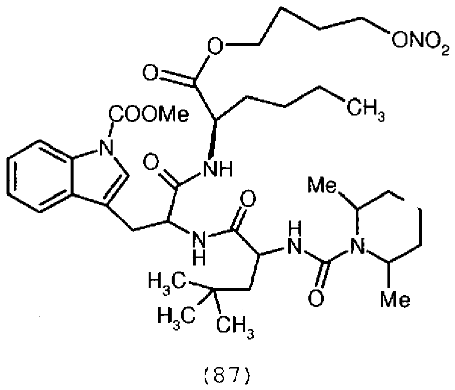

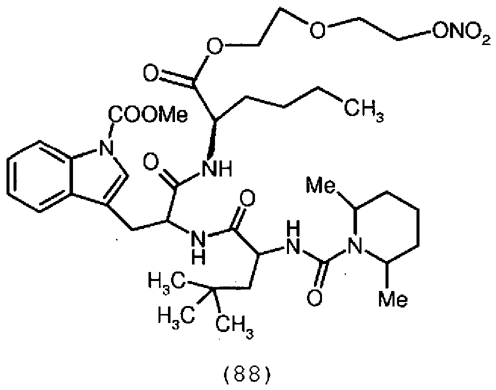

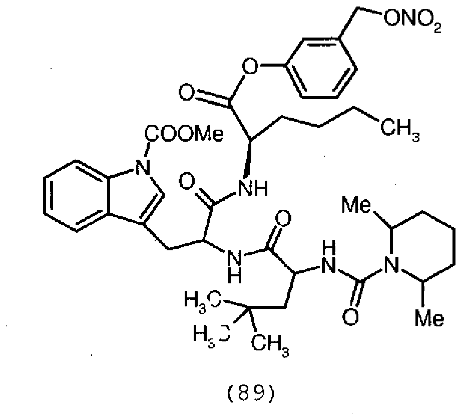

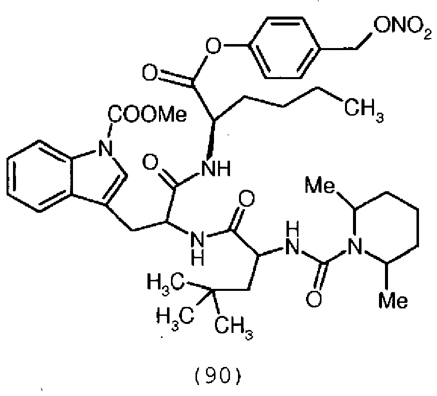

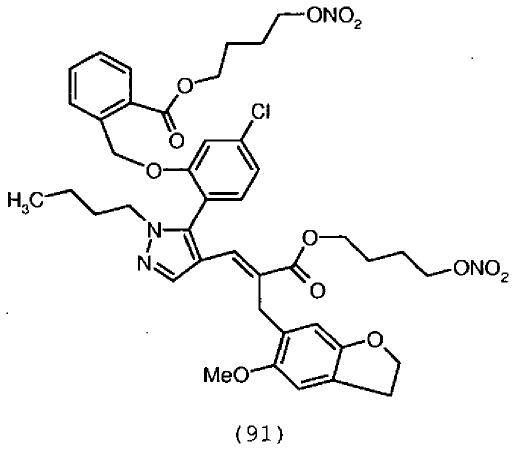

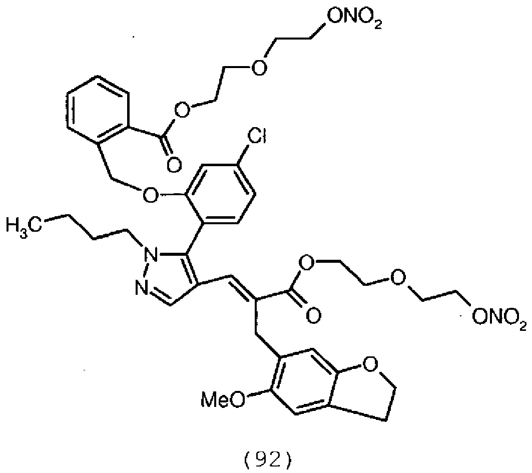

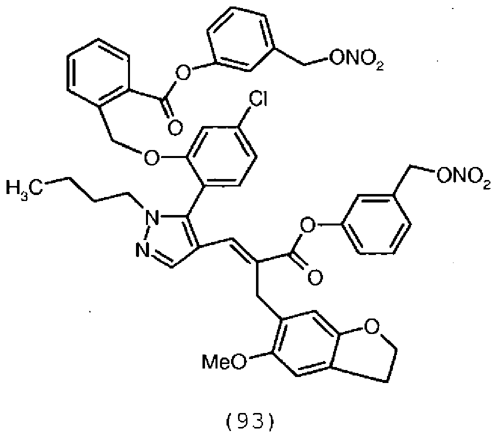

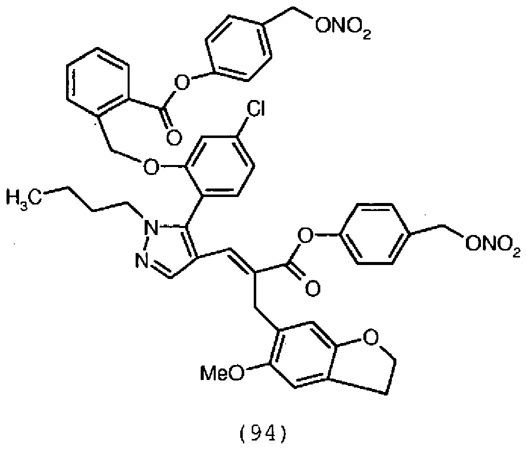

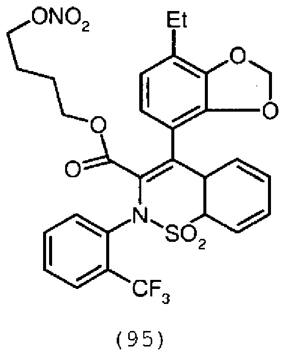

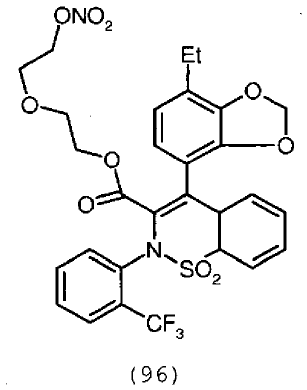

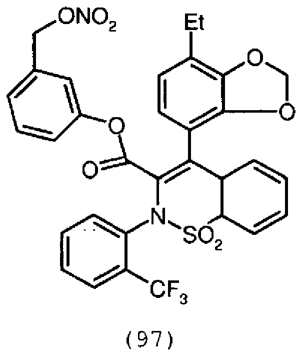

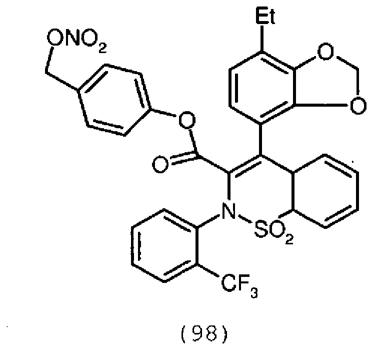

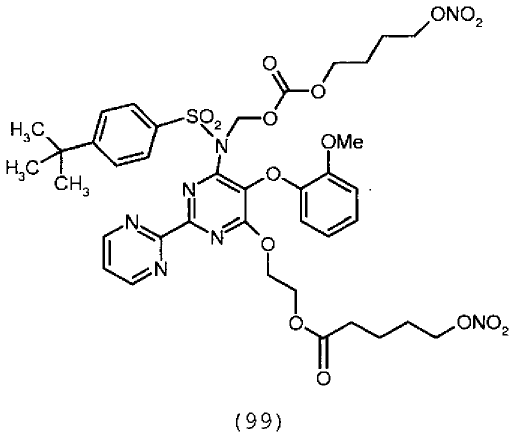

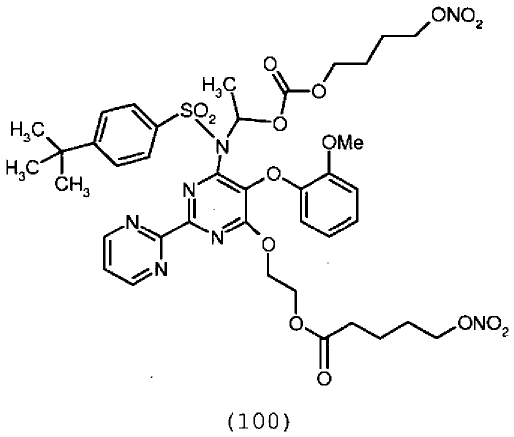

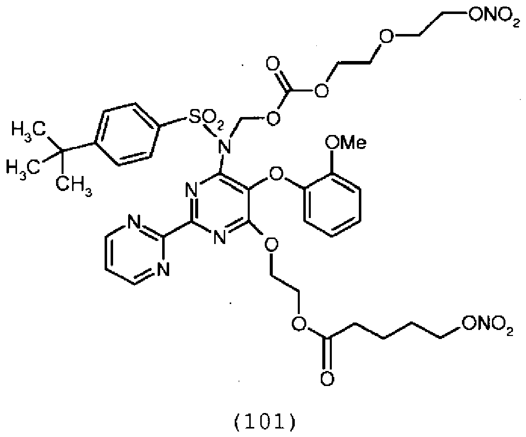

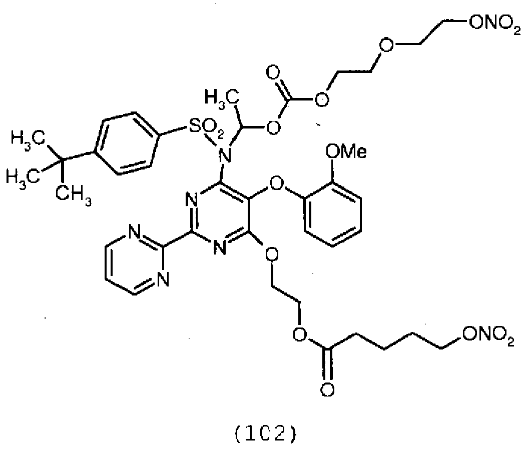

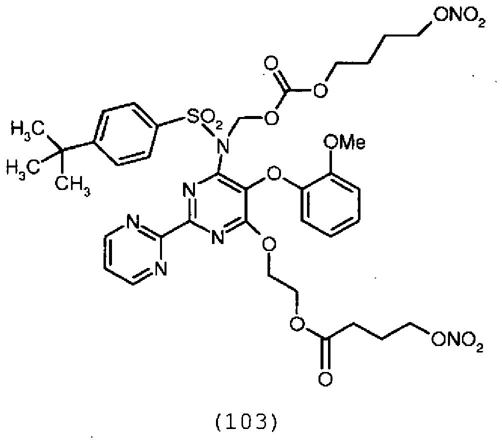

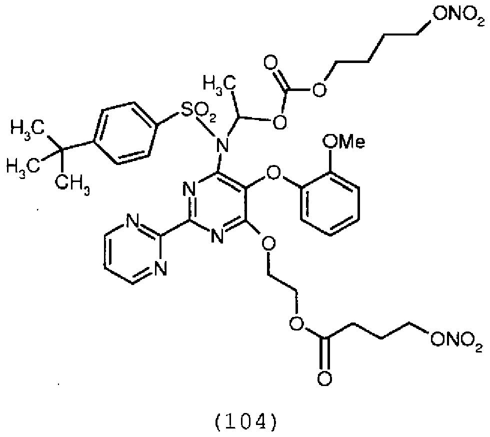

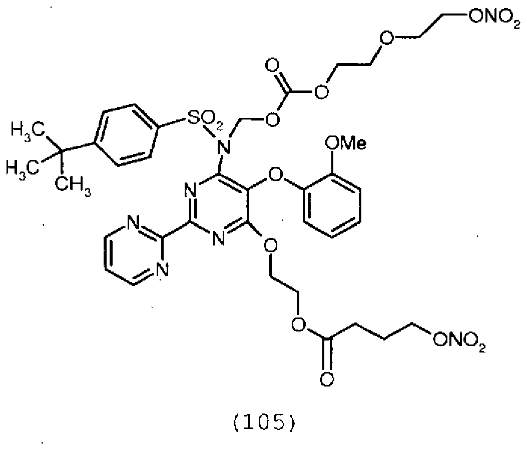

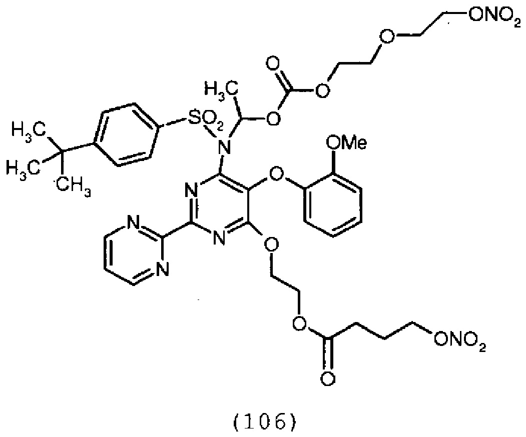

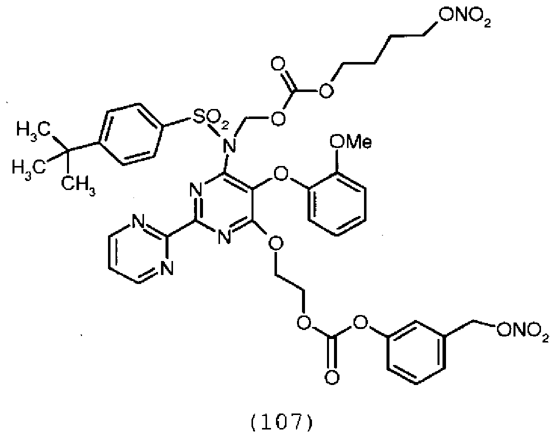

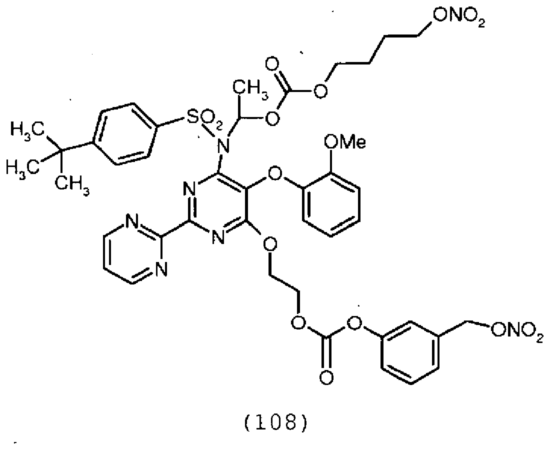

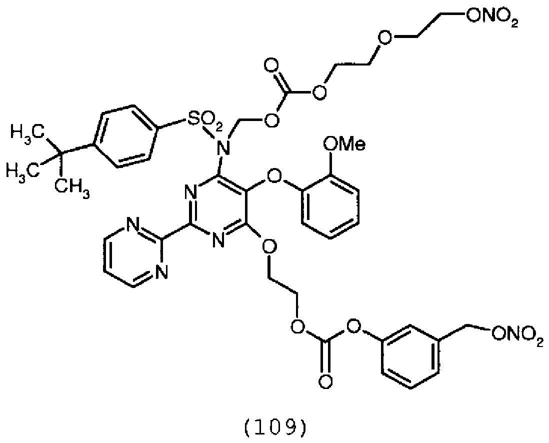

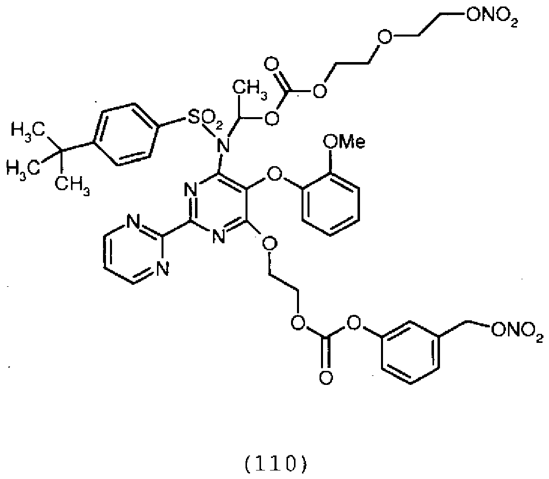

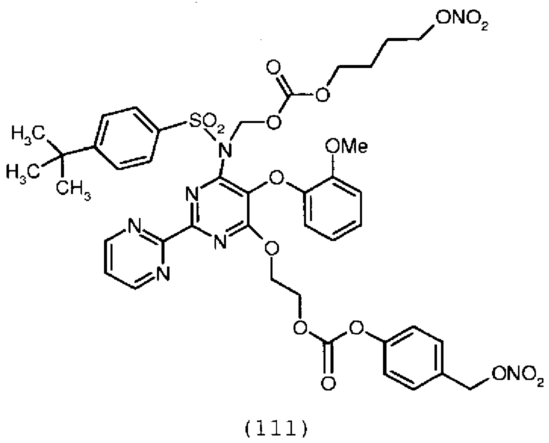

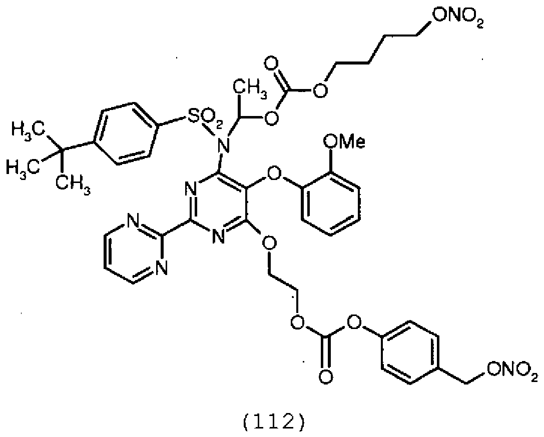

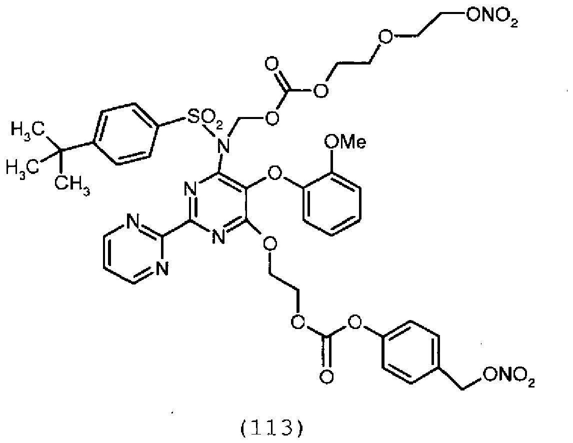

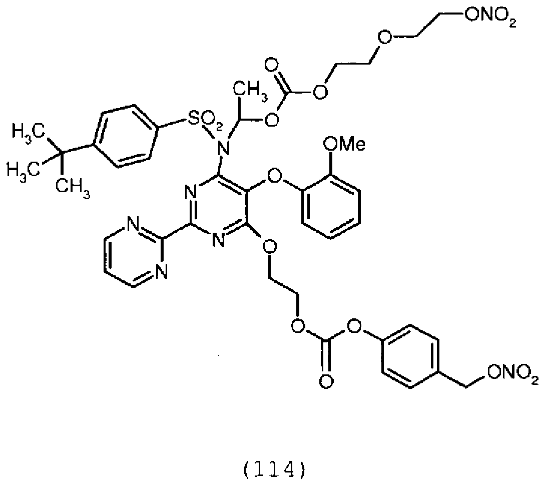

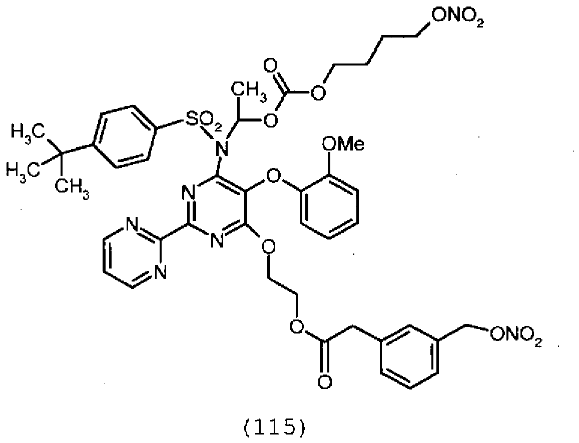

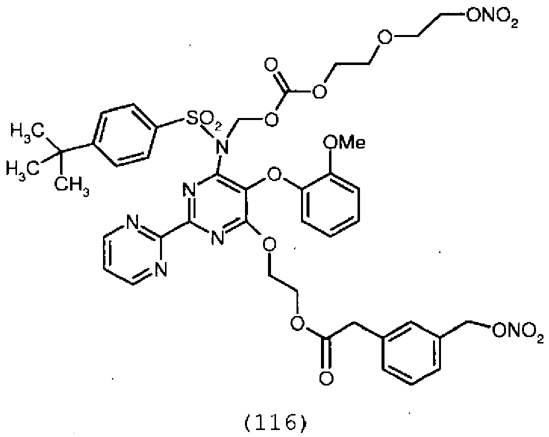

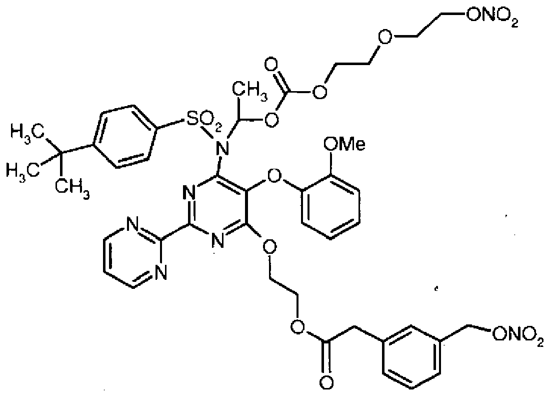

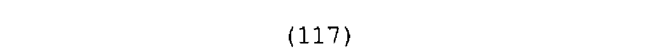

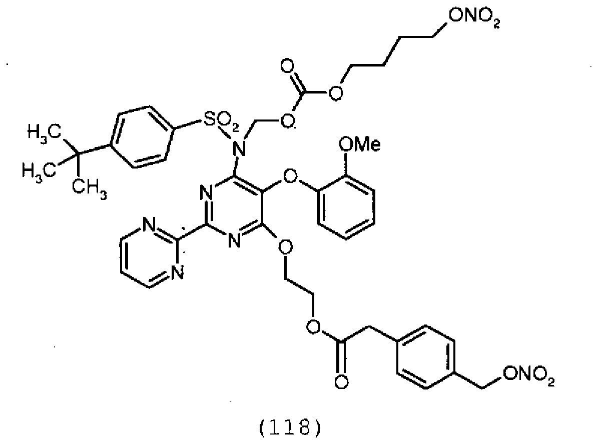

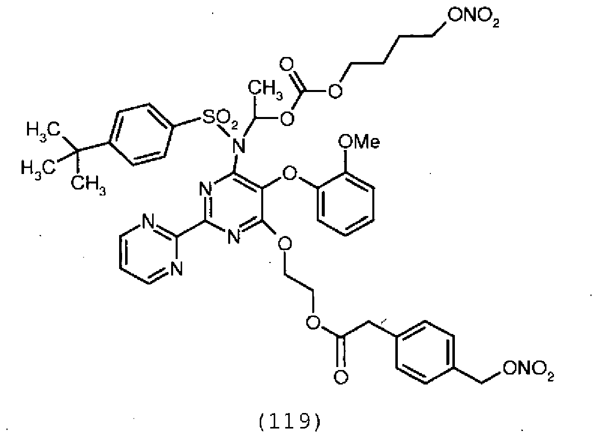

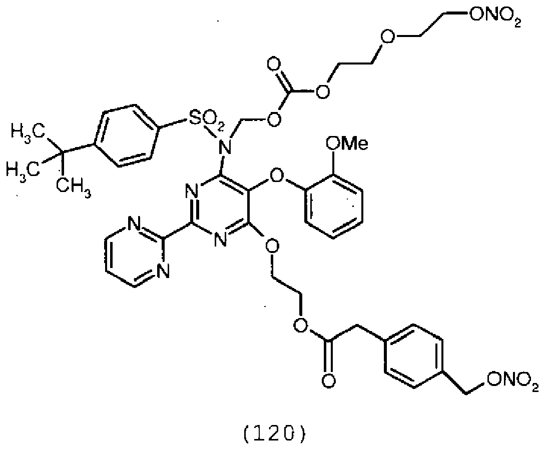

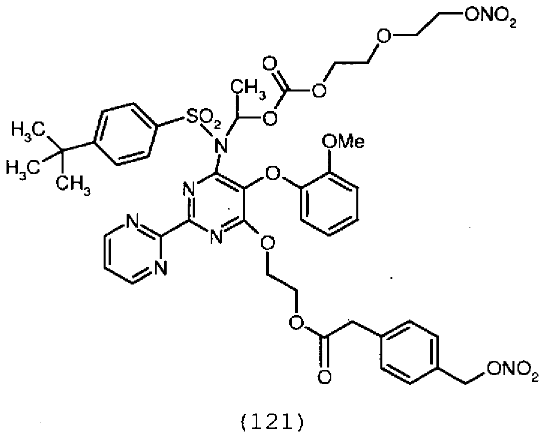

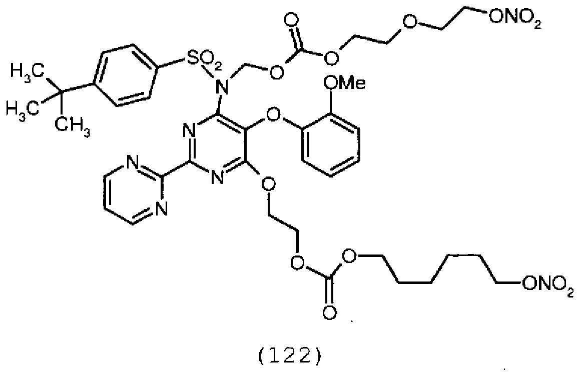

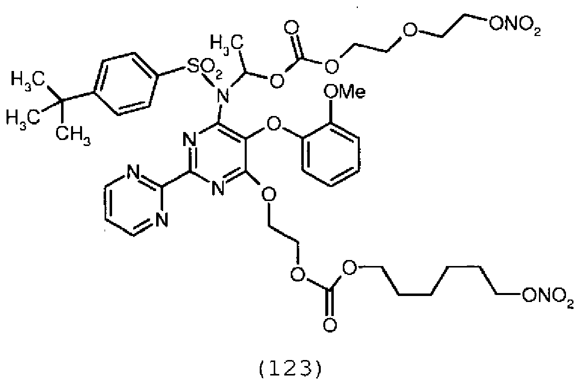

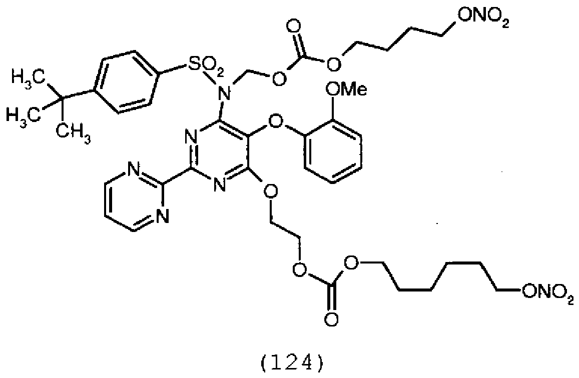

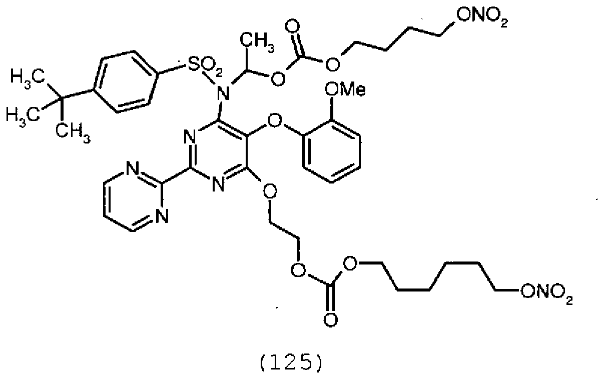

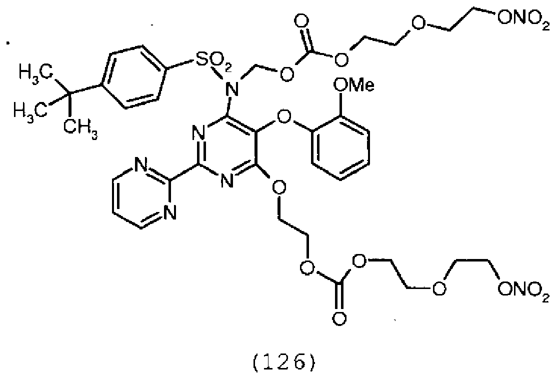

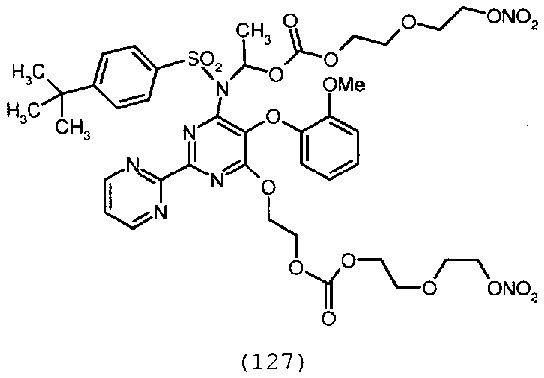

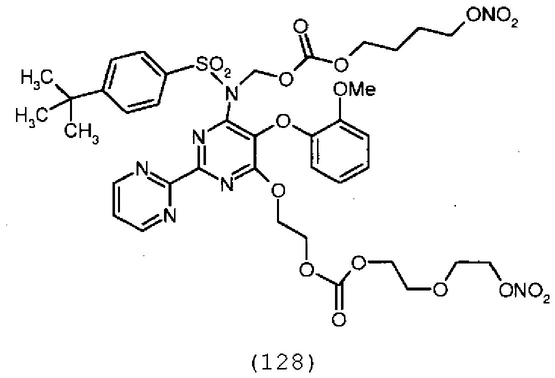

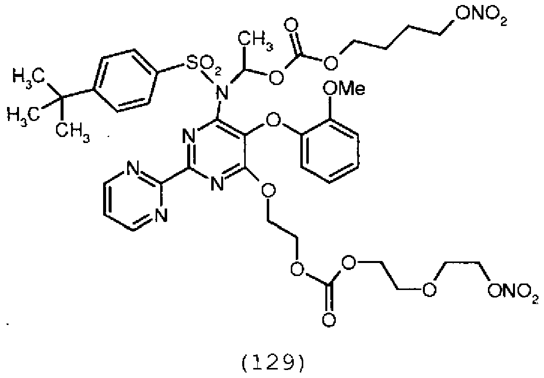

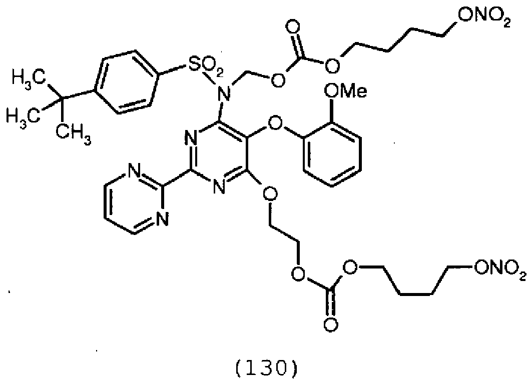

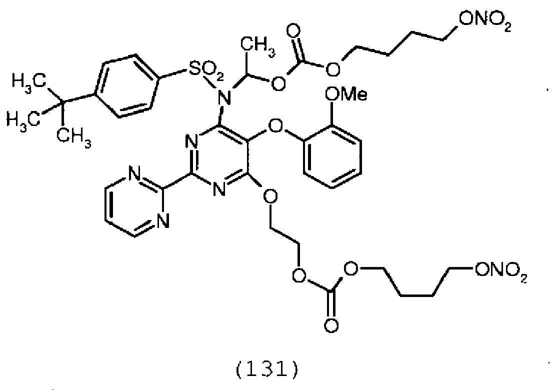

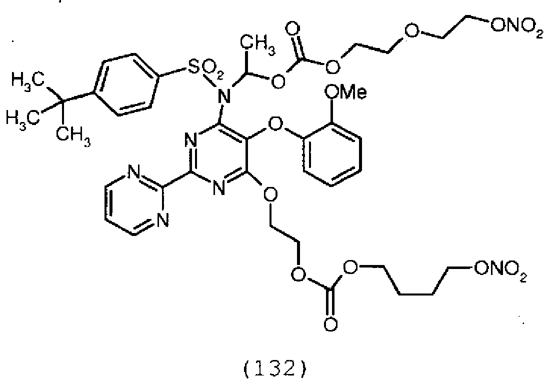

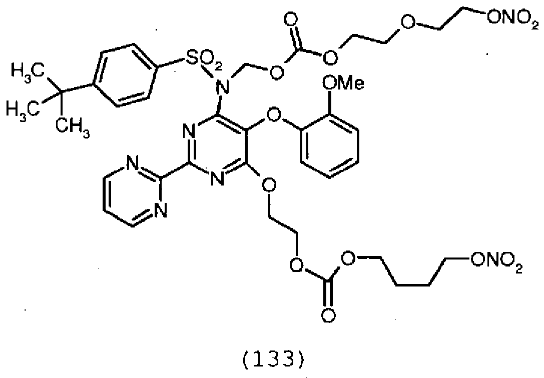

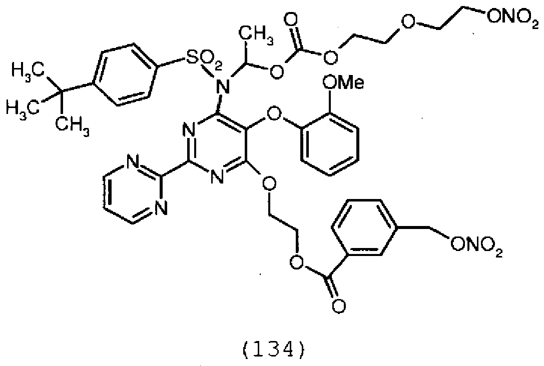

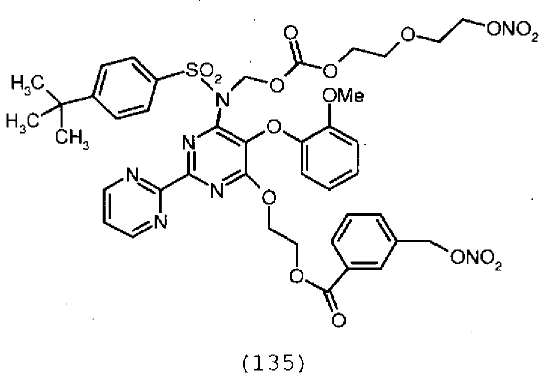

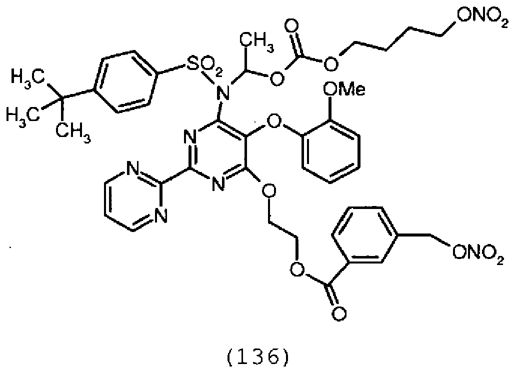

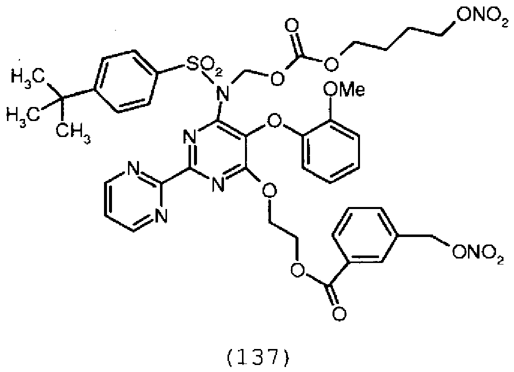

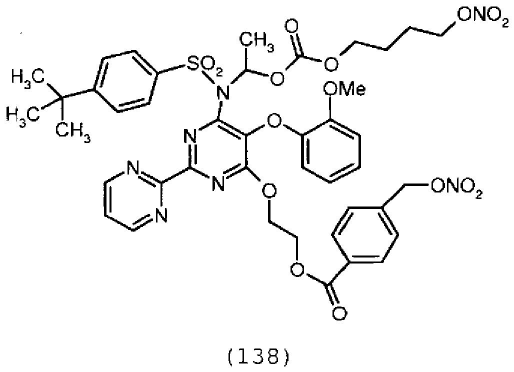

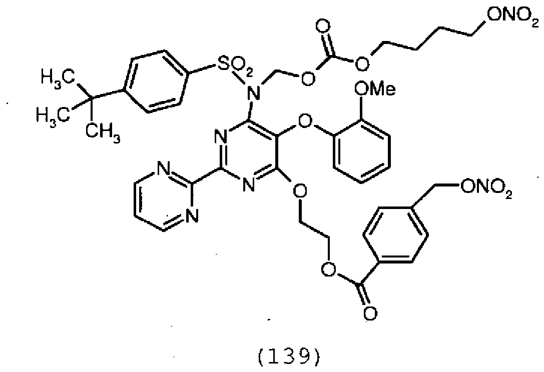

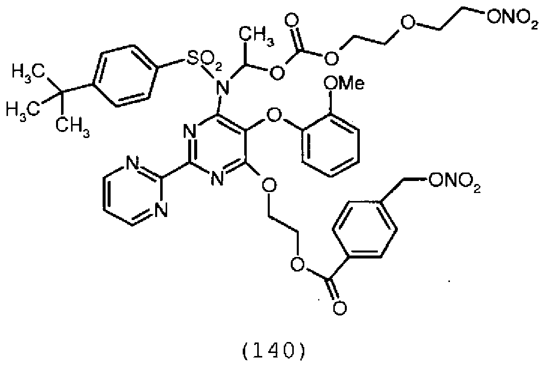

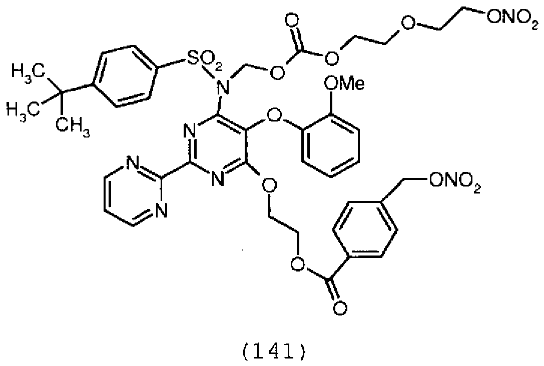

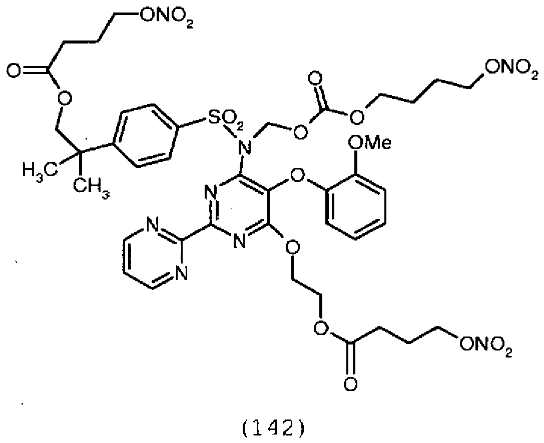

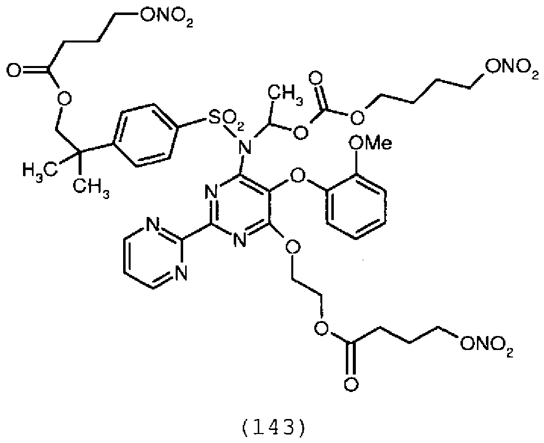

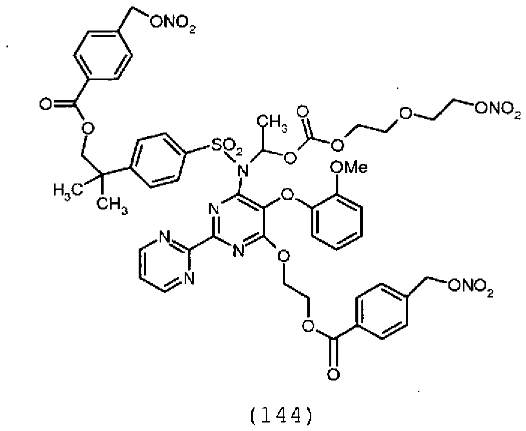

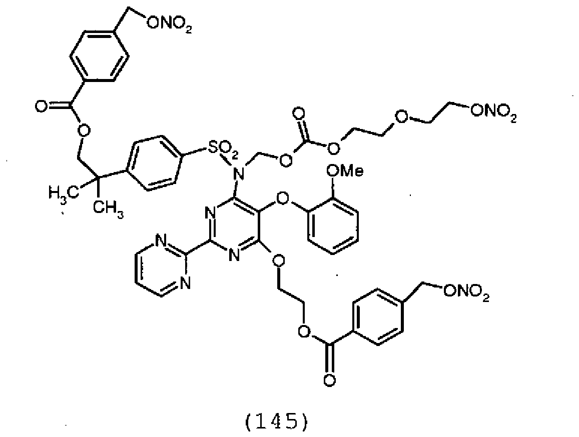

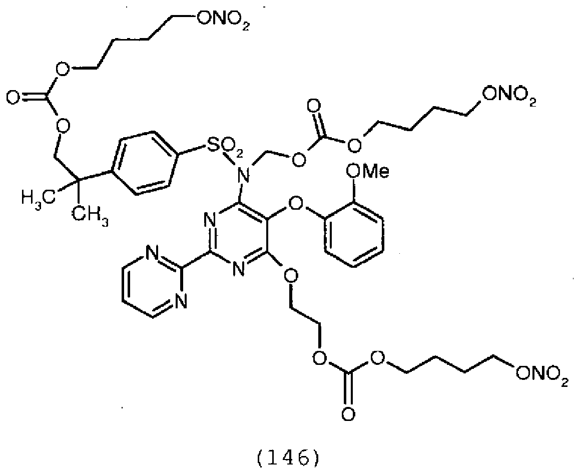

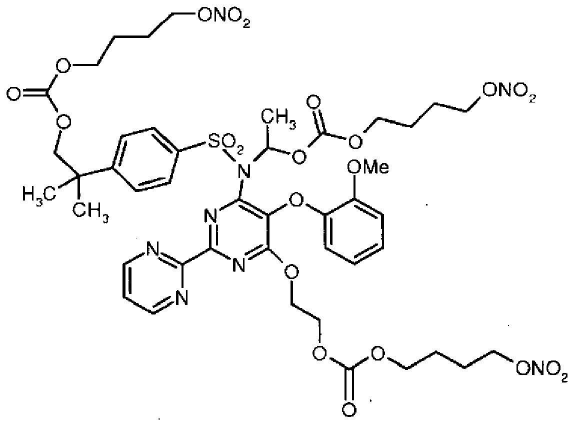

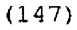

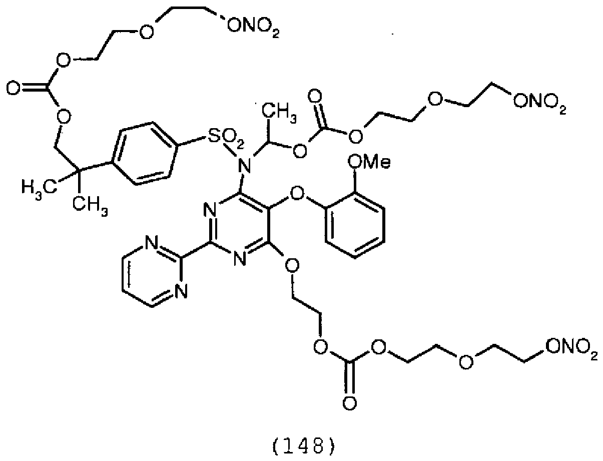

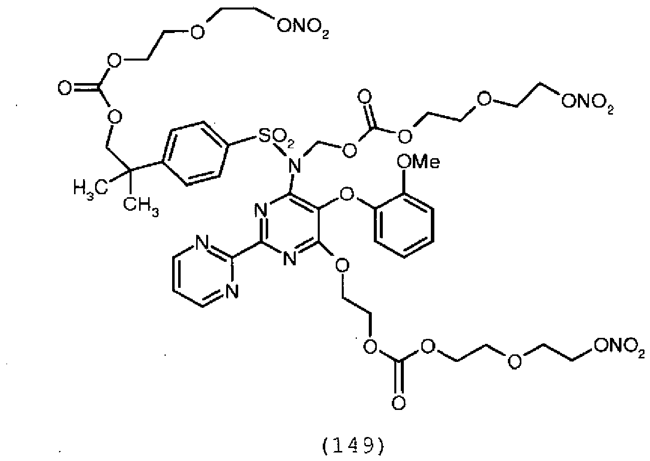

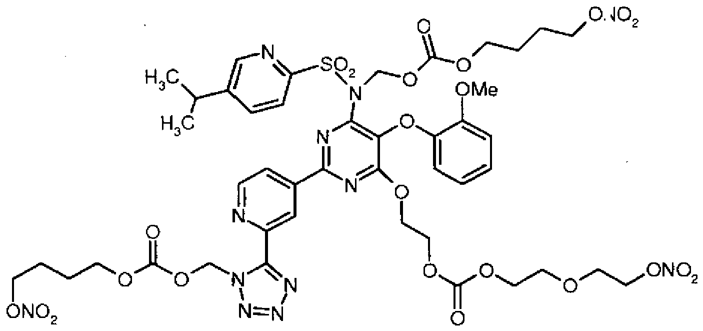

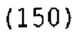

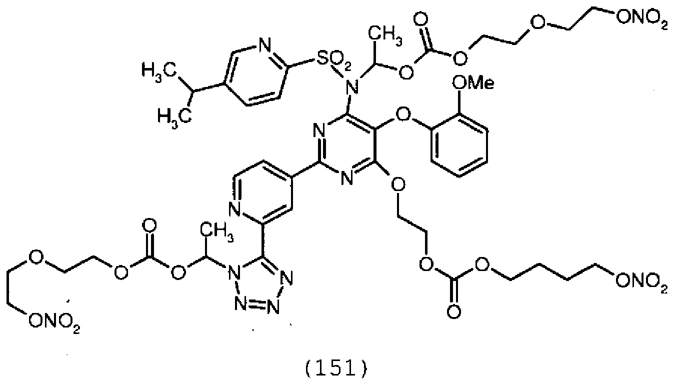

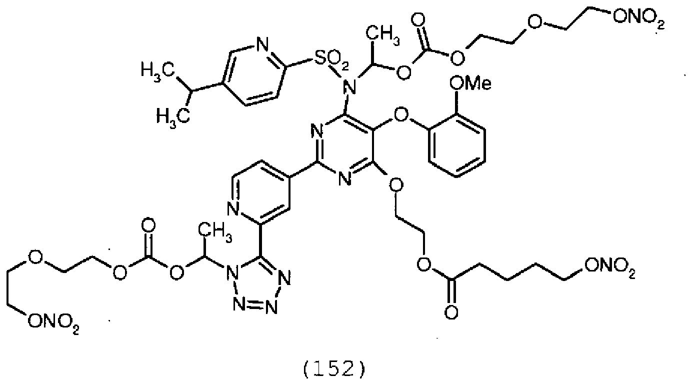

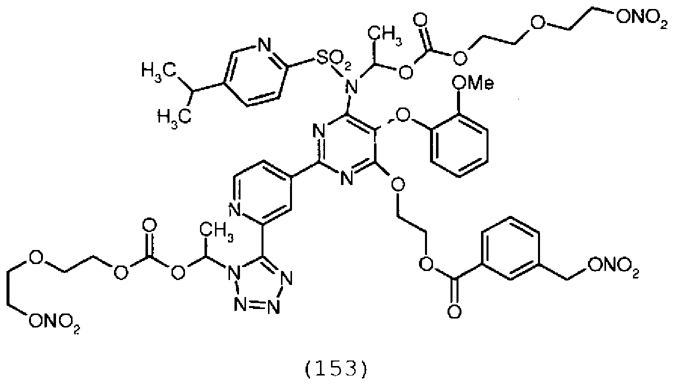

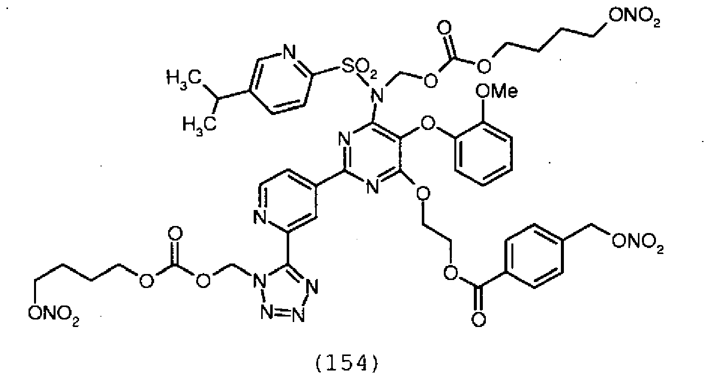

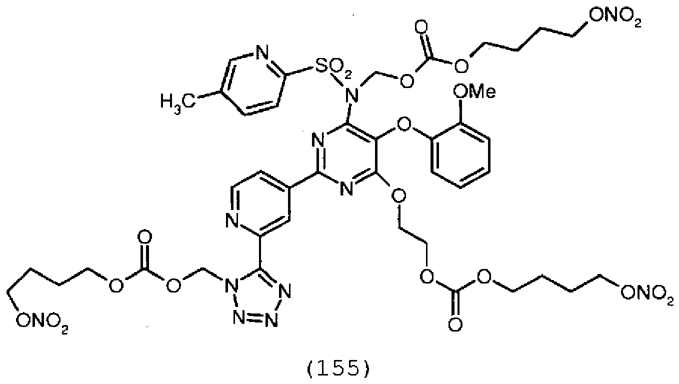

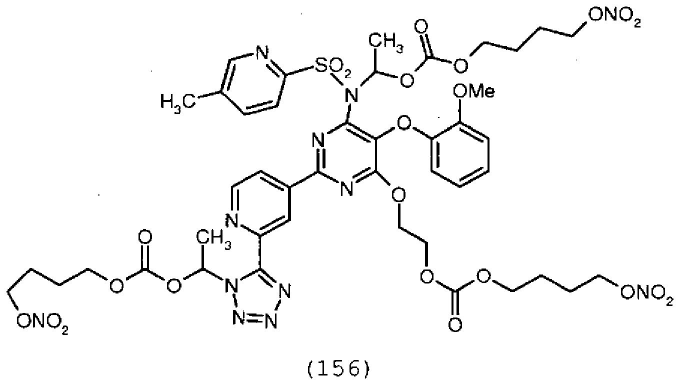

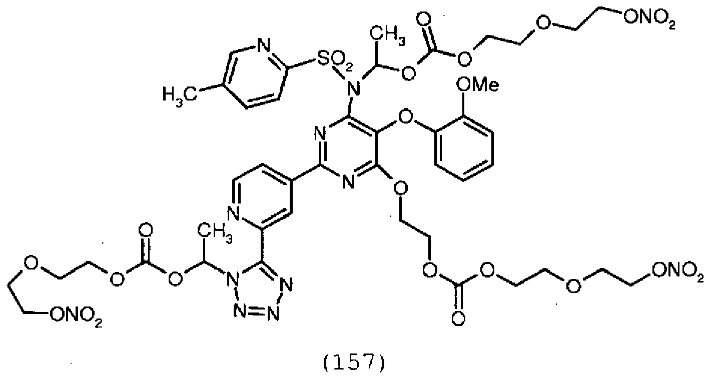

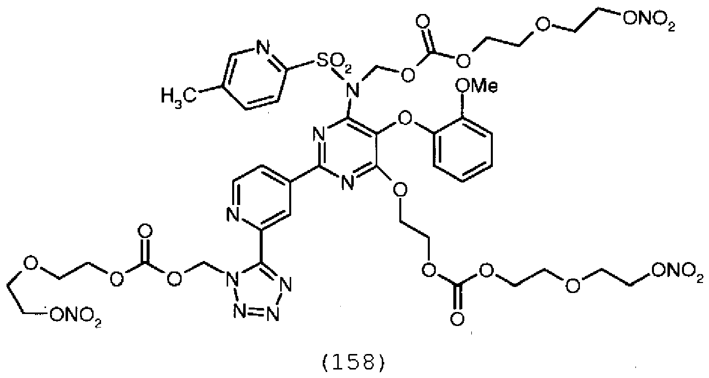

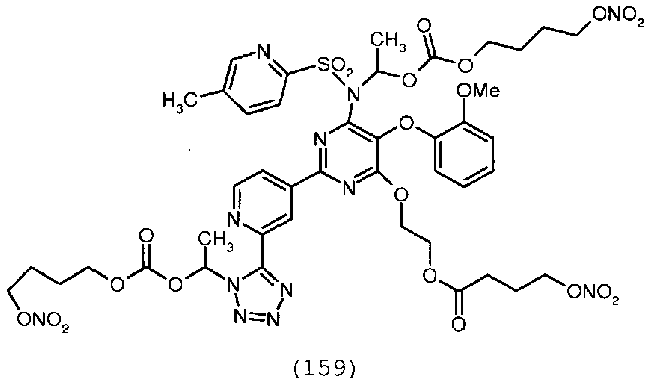

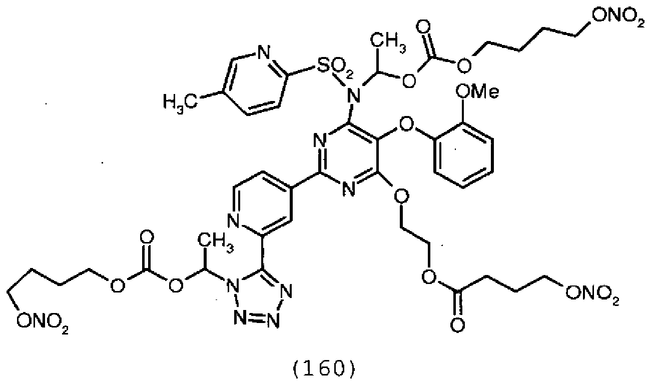

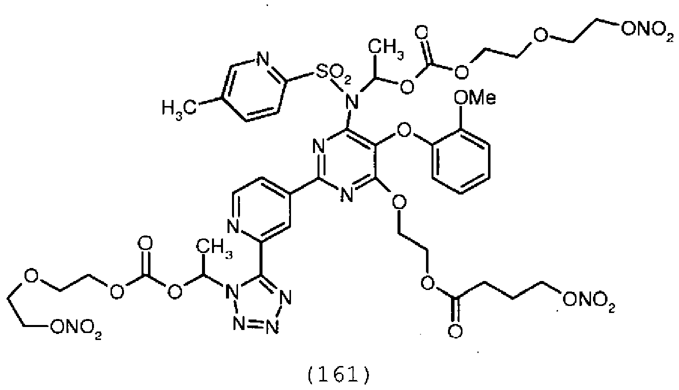

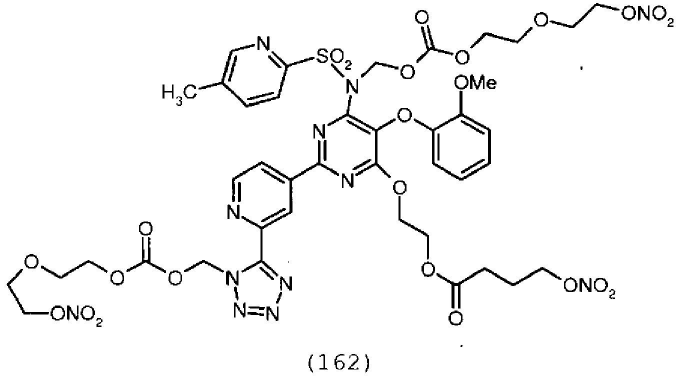

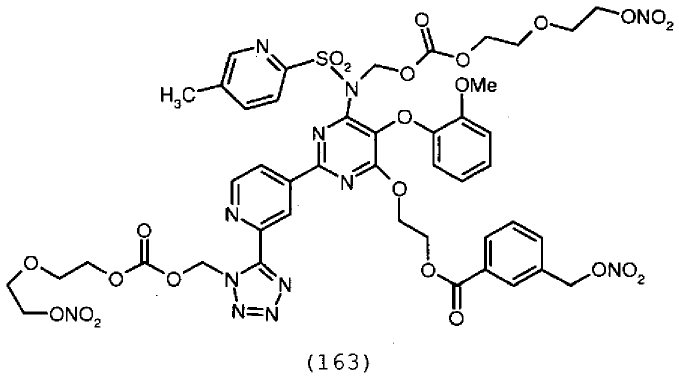

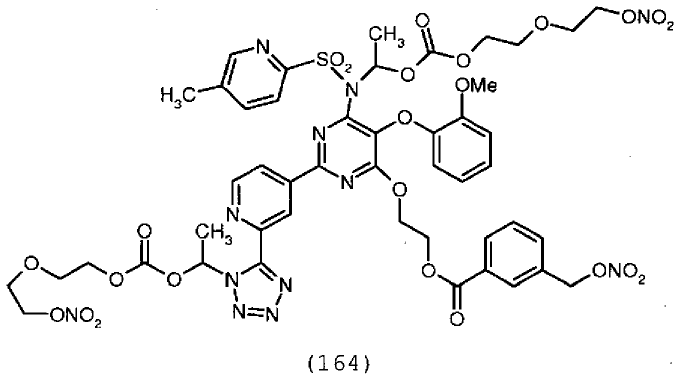

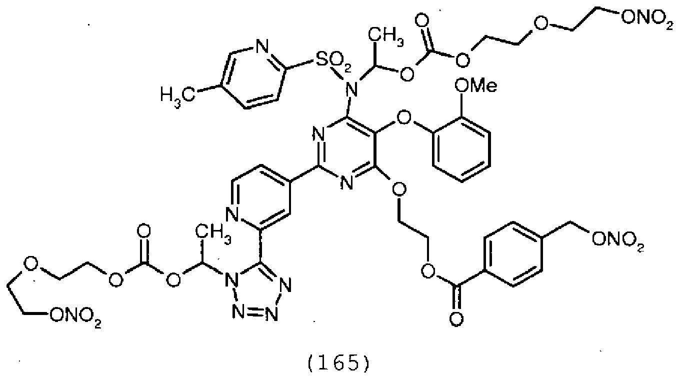

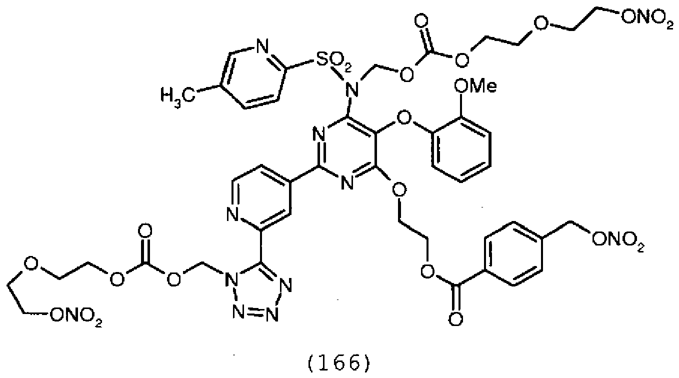

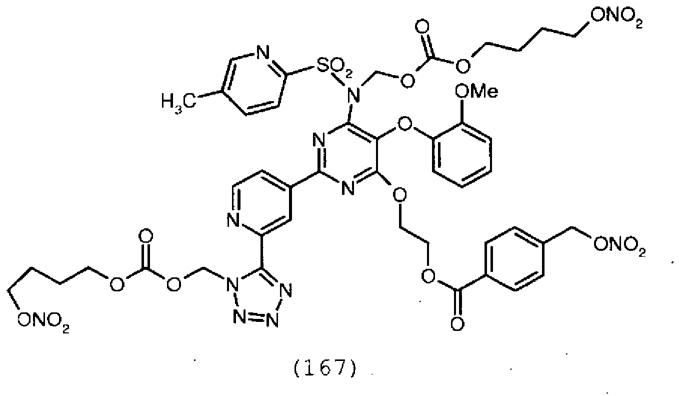

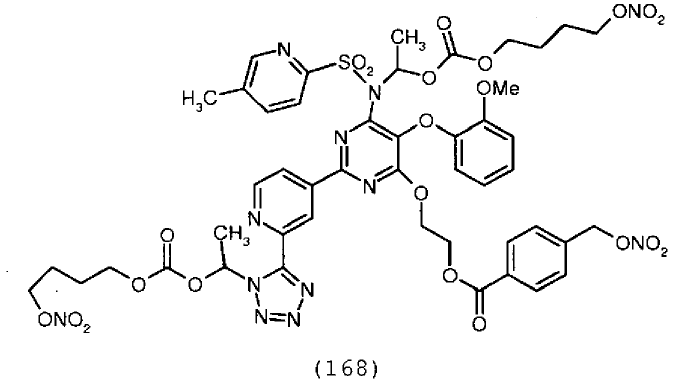

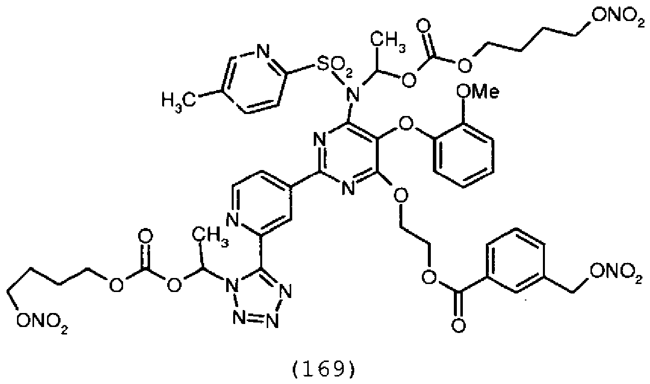

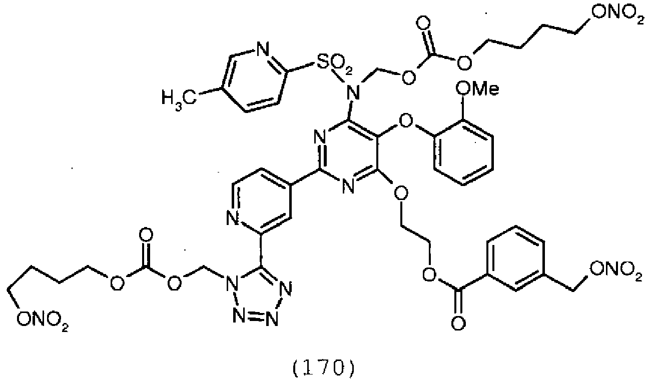

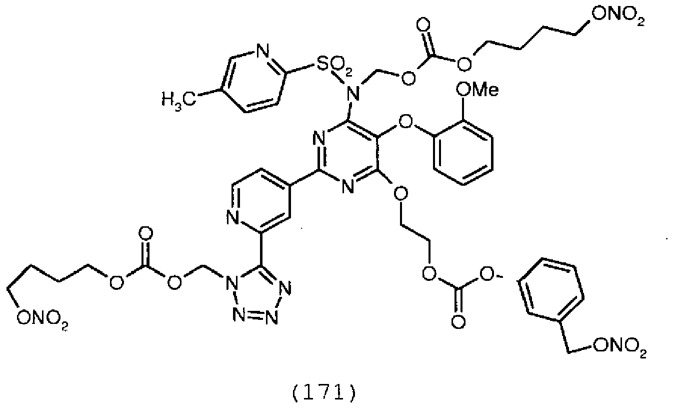

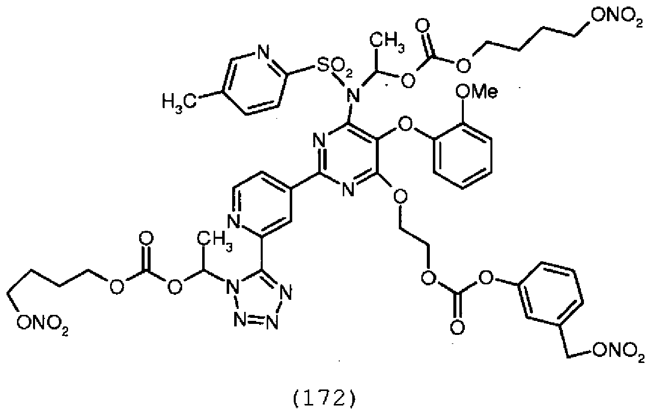

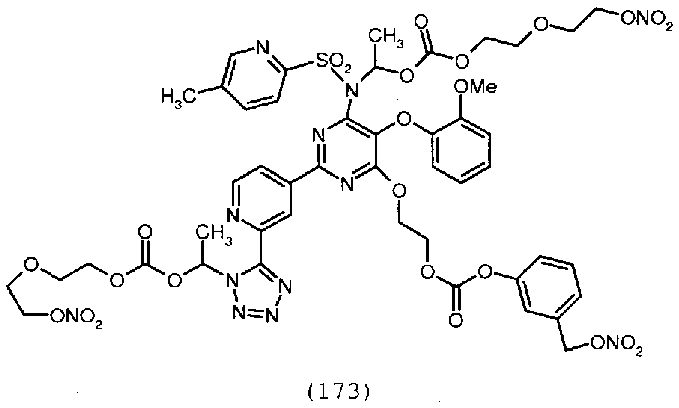

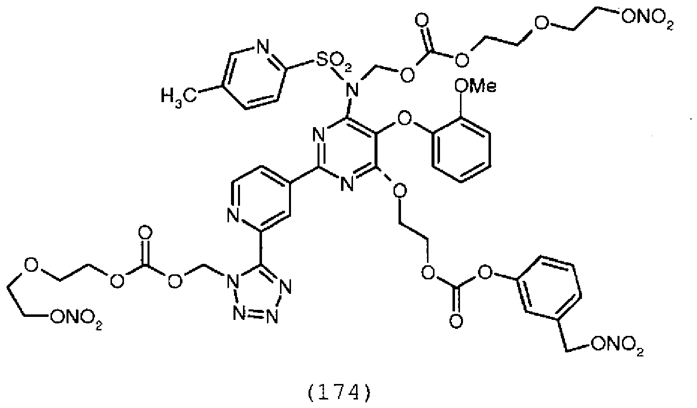

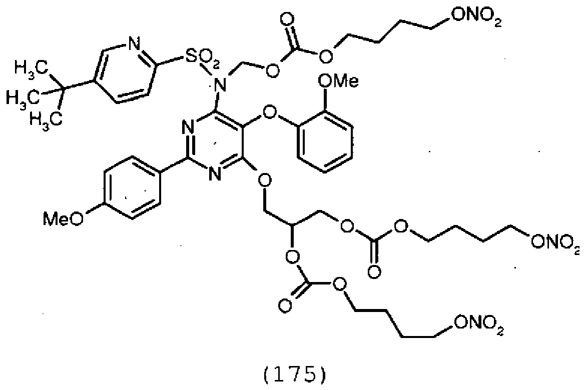

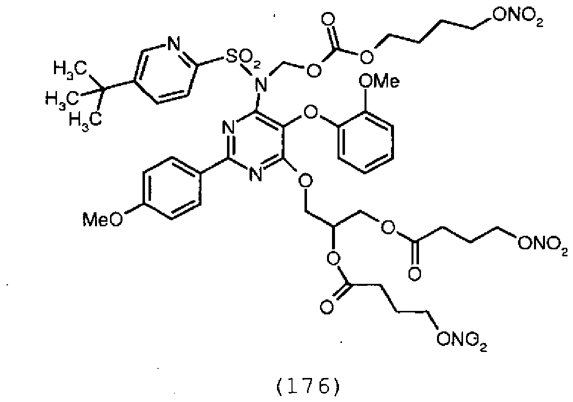

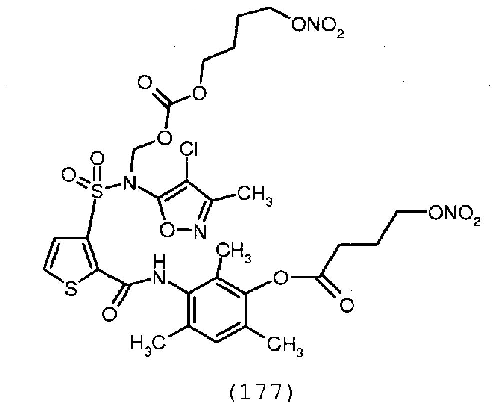

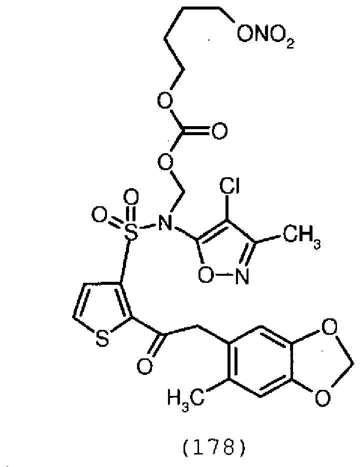

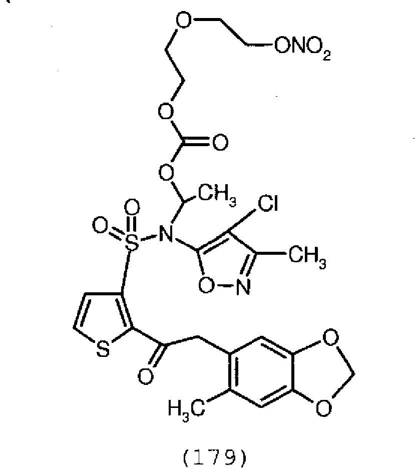

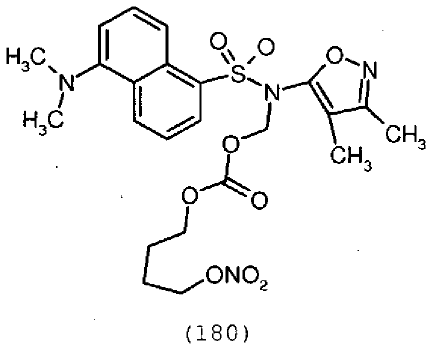

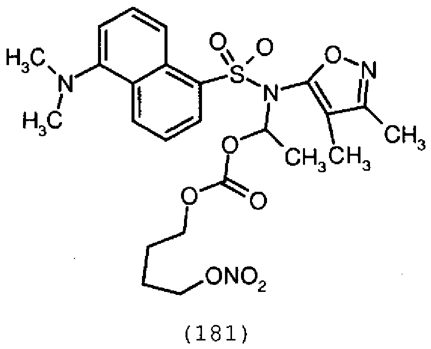

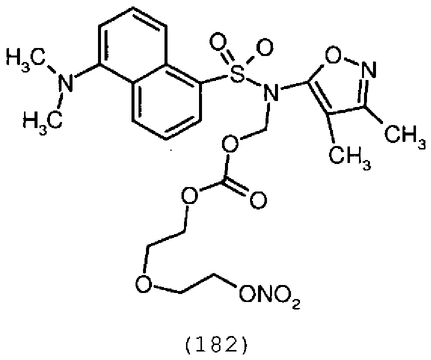

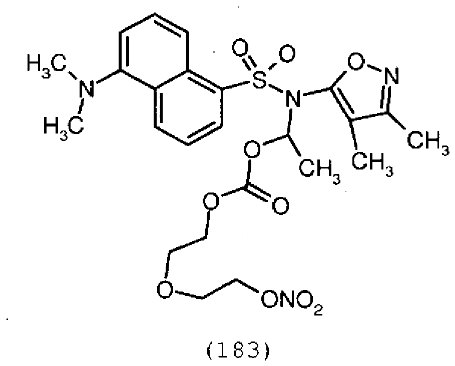

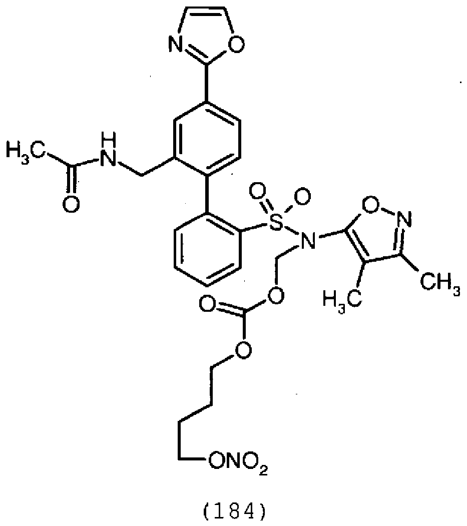

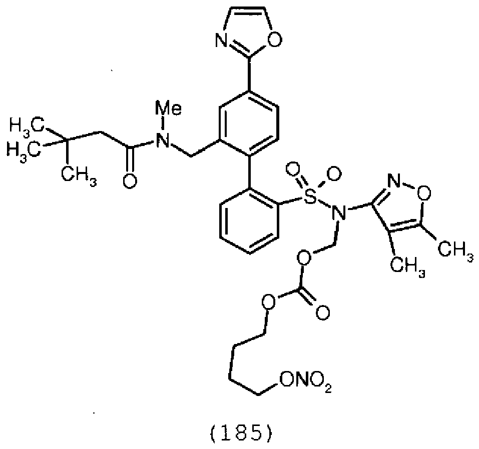

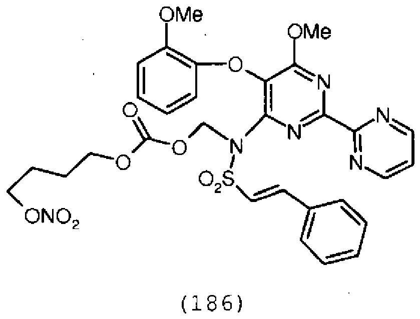

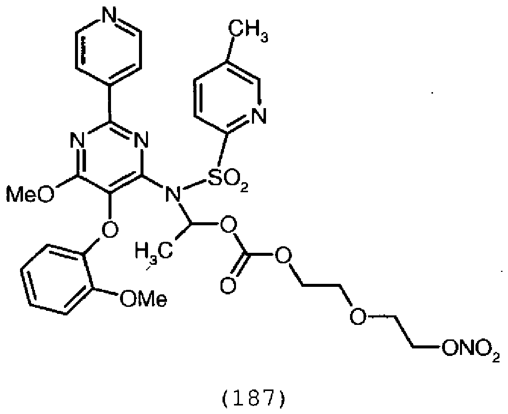

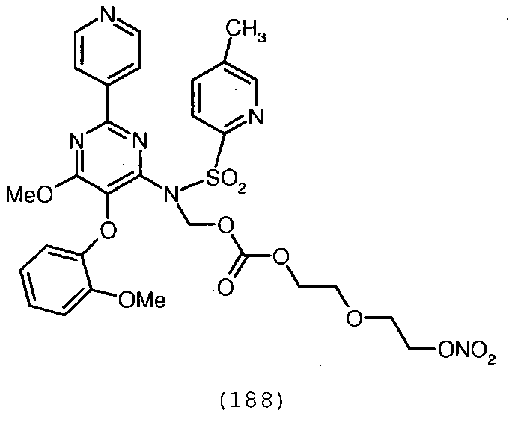

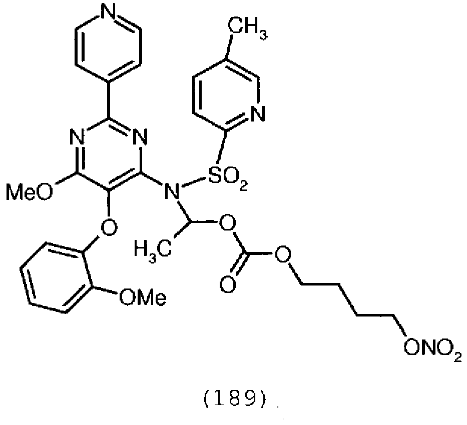

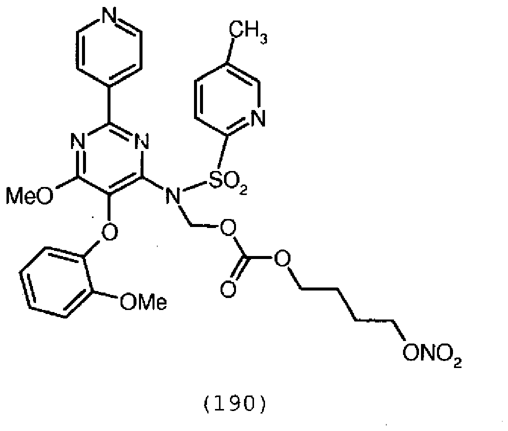

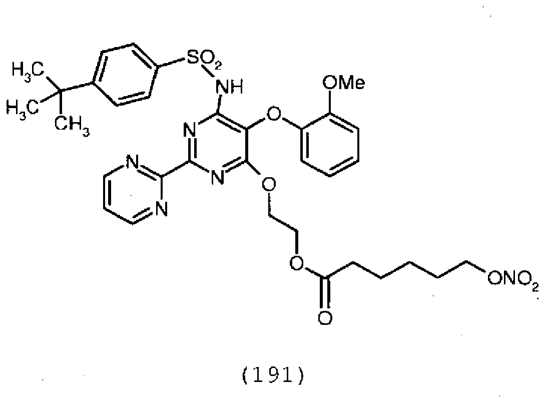

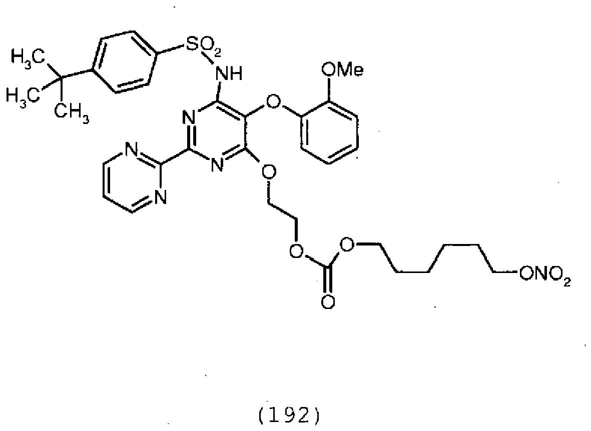

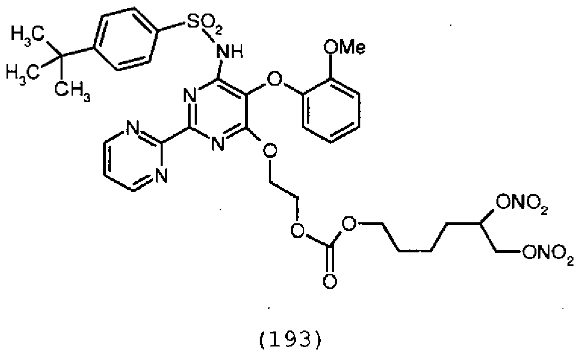

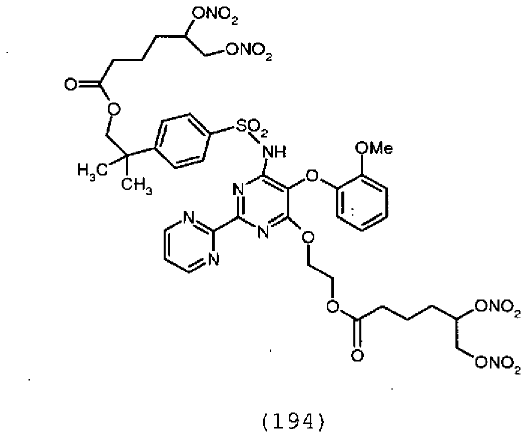

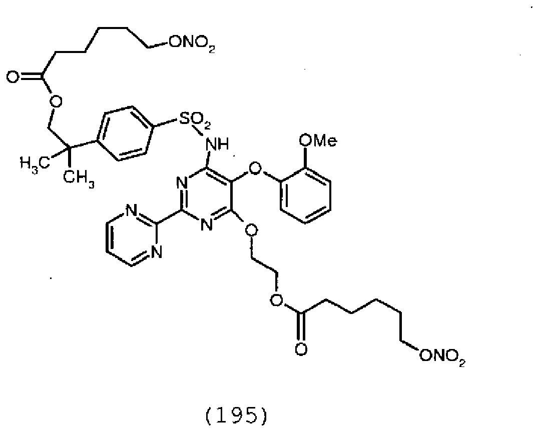

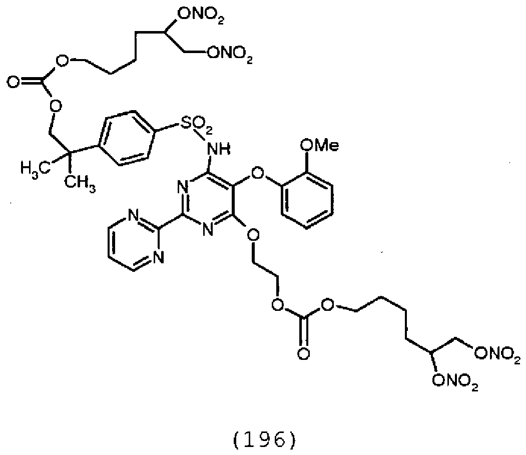

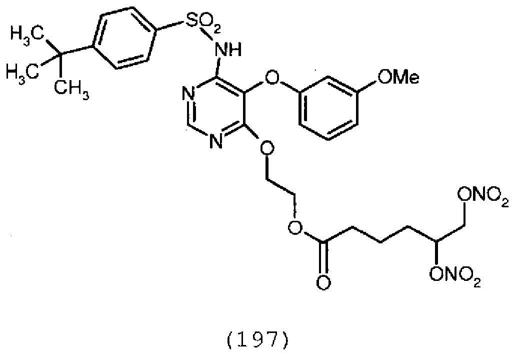

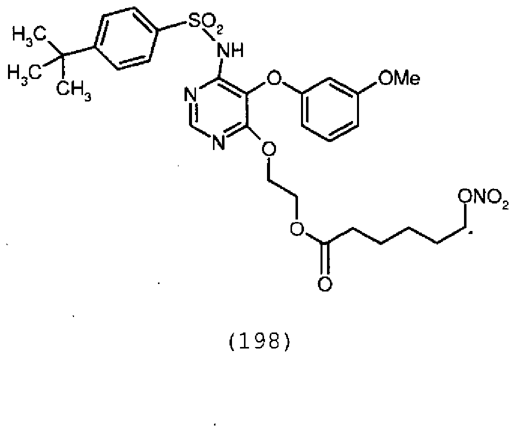

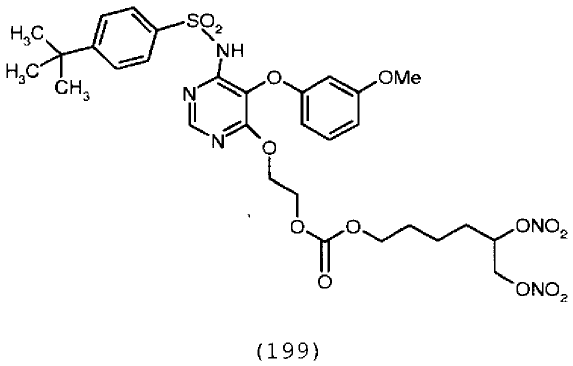

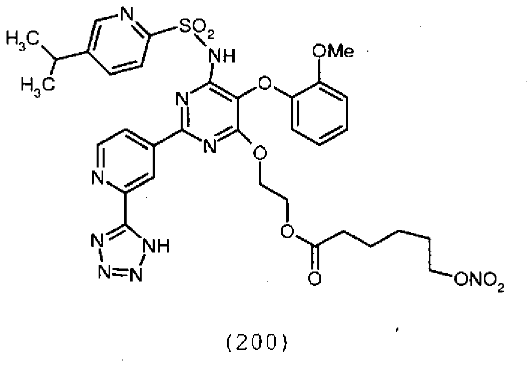

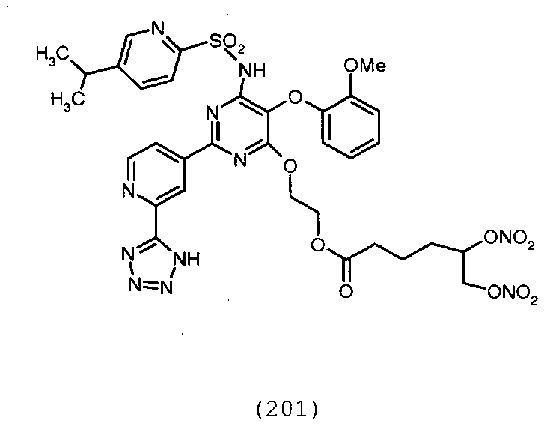

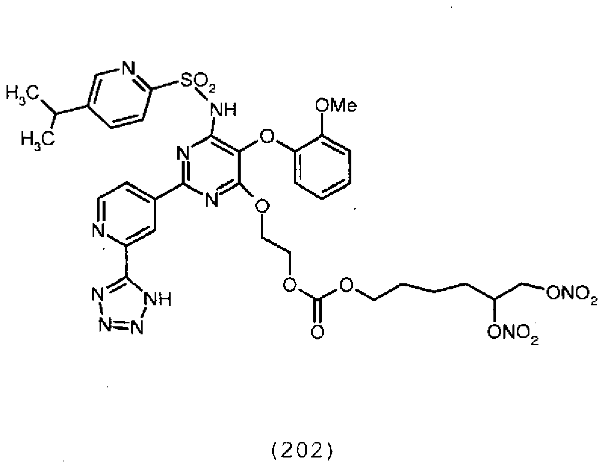

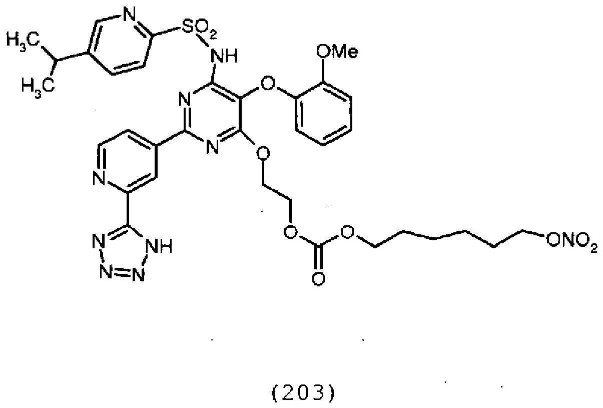

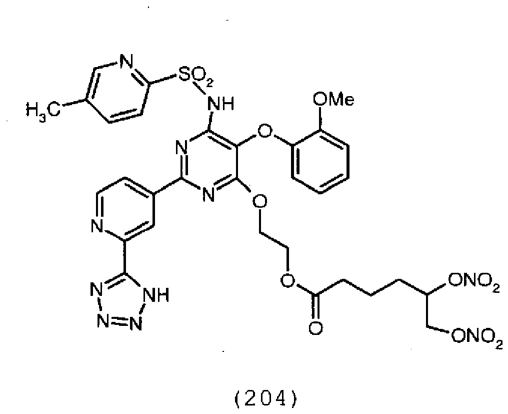

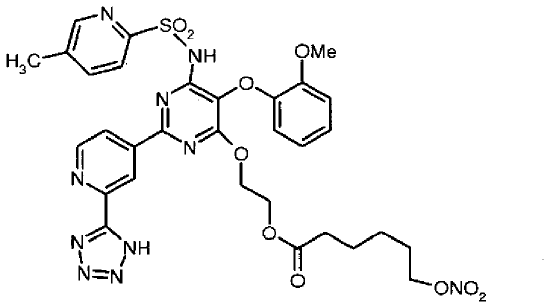

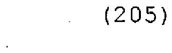

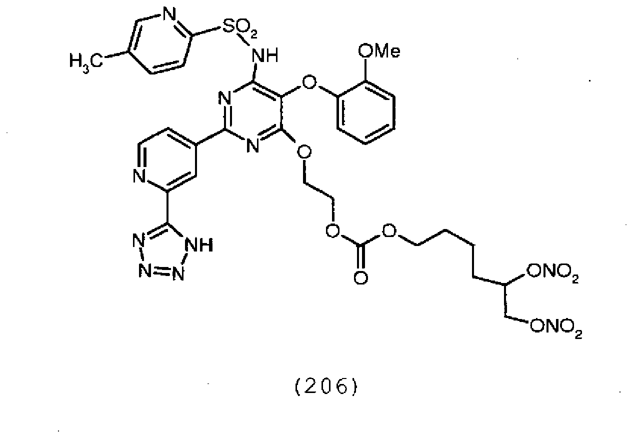

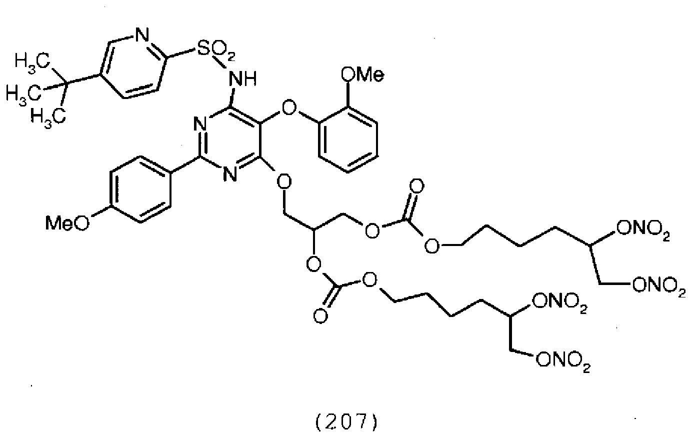

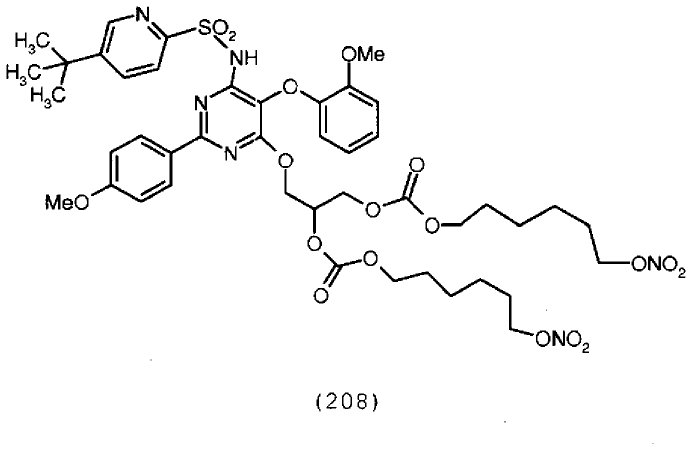

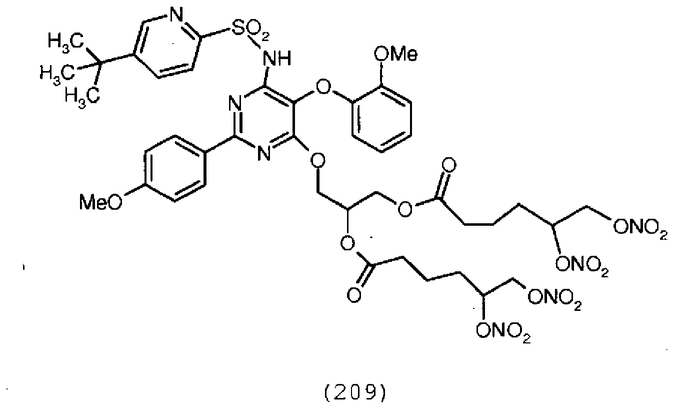

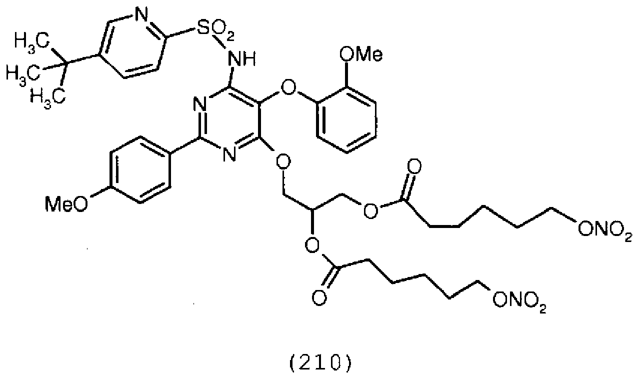

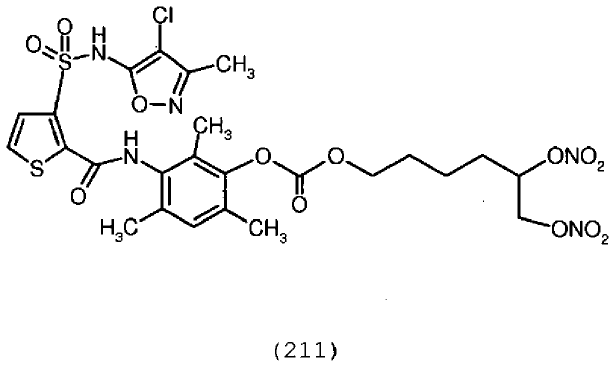

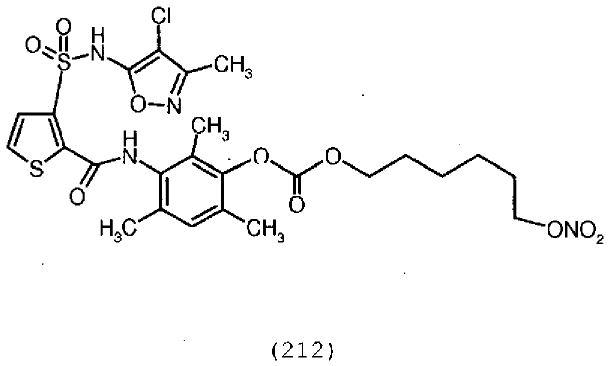

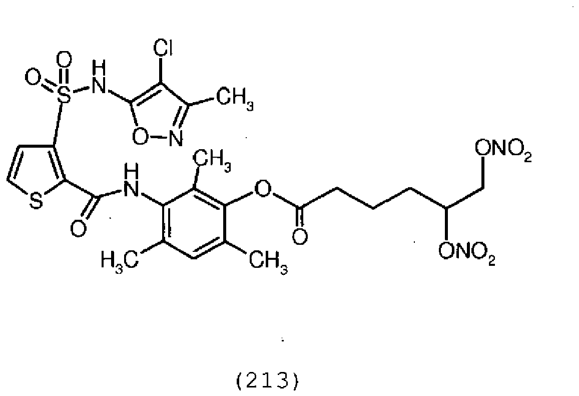

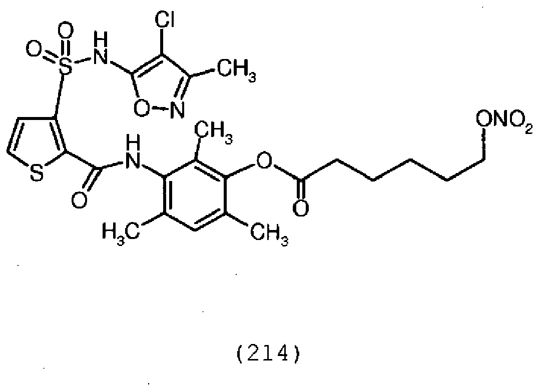

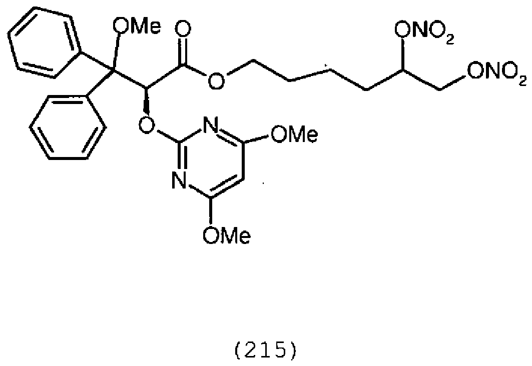

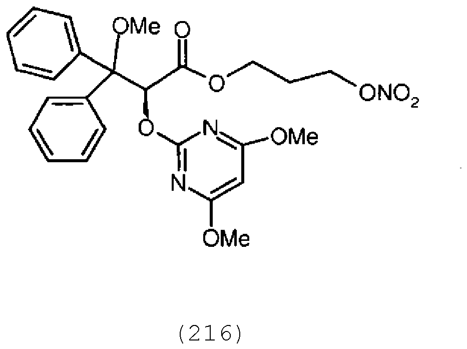

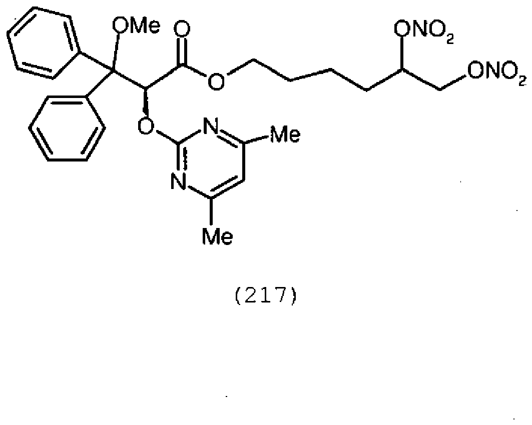

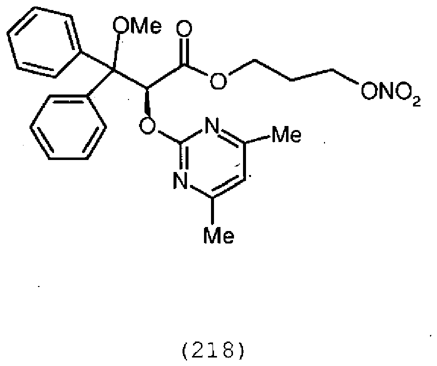

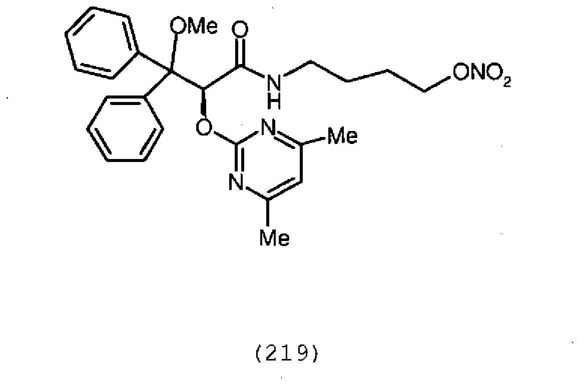

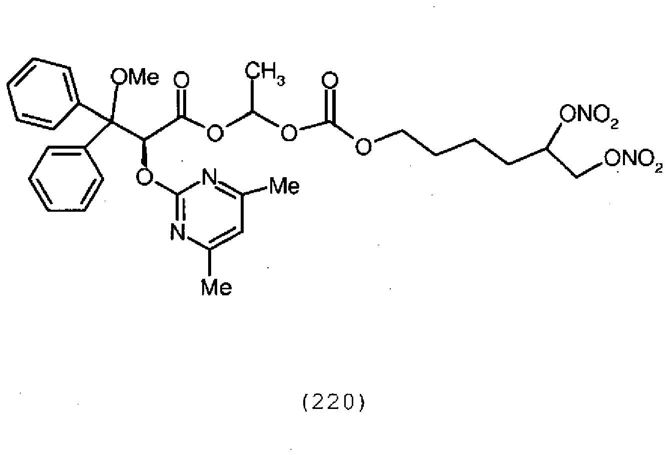

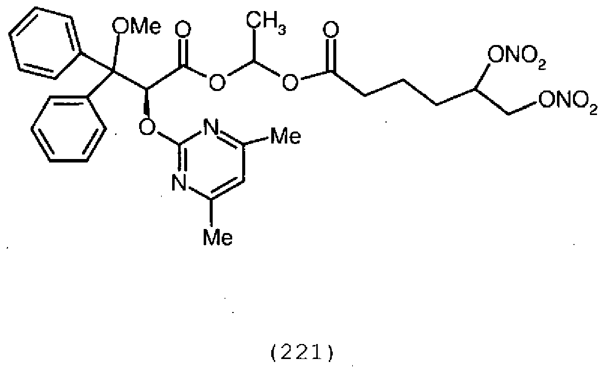

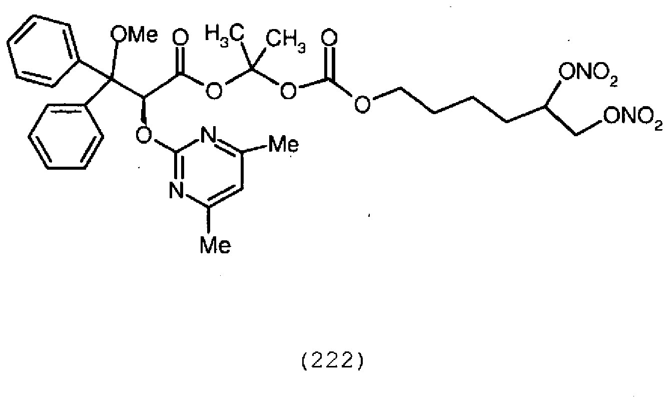

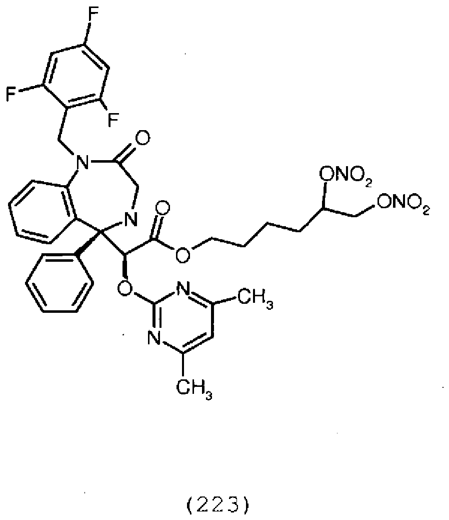

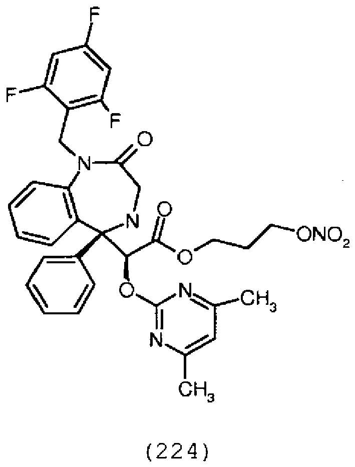

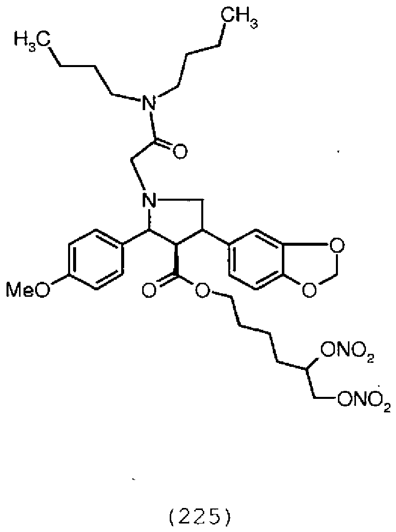

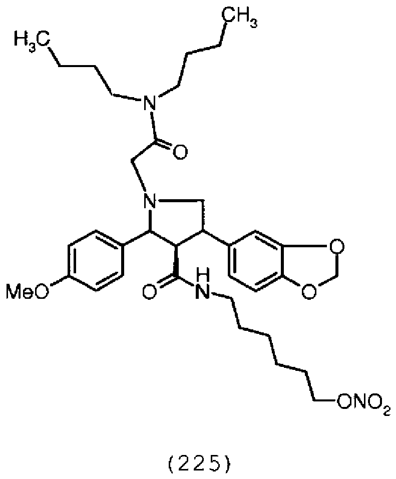

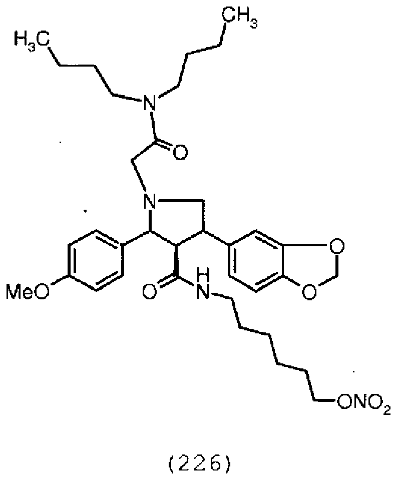

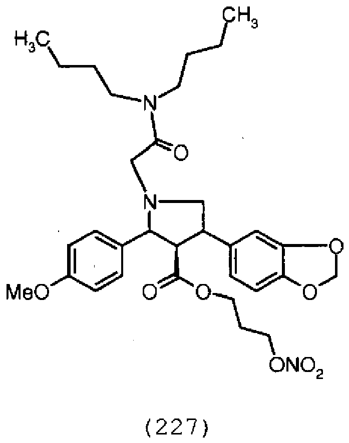

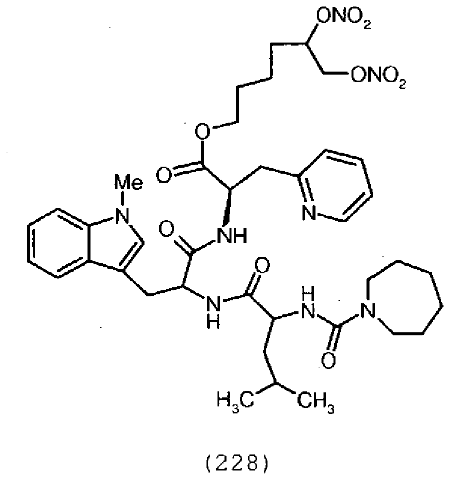

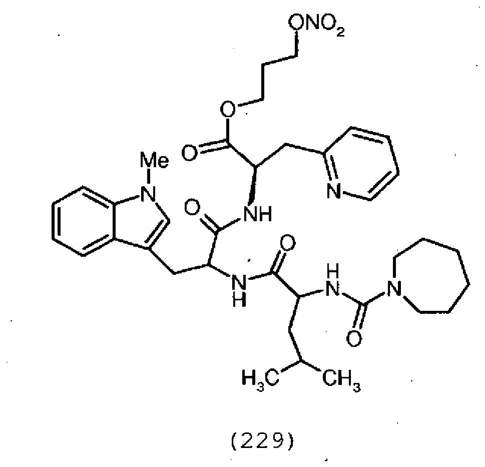

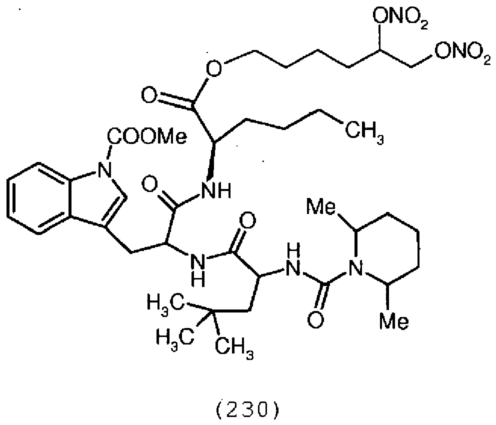

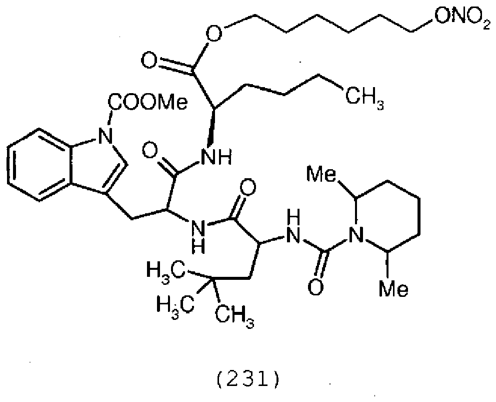

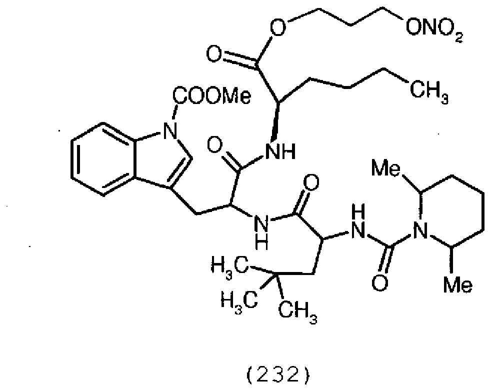

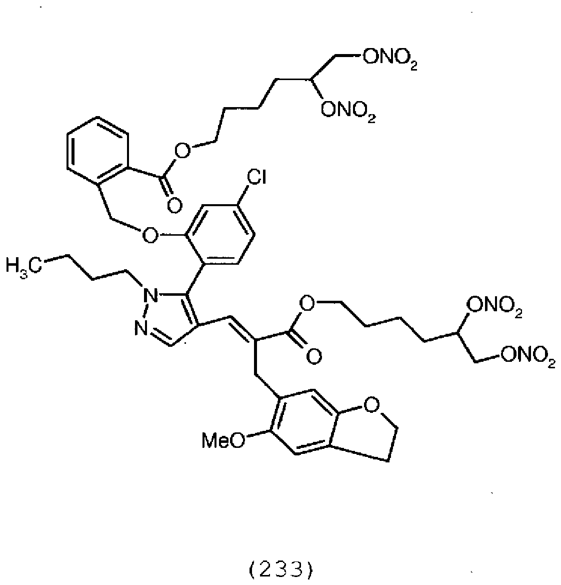

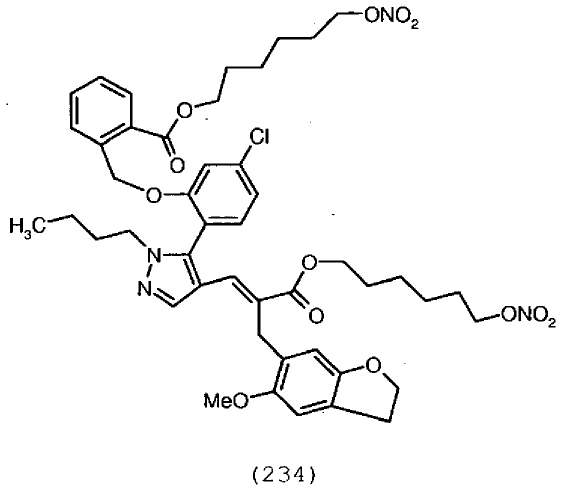

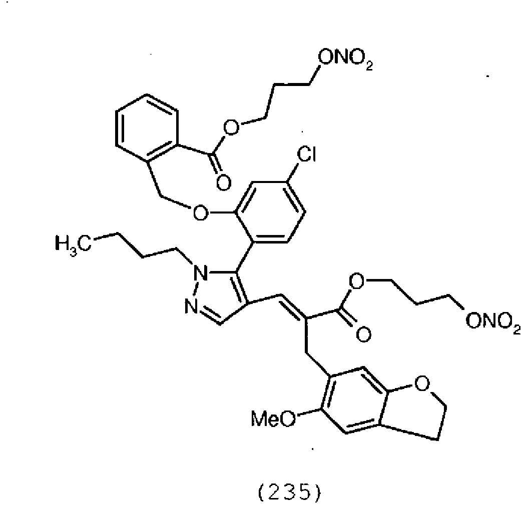

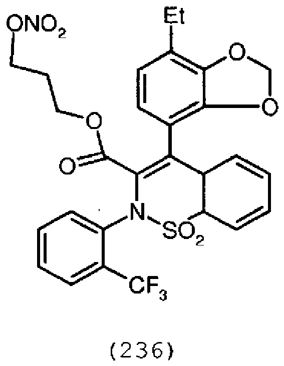

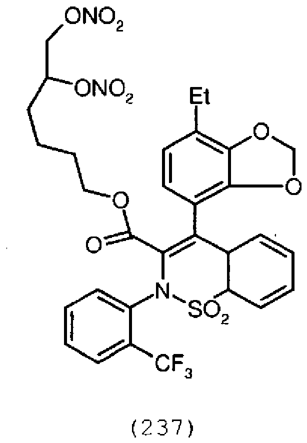

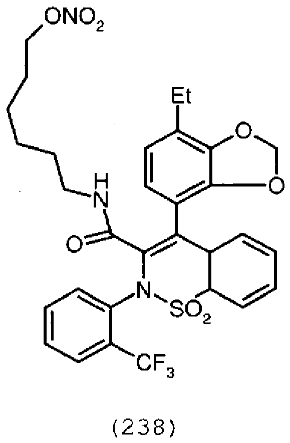

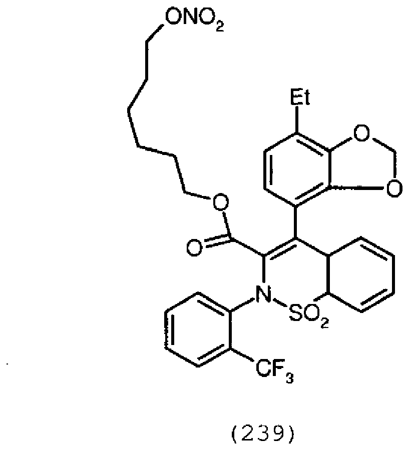

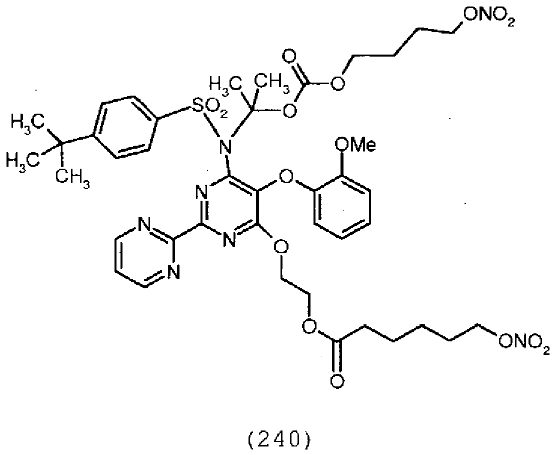

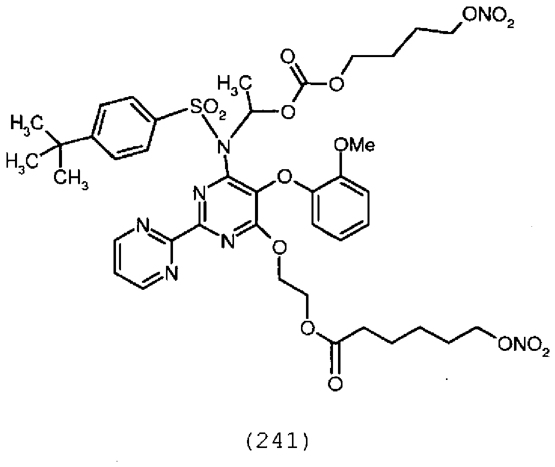

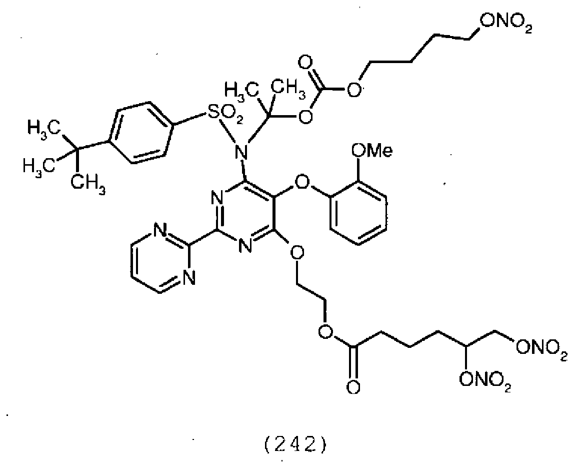

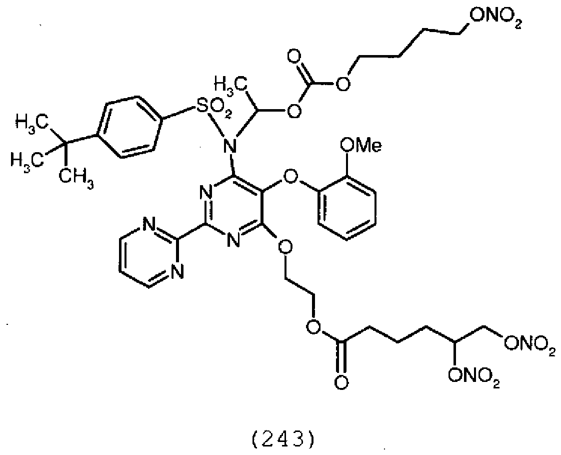

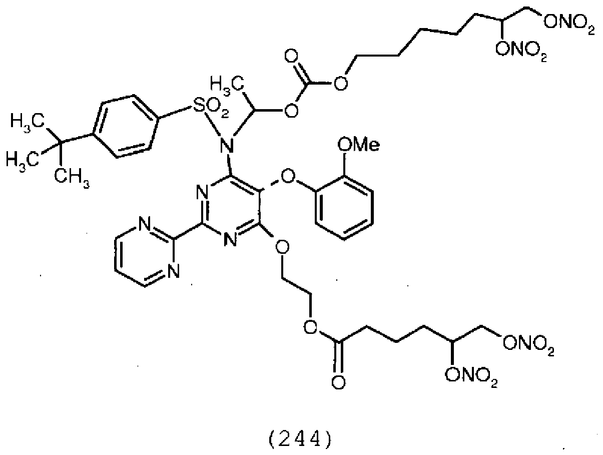

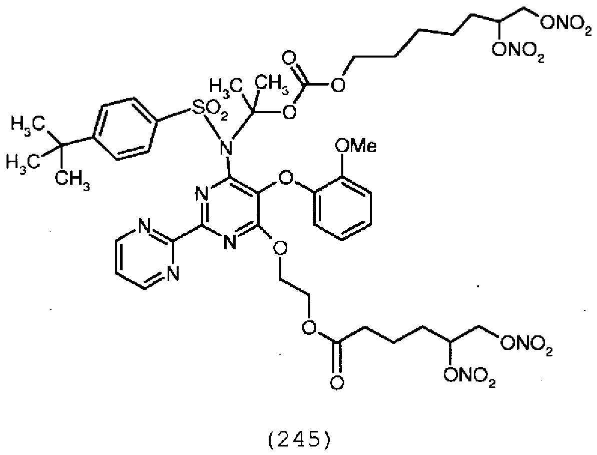

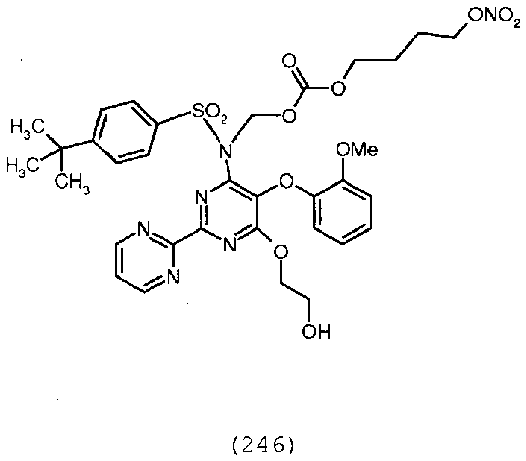

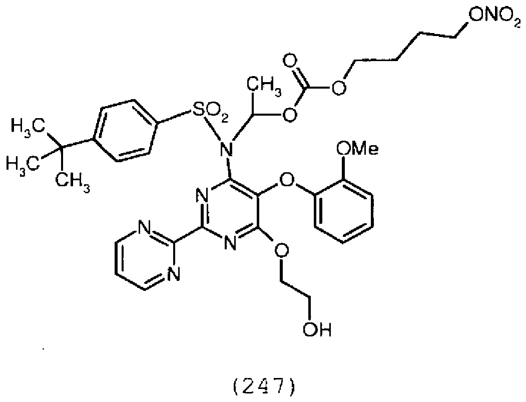

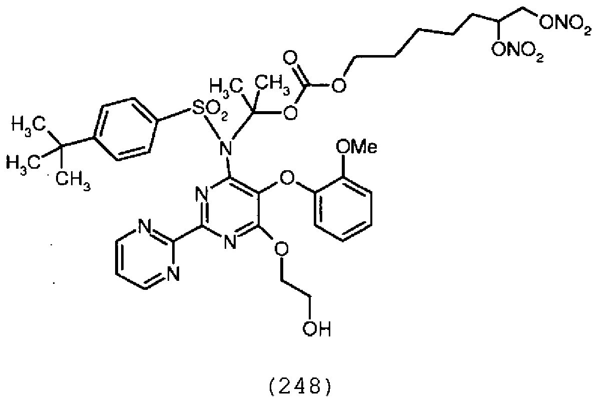

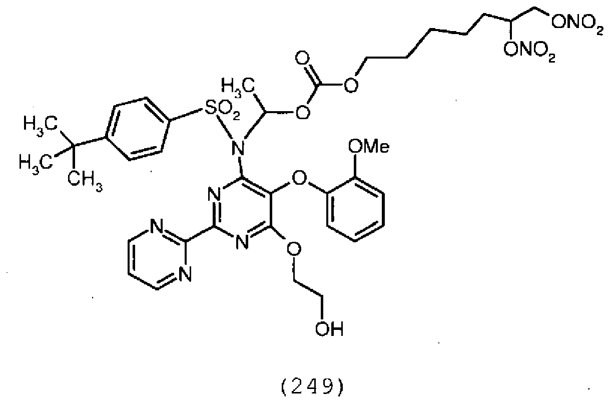

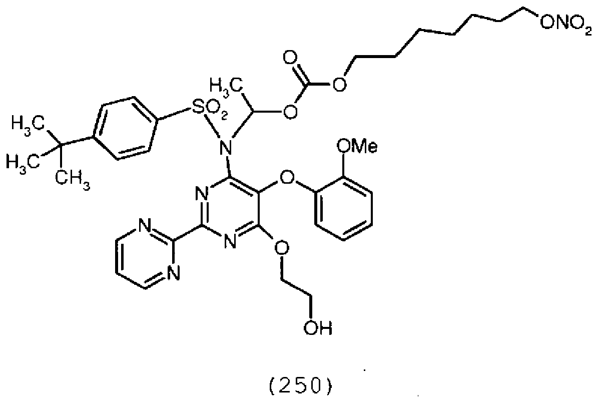

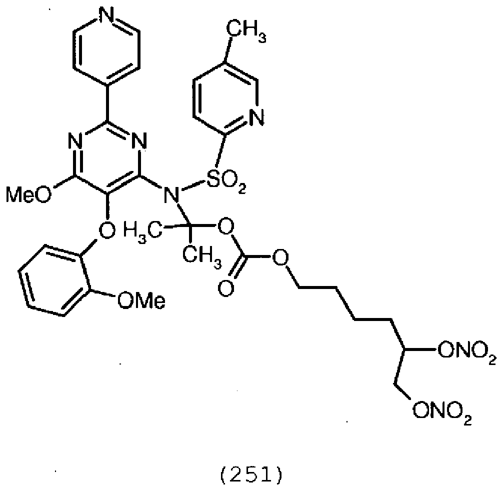

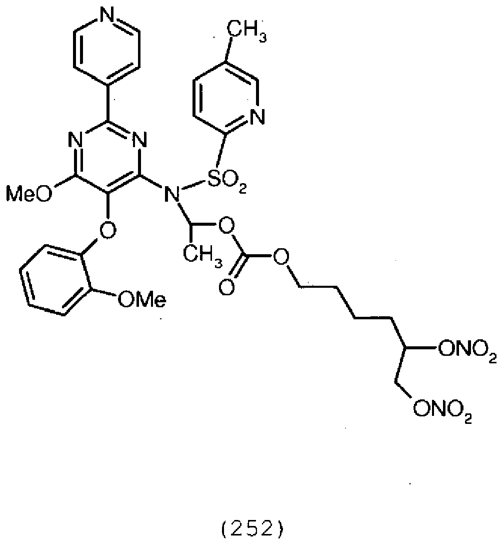

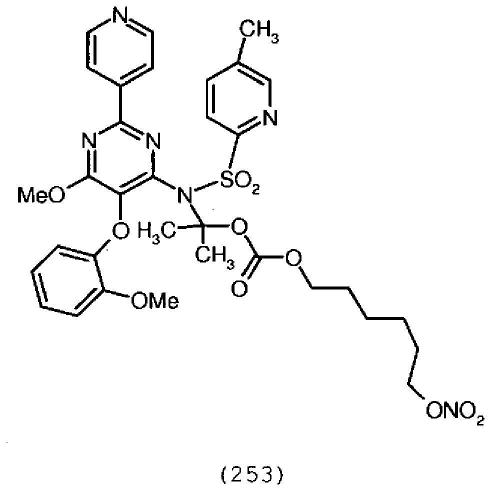

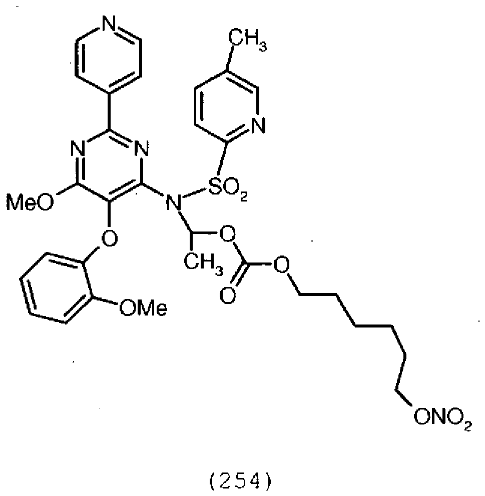

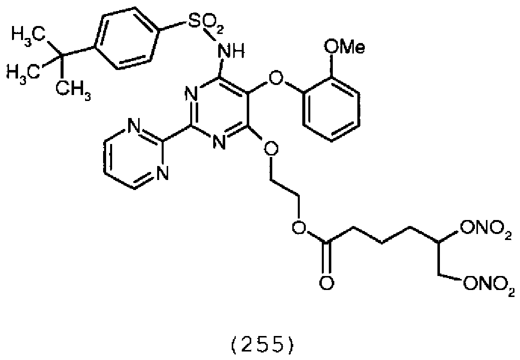

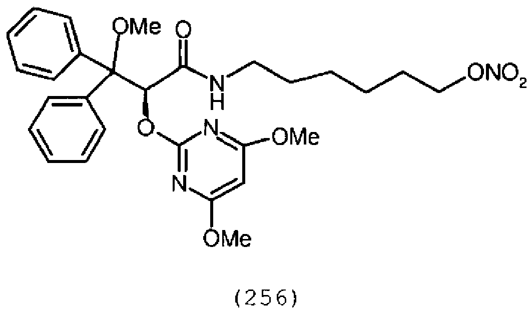

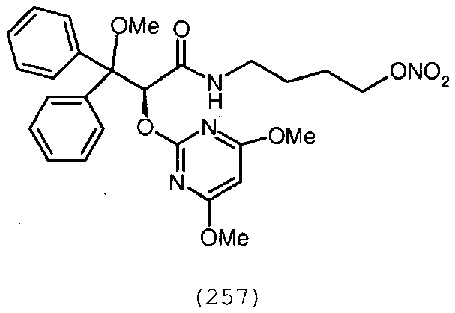

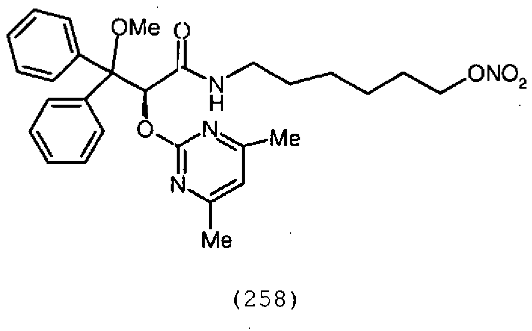

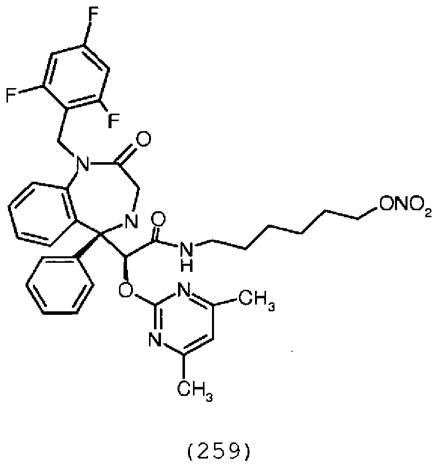

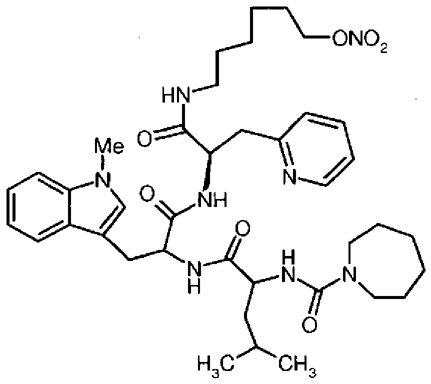

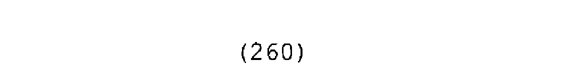

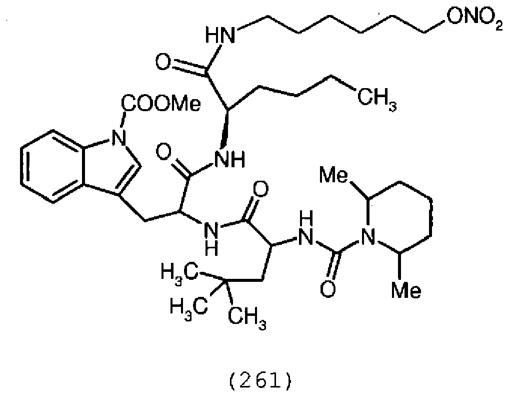

4. Composé de formule générale (I) selon les revendications 1 à 3 pour usage comme médicament.

5. Utilisation d'un composé selon les revendications 1 à 3 pour préparer un médicament qui peut être employé dans le traitement ou la prophylaxie de troubles liés à l'endothélium, de maladies rénales, pulmonaires, cardiaques et vasculaires, et de processus inflammatoires.

6. Utilisation d'un composé selon la revendication 5, pour préparer un médicament que l'on peut employer dans le traitement ou la prophylaxie d'une insuffisance cardiaque congestive, de maladies coronariennes, d'un dysfonctionnement et d'une hypertrophie du ventricule gauche, d'une fibrose cardiaque, d'une ischémie myocardique, d'une attaque, d'une hémorragie sous-arachnoïdienne, d'un vasospasme cérébrovasculaire, d'un vasospasme coronaire, d'une athérosclérose, d'une resténose post-angioplastie, d'une ischémie rénale, d'une insuffisance rénale, d'une fibrose rénale et pulmonaire, d'une glomérulonéphrite, d'une colique rénale, d'une hypertension oculaire, d'un glaucome, d'une hypertension systémique, d'une hypertension artérielle pulmonaire (PAH), de complications diabétiques, telles que néphropathie, vasculopathie et neuropathie, de maladies vasculaires périphériques, d'une fibrose du foie, d'une hypertension portale, de syndromes métaboliques, d'un dysfonctionnement érectile, de complications après une intervention chirurgicale vasculaire ou cardiaque, de complications de traitement avec des agents immunosuppresseurs à la suite d'une transplantation d'organe, d'un hyperaldostéronisme, de fibrose pulmonaire, de sclérodermie, de drépanocytose, d'hyperplasie prostatique bénigne, de cancer, d'anxiété et de troubles cognitifs.

7. Composition pharmaceutique comprenant un véhicule acceptable au plan pharmaceutique et une quantité efficace au plan pharmaceutique d'un composé de formule générale (I) ou d'un sel ou d'un stéréoisomère de celui-ci selon les revendications 1 à 3.

8. Composition pharmaceutique selon la revendication 7 sous forme appropriée pour une administration par voie orale, parentérale, rectale, topique et transdermique, par des dispositifs d'inhalation, de pulvérisation ou d'aérosol ou encore d'iontophorèse.

9. Composition pharmaceutique liquide ou solide pour administration par voie orale, parentérale, rectale, topique et transdermique ou par inhalation sous la forme de comprimés, de gélules et de pilules, éventuellement avec un enrobage entérique, de poudres, de granulés, de gels, d'émulsions, de solutions, de suspensions, de sirops, d'élixirs, de formes injectables, de suppositoires, de timbres transdermiques ou de liposomes, contenant un composé de formule (I) ou un sel ou un stéréoisomère de celui-ci selon les revendications 1 à 3 et un véhicule acceptable au plan pharmaceutique.

10. Composition pharmaceutique comprenant un composé de formule générale (I) selon les revendications 1 à 3, au moins un composé utilisé pour traiter une maladie cardiovasculaire et un véhicule acceptable au plan pharmaceutique.

11. Composition pharmaceutique selon la revendication 10, dans laquelle le composé utilisé pour traiter une maladie cardiovasculaire est sélectionné dans le groupe constitué d'antagonistes de l'aldostérone, d'agents bloquants des récepteurs de l'angiotensine II, d'inhibiteurs de l'ACE, d'inhibiteurs de la HMGCoA réductase, d'agents bloquants bêta-adrénergiques, d'antagonistes alpha-adrénergiques, d'agents sympatholytiques, d'agents de blocage des canaux calciques, d'inhibiteurs de rénine, d'inhibiteurs d'endopeptidase neutres, d'activateurs de potassium, de diurétiques, de vasodilatateurs, d'agents antithrombotiques, tels que l'aspirine ou ses composés nitrosés.

12. Trousse pharmaceutique comprenant un composé de formule générale (I), tel que défini dans la revendication 1, un composé utilisé pour traiter une maladie cardiovasculaire sous la forme d'une préparation combinée pour un usage simultané, séparé ou séquentiel pour le traitement d'une maladie cardiovasculaire.

13. Trousse pharmaceutique selon la revendication 12, dans laquelle le composé utilisé pour traiter la maladie cardiovasculaire est sélectionné dans le groupe constitué des antagonistes de l'aldostérone, des agents bloquants des récepteurs de l'angiotensine II, des inhibiteurs de l'ACE, des inhibiteurs de la HMGCoA réductase, des agents bloquants bêta-adrénergiques, des antagonistes alpha-adrénergiques, des agents sympatholytiques, des agents de blocage des canaux calciques, des inhibiteurs de rénine, des inhibiteurs d'endopeptidase neutres, des activateurs de potassium, des diurétiques, des vasodilatateurs, des agents antithrombotiques, tels que l'aspirine ou ses composés nitrosés.
